# EUROPÄISCHE PATENTSCHRIFT

(11) **EP 1 991 684 B1**
(45) Veröffentlichungstag und Bekanntmachung des Hinweises auf die Patenterteilung: **10.04.2013**
(21) Anmeldenummer: 07712274.5
(22) Anmeldetag: 21.02.2007
(51) Int. Cl.: C12N 9/10, C12N 9/00, C12N 15/82

(54) **VERFAHREN ZUR HERSTELLUNG VON MEHRFACH UNGESÄTTIGTEN FETTSÄUREN**
METHOD FOR PRODUCING POLYUNSATURATED FATTY ACIDS
PROCÉDÉ POUR PRODUIRE DES ACIDES GRAS POLYINSATURÉS

(30) Priorität: 21.02.2006 DE 102006008030; 07.09.2006 EP 06120309
(43) Veröffentlichungstag der Anmeldung: 19.11.2008
(73) Patentinhaber: BASF Plant Science GmbH, 67056 Ludwigshafen (DE)
(72) Erfinder: CIRPUS, Petra, 68163 Mannheim (DE); BAUER, Jörg, 67117 Limburgerhof (DE); QIU, Xiao, Saskatoon, Saskatchewan S7N 3S5 (CA); WU, Guohai, Saskatoon, Saskatchewan S7N 3S5 (CA); CHENG, Bifang, Saskatoon, Saskatchewan S7N 0W9 (CA); TRUKSA, Martin, Saskatoon, Saskatchewan S7N 0W9 (CA); WETJEN, Tom, 68163 Mannheim (DE)
(86) Internationale Anmeldenummer: PCT/EP2007/051675
(87) Internationale Veröffentlichungsnummer: WO 2007/096387

(56) Entgegenhaltungen:
- WO-A-2005/083093
- WO-A1-2005/103253
- WO-A2-02/081668
- QI B ET AL: "Production of very long chain polyunsaturated omega-3 and omega-6 fatty acids in plants" NATURE BIOTECHNOLOGY, NATURE PUBLISHING GROUP, NEW YORK, NY, US, Bd. 22, Nr. 6, Juni 2004 (2004-06), Seiten 739-745, XP002348313 ISSN: 1087-0156

### SEQUENCE LISTING

<110> BASF Plant Science GmbH
<120> verfahren zur Herstellung von mehrfach ungesättigten Fettsäuren
<130> B 8172
<150> DE 10 2006 008 030.0
   <151> 2006 02 21
<150> EP 06120309.7
   <151> 2006 09 07
<160> 199
<170> Biomax PatentTool according to PatentIN 3.1 format
<210> 1
   <211> 1380
   <212> DNA
   <213> Phytium irregulare
<220>
   <221> CDS
   <222> (1)..(1380)
<400> 1
   atg gtg gac ctc aag cct gga gtg aag cgc ctg gtg agc tgg aag gag      48
   Met Val Asp Leu Lys Pro Gly Val Lys Arg Leu Val Ser Trp Lys Glu
   1               5                   10                  15
   atc cgc gag cac gcg acg ccc gcg acc gcg tgg atc gtg att cac cac      96
   Ile Arg Glu His Ala Thr Pro Ala Thr Ala Trp Ile Val Ile His His
               20                  25                  30
   aag gtc tac gac atc tcc aag tgg gac tcg cac ccg ggt ggc tcc gtg      144
   Lys Val Tyr Asp Ile Ser Lys Trp Asp Ser His Pro Gly Gly Ser Val
           35                  40                  45
   atg ctc acg cag gcc ggc gag gac gcc acg gac gcc ttc gcg gtc ttc      192
   Met Leu Thr Gln Ala Gly Glu Asp Ala Thr Asp Ala Phe Ala Val Phe
       50                  55                  60
   cac ccg tcc tcg gcg ctc aag ctg ctc gag cag ttc tac gtc ggc gac      240
   His Pro Ser Ser Ala Leu Lys Leu Leu Glu Gln Phe Tyr Val Gly Asp
   65                  70                  75                  80
   gtg gac gaa acc tcc aag gcc gag atc gag ggg gag ccg gcg agc gac      288
   Val Asp Glu Thr Ser Lys Ala Glu Ile Glu Gly Glu Pro Ala Ser Asp
                   85                  90                  95
   gag gag cgc gcg cgc cgc gag cgc atc aac gag ttc atc gcg tcc tac      336
   Glu Glu Arg Ala Arg Arg Glu Arg Ile Asn Glu Phe Ile Ala Ser Tyr
               100                 105                 110
   cgc cgt ctg cgc gtc aag gtc aag ggc atg ggg ctc tac gac gcc agc      384
   Arg Arg Leu Arg Val Lys Val Lys Gly Met Gly Leu Tyr Asp Ala Ser
           115                 120                 125
   gcg ctc tac tac gcg tgg aag ctc gtg agc acg ttc ggc atc gcg gtg      432
   Ala Leu Tyr Tyr Ala Trp Lys Leu Val Ser Thr Phe Gly Ile Ala Val
       130                 135                 140
   ctc tcg atg gcg atc tgc ttc ttc ttc aac agt ttc gcc atg tac atg      480
   Leu Ser Met Ala Ile Cys Phe Phe Phe Asn Ser Phe Ala Met Tyr Met
   145                 150                 155                 160
   gtc gcc ggc gtg att atg ggg ctc ttc tac cag cag tcc gga tgg ctg      528
   Val Ala Gly Val Ile Met Gly Leu Phe Tyr Gln Gln Ser Gly Trp Leu
                   165                 170                 175
   gcg cac gac ttc ttg cac aac cag gtg tgc gag aac cgc acg ctc ggc      576
   Ala His Asp Phe Leu His Asn Gln Val Cys Glu Asn Arg Thr Leu Gly
               180                 185                 190
   aac ctt atc ggc tgc ctc gtg ggc aac gcc tgg cag ggc ttc agc atg      624
   Asn Leu Ile Gly Cys Leu Val Gly Asn Ala Trp Gln Gly Phe Ser Met
           195                 200                 205
   cag tgg tgg aag aac aag cac aac ctg cac cac gcg gtg ccg aac ctg      672
   Gln Trp Trp Lys Asn Lys His Asn Leu His His Ala Val Pro Asn Leu
       210                 215                 220
   cac agc gcc aag gac gag ggc ttc atc ggc gac ccg gac atc gac acc      720
   His Ser Ala Lys Asp Glu Gly Phe Ile Gly Asp Pro Asp Ile Asp Thr
   225                     230                 235                 240
   atg ccg ctg ctg gcg tgg tct aag gag atg gcg cgc aag gcg ttc gag      768
   Met Pro Leu Leu Ala Trp Ser Lys Glu Met Ala Arg Lys Ala Phe Glu
                   245                 250                 255
   tcg gcg cac ggc ccg ttc ttc atc cgc aac cag gcg ttc cta tac ttc      816
   Ser Ala His Gly Pro Phe Phe Ile Arg Asn Gln Ala Phe Leu Tyr Phe
               260                 265                 270
   ccg ctg ctg ctg ctc gcg cgc ctg agc tgg ctc gcg cag tcg ttc ttc      864
   Pro Leu Leu Leu Leu Ala Arg Leu Ser Trp Leu Ala Gln Ser Phe Phe
           275                 280                 285
   tac gtg ttc acc gag ttc tcg ttc ggc atc ttc gac aag gtc gag ttc      912
   Tyr Val Phe Thr Glu Phe Ser Phe Gly Ile Phe Asp Lys Val Glu Phe
       290                 295                 300
   gac gga ccg gag aag gcg ggt ctg atc gtg cac tac atc tgg cag ctc      960
   Asp Gly Pro Glu Lys Ala Gly Leu Ile Val His Tyr Ile Trp Gln Leu
   305                     310                 315                 320
   gcg atc ccg tac ttc tgc aac atg agc ctg ttt gag ggc gtg gca tac     1008
   Ala Ile Pro Tyr Phe Cys Asn Met Ser Leu Phe Glu Gly Val Ala Tyr
                   325                 330                 335
   ttc ctc atg ggc cag gcg tcc tgc ggc ttg ctc ctg gcg ctg gtg ttc     1056
   Phe Leu Met Gly Gln Ala Ser Cys Gly Leu Leu Leu Ala Leu Val Phe
               340                 345                 350
   agt att ggc cac aac ggc atg tcg gtg tac gag cgc gaa acc aag ccg     1104
   Ser Ile Gly His Asn Gly Met Ser Val Tyr Glu Arg Glu Thr Lys Pro
           355                 360                 365
   gac ttc tgg cag ctg cag gtg acc acg acg cgc aac atc cgc gcg tcg     1152
   Asp Phe Trp Gln Leu Gln Val Thr Thr Thr Arg Asn Ile Arg Ala Ser
       370                 375                 380
   gta ttc atg gac tgg ttc acc ggt ggc ttg aac tac cag atc gac cat     1200
   Val Phe Met Asp Trp Phe Thr Gly Gly Leu Asn Tyr Gln Ile Asp His
   385                     390                 395                 400
   cac ctg ttc ccg ctc gtg ccg cgc cac aac ttg cca aag gtc aac gtg     1248
   His Leu Phe Pro Leu Val Pro Arg His Asn Leu Pro Lys Val Asn Val
                   405                 410                 415
   ctc atc aag tcg cta tgc aag gag ttc gac atc ccg ttc cac gag acc     1296
   Leu Ile Lys Ser Leu Cys Lys Glu Phe Asp Ile Pro Phe His Glu Thr
               420                 425                 430
   ggc ttc tgg gag ggc atc tac gag gtc gtg gac cac ctg gcg gac atc     1344
   Gly Phe Trp Glu Gly Ile Tyr Glu Val Val Asp His Leu Ala Asp Ile
           435                 440                 445
   agc aag gaa ttt atc acc gag ttc cca gcg atg taa                     1380
   Ser Lys Glu Phe Ile Thr Glu Phe Pro Ala Met
       450                 455                 460
<210> 2
   <211> 459
   <212> PRT
   <213> Phytium irregulare
<400> 2
   Met Val Asp Leu Lys Pro Gly Val Lys Arg Leu Val Ser Trp Lys Glu
   1               5                   10                  15
   Ile Arg Glu His Ala Thr Pro Ala Thr Ala Trp Ile Val Ile His His
               20                  25                  30
   Lys Val Tyr Asp Ile Ser Lys Trp Asp Ser His Pro Gly Gly Ser Val
           35                  40                  45
   Met Leu Thr Gln Ala Gly Glu Asp Ala Thr Asp Ala Phe Ala Val Phe
       50                  55                  60
   His Pro Ser Ser Ala Leu Lys Leu Leu Glu Gln Phe Tyr Val Gly Asp
   65                  70                  75                  80
   Val Asp Glu Thr Ser Lys Ala Glu Ile Glu Gly Glu Pro Ala Ser Asp
                   85                  90                  95
   Glu Glu Arg Ala Arg Arg Glu Arg Ile Asn Glu Phe Ile Ala Ser Tyr
               100                 105                 110
   Arg Arg Leu Arg Val Lys Val Lys Gly Met Gly Leu Tyr Asp Ala Ser
           115                 120                 125
   Ala Leu Tyr Tyr Ala Trp Lys Leu Val Ser Thr Phe Gly Ile Ala Val
       130                 135                 140
   Leu Ser Met Ala Ile Cys Phe Phe Phe Asn Ser Phe Ala Met Tyr Met
   145                 150                 155                 160
   Val Ala Gly Val Ile Met Gly Leu Phe Tyr Gln Gln Ser Gly Trp Leu
                   165                 170                 175
   Ala His Asp Phe Leu His Asn Gln Val Cys Glu Asn Arg Thr Leu Gly
               180                 185                 190
   Asn Leu Ile Gly Cys Leu Val Gly Asn Ala Trp Gln Gly Phe Ser Met
           195                 200                 205
   Gln Trp Trp Lys Asn Lys His Asn Leu His His Ala Val Pro Asn Leu
       210                 215                 220
   His Ser Ala Lys Asp Glu Gly Phe Ile Gly Asp Pro Asp Ile Asp Thr
   225                 230                 235                 240
   Met Pro Leu Leu Ala Trp Ser Lys Glu Met Ala Arg Lys Ala Phe Glu
                   245                 250                 255
   Ser Ala His Gly Pro Phe Phe Ile Arg Asn Gln Ala Phe Leu Tyr Phe
               260                 265                 270
   Pro Leu Leu Leu Leu Ala Arg Leu Ser Trp Leu Ala Gln Ser Phe Phe
           275                 280                 285
   Tyr Val Phe Thr Glu Phe Ser Phe Gly Ile Phe Asp Lys Val Glu Phe
       290                 295                 300
   Asp Gly Pro Glu Lys Ala Gly Leu Ile Val His Tyr Ile Trp Gln Leu
   305                 310                 315                 320
   Ala Ile Pro Tyr Phe Cys Asn Met Ser Leu Phe Glu Gly Val Ala Tyr
                   325                 330                 335
   Phe Leu Met Gly Gln Ala Ser Cys Gly Leu Leu Leu Ala Leu Val Phe
               340                 345                 350
   Ser Ile Gly His Asn Gly Met Ser Val Tyr Glu Arg Glu Thr Lys Pro
           355                 360                 365
   Asp Phe Trp Gln Leu Gln Val Thr Thr Thr Arg Asn Ile Arg Ala Ser
       370                 375                 380
   Val Phe Met Asp Trp Phe Thr Gly Gly Leu Asn Tyr Gln Ile Asp His
   385                 390                 395                 400
   His Leu Phe Pro Leu Val Pro Arg His Asn Leu Pro Lys Val Asn Val
                   405                 410                 415
   Leu Ile Lys Ser Leu Cys Lys Glu Phe Asp Ile Pro Phe His Glu Thr
               420                 425                 430
   Gly Phe Trp Glu Gly Ile Tyr Glu Val Val Asp His Leu Ala Asp Ile
           435                 440                 445
   Ser Lys Glu Phe Ile Thr Glu Phe Pro Ala Met
       450                 455
<210> 3
   <211> 1380
   <212> DNA
   <213> Rhizopus stolonifer
<220>
   <221> CDS
   <222> (1)..(1380)
<400> 3
   atg agt aca tta gat cgt caa tct att ttc aca ata aag gaa tta gag      48
   Met Ser Thr Leu Asp Arg Gln Ser Ile Phe Thr Ile Lys Glu Leu Glu
   1               5                   10                  15
   tcg atc agt caa cgt att cat gat gga gat gaa gag gct atg aag ttc      96
   Ser Ile Ser Gln Arg Ile His Asp Gly Asp Glu Glu Ala Met Lys Phe
               20                  25                  30
   att att atc gac aag aag gtg tac gat gtt act gaa ttt att gaa gac      144
   Ile Ile Ile Asp Lys Lys Val Tyr Asp Val Thr Glu Phe Ile Glu Asp
           35                  40                  45
   cac cct ggt ggt gct caa gtt ttg ttg act cat gtt ggg aaa gat gca      192
   His Pro Gly Gly Ala Gln Val Leu Leu Thr His Val Gly Lys Asp Ala
       50                  55                  60
   tct gac gta ttt cat gcc atg cat ccc gaa tct gct tac gaa gtc ttg      240
   Ser Asp Val Phe His Ala Met His Pro Glu Ser Ala Tyr Glu Val Leu
   65                  70                  75                  80
   aac aat tac ttc gta ggc gat gtt caa gaa aca gta gtc act gaa aaa      288
   Asn Asn Tyr Phe Val Gly Asp Val Gln Glu Thr Val Val Thr Glu Lys
                   85                  90                  95
   tct tct tct gcc caa ttc gct gta gaa atg cgt cag ctt cgg gat caa      336
   Ser Ser Ser Ala Gln Phe Ala Val Glu Met Arg Gln Leu Arg Asp Gln
               100                 105                 110
   ttg aaa aag gaa ggt tat ttc cac tcc agc aaa ctc ttt tat gct tac      384
   Leu Lys Lys Glu Gly Tyr Phe His Ser Ser Lys Leu Phe Tyr Ala Tyr
           115                 120                 125
   aaa gtt ctt tct act ttg gct atc tgc ata gct ggt ctc tct cct ttg      432
   Lys Val Leu Ser Thr Leu Ala Ile Cys Ile Ala Gly Leu Ser Pro Leu
       130                 135                 140
   tat gct tat ggt cgt act tcc act ttg gct gtc gtc gca tct gct att      480
   Tyr Ala Tyr Gly Arg Thr Ser Thr Leu Ala Val Val Ala Ser Ala Ile
   145                 150                 155                 160
   act gtt ggt atc ttt tgg caa cag tgt ggt tgg ctg gct cac gat ttc      528
   Thr Val Gly Ile Phe Trp Gln Gln Cys Gly Trp Leu Ala His Asp Phe
                   165                 170                 175
   gga cat cat caa tgc ttc gaa gac cgc act tgg aac gat gtt ctc gtc      576
   Gly His His Gln Cys Phe Glu Asp Arg Thr Trp Asn Asp Val Leu Val
               180                 185                 190
   gta ttt ctc gga aac ttt tgt caa gga ttt tca tta tca tgg tgg aag      624
   Val Phe Leu Gly Asn Phe Cys Gln Gly Phe Ser Leu Ser Trp Trp Lys
           195                 200                 205
   aac aag cac aac aca cat cac gca agt aca aat gtt cat gga cag gat      672
   Asn Lys His Asn Thr His His Ala Ser Thr Asn Val His Gly Gln Asp
       210                 215                 220
   ccc gac att gac aca gcc cct gtt ttg ttg tgg gat gaa tat gct tct      720
   Pro Asp Ile Asp Thr Ala Pro Val Leu Leu Trp Asp Glu Tyr Ala Ser
   225                 230                 235                 240
   gca gct tac tat gct tct ctc gat caa gaa ccc aca atg gta tct cga      768
   Ala Ala Tyr Tyr Ala Ser Leu Asp Gln Glu Pro Thr Met Val Ser Arg
                   245                 250                 255
   ttc ctt gct gaa caa gtc ttg ccc cac caa acc cgt tat ttt ttc ttt      816
   Phe Leu Ala Glu Gln Val Leu Pro His Gln Thr Arg Tyr Phe Phe Phe
               260                 265                 270
   atc ctc gct ttt gct cgt ttg tct tgg gct ctt caa tct ttg agc tac      864
   Ile Leu Ala Phe Ala Arg Leu Ser Trp Ala Leu Gln Ser Leu Ser Tyr
           275                 280                 285
   tct ttc aag aag gag tca att aac aag tcc cgt caa ctt aac ctt ttt      912
   Ser Phe Lys Lys Glu Ser Ile Asn Lys Ser Arg Gln Leu Asn Leu Phe
       290                 295                 300
   gaa cgt gtt tgt att gtt ggt cac tgg gct ctc tct gct ttt tgt atc      960
   Glu Arg Val Cys Ile Val Gly His Trp Ala Leu Ser Ala Phe Cys Ile
   305                 310                 315                 320
   tac tct tgg tgt agc aac gtc tat cac atg gtt ttg ttt ttc ttg gtt     1008
   Tyr Ser Trp Cys Ser Asn Val Tyr His Met Val Leu Phe Phe Leu Val
                   325                 330                 335
   agt cag gct acc aca ggc tac act tta gct ctt gtc ttt gct ttg aac     1056
   Ser Gln Ala Thr Thr Gly Tyr Thr Leu Ala Leu Val Phe Ala Leu Asn
               340                 345                 350
   cac aat ggt atg cct gtt atc act gaa gaa aag gct gaa tcg atg gaa     1104
   His Asn Gly Met Pro Val Ile Thr Glu Glu Lys Ala Glu Ser Met Glu
           355                 360                 365
   ttt ttt gag att caa gtt atc act ggt cgc gat gtt act ctt tcc ccc     1152
   Phe Phe Glu Ile Gln Val Ile Thr Gly Arg Asp Val Thr Leu Ser Pro
       370                 375                 380
   ttg ggt gat tgg ttc atg ggt ggt ttg aac tat caa atc gaa cat cat     1200
   Leu Gly Asp Trp Phe Met Gly Gly Leu Asn Tyr Gln Ile Glu His His
   385                 390                 395                 400
   gtt ttc ccc aac atg cct cgt cac aac ttg cct aca gtt aag ccc atg     1248
   Val Phe Pro Asn Met Pro Arg His Asn Leu Pro Thr Val Lys Pro Met
                   405                 410                 415
   gtg aaa tcg ctt tgt cag aaa tat gac att aac tat cac gat act gga     1296
   Val Lys Ser Leu Cys Gln Lys Tyr Asp Ile Asn Tyr His Asp Thr Gly
                       420                     425                     430
   ttc tta aag gga aca tta gaa gta tta caa aca ctg gat att aca tcc        1344
   Phe Leu Lys Gly Thr Leu Glu Val Leu Gln Thr Leu Asp Ile Thr Ser
                   435                     440                     445
   aag ctc tct ttg caa tta agc aaa aag tcg ttt taa                        1380
   Lys Leu Ser Leu Gln Leu Ser Lys Lys Ser Phe
           450                     455
<210> 4
   <211> 459
   <212> PRT
   <213> Rhizopus stolonifer
<400> 4
   Met Ser Thr Leu Asp Arg Gln Ser Ile Phe Thr Ile Lys Glu Leu Glu
   1               5                   10                  15
   Ser Ile Ser Gln Arg Ile His Asp Gly Asp Glu Glu Ala Met Lys Phe
               20                  25                  30
   Ile Ile Ile Asp Lys Lys Val Tyr Asp Val Thr Glu Phe Ile Glu Asp
           35                  40                  45
   His Pro Gly Gly Ala Gln Val Leu Leu Thr His Val Gly Lys Asp Ala
       50                  55                  60
   Ser Asp Val Phe His Ala Met His Pro Glu Ser Ala Tyr Glu Val Leu
   65                  70                  75                  80
   Asn Asn Tyr Phe Val Gly Asp Val Gln Glu Thr Val Val Thr Glu Lys
                   85                  90                  95
   Ser Ser Ser Ala Gln Phe Ala Val Glu Met Arg Gln Leu Arg Asp Gln
               100                 105                 110
   Leu Lys Lys Glu Gly Tyr Phe His Ser Ser Lys Leu Phe Tyr Ala Tyr
           115                 120                 125
   Lys Val Leu Ser Thr Leu Ala Ile Cys Ile Ala Gly Leu Ser Pro Leu
       130                 135                 140
   Tyr Ala Tyr Gly Arg Thr Ser Thr Leu Ala Val Val Ala Ser Ala Ile
   145                 150                 155                 160
   Thr Val Gly Ile Phe Trp Gln Gln Cys Gly Trp Leu Ala His Asp Phe
                   165                 170                 175
   Gly His His Gln Cys Phe Glu Asp Arg Thr Trp Asn Asp Val Leu Val
               180                 185                 190
   Val Phe Leu Gly Asn Phe Cys Gln Gly Phe Ser Leu Ser Trp Trp Lys
           195                 200                 205
   Asn Lys His Asn Thr His His Ala Ser Thr Asn Val His Gly Gln Asp
       210                 215                 220
   Pro Asp Ile Asp Thr Ala Pro Val Leu Leu Trp Asp Glu Tyr Ala Ser
   225                 230                 235                 240
   Ala Ala Tyr Tyr Ala Ser Leu Asp Gln Glu Pro Thr Met Val Ser Arg
                   245                 250                 255
   Phe Leu Ala Glu Gln Val Leu Pro His Gln Thr Arg Tyr Phe Phe Phe
               260                 265                 270
   Ile Leu Ala Phe Ala Arg Leu Ser Trp Ala Leu Gln Ser Leu Ser Tyr
           275                 280                 285
   Ser Phe Lys Lys Glu Ser Ile Asn Lys Ser Arg Gln Leu Asn Leu Phe
       290                 295                 300
   Glu Arg Val Cys Ile Val Gly His Trp Ala Leu Ser Ala Phe Cys Ile
   305                 310                 315                 320
   Tyr Ser Trp Cys Ser Asn Val Tyr His Met Val Leu Phe Phe Leu Val
                   325                 330                 335
   Ser Gln Ala Thr Thr Gly Tyr Thr Leu Ala Leu Val Phe Ala Leu Asn
               340                 345                 350
   His Asn Gly Met Pro Val Ile Thr Glu Glu Lys Ala Glu Ser Met Glu
           355                 360                 365
   Phe Phe Glu Ile Gln Val Ile Thr Gly Arg Asp Val Thr Leu Ser Pro
       370                 375                 380
   Leu Gly Asp Trp Phe Met Gly Gly Leu Asn Tyr Gln Ile Glu His His
   385                 390                 395                 400
   Val Phe Pro Asn Met Pro Arg His Asn Leu Pro Thr Val Lys Pro Met
                   405                 410                 415
   Val Lys Ser Leu Cys Gln Lys Tyr Asp Ile Asn Tyr His Asp Thr Gly
               420                 425                 430
   Phe Leu Lys Gly Thr Leu Glu Val Leu Gln Thr Leu Asp Ile Thr Ser
           435                 440                 445
   Lys Leu Ser Leu Gln Leu Ser Lys Lys Ser Phe
       450                 455
<210> 5
   <211> 1380
   <212> DNA
   <213> Rhizopus stolonifer
<220>
   <221> CDS
   <222> (1)..(1380)
<400> 5
   atg agt aca tta gat cgt caa tct att ttc aca ata aag gaa tta gag        48
   Met Ser Thr Leu Asp Arg Gln Ser Ile Phe Thr Ile Lys Glu Leu Glu
   1               5                   10                  15
   tcg atc agt caa cgt att cat gat gga gat gaa gag gct atg aag ttc        96
   Ser Ile Ser Gln Arg Ile His Asp Gly Asp Glu Glu Ala Met Lys Phe
               20                  25                  30
   att att atc gac aag aag gtg tac gat gtt act gaa ttt att gaa gac        144
   Ile Ile Ile Asp Lys Lys Val Tyr Asp Val Thr Glu Phe Ile Glu Asp
           35                  40                  45
   cac cct ggt ggt gct caa gtt ttg ttg act cat gtt ggg aaa gat gca        192
   His Pro Gly Gly Ala Gln Val Leu Leu Thr His Val Gly Lys Asp Ala
       50                  55                  60
   tct gac gta ttt cat gcc atg cat ccc gaa tct gct tac gaa gtc ttg        240
   Ser Asp Val Phe His Ala Met His Pro Glu Ser Ala Tyr Glu Val Leu
   65                  70                  75                  80
   aac aat tac ttc gta ggc gat gtt caa gaa aca gta gtc act gaa aaa        288
   Asn Asn Tyr Phe Val Gly Asp Val Gln Glu Thr Val Val Thr Glu Lys
                   85                  90                  95
   tct tct tct gcc caa ttc gct gta gaa atg cgt cag ctt cgg gat caa        336
   Ser Ser Ser Ala Gln Phe Ala Val Glu Met Arg Gln Leu Arg Asp Gln
               100                 105                 110
   ttg aaa aag gaa ggt tat ttc cac tcc agc aaa ctc ttt tat gct tac        384
   Leu Lys Lys Glu Gly Tyr Phe His Ser Ser Lys Leu Phe Tyr Ala Tyr
           115                 120                 125
   aaa gtt ctt tct act ttg gct atc tgc ata gct ggt ctc tct ctt ttg        432
   Lys Val Leu Ser Thr Leu Ala Ile Cys Ile Ala Gly Leu Ser Leu Leu
       130                 135                 140
   tat gct tat ggt cgt act tcc act ttg gct gtc gtc gca tct gct att        480
   Tyr Ala Tyr Gly Arg Thr Ser Thr Leu Ala Val Val Ala Ser Ala Ile
   145                 150                 155                 160
   act gtt ggt atc ttt tgg caa cag tgt ggt tgg ctg gct cac gat ttc        528
   Thr Val Gly Ile Phe Trp Gln Gln Cys Gly Trp Leu Ala His Asp Phe
                   165                 170                 175
   gga cat cat caa tgc ttc gaa gac cgc act tgg aac gat gtt ctc gtc        576
   Gly His His Gln Cys Phe Glu Asp Arg Thr Trp Asn Asp Val Leu Val
               180                 185                 190
   gta ttt ctc gga aac ttt tgt caa gga ttt tca tta tca tgg tgg aag        624
   Val Phe Leu Gly Asn Phe Cys Gln Gly Phe Ser Leu Ser Trp Trp Lys
           195                 200                 205
   aac aag cac aac aca cat cac gca agt aca aat gtt cat gga cag gat        672
   Asn Lys His Asn Thr His His Ala Ser Thr Asn Val His Gly Gln Asp
       210                 215                 220
   ccc gac att gac aca gcc cct gtt ttg ttg tgg gat gaa tat gct tct        720
   Pro Asp Ile Asp Thr Ala Pro Val Leu Leu Trp Asp Glu Tyr Ala Ser
   225                 230                 235                 240
   gca gct tac tat gct tct ctc gat caa gaa ccc aca atg gta tct cga        768
   Ala Ala Tyr Tyr Ala Ser Leu Asp Gln Glu Pro Thr Met Val Ser Arg
                   245                 250                 255
   ttc ctt gct gaa caa gtc ttg ccc cac caa acc cgt tat ttt ttc ttt        816
   Phe Leu Ala Glu Gln Val Leu Pro His Gln Thr Arg Tyr Phe Phe Phe
               260                 265                 270
   atc ctc gct ttt gct cgt ttg tct tgg gct ctt caa tct ttg agc tac        864
   Ile Leu Ala Phe Ala Arg Leu Ser Trp Ala Leu Gln Ser Leu Ser Tyr
               275                     280                     285
   tct ttc aag aag gag tca att aac aag tcc cgt caa ctt aac ctt ttt        912
   Ser Phe Lys Lys Glu Ser Ile Asn Lys Ser Arg Gln Leu Asn Leu Phe
           290                     295                     300
   gaa cgt gtt tgt att gtt ggt cac tgg gct ctc ttt gct ttt tgt atc        960
   Glu Arg Val Cys Ile Val Gly His Trp Ala Leu Phe Ala Phe Cys Ile
   305                     310                     315                     320
   tac tct tgg tgt agc aac gtc tat cac atg gtt ttg ttt ttc ttg gtt        1008
   Tyr Ser Trp Cys Ser Asn Val Tyr His Met Val Leu Phe Phe Leu Val
                   325                     330                     335
   agt cag gct acc aca ggc tac act tta gct ctt gtc ttt gct ttg aac        1056
   Ser Gln Ala Thr Thr Gly Tyr Thr Leu Ala Leu Val Phe Ala Leu Asn
               340                     345                     350
   cac aat ggt atg cct gtt atc act gaa gaa aag gct gaa tcg atg gaa        1104
   His Asn Gly Met Pro Val Ile Thr Glu Glu Lys Ala Glu Ser Met Glu
           355                     360                     365
   ttt ttt gag att caa gtt atc act ggt cgt gat gtt act ctt tcc ccc        1152
   Phe Phe Glu Ile Gln Val Ile Thr Gly Arg Asp Val Thr Leu Ser Pro
       370                     375                     380
   ttg ggt gat tgg ttc atg ggt ggt ttg aac tat caa atc gaa cat cat        1200
   Leu Gly Asp Trp Phe Met Gly Gly Leu Asn Tyr Gln Ile Glu His His
   385                     390                     395                     400
   gtt ttc ccc aac atg cct cgt cac aac ttg cct aca gtt aag ccc atg        1248
   Val Phe Pro Asn Met Pro Arg His Asn Leu Pro Thr Val Lys Pro Met
                   405                     410                     415
   gtg aaa tcg ctt tgt cag aaa tat gac att aac tat cac gat act gga        1296
   Val Lys Ser Leu Cys Gln Lys Tyr Asp Ile Asn Tyr His Asp Thr Gly
               420                     425                     430
   ttc tta aag gga aca tta gaa gta tta caa aca ctg gat att aca tcc        1344
   Phe Leu Lys Gly Thr Leu Glu Val Leu Gln Thr Leu Asp Ile Thr Ser
           435                     440                     445
   aag ctc tct ttg caa tta agc aaa aag tcg ttt taa                        1380
   Lys Leu Ser Leu Gln Leu Ser Lys Lys Ser Phe
       450                     455
<210> 6
   <211> 459
   <212> PRT
   <213> Rhizopus stolonifer
<400> 6
   Met Ser Thr Leu Asp Arg Gln Ser Ile Phe Thr Ile Lys Glu Leu Glu
   1               5                   10                  15
   Ser Ile Ser Gln Arg Ile His Asp Gly Asp Glu Glu Ala Met Lys Phe
               20                  25                  30
   Ile Ile Ile Asp Lys Lys Val Tyr Asp Val Thr Glu Phe Ile Glu Asp
           35                  40                  45
   His Pro Gly Gly Ala Gln Val Leu Leu Thr His Val Gly Lys Asp Ala
       50                  55                  60
   Ser Asp Val Phe His Ala Met His Pro Glu Ser Ala Tyr Glu Val Leu
   65                  70                  75                  80
   Asn Asn Tyr Phe Val Gly Asp Val Gln Glu Thr Val Val Thr Glu Lys
                   85                  90                  95
   Ser Ser Ser Ala Gln Phe Ala Val Glu Met Arg Gln Leu Arg Asp Gln
               100                 105                 110
   Leu Lys Lys Glu Gly Tyr Phe His Ser Ser Lys Leu Phe Tyr Ala Tyr
           115                 120                 125
   Lys Val Leu Ser Thr Leu Ala Ile Cys Ile Ala Gly Leu Ser Leu Leu
       130                 135                 140
   Tyr Ala Tyr Gly Arg Thr Ser Thr Leu Ala Val Val Ala Ser Ala Ile
   145                 150                 155                 160
   Thr Val Gly Ile Phe Trp Gln Gln Cys Gly Trp Leu Ala His Asp Phe
                   165                 170                 175
   Gly His His Gln Cys Phe Glu Asp Arg Thr Trp Asn Asp Val Leu Val
               180                 185                 190
   Val Phe Leu Gly Asn Phe Cys Gln Gly Phe Ser Leu Ser Trp Trp Lys
           195                 200                 205
   Asn Lys His Asn Thr His His Ala Ser Thr Asn Val His Gly Gln Asp
       210                 215                 220
   Pro Asp Ile Asp Thr Ala Pro Val Leu Leu Trp Asp Glu Tyr Ala Ser
   225                 230                 235                 240
   Ala Ala Tyr Tyr Ala Ser Leu Asp Gln Glu Pro Thr Met Val Ser Arg
                   245                 250                 255
   Phe Leu Ala Glu Gln Val Leu Pro His Gln Thr Arg Tyr Phe Phe Phe
               260                 265                 270
   Ile Leu Ala Phe Ala Arg Leu Ser Trp Ala Leu Gln Ser Leu Ser Tyr
           275                 280                 285
   Ser Phe Lys Lys Glu Ser Ile Asn Lys Ser Arg Gln Leu Asn Leu Phe
       290                 295                 300
   Glu Arg Val Cys Ile Val Gly His Trp Ala Leu Phe Ala Phe Cys Ile
   305                 310                 315                 320
   Tyr Ser Trp Cys Ser Asn Val Tyr His Met Val Leu Phe Phe Leu Val
                   325                 330                 335
   Ser Gln Ala Thr Thr Gly Tyr Thr Leu Ala Leu Val Phe Ala Leu Asn
               340                 345                 350
   His Asn Gly Met Pro Val Ile Thr Glu Glu Lys Ala Glu Ser Met Glu
           355                 360                 365
   Phe Phe Glu Ile Gln Val Ile Thr Gly Arg Asp Val Thr Leu Ser Pro
       370                 375                 380
   Leu Gly Asp Trp Phe Met Gly Gly Leu Asn Tyr Gln Ile Glu His His
   385                 390                 395                 400
   Val Phe Pro Asn Met Pro Arg His Asn Leu Pro Thr Val Lys Pro Met
                   405                 410                 415
   Val Lys Ser Leu Cys Gln Lys Tyr Asp Ile Asn Tyr His Asp Thr Gly
               420                 425                 430
   Phe Leu Lys Gly Thr Leu Glu Val Leu Gln Thr Leu Asp Ile Thr Ser
           435                 440                 445
   Lys Leu Ser Leu Gln Leu Ser Lys Lys Ser Phe
       450                 455
<210> 7
   <211> 1401
   <212> DNA
   <213> Cunninghamella echinulata
<220>
   <221> CDS
   <222> (1)..(1401)
<400> 7
   atg tca ggg caa act cga gtt ttt aag cga tcc gaa gtg tcc gat tcg          48
   Met Ser Gly Gln Thr Arg Val Phe Lys Arg Ser Glu Val Ser Asp Ser
   1               5                   10                  15
   ctc aag gcg tac caa gcg ggc gac aag aac gcc gac aag ttc ctg att          96
   Leu Lys Ala Tyr Gln Ala Gly Asp Lys Asn Ala Asp Lys Phe Leu Ile
               20                  25                  30
   gtc gac aac aag gtg tac gac atc acg gat ttc att gct gat cat cct          144
   Val Asp Asn Lys Val Tyr Asp Ile Thr Asp Phe Ile Ala Asp His Pro
           35                  40                  45
   ggc ggt gct cag gtc atc tct acg cac att ggc aag gat gcc tct gat          192
   Gly Gly Ala Gln Val Ile Ser Thr His Ile Gly Lys Asp Ala Ser Asp
       50                  55                  60
   gtg ttt cat gcg atg cat ccc gag tct gcg tac gaa ttg ctt gcg aat          240
   Val Phe His Ala Met His Pro Glu Ser Ala Tyr Glu Leu Leu Ala Asn
   65                  70                  75                  80
   tgc tac gtt ggc gat ctt gct gcg gac cat gct ggt gtg cag ggc gaa          288
   Cys Tyr Val Gly Asp Leu Ala Ala Asp His Ala Gly Val Gln Gly Glu
                   85                  90                  95
   ttg gtg aat ggt gta cac aaa aag tcc aag gca ttt gcg gac gag atg          336
   Leu Val Asn Gly Val His Lys Lys Ser Lys Ala Phe Ala Asp Glu Met
               100                 105                 110
   cga tcg ttg cgc gag cgt ctt gag acg gag ggt gcc ttt aat ggc agt          384
   Arg Ser Leu Arg Glu Arg Leu Glu Thr Glu Gly Ala Phe Asn Gly Ser
           115                 120                 125
   gtt ccg ttt tac att tac aag gtg gtg tcg acg ttg gcc att ggt gcg          432
   Val Pro Phe Tyr Ile Tyr Lys Val Val Ser Thr Leu Ala Ile Gly Ala
       130                     135                     140
   acg ggt ctg gcc atg ctg tac tat ggc ggc cat tcg acg tcg gtc gtg          480
   Thr Gly Leu Ala Met Leu Tyr Tyr Gly Gly His Ser Thr Ser Val Val
   145                     150                     155                 160
   ctt gcg gcg gcg gtg gtt gtc ggt ctg ttt tgg caa cag tgc ggc tgg          528
   Leu Ala Ala Ala Val Val Val Gly Leu Phe Trp Gln Gln Cys Gly Trp
                   165                     170                     175
   ctc gcc cac gac ttt ggc cac cac cag gcg ttt gcg gac cat acg gtc          576
   Leu Ala His Asp Phe Gly His His Gln Ala Phe Ala Asp His Thr Val
               180                     185                     190
   aat gac gtg atg att gcg ttt ttg ggt gga ttc tgc caa ggt ttc tcg          624
   Asn Asp Val Met Ile Ala Phe Leu Gly Gly Phe Cys Gln Gly Phe Ser
           195                     200                     205
   ctg tcg tgg tgg aag aac aag cac aac act cac cac gcc tcc acc aac          672
   Leu Ser Trp Trp Lys Asn Lys His Asn Thr His His Ala Ser Thr Asn
       210                     215                     220
   gtc cac ggc cac gat ccc gat atc gat acg gcc cct gtc ctc ctc tgg          720
   Val His Gly His Asp Pro Asp Ile Asp Thr Ala Pro Val Leu Leu Trp
   225                     230                     235                 240
   gac gaa ttt gcc acc gcc aat ttc tat gga aac ctc gag ggt caa aag          768
   Asp Glu Phe Ala Thr Ala Asn Phe Tyr Gly Asn Leu Glu Gly Gln Lys
                   245                     250                     255
   gat tcg gcg ttc tcg cgc ttc att gcc gaa cac gtg ctc ccc tac cag          816
   Asp Ser Ala Phe Ser Arg Phe Ile Ala Glu His Val Leu Pro Tyr Gln
               260                     265                     270
   acg cgc tat tac ttt ttt gtc ctc ggc ttt gcg cgt ctc tcg tgg gcc          864
   Thr Arg Tyr Tyr Phe Phe Val Leu Gly Phe Ala Arg Leu Ser Trp Ala
           275                     280                     285
   atc caa tcg ctc caa tac tcg ttc act gtc ggc aca ctc aac aag tca          912
   Ile Gln Ser Leu Gln Tyr Ser Phe Thr Val Gly Thr Leu Asn Lys Ser
       290                     295                     300
   aag acc ctc aat ctg ttt gag cgc acc atg ctt gtc agc cat tgg atc          960
   Lys Thr Leu Asn Leu Phe Glu Arg Thr Met Leu Val Ser His Trp Ile
   305                     310                     315                 320
   ttg ttc acc act tgg acg ctc ctc ttc atc aat tca tgg acc aac atg         1008
   Leu Phe Thr Thr Trp Thr Leu Leu Phe Ile Asn Ser Trp Thr Asn Met
                   325                     330                     335
   gtc atg ttc ttt gtc gtc agc cag gcc acc acc ggc tac gcg ctt gcc         1056
   Val Met Phe Phe Val Val Ser Gln Ala Thr Thr Gly Tyr Ala Leu Ala
               340                     345                     350
   ctc gtt ttc gcc atg aac cac tcg ggc atg ccc gtc ctc acc caa gaa         1104
   Leu Val Phe Ala Met Asn His Ser Gly Met Pro Val Leu Thr Gln Glu
           355                     360                     365
   cag gcc caa aag atg gag ttc tac gag atc cag gtc gtc acc gcc cgc         1152
   Gln Ala Gln Lys Met Glu Phe Tyr Glu Ile Gln Val Val Thr Ala Arg
       370                     375                     380
   gac gtc acc ctc ggt gcc ctt ggc gac tgg ttc tgc ggc ggc ctc aac         1200
   Asp Val Thr Leu Gly Ala Leu Gly Asp Trp Phe Cys Gly Gly Leu Asn
   385                     390                     395                 400
   tat caa atc gag cac cat gta ttc cct gat atg ccc cgc cac tac ctg         1248
   Tyr Gln Ile Glu His His Val Phe Pro Asp Met Pro Arg His Tyr Leu
                   405                     410                     415
   ccc aag gtc aag cct caa gtc aag gct ctc tgc aaa aaa cac aac att         1296
   Pro Lys Val Lys Pro Gln Val Lys Ala Leu Cys Lys Lys His Asn Ile
               420                     425                     430
   ttg tac cat gat acc tct gct ttg cgc ggc acc ttg gaa gtg ctg caa         1344
   Leu Tyr His Asp Thr Ser Ala Leu Arg Gly Thr Leu Glu Val Leu Gln
           435                     440                     445
   acc ttg gat gtt gtc caa aag ctc tgc gca agt tct ctc aaa aga tgt         1392
   Thr Leu Asp Val Val Gln Lys Leu Cys Ala Ser Ser Leu Lys Arg Cys
       450                     455                     460
   ttt aga tga                                                             1401
   Phe Arg
   465
<210> 8
   <211> 466
   <212> PRT
   <213> Cunninghamella echinulata
<400> 8
   Met Ser Gly Gln Thr Arg Val Phe Lys Arg Ser Glu Val Ser Asp Ser
   1               5                   10                  15
   Leu Lys Ala Tyr Gln Ala Gly Asp Lys Asn Ala Asp Lys Phe Leu Ile
               20                  25                  30
   Val Asp Asn Lys Val Tyr Asp Ile Thr Asp Phe Ile Ala Asp His Pro
           35                  40                  45
   Gly Gly Ala Gln Val Ile Ser Thr His Ile Gly Lys Asp Ala Ser Asp
       50                  55                  60
   Val Phe His Ala Met His Pro Glu Ser Ala Tyr Glu Leu Leu Ala Asn
   65                  70                  75                  80
   Cys Tyr Val Gly Asp Leu Ala Ala Asp His Ala Gly Val Gln Gly Glu
                   85                  90                  95
   Leu Val Asn Gly Val His Lys Lys Ser Lys Ala Phe Ala Asp Glu Met
               100                 105                 110
   Arg Ser Leu Arg Glu Arg Leu Glu Thr Glu Gly Ala Phe Asn Gly Ser
           115                 120                 125
   Val Pro Phe Tyr Ile Tyr Lys Val Val Ser Thr Leu Ala Ile Gly Ala
       130                 135                 140
   Thr Gly Leu Ala Met Leu Tyr Tyr Gly Gly His Ser Thr Ser Val Val
   145                 150                 155                 160
   Leu Ala Ala Ala Val Val Val Gly Leu Phe Trp Gln Gln Cys Gly Trp
                   165                 170                 175
   Leu Ala His Asp Phe Gly His His Gln Ala Phe Ala Asp His Thr Val
               180                 185                 190
   Asn Asp Val Met Ile Ala Phe Leu Gly Gly Phe Cys Gln Gly Phe Ser
           195                 200                 205
   Leu Ser Trp Trp Lys Asn Lys His Asn Thr His His Ala Ser Thr Asn
       210                 215                 220
   Val His Gly His Asp Pro Asp Ile Asp Thr Ala Pro Val Leu Leu Trp
   225                 230                 235                 240
   Asp Glu Phe Ala Thr Ala Asn Phe Tyr Gly Asn Leu Glu Gly Gln Lys
                   245                 250                 255
   Asp Ser Ala Phe Ser Arg Phe Ile Ala Glu His Val Leu Pro Tyr Gln
               260                 265                 270
   Thr Arg Tyr Tyr Phe Phe Val Leu Gly Phe Ala Arg Leu Ser Trp Ala
           275                 280                 285
   Ile Gln Ser Leu Gln Tyr Ser Phe Thr Val Gly Thr Leu Asn Lys Ser
       290                 295                 300
   Lys Thr Leu Asn Leu Phe Glu Arg Thr Met Leu Val Ser His Trp Ile
   305                 310                 315                 320
   Leu Phe Thr Thr Trp Thr Leu Leu Phe Ile Asn Ser Trp Thr Asn Met
                   325                 330                 335
   Val Met Phe Phe Val Val Ser Gln Ala Thr Thr Gly Tyr Ala Leu Ala
               340                 345                 350
   Leu Val Phe Ala Met Asn His Ser Gly Met Pro Val Leu Thr Gln Glu
           355                 360                 365
   Gln Ala Gln Lys Met Glu Phe Tyr Glu Ile Gln Val Val Thr Ala Arg
       370                 375                 380
   Asp Val Thr Leu Gly Ala Leu Gly Asp Trp Phe Cys Gly Gly Leu Asn
   385                 390                 395                 400
   Tyr Gln Ile Glu His His Val Phe Pro Asp Met Pro Arg His Tyr Leu
                   405                 410                 415
   Pro Lys Val Lys Pro Gln Val Lys Ala Leu Cys Lys Lys His Asn Ile
               420                 425                 430
   Leu Tyr His Asp Thr Ser Ala Leu Arg Gly Thr Leu Glu Val Leu Gln
           435                 440                 445
   Thr Leu Asp Val Val Gln Lys Leu Cys Ala Ser Ser Leu Lys Arg Cys
       450                 455                 460
   Phe Arg
   465
<210> 9
   <211> 1398
   <212> DNA
   <213> Glossomastix chrysoplasta
<220>
   <221> CDS
   <222> (1)..(1398)
<400> 9
   atg ggt aaa gga ggc cgg gac gcc ggc gct gtc ggc ggc gaa gcg gag     48
   Met Gly Lys Gly Gly Arg Asp Ala Gly Ala Val Gly Gly Glu Ala Glu
   1               5                   10                  15
   aag acg ctg ccc aag ttt acg ttg gag gag atc cag aag cat cgc acg     96
   Lys Thr Leu Pro Lys Phe Thr Leu Glu Glu Ile Gln Lys His Arg Thr
               20                  25                  30
   ccg cag gat gcg tgg atg gtg cac cac aac aag gtg tac gat gtg tcc    144
   Pro Gln Asp Ala Trp Met Val His His Asn Lys Val Tyr Asp Val Ser
           35                  40                  45
   aac tat atg gac cac ccc ggc ggt ctg gtg atc ttt agc cac gcg ggc    192
   Asn Tyr Met Asp His Pro Gly Gly Leu Val Ile Phe Ser His Ala Gly
       50                  55                  60
   gat gac atg acc gac gtc ttc gcg gcg ttc cac ccg ccc tcg gcg ttc    240
   Asp Asp Met Thr Asp Val Phe Ala Ala Phe His Pro Pro Ser Ala Phe
   65                  70                  75                  80
   aac ttc atg gac aag ttc ttg att ggc gtg gtg gac agc aag ggc tcg    288
   Asn Phe Met Asp Lys Phe Leu Ile Gly Val Val Asp Ser Lys Gly Ser
                   85                  90                  95
   tcg ccg cag ctc cag aag gac gcg tcc cag gcc agc ttc gag aag gca    336
   Ser Pro Gln Leu Gln Lys Asp Ala Ser Gln Ala Ser Phe Glu Lys Ala
               100                 105                 110
   tac cgc aat ctg cga gtc cag ctc aag aag gcc ggc atg ttc aag gcc    384
   Tyr Arg Asn Leu Arg Val Gln Leu Lys Lys Ala Gly Met Phe Lys Ala
           115                 120                 125
   tcg agc ctc ttc tac acc tac aag gtg ctc tcg acc ctg gcg ctg tgc    432
   Ser Ser Leu Phe Tyr Thr Tyr Lys Val Leu Ser Thr Leu Ala Leu Cys
       130                 135                 140
   ctc gtg tcc tgg ggc ctc gtg ctg ggg tcg gac cac ttc ggt gtg cac    480
   Leu Val Ser Trp Gly Leu Val Leu Gly Ser Asp His Phe Gly Val His
   145                 150                 155                 160
   ctc gtg ggc gct ctc ttc ctc gcg ctc ttc tgg cag cag tgc ggc tgg    528
   Leu Val Gly Ala Leu Phe Leu Ala Leu Phe Trp Gln Gln Cys Gly Trp
                   165                 170                 175
   ttg gcc cac gac ttc ctg cac cac cag gtc ttc cag aac cgg gct cac    576
   Leu Ala His Asp Phe Leu His His Gln Val Phe Gln Asn Arg Ala His
               180                 185                 190
   ggc gac ctc gcc ggt atc atg atc ggc aac gtg tgg cag ggc ttc tcc    624
   Gly Asp Leu Ala Gly Ile Met Ile Gly Asn Val Trp Gln Gly Phe Ser
           195                 200                 205
   gtg gcc tgg tgg aag aac aag cac aac acc cac cac tcg gtg ccg aac    672
   Val Ala Trp Trp Lys Asn Lys His Asn Thr His His Ser Val Pro Asn
       210                 215                 220
   ctc tac gag tcg cag ccc gac gcc gcc gac ggc gat ccg gac atc gac    720
   Leu Tyr Glu Ser Gln Pro Asp Ala Ala Asp Gly Asp Pro Asp Ile Asp
   225                 230                 235                 240
   acc atg ccc ctc ctc gcc tgg tcg ctg cgc atg gcc aag aac gcg gac    768
   Thr Met Pro Leu Leu Ala Trp Ser Leu Arg Met Ala Lys Asn Ala Asp
                   245                 250                 255
   aac gcg cta tcc cgg tgg ttc gtg tcc cac cag gcc ttc tgc tac ttc    816
   Asn Ala Leu Ser Arg Trp Phe Val Ser His Gln Ala Phe Cys Tyr Phe
               260                 265                 270
   ccc atc ctt ggc ctc gcg cgg ctc tcg tgg ctc gag ggg tcc ttc tcc    864
   Pro Ile Leu Gly Leu Ala Arg Leu Ser Trp Leu Glu Gly Ser Phe Ser
           275                 280                 285
   ttc gtg ttc tcc aac ccg ttg gca tgg aag acg aag aac ctt gat gta    912
   Phe Val Phe Ser Asn Pro Leu Ala Trp Lys Thr Lys Asn Leu Asp Val
       290                 295                 300
   gcc aag cag ctc gtg acc aac ccc ctg ttg gag cag gcc ggc ctc ctg    960
   Ala Lys Gln Leu Val Thr Asn Pro Leu Leu Glu Gln Ala Gly Leu Leu
   305                 310                 315                 320
   gtc cac tac gcc tgg gtc ttc gcc ctc tgc gcg tgc acg ggc tcc ctg   1008
   Val His Tyr Ala Trp Val Phe Ala Leu Cys Ala Cys Thr Gly Ser Leu
                       325                     330                     335
   gtg cgc gcc ctg gcg ttc ttc ttc gtg gcc acc tgc acc agc ggc ctt      1056
   Val Arg Ala Leu Ala Phe Phe Phe Val Ala Thr Cys Thr Ser Gly Leu
               340                     345                     350
   ctc ctc gcc atc gtc ttc ggc ctc ggc cac aac ggc atg gcg ctc tac      1104
   Leu Leu Ala Ile Val Phe Gly Leu Gly His Asn Gly Met Ala Leu Tyr
           355                     360                     365
   gag gcc aat gcc cgg ccg gac ttc tgg aag ctc cag gtg acc acc acg      1152
   Glu Ala Asn Ala Arg Pro Asp Phe Trp Lys Leu Gln Val Thr Thr Thr
       370                     375                     380
   cgg aac atc acc ggc agc ccc ttt gtg cac tgg ttc tgc ggc ggc ctc      1200
   Arg Asn Ile Thr Gly Ser Pro Phe Val His Trp Phe Cys Gly Gly Leu
   385                 390                     395                     400
   cag ttc cag gtg gag cac cac ctc ttc ccc tcg ctg ccc cgg cac aac      1248
   Gln Phe Gln Val Glu His His Leu Phe Pro Ser Leu Pro Arg His Asn
                   405                     410                     415
   ctc ccc cgg gcc cac gag atc gtg acg gcc ttc tgc aag gag cag ggc      1296
   Leu Pro Arg Ala His Glu Ile Val Thr Ala Phe Cys Lys Glu Gln Gly
               420                     425                     430
   gtc aag tac cac gag gcc gac ctc ctt acc ggg acc aag gag att ctt      1344
   Val Lys Tyr His Glu Ala Asp Leu Leu Thr Gly Thr Lys Glu Ile Leu
           435                     440                     445
   tcg tgc ttg tcg gag gta acg acg gag ttc ctc gac gag ttc cca gcc      1392
   Ser Cys Leu Ser Glu Val Thr Thr Glu Phe Leu Asp Glu Phe Pro Ala
       450                     455                     460
   atg taa                                                              1398
   Met
   465
<210> 10
   <211> 465
   <212> PRT
   <213> Glossomastix chrysoplasta
<400> 10
   Met Gly Lys Gly Gly Arg Asp Ala Gly Ala Val Gly Gly Glu Ala Glu
   1 5 10 15
   Lys Thr Leu Pro Lys Phe Thr Leu Glu Glu Ile Gln Lys His Arg Thr
   20 25 30
   Pro Gln Asp Ala Trp Met Val His His Asn Lys Val Tyr Asp Val Ser
   35 40 45
   Asn Tyr Met Asp His Pro Gly Gly Leu Val Ile Phe Ser His Ala Gly
   50 55 60
   Asp Asp Met Thr Asp Val Phe Ala Ala Phe His Pro Pro Ser Ala Phe
   65 70 75 80
   Asn Phe Met Asp Lys Phe Leu Ile Gly Val Val Asp Ser Lys Gly Ser
   85 90 95
   Ser Pro Gln Leu Gln Lys Asp Ala Ser Gln Ala Ser Phe Glu Lys Ala
   100 105 110
   Tyr Arg Asn Leu Arg Val Gln Leu Lys Lys Ala Gly Met Phe Lys Ala
   115 120 125
   Ser Ser Leu Phe Tyr Thr Tyr Lys Val Leu Ser Thr Leu Ala Leu Cys
   130 135 140
   Leu Val Ser Trp Gly Leu Val Leu Gly Ser Asp His Phe Gly Val His
   145 150 155 160
   Leu Val Gly Ala Leu Phe Leu Ala Leu Phe Trp Gln Gln Cys Gly Trp
   165 170 175
   Leu Ala His Asp Phe Leu His His Gln Val Phe Gln Asn Arg Ala His
   180 185 190
   Gly Asp Leu Ala Gly Ile Met Ile Gly Asn Val Trp Gln Gly Phe Ser
   195 200 205
   Val Ala Trp Trp Lys Asn Lys His Asn Thr His His Ser Val Pro Asn
   210 215 220
   Leu Tyr Glu Ser Gln Pro Asp Ala Ala Asp Gly Asp Pro Asp Ile Asp
   225 230 235 240
   Thr Met Pro Leu Leu Ala Trp Ser Leu Arg Met Ala Lys Asn Ala Asp
   245 250 255
   Asn Ala Leu Ser Arg Trp Phe Val Ser His Gln Ala Phe Cys Tyr Phe
   260 265 270
   Pro Ile Leu Gly Leu Ala Arg Leu Ser Trp Leu Glu Gly Ser Phe Ser
   275 280 285
   Phe Val Phe Ser Asn Pro Leu Ala Trp Lys Thr Lys Asn Leu Asp Val
   290 295 300
   Ala Lys Gln Leu Val Thr Asn Pro Leu Leu Glu Gln Ala Gly Leu Leu
   305 310 315 320
   Val His Tyr Ala Trp Val Phe Ala Leu Cys Ala Cys Thr Gly Ser Leu
   325 330 335
   Val Arg Ala Leu Ala Phe Phe Phe Val Ala Thr Cys Thr Ser Gly Leu
   340 345 350
   Leu Leu Ala Ile Val Phe Gly Leu Gly His Asn Gly Met Ala Leu Tyr
   355 360 365
   Glu Ala Asn Ala Arg Pro Asp Phe Trp Lys Leu Gln Val Thr Thr Thr
   370 375 380
   Arg Asn Ile Thr Gly Ser Pro Phe Val His Trp Phe Cys Gly Gly Leu
   385 390 395 400
   Gln Phe Gln Val Glu His His Leu Phe Pro Ser Leu Pro Arg His Asn
   405 410 415
   Leu Pro Arg Ala His Glu Ile Val Thr Ala Phe Cys Lys Glu Gln Gly
   420 425 430
   Val Lys Tyr His Glu Ala Asp Leu Leu Thr Gly Thr Lys Glu Ile Leu
   435 440 445
   Ser Cys Leu Ser Glu Val Thr Thr Glu Phe Leu Asp Glu Phe Pro Ala
   450 455 460
   Met
   465
<210> 11
   <211> 1455
   <212> DNA
   <213> Thalassiosira pseudonana
<220>
   <221> CDS
   <222> (1)..(1455)
<400> 11
   atg gga aaa gga gga gac gca gcc gca gct acc aag cgt agt gga gca        48
   Met Gly Lys Gly Gly Asp Ala Ala Ala Ala Thr Lys Arg Ser Gly Ala
   1               5                   10                  15
   ttg aaa ttg gcg gag aag ccg cag aag tac act tgg cag gag gtg aag        96
   Leu Lys Leu Ala Glu Lys Pro Gln Lys Tyr Thr Trp Gln Glu Val Lys
               20                  25                  30
   aag cac atc acc ccc gac gat gcc tgg gta gtc cac caa aac aaa gtc        144
   Lys His Ile Thr Pro Asp Asp Ala Trp Val Val His Gln Asn Lys Val
           35                  40                  45
   tac gac gtc tcc aac tgg tac gac cac ccc ggt gga gcc gtg gtg ttc        192
   Tyr Asp Val Ser Asn Trp Tyr Asp His Pro Gly Gly Ala Val Val Phe
       50                  55                  60
   acc cac gcc gga gac gac atg acg gac atc ttc gcc gcc ttc cac gcc        240
   Thr His Ala Gly Asp Asp Met Thr Asp Ile Phe Ala Ala Phe His Ala
   65                  70                  75                  80
   caa ggc tct cag gcc atg atg aag aag ttt tac att gga gat ttg att        288
   Gln Gly Ser Gln Ala Met Met Lys Lys Phe Tyr Ile Gly Asp Leu Ile
                   85                  90                  95
   ccg gag agt gtg gag cat aag gat caa aga cag ttg gat ttc gag aag        336
   Pro Glu Ser Val Glu His Lys Asp Gln Arg Gln Leu Asp Phe Glu Lys
               100                 105                 110
   gga tat cgt gat tta cgg gcc aag ctt gtc atg atg ggg atg ttc aag        384
   Gly Tyr Arg Asp Leu Arg Ala Lys Leu Val Met Met Gly Met Phe Lys
           115                 120                 125
   tcg agt aag atg tat tat gca tac aag tgc tcg ttc aat atg tgc atg        432
   Ser Ser Lys Met Tyr Tyr Ala Tyr Lys Cys Ser Phe Asn Met Cys Met
       130                 135                 140
   tgg ttg gtg gcg gtg gcc atg gtg tac tac tcg gac agt ttg gca atg        480
   Trp Leu Val Ala Val Ala Met Val Tyr Tyr Ser Asp Ser Leu Ala Met
   145                 150                 155                 160
   cac att gga tcg gct ctc ttg ttg gga ttg ttc tgg cag cag tgt gga      528
   His Ile Gly Ser Ala Leu Leu Leu Gly Leu Phe Trp Gln Gln Cys Gly
                   165                 170                 175
   tgg ctt gcg cac gac ttt ctt cac cac caa gtc ttt aag caa cga aag      576
   Trp Leu Ala His Asp Phe Leu His His Gln Val Phe Lys Gln Arg Lys
               180                 185                 190
   tac gga gat ctc gtt ggc atc ttt tgg gga gat ctc atg cag ggg ttc      624
   Tyr Gly Asp Leu Val Gly Ile Phe Trp Gly Asp Leu Met Gln Gly Phe
           195                 200                 205
   tcg atg cag tgg tgg aag aac aag cac aat ggc cac cat gct gtt ccc      672
   Ser Met Gln Trp Trp Lys Asn Lys His Asn Gly His His Ala Val Pro
       210                 215                 220
   aac ttg cac aac tct tcc ttg gac agt cag gat ggt gat ccc gat att      720
   Asn Leu His Asn Ser Ser Leu Asp Ser Gln Asp Gly Asp Pro Asp Ile
   225                 230                 235                 240
   gat acc atg cca ctc ctt gct tgg agt ctc aag cag gct cag agt ttc      768
   Asp Thr Met Pro Leu Leu Ala Trp Ser Leu Lys Gln Ala Gln Ser Phe
                   245                 250                 255
   aga gag atc aat aag gga aag gac agt acc ttc gtc aag tac gct atc      816
   Arg Glu Ile Asn Lys Gly Lys Asp Ser Thr Phe Val Lys Tyr Ala Ile
               260                 265                 270
   aaa ttc cag gca ttc aca tac ttc ccc atc ctc ctc ttg gct cgc atc      864
   Lys Phe Gln Ala Phe Thr Tyr Phe Pro Ile Leu Leu Leu Ala Arg Ile
           275                 280                 285
   tct tgg ttg aat gaa tcc ttc aaa act gca ttc gga ctc gga gct gcc      912
   Ser Trp Leu Asn Glu Ser Phe Lys Thr Ala Phe Gly Leu Gly Ala Ala
       290                 295                 300
   tcg gag aat gcc aag ttg gag ttg gag aag cgt gga ctt cag tac cca      960
   Ser Glu Asn Ala Lys Leu Glu Leu Glu Lys Arg Gly Leu Gln Tyr Pro
   305                 310                 315                 320
   ctt ttg gag aag ctt gga atc acc ctt cac tac act tgg atg ttc gtc     1008
   Leu Leu Glu Lys Leu Gly Ile Thr Leu His Tyr Thr Trp Met Phe Val
                   325                 330                 335
   ctc tct tcc gga ttt gga agg tgg tct ctt cca tat tcc atc atg tat     1056
   Leu Ser Ser Gly Phe Gly Arg Trp Ser Leu Pro Tyr Ser Ile Met Tyr
               340                 345                 350
   ttc ttc act gcc aca tgc tcc tcg gga ctt ttc ctc gca ttg gtc ttt     1104
   Phe Phe Thr Ala Thr Cys Ser Ser Gly Leu Phe Leu Ala Leu Val Phe
           355                 360                 365
   gga ttg gga cac aac ggt atg tca gtg tac gat gcc acc acc cga cct     1152
   Gly Leu Gly His Asn Gly Met Ser Val Tyr Asp Ala Thr Thr Arg Pro
       370                 375                 380
   gac ttc tgg caa ctc caa gtc acc act aca cgt aac atc att ggt gga     1200
   Asp Phe Trp Gln Leu Gln Val Thr Thr Thr Arg Asn Ile Ile Gly Gly
   385                 390                 395                 400
   cac ggc att ccc caa ttc ttt gtg gat tgg ttc tgc ggt gga ttg caa     1248
   His Gly Ile Pro Gln Phe Phe Val Asp Trp Phe Cys Gly Gly Leu Gln
                   405                 410                 415
   tac caa gtg gat cac cac ctc ttc ccc atg atg cct aga aac aat atc     1296
   Tyr Gln Val Asp His His Leu Phe Pro Met Met Pro Arg Asn Asn Ile
               420                 425                 430
   gcg aag tgc cac aag ctt gtg gag tca ttc tgt aag gag tgg ggt gtg     1344
   Ala Lys Cys His Lys Leu Val Glu Ser Phe Cys Lys Glu Trp Gly Val
           435                 440                 445
   aag tac cat gag gct gat atg tgg gat ggt acc gtg gaa gtg ttg caa     1392
   Lys Tyr His Glu Ala Asp Met Trp Asp Gly Thr Val Glu Val Leu Gln
       450                 455                 460
   cat ctc tcc aag gtg tcg gat gat ttc ctt gtg gag atg gtg aag gat     1440
   His Leu Ser Lys Val Ser Asp Asp Phe Leu Val Glu Met Val Lys Asp
   465                 470                 475                 480
   ttc cct gcc atg taa                                                 1455
   Phe Pro Ala Met
<210> 12
   <211> 484
   <212> PRT
   <213> Thalassiosira pseudonana
<400> 12
   Met Gly Lys Gly Gly Asp Ala Ala Ala Ala Thr Lys Arg Ser Gly Ala
   1               5                   10                  15
   Leu Lys Leu Ala Glu Lys Pro Gln Lys Tyr Thr Trp Gln Glu Val Lys
               20                  25                  30
   Lys His Ile Thr Pro Asp Asp Ala Trp Val Val His Gln Asn Lys Val
           35                  40                  45
   Tyr Asp Val Ser Asn Trp Tyr Asp His Pro Gly Gly Ala Val Val Phe
       50                  55                  60
   Thr His Ala Gly Asp Asp Met Thr Asp Ile Phe Ala Ala Phe His Ala
   65                  70                  75                  80
   Gln Gly Ser Gln Ala Met Met Lys Lys Phe Tyr Ile Gly Asp Leu Ile
                   85                  90                  95
   Pro Glu Ser Val Glu His Lys Asp Gln Arg Gln Leu Asp Phe Glu Lys
               100                 105                 110
   Gly Tyr Arg Asp Leu Arg Ala Lys Leu Val Met Met Gly Met Phe Lys
           115                 120                 125
   Ser Ser Lys Met Tyr Tyr Ala Tyr Lys Cys Ser Phe Asn Met Cys Met
       130                 135                 140
   Trp Leu Val Ala Val Ala Met Val Tyr Tyr Ser Asp Ser Leu Ala Met
   145                 150                 155                 160
   His Ile Gly Ser Ala Leu Leu Leu Gly Leu Phe Trp Gln Gln Cys Gly
                   165                 170                 175
   Trp Leu Ala His Asp Phe Leu His His Gln Val Phe Lys Gln Arg Lys
               180                 185                 190
   Tyr Gly Asp Leu Val Gly Ile Phe Trp Gly Asp Leu Met Gln Gly Phe
           195                 200                 205
   Ser Met Gln Trp Trp Lys Asn Lys His Asn Gly His His Ala Val Pro
       210                 215                 220
   Asn Leu His Asn Ser Ser Leu Asp Ser Gln Asp Gly Asp Pro Asp Ile
   225                 230                 235                 240
   Asp Thr Met Pro Leu Leu Ala Trp Ser Leu Lys Gln Ala Gln Ser Phe
                   245                 250                 255
   Arg Glu Ile Asn Lys Gly Lys Asp Ser Thr Phe Val Lys Tyr Ala Ile
               260                 265                 270
   Lys Phe Gln Ala Phe Thr Tyr Phe Pro Ile Leu Leu Leu Ala Arg Ile
           275                 280                 285
   Ser Trp Leu Asn Glu Ser Phe Lys Thr Ala Phe Gly Leu Gly Ala Ala
       290                 295                 300
   Ser Glu Asn Ala Lys Leu Glu Leu Glu Lys Arg Gly Leu Gln Tyr Pro
   305                 310                 315                 320
   Leu Leu Glu Lys Leu Gly Ile Thr Leu His Tyr Thr Trp Met Phe Val
                   325                 330                 335
   Leu Ser Ser Gly Phe Gly Arg Trp Ser Leu Pro Tyr Ser Ile Met Tyr
               340                 345                 350
   Phe Phe Thr Ala Thr Cys Ser Ser Gly Leu Phe Leu Ala Leu Val Phe
           355                 360                 365
   Gly Leu Gly His Asn Gly Met Ser Val Tyr Asp Ala Thr Thr Arg Pro
       370                 375                 380
   Asp Phe Trp Gln Leu Gln Val Thr Thr Thr Arg Asn Ile Ile Gly Gly
   385                 390                 395                 400
   His Gly Ile Pro Gln Phe Phe Val Asp Trp Phe Cys Gly Gly Leu Gln
                   405                 410                 415
   Tyr Gln Val Asp His His Leu Phe Pro Met Met Pro Arg Asn Asn Ile
               420                 425                 430
   Ala Lys Cys His Lys Leu Val Glu Ser Phe Cys Lys Glu Trp Gly Val
           435                 440                 445
   Lys Tyr His Glu Ala Asp Met Trp Asp Gly Thr Val Glu Val Leu Gln
       450                 455                 460
   His Leu Ser Lys Val Ser Asp Asp Phe Leu Val Glu Met Val Lys Asp
   465                 470                 475                 480
   Phe Pro Ala Met
<210> 13
   <211> 1446
   <212> DNA
   <213> Marchantia polymorpha
<220>
   <221> CDS
   <222> (1)..(1446)
<400> 13
   atg gcc tcg tcc acc acc acc gcc gtg aag caa tct tcg ggt ggg ctg       48
   Met Ala Ser Ser Thr Thr Thr Ala Val Lys Gln Ser Ser Gly Gly Leu
   1               5                   10                  15
   tgg tcg aaa tgg ggc acc ggc agc aac ttg agc ttc gtg tcg cgc aag       96
   Trp Ser Lys Trp Gly Thr Gly Ser Asn Leu Ser Phe Val Ser Arg Lys
               20                  25                  30
   gag cag cag cag cag cag cag cag agc tct ccc gag gcg tcg act ccc      144
   Glu Gln Gln Gln Gln Gln Gln Gln Ser Ser Pro Glu Ala Ser Thr Pro
           35                  40                  45
   gcg gcg cag cag gag aaa tcc atc agt aga gaa tcc atc ccc gag ggc      192
   Ala Ala Gln Gln Glu Lys Ser Ile Ser Arg Glu Ser Ile Pro Glu Gly
       50                  55                  60
   ttc ttg acc gtg gag gag gtg tcg aag cac gac aat ccg agc gac tgc      240
   Phe Leu Thr Val Glu Glu Val Ser Lys His Asp Asn Pro Ser Asp Cys
   65                  70                  75                  80
   tgg atc gtc atc aac gac aag gtg tac gac gtg agc gca ttc ggg aag      288
   Trp Ile Val Ile Asn Asp Lys Val Tyr Asp Val Ser Ala Phe Gly Lys
                   85                  90                  95
   acg cat ccg ggc ggc cct gtg atc ttc acg cag gcc ggc cgc gac gcc      336
   Thr His Pro Gly Gly Pro Val Ile Phe Thr Gln Ala Gly Arg Asp Ala
               100                 105                 110
   acg gat tct ttc aag gtt ttc cac tcc gcc aag gcg tgg cag ttt ctc      384
   Thr Asp Ser Phe Lys Val Phe His Ser Ala Lys Ala Trp Gln Phe Leu
           115                 120                 125
   cag gac ctg tac atc gga gat ctg tac aat gcc gag cca gtg tcg gag      432
   Gln Asp Leu Tyr Ile Gly Asp Leu Tyr Asn Ala Glu Pro Val Ser Glu
       130                 135                 140
   ctg gtg aag gat tac cga gac ctg agg acg gcg ttc atg cgt tct cag      480
   Leu Val Lys Asp Tyr Arg Asp Leu Arg Thr Ala Phe Met Arg Ser Gln
   145                 150                 155                 160
   cta ttc aag agc agt aaa atg tac tac gtg acc aag tgc gtc aca aat      528
   Leu Phe Lys Ser Ser Lys Met Tyr Tyr Val Thr Lys Cys Val Thr Asn
                   165                 170                 175
   ttt gca att ctt gcc gcc agt ctc gca gtc atc gcg tgg agc cag acg      576
   Phe Ala Ile Leu Ala Ala Ser Leu Ala Val Ile Ala Trp Ser Gln Thr
               180                 185                 190
   tat ctg gcg gtt ttg tgc tcc agt ttc ctg ttg gct ctc ttc tgg cag      624
   Tyr Leu Ala Val Leu Cys Ser Ser Phe Leu Leu Ala Leu Phe Trp Gln
           195                 200                 205
   caa tgt gga tgg tta tcg cac gat ttt ctc cac cac cag gtg acc gag      672
   Gln Cys Gly Trp Leu Ser His Asp Phe Leu His His Gln Val Thr Glu
       210                 215                 220
   aac cga tcg ctc aac acg tac ttc ggc ggc ctg ttc tgg ggt aac ttc      720
   Asn Arg Ser Leu Asn Thr Tyr Phe Gly Gly Leu Phe Trp Gly Asn Phe
   225                 230                 235                 240
   gcc cag ggc tac agc gtg gga tgg tgg aag acc aag cac aat gtg cac      768
   Ala Gln Gly Tyr Ser Val Gly Trp Trp Lys Thr Lys His Asn Val His
                   245                 250                 255
   cac gcg gcc acg aac gaa tgc gac gac aag tat cag ccc atc gat ccc      816
   His Ala Ala Thr Asn Glu Cys Asp Asp Lys Tyr Gln Pro Ile Asp Pro
               260                 265                 270
   gac atc gac acc gtg ccc ctg ctc gcc tgg agc aag gaa atc ttg gcc      864
   Asp Ile Asp Thr Val Pro Leu Leu Ala Trp Ser Lys Glu Ile Leu Ala
           275                 280                 285
   acc gtc gac gac caa ttc ttc cga tcg atc atc agc gtg cag cac ctt      912
   Thr Val Asp Asp Gln Phe Phe Arg Ser Ile Ile Ser Val Gln His Leu
       290                 295                 300
   ctg ttc ttc ccg ctc ctc ttc ttg gca aga ttc agc tgg ctg cat tcg      960
   Leu Phe Phe Pro Leu Leu Phe Leu Ala Arg Phe Ser Trp Leu His Ser
   305                 310                 315                 320
   agt tgg gcc cac gcc agc aac ttc gag atg cct cgg tac atg aga tgg     1008
   Ser Trp Ala His Ala Ser Asn Phe Glu Met Pro Arg Tyr Met Arg Trp
                   325                 330                 335
   gcg gag aag gcc tcg ctc ctc ggg cac tac ggc gcc tca atc ggc gcc      1056
   Ala Glu Lys Ala Ser Leu Leu Gly His Tyr Gly Ala Ser Ile Gly Ala
               340                 345                 350
   gcc ttc tac att ttg ccc atc ccc cag gcc atc tgc tgg ctc ttc ttg      1104
   Ala Phe Tyr Ile Leu Pro Ile Pro Gln Ala Ile Cys Trp Leu Phe Leu
           355                 360                 365
   tcg caa ctg ttt tgc ggc gct ctg ctc agc att gtc ttc gtg atc agc      1152
   Ser Gln Leu Phe Cys Gly Ala Leu Leu Ser Ile Val Phe Val Ile Ser
       370                 375                 380
   cac aat ggc atg gat gtg tac aac gac ccc cgg gac ttc gtg acg gcc      1200
   His Asn Gly Met Asp Val Tyr Asn Asp Pro Arg Asp Phe Val Thr Ala
   385                 390                 395                 400
   caa gtc acc tcg acc aga aac atc gaa ggc aac ttc ttc aac gac tgg      1248
   Gln Val Thr Ser Thr Arg Asn Ile Glu Gly Asn Phe Phe Asn Asp Trp
                   405                 410                 415
   ttc acc gga ggc ctg aac agg cag att gag cac cat ctg ttt ccg tct      1296
   Phe Thr Gly Gly Leu Asn Arg Gln Ile Glu His His Leu Phe Pro Ser
               420                 425                 430
   ctt ccg agg cac aac ctc gcc aag gtc gcg cca cac gtc aag gcg ctc      1344
   Leu Pro Arg His Asn Leu Ala Lys Val Ala Pro His Val Lys Ala Leu
           435                 440                 445
   tgc gcc aag cac ggt ttg cat tac gaa gaa ttg agt ctg ggc acg gga      1392
   Cys Ala Lys His Gly Leu His Tyr Glu Glu Leu Ser Leu Gly Thr Gly
       450                 455                 460
   gtc tgt cgt gtc ttc aat cgg cta gta gag gta gca tac gct gcg aaa      1440
   Val Cys Arg Val Phe Asn Arg Leu Val Glu Val Ala Tyr Ala Ala Lys
   465                 470                 475                 480
   gta tag                                                              1446
   Val

<210> 14
   <211> 481
   <212> PRT
   <213> Marchantia polymorpha
<400> 14
   Met Ala Ser Ser Thr Thr Thr Ala Val Lys Gln Ser Ser Gly Gly Leu
   1               5                   10                  15
   Trp Ser Lys Trp Gly Thr Gly Ser Asn Leu Ser Phe Val Ser Arg Lys
               20                  25                  30
   Glu Gln Gln Gln Gln Gln Gln Gln Ser Ser Pro Glu Ala Ser Thr Pro
           35                  40                  45
   Ala Ala Gln Gln Glu Lys Ser Ile Ser Arg Glu Ser Ile Pro Glu Gly
       50                  55                  60
   Phe Leu Thr Val Glu Glu Val Ser Lys His Asp Asn Pro Ser Asp Cys
   65                  70                  75                  80
   Trp Ile Val Ile Asn Asp Lys Val Tyr Asp Val Ser Ala Phe Gly Lys
                   85                  90                  95
   Thr His Pro Gly Gly Pro Val Ile Phe Thr Gln Ala Gly Arg Asp Ala
               100                 105                 110
   Thr Asp Ser Phe Lys Val Phe His Ser Ala Lys Ala Trp Gln Phe Leu
           115                 120                 125
   Gln Asp Leu Tyr Ile Gly Asp Leu Tyr Asn Ala Glu Pro Val Ser Glu
       130                 135                 140
   Leu Val Lys Asp Tyr Arg Asp Leu Arg Thr Ala Phe Met Arg Ser Gln
   145                 150                 155                 160
   Leu Phe Lys Ser Ser Lys Met Tyr Tyr Val Thr Lys Cys Val Thr Asn
                   165                 170                 175
   Phe Ala Ile Leu Ala Ala Ser Leu Ala Val Ile Ala Trp Ser Gln Thr
               180                 185                 190
   Tyr Leu Ala Val Leu Cys Ser Ser Phe Leu Leu Ala Leu Phe Trp Gln
           195                 200                 205
   Gln Cys Gly Trp Leu Ser His Asp Phe Leu His His Gln Val Thr Glu
       210                 215                 220
   Asn Arg Ser Leu Asn Thr Tyr Phe Gly Gly Leu Phe Trp Gly Asn Phe
   225                 230                 235                 240
   Ala Gln Gly Tyr Ser Val Gly Trp Trp Lys Thr Lys His Asn Val His
                   245                 250                 255
   His Ala Ala Thr Asn Glu Cys Asp Asp Lys Tyr Gln Pro Ile Asp Pro
               260                 265                 270
   Asp Ile Asp Thr Val Pro Leu Leu Ala Trp Ser Lys Glu Ile Leu Ala
           275                 280                 285
   Thr Val Asp Asp Gln Phe Phe Arg Ser Ile Ile Ser Val Gln His Leu
       290                 295                 300
   Leu Phe Phe Pro Leu Leu Phe Leu Ala Arg Phe Ser Trp Leu His Ser
   305                 310                 315                 320
   Ser Trp Ala His Ala Ser Asn Phe Glu Met Pro Arg Tyr Met Arg Trp
                   325                 330                 335
   Ala Glu Lys Ala Ser Leu Leu Gly His Tyr Gly Ala Ser Ile Gly Ala
               340                 345                 350
   Ala Phe Tyr Ile Leu Pro Ile Pro Gln Ala Ile Cys Trp Leu Phe Leu
           355                 360                 365
   Ser Gln Leu Phe Cys Gly Ala Leu Leu Ser Ile Val Phe Val Ile Ser
       370                 375                 380
   His Asn Gly Met Asp Val Tyr Asn Asp Pro Arg Asp Phe Val Thr Ala
   385                 390                 395                 400
   Gln Val Thr Ser Thr Arg Asn Ile Glu Gly Asn Phe Phe Asn Asp Trp
                   405                 410                 415
   Phe Thr Gly Gly Leu Asn Arg Gln Ile Glu His His Leu Phe Pro Ser
               420                 425                 430
   Leu Pro Arg His Asn Leu Ala Lys Val Ala Pro His Val Lys Ala Leu
           435                 440                 445
   Cys Ala Lys His Gly Leu His Tyr Glu Glu Leu Ser Leu Gly Thr Gly
       450                 455                 460
   Val Cys Arg Val Phe Asn Arg Leu Val Glu Val Ala Tyr Ala Ala Lys
   465                 470                 475                 480
   Val
<210> 15
   <211> 1377
   <212> DNA
   <213> Rhizopus oryzae
<220>
   <221> CDS
   <222> (1)..(1377)
<400> 15
   atg agt aca tca gat cgt caa tca gtt ttc aca tta aaa gag ttg gaa       48
   Met Ser Thr Ser Asp Arg Gln Ser Val Phe Thr Leu Lys Glu Leu Glu
   1               5                   10                  15
   ctg att aat caa aaa cat cga gat gga gat aaa agt gcg atg aag ttc       96
   Leu Ile Asn Gln Lys His Arg Asp Gly Asp Lys Ser Ala Met Lys Phe
               20                  25                  30
   att atc att gat cgt aag gtg tac gat gtg act gaa ttc ttg gaa gat      144
   Ile Ile Ile Asp Arg Lys Val Tyr Asp Val Thr Glu Phe Leu Glu Asp
           35                  40                  45
   cat cct ggt ggt gca caa gta tta ctg aca cac gtt gga aaa gat gca      192
   His Pro Gly Gly Ala Gln Val Leu Leu Thr His Val Gly Lys Asp Ala
       50                  55                  60
   tct gat gta ttt cat gcc atg cat ccc gag tca gca tat gaa atc ttg      240
   Ser Asp Val Phe His Ala Met His Pro Glu Ser Ala Tyr Glu Ile Leu
   65                  70                  75                  80
   aac aat tat ttt gta gga gat gta aaa gat gca cat gta aag gag acc      288
   Asn Asn Tyr Phe Val Gly Asp Val Lys Asp Ala His Val Lys Glu Thr
                   85                  90                  95
   cct tct gct caa ttt gct tca gaa atg cgt caa ctt cgg gat caa ttg      336
   Pro Ser Ala Gln Phe Ala Ser Glu Met Arg Gln Leu Arg Asp Gln Leu
               100                 105                 110
   aaa aaa gaa ggt tat ttt cat tct agt aaa gct tat tat gtt tac aag      384
   Lys Lys Glu Gly Tyr Phe His Ser Ser Lys Ala Tyr Tyr Val Tyr Lys
           115                 120                 125
   gtc ctc tct act ctt gct ctt tgt gca gct ggt ctt act ctt ttg tat 432
   Val Leu Ser Thr Leu Ala Leu Cys Ala Ala Gly Leu Thr Leu Leu Tyr
   130 135 140
   gct tat gga cat aca tct act tta gct gtt gtt gca tct gct att att 480
   Ala Tyr Gly His Thr Ser Thr Leu Ala Val Val Ala Ser Ala Ile Ile
   145 150 155 160
   gtt ggt atc ttt tgg caa caa tgt ggt tgg ttg gct cat gat ttt gga 528
   Val Gly Ile Phe Trp Gln Gln Cys Gly Trp Leu Ala His Asp Phe Gly
   165 170 175
   cat cat caa tgc ttt gaa gat cgc tct tgg aat gat gtt ctt gtt gtt 576
   His His Gln Cys Phe Glu Asp Arg Ser Trp Asn Asp Val Leu Val Val
   180 185 190
   ttc ctt gga aac ttt tgt caa ggt ttt tca ttg tca tgg tgg aag aat 624
   Phe Leu Gly Asn Phe Cys Gln Gly Phe Ser Leu Ser Trp Trp Lys Asn
   195 200 205
   aaa cat aac act cac cac gct agt acg aat gtt cat gga cat gat ccc 672
   Lys His Asn Thr His His Ala Ser Thr Asn Val His Gly His Asp Pro
   210 215 220
   gat att gac act gct cct gtc tta tta tgg gat gaa tat gca tct gca 720
   Asp Ile Asp Thr Ala Pro Val Leu Leu Trp Asp Glu Tyr Ala Ser Ala
   225 230 235 240
   gct tat tat gcc tca ctg gat gaa gaa ccc aca atg att tct cga ttc 768
   Ala Tyr Tyr Ala Ser Leu Asp Glu Glu Pro Thr Met Ile Ser Arg Phe
   245 250 255
   ctt gct gaa agt gtc ttg cct cat caa act cgt tat tac ttc ttt gtt 816
   Leu Ala Glu Ser Val Leu Pro His Gln Thr Arg Tyr Tyr Phe Phe Val
   260 265 270
   ctc ggc ttt gct cgt tta tca tgg gcc atc caa tcc tta ctc tac tca 864
   Leu Gly Phe Ala Arg Leu Ser Trp Ala Ile Gln Ser Leu Leu Tyr Ser
   275 280 285
   ttc aaa caa ggt gct att aac aag tct cat caa ctc aac ctc ttt gaa 912
   Phe Lys Gln Gly Ala Ile Asn Lys Ser His Gln Leu Asn Leu Phe Glu
   290 295 300
   cgc ttt tgt ctt gtt agt cac tgg act tta ttc act tac tgt aca ctt 960
   Arg Phe Cys Leu Val Ser His Trp Thr Leu Phe Thr Tyr Cys Thr Leu
   305 310 315 320
   gcc tgg tgt agc aac gtc tat cac atg att ttg ttc ttt ttg gtt agt 1008
   Ala Trp Cys Ser Asn Val Tyr His Met Ile Leu Phe Phe Leu Val Ser
   325 330 335
   caa gca act acc ggt tac aca ttg gcc ctt gta ttt gct ttg aat cac 1056
   Gln Ala Thr Thr Gly Tyr Thr Leu Ala Leu Val Phe Ala Leu Asn His
   340 345 350
   aat ggt atg cct gtg att act gaa gaa aag gcc gag tca atg gaa ttt 1104
   Asn Gly Met Pro Val Ile Thr Glu Glu Lys Ala Glu Ser Met Glu Phe
   355 360 365
   ttc gaa att caa gtg att aca ggt cgt gat gta aca ctc tct cct tta 1152
   Phe Glu Ile Gln Val Ile Thr Gly Arg Asp Val Thr Leu Ser Pro Leu
   370 375 380
   ggt gat tgg ttc atg ggt gga ttg aac tat caa atc gag cat cat gtt 1200
   Gly Asp Trp Phe Met Gly Gly Leu Asn Tyr Gln Ile Glu His His Val
   385 390 395 400
   ttc cct aat atg cct cgt cat aat tta cct aaa gta aag cca atg gtc 1248
   Phe Pro Asn Met Pro Arg His Asn Leu Pro Lys Val Lys Pro Met Val
   405 410 415
   aag tca ctt tgt aag aaa tat gat att aat tat cac gat act gga ttc 1296
   Lys Ser Leu Cys Lys Lys Tyr Asp Ile Asn Tyr His Asp Thr Gly Phe
   420 425 430
   ttg aaa gga aca ttg gaa gta cta aag aca tta gat att act tcc aag 1344
   Leu Lys Gly Thr Leu Glu Val Leu Lys Thr Leu Asp Ile Thr Ser Lys
   435 440 445
   ttg tct ttg caa ttg agc aaa aag tca ttt taa 1377
   Leu Ser Leu Gln Leu Ser Lys Lys Ser Phe
   450 455
<210> 16
   <211> 458
   <212> PRT
   <213> Rhizopus oryzae
<400> 16
   Met Ser Thr Ser Asp Arg Gln Ser Val Phe Thr Leu Lys Glu Leu Glu
   1               5                   10                  15
   Leu Ile Asn Gln Lys His Arg Asp Gly Asp Lys Ser Ala Met Lys Phe
               20                  25                  30
   Ile Ile Ile Asp Arg Lys Val Tyr Asp Val Thr Glu Phe Leu Glu Asp
           35                  40                  45
   His Pro Gly Gly Ala Gln Val Leu Leu Thr His Val Gly Lys Asp Ala
       50                  55                  60
   Ser Asp Val Phe His Ala Met His Pro Glu Ser Ala Tyr Glu Ile Leu
   65                  70                  75                  80
   Asn Asn Tyr Phe Val Gly Asp Val Lys Asp Ala His Val Lys Glu Thr
                   85                  90                  95
   Pro Ser Ala Gln Phe Ala Ser Glu Met Arg Gln Leu Arg Asp Gln Leu
               100                 105                 110
   Lys Lys Glu Gly Tyr Phe His Ser Ser Lys Ala Tyr Tyr Val Tyr Lys
           115                 120                 125
   Val Leu Ser Thr Leu Ala Leu Cys Ala Ala Gly Leu Thr Leu Leu Tyr
       130                 135                 140
   Ala Tyr Gly His Thr Ser Thr Leu Ala Val Val Ala Ser Ala Ile Ile
   145                 150                 155                 160
   Val Gly Ile Phe Trp Gln Gln Cys Gly Trp Leu Ala His Asp Phe Gly
                   165                 170                 175
   His His Gln Cys Phe Glu Asp Arg Ser Trp Asn Asp Val Leu Val Val
               180                 185                 190
   Phe Leu Gly Asn Phe Cys Gln Gly Phe Ser Leu Ser Trp Trp Lys Asn
           195                 200                 205
   Lys His Asn Thr His His Ala Ser Thr Asn Val His Gly His Asp Pro
       210                 215                 220
   Asp Ile Asp Thr Ala Pro Val Leu Leu Trp Asp Glu Tyr Ala Ser Ala
   225                 230                 235                 240
   Ala Tyr Tyr Ala Ser Leu Asp Glu Glu Pro Thr Met Ile Ser Arg Phe
                   245                 250                 255
   Leu Ala Glu Ser Val Leu Pro His Gln Thr Arg Tyr Tyr Phe Phe Val
               260                 265                 270
   Leu Gly Phe Ala Arg Leu Ser Trp Ala Ile Gln Ser Leu Leu Tyr Ser
           275                 280                 285
   Phe Lys Gln Gly Ala Ile Asn Lys Ser His Gln Leu Asn Leu Phe Glu
       290                 295                 300
   Arg Phe Cys Leu Val Ser His Trp Thr Leu Phe Thr Tyr Cys Thr Leu
   305                 310                 315                 320
   Ala Trp Cys Ser Asn Val Tyr His Met Ile Leu Phe Phe Leu Val Ser
                   325                 330                 335
   Gln Ala Thr Thr Gly Tyr Thr Leu Ala Leu Val Phe Ala Leu Asn His
               340                 345                 350
   Asn Gly Met Pro Val Ile Thr Glu Glu Lys Ala Glu Ser Met Glu Phe
           355                 360                 365
   Phe Glu Ile Gln Val Ile Thr Gly Arg Asp Val Thr Leu Ser Pro Leu
       370                 375                 380
   Gly Asp Trp Phe Met Gly Gly Leu Asn Tyr Gln Ile Glu His His Val
   385                 390                 395                 400
   Phe Pro Asn Met Pro Arg His Asn Leu Pro Lys Val Lys Pro Met Val
                   405                 410                 415
   Lys Ser Leu Cys Lys Lys Tyr Asp Ile Asn Tyr His Asp Thr Gly Phe
               420                 425                 430
   Leu Lys Gly Thr Leu Glu Val Leu Lys Thr Leu Asp Ile Thr Ser Lys
           435                 440                 445
   Leu Ser Leu Gln Leu Ser Lys Lys Ser Phe
       450                 455
<210> 17
   <211> 1404
   <212> DNA
   <213> Mucor rouxii
<220>
   <221> CDS
   <222> (1)..(1404)
<400> 17
   atg agc agc gac gta gga gca aca gta ccc cat ttt tat act cgt gct        48
   Met Ser Ser Asp Val Gly Ala Thr Val Pro His Phe Tyr Thr Arg Ala
   1               5                   10                  15
   gaa ttg gct gat atc cat caa gat gtt ctg gac aag aaa cct gaa gct        96
   Glu Leu Ala Asp Ile His Gln Asp Val Leu Asp Lys Lys Pro Glu Ala
               20                  25                  30
   aga aaa cta att gtc gtc gag aat aag gta tac gat atc aca gat ttc        144
   Arg Lys Leu Ile Val Val Glu Asn Lys Val Tyr Asp Ile Thr Asp Phe
           35                  40                  45
   gta ttt gat cac cct ggc ggt gag cga gta ctt ttg act caa gag ggc        192
   Val Phe Asp His Pro Gly Gly Glu Arg Val Leu Leu Thr Gln Glu Gly
       50                  55                  60
   aga gac gct aca gat gtc ttt cat gaa atg cat cct ccc tct gct tac        240
   Arg Asp Ala Thr Asp Val Phe His Glu Met His Pro Pro Ser Ala Tyr
   65                  70                  75                  80
   gaa ttg ctg gcg aat tgc tat gtt ggc gat tgt gag ccc aag ctg cct        288
   Glu Leu Leu Ala Asn Cys Tyr Val Gly Asp Cys Glu Pro Lys Leu Pro
                   85                  90                  95
   atc gat tca acc gat aag aag gca ttg aat tct gct gct ttt gct caa        336
   Ile Asp Ser Thr Asp Lys Lys Ala Leu Asn Ser Ala Ala Phe Ala Gln
               100                 105                 110
   gaa att cgt gat ctc cga gat aaa tta gaa aaa caa ggc tat ttc gac        384
   Glu Ile Arg Asp Leu Arg Asp Lys Leu Glu Lys Gln Gly Tyr Phe Asp
           115                 120                 125
   gct agt act ggt ttc tac atc tat aaa gtt tcc act acc cta ctt gtc        432
   Ala Ser Thr Gly Phe Tyr Ile Tyr Lys Val Ser Thr Thr Leu Leu Val
       130                 135                 140
   tgt att gta ggt tta gct atc ctc aaa gct tgg ggt aga gag tct act        480
   Cys Ile Val Gly Leu Ala Ile Leu Lys Ala Trp Gly Arg Glu Ser Thr
   145                 150                 155                 160
   ttg gct gtg ttt att gct gct tct tta gtt ggt ctt ttt tgg cag cag        528
   Leu Ala Val Phe Ile Ala Ala Ser Leu Val Gly Leu Phe Trp Gln Gln
                   165                 170                 175
   tgc ggt tgg ctt gcc cat gat tat gct cac tat caa gtc atc aaa gac        576
   Cys Gly Trp Leu Ala His Asp Tyr Ala His Tyr Gln Val Ile Lys Asp
               180                 185                 190
   ccc aat gtc aat aat ctc ttt tta gtt act ttt ggc aac ctg gtt caa        624
   Pro Asn Val Asn Asn Leu Phe Leu Val Thr Phe Gly Asn Leu Val Gln
           195                 200                 205
   ggt ttc tct ctt tca tgg tgg aaa aac aag cac aat acc cac cac gct        672
   Gly Phe Ser Leu Ser Trp Trp Lys Asn Lys His Asn Thr His His Ala
       210                 215                 220
   agc acc aat gtc tct ggc gaa gat ccc gat atc gat act gcc ccc att        720
   Ser Thr Asn Val Ser Gly Glu Asp Pro Asp Ile Asp Thr Ala Pro Ile
   225                 230                 235                 240
   ttg cta tgg gac gag ttt gca gtc gct aat ttc tat gga tcc ttg aag        768
   Leu Leu Trp Asp Glu Phe Ala Val Ala Asn Phe Tyr Gly Ser Leu Lys
                   245                 250                 255
   gac aat gcc agt gga ttt gac aga ttc att gct gaa cac att ttg ccc        816
   Asp Asn Ala Ser Gly Phe Asp Arg Phe Ile Ala Glu His Ile Leu Pro
               260                 265                 270
   tat caa act cgt tac tac ttc ttc att ctt ggt ttc gct cgt acc tct        864
   Tyr Gln Thr Arg Tyr Tyr Phe Phe Ile Leu Gly Phe Ala Arg Thr Ser
           275                 280                 285
   tgg gct atc caa tct att atc tat tcc ttc aag aac gaa aca ttg aat        912
   Trp Ala Ile Gln Ser Ile Ile Tyr Ser Phe Lys Asn Glu Thr Leu Asn
       290                 295                 300
   aaa tca aag ctc ttg tcc tgg tgt gag cgc atc ttt ttg att gtt cat        960
   Lys Ser Lys Leu Leu Ser Trp Cys Glu Arg Ile Phe Leu Ile Val His
   305                 310                 315                 320
   tgg gtc ttt ttc act tac tgc act att gcc tgg atc agc tct atc aga        1008
   Trp Val Phe Phe Thr Tyr Cys Thr Ile Ala Trp Ile Ser Ser Ile Arg
                   325                 330                 335
   aac atc gcc atg ttc ttt gtt gtt agc caa atc act act ggt tac ttg        1056
   Asn Ile Ala Met Phe Phe Val Val Ser Gln Ile Thr Thr Gly Tyr Leu
                       340                     345                     350
   ctc gcc atc gtc ttt gct atg aac cat aat ggc atg cct gtt tac agc         1104
   Leu Ala Ile Val Phe Ala Met Asn His Asn Gly Met Pro Val Tyr Ser
               355                     360                     365
   ccc gaa gaa gca aac cat acc gag ttt tat gaa ttg cag tgt atc act         1152
   Pro Glu Glu Ala Asn His Thr Glu Phe Tyr Glu Leu Gln Cys Ile Thr
           370                     375                     380
   ggt cgc gat gtc aac tgc act gta ttt ggc gat tgg ctc atg ggt gga         1200
   Gly Arg Asp Val Asn Cys Thr Val Phe Gly Asp Trp Leu Met Gly Gly
   385                     390                     395                     400
   ttg aat tat caa att gag cac cat ctt ttc ccc gaa atg cct cga cat         1248
   Leu Asn Tyr Gln Ile Glu His His Leu Phe Pro Glu Met Pro Arg His
                   405                     410                     415
   cat tta tcc aag gtg aaa tcc atg gtc aaa ccc att gct caa aag tat         1296
   His Leu Ser Lys Val Lys Ser Met Val Lys Pro Ile Ala Gln Lys Tyr
               420                     425                     430
   aat atc cct tac cat gat acc aca gtc att ggt ggt acc att gaa gtc         1344
   Asn Ile Pro Tyr His Asp Thr Thr Val Ile Gly Gly Thr Ile Glu Val
           435                     440                     445
   ttg caa acc ttg gat ttt gtt caa aag att tcg cag aaa ttc agc aaa         1392
   Leu Gln Thr Leu Asp Phe Val Gln Lys Ile Ser Gln Lys Phe Ser Lys
       450                     455                     460
   aaa atg ctc taa                                                         1404
   Lys Met Leu
   465
<210> 18
   <211> 467
   <212> PRT
   <213> Mucor rouxii
<400> 18
   Met Ser Ser Asp Val Gly Ala Thr Val Pro His Phe Tyr Thr Arg Ala
   1 5 10 15
   Glu Leu Ala Asp Ile His Gln Asp Val Leu Asp Lys Lys Pro Glu Ala
   20 25 30
   Arg Lys Leu Ile Val Val Glu Asn Lys Val Tyr Asp Ile Thr Asp Phe
   35 40 45
   Val Phe Asp His Pro Gly Gly Glu Arg Val Leu Leu Thr Gln Glu Gly
   50 55 60
   Arg Asp Ala Thr Asp Val Phe His Glu Met His Pro Pro Ser Ala Tyr
   65 70 75 80
   Glu Leu Leu Ala Asn Cys Tyr Val Gly Asp Cys Glu Pro Lys Leu Pro
   85 90 95
   Ile Asp Ser Thr Asp Lys Lys Ala Leu Asn Ser Ala Ala Phe Ala Gln
   100 105 110
   Glu Ile Arg Asp Leu Arg Asp Lys Leu Glu Lys Gln Gly Tyr Phe Asp
   115 120 125
   Ala Ser Thr Gly Phe Tyr Ile Tyr Lys Val Ser Thr Thr Leu Leu Val
   130 135 140
   Cys Ile Val Gly Leu Ala Ile Leu Lys Ala Trp Gly Arg Glu Ser Thr
   145 150 155 160
   Leu Ala Val Phe Ile Ala Ala Ser Leu Val Gly Leu Phe Trp Gln Gln
   165 170 175
   Cys Gly Trp Leu Ala His Asp Tyr Ala His Tyr Gln Val Ile Lys Asp
   180 185 190
   Pro Asn Val Asn Asn Leu Phe Leu Val Thr Phe Gly Asn Leu Val Gln
   195 200 205
   Gly Phe Ser Leu Ser Trp Trp Lys Asn Lys His Asn Thr His His Ala
   210 215 220
   Ser Thr Asn Val Ser Gly Glu Asp Pro Asp Ile Asp Thr Ala Pro Ile
   225 230 235 240
   Leu Leu Trp Asp Glu Phe Ala Val Ala Asn Phe Tyr Gly Ser Leu Lys
   245 250 255
   Asp Asn Ala Ser Gly Phe Asp Arg Phe Ile Ala Glu His Ile Leu Pro
   260 265 270
   Tyr Gln Thr Arg Tyr Tyr Phe Phe Ile Leu Gly Phe Ala Arg Thr Ser
   275 280 285
   Trp Ala Ile Gln Ser Ile Ile Tyr Ser Phe Lys Asn Glu Thr Leu Asn
   290 295 300
   Lys Ser Lys Leu Leu Ser Trp Cys Glu Arg Ile Phe Leu Ile Val His
   305 310 315 320
   Trp Val Phe Phe Thr Tyr Cys Thr Ile Ala Trp Ile Ser Ser Ile Arg
   325 330 335
   Asn Ile Ala Met Phe Phe Val Val Ser Gln Ile Thr Thr Gly Tyr Leu
   340 345 350
   Leu Ala Ile Val Phe Ala Met Asn His Asn Gly Met Pro Val Tyr Ser
   355 360 365
   Pro Glu Glu Ala Asn His Thr Glu Phe Tyr Glu Leu Gln Cys Ile Thr
   370 375 380
   Gly Arg Asp Val Asn Cys Thr Val Phe Gly Asp Trp Leu Met Gly Gly
   385 390 395 400
   Leu Asn Tyr Gln Ile Glu His His Leu Phe Pro Glu Met Pro Arg His
   405 410 415
   His Leu Ser Lys Val Lys Ser Met Val Lys Pro Ile Ala Gln Lys Tyr
   420 425 430
   Asn Ile Pro Tyr His Asp Thr Thr Val Ile Gly Gly Thr Ile Glu Val
   435 440 445
   Leu Gln Thr Leu Asp Phe Val Gln Lys Ile Ser Gln Lys Phe Ser Lys
   450 455 460
   Lys Met Leu
   465
<210> 19
   <211> 1374
   <212> DNA
   <213> Mortierella alpina
<220>
   <221> CDS
   <222> (1)..(1374)
<400> 19
   atg gct gct gct ccc agt gtg agg acg ttt act cgg gcc gag att ttg        48
   Met Ala Ala Ala Pro Ser Val Arg Thr Phe Thr Arg Ala Glu Ile Leu
   1               5                   10                  15
   aat gcc gag gct ctg aat gag ggc aag aag gat gcc gag gca ccc ttc        96
   Asn Ala Glu Ala Leu Asn Glu Gly Lys Lys Asp Ala Glu Ala Pro Phe
               20                  25                  30
   ctg atg atc atc gac aac aag gtg tac gat gtc cgc gag ttc gtc cct        144
   Leu Met Ile Ile Asp Asn Lys Val Tyr Asp Val Arg Glu Phe Val Pro
           35                  40                  45
   gat cat ccc ggt gga agt gtg att ctc acg cac gtt ggc aag gac ggc        192
   Asp His Pro Gly Gly Ser Val Ile Leu Thr His Val Gly Lys Asp Gly
       50                  55                  60
   act gac gtc ttt gac act ttt cac ccc gag gct gct tgg gag act ctt        240
   Thr Asp Val Phe Asp Thr Phe His Pro Glu Ala Ala Trp Glu Thr Leu
   65                  70                  75                  80
   gcc aac ttt tac gtt ggt gat att gac gag agc gac cgc gat atc aag        288
   Ala Asn Phe Tyr Val Gly Asp Ile Asp Glu Ser Asp Arg Asp Ile Lys
                   85                  90                  95
   aat gat gac ttt gcg gcc gag gtc cgc aag ctg cgt acc ttg ttc cag        336
   Asn Asp Asp Phe Ala Ala Glu Val Arg Lys Leu Arg Thr Leu Phe Gln
               100                 105                 110
   tct ctt ggt tac tac gat tct tcc aag gca tac tac gcc ttc aag gtc        384
   Ser Leu Gly Tyr Tyr Asp Ser Ser Lys Ala Tyr Tyr Ala Phe Lys Val
           115                 120                 125
   tcg ttc aac ctc tgc atc tgg ggt ttg tcg acg gtc att gtg gcc aag        432
   Ser Phe Asn Leu Cys Ile Trp Gly Leu Ser Thr Val Ile Val Ala Lys
       130                 135                 140
   tgg ggc cag acc tcg acc ctc gcc aac gtg ctc tcg gct gcg ctt ttg        480
   Trp Gly Gln Thr Ser Thr Leu Ala Asn Val Leu Ser Ala Ala Leu Leu
   145                 150                 155                 160
   ggt ctg ttc tgg cag cag tgc gga tgg ttg gct cac gac ttt ttg cat        528
   Gly Leu Phe Trp Gln Gln Cys Gly Trp Leu Ala His Asp Phe Leu His
                   165                 170                 175
   cac cag gtc ttc cag gac cgt ttc tgg ggt gat ctt ttc ggc gcc ttc      576
   His Gln Val Phe Gln Asp Arg Phe Trp Gly Asp Leu Phe Gly Ala Phe
               180                 185                 190
   ttg gga ggt gtc tgc cag ggc ttc tcg tcc tcg tgg tgg aag gac aag      624
   Leu Gly Gly Val Cys Gln Gly Phe Ser Ser Ser Trp Trp Lys Asp Lys
           195                 200                 205
   cac aac act cac cac gcc gcc ccc aac gtc cac ggc gaa gat ccc gac      672
   His Asn Thr His His Ala Ala Pro Asn Val His Gly Glu Asp Pro Asp
       210                 215                 220
   att gac acc cac cct ctg ttg acc tgg agt gag cat gcg ttg gag atg      720
   Ile Asp Thr His Pro Leu Leu Thr Trp Ser Glu His Ala Leu Glu Met
   225                 230                 235                 240
   ttc tcg gat gtc cca gat gag gag ctg acc cgc atg tgg tcg cgt ttc      768
   Phe Ser Asp Val Pro Asp Glu Glu Leu Thr Arg Met Trp Ser Arg Phe
                   245                 250                 255
   atg gtc ctg aac cag acc tgg ttt tac ttc ccc att ctc tcg ttt gcc      816
   Met Val Leu Asn Gln Thr Trp Phe Tyr Phe Pro Ile Leu Ser Phe Ala
               260                 265                 270
   cgt ctc tcc tgg tgc ctc cag tcc att ctc ttt gtg ctg cct aac ggt      864
   Arg Leu Ser Trp Cys Leu Gln Ser Ile Leu Phe Val Leu Pro Asn Gly
           275                 280                 285
   cag gcc cac aag ccc tcg ggc gcg cgt gtg ccc atc tcg ttg gtc gag      912
   Gln Ala His Lys Pro Ser Gly Ala Arg Val Pro Ile Ser Leu Val Glu
       290                 295                 300
   cag ctg tcg ctt gcg atg cac tgg acc tgg tac ctc gcc acc atg ttc      960
   Gln Leu Ser Leu Ala Met His Trp Thr Trp Tyr Leu Ala Thr Met Phe
   305                 310                 315                 320
   ctg ttc atc aag gat ccc gtc aac atg ctg gtg tac ttt ttg gtg tcg     1008
   Leu Phe Ile Lys Asp Pro Val Asn Met Leu Val Tyr Phe Leu Val Ser
                   325                 330                 335
   cag ccg gtg tgc gga aac ttg ttg gcg atc gtg ttc tcg ctc aac cac     1056
   Gln Pro Val Cys Gly Asn Leu Leu Ala Ile Val Phe Ser Leu Asn His
               340                 345                 350
   aac ggt atg cct gtg atc tcg aag gag gag gcg gtc gat atg gat ttc     1104
   Asn Gly Met Pro Val Ile Ser Lys Glu Glu Ala Val Asp Met Asp Phe
           355                 360                 365
   ttc acg aag cag atc atc acg ggt cgt gat gtc cac ccg ggt cta ttt     1152
   Phe Thr Lys Gln Ile Ile Thr Gly Arg Asp Val His Pro Gly Leu Phe
       370                 375                 380
   gcc aac tgg ttc acg ggt gga ttg aac tat cag atc gag cac cac ttg     1200
   Ala Asn Trp Phe Thr Gly Gly Leu Asn Tyr Gln Ile Glu His His Leu
   385                 390                 395                 400
   ttc cct tcg atg cct cgc cac aac ttt tca aag atc cag cct gct gtc     1248
   Phe Pro Ser Met Pro Arg His Asn Phe Ser Lys Ile Gln Pro Ala Val
                   405                 410                 415
   gag acc ctg tgc aaa aag tac aat gtc cga tac cac acc acc ggt atg     1296
   Glu Thr Leu Cys Lys Lys Tyr Asn Val Arg Tyr His Thr Thr Gly Met
               420                 425                 430
   atc gag gga act gca gag gtc ttt agc cgt ctg aac gag gtc tcc aag     1344
   Ile Glu Gly Thr Ala Glu Val Phe Ser Arg Leu Asn Glu Val Ser Lys
           435                 440                 445
   gct gcc tcc aag atg ggc aag gca cag taa                             1374
   Ala Ala Ser Lys Met Gly Lys Ala Gln
       450                 455
<210> 20
   <211> 457
   <212> PRT
   <213> Mortierella alpina
<400> 20
   Met Ala Ala Ala Pro Ser Val Arg Thr Phe Thr Arg Ala Glu Ile Leu
   1               5                   10                  15
   Asn Ala Glu Ala Leu Asn Glu Gly Lys Lys Asp Ala Glu Ala Pro Phe
               20                  25                  30
   Leu Met Ile Ile Asp Asn Lys Val Tyr Asp Val Arg Glu Phe Val Pro
           35                  40                  45
   Asp His Pro Gly Gly Ser Val Ile Leu Thr His Val Gly Lys Asp Gly
       50                  55                  60
   Thr Asp Val Phe Asp Thr Phe His Pro Glu Ala Ala Trp Glu Thr Leu
   65                  70                  75                  80
   Ala Asn Phe Tyr Val Gly Asp Ile Asp Glu Ser Asp Arg Asp Ile Lys
                   85                  90                  95
   Asn Asp Asp Phe Ala Ala Glu Val Arg Lys Leu Arg Thr Leu Phe Gln
               100                 105                 110
   Ser Leu Gly Tyr Tyr Asp Ser Ser Lys Ala Tyr Tyr Ala Phe Lys Val
           115                 120                 125
   Ser Phe Asn Leu Cys Ile Trp Gly Leu Ser Thr Val Ile Val Ala Lys
       130                 135                 140
   Trp Gly Gln Thr Ser Thr Leu Ala Asn Val Leu Ser Ala Ala Leu Leu
   145                 150                 155                 160
   Gly Leu Phe Trp Gln Gln Cys Gly Trp Leu Ala His Asp Phe Leu His
                   165                 170                 175
   His Gln Val Phe Gln Asp Arg Phe Trp Gly Asp Leu Phe Gly Ala Phe
               180                 185                 190
   Leu Gly Gly Val Cys Gln Gly Phe Ser Ser Ser Trp Trp Lys Asp Lys
           195                 200                 205
   His Asn Thr His His Ala Ala Pro Asn Val His Gly Glu Asp Pro Asp
       210                 215                 220
   Ile Asp Thr His Pro Leu Leu Thr Trp Ser Glu His Ala Leu Glu Met
   225                 230                 235                 240
   Phe Ser Asp Val Pro Asp Glu Glu Leu Thr Arg Met Trp Ser Arg Phe
                   245                 250                 255
   Met Val Leu Asn Gln Thr Trp Phe Tyr Phe Pro Ile Leu Ser Phe Ala
               260                 265                 270
   Arg Leu Ser Trp Cys Leu Gln Ser Ile Leu Phe Val Leu Pro Asn Gly
           275                 280                 285
   Gln Ala His Lys Pro Ser Gly Ala Arg Val Pro Ile Ser Leu Val Glu
       290                 295                 300
   Gln Leu Ser Leu Ala Met His Trp Thr Trp Tyr Leu Ala Thr Met Phe
   305                 310                 315                 320
   Leu Phe Ile Lys Asp Pro Val Asn Met Leu Val Tyr Phe Leu Val Ser
                   325                 330                 335
   Gln Pro Val Cys Gly Asn Leu Leu Ala Ile Val Phe Ser Leu Asn His
               340                 345                 350
   Asn Gly Met Pro Val Ile Ser Lys Glu Glu Ala Val Asp Met Asp Phe
           355                 360                 365
   Phe Thr Lys Gln Ile Ile Thr Gly Arg Asp Val His Pro Gly Leu Phe
       370                 375                 380
   Ala Asn Trp Phe Thr Gly Gly Leu Asn Tyr Gln Ile Glu His His Leu
   385                 390                 395                 400
   Phe Pro Ser Met Pro Arg His Asn Phe Ser Lys Ile Gln Pro Ala Val
                   405                 410                 415
   Glu Thr Leu Cys Lys Lys Tyr Asn Val Arg Tyr His Thr Thr Gly Met
               420                 425                 430
   Ile Glu Gly Thr Ala Glu Val Phe Ser Arg Leu Asn Glu Val Ser Lys
           435                 440                 445
   Ala Ala Ser Lys Met Gly Lys Ala Gln
       450                 455
<210> 21
   <211> 1377
   <212> DNA
   <213> Mortierella alpina
<220>
   <221> CDS
   <222> (1)..(1377)
<400> 21
   atg acc acc agc gac cca tct gtc aga gcg ttc aca cgc tca gaa gtg       48
   Met Thr Thr Ser Asp Pro Ser Val Arg Ala Phe Thr Arg Ser Glu Val
   1               5                   10                  15
   ttg cac gcc gat gcc ttg aac gag ggc aaa aag aac gcc gag gca ccg       96
   Leu His Ala Asp Ala Leu Asn Glu Gly Lys Lys Asn Ala Glu Ala Pro
               20                  25                  30
   ttt ctc atg atc atc gac aac aag gtc tac gat gtg cgc gag ttt atc      144
   Phe Leu Met Ile Ile Asp Asn Lys Val Tyr Asp Val Arg Glu Phe Ile
           35                  40                  45
   ccc gac cat cct ggt ggg agc gtc att ttg acc cac gta ggc aag gac      192
   Pro Asp His Pro Gly Gly Ser Val Ile Leu Thr His Val Gly Lys Asp
       50                  55                  60
   ggc acc gac gtt ttc gag acc ttc cat cct gag gct gct tgg gag acg      240
   Gly Thr Asp Val Phe Glu Thr Phe His Pro Glu Ala Ala Trp Glu Thr
   65                  70                  75                  80
   ctc gcc aat ttt tat gtc ggt gac att gta gaa tcc gat cgc gcc atc      288
   Leu Ala Asn Phe Tyr Val Gly Asp Ile Val Glu Ser Asp Arg Ala Ile
                   85                  90                  95
   gag aac gac gag ttt gca gct gag gtt cgt aag ctg cgg aca ttg ttt      336
   Glu Asn Asp Glu Phe Ala Ala Glu Val Arg Lys Leu Arg Thr Leu Phe
               100                 105                 110
   tat tct ttg ggc tac tac gac tca tcc aag gtt tac tac gcc ttc aag      384
   Tyr Ser Leu Gly Tyr Tyr Asp Ser Ser Lys Val Tyr Tyr Ala Phe Lys
           115                 120                 125
   gtc tcg ttc aac ctc tgc atc tgg ggc ctg tct gca ttc att gtt gcc      432
   Val Ser Phe Asn Leu Cys Ile Trp Gly Leu Ser Ala Phe Ile Val Ala
       130                 135                 140
   aaa tgg ggc cag acc tcg acc ctc gca aac gtg ata tca gcc tca ctc      480
   Lys Trp Gly Gln Thr Ser Thr Leu Ala Asn Val Ile Ser Ala Ser Leu
   145                 150                 155                 160
   ctg ggt gtc ttt tgg caa cag tgc ggt tgg ctc gcc cat gat ttc ttg      528
   Leu Gly Val Phe Trp Gln Gln Cys Gly Trp Leu Ala His Asp Phe Leu
                   165                 170                 175
   cac cat cag gtc ttt cac gat cga ttc tgg ggc gat ctg ttc ggt gca      576
   His His Gln Val Phe His Asp Arg Phe Trp Gly Asp Leu Phe Gly Ala
               180                 185                 190
   ttt ctc ggc gga gtc tgt caa ggt ttc tcc tcg tcc tgg tgg aag gac      624
   Phe Leu Gly Gly Val Cys Gln Gly Phe Ser Ser Ser Trp Trp Lys Asp
           195                 200                 205
   aaa cac aac acc cac cac gcg gcg ccc aat gtc cat gga gag gat ccc      672
   Lys His Asn Thr His His Ala Ala Pro Asn Val His Gly Glu Asp Pro
       210                 215                 220
   gat atc gac aca cat ccg ctt ttg acg tgg agt gag cat gcg ctc gag      720
   Asp Ile Asp Thr His Pro Leu Leu Thr Trp Ser Glu His Ala Leu Glu
   225                 230                 235                 240
   atg ttt tcg gat gtg ccc gat gag gag ctt acc caa atg tgg tcc cgg      768
   Met Phe Ser Asp Val Pro Asp Glu Glu Leu Thr Gln Met Trp Ser Arg
                   245                 250                 255
   ttt atg gtt ctg aac cag gcc tgg ttt tac ttt ccc att ctg tca ttt      816
   Phe Met Val Leu Asn Gln Ala Trp Phe Tyr Phe Pro Ile Leu Ser Phe
               260                 265                 270
   gcc cgc ctg tcc tgg tgc atc cag tcg att ctt ttt gtg cta ccg aac      864
   Ala Arg Leu Ser Trp Cys Ile Gln Ser Ile Leu Phe Val Leu Pro Asn
           275                 280                 285
   gga cag gca cac aaa cct gcg ggg gct cgg gtt ccc atc tcg ctg gtg      912
   Gly Gln Ala His Lys Pro Ala Gly Ala Arg Val Pro Ile Ser Leu Val
       290                 295                 300
   gag caa ttg tcg ttg gcg atg cac tgg acc tgg tac ctg gca acc atg      960
   Glu Gln Leu Ser Leu Ala Met His Trp Thr Trp Tyr Leu Ala Thr Met
   305                 310                 315                 320
   ttc ctg ttc atc aag gat ccc gtc aac atg atg gtg tat ttc ttg gtc     1008
   Phe Leu Phe Ile Lys Asp Pro Val Asn Met Met Val Tyr Phe Leu Val
                   325                 330                 335
   tcg caa gct gtc tgc ggc aac ctg tta gcg att gtg ttc tcg ctg aac     1056
   Ser Gln Ala Val Cys Gly Asn Leu Leu Ala Ile Val Phe Ser Leu Asn
               340                 345                 350
   cat aac ggt atg cct gtg atc tcg cag gag gaa gcg gtc gag atg gat     1104
   His Asn Gly Met Pro Val Ile Ser Gln Glu Glu Ala Val Glu Met Asp
           355                 360                 365
   ttc ttc aca aag cag atc att acg ggt cgt gat gtc tac ccg ggt tgg     1152
   Phe Phe Thr Lys Gln Ile Ile Thr Gly Arg Asp Val Tyr Pro Gly Trp
       370                 375                 380
   ttt gca gac tgg ttc acg ggt gga ttg aac tat cag att gaa cac cat     1200
   Phe Ala Asp Trp Phe Thr Gly Gly Leu Asn Tyr Gln Ile Glu His His
   385                 390                 395                 400
   ctg ttc ccg tcg atg cct cga cac cat ttc tca aag atc cag ccc gcg      1248
   Leu Phe Pro Ser Met Pro Arg His His Phe Ser Lys Ile Gln Pro Ala
                   405                 410                 415
   gtt gaa tcg ctg tgc aag aag tac ggg gtc cga tac cat acg acg ggg      1296
   Val Glu Ser Leu Cys Lys Lys Tyr Gly Val Arg Tyr His Thr Thr Gly
               420                 425                 430
   atg att gct ggc acc gca gag gtc ttt tcg cga ctg aac gag gtg tcc      1344
   Met Ile Ala Gly Thr Ala Glu Val Phe Ser Arg Leu Asn Glu Val Ser
           435                 440                 445
   cag gct gca agc aag ctc ggc aag tct gct tga                          1377
   Gln Ala Ala Ser Lys Leu Gly Lys Ser Ala
       450                 455
<210> 22
   <211> 458
   <212> PRT
   <213> Mortierella alpina
<400> 22
   Met Thr Thr Ser Asp Pro Ser Val Arg Ala Phe Thr Arg Ser Glu Val
   1               5                   10                  15
   Leu His Ala Asp Ala Leu Asn Glu Gly Lys Lys Asn Ala Glu Ala Pro
               20                  25                  30
   Phe Leu Met Ile Ile Asp Asn Lys Val Tyr Asp Val Arg Glu Phe Ile
           35                  40                  45
   Pro Asp His Pro Gly Gly Ser Val Ile Leu Thr His Val Gly Lys Asp
       50                  55                  60
   Gly Thr Asp Val Phe Glu Thr Phe His Pro Glu Ala Ala Trp Glu Thr
   65                  70                  75                  80
   Leu Ala Asn Phe Tyr Val Gly Asp Ile Val Glu Ser Asp Arg Ala Ile
                   85                  90                  95
   Glu Asn Asp Glu Phe Ala Ala Glu Val Arg Lys Leu Arg Thr Leu Phe
               100                 105                 110
   Tyr Ser Leu Gly Tyr Tyr Asp Ser Ser Lys Val Tyr Tyr Ala Phe Lys
           115                 120                 125
   Val Ser Phe Asn Leu Cys Ile Trp Gly Leu Ser Ala Phe Ile Val Ala
       130                 135                 140
   Lys Trp Gly Gln Thr Ser Thr Leu Ala Asn Val Ile Ser Ala Ser Leu
   145                 150                 155                 160
   Leu Gly Val Phe Trp Gln Gln Cys Gly Trp Leu Ala His Asp Phe Leu
                   165                 170                 175
   His His Gln Val Phe His Asp Arg Phe Trp Gly Asp Leu Phe Gly Ala
               180                 185                 190
   Phe Leu Gly Gly Val Cys Gln Gly Phe Ser Ser Ser Trp Trp Lys Asp
           195                 200                 205
   Lys His Asn Thr His His Ala Ala Pro Asn Val His Gly Glu Asp Pro
       210                 215                 220
   Asp Ile Asp Thr His Pro Leu Leu Thr Trp Ser Glu His Ala Leu Glu
   225                 230                 235                 240
   Met Phe Ser Asp Val Pro Asp Glu Glu Leu Thr Gln Met Trp Ser Arg
                   245                 250                 255
   Phe Met Val Leu Asn Gln Ala Trp Phe Tyr Phe Pro Ile Leu Ser Phe
               260                 265                 270
   Ala Arg Leu Ser Trp Cys Ile Gln Ser Ile Leu Phe Val Leu Pro Asn
           275                 280                 285
   Gly Gln Ala His Lys Pro Ala Gly Ala Arg Val Pro Ile Ser Leu Val
       290                 295                 300
   Glu Gln Leu Ser Leu Ala Met His Trp Thr Trp Tyr Leu Ala Thr Met
   305                 310                 315                 320
   Phe Leu Phe Ile Lys Asp Pro Val Asn Met Met Val Tyr Phe Leu Val
                   325                 330                 335
   Ser Gln Ala Val Cys Gly Asn Leu Leu Ala Ile Val Phe Ser Leu Asn
               340                 345                 350
   His Asn Gly Met Pro Val Ile Ser Gln Glu Glu Ala Val Glu Met Asp
           355                 360                 365
   Phe Phe Thr Lys Gln Ile Ile Thr Gly Arg Asp Val Tyr Pro Gly Trp
       370                 375                 380
   Phe Ala Asp Trp Phe Thr Gly Gly Leu Asn Tyr Gln Ile Glu His His
   385                 390                 395                 400
   Leu Phe Pro Ser Met Pro Arg His His Phe Ser Lys Ile Gln Pro Ala
                   405                 410                 415
   Val Glu Ser Leu Cys Lys Lys Tyr Gly Val Arg Tyr His Thr Thr Gly
               420                 425                 430
   Met Ile Ala Gly Thr Ala Glu Val Phe Ser Arg Leu Asn Glu Val Ser
           435                 440                 445
   Gln Ala Ala Ser Lys Leu Gly Lys Ser Ala
       450                 455
<210> 23
   <211> 1434
   <212> DNA
   <213> Phaeodactylum tricornutum
<220>
   <221> CDS
   <222> (1)..(1434)
<400> 23
   atg ggc aaa gga ggg gac gct cgg gcc tcg aag ggc tca acg gcg gct        48
   Met Gly Lys Gly Gly Asp Ala Arg Ala Ser Lys Gly Ser Thr Ala Ala
   1               5                   10                  15
   cgc aag atc agt tgg cag gaa gtc aag acc cac gcg tct ccg gag gac        96
   Arg Lys Ile Ser Trp Gln Glu Val Lys Thr His Ala Ser Pro Glu Asp
               20                  25                  30
   gcc tgg atc att cac tcc aat aag gtc tac gac gtg tcc aac tgg cac       144
   Ala Trp Ile Ile His Ser Asn Lys Val Tyr Asp Val Ser Asn Trp His
           35                  40                  45
   gaa cat ccc gga ggc gcc gtc att ttc acg cac gcc ggt gac gac atg       192
   Glu His Pro Gly Gly Ala Val Ile Phe Thr His Ala Gly Asp Asp Met
       50                  55                  60
   acg gac att ttc gct gcc ttt cac gca ccc gga tcg cag tcg ctc atg       240
   Thr Asp Ile Phe Ala Ala Phe His Ala Pro Gly Ser Gln Ser Leu Met
   65                  70                  75                  80
   aag aag ttc tac att ggc gaa ttg ctc ccg gaa acc acc ggc aag gag       288
   Lys Lys Phe Tyr Ile Gly Glu Leu Leu Pro Glu Thr Thr Gly Lys Glu
                   85                  90                  95
   ccg cag caa atc gcc ttt gaa aag ggc tac cgc gat ctg cgc tcc aaa       336
   Pro Gln Gln Ile Ala Phe Glu Lys Gly Tyr Arg Asp Leu Arg Ser Lys
               100                 105                 110
   ctc atc atg atg ggc atg ttc aag tcc aac aag tgg ttc tac gtc tac       384
   Leu Ile Met Met Gly Met Phe Lys Ser Asn Lys Trp Phe Tyr Val Tyr
           115                 120                 125
   aag tgc ctc agc aac atg gcc att tgg gcc gcc gcc tgt gct ctc gtc       432
   Lys Cys Leu Ser Asn Met Ala Ile Trp Ala Ala Ala Cys Ala Leu Val
       130                 135                 140
   ttt tac tcg gac cgc ttc tgg gta cac ctg gcc agc gcc gtc atg ctg       480
   Phe Tyr Ser Asp Arg Phe Trp Val His Leu Ala Ser Ala Val Met Leu
   145                 150                 155                 160
   gga aca ttc ttt cag cag tcg gga tgg ttg gca cac gac ttt ctg cac       528
   Gly Thr Phe Phe Gln Gln Ser Gly Trp Leu Ala His Asp Phe Leu His
                   165                 170                 175
   cac cag gtc ttc acc aag cgc aag cac ggg gat ctc gga gga ctc ttt       576
   His Gln Val Phe Thr Lys Arg Lys His Gly Asp Leu Gly Gly Leu Phe
               180                 185                 190
   tgg ggg aac ctc atg cag ggt tac tcc gta cag tgg tgg aaa aac aag       624
   Trp Gly Asn Leu Met Gln Gly Tyr Ser Val Gln Trp Trp Lys Asn Lys
           195                 200                 205
   cac aac gga cac cac gcc gtc ccc aac ctc cac tgc tcc tcc gca gtc       672
   His Asn Gly His His Ala Val Pro Asn Leu His Cys Ser Ser Ala Val
       210                 215                 220
   gcg caa gat ggg gac ccg gac atc gat acc atg ccc ctt ctc gcc tgg       720
   Ala Gln Asp Gly Asp Pro Asp Ile Asp Thr Met Pro Leu Leu Ala Trp
   225                 230                 235                 240
   tcc gtc cag caa gcc cag tct tac cgg gaa ctc caa gcc gac gga aag       768
   Ser Val Gln Gln Ala Gln Ser Tyr Arg Glu Leu Gln Ala Asp Gly Lys
                       245                 250                 255
   gat tcg ggt ttg gtc aag ttc atg atc cgt aac caa tcc tac ttt tac      816
   Asp Ser Gly Leu Val Lys Phe Met Ile Arg Asn Gln Ser Tyr Phe Tyr
                   260                 265                 270
   ttt ccc atc ttg ttg ctc gcc cgc ctg tcg tgg ttg aac gag tcc ttc      864
   Phe Pro Ile Leu Leu Leu Ala Arg Leu Ser Trp Leu Asn Glu Ser Phe
               275                 280                 285
   aag tgc gcc ttt ggg ctt gga gct gcg tcg gag aac gct gct ctc gaa      912
   Lys Cys Ala Phe Gly Leu Gly Ala Ala Ser Glu Asn Ala Ala Leu Glu
       290                 295                 300
   ctc aag gcc aag ggt ctt cag tac ccc ctt ttg gaa aag gct ggc atc      960
   Leu Lys Ala Lys Gly Leu Gln Tyr Pro Leu Leu Glu Lys Ala Gly Ile
   305                 310                 315                 320
   ctg ctg cac tac gct tgg atg ctt aca gtt tcg tcc ggc ttt gga cgc     1008
   Leu Leu His Tyr Ala Trp Met Leu Thr Val Ser Ser Gly Phe Gly Arg
                   325                 330                 335
   ttc tcg ttc gcg tac acc gca ttt tac ttt cta acc gcg acc gcg tcc     1056
   Phe Ser Phe Ala Tyr Thr Ala Phe Tyr Phe Leu Thr Ala Thr Ala Ser
               340                 345                 350
   tgt gga ttc ttg ctc gcc att gtc ttt ggc ctc ggc cac aac ggc atg     1104
   Cys Gly Phe Leu Leu Ala Ile Val Phe Gly Leu Gly His Asn Gly Met
           355                 360                 365
   gcc acc tac aat gcc gac gcc cgt ccg gac ttc tgg aag ctc caa gtc     1152
   Ala Thr Tyr Asn Ala Asp Ala Arg Pro Asp Phe Trp Lys Leu Gln Val
       370                 375                 380
   acc acg act cgc aac gtc acg ggc gga cac ggt ttc ccc caa gcc ttt     1200
   Thr Thr Thr Arg Asn Val Thr Gly Gly His Gly Phe Pro Gln Ala Phe
   385                 390                 395                 400
   gtc gac tgg ttc tgt ggt ggc ctc cag tac caa gtc gac cac cac tta     1248
   Val Asp Trp Phe Cys Gly Gly Leu Gln Tyr Gln Val Asp His His Leu
                   405                 410                 415
   ttc ccc agc ctg ccc cga cac aat ctg gcc aag aca cac gca ctg gtc     1296
   Phe Pro Ser Leu Pro Arg His Asn Leu Ala Lys Thr His Ala Leu Val
               420                 425                 430
   gaa tcg ttc tgc aag gag tgg ggt gtc cag tac cac gaa gcc gac ctt     1344
   Glu Ser Phe Cys Lys Glu Trp Gly Val Gln Tyr His Glu Ala Asp Leu
           435                 440                 445
   gtg gac ggg acc atg gaa gtc ttg cac cat ttg ggc agc gtg gcc ggc     1392
   Val Asp Gly Thr Met Glu Val Leu His His Leu Gly Ser Val Ala Gly
       450                 455                 460
   gaa ttc gtc gtg gat ttt gta cgc gat gga ccc gcc atg taa             1434
   Glu Phe Val Val Asp Phe Val Arg Asp Gly Pro Ala Met
   465                 470                 475
<210> 24
   <211> 477
   <212> PRT
   <213> Phaeodactylum tricornutum
<400> 24
   Met Gly Lys Gly Gly Asp Ala Arg Ala Ser Lys Gly Ser Thr Ala Ala
   1 5 10 15
   Arg Lys Ile Ser Trp Gln Glu Val Lys Thr His Ala Ser Pro Glu Asp
   20 25 30
   Ala Trp Ile Ile His Ser Asn Lys Val Tyr Asp Val Ser Asn Trp His
   35 40 45
   Glu His Pro Gly Gly Ala Val Ile Phe Thr His Ala Gly Asp Asp Met
   50 55 60
   Thr Asp Ile Phe Ala Ala Phe His Ala Pro Gly Ser Gln Ser Leu Met
   65 70 75 80
   Lys Lys Phe Tyr Ile Gly Glu Leu Leu Pro Glu Thr Thr Gly Lys Glu
   85 90 95
   Pro Gln Gln Ile Ala Phe Glu Lys Gly Tyr Arg Asp Leu Arg Ser Lys
   100 105 110
   Leu Ile Met Met Gly Met Phe Lys Ser Asn Lys Trp Phe Tyr Val Tyr
   115 120 125
   Lys Cys Leu Ser Asn Met Ala Ile Trp Ala Ala Ala Cys Ala Leu Val
   130 135 140
   Phe Tyr Ser Asp Arg Phe Trp Val His Leu Ala Ser Ala Val Met Leu
   145 150 155 160
   Gly Thr Phe Phe Gln Gln Ser Gly Trp Leu Ala His Asp Phe Leu His
   165 170 175
   His Gln Val Phe Thr Lys Arg Lys His Gly Asp Leu Gly Gly Leu Phe
   180 185 190
   Trp Gly Asn Leu Met Gln Gly Tyr Ser Val Gln Trp Trp Lys Asn Lys
   195 200 205
   His Asn Gly His His Ala Val Pro Asn Leu His Cys Ser Ser Ala Val
   210 215 220
   Ala Gln Asp Gly Asp Pro Asp Ile Asp Thr Met Pro Leu Leu Ala Trp
   225 230 235 240
   Ser Val Gln Gln Ala Gln Ser Tyr Arg Glu Leu Gln Ala Asp Gly Lys
   245 250 255
   Asp Ser Gly Leu Val Lys Phe Met Ile Arg Asn Gln Ser Tyr Phe Tyr
   260 265 270
   Phe Pro Ile Leu Leu Leu Ala Arg Leu Ser Trp Leu Asn Glu Ser Phe
   275 280 285
   Lys Cys Ala Phe Gly Leu Gly Ala Ala Ser Glu Asn Ala Ala Leu Glu
   290 295 300
   Leu Lys Ala Lys Gly Leu Gln Tyr Pro Leu Leu Glu Lys Ala Gly Ile
   305 310 315 320
   Leu Leu His Tyr Ala Trp Met Leu Thr Val Ser Ser Gly Phe Gly Arg
   325 330 335
   Phe Ser Phe Ala Tyr Thr Ala Phe Tyr Phe Leu Thr Ala Thr Ala Ser
   340 345 350
   Cys Gly Phe Leu Leu Ala Ile Val Phe Gly Leu Gly His Asn Gly Met
   355 360 365
   Ala Thr Tyr Asn Ala Asp Ala Arg Pro Asp Phe Trp Lys Leu Gln Val
   370 375 380
   Thr Thr Thr Arg Asn Val Thr Gly Gly His Gly Phe Pro Gln Ala Phe
   385 390 395 400
   Val Asp Trp Phe Cys Gly Gly Leu Gln Tyr Gln Val Asp His His Leu
   405 410 415
   Phe Pro Ser Leu Pro Arg His Asn Leu Ala Lys Thr His Ala Leu Val
   420 425 430
   Glu Ser Phe Cys Lys Glu Trp Gly Val Gln Tyr His Glu Ala Asp Leu
   435 440 445
   Val Asp Gly Thr Met Glu Val Leu His His Leu Gly Ser Val Ala Gly
   450 455 460
   Glu Phe Val Val Asp Phe Val Arg Asp Gly Pro Ala Met
   465 470 475
<210> 25
   <211> 1374
   <212> DNA
   <213> Mortierella isabellina
<220>
   <221> CDS
   <222> (1)..(1374)
<400> 25
   atg gct gct gct ccc agt gtg agg acg ttt act cgg gcc gag att ctg        48
   Met Ala Ala Ala Pro Ser Val Arg Thr Phe Thr Arg Ala Glu Ile Leu
   1               5                   10                  15
   aat gcc gag gcc ctg aat gag ggc aag aag gat gcc gag gca cct ttc        96
   Asn Ala Glu Ala Leu Asn Glu Gly Lys Lys Asp Ala Glu Ala Pro Phe
               20                  25                  30
   ttg atg atc atc gac aac aag gtg tat gat gtc cgc gag ttt gtc cct       144
   Leu Met Ile Ile Asp Asn Lys Val Tyr Asp Val Arg Glu Phe Val Pro
           35                  40                  45
   gat cat ccc ggt gga agt gtt att ctc acg cac gtt ggc aag gac ggc       192
   Asp His Pro Gly Gly Ser Val Ile Leu Thr His Val Gly Lys Asp Gly
       50                  55                  60
   act gac gtc ttt gac act ttc cac ccc gag gct gct tgg gag aca ctt       240
   Thr Asp Val Phe Asp Thr Phe His Pro Glu Ala Ala Trp Glu Thr Leu
   65                  70                  75                  80
   gcc aac ttt tac gtt ggt gat att gat gag agc gac cgt gcc atc aag 288
   Ala Asn Phe Tyr Val Gly Asp Ile Asp Glu Ser Asp Arg Ala Ile Lys
   85 90 95
   aac gat gac ttt gcg gcc gag gtc cgc aag ctg cgt act ttg ttc cag 336
   Asn Asp Asp Phe Ala Ala Glu Val Arg Lys Leu Arg Thr Leu Phe Gln
   100 105 110
   tct ctg ggt tac tac gat tcc tcc aag gca tac tac gcc ttc aag gtc 384
   Ser Leu Gly Tyr Tyr Asp Ser Ser Lys Ala Tyr Tyr Ala Phe Lys Val
   115 120 125
   tca ttc aac ctc tgc atc tgg ggc ctg tcg acg ttc att gtt gcc aag 432
   Ser Phe Asn Leu Cys Ile Trp Gly Leu Ser Thr Phe Ile Val Ala Lys
   130 135 140
   tgg ggc cag acc tcg acc ctc gcc aac gtg ctc tcg gct gcg ctc ttg 480
   Trp Gly Gln Thr Ser Thr Leu Ala Asn Val Leu Ser Ala Ala Leu Leu
   145 150 155 160
   ggt ctc ttc tgg cag cag tgt gga tgg ttg gcg cac gac ttt ttg cac 528
   Gly Leu Phe Trp Gln Gln Cys Gly Trp Leu Ala His Asp Phe Leu His
   165 170 175
   cac cag gtc ttc cag gac cgt ttc tgg ggt gat ctt ttc ggc gcc ttc 576
   His Gln Val Phe Gln Asp Arg Phe Trp Gly Asp Leu Phe Gly Ala Phe
   180 185 190
   ttg gga ggt gtc tgc cag ggc ttc tcg tcc tca tgg tgg aag gac aag 624
   Leu Gly Gly Val Cys Gln Gly Phe Ser Ser Ser Trp Trp Lys Asp Lys
   195 200 205
   cac aac act cac cac gcc gcc ccc aac gtc cac ggc gag gat ccc gac 672
   His Asn Thr His His Ala Ala Pro Asn Val His Gly Glu Asp Pro Asp
   210 215 220
   att gac act cac cct ctg ttg acg tgg agt gag cat gct ttg gag atg 720
   Ile Asp Thr His Pro Leu Leu Thr Trp Ser Glu His Ala Leu Glu Met
   225 230 235 240
   ttc tcg gac gtc cct gac gag gag ctg acc cgc atg tgg tcg cgc ttc 768
   Phe Ser Asp Val Pro Asp Glu Glu Leu Thr Arg Met Trp Ser Arg Phe
   245 250 255
   atg gtc ctt aac cag acc tgg ttc tac ttt ccc att ctc tcg ttt gcc 816
   Met Val Leu Asn Gln Thr Trp Phe Tyr Phe Pro Ile Leu Ser Phe Ala
   260 265 270
   cgt ctc tcc tgg tgc ctc cag tcc atc ctc ttt gtt ctg cct aac ggt 864
   Arg Leu Ser Trp Cys Leu Gln Ser Ile Leu Phe Val Leu Pro Asn Gly
   275 280 285
   cag gcc cac aag ccc tct gga gcc cgt gtg ccc att tcc ttg gtc gag 912
   Gln Ala His Lys Pro Ser Gly Ala Arg Val Pro Ile Ser Leu Val Glu
   290 295 300
   cag ctg tct ctt gcc gtg cac tgg acc tgg tac ctc gcc acc atg ttc 960
   Gln Leu Ser Leu Ala Val His Trp Thr Trp Tyr Leu Ala Thr Met Phe
   305 310 315 320
   ttg ttc att aag gac ccc gtc aac atg atg gtg tac ttt ttg gtg tct 1008
   Leu Phe Ile Lys Asp Pro Val Asn Met Met Val Tyr Phe Leu Val Ser
   325 330 335
   cag gct gtt tgc ggt aac ctg ttg gcg att gtg ttc tcg ctc aac cac 1056
   Gln Ala Val Cys Gly Asn Leu Leu Ala Ile Val Phe Ser Leu Asn His
   340 345 350
   aac ggt atg cct gtg atc tcc aag gag gaa gcc gtc gac atg gat ttc 1104
   Asn Gly Met Pro Val Ile Ser Lys Glu Glu Ala Val Asp Met Asp Phe
   355 360 365
   ttc acc aag cag atc atc acg ggt cgt gat gtt cac cct ggt ctg ttc 1152
   Phe Thr Lys Gln Ile Ile Thr Gly Arg Asp Val His Pro Gly Leu Phe
   370 375 380
   gcc aac tgg ttc acg ggt gga ttg aac tac cag att gag cac cac ttg 1200
   Ala Asn Trp Phe Thr Gly Gly Leu Asn Tyr Gln Ile Glu His His Leu
   385 390 395 400
   ttc cct tcg atg cct cgc cac aac ttt tca aag atc cag cct gct gtc 1248
   Phe Pro Ser Met Pro Arg His Asn Phe Ser Lys Ile Gln Pro Ala Val
   405 410 415
   gag acc ttg tgc aaa aag tat ggt gtc cga tac cac acc act ggc atg 1296
   Glu Thr Leu Cys Lys Lys Tyr Gly Val Arg Tyr His Thr Thr Gly Met
   420 425 430
   atc gag gga act gca gag gtc ttt agc cgt ttg aac gag gtc tcc aag 1344
   Ile Glu Gly Thr Ala Glu Val Phe Ser Arg Leu Asn Glu Val Ser Lys
           435                      440                      445
   gcc gcc tcc aag atg ggt aag gcg cag taa                                  1374
   Ala Ala Ser Lys Met Gly Lys Ala Gln
       450                     455
<210> 26
   <211> 457
   <212> PRT
   <213> Mortierella isabellina
<400> 26
   Met Ala Ala Ala Pro Ser Val Arg Thr Phe Thr Arg Ala Glu Ile Leu
   1               5                   10                  15
   Asn Ala Glu Ala Leu Asn Glu Gly Lys Lys Asp Ala Glu Ala Pro Phe
               20                  25                  30
   Leu Met Ile Ile Asp Asn Lys Val Tyr Asp Val Arg Glu Phe Val Pro
           35                  40                  45
   Asp His Pro Gly Gly Ser Val Ile Leu Thr His Val Gly Lys Asp Gly
       50                  55                  60
   Thr Asp Val Phe Asp Thr Phe His Pro Glu Ala Ala Trp Glu Thr Leu
   65                  70                  75                  80
   Ala Asn Phe Tyr Val Gly Asp Ile Asp Glu Ser Asp Arg Ala Ile Lys
                   85                  90                  95
   Asn Asp Asp Phe Ala Ala Glu Val Arg Lys Leu Arg Thr Leu Phe Gln
               100                 105                 110
   Ser Leu Gly Tyr Tyr Asp Ser Ser Lys Ala Tyr Tyr Ala Phe Lys Val
           115                 120                 125
   Ser Phe Asn Leu Cys Ile Trp Gly Leu Ser Thr Phe Ile Val Ala Lys
       130                 135                 140
   Trp Gly Gln Thr Ser Thr Leu Ala Asn Val Leu Ser Ala Ala Leu Leu
   145                 150                 155                 160
   Gly Leu Phe Trp Gln Gln Cys Gly Trp Leu Ala His Asp Phe Leu His
                   165                 170                 175
   His Gln Val Phe Gln Asp Arg Phe Trp Gly Asp Leu Phe Gly Ala Phe
               180                 185                 190
   Leu Gly Gly Val Cys Gln Gly Phe Ser Ser Ser Trp Trp Lys Asp Lys
           195                 200                 205
   His Asn Thr His His Ala Ala Pro Asn Val His Gly Glu Asp Pro Asp
       210                 215                 220
   Ile Asp Thr His Pro Leu Leu Thr Trp Ser Glu His Ala Leu Glu Met
   225                 230                 235                 240
   Phe Ser Asp Val Pro Asp Glu Glu Leu Thr Arg Met Trp Ser Arg Phe
                   245                 250                 255
   Met Val Leu Asn Gln Thr Trp Phe Tyr Phe Pro Ile Leu Ser Phe Ala
               260                 265                 270
   Arg Leu Ser Trp Cys Leu Gln Ser Ile Leu Phe Val Leu Pro Asn Gly
           275                 280                 285
   Gln Ala His Lys Pro Ser Gly Ala Arg Val Pro Ile Ser Leu Val Glu
       290                 295                 300
   Gln Leu Ser Leu Ala Val His Trp Thr Trp Tyr Leu Ala Thr Met Phe
   305                 310                 315                 320
   Leu Phe Ile Lys Asp Pro Val Asn Met Met Val Tyr Phe Leu Val Ser
                   325                 330                 335
   Gln Ala Val Cys Gly Asn Leu Leu Ala Ile Val Phe Ser Leu Asn His
               340                 345                 350
   Asn Gly Met Pro Val Ile Ser Lys Glu Glu Ala Val Asp Met Asp Phe
           355                 360                 365
   Phe Thr Lys Gln Ile Ile Thr Gly Arg Asp Val His Pro Gly Leu Phe
       370                 375                 380
   Ala Asn Trp Phe Thr Gly Gly Leu Asn Tyr Gln Ile Glu His His Leu
   385                 390                 395                 400
   Phe Pro Ser Met Pro Arg His Asn Phe Ser Lys Ile Gln Pro Ala Val
                   405                 410                 415
   Glu Thr Leu Cys Lys Lys Tyr Gly Val Arg Tyr His Thr Thr Gly Met
               420                 425                 430
   Ile Glu Gly Thr Ala Glu Val Phe Ser Arg Leu Asn Glu Val Ser Lys
           435                 440                 445
   Ala Ala Ser Lys Met Gly Lys Ala Gln
                       450                 455
<210> 27
   <211> 1374
   <212> DNA
   <213> Mortierella alpina
<220>
   <221> CDS
   <222> (1)..(1374)
<400> 27
   atg gct gct gct ccc agt gtg agg acg ttt act cgg gcc gag att ctg      48
   Met Ala Ala Ala Pro Ser Val Arg Thr Phe Thr Arg Ala Glu Ile Leu
   1               5                   10                  15
   aat gcc gag gcc ctg aat gag ggc aag aag gat gcc gag gca cct ttc      96
   Asn Ala Glu Ala Leu Asn Glu Gly Lys Lys Asp Ala Glu Ala Pro Phe
               20                  25                  30
   ttg atg atc atc gac aac aag gtg tat gat gtc cgc gag ttt gtc cct     144
   Leu Met Ile Ile Asp Asn Lys Val Tyr Asp Val Arg Glu Phe Val Pro
           35                  40                  45
   gat cat ccc ggt gga agt gtt att ctc acg cac gtt ggc aag gac ggc     192
   Asp His Pro Gly Gly Ser Val Ile Leu Thr His Val Gly Lys Asp Gly
       50                  55                  60
   act gac gtc ttt gac act ttc cac ccc gag gct gct tgg gag aca ctt     240
   Thr Asp Val Phe Asp Thr Phe His Pro Glu Ala Ala Trp Glu Thr Leu
   65                  70                  75                  80
   gcc aac ttt tac gtt ggt gat att gat gag agc gac cgt gcc atc aag     288
   Ala Asn Phe Tyr Val Gly Asp Ile Asp Glu Ser Asp Arg Ala Ile Lys
                   85                  90                  95
   aac gat gac ttt gcg gcc gag gtc cgc aag ctg cgt act ttg ttc cag     336
   Asn Asp Asp Phe Ala Ala Glu Val Arg Lys Leu Arg Thr Leu Phe Gln
               100                 105                 110
   tct ctg ggt tac tac gat tcc tcc aag gca tac tac gcc ttc aag gtc     384
   Ser Leu Gly Tyr Tyr Asp Ser Ser Lys Ala Tyr Tyr Ala Phe Lys Val
           115                 120                 125
   tca ttc aac ctc tgc atc tgg ggc ctg tcg acg ttc att gtt gcc aag     432
   Ser Phe Asn Leu Cys Ile Trp Gly Leu Ser Thr Phe Ile Val Ala Lys
       130                 135                 140
   tgg ggc cag acc tcg acc ctc gcc aac gag ctc tcg gct gcg ctc ttg     480
   Trp Gly Gln Thr Ser Thr Leu Ala Asn Glu Leu Ser Ala Ala Leu Leu
   145                 150                 155                 160
   ggt ctc ttc tgg cag cag aga gga tgg ttg gcg cac gac ttt ttg cac     528
   Gly Leu Phe Trp Gln Gln Arg Gly Trp Leu Ala His Asp Phe Leu His
                   165                 170                 175
   cac cag gtc ttc cag gac cgt ttc tgg gga gat ctt ttc ggc gcc ttc     576
   His Gln Val Phe Gln Asp Arg Phe Trp Gly Asp Leu Phe Gly Ala Phe
               180                 185                 190
   ttg gga gga gac tgc cag ggc ttc tcg tcc tca tgg tgg aag gac aag     624
   Leu Gly Gly Asp Cys Gln Gly Phe Ser Ser Ser Trp Trp Lys Asp Lys
           195                 200                 205
   cac aac act cac cac gcc gcc ccc aac gtc cac ggc gag gat ccc gac     672
   His Asn Thr His His Ala Ala Pro Asn Val His Gly Glu Asp Pro Asp
       210                 215                 220
   att gac act cac cct ctg ttg acg tgg agt gag cat gct ttg gag atg     720
   Ile Asp Thr His Pro Leu Leu Thr Trp Ser Glu His Ala Leu Glu Met
   225                 230                 235                 240
   ttc tcg gac gtc cct gac gag gag ctg acc cgc atg tgg tcg cgc ttc     768
   Phe Ser Asp Val Pro Asp Glu Glu Leu Thr Arg Met Trp Ser Arg Phe
                   245                 250                 255
   atg gtc ctt aac cag acc tgg ttc tac ttt ccc att ctc tcg ttt gcc     816
   Met Val Leu Asn Gln Thr Trp Phe Tyr Phe Pro Ile Leu Ser Phe Ala
               260                 265                 270
   cgt ctc tcc tgg tgc ctc cag tcc atc ctc ttt gtt ctg cct aac ggt     864
   Arg Leu Ser Trp Cys Leu Gln Ser Ile Leu Phe Val Leu Pro Asn Gly
           275                 280                 285
   cag gcc cac aag ccc tct gga gcc cgt gtg ccc att tcc ttg gtc gag     912
   Gln Ala His Lys Pro Ser Gly Ala Arg Val Pro Ile Ser Leu Val Glu
               290                     295                     300
   cag ctg tct ctt gcc atg cac tgg acc tgg tac ctc gcc acc atg ttc      960
   Gln Leu Ser Leu Ala Met His Trp Thr Trp Tyr Leu Ala Thr Met Phe
   305                     310                     315                 320
   ttg ttc att aag gac ccc gtc aac atg atg gtg tac ttt ttg gtg tct     1008
   Leu Phe Ile Lys Asp Pro Val Asn Met Met Val Tyr Phe Leu Val Ser
                       325                     330                 335
   cag gct gtt tgc ggt aac ctg ttg gcg att gtg ttc tcg ctc aac cac     1056
   Gln Ala Val Cys Gly Asn Leu Leu Ala Ile Val Phe Ser Leu Asn His
               340                     345                 350
   aac ggt atg cct gtg atc tcc aag gag gaa gcc gtc gac atg gat ttc     1104
   Asn Gly Met Pro Val Ile Ser Lys Glu Glu Ala Val Asp Met Asp Phe
               355                     360                 365
   ttc acc aag cag atc atc acg ggt cgt gat gtt cac cct ggt ctg ttc     1152
   Phe Thr Lys Gln Ile Ile Thr Gly Arg Asp Val His Pro Gly Leu Phe
       370                     375                     380
   gcc aac tgg ttc acg ggt gga ttg aac tac cag att gag cac cac ttg     1200
   Ala Asn Trp Phe Thr Gly Gly Leu Asn Tyr Gln Ile Glu His His Leu
   385                     390                     395                 400
   ttc cct tcg atg cct cgc cac aac ttt tca aag atc cag cct gct gtc     1248
   Phe Pro Ser Met Pro Arg His Asn Phe Ser Lys Ile Gln Pro Ala Val
                       405                     410                 415
   gag acc ttg tgc aaa aag tat ggt gtc cga tac cac acc act ggc atg     1296
   Glu Thr Leu Cys Lys Lys Tyr Gly Val Arg Tyr His Thr Thr Gly Met
               420                     425                 430
   atc gag gga act gca gag gtc ttt agc cgt ttg aac gag gtc tcc aag     1344
   Ile Glu Gly Thr Ala Glu Val Phe Ser Arg Leu Asn Glu Val Ser Lys
           435                     440                 445
   gcc gcc tcc aag atg ggt aag gcg cag taa                             1374
   Ala Ala Ser Lys Met Gly Lys Ala Gln
       450                     455
<210> 28
   <211> 457
   <212> PRT
   <213> Mortierella alpina
<400> 28
   Met Ala Ala Ala Pro Ser Val Arg Thr Phe Thr Arg Ala Glu Ile Leu
   1               5                   10                  15
   Asn Ala Glu Ala Leu Asn Glu Gly Lys Lys Asp Ala Glu Ala Pro Phe
               20                  25                  30
   Leu Met Ile Ile Asp Asn Lys Val Tyr Asp Val Arg Glu Phe Val Pro
           35                  40                  45
   Asp His Pro Gly Gly Ser Val Ile Leu Thr His Val Gly Lys Asp Gly
       50                  55                  60
   Thr Asp Val Phe Asp Thr Phe His Pro Glu Ala Ala Trp Glu Thr Leu
   65                  70                  75                  80
   Ala Asn Phe Tyr Val Gly Asp Ile Asp Glu Ser Asp Arg Ala Ile Lys
                   85                  90                  95
   Asn Asp Asp Phe Ala Ala Glu Val Arg Lys Leu Arg Thr Leu Phe Gln
               100                 105                 110
   Ser Leu Gly Tyr Tyr Asp Ser Ser Lys Ala Tyr Tyr Ala Phe Lys Val
           115                 120                 125
   Ser Phe Asn Leu Cys Ile Trp Gly Leu Ser Thr Phe Ile Val Ala Lys
       130                 135                 140
   Trp Gly Gln Thr Ser Thr Leu Ala Asn Glu Leu Ser Ala Ala Leu Leu
   145                 150                 155                 160
   Gly Leu Phe Trp Gln Gln Arg Gly Trp Leu Ala His Asp Phe Leu His
                   165                 170                 175
   His Gln Val Phe Gln Asp Arg Phe Trp Gly Asp Leu Phe Gly Ala Phe
               180                 185                 190
   Leu Gly Gly Asp Cys Gln Gly Phe Ser Ser Ser Trp Trp Lys Asp Lys
           195                 200                 205
   His Asn Thr His His Ala Ala Pro Asn Val His Gly Glu Asp Pro Asp
       210                 215                 220
   Ile Asp Thr His Pro Leu Leu Thr Trp Ser Glu His Ala Leu Glu Met
   225                 230                 235                 240
   Phe Ser Asp Val Pro Asp Glu Glu Leu Thr Arg Met Trp Ser Arg Phe
                   245                 250                 255
   Met Val Leu Asn Gln Thr Trp Phe Tyr Phe Pro Ile Leu Ser Phe Ala
               260                 265                 270
   Arg Leu Ser Trp Cys Leu Gln Ser Ile Leu Phe Val Leu Pro Asn Gly
           275                 280                 285
   Gln Ala His Lys Pro Ser Gly Ala Arg Val Pro Ile Ser Leu Val Glu
       290                 295                 300
   Gln Leu Ser Leu Ala Met His Trp Thr Trp Tyr Leu Ala Thr Met Phe
   305                 310                 315                 320
   Leu Phe Ile Lys Asp Pro Val Asn Met Met Val Tyr Phe Leu Val Ser
                   325                 330                 335
   Gln Ala Val Cys Gly Asn Leu Leu Ala Ile Val Phe Ser Leu Asn His
               340                 345                 350
   Asn Gly Met Pro Val Ile Ser Lys Glu Glu Ala Val Asp Met Asp Phe
           355                 360                 365
   Phe Thr Lys Gln Ile Ile Thr Gly Arg Asp Val His Pro Gly Leu Phe
       370                 375                 380
   Ala Asn Trp Phe Thr Gly Gly Leu Asn Tyr Gln Ile Glu His His Leu
   385                 390                 395                 400
   Phe Pro Ser Met Pro Arg His Asn Phe Ser Lys Ile Gln Pro Ala Val
                   405                 410                 415
   Glu Thr Leu Cys Lys Lys Tyr Gly Val Arg Tyr His Thr Thr Gly Met
               420                 425                 430
   Ile Glu Gly Thr Ala Glu Val Phe Ser Arg Leu Asn Glu Val Ser Lys
           435                 440                 445
   Ala Ala Ser Lys Met Gly Lys Ala Gln
       450                 455
<210> 29
   <211> 1380
   <212> DNA
   <213> Pythium irregulare
<220>
   <221> CDS
   <222> (1)..(1380)
<400> 29
   atg gtg gac ctc aag cct gga gtg aag cgc ctg gtg agc tgg aag gag        48
   Met Val Asp Leu Lys Pro Gly Val Lys Arg Leu Val Ser Trp Lys Glu
   1               5                   10                  15
   atc cgc gag cac gcg acg ccc gcg acc gcg tgg atc gtg att cac cac        96
   Ile Arg Glu His Ala Thr Pro Ala Thr Ala Trp Ile Val Ile His His
               20                  25                  30
   aag gtc tac gac atc tcc aag tgg gac tcg cac ccg ggt ggc tcc gtg       144
   Lys Val Tyr Asp Ile Ser Lys Trp Asp Ser His Pro Gly Gly Ser Val
           35                  40                  45
   atg ctc acg cag gcc ggc gag gac gcc acg gac gcc ttc gcg gtc ttc       192
   Met Leu Thr Gln Ala Gly Glu Asp Ala Thr Asp Ala Phe Ala Val Phe
       50                  55                  60
   cac ccg tcc tcg gcg ctc aag ctg ctc gag cag ttc tac gtc ggc gac       240
   His Pro Ser Ser Ala Leu Lys Leu Leu Glu Gln Phe Tyr Val Gly Asp
   65                  70                  75                  80
   gtg gac gaa acc tcc aag gcc gag atc gag ggg gag ccg gcg agc gac       288
   Val Asp Glu Thr Ser Lys Ala Glu Ile Glu Gly Glu Pro Ala Ser Asp
                   85                  90                  95
   gag gag cgc gcg cgc cgc gag cgc atc aac gag ttc atc gcg tcc tac       336
   Glu Glu Arg Ala Arg Arg Glu Arg Ile Asn Glu Phe Ile Ala Ser Tyr
               100                 105                 110
   cgt cgt ctg cgc gtc aag gtc aag ggc atg ggg ctc tac gac gcc agc       384
   Arg Arg Leu Arg Val Lys Val Lys Gly Met Gly Leu Tyr Asp Ala Ser
           115                 120                 125
   gcg ctc tac tac gcg tgg aag ctc gtg agc acg ttc ggc atc gcg gtg       432
   Ala Leu Tyr Tyr Ala Trp Lys Leu Val Ser Thr Phe Gly Ile Ala Val
       130                 135                 140
   ctc tcg atg gcg atc tgc ttc ttc ttc aac agt ttc gcc atg tac atg       480
   Leu Ser Met Ala Ile Cys Phe Phe Phe Asn Ser Phe Ala Met Tyr Met
   145 150 155 160
   gtc gcc ggc gtg att atg ggg ctc ttc tac cag cag tcc gga tgg ctg 528
   Val Ala Gly Val Ile Met Gly Leu Phe Tyr Gln Gln Ser Gly Trp Leu
   165 170 175
   gcg cac gac ttc ttg cac aac cag gtg tgc gag aac cgc acg ctc ggc 576
   Ala His Asp Phe Leu His Asn Gln Val Cys Glu Asn Arg Thr Leu Gly
   180 185 190
   aac ctt atc ggc tgc ctc gtg ggc aac gcc tgg cag ggc ttc agc gtg 624
   Asn Leu Ile Gly Cys Leu Val Gly Asn Ala Trp Gln Gly Phe Ser Val
   195 200 205
   cag tgg tgg aag aac aag cac aac ctg cac cac gcg gtg ccg aac ctg 672
   Gln Trp Trp Lys Asn Lys His Asn Leu His His Ala Val Pro Asn Leu
   210 215 220
   cac agc gcc aag gac gag ggc ttc atc ggc gac ccg gac atc gac acc 720
   His Ser Ala Lys Asp Glu Gly Phe Ile Gly Asp Pro Asp Ile Asp Thr
   225 230 235 240
   atg ccg ctg ctg gcg tgg tct aag gag atg gcg cgc aag gcg ttc gag 768
   Met Pro Leu Leu Ala Trp Ser Lys Glu Met Ala Arg Lys Ala Phe Glu
   245 250 255
   tcg gcg cac ggc ccg ttc ttc atc cgc aac cag gcg ttc cta tac ttc 816
   Ser Ala His Gly Pro Phe Phe Ile Arg Asn Gln Ala Phe Leu Tyr Phe
   260 265 270
   ccg ctg ctg ctg ctc gcg cgc ctg agc tgg ctc gcg cag tcg ttc ttc 864
   Pro Leu Leu Leu Leu Ala Arg Leu Ser Trp Leu Ala Gln Ser Phe Phe
   275 280 285
   tac gtg ttc acc gag ttc tcg ttc ggc atc ttc gac aag gtc gag ttc 912
   Tyr Val Phe Thr Glu Phe Ser Phe Gly Ile Phe Asp Lys Val Glu Phe
   290 295 300
   gac gga ccg gag aag gcg ggt ctg atc gtg cac tac atc tgg cag ctc 960
   Asp Gly Pro Glu Lys Ala Gly Leu Ile Val His Tyr Ile Trp Gln Leu
   305 310 315 320
   gcg atc ccg tac ttc tgc aac atg agc ctg ttt gag ggc gtg gca tac 1008
   Ala Ile Pro Tyr Phe Cys Asn Met Ser Leu Phe Glu Gly Val Ala Tyr
   325 330 335
   ttc ctc atg ggc cag gcg tcc tgc ggc ttg ctc ctg gcg ctg gtg ttc 1056
   Phe Leu Met Gly Gln Ala Ser Cys Gly Leu Leu Leu Ala Leu Val Phe
   340 345 350
   agt att ggc cac aac ggc atg tcg gtg tac gag cgc gaa acc aag ccg 1104
   Ser Ile Gly His Asn Gly Met Ser Val Tyr Glu Arg Glu Thr Lys Pro
   355 360 365
   gac ttc tgg cag ctg cag gtg acc acg acg cgc aac atc cgc gcg tcg 1152
   Asp Phe Trp Gln Leu Gln Val Thr Thr Thr Arg Asn Ile Arg Ala Ser
   370 375 380
   gta ttc atg gac tgg ttc acc ggt ggc ttg aac tac cag atc gac cat 1200
   Val Phe Met Asp Trp Phe Thr Gly Gly Leu Asn Tyr Gln Ile Asp His
   385 390 395 400
   cac ctg ttc ccg ctc gtg ccg cgc cac aac ttg cca aag gtc aac gtg 1248
   His Leu Phe Pro Leu Val Pro Arg His Asn Leu Pro Lys Val Asn Val
   405 410 415
   ctc atc aag tcg cta tgc aag gag ttc gac atc ccg ttc cac gag acc 1296
   Leu Ile Lys Ser Leu Cys Lys Glu Phe Asp Ile Pro Phe His Glu Thr
   420 425 430
   ggc ttc tgg gag ggc atc tac gag gtc gtg gac cac ctg gcg gac atc 1344
   Gly Phe Trp Glu Gly Ile Tyr Glu Val Val Asp His Leu Ala Asp Ile
   435 440 445
   agc aag gaa ttc atc acc gag ttc cca gcg atg taa 1380
   Ser Lys Glu Phe Ile Thr Glu Phe Pro Ala Met
   450 455
<210> 30
   <211> 459
   <212> PRT
   <213> Pythium irregulare
<400> 30
   Met Val Asp Leu Lys Pro Gly Val Lys Arg Leu Val Ser Trp Lys Glu
   1               5                   10                  15
   Ile Arg Glu His Ala Thr Pro Ala Thr Ala Trp Ile Val Ile His His
               20                  25                  30
   Lys Val Tyr Asp Ile Ser Lys Trp Asp Ser His Pro Gly Gly Ser Val
           35                  40                  45
   Met Leu Thr Gln Ala Gly Glu Asp Ala Thr Asp Ala Phe Ala Val Phe
       50                  55                  60
   His Pro Ser Ser Ala Leu Lys Leu Leu Glu Gln Phe Tyr Val Gly Asp
   65                  70                  75                  80
   Val Asp Glu Thr Ser Lys Ala Glu Ile Glu Gly Glu Pro Ala Ser Asp
                   85                  90                  95
   Glu Glu Arg Ala Arg Arg Glu Arg Ile Asn Glu Phe Ile Ala Ser Tyr
               100                 105                 110
   Arg Arg Leu Arg Val Lys Val Lys Gly Met Gly Leu Tyr Asp Ala Ser
           115                 120                 125
   Ala Leu Tyr Tyr Ala Trp Lys Leu Val Ser Thr Phe Gly Ile Ala Val
       130                 135                 140
   Leu Ser Met Ala Ile Cys Phe Phe Phe Asn Ser Phe Ala Met Tyr Met
   145                 150                 155                 160
   Val Ala Gly Val Ile Met Gly Leu Phe Tyr Gln Gln Ser Gly Trp Leu
                   165                 170                 175
   Ala His Asp Phe Leu His Asn Gln Val Cys Glu Asn Arg Thr Leu Gly
               180                 185                 190
   Asn Leu Ile Gly Cys Leu Val Gly Asn Ala Trp Gln Gly Phe Ser Val
           195                 200                 205
   Gln Trp Trp Lys Asn Lys His Asn Leu His His Ala Val Pro Asn Leu
       210                 215                 220
   His Ser Ala Lys Asp Glu Gly Phe Ile Gly Asp Pro Asp Ile Asp Thr
   225                 230                 235                 240
   Met Pro Leu Leu Ala Trp Ser Lys Glu Met Ala Arg Lys Ala Phe Glu
                   245                 250                 255
   Ser Ala His Gly Pro Phe Phe Ile Arg Asn Gln Ala Phe Leu Tyr Phe
               260                 265                 270
   Pro Leu Leu Leu Leu Ala Arg Leu Ser Trp Leu Ala Gln Ser Phe Phe
           275                 280                 285
   Tyr Val Phe Thr Glu Phe Ser Phe Gly Ile Phe Asp Lys Val Glu Phe
       290                 295                 300
   Asp Gly Pro Glu Lys Ala Gly Leu Ile Val His Tyr Ile Trp Gln Leu
   305                 310                 315                 320
   Ala Ile Pro Tyr Phe Cys Asn Met Ser Leu Phe Glu Gly Val Ala Tyr
                   325                 330                 335
   Phe Leu Met Gly Gln Ala Ser Cys Gly Leu Leu Leu Ala Leu Val Phe
               340                 345                 350
   Ser Ile Gly His Asn Gly Met Ser Val Tyr Glu Arg Glu Thr Lys Pro
           355                 360                 365
   Asp Phe Trp Gln Leu Gln Val Thr Thr Thr Arg Asn Ile Arg Ala Ser
       370                 375                 380
   Val Phe Met Asp Trp Phe Thr Gly Gly Leu Asn Tyr Gln Ile Asp His
   385                 390                 395                 400
   His Leu Phe Pro Leu Val Pro Arg His Asn Leu Pro Lys Val Asn Val
                   405                 410                 415
   Leu Ile Lys Ser Leu Cys Lys Glu Phe Asp Ile Pro Phe His Glu Thr
               420                 425                 430
   Gly Phe Trp Glu Gly Ile Tyr Glu Val Val Asp His Leu Ala Asp Ile
           435                 440                 445
   Ser Lys Glu Phe Ile Thr Glu Phe Pro Ala Met
       450                 455

<210> 31
   <211> 1374
   <212> DNA
   <213> Mortierella alpina
<220>
   <221> CDS
   <222> (1)..(1374)
<400> 31
   atg gct gct gct ccc agt gtg agg acg ttt act cgg gcc gag att ctg        48
   Met Ala Ala Ala Pro Ser Val Arg Thr Phe Thr Arg Ala Glu Ile Leu
   1               5                   10                  15
   aat gcc gag gcc ctg aat gag ggc aag aag gat gcc gag gca cct ttc      96
   Asn Ala Glu Ala Leu Asn Glu Gly Lys Lys Asp Ala Glu Ala Pro Phe
               20                  25                  30
   ttg atg atc atc gac aac aag gtg tat gat gtc cgc gag ttt gtc cct      144
   Leu Met Ile Ile Asp Asn Lys Val Tyr Asp Val Arg Glu Phe Val Pro
           35                  40                  45
   gat cat ccc ggt gga agt gtt att ctc acg cac gtt ggc aag gac ggc      192
   Asp His Pro Gly Gly Ser Val Ile Leu Thr His Val Gly Lys Asp Gly
       50                  55                  60
   act gac gtc ttt gac act ttc cac ccc gag gct gct tgg gag aca ctt      240
   Thr Asp Val Phe Asp Thr Phe His Pro Glu Ala Ala Trp Glu Thr Leu
   65                  70                  75                  80
   gcc aac ttt tac gtt gga gat att gat gag agc gac cgt gcc atc aag      288
   Ala Asn Phe Tyr Val Gly Asp Ile Asp Glu Ser Asp Arg Ala Ile Lys
                   85                  90                  95
   aac gat gac ttt gcg gcc gag gtc cgc aag ctg cgt act ttg ttc cag      336
   Asn Asp Asp Phe Ala Ala Glu Val Arg Lys Leu Arg Thr Leu Phe Gln
               100                 105                 110
   tct ctg ggt tac tac gat tcc tcc aag gca tac tac gcc ttc aag gtc      384
   Ser Leu Gly Tyr Tyr Asp Ser Ser Lys Ala Tyr Tyr Ala Phe Lys Val
           115                 120                 125
   tca ttc aac ctc tgc atc tgg ggc ctg tcg acg ttc att gtt gcc aag      432
   Ser Phe Asn Leu Cys Ile Trp Gly Leu Ser Thr Phe Ile Val Ala Lys
       130                 135                 140
   agg ggc cag acc tcg acc ctc gcc aac gag ctc tcg gct gcg ctc ttg      480
   Arg Gly Gln Thr Ser Thr Leu Ala Asn Glu Leu Ser Ala Ala Leu Leu
   145                 150                 155                 160
   ggt ctc ttc tgg cag cag aga gga tgg ttg gcg cac gac ttt ttg cac      528
   Gly Leu Phe Trp Gln Gln Arg Gly Trp Leu Ala His Asp Phe Leu His
                   165                 170                 175
   cac cag gtc ttc cag gac cgt ttc tgg gga gat ctt ttc ggc gcc ttc      576
   His Gln Val Phe Gln Asp Arg Phe Trp Gly Asp Leu Phe Gly Ala Phe
               180                 185                 190
   ttg gga gga gac tgc cag ggc ttc tcg tcc tca tgg tgg aag gac aag      624
   Leu Gly Gly Asp Cys Gln Gly Phe Ser Ser Ser Trp Trp Lys Asp Lys
           195                 200                 205
   cac aac act cac cac gcc gcc ccc aac gtc cac ggc gag gat ccc gac      672
   His Asn Thr His His Ala Ala Pro Asn Val His Gly Glu Asp Pro Asp
       210                 215                 220
   att gac act cac cct ctg ttg acg tgg agt gag cat gct ttg gag atg      720
   Ile Asp Thr His Pro Leu Leu Thr Trp Ser Glu His Ala Leu Glu Met
   225                 230                 235                 240
   ttc tcg gac gtc cct gac gag gag ctg acc cgc atg tgg tcg cgc ttc      768
   Phe Ser Asp Val Pro Asp Glu Glu Leu Thr Arg Met Trp Ser Arg Phe
                   245                 250                 255
   atg gtc ctt aac cag acc tgg ttc tac ttt ccc att ctc tcg ttt gcc      816
   Met Val Leu Asn Gln Thr Trp Phe Tyr Phe Pro Ile Leu Ser Phe Ala
               260                 265                 270
   cgt ctc tcc tgg tgc ctc cag tcc atc ctc ttt gtt ctg cct aac ggt      864
   Arg Leu Ser Trp Cys Leu Gln Ser Ile Leu Phe Val Leu Pro Asn Gly
           275                 280                 285
   cag gcc cac aag ccc tct gga gcc cgt gtg ccc att tcc ttg gtc gag      912
   Gln Ala His Lys Pro Ser Gly Ala Arg Val Pro Ile Ser Leu Val Glu
       290                 295                 300
   cag ctg tct ctt gcc atg cac tgg acc tgg tac ctc gcc acc atg ttc      960
   Gln Leu Ser Leu Ala Met His Trp Thr Trp Tyr Leu Ala Thr Met Phe
   305                 310                 315                 320
   ttg ttc att aag gac ccc gtc aac atg atg gtg tac ttt ttg gtg tct      1008
   Leu Phe Ile Lys Asp Pro Val Asn Met Met Val Tyr Phe Leu Val Ser
                   325                 330                 335
   cag gct gtt tgc ggt aac ctg ttg gcg att gtg ttc tcg ctc aac cac      1056
   Gln Ala Val Cys Gly Asn Leu Leu Ala Ile Val Phe Ser Leu Asn His
               340                 345                 350
   aac ggt atg cct gtg atc tcc aag gag gaa gcc gtc gac atg gat ttc      1104
   Asn Gly Met Pro Val Ile Ser Lys Glu Glu Ala Val Asp Met Asp Phe
           355                 360                 365
   ttc acc aag cag atc atc acg ggt cgt gat gtt cac cct ggt ctg ttc      1152
   Phe Thr Lys Gln Ile Ile Thr Gly Arg Asp Val His Pro Gly Leu Phe
       370                 375                 380
   gcc aac tgg ttc acg ggt gga ttg aac tac cag att gag cac cac ttg      1200
   Ala Asn Trp Phe Thr Gly Gly Leu Asn Tyr Gln Ile Glu His His Leu
   385                 390                 395                 400
   ttc cct tcg atg cct cgc cac aac ttt tca aag atc cag cct gct gtc      1248
   Phe Pro Ser Met Pro Arg His Asn Phe Ser Lys Ile Gln Pro Ala Val
                   405                 410                 415
   gag acc ttg tgc aaa aag tat ggt gtc cga tac cac acc act ggc atg      1296
   Glu Thr Leu Cys Lys Lys Tyr Gly Val Arg Tyr His Thr Thr Gly Met
               420                 425                 430
   atc gag gga act gca gag gtc ttt agc cgt ttg aac gag gtc tcc aag      1344
   Ile Glu Gly Thr Ala Glu Val Phe Ser Arg Leu Asn Glu Val Ser Lys
           435                 440                 445
   gcc gcc tcc aag atg ggt aag gcg cag taa                              1374
   Ala Ala Ser Lys Met Gly Lys Ala Gln
       450                 455
<210> 32
   <211> 457
   <212> PRT
   <213> Mortierella alpina
<400> 32
   Met Ala Ala Ala Pro Ser Val Arg Thr Phe Thr Arg Ala Glu Ile Leu
   1               5                   10                  15
   Asn Ala Glu Ala Leu Asn Glu Gly Lys Lys Asp Ala Glu Ala Pro Phe
               20                  25                  30
   Leu Met Ile Ile Asp Asn Lys Val Tyr Asp Val Arg Glu Phe Val Pro
           35                  40                  45
   Asp His Pro Gly Gly Ser Val Ile Leu Thr His Val Gly Lys Asp Gly
       50                  55                  60
   Thr Asp Val Phe Asp Thr Phe His Pro Glu Ala Ala Trp Glu Thr Leu
   65                  70                  75                  80
   Ala Asn Phe Tyr Val Gly Asp Ile Asp Glu Ser Asp Arg Ala Ile Lys
                   85                  90                  95
   Asn Asp Asp Phe Ala Ala Glu Val Arg Lys Leu Arg Thr Leu Phe Gln
               100                 105                 110
   Ser Leu Gly Tyr Tyr Asp Ser Ser Lys Ala Tyr Tyr Ala Phe Lys Val
           115                 120                 125
   Ser Phe Asn Leu Cys Ile Trp Gly Leu Ser Thr Phe Ile Val Ala Lys
       130                 135                 140
   Arg Gly Gln Thr Ser Thr Leu Ala Asn Glu Leu Ser Ala Ala Leu Leu
   145                 150                 155                 160
   Gly Leu Phe Trp Gln Gln Arg Gly Trp Leu Ala His Asp Phe Leu His
                   165                 170                 175
   His Gln Val Phe Gln Asp Arg Phe Trp Gly Asp Leu Phe Gly Ala Phe
               180                 185                 190
   Leu Gly Gly Asp Cys Gln Gly Phe Ser Ser Ser Trp Trp Lys Asp Lys
           195                 200                 205
   His Asn Thr His His Ala Ala Pro Asn Val His Gly Glu Asp Pro Asp
       210                 215                 220
   Ile Asp Thr His Pro Leu Leu Thr Trp Ser Glu His Ala Leu Glu Met
   225                 230                 235                 240
   Phe Ser Asp Val Pro Asp Glu Glu Leu Thr Arg Met Trp Ser Arg Phe
                   245                 250                 255
   Met Val Leu Asn Gln Thr Trp Phe Tyr Phe Pro Ile Leu Ser Phe Ala
               260                 265                 270
   Arg Leu Ser Trp Cys Leu Gln Ser Ile Leu Phe Val Leu Pro Asn Gly
           275                 280                 285
   Gln Ala His Lys Pro Ser Gly Ala Arg Val Pro Ile Ser Leu Val Glu
       290                 295                 300
   Gln Leu Ser Leu Ala Met His Trp Thr Trp Tyr Leu Ala Thr Met Phe
   305                 310                 315                 320
   Leu Phe Ile Lys Asp Pro Val Asn Met Met Val Tyr Phe Leu Val Ser
                   325                 330                 335
   Gln Ala Val Cys Gly Asn Leu Leu Ala Ile Val Phe Ser Leu Asn His
               340                 345                 350
   Asn Gly Met Pro Val Ile Ser Lys Glu Glu Ala Val Asp Met Asp Phe
           355                 360                 365
   Phe Thr Lys Gln Ile Ile Thr Gly Arg Asp Val His Pro Gly Leu Phe
       370                 375                 380
   Ala Asn Trp Phe Thr Gly Gly Leu Asn Tyr Gln Ile Glu His His Leu
   385                 390                 395                 400
   Phe Pro Ser Met Pro Arg His Asn Phe Ser Lys Ile Gln Pro Ala Val
                   405                 410                 415
   Glu Thr Leu Cys Lys Lys Tyr Gly Val Arg Tyr His Thr Thr Gly Met
               420                 425                 430
   Ile Glu Gly Thr Ala Glu Val Phe Ser Arg Leu Asn Glu Val Ser Lys
           435                 440                 445
   Ala Ala Ser Lys Met Gly Lys Ala Gln
       450                 455
<210> 33
   <211> 1374
   <212> DNA
   <213> Mortierella isabellina
<220>
   <221> CDS
   <222> (1)..(1374)
<400> 33
   atg gct gct gct ccc agt gtg agg acg ttt act cgg gcc gag att ctg      48
   Met Ala Ala Ala Pro Ser Val Arg Thr Phe Thr Arg Ala Glu Ile Leu
   1               5                   10                  15
   aat gcc gag gcc ctg aat gag ggc aag aag gat gcc gag gca cct ttc      96
   Asn Ala Glu Ala Leu Asn Glu Gly Lys Lys Asp Ala Glu Ala Pro Phe
               20                  25                  30
   ttg atg atc atc gac aac aag gtg tat gat gtc cgc gag ttt gtc cct      144
   Leu Met Ile Ile Asp Asn Lys Val Tyr Asp Val Arg Glu Phe Val Pro
           35                  40                  45
   gat cat ccc ggt gga agt gtt att ctc acg cac gtt ggc aag gac ggc      192
   Asp His Pro Gly Gly Ser Val Ile Leu Thr His Val Gly Lys Asp Gly
       50                  55                  60
   act gac gtc ttt gac act ttc cac ccc gag gct gct tgg gag aca ctt      240
   Thr Asp Val Phe Asp Thr Phe His Pro Glu Ala Ala Trp Glu Thr Leu
   65                  70                  75                  80
   gcc aac ttt tat gtt ggt gat att gat gag agc gac cgt gcc atc aaa      288
   Ala Asn Phe Tyr Val Gly Asp Ile Asp Glu Ser Asp Arg Ala Ile Lys
                   85                  90                  95
   aac gat gac ttt gcg gcc gag gtc cgc aag ctg cgt act ttg ttc cag      336
   Asn Asp Asp Phe Ala Ala Glu Val Arg Lys Leu Arg Thr Leu Phe Gln
               100                 105                 110
   tct ctg ggt tac tac gat tcc tcc aag gca tac tac gcc ttc aag gtc      384
   Ser Leu Gly Tyr Tyr Asp Ser Ser Lys Ala Tyr Tyr Ala Phe Lys Val
           115                 120                 125
   tca ttc aac ctc tgc atc tgg ggc ctg tcg acg ttc att gtt gcc aag      432
   Ser Phe Asn Leu Cys Ile Trp Gly Leu Ser Thr Phe Ile Val Ala Lys
       130                 135                 140
   tgg ggc cag acc tcg acc ctc gcc aac gta ctc tcg gct gcg ctc ttg      480
   Trp Gly Gln Thr Ser Thr Leu Ala Asn Val Leu Ser Ala Ala Leu Leu
   145                 150                 155                 160
   ggt ctc ttc tgg cag cag tgt gga tgg ttg gcg cac gac ttt ttg cac      528
   Gly Leu Phe Trp Gln Gln Cys Gly Trp Leu Ala His Asp Phe Leu His
                   165                 170                 175
   cac cag gtc ttc cag gac cgt ttc tgg ggt gat ctt ttc ggc gcc ttc      576
   His Gln Val Phe Gln Asp Arg Phe Trp Gly Asp Leu Phe Gly Ala Phe
               180                 185                 190
   ttg gga ggt gtc tgc cag ggc ttc tcg tcc tca tgg tgg aag gac aag      624
   Leu Gly Gly Val Cys Gln Gly Phe Ser Ser Ser Trp Trp Lys Asp Lys
           195                 200                 205
   cac aac act cac cac gcc gcc ccc aac gtc cac ggc gag gat ccc gac      672
   His Asn Thr His His Ala Ala Pro Asn Val His Gly Glu Asp Pro Asp
       210                 215                 220
   att gac act cac cct ctg ttg acg tgg agt gag cat gct ttg gag atg      720
   Ile Asp Thr His Pro Leu Leu Thr Trp Ser Glu His Ala Leu Glu Met
   225                 230                 235                 240
   ttc tcg gac gtc cct gac gag gag ctg acc cgc atg tgg tcg cgc ttc      768
   Phe Ser Asp Val Pro Asp Glu Glu Leu Thr Arg Met Trp Ser Arg Phe
                   245                 250                 255
   atg gtc ctt aac cag acc tgg ttc tac ttt ccc att ctc tcg ttt gcc      816
   Met Val Leu Asn Gln Thr Trp Phe Tyr Phe Pro Ile Leu Ser Phe Ala
               260                 265                 270
   cgt ctc tcc tgg tgc ctc cag tcc atc ctc ctt gtt ctg cct aac ggt      864
   Arg Leu Ser Trp Cys Leu Gln Ser Ile Leu Leu Val Leu Pro Asn Gly
           275                 280                 285
   cag gcc cac aag ccc tct gga gcc cgt gtg tcc att tcc ttg gtc gag      912
   Gln Ala His Lys Pro Ser Gly Ala Arg Val Ser Ile Ser Leu Val Glu
       290                 295                 300
   cag ctg tct ctt gcc atg cac tgg acc tgg tac ctc gcc acc atg ttc      960
   Gln Leu Ser Leu Ala Met His Trp Thr Trp Tyr Leu Ala Thr Met Phe
   305                 310                 315                 320
   ttg ttc att aag gac ccc gtc aac atg atg gtg tac ttt ttg gtg tct      1008
   Leu Phe Ile Lys Asp Pro Val Asn Met Met Val Tyr Phe Leu Val Ser
                   325                 330                 335
   cag gct gtt tgc ggt aac ctg ttg gcg att gtg ttc tcg ctc aac cac      1056
   Gln Ala Val Cys Gly Asn Leu Leu Ala Ile Val Phe Ser Leu Asn His
               340                 345                 350
   aac ggt atg cct gtg atc tcc aag gag gaa gcc gtc gac atg gat ttc      1104
   Asn Gly Met Pro Val Ile Ser Lys Glu Glu Ala Val Asp Met Asp Phe
           355                 360                 365
   ttc acc aag cag atc atc acg ggt cgt gat gtt cac cct ggt ctg ttc      1152
   Phe Thr Lys Gln Ile Ile Thr Gly Arg Asp Val His Pro Gly Leu Phe
       370                 375                 380
   gcc aat tgg ttc acg ggt gga ttg aac tac cag att gag cac cac ttg      1200
   Ala Asn Trp Phe Thr Gly Gly Leu Asn Tyr Gln Ile Glu His His Leu
   385                 390                 395                 400
   ttc cct tcg atg cct cgc cac aac ttt tca aag atc cag cct gct gtc      1248
   Phe Pro Ser Met Pro Arg His Asn Phe Ser Lys Ile Gln Pro Ala Val
                   405                 410                 415
   gag acc ttg tgc aaa aag tat ggt gtc cga tac cac acc act ggc atg      1296
   Glu Thr Leu Cys Lys Lys Tyr Gly Val Arg Tyr His Thr Thr Gly Met
               420                 425                 430
   atc gag gga act gca gag gtc ttt agc cgt ttg aac gag gtc tcc aag      1344
   Ile Glu Gly Thr Ala Glu Val Phe Ser Arg Leu Asn Glu Val Ser Lys
           435                 440                 445
   gcc gcc tcc aag atg ggt aag gcg cag taa                              1374
   Ala Ala Ser Lys Met Gly Lys Ala Gln
       450                 455
<210> 34
   <211> 457
   <212> PRT
   <213> Mortierella isabellina
<400> 34
   Met Ala Ala Ala Pro Ser Val Arg Thr Phe Thr Arg Ala Glu Ile Leu
   1               5                   10                  15
   Asn Ala Glu Ala Leu Asn Glu Gly Lys Lys Asp Ala Glu Ala Pro Phe
               20                  25                  30
   Leu Met Ile Ile Asp Asn Lys Val Tyr Asp Val Arg Glu Phe Val Pro
           35                  40                  45
   Asp His Pro Gly Gly Ser Val Ile Leu Thr His Val Gly Lys Asp Gly
       50                  55                  60
   Thr Asp Val Phe Asp Thr Phe His Pro Glu Ala Ala Trp Glu Thr Leu
   65                  70                  75                  80
   Ala Asn Phe Tyr Val Gly Asp Ile Asp Glu Ser Asp Arg Ala Ile Lys
                   85                  90                  95
   Asn Asp Asp Phe Ala Ala Glu Val Arg Lys Leu Arg Thr Leu Phe Gln
               100                 105                 110
   Ser Leu Gly Tyr Tyr Asp Ser Ser Lys Ala Tyr Tyr Ala Phe Lys Val
           115                 120                 125
   Ser Phe Asn Leu Cys Ile Trp Gly Leu Ser Thr Phe Ile Val Ala Lys
       130                 135                 140
   Trp Gly Gln Thr Ser Thr Leu Ala Asn Val Leu Ser Ala Ala Leu Leu
   145                 150                 155                 160
   Gly Leu Phe Trp Gln Gln Cys Gly Trp Leu Ala His Asp Phe Leu His
                   165                 170                 175
   His Gln Val Phe Gln Asp Arg Phe Trp Gly Asp Leu Phe Gly Ala Phe
               180                 185                 190
   Leu Gly Gly Val Cys Gln Gly Phe Ser Ser Ser Trp Trp Lys Asp Lys
           195                 200                 205
   His Asn Thr His His Ala Ala Pro Asn Val His Gly Glu Asp Pro Asp
       210                 215                 220
   Ile Asp Thr His Pro Leu Leu Thr Trp Ser Glu His Ala Leu Glu Met
   225                 230                 235                 240
   Phe Ser Asp Val Pro Asp Glu Glu Leu Thr Arg Met Trp Ser Arg Phe
                   245                 250                 255
   Met Val Leu Asn Gln Thr Trp Phe Tyr Phe Pro Ile Leu Ser Phe Ala
               260                 265                 270
   Arg Leu Ser Trp Cys Leu Gln Ser Ile Leu Leu Val Leu Pro Asn Gly
           275                 280                 285
   Gln Ala His Lys Pro Ser Gly Ala Arg Val Ser Ile Ser Leu Val Glu
       290                 295                 300
   Gln Leu Ser Leu Ala Met His Trp Thr Trp Tyr Leu Ala Thr Met Phe
   305                 310                 315                 320
   Leu Phe Ile Lys Asp Pro Val Asn Met Met Val Tyr Phe Leu Val Ser
                   325                 330                 335
   Gln Ala Val Cys Gly Asn Leu Leu Ala Ile Val Phe Ser Leu Asn His
               340                 345                 350
   Asn Gly Met Pro Val Ile Ser Lys Glu Glu Ala Val Asp Met Asp Phe
           355                 360                 365
   Phe Thr Lys Gln Ile Ile Thr Gly Arg Asp Val His Pro Gly Leu Phe
       370                 375                 380
   Ala Asn Trp Phe Thr Gly Gly Leu Asn Tyr Gln Ile Glu His His Leu
   385                 390                 395                 400
   Phe Pro Ser Met Pro Arg His Asn Phe Ser Lys Ile Gln Pro Ala Val
                   405                 410                 415
   Glu Thr Leu Cys Lys Lys Tyr Gly Val Arg Tyr His Thr Thr Gly Met
               420                 425                 430
   Ile Glu Gly Thr Ala Glu Val Phe Ser Arg Leu Asn Glu Val Ser Lys
           435                 440                 445
   Ala Ala Ser Lys Met Gly Lys Ala Gln
       450                 455
<210> 35
   <211> 1563
   <212> DNA
   <213> Ceratodon purpureus
<220>
   <221> CDS
   <222> (1)..(1563)
<400> 35
   atg gtg tcc cag ggc ggc ggt ctc tcg cag ggt tcc att gaa gaa aac        48
   Met Val Ser Gln Gly Gly Gly Leu Ser Gln Gly Ser Ile Glu Glu Asn
   1               5                   10                  15
   att gac gtt gag cac ttg gca acg atg ccc ctc gtc agt gac ttc cta        96
   Ile Asp Val Glu His Leu Ala Thr Met Pro Leu Val Ser Asp Phe Leu
               20                  25                  30
   aat gtc ctg gga acg act ttg ggc cag tgg agt ctt tcc act aca ttc        144
   Asn Val Leu Gly Thr Thr Leu Gly Gln Trp Ser Leu Ser Thr Thr Phe
           35                  40                  45
   gct ttc aag agg ctc acg act aag aaa cac agt tcg gac atc tcg gtg        192
   Ala Phe Lys Arg Leu Thr Thr Lys Lys His Ser Ser Asp Ile Ser Val
       50                  55                  60
   gag gca caa aaa gaa tcg gtt gcg cgg ggg cca gtt gag aat att tct        240
   Glu Ala Gln Lys Glu Ser Val Ala Arg Gly Pro Val Glu Asn Ile Ser
   65                  70                  75                  80
   caa tcg gtt gcg cag ccc atc agg cgg agg tgg gtg cag gat aaa aag        288
   Gln Ser Val Ala Gln Pro Ile Arg Arg Arg Trp Val Gln Asp Lys Lys
                   85                  90                  95
   ccg gtt act tac agc ctg aag gat gta gct tcg cac gat atg ccc cag      336
   Pro Val Thr Tyr Ser Leu Lys Asp Val Ala Ser His Asp Met Pro Gln
               100                 105                 110
   gac tgc tgg att ata atc aaa gag aag gtg tat gat gtg agc acc ttc      384
   Asp Cys Trp Ile Ile Ile Lys Glu Lys Val Tyr Asp Val Ser Thr Phe
           115                 120                 125
   gct gag cag cac cct gga ggc acg gtt atc aac acc tac ttc gga cga      432
   Ala Glu Gln His Pro Gly Gly Thr Val Ile Asn Thr Tyr Phe Gly Arg
       130                 135                 140
   gac gcc aca gat gtt ttc tct act ttc cac gca tcc acc tca tgg aag      480
   Asp Ala Thr Asp Val Phe Ser Thr Phe His Ala Ser Thr Ser Trp Lys
   145                 150                 155                 160
   att ctt cag aat ttc tac atc ggg aac ctt gtt agg gag gag ccg act      528
   Ile Leu Gln Asn Phe Tyr Ile Gly Asn Leu Val Arg Glu Glu Pro Thr
                   165                 170                 175
   ttg gag ctg ctg aag gag tac aga gag ttg aga gcc ctt ttc ttg aga      576
   Leu Glu Leu Leu Lys Glu Tyr Arg Glu Leu Arg Ala Leu Phe Leu Arg
               180                 185                 190
   gaa cag ctt ttc aag agt tcc aaa tcc tac tac ctt ttc aag act ctc      624
   Glu Gln Leu Phe Lys Ser Ser Lys Ser Tyr Tyr Leu Phe Lys Thr Leu
           195                 200                 205
   ata aat gtt tcc att gtt gcc aca agc att gcg ata atc agt ctg tac      672
   Ile Asn Val Ser Ile Val Ala Thr Ser Ile Ala Ile Ile Ser Leu Tyr
       210                 215                 220
   aag tct tac cgg gcg gtt ctg tta tca gcc agt ttg atg ggc ttg ttt      720
   Lys Ser Tyr Arg Ala Val Leu Leu Ser Ala Ser Leu Met Gly Leu Phe
   225                 230                 235                 240
   att caa cag tgc gga tgg ttg tct cac gat ttt cta cac cat cag gta      768
   Ile Gln Gln Cys Gly Trp Leu Ser His Asp Phe Leu His His Gln Val
                   245                 250                 255
   ttt gag aca cgc tgg ctc aat gac gtt gtt ggc tat gtg gtc ggc aac      816
   Phe Glu Thr Arg Trp Leu Asn Asp Val Val Gly Tyr Val Val Gly Asn
               260                 265                 270
   gtt gtt ctg gga ttc agt gtc tcg tgg tgg aag acc aag cac aac ctg      864
   Val Val Leu Gly Phe Ser Val Ser Trp Trp Lys Thr Lys His Asn Leu
           275                 280                 285
   cat cat gct gct ccg aat gaa tgc gac caa aag tac aca ccg att gat      912
   His His Ala Ala Pro Asn Glu Cys Asp Gln Lys Tyr Thr Pro Ile Asp
       290                 295                 300
   gag gat att gat act ctc ccc atc att gct tgg agt aaa gat ctc ttg      960
   Glu Asp Ile Asp Thr Leu Pro Ile Ile Ala Trp Ser Lys Asp Leu Leu
   305                 310                 315                 320
   gcc act gtt gag agc aag acc atg ttg cga gtt ctt cag tac cag cac      1008
   Ala Thr Val Glu Ser Lys Thr Met Leu Arg Val Leu Gln Tyr Gln His
                   325                 330                 335
   cta ttc ttt ttg gtt ctt ttg acg ttt gcc cgg gcg agt tgg cta ttt      1056
   Leu Phe Phe Leu Val Leu Leu Thr Phe Ala Arg Ala Ser Trp Leu Phe
               340                 345                 350
   tgg agc gcg gcc ttc act ctc agg ccc gag ttg acc ctt ggc gag aag      1104
   Trp Ser Ala Ala Phe Thr Leu Arg Pro Glu Leu Thr Leu Gly Glu Lys
           355                 360                 365
   ctt ttg gag agg gga acg atg gct ttg cac tac att tgg ttt aat agt      1152
   Leu Leu Glu Arg Gly Thr Met Ala Leu His Tyr Ile Trp Phe Asn Ser
       370                 375                 380
   gtt gcg ttt tat ctg ctc ccc gga tgg aaa cca gtt gta tgg atg gtg      1200
   Val Ala Phe Tyr Leu Leu Pro Gly Trp Lys Pro Val Val Trp Met Val
   385                 390                 395                 400
   gtc agc gag ctc atg tct ggt ttc ctg ctg gga tac gta ttt gta ctc      1248
   Val Ser Glu Leu Met Ser Gly Phe Leu Leu Gly Tyr Val Phe Val Leu
                   405                 410                 415
   agt cac aat gga atg gag gtg tac aat acg tca aag gac ttc gtg aat      1296
   Ser His Asn Gly Met Glu Val Tyr Asn Thr Ser Lys Asp Phe Val Asn
               420                 425                 430
   gcc cag att gca tcg act cgc gac atc aaa gca ggg gtg ttt aat gat      1344
   Ala Gln Ile Ala Ser Thr Arg Asp Ile Lys Ala Gly Val Phe Asn Asp
           435                 440                 445
   tgg ttc acc gga ggt ctc aac aga cag att gag cat cat cta ttt cca      1392
   Trp Phe Thr Gly Gly Leu Asn Arg Gln Ile Glu His His Leu Phe Pro
       450                 455                 460
   acg atg ccc agg cac aac ctt aat aaa att tct cct cac gtg gag act      1440
   Thr Met Pro Arg His Asn Leu Asn Lys Ile Ser Pro His Val Glu Thr
   465                 470                 475                 480
   ttg tgc aag aag cat gga ctg gtc tac gaa gac gtg agc atg gct tcg      1488
   Leu Cys Lys Lys His Gly Leu Val Tyr Glu Asp Val Ser Met Ala Ser
                   485                 490                 495
   ggc act tac cgg gtt ttg aaa aca ctt aag gac gtt gcc gat gct gct      1536
   Gly Thr Tyr Arg Val Leu Lys Thr Leu Lys Asp Val Ala Asp Ala Ala
               500                 505                 510
   tca cac cag cag ctt gct gcg agt tga                                  1563
   Ser His Gln Gln Leu Ala Ala Ser
           515                 520
<210> 36
   <211> 520
   <212> PRT
   <213> Ceratodon purpureus
<400> 36
   Met Val Ser Gln Gly Gly Gly Leu Ser Gln Gly Ser Ile Glu Glu Asn
   1 5 10 15
   Ile Asp Val Glu His Leu Ala Thr Met Pro Leu Val Ser Asp Phe Leu
   20 25 30
   Asn Val Leu Gly Thr Thr Leu Gly Gln Trp Ser Leu Ser Thr Thr Phe
   35 40 45
   Ala Phe Lys Arg Leu Thr Thr Lys Lys His Ser Ser Asp Ile Ser Val
   50 55 60
   Glu Ala Gln Lys Glu Ser Val Ala Arg Gly Pro Val Glu Asn Ile Ser
   65 70 75 80
   Gln Ser Val Ala Gln Pro Ile Arg Arg Arg Trp Val Gln Asp Lys Lys
   85 90 95
   Pro Val Thr Tyr Ser Leu Lys Asp Val Ala Ser His Asp Met Pro Gln
   100 105 110
   Asp Cys Trp Ile Ile Ile Lys Glu Lys Val Tyr Asp Val Ser Thr Phe
   115 120 125
   Ala Glu Gln His Pro Gly Gly Thr Val Ile Asn Thr Tyr Phe Gly Arg
   130 135 140
   Asp Ala Thr Asp Val Phe Ser Thr Phe His Ala Ser Thr Ser Trp Lys
   145 150 155 160
   Ile Leu Gln Asn Phe Tyr Ile Gly Asn Leu Val Arg Glu Glu Pro Thr
   165 170 175
   Leu Glu Leu Leu Lys Glu Tyr Arg Glu Leu Arg Ala Leu Phe Leu Arg
   180 185 190
   Glu Gln Leu Phe Lys Ser Ser Lys Ser Tyr Tyr Leu Phe Lys Thr Leu
   195 200 205
   Ile Asn Val Ser Ile Val Ala Thr Ser Ile Ala Ile Ile Ser Leu Tyr
   210 215 220
   Lys Ser Tyr Arg Ala Val Leu Leu Ser Ala Ser Leu Met Gly Leu Phe
   225 230 235 240
   Ile Gln Gln Cys Gly Trp Leu Ser His Asp Phe Leu His His Gln Val
   245 250 255
   Phe Glu Thr Arg Trp Leu Asn Asp Val Val Gly Tyr Val Val Gly Asn
   260 265 270
   Val Val Leu Gly Phe Ser Val Ser Trp Trp Lys Thr Lys His Asn Leu
   275 280 285
   His His Ala Ala Pro Asn Glu Cys Asp Gln Lys Tyr Thr Pro Ile Asp
   290 295 300
   Glu Asp Ile Asp Thr Leu Pro Ile Ile Ala Trp Ser Lys Asp Leu Leu
   305 310 315 320
   Ala Thr Val Glu Ser Lys Thr Met Leu Arg Val Leu Gln Tyr Gln His
   325 330 335
   Leu Phe Phe Leu Val Leu Leu Thr Phe Ala Arg Ala Ser Trp Leu Phe
   340 345 350
   Trp Ser Ala Ala Phe Thr Leu Arg Pro Glu Leu Thr Leu Gly Glu Lys
   355 360 365
   Leu Leu Glu Arg Gly Thr Met Ala Leu His Tyr Ile Trp Phe Asn Ser
       370                 375                 380
   Val Ala Phe Tyr Leu Leu Pro Gly Trp Lys Pro Val Val Trp Met Val
   385                 390                 395                 400
   Val Ser Glu Leu Met Ser Gly Phe Leu Leu Gly Tyr Val Phe Val Leu
                   405                 410                 415
   Ser His Asn Gly Met Glu Val Tyr Asn Thr Ser Lys Asp Phe Val Asn
               420                 425                 430
   Ala Gln Ile Ala Ser Thr Arg Asp Ile Lys Ala Gly Val Phe Asn Asp
           435                 440                 445
   Trp Phe Thr Gly Gly Leu Asn Arg Gln Ile Glu His His Leu Phe Pro
       450                 455                 460
   Thr Met Pro Arg His Asn Leu Asn Lys Ile Ser Pro His Val Glu Thr
   465                 470                 475                 480
   Leu Cys Lys Lys His Gly Leu Val Tyr Glu Asp Val Ser Met Ala Ser
                   485                 490                 495
   Gly Thr Tyr Arg Val Leu Lys Thr Leu Lys Asp Val Ala Asp Ala Ala
               500                 505                 510
   Ser His Gln Gln Leu Ala Ala Ser
           515                 520
<210> 37
   <211> 1452
   <212> DNA
   <213> Ceratodon purpureus
<220>
   <221> CDS
   <222> (1)..(1452)
<400> 37
   atg gcc ctc gtt acc gac ttt ctg aac ttt ctg ggc acg aca tgg agc      48
   Met Ala Leu Val Thr Asp Phe Leu Asn Phe Leu Gly Thr Thr Trp Ser
   1               5                   10                  15
   aag tac agc gtg tac acc cat agc tat gct gga aac tat ggg cct act      96
   Lys Tyr Ser Val Tyr Thr His Ser Tyr Ala Gly Asn Tyr Gly Pro Thr
               20                  25                  30
   ttg aag cac gcc aaa aag gtt tct gct caa ggt aaa act gcg gga cag     144
   Leu Lys His Ala Lys Lys Val Ser Ala Gln Gly Lys Thr Ala Gly Gln
           35                  40                  45
   aca ctg aga cag aga tcg gtg cag gac aaa aag cca ggc act tac tct     192
   Thr Leu Arg Gln Arg Ser Val Gln Asp Lys Lys Pro Gly Thr Tyr Ser
       50                  55                  60
   ctg gcc gat gtt gct tct cac gac agg cct gga gac tgc tgg atg atc     240
   Leu Ala Asp Val Ala Ser His Asp Arg Pro Gly Asp Cys Trp Met Ile
   65                  70                  75                  80
   gtc aaa gag aag gtg tat gat att agc cgt ttt gcg gac gac cac cct     288
   Val Lys Glu Lys Val Tyr Asp Ile Ser Arg Phe Ala Asp Asp His Pro
                   85                  90                  95
   gga ggg acg gta att agc acc tac ttt ggg cgg gat ggc aca gac gtt     336
   Gly Gly Thr Val Ile Ser Thr Tyr Phe Gly Arg Asp Gly Thr Asp Val
               100                 105                 110
   ttc gca aca ttc cat cca cct gcc gca tgg aag caa ctc aat gac tac     384
   Phe Ala Thr Phe His Pro Pro Ala Ala Trp Lys Gln Leu Asn Asp Tyr
           115                 120                 125
   tac att gga gac ctt gct agg gaa gag ccc ctt gat gaa ttg ctt aaa     432
   Tyr Ile Gly Asp Leu Ala Arg Glu Glu Pro Leu Asp Glu Leu Leu Lys
       130                 135                 140
   gac tac aga gat atg aga gcc gag ttt gtt aga gaa ggg ctt ttc aag     480
   Asp Tyr Arg Asp Met Arg Ala Glu Phe Val Arg Glu Gly Leu Phe Lys
   145                 150                 155                 160
   agt tcc aag gcc tgg ttc ctg ctt cag act ctg att aat gca gct ctc     528
   Ser Ser Lys Ala Trp Phe Leu Leu Gln Thr Leu Ile Asn Ala Ala Leu
                   165                 170                 175
   ttt gct gcg agc att gcg act atc tgt tac gac aag agt tac tgg gct     576
   Phe Ala Ala Ser Ile Ala Thr Ile Cys Tyr Asp Lys Ser Tyr Trp Ala
               180                 185                 190
   att gtg ctg tca gcc agt ttg atg ggt ctc ttc gtc caa cag tgt gga     624
   Ile Val Leu Ser Ala Ser Leu Met Gly Leu Phe Val Gln Gln Cys Gly
           195                 200                 205
   tgg ctt gcc cat gat ttc ctt cat caa cag gtc ttt gag aac cgt acc      672
   Trp Leu Ala His Asp Phe Leu His Gln Gln Val Phe Glu Asn Arg Thr
       210                 215                 220
   gcg aac tcc ttc ttt ggc tat ttg ttc ggc aat tgc gtg ctt ggc ttt      720
   Ala Asn Ser Phe Phe Gly Tyr Leu Phe Gly Asn Cys Val Leu Gly Phe
   225                 230                 235                 240
   agt gta tca tgg tgg agg acg aag cac aac att cat cat act gct ccg      768
   Ser Val Ser Trp Trp Arg Thr Lys His Asn Ile His His Thr Ala Pro
                   245                 250                 255
   aat gag tgc gac gaa cag tac aca cct cta gac gaa gac att gat act      816
   Asn Glu Cys Asp Glu Gln Tyr Thr Pro Leu Asp Glu Asp Ile Asp Thr
               260                 265                 270
   ctc ccc atc att gcc tgg agc aag gaa att ttg gcc acc gtt gag agc      864
   Leu Pro Ile Ile Ala Trp Ser Lys Glu Ile Leu Ala Thr Val Glu Ser
           275                 280                 285
   aag aga att ttg cga gtg ctt caa tat cag cac tac atg att ctg cct      912
   Lys Arg Ile Leu Arg Val Leu Gln Tyr Gln His Tyr Met Ile Leu Pro
       290                 295                 300
   cta ttg ttc atg gcc cgg tac agt tgg act ttt gga agt ttg ctc ttc      960
   Leu Leu Phe Met Ala Arg Tyr Ser Trp Thr Phe Gly Ser Leu Leu Phe
   305                 310                 315                 320
   aca ttc aat cct gat ttg agc acg acc aag gga ttg ata gag aag gga      1008
   Thr Phe Asn Pro Asp Leu Ser Thr Thr Lys Gly Leu Ile Glu Lys Gly
                   325                 330                 335
   aca gtt gct ttt cac tac gcc tgg ttc agt tgg gct gcg ttc cat att      1056
   Thr Val Ala Phe His Tyr Ala Trp Phe Ser Trp Ala Ala Phe His Ile
               340                 345                 350
   ttg ccg ggt gtc gct aag cct ctt gcg tgg atg gta gca act gag ctt      1104
   Leu Pro Gly Val Ala Lys Pro Leu Ala Trp Met Val Ala Thr Glu Leu
           355                 360                 365
   gtg gcc ggt ttg ttg ttg gga ttc gtg ttt acg ttg agt cac aat gga      1152
   Val Ala Gly Leu Leu Leu Gly Phe Val Phe Thr Leu Ser His Asn Gly
       370                 375                 380
   aag gag gtt tac aat gaa tcg aag gac ttc gtg aga gcc cag gtt att      1200
   Lys Glu Val Tyr Asn Glu Ser Lys Asp Phe Val Arg Ala Gln Val Ile
   385                 390                 395                 400
   acc acc cgt aac acc aag cga ggc tgg ttc aac gat tgg ttc act ggg      1248
   Thr Thr Arg Asn Thr Lys Arg Gly Trp Phe Asn Asp Trp Phe Thr Gly
                   405                 410                 415
   gga ctc gac acc cag att gag cat cac ctg ttt cca aca atg ccc agg      1296
   Gly Leu Asp Thr Gln Ile Glu His His Leu Phe Pro Thr Met Pro Arg
               420                 425                 430
   cac aac tac ccc aag atc gca cct cag gtc gag gct ctt tgc aag aag      1344
   His Asn Tyr Pro Lys Ile Ala Pro Gln Val Glu Ala Leu Cys Lys Lys
           435                 440                 445
   cac ggc ctc gag tac gat aat gtc tcc gtc gtt ggt gcc tct gtc gcg      1392
   His Gly Leu Glu Tyr Asp Asn Val Ser Val Val Gly Ala Ser Val Ala
       450                 455                 460
   gtt gtg aag gcg ctc aag gaa att gct gat gaa gcg tca att cgg ctt      1440
   Val Val Lys Ala Leu Lys Glu Ile Ala Asp Glu Ala Ser Ile Arg Leu
   465                 470                 475                 480
   cac gct cac taa                                                      1452
   His Ala His
<210> 38
   <211> 483
   <212> PRT
   <213> Ceratodon purpureus
<400> 38
   Met Ala Leu Val Thr Asp Phe Leu Asn Phe Leu Gly Thr Thr Trp Ser
   1               5                   10                  15
   Lys Tyr Ser Val Tyr Thr His Ser Tyr Ala Gly Asn Tyr Gly Pro Thr
               20                  25                  30
   Leu Lys His Ala Lys Lys Val Ser Ala Gln Gly Lys Thr Ala Gly Gln
           35                  40                  45
   Thr Leu Arg Gln Arg Ser Val Gln Asp Lys Lys Pro Gly Thr Tyr Ser
       50                  55                  60
   Leu Ala Asp Val Ala Ser His Asp Arg Pro Gly Asp Cys Trp Met Ile
   65                  70                  75                  80
   Val Lys Glu Lys Val Tyr Asp Ile Ser Arg Phe Ala Asp Asp His Pro
                   85                  90                  95
   Gly Gly Thr Val Ile Ser Thr Tyr Phe Gly Arg Asp Gly Thr Asp Val
               100                 105                 110
   Phe Ala Thr Phe His Pro Pro Ala Ala Trp Lys Gln Leu Asn Asp Tyr
           115                 120                 125
   Tyr Ile Gly Asp Leu Ala Arg Glu Glu Pro Leu Asp Glu Leu Leu Lys
       130                 135                 140
   Asp Tyr Arg Asp Met Arg Ala Glu Phe Val Arg Glu Gly Leu Phe Lys
   145                 150                 155                 160
   Ser Ser Lys Ala Trp Phe Leu Leu Gln Thr Leu Ile Asn Ala Ala Leu
                   165                 170                 175
   Phe Ala Ala Ser Ile Ala Thr Ile Cys Tyr Asp Lys Ser Tyr Trp Ala
               180                 185                 190
   Ile Val Leu Ser Ala Ser Leu Met Gly Leu Phe Val Gln Gln Cys Gly
           195                 200                 205
   Trp Leu Ala His Asp Phe Leu His Gln Gln Val Phe Glu Asn Arg Thr
       210                 215                 220
   Ala Asn Ser Phe Phe Gly Tyr Leu Phe Gly Asn Cys Val Leu Gly Phe
   225                 230                 235                 240
   Ser Val Ser Trp Trp Arg Thr Lys His Asn Ile His His Thr Ala Pro
                   245                 250                 255
   Asn Glu Cys Asp Glu Gln Tyr Thr Pro Leu Asp Glu Asp Ile Asp Thr
               260                 265                 270
   Leu Pro Ile Ile Ala Trp Ser Lys Glu Ile Leu Ala Thr Val Glu Ser
           275                 280                 285
   Lys Arg Ile Leu Arg Val Leu Gln Tyr Gln His Tyr Met Ile Leu Pro
       290                 295                 300
   Leu Leu Phe Met Ala Arg Tyr Ser Trp Thr Phe Gly Ser Leu Leu Phe
   305                 310                 315                 320
   Thr Phe Asn Pro Asp Leu Ser Thr Thr Lys Gly Leu Ile Glu Lys Gly
                   325                 330                 335
   Thr Val Ala Phe His Tyr Ala Trp Phe Ser Trp Ala Ala Phe His Ile
               340                 345                 350
   Leu Pro Gly Val Ala Lys Pro Leu Ala Trp Met Val Ala Thr Glu Leu
           355                 360                 365
   Val Ala Gly Leu Leu Leu Gly Phe Val Phe Thr Leu Ser His Asn Gly
       370                 375                 380
   Lys Glu Val Tyr Asn Glu Ser Lys Asp Phe Val Arg Ala Gln Val Ile
   385                 390                 395                 400
   Thr Thr Arg Asn Thr Lys Arg Gly Trp Phe Asn Asp Trp Phe Thr Gly
                   405                 410                 415
   Gly Leu Asp Thr Gln Ile Glu His His Leu Phe Pro Thr Met Pro Arg
               420                 425                 430
   His Asn Tyr Pro Lys Ile Ala Pro Gln Val Glu Ala Leu Cys Lys Lys
           435                 440                 445
   His Gly Leu Glu Tyr Asp Asn Val Ser Val Val Gly Ala Ser Val Ala
       450                 455                 460
   Val Val Lys Ala Leu Lys Glu Ile Ala Asp Glu Ala Ser Ile Arg Leu
   465                 470                 475                 480
   His Ala His
<210> 39
   <211> 1374
   <212> DNA
   <213> Mortierella alpina
<220>
   <221> CDS
   <222> (1)..(1374)
<400> 39
   atg gct gct gct ccc agt gtg agg acg ttt act cgg gcc gag gtt ttg      48
   Met Ala Ala Ala Pro Ser Val Arg Thr Phe Thr Arg Ala Glu Val Leu
   1               5                   10                  15
   aat gcc gag gct ctg aat gag ggc aag aag gat gcc gag gca ccc ttc      96
   Asn Ala Glu Ala Leu Asn Glu Gly Lys Lys Asp Ala Glu Ala Pro Phe
               20                  25                  30
   ttg atg atc atc gac aac aag gtg tac gat gtc cgc gag ttc gtc cct     144
   Leu Met Ile Ile Asp Asn Lys Val Tyr Asp Val Arg Glu Phe Val Pro
           35                  40                  45
   gat cat ccc ggt gga agt gtg att ctc acg cac gtt ggc aag gac ggc     192
   Asp His Pro Gly Gly Ser Val Ile Leu Thr His Val Gly Lys Asp Gly
       50                  55                  60
   act gac gtc ttt gac act ttt cac ccc gag gct gct tgg gag act ctt     240
   Thr Asp Val Phe Asp Thr Phe His Pro Glu Ala Ala Trp Glu Thr Leu
   65                  70                  75                  80
   gcc aac ttt tac gtt ggt gat att gac gag agc gac cgc gat atc aag     288
   Ala Asn Phe Tyr Val Gly Asp Ile Asp Glu Ser Asp Arg Asp Ile Lys
                   85                  90                  95
   aat gat gac ttt gcg gcc gag gtc cgc aag ctg cgt acc ttg ttc cag     336
   Asn Asp Asp Phe Ala Ala Glu Val Arg Lys Leu Arg Thr Leu Phe Gln
               100                 105                 110
   tct ctt ggt tac tac gat tct tcc aag gca tac tac gcc ttc aag gtc     384
   Ser Leu Gly Tyr Tyr Asp Ser Ser Lys Ala Tyr Tyr Ala Phe Lys Val
           115                 120                 125
   tcg ttc aac ctc tgc atc tgg ggt ttg tcg acg gtc att gtg gcc aag     432
   Ser Phe Asn Leu Cys Ile Trp Gly Leu Ser Thr Val Ile Val Ala Lys
       130                 135                 140
   tgg ggc cag acc tcg acc ctc gcc aac gtg ctc tcg gct gcg ctt ttg     480
   Trp Gly Gln Thr Ser Thr Leu Ala Asn Val Leu Ser Ala Ala Leu Leu
   145                 150                 155                 160
   ggt ctg ttc tgg cag cag tgc gga tgg ttg gct cac gac ttt ttg cat     528
   Gly Leu Phe Trp Gln Gln Cys Gly Trp Leu Ala His Asp Phe Leu His
                   165                 170                 175
   cac cag gtc ttc cag gac cgt ttc tgg ggt gat ctt ttc ggc gcc ttc     576
   His Gln Val Phe Gln Asp Arg Phe Trp Gly Asp Leu Phe Gly Ala Phe
               180                 185                 190
   ttg gga ggt gtc tgc cag ggc ttc tcg tcc tcg tgg tgg aag gac aag     624
   Leu Gly Gly Val Cys Gln Gly Phe Ser Ser Ser Trp Trp Lys Asp Lys
           195                 200                 205
   cac aac act cac cac gcc gcc ccc aac gtc cac ggc gag gat ccc gac     672
   His Asn Thr His His Ala Ala Pro Asn Val His Gly Glu Asp Pro Asp
       210                 215                 220
   att gac acc cac cct ctg ttg acc tgg agt gag cat gcg ttg gag atg     720
   Ile Asp Thr His Pro Leu Leu Thr Trp Ser Glu His Ala Leu Glu Met
   225                 230                 235                 240
   ttc tcg gat gtc cca gat gag gag ctg acc cgc atg tgg tcg cgt ttc     768
   Phe Ser Asp Val Pro Asp Glu Glu Leu Thr Arg Met Trp Ser Arg Phe
                   245                 250                 255
   atg gtc ctg aac cag acc tgg ttt tac ttc ccc att ctc tcg ttt gcc     816
   Met Val Leu Asn Gln Thr Trp Phe Tyr Phe Pro Ile Leu Ser Phe Ala
               260                 265                 270
   cgt ctc tcc tgg tgc ctc cag tcc att ctc ttt gtg ctg cct aac ggt     864
   Arg Leu Ser Trp Cys Leu Gln Ser Ile Leu Phe Val Leu Pro Asn Gly
           275                 280                 285
   cag gcc cac aag ccc tcg ggc gcg cgt gtg ccc atc tcg ttg gtc gag     912
   Gln Ala His Lys Pro Ser Gly Ala Arg Val Pro Ile Ser Leu Val Glu
       290                 295                 300
   cag ctg tcg ctt gcg atg cac tgg acc tgg tac ctc gcc acc atg ttc     960
   Gln Leu Ser Leu Ala Met His Trp Thr Trp Tyr Leu Ala Thr Met Phe
   305                 310                 315                 320
   ctg ttc atc aag gat ccc gtc aac atg ctg gtg tac ttt ttg gtg tcg    1008
   Leu Phe Ile Lys Asp Pro Val Asn Met Leu Val Tyr Phe Leu Val Ser
                   325                 330                 335
   cag gcg gtg tgc gga aac ttg ttg gcg atc gtg ttc tcg ctc aac cac    1056
   Gln Ala Val Cys Gly Asn Leu Leu Ala Ile Val Phe Ser Leu Asn His
               340                 345                 350
   aac ggt atg cct gtg atc tcg aag gag gag gcg gtc gat atg gat ttc    1104
   Asn Gly Met Pro Val Ile Ser Lys Glu Glu Ala Val Asp Met Asp Phe
           355                 360                 365
   ttc acg aag cag atc atc acg ggt cgt gat gtc cac ccg ggt cta ttt      1152
   Phe Thr Lys Gln Ile Ile Thr Gly Arg Asp Val His Pro Gly Leu Phe
       370                 375                 380
   gcc aac tgg ttc acg ggt gga ttg aac tat cag atc gag cac cac ttg      1200
   Ala Asn Trp Phe Thr Gly Gly Leu Asn Tyr Gln Ile Glu His His Leu
   385                 390                 395                 400
   ttc cct tcg atg cct cgc cac aac ttt tca aag atc cag cct gct gtc      1248
   Phe Pro Ser Met Pro Arg His Asn Phe Ser Lys Ile Gln Pro Ala Val
                   405                 410                 415
   gag acc ctg tgc aaa aag tac aat gtc cga tac cac acc acc ggt atg      1296
   Glu Thr Leu Cys Lys Lys Tyr Asn Val Arg Tyr His Thr Thr Gly Met
               420                 425                 430
   atc gag gga act gca gag gtc ttt agc cgt ctg aac gag gtc tcc aag      1344
   Ile Glu Gly Thr Ala Glu Val Phe Ser Arg Leu Asn Glu Val Ser Lys
           435                 440                 445
   gct gcc tcc aag atg ggt aag gcg cag taa                              1374
   Ala Ala Ser Lys Met Gly Lys Ala Gln
       450                 455
<210> 40
   <211> 457
   <212> PRT
   <213> Mortierella alpina
<400> 40
   Met Ala Ala Ala Pro Ser Val Arg Thr Phe Thr Arg Ala Glu Val Leu
   1               5                   10                  15
   Asn Ala Glu Ala Leu Asn Glu Gly Lys Lys Asp Ala Glu Ala Pro Phe
               20                  25                  30
   Leu Met Ile Ile Asp Asn Lys Val Tyr Asp Val Arg Glu Phe Val Pro
           35                  40                  45
   Asp His Pro Gly Gly Ser Val Ile Leu Thr His Val Gly Lys Asp Gly
       50                  55                  60
   Thr Asp Val Phe Asp Thr Phe His Pro Glu Ala Ala Trp Glu Thr Leu
   65                  70                  75                  80
   Ala Asn Phe Tyr Val Gly Asp Ile Asp Glu Ser Asp Arg Asp Ile Lys
                   85                  90                  95
   Asn Asp Asp Phe Ala Ala Glu Val Arg Lys Leu Arg Thr Leu Phe Gln
               100                 105                 110
   Ser Leu Gly Tyr Tyr Asp Ser Ser Lys Ala Tyr Tyr Ala Phe Lys Val
           115                 120                 125
   Ser Phe Asn Leu Cys Ile Trp Gly Leu Ser Thr Val Ile Val Ala Lys
       130                 135                 140
   Trp Gly Gln Thr Ser Thr Leu Ala Asn Val Leu Ser Ala Ala Leu Leu
   145                 150                 155                 160
   Gly Leu Phe Trp Gln Gln Cys Gly Trp Leu Ala His Asp Phe Leu His
                   165                 170                 175
   His Gln Val Phe Gln Asp Arg Phe Trp Gly Asp Leu Phe Gly Ala Phe
               180                 185                 190
   Leu Gly Gly Val Cys Gln Gly Phe Ser Ser Ser Trp Trp Lys Asp Lys
           195                 200                 205
   His Asn Thr His His Ala Ala Pro Asn Val His Gly Glu Asp Pro Asp
       210                 215                 220
   Ile Asp Thr His Pro Leu Leu Thr Trp Ser Glu His Ala Leu Glu Met
   225                 230                 235                 240
   Phe Ser Asp Val Pro Asp Glu Glu Leu Thr Arg Met Trp Ser Arg Phe
                   245                 250                 255
   Met Val Leu Asn Gln Thr Trp Phe Tyr Phe Pro Ile Leu Ser Phe Ala
               260                 265                 270
   Arg Leu Ser Trp Cys Leu Gln Ser Ile Leu Phe Val Leu Pro Asn Gly
           275                 280                 285
   Gln Ala His Lys Pro Ser Gly Ala Arg Val Pro Ile Ser Leu Val Glu
       290                 295                 300
   Gln Leu Ser Leu Ala Met His Trp Thr Trp Tyr Leu Ala Thr Met Phe
   305                 310                 315                 320
   Leu Phe Ile Lys Asp Pro Val Asn Met Leu Val Tyr Phe Leu Val Ser
                   325                 330                 335
   Gln Ala Val Cys Gly Asn Leu Leu Ala Ile Val Phe Ser Leu Asn His
               340                 345                 350
   Asn Gly Met Pro Val Ile Ser Lys Glu Glu Ala Val Asp Met Asp Phe
           355                 360                 365
   Phe Thr Lys Gln Ile Ile Thr Gly Arg Asp Val His Pro Gly Leu Phe
       370                 375                 380
   Ala Asn Trp Phe Thr Gly Gly Leu Asn Tyr Gln Ile Glu His His Leu
   385                 390                 395                 400
   Phe Pro Ser Met Pro Arg His Asn Phe Ser Lys Ile Gln Pro Ala Val
                   405                 410                 415
   Glu Thr Leu Cys Lys Lys Tyr Asn Val Arg Tyr His Thr Thr Gly Met
               420                 425                 430
   Ile Glu Gly Thr Ala Glu Val Phe Ser Arg Leu Asn Glu Val Ser Lys
           435                 440                 445
   Ala Ala Ser Lys Met Gly Lys Ala Gln
       450                 455
<210> 41
   <211> 1374
   <212> DNA
   <213> Mortierella alpina
<220>
   <221> CDS
   <222> (1)..(1374)
<400> 41
   atg gct gct gct ccc agt gtg agg acg ttt act cgg gcc gag att ttg      48
   Met Ala Ala Ala Pro Ser Val Arg Thr Phe Thr Arg Ala Glu Ile Leu
   1               5                   10                  15
   aat gcc gag gcc ctg aat gag ggc aag aag gat gcc gag gca ccc ttt      96
   Asn Ala Glu Ala Leu Asn Glu Gly Lys Lys Asp Ala Glu Ala Pro Phe
               20                  25                  30
   ctg atg atc att gac aac aag gtg tac gat gtc cgc gag ttt gtc cct      144
   Leu Met Ile Ile Asp Asn Lys Val Tyr Asp Val Arg Glu Phe Val Pro
           35                  40                  45
   gat cat ccc ggt gga agt gtg att ctc acg cac gtt ggc aag gac ggc      192
   Asp His Pro Gly Gly Ser Val Ile Leu Thr His Val Gly Lys Asp Gly
       50                  55                  60
   act gac gtc ttt gac act ttc cac ccc gag gct gct tgg gag act ctt      240
   Thr Asp Val Phe Asp Thr Phe His Pro Glu Ala Ala Trp Glu Thr Leu
   65                  70                  75                  80
   gcc aac ttt tac gtt ggt gat att gat gag agc gat cgt gcc atc aag      288
   Ala Asn Phe Tyr Val Gly Asp Ile Asp Glu Ser Asp Arg Ala Ile Lys
                   85                  90                  95
   aat gat gac ttt gcg gcc gag gtt cgc aag ctg cgc acc ttg ttc cag      336
   Asn Asp Asp Phe Ala Ala Glu Val Arg Lys Leu Arg Thr Leu Phe Gln
               100                 105                 110
   tcc ctt ggc tac tac gac tcg tcc aag gca tac tat gcc ttc aag gtc      384
   Ser Leu Gly Tyr Tyr Asp Ser Ser Lys Ala Tyr Tyr Ala Phe Lys Val
           115                 120                 125
   tcg ttc aac ctc tgc atc tgg ggc ttg tcg act ttc att gtt gcc aag      432
   Ser Phe Asn Leu Cys Ile Trp Gly Leu Ser Thr Phe Ile Val Ala Lys
       130                 135                 140
   tgg ggc cag acc tcg acc ctc gcc aac gtg ctc tcg gct gcg ctc ttg      480
   Trp Gly Gln Thr Ser Thr Leu Ala Asn Val Leu Ser Ala Ala Leu Leu
   145                 150                 155                 160
   ggt ctc ttc tgg cag cag tgc gga tgg ttg gcg cac gac ttt ttg cac      528
   Gly Leu Phe Trp Gln Gln Cys Gly Trp Leu Ala His Asp Phe Leu His
                   165                 170                 175
   cac cag gtc ttc cag gac cgt ttc tgg ggt gat ctt ttc ggc gcc ttc      576
   His Gln Val Phe Gln Asp Arg Phe Trp Gly Asp Leu Phe Gly Ala Phe
               180                 185                 190
   ttg gga ggt gtc tgc cag ggt ttc tcg tcc tcc tgg tgg aag gac aag      624
   Leu Gly Gly Val Cys Gln Gly Phe Ser Ser Ser Trp Trp Lys Asp Lys
           195                 200                 205
   cac aac act cac cac gct gct ccc aac gtc cac ggc gag gat ccc gac      672
   His Asn Thr His His Ala Ala Pro Asn Val His Gly Glu Asp Pro Asp
       210                 215                 220
   att gac act cac cct ctg ttg acc tgg agt gag cat gct ctg gag atg      720
   Ile Asp Thr His Pro Leu Leu Thr Trp Ser Glu His Ala Leu Glu Met
   225                 230                 235                 240
   ttc tcg gat gtt cct gac gag gag ctg acc cgt atg tgg tcg cgc ttc      768
   Phe Ser Asp Val Pro Asp Glu Glu Leu Thr Arg Met Trp Ser Arg Phe
                   245                 250                 255
   atg gtc ctc aac cag acc tgg ttc tac ttc ccc att ctc tcg ttt gcc      816
   Met Val Leu Asn Gln Thr Trp Phe Tyr Phe Pro Ile Leu Ser Phe Ala
               260                 265                 270
   cgt ctg tcc tgg tgc ctc cag tcc att atg ttt gtt ctg ccc aac ggt      864
   Arg Leu Ser Trp Cys Leu Gln Ser Ile Met Phe Val Leu Pro Asn Gly
           275                 280                 285
   cag gcc cac aag ccc tct gga gcg cgt gtg ccc att tcg ttg gtc gag      912
   Gln Ala His Lys Pro Ser Gly Ala Arg Val Pro Ile Ser Leu Val Glu
       290                 295                 300
   cag ctg tct ctg gct atg cac tgg acc tgg tac ctc gcc acc atg ttc      960
   Gln Leu Ser Leu Ala Met His Trp Thr Trp Tyr Leu Ala Thr Met Phe
   305                 310                 315                 320
   ctg ttc att aag gat ccc gtc aac atg att gtg tac ttt ttg gtg tcg      1008
   Leu Phe Ile Lys Asp Pro Val Asn Met Ile Val Tyr Phe Leu Val Ser
                   325                 330                 335
   cag gct gtt tgc ggc aac ttg ttg gcg att gtg ttc tcg ctc aac cac      1056
   Gln Ala Val Cys Gly Asn Leu Leu Ala Ile Val Phe Ser Leu Asn His
               340                 345                 350
   aac ggc atg cct gtg atc tcc aag gag gaa gcg gtc gat atg gac ttc      1104
   Asn Gly Met Pro Val Ile Ser Lys Glu Glu Ala Val Asp Met Asp Phe
           355                 360                 365
   ttc acc aag cag atc atc acg ggt cgt gat gtt cac cct ggt ctg ttt      1152
   Phe Thr Lys Gln Ile Ile Thr Gly Arg Asp Val His Pro Gly Leu Phe
       370                 375                 380
   gcc aac tgg ttc acg ggt gga ttg aac tac cag att gag cac cac ttg      1200
   Ala Asn Trp Phe Thr Gly Gly Leu Asn Tyr Gln Ile Glu His His Leu
   385                 390                 395                 400
   ttc cct tcg atg ccc cgc cac aac ttt tca aag atc cag cct gct gtc      1248
   Phe Pro Ser Met Pro Arg His Asn Phe Ser Lys Ile Gln Pro Ala Val
                   405                 410                 415
   gag act ttg tgc aaa aag tac ggt gtc cga tac cat acc act ggt atg      1296
   Glu Thr Leu Cys Lys Lys Tyr Gly Val Arg Tyr His Thr Thr Gly Met
               420                 425                 430
   atc gag gga act gca gag gtc ttt agc cgt ttg aac gag gtc tcc aag      1344
   Ile Glu Gly Thr Ala Glu Val Phe Ser Arg Leu Asn Glu Val Ser Lys
           435                 440                 445
   gcg gcc tcc aag atg ggc aag gca cag taa                              1374
   Ala Ala Ser Lys Met Gly Lys Ala Gln
       450                 455
<210> 42
   <211> 457
   <212> PRT
   <213> Mortierella alpina
<400> 42
   Met Ala Ala Ala Pro Ser Val Arg Thr Phe Thr Arg Ala Glu Ile Leu
   1               5                   10                  15
   Asn Ala Glu Ala Leu Asn Glu Gly Lys Lys Asp Ala Glu Ala Pro Phe
               20                  25                  30
   Leu Met Ile Ile Asp Asn Lys Val Tyr Asp Val Arg Glu Phe Val Pro
           35                  40                  45
   Asp His Pro Gly Gly Ser Val Ile Leu Thr His Val Gly Lys Asp Gly
       50                  55                  60
   Thr Asp Val Phe Asp Thr Phe His Pro Glu Ala Ala Trp Glu Thr Leu
   65                  70                  75                  80
   Ala Asn Phe Tyr Val Gly Asp Ile Asp Glu Ser Asp Arg Ala Ile Lys
                   85                  90                  95
   Asn Asp Asp Phe Ala Ala Glu Val Arg Lys Leu Arg Thr Leu Phe Gln
               100                 105                 110
   Ser Leu Gly Tyr Tyr Asp Ser Ser Lys Ala Tyr Tyr Ala Phe Lys Val
           115                 120                 125
   Ser Phe Asn Leu Cys Ile Trp Gly Leu Ser Thr Phe Ile Val Ala Lys
       130                 135                 140
   Trp Gly Gln Thr Ser Thr Leu Ala Asn Val Leu Ser Ala Ala Leu Leu
   145                 150                 155                 160
   Gly Leu Phe Trp Gln Gln Cys Gly Trp Leu Ala His Asp Phe Leu His
                   165                 170                 175
   His Gln Val Phe Gln Asp Arg Phe Trp Gly Asp Leu Phe Gly Ala Phe
               180                 185                 190
   Leu Gly Gly Val Cys Gln Gly Phe Ser Ser Ser Trp Trp Lys Asp Lys
           195                 200                 205
   His Asn Thr His His Ala Ala Pro Asn Val His Gly Glu Asp Pro Asp
       210                 215                 220
   Ile Asp Thr His Pro Leu Leu Thr Trp Ser Glu His Ala Leu Glu Met
   225                 230                 235                 240
   Phe Ser Asp Val Pro Asp Glu Glu Leu Thr Arg Met Trp Ser Arg Phe
                   245                 250                 255
   Met Val Leu Asn Gln Thr Trp Phe Tyr Phe Pro Ile Leu Ser Phe Ala
               260                 265                 270
   Arg Leu Ser Trp Cys Leu Gln Ser Ile Met Phe Val Leu Pro Asn Gly
           275                 280                 285
   Gln Ala His Lys Pro Ser Gly Ala Arg Val Pro Ile Ser Leu Val Glu
       290                 295                 300
   Gln Leu Ser Leu Ala Met His Trp Thr Trp Tyr Leu Ala Thr Met Phe
   305                 310                 315                 320
   Leu Phe Ile Lys Asp Pro Val Asn Met Ile Val Tyr Phe Leu Val Ser
                   325                 330                 335
   Gln Ala Val Cys Gly Asn Leu Leu Ala Ile Val Phe Ser Leu Asn His
               340                 345                 350
   Asn Gly Met Pro Val Ile Ser Lys Glu Glu Ala Val Asp Met Asp Phe
           355                 360                 365
   Phe Thr Lys Gln Ile Ile Thr Gly Arg Asp Val His Pro Gly Leu Phe
       370                 375                 380
   Ala Asn Trp Phe Thr Gly Gly Leu Asn Tyr Gln Ile Glu His His Leu
   385                 390                 395                 400
   Phe Pro Ser Met Pro Arg His Asn Phe Ser Lys Ile Gln Pro Ala Val
                   405                 410                 415
   Glu Thr Leu Cys Lys Lys Tyr Gly Val Arg Tyr His Thr Thr Gly Met
               420                 425                 430
   Ile Glu Gly Thr Ala Glu Val Phe Ser Arg Leu Asn Glu Val Ser Lys
           435                 440                 445
   Ala Ala Ser Lys Met Gly Lys Ala Gln
       450                 455
<210> 43
   <211> 444
   <212> PRT
   <213> Artificial sequence
<220>
   <221> Variant
   <222> (5)..(10)
   <223> xaa in position 5 to 10 is any amino acid
<220>
   <221> Variant
   <222> (11)..(11)
   <223> xaa in position 11 is any or no amino acid
<220>
   <221> Variant
   <222> (16)..(16)
   <223> xaa in position 16 is any amino acid
<220>
   <221> Variant
   <222> (17)..(17)
   <223> xaa in position 17 is any or no amino acid
<220>
   <221> Variant
   <222> (19)..(20)
   <223> xaa in position 19 to 20 is any amino acid
<220>
   <221> Variant
   <222> (22)..(23)
   <223> xaa in position 22 to 23 is any amino acid
<220>
   <221> variant
   <222> (25)..(25)
   <223> xaa in position 25 is any amino acid
<220>
   <221> Variant
   <222> (27)..(27)
   <223> xaa in position 27 is any amino acid
<220>
   <221> Variant
   <222> (30)..(30)
   <223> xaa in position 30 is any amino acid
<220>
   <221> Variant
   <222> (32)..(34)
   <223> xaa in position 32 to 34 is any amino acid
<220>
   <221> Variant
   <222> (37)..(38)
   <223> xaa in position 37 to 38 is any amino acid
<220>
   <221> Variant
   <222> (40)..(42)
   <223> xaa in position 40 to 42 is any amino acid
<220>
   <221> Variant
   <222> (44)..(46)
   <223> xaa in position 44 to 46 is any amino acid
<220>
   <221> Variant
   <222> (48)..(48)
   <223> xaa in position 48 is any amino acid
<220>
   <221> Variant
   <222> (51)..(59)
   <223> xaa in position 51 to 59 is any amino acid
<220>
   <221> Variant
   <222> (60)..(77)
   <223> xaa in position 60 to 77 is any or no amino acid
<220>
   <221> Variant
   <222> (79)..(82)
   <223> xaa in position 79 to 82 is any amino acid
<220>
   <221> Variant
   <222> (84)..(84)
   <223> xaa in position 84 is any amino acid
<220>
   <221> Variant
   <222> (87)..(92)
   <223> xaa in position 87 to 92 is any amino acid
<220>
   <221> Variant
   <222> (94)..(97)
   <223> xaa in position 94 to 97 is any amino acid
<220>
   <221> variant
   <222> (99)..(101)
   <223> xaa in position 99 to 101 is any amino acid
<220>
   <221> variant
   <222> (103)..(104)
   <223> xaa in position 103 to 104 is any amino acid
<220>
   <221> Variant
   <222> (106)..(132)
   <223> xaa in position 106 to 132 is any amino acid
<220>
   <221> Variant
   <222> (133)..(134)
   <223> xaa in position 133 to 134 is any or no amino acid
<220>
   <221> Variant
   <222> (136)..(138)
   <223> xaa in position 136 to 138 is any amino acid
<220>
   <221> Variant
   <222> (140)..(140)
   <223> xaa in position 140 is any amino acid
<220>
   <221> Variant
   <222> (142)..(142)
   <223> xaa in position 142 is any amino acid
<220>
   <221> Variant
   <222> (145)..(145)
   <223> xaa in position 145 is any amino acid
<220>
   <221> Variant
   <222> (153)..(153)
   <223> xaa in position 153 is any amino acid
<220>
   <221> Variant
   <222> (159)..(160)
   <223> xaa in position 159 to 160 is any amino acid
<220>
   <221> Variant
   <222> (162)..(171)
   <223> xaa in position 162 to 171 is any amino acid
<220>
   <221> Variant
   <222> (172)..(172)
   <223> xaa in position 172 is any or no amino acid
<220>
   <221> Variant
   <222> (174)..(176)
   <223> xaa in position 174 to 176 is any amino acid
<220>
   <221> Variant
   <222> (181)..(182)
   <223> xaa in position 181 to 182 is any amino acid
<220>
   <221> variant
   <222> (186)..(186)
   <223> xaa in position 186 is any amino acid
<220>
   <221> Variant
   <222> (190)..(190)
   <223> xaa in position 190 is any amino acid
<220>
   <221> variant
   <222> (194)..(195)
   <223> xaa in position 194 to 195 is any amino acid
<220>
   <221> Variant
   <222> (197)..(200)
   <223> xaa in position 197 to 200 is any amino acid
<220>
   <221> Variant
   <222> (201)..(207)
   <223> xaa in position 201 to 207 is any or no amino acid
<220>
   <221> Variant
   <222> (214)..(214)
   <223> xaa in position 214 is any amino acid
<220>
   <221> Variant
   <222> (216)..(216)
   <223> xaa in position 216 is any amino acid
<220>
   <221> Variant
   <222> (218)..(218)
   <223> xaa in position 218 is any amino acid
<220>
   <221> Variant
   <222> (220)..(238)
   <223> xaa in position 220 to 238 is any amino acid
<220>
   <221> Variant
   <222> (239)..(250)
   <223> xaa in position 239 to 250 is any or no amino acid
<220>
   <221> Variant
   <222> (252)..(255)
   <223> xaa in position 252 to 255 is any amino acid
<220>
   <221> Variant
   <222> (257)..(258)
   <223> xaa in position 257 to 258 is any amino acid
<220>
   <221> Variant
   <222> (260)..(261)
   <223> xaa in position 260 to 261 is any amino acid
<220>
   <221> Variant
   <222> (264)..(264)
   <223> xaa in position 264 is any amino acid
<220>
   <221> Variant
   <222> (267)..(269)
   <223> xaa in position 267 to 269 is any amino acid
<220>
   <221> Variant
   <222> (271)..(284)
   <223> xaa in position 271 to 284 is any amino acid
<220>
   <221> Variant
   <222> (285)..(296)
   <223> xaa in position 285 to 296 is any or no amino acid
<220>
   <221> Variant
   <222> (298)..(298)
   <223> xaa in position 298 is any amino acid
<220>
   <221> variant
   <222> (300)..(305)
   <223> xaa in position 300 to 305 is any amino acid
<220>
   <221> Variant
   <222> (307)..(326)
   <223> xaa in position 307 to 326 is any amino acid
<220>
   <221> Variant
   <222> (327)..(328)
   <223> xaa in position 327 to 328 is any or no amino acid
<220>
   <221> Variant
   <222> (330)..(336)
   <223> xaa in position 330 to 336 is any amino acid
<220>
   <221> Variant
   <222> (338)..(339)
   <223> xaa in position 338 to 339 is any amino acid
<220>
   <221> Variant
   <222> (342)..(342)
   <223> xaa in position 342 is any amino acid
<220>
   <221> Variant
   <222> (345)..(347)
   <223> xaa in position 345 to 347 is any amino acid
<220>
   <221> Variant
   <222> (352)..(352)
   <223> xaa in position 352 is any amino acid
<220>
   <221> Variant
   <222> (354)..(359)
   <223> xaa in position 354 to 359 is any amino acid
<220>
   <221> Variant
   <222> (360)..(363)
   <223> xaa in position 360 to 363 is any or no amino acid
<220>
   <221> Variant
   <222> (365)..(367)
   <223> xaa in position 365 to 367 is any amino acid
<220>
   <221> Variant
   <222> (369)..(370)
   <223> xaa in position 369 to 370 is any amino acid
<220>
   <221> Variant
   <222> (372)..(372)
   <223> xaa in position 372 is any amino acid
<220>
   <221> Variant
   <222> (374)..(379)
   <223> xaa in position 374 to 379 is any amino acid
<220>
   <221> Variant
   <222> (380)..(384)
   <223> xaa in position 380 to 384 is any or no amino acid
<220>
   <221> Variant
   <222> (386)..(387)
   <223> xaa in position 386 to 387 is any amino acid
<220>
   <221> Variant
   <222> (390)..(390)
   <223> xaa in position 390 is any amino acid
<220>
   <221> Variant
   <222> (404)..(405)
   <223> xaa in position 404 to 405 is any amino acid
<220>
   <221> Variant
   <222> (410)..(411)
   <223> xaa in position 410 to 411 is any amino acid
<220>
   <221> Variant
   <222> (413)..(416)
   <223> xaa in position 413 to 416 is any amino acid
<220>
   <221> Variant
   <222> (418)..(419)
   <223> xaa in position 418 to 419 is any amino acid

<220>
   <221> variant
   <222> (423)..(427)
   <223> xaa in position 423 to 427 is any amino acid
<220>
   <221> Variant
   <222> (430)..(435)
   <223> xaa in position 430 to 435 is any amino acid
<220>
   <221> Variant
   <222> (438)..(438)
   <223> xaa in position 438 is any amino acid
<220>
   <221> Variant
   <222> (441)..(443)
   <223> xaa in position 441 to 443 is any amino acid
<400> 43
<210> 44
   <211> 31
   <212> PRT
   <213> Artificial sequence
<220>
   <221> variant
   <222> (2)..(3)
   <223> xaa in position 2 to 3 is any amino acid
<220>
   <221> Variant
   <222> (6)..(6)
   <223> xaa in position 6 is Asp or Glu
<220>
   <221> Variant
   <222> (9)..(9)
   <223> xaa in position 9 is Leu or Val
<220>
   <221> variant
   <222> (12)..(12)
   <223> xaa in position 12 is any amino acid
<220>
   <221> Variant
   <222> (13)..(13)
   <223> xaa in position 13 is Leu, Met or Val
<220>
   <221> variant
   <222> (17)..(18)
   <223> xaa in position 17 to 18 is any amino acid
<220>
   <221> Variant
   <222> (19)..(19)
   <223> xaa in position 19 is Ala, Asn, Pro or Ser
<220>
   <221> Variant
   <222> (20)..(20)
   <223> xaa in position 20 is any amino acid
<220>
   <221> Variant
   <222> (21)..(21)
   <223> xaa in position 21 is Ile or Val
<220>
   <221> Variant
   <222> (22)..(22)
   <223> xaa in position 22 is any amino acid
<220>
   <221> Variant
   <222> (23)..(23)
   <223> xaa in position 23 is Pro or Val
<220>
   <221> Variant
   <222> (24)..(24)
   <223> xaa in position 24 is any amino acid
<220>
   <221> Variant
   <222> (25)..(25)
   <223> xaa in position 25 is Ile or Val
<220>
   <221> Variant
   <222> (26)..(26)
   <223> xaa in position 26 is Glu or Lys
<220>
   <221> Variant
   <222> (27)..(27)
   <223> xaa in position 27 is Ala, Ser or Thr
<220>
   <221> Variant
   <222> (30)..(30)
   <223> xaa in position 30 is any or no amino acid
<400> 44
<210> 45
   <211> 27
   <212> PRT
   <213> Artificial sequence
<220>
   <221> Variant
   <222> (2)..(2)
   <223> xaa in position 2 is Asp, Gly or Asn
<220>
   <221> Variant
   <222> (3)..(4)
   <223> xaa in position 3 to 4 is any amino acid
<220>
   <221> Variant
   <222> (9)..(9)
   <223> xaa in position 9 is any amino acid
<220>
   <221> Variant
   <222> (10)..(10)
   <223> xaa in position 10 is Ala, Gln or Ser
<220>
   <221> Variant
   <222> (14)..(14)
   <223> xaa in position 14 is Asp or Asn
<220>
   <221> Variant
   <222> (18)..(18)
   <223> xaa in position 18 is any amino acid
<220>
   <221> Variant
   <222> (21)..(21)
   <223> xaa in position 21 is Ala or Ser
<220>
   <221> Variant
   <222> (22)..(22)
   <223> xaa in position 22 is Ala, Ser or Val
<220>
   <221> Variant
   <222> (23)..(23)
   <223> xaa in position 23 is Pro or Thr
<220>
   <221> Variant
   <222> (25)..(25)
   <223> xaa in position 25 is Leu or Val
<220>
   <221> Variant
   <222> (26)..(26)
   <223> xaa in position 26 is any amino acid
<220>
   <221> Variant
   <222> (27)..(27)
   <223> xaa in position 27 is Glu, Gly, Asn or Ser
<400> 45
   Gly xaa xaa xaa Gln Gly Phe Ser xaa xaa Trp Trp Lys xaa Lys His
    1               5                  10                  15
   Asn xaa His His xaa xaa xaa Asn xaa xaa xaa
                20                  25
<210> 46
   <211> 31
   <212> PRT
   <213> Artificial sequence
<220>
   <221> Variant
   <222> (2)..(3)
   <223> xaa in position 2 to 3 is any amino acid
<220>
   <221> Variant
   <222> (5)..(5)
   <223> xaa in position 5 is Glu, Lys or Arg
<220>
   <221> Variant
   <222> (7)..(7)
   <223> xaa in position 7 is Ala or Gly
<220>
   <221> Variant
   <222> (8)..(8)
   <223> xaa in position 8 is Ser or Thr
<220>
   <221> Variant
   <222> (10)..(10)
   <223> xaa in position 10 is Ala, Ser or Val
<220>
   <221> Variant
   <222> (12)..(13)
   <223> xaa in position 12 to 13 is any amino acid
<220>
   <221> Variant
   <222> (14)..(14)
   <223> xaa in position 14 is Phe or Met
<220>
   <221> Variant
   <222> (16)..(16)
   <223> xaa in position 16 is Pro or Ser
<220>
   <221> Variant
   <222> (17)..(18)
   <223> xaa in position 17 to 18 is any amino acid
<220>
   <221> variant
   <222> (20)..(22)
   <223> xaa in position 20 to 22 is any amino acid
<220>
   <221> Variant
   <222> (24)..(25)
   <223> xaa in position 24 to 25 is any amino acid
<220>
   <221> Variant
   <222> (26)..(26)
   <223> xaa in position 26 is any or no amino acid
<220>
   <221> Variant
   <222> (28)..(28)
   <223> xaa in position 28 is any amino acid
<220>
   <221> Variant
   <222> (29)..(29)
   <223> xaa in position 29 is any or no amino acid
<400> 46
<210> 47
   <211> 26
   <212> PRT
   <213> Artificial sequence
<220>
   <221> Variant
   <222> (2)..(2)
   <223> xaa in position 2 is Phe, Leu or Val
<220>
   <221> Variant
   <222> (3)..(3)
   <223> xaa in position 3 is any amino acid
<220>
   <221> Variant
   <222> (4)..(4)
   <223> xaa in position 4 is Ala, Gly or Ser
<220>
   <221> Variant
   <222> (5)..(5)
   <223> xaa in position 5 is Gln or Thr
<220>
   <221> Variant
   <222> (6)..(6)
   <223> xaa in position 6 is Ala, Cys or Pro
<220>
   <221> Variant
   <222> (7)..(7)
   <223> xaa in position 7 is ser, Thr or Val
<220>
   <221> Variant
   <222> (8)..(8)
   <223> xaa in position 8 is Cys, Ser or Thr
<220>
   <221> Variant
   <222> (10)..(11)
   <223> xaa in position 10 to 11 is any amino acid
<220>
   <221> Variant
   <222> (14)..(14)
   <223> xaa in position 14 is Ile or Leu
<220>
   <221> Variant
   <222> (17)..(18)
   <223> xaa in position 17 to 18 is any amino acid
<220>
   <221> Variant
   <222> (19)..(20)
   <223> xaa in position 19 to 20 is any or no amino acid
<220>
   <221> Variant
   <222> (22)..(23)
   <223> xaa in position 22 to 23 is any or no amino acid
<220>
   <221> Variant
   <222> (26)..(26)
   <223> xaa in position 26 is Ala, Pro or Ser
<400> 47
<210> 48
   <211> 13
   <212> PRT
   <213> Artificial sequence
<220>
   <221> variant
   <222> (6)..(6)
   <223> xaa in position 6 is any amino acid
<220>
   <221> variant
   <222> (8)..(8)
   <223> xaa in position 8 is any or no amino acid
<220>
   <221> Variant
   <222> (11)..(11)
   <223> xaa in position 11 is any or no amino acid
<220>
   <221> Variant
   <222> (13)..(13)
   <223> xaa in position 13 is Asp or Ser
<400> 48
   Pro Asp Ile Asp Thr xaa Pro xaa Leu Leu xaa Trp xaa
    1               5                  10
<210> 49
   <211> 17
   <212> PRT
   <213> Artificial sequence
<220>
   <221> variant
   <222> (2)..(3)
   <223> xaa in position 2 to 3 is any amino acid
<220>
   <221> Variant
   <222> (4)..(4)
   <223> xaa in position 4 is any or no amino acid
<220>
   <221> Variant
   <222> (7)..(8)
   <223> xaa in position 7 to 8 is any amino acid
<220>
   <221> Variant
   <222> (9)..(9)
   <223> xaa in position 9 is any or no amino acid
<220>
   <221> Variant
   <222> (11)..(11)
   <223> xaa in position 11 is any amino acid
<220>
   <221> Variant
   <222> (12)..(12)
   <223> xaa in position 12 is Phe or Leu
<220>
   <221> Variant
   <222> (15)..(15)
   <223> xaa in position 15 is Ile or Leu
<400> 49
   Gln xaa xaa xaa Tyr Phe xaa xaa xaa Leu xaa xaa Ala Arg xaa Ser
     1               5                  10                  15
   Trp
<210> 50
   <211> 20
   <212> PRT
   <213> Artificial sequence
<220>
   <221> Variant
   <222> (2)..(2)
   <223> xaa in position 2 is Asp, Glu or Thr
<220>
   <221> Variant
   <222> (3)..(3)
   <223> xaa in position 3 is Ala or Thr
<220>
   <221> Variant
   <222> (4)..(4)
   <223> xaa in position 4 is Asp, Gly, Ser or Thr
<220>
   <221> Variant
   <222> (5)..(7)
   <223> xaa in position 5 to 7 is any amino acid
<220>
   <221> Variant
   <222> (9)..(10)
   <223> xaa in position 9 to 10 is any amino acid
<220>
   <221> Variant
   <222> (13)..(13)
   <223> xaa in position 13 is Phe, Leu or Val
<220>
   <221> Variant
   <222> (14)..(15)
   <223> xaa in position 14 to 15 is any amino acid
<220>
   <221> Variant
   <222> (17)..(17)
   <223> xaa in position 17 is Ala, Asp, Gly, Asn or Ser
<220>
   <221> Variant
   <222> (18)..(19)
   <223> xaa in position 18 to 19 is any amino acid
<220>
   <221> Variant
   <222> (20)..(20)
   <223> xaa in position 20 is Ala, Gln or Ser
<400> 50
<210> 51
   <211> 1320
   <212> DNA
   <213> Thraustochytrium ssp.
<220>
   <221> CDS
   <222> (1)..(1320)
<400> 51
   atg ggc aag ggc agc gag ggc cgc agc gcg gcg cgc gag atg acg gcc      48
   Met Gly Lys Gly Ser Glu Gly Arg Ser Ala Ala Arg Glu Met Thr Ala
   1               5                   10                  15
   gag gcg aac ggc gac aag cgg aaa acg att ctg atc gag ggc gtc ctg      96
   Glu Ala Asn Gly Asp Lys Arg Lys Thr Ile Leu Ile Glu Gly Val Leu
               20                  25                  30
   tac gac gcg acg aac ttt aag cac ccg ggc ggt tcg atc atc aac ttc     144
   Tyr Asp Ala Thr Asn Phe Lys His Pro Gly Gly Ser Ile Ile Asn Phe
           35                  40                  45
   ttg acc gag ggc gag gcc ggc gtg gac gcg acg cag gcg tac cgc gag     192
   Leu Thr Glu Gly Glu Ala Gly Val Asp Ala Thr Gln Ala Tyr Arg Glu
       50                  55                  60
   ttt cat cag cgg tcc ggc aag gcc gac aag tac ctc aag tcg ctg ccg     240
   Phe His Gln Arg Ser Gly Lys Ala Asp Lys Tyr Leu Lys Ser Leu Pro
   65                  70                  75                  80
   aag ctg gat gcg tcc aag gtg gag tcg cgg ttc tcg gcc aaa gag cag     288
   Lys Leu Asp Ala Ser Lys Val Glu Ser Arg Phe Ser Ala Lys Glu Gln
                   85                  90                  95
   gcg cgg cgc gac gcc atg acg cgc gac tac gcg gcc ttt cgc gag gag     336
   Ala Arg Arg Asp Ala Met Thr Arg Asp Tyr Ala Ala Phe Arg Glu Glu
               100                 105                 110
   ctc gtc gcc gag ggg tac ttt gac ccg tcg atc ccg cac atg att tac     384
   Leu Val Ala Glu Gly Tyr Phe Asp Pro Ser Ile Pro His Met Ile Tyr
           115                 120                 125
   cgc gtc gtg gag atc gtg gcg ctc ttc gcg ctc tcg ttc tgg ctc atg     432
   Arg Val Val Glu Ile Val Ala Leu Phe Ala Leu Ser Phe Trp Leu Met
       130                 135                 140
   tcc aag gcc tcg ccc acc tcg ctc gtg ctg ggc gtg gtg atg aac ggc     480
   Ser Lys Ala Ser Pro Thr Ser Leu Val Leu Gly Val Val Met Asn Gly
   145                 150                 155                 160
   att gcg cag ggc cgc tgc ggc tgg gtc atg cac gag atg ggc cac ggg     528
   Ile Ala Gln Gly Arg Cys Gly Trp Val Met His Glu Met Gly His Gly
                   165                 170                 175
   tcg ttc acg ggc gtc atc tgg ctc gac gac cgg atg tgc gag ttc ttc     576
   Ser Phe Thr Gly Val Ile Trp Leu Asp Asp Arg Met Cys Glu Phe Phe
               180                 185                 190
   tac ggc gtc ggc tgc ggc atg agc ggg cac tac tgg aag aac cag cac     624
   Tyr Gly Val Gly Cys Gly Met Ser Gly His Tyr Trp Lys Asn Gln His
           195                 200                 205
   agc aag cac cac gcc gcg ccc aac cgc ctc gag cac gat gtc gat ctc     672
   Ser Lys His His Ala Ala Pro Asn Arg Leu Glu His Asp Val Asp Leu
       210                 215                 220
   aac acg ctg ccc ctg gtc gcc ttt aac gag cgc gtc gtg cgc aag gtc     720
   Asn Thr Leu Pro Leu Val Ala Phe Asn Glu Arg Val Val Arg Lys Val
   225                 230                 235                 240
   aag ccg gga tcg ctg ctg gcg ctc tgg ctg cgc gtg cag gcg tac ctc     768
   Lys Pro Gly Ser Leu Leu Ala Leu Trp Leu Arg Val Gln Ala Tyr Leu
                   245                 250                 255
   ttt gcg ccc gtc tcg tgc ctg ctc atc ggc ctt ggc tgg acg ctc tac     816
   Phe Ala Pro Val Ser Cys Leu Leu Ile Gly Leu Gly Trp Thr Leu Tyr
               260                 265                 270
   ctg cac ccg cgc tac atg ctg cgc acc aag cgg cac atg gag ttc gtc     864
   Leu His Pro Arg Tyr Met Leu Arg Thr Lys Arg His Met Glu Phe Val
           275                 280                 285
   tgg atc ttc gcg cgc tac att ggc tgg ttc tcg ctc atg ggc gct ctc     912
   Trp Ile Phe Ala Arg Tyr Ile Gly Trp Phe Ser Leu Met Gly Ala Leu
       290                 295                 300
   ggc tac tcg ccg ggc acc tcg gtc ggg atg tac ctg tgc tcg ttc ggc     960
   Gly Tyr Ser Pro Gly Thr Ser Val Gly Met Tyr Leu Cys Ser Phe Gly
   305                 310                 315                 320
   ctc ggc tgc att tac att ttc ctg cag ttc gcc gtc agc cac acg cac    1008
   Leu Gly Cys Ile Tyr Ile Phe Leu Gln Phe Ala Val Ser His Thr His
                   325                 330                 335
   ctg ccg gtg acc aac ccg gag gac cag ctg cac tgg ctc gag tac gcg    1056
   Leu Pro Val Thr Asn Pro Glu Asp Gln Leu His Trp Leu Glu Tyr Ala
               340                 345                 350
   gcc gac cac acg gtg aac att agc acc aag tcc tgg ctc gtc acg tgg      1104
   Ala Asp His Thr Val Asn Ile Ser Thr Lys Ser Trp Leu Val Thr Trp
           355                 360                 365
   tgg atg tcg aac ctg aac ttt cag atc gag cac cac ctc ttc ccc acg      1152
   Trp Met Ser Asn Leu Asn Phe Gln Ile Glu His His Leu Phe Pro Thr
       370                 375                 380
   gcg ccg cag ttc cgc ttc aag gaa atc agt cct cgc gtc gag gcc ctc      1200
   Ala Pro Gln Phe Arg Phe Lys Glu Ile Ser Pro Arg Val Glu Ala Leu
   385                 390                 395                 400
   ttc aag cgc cac aac ctc ccg tac tac gac ctg ccc tac acg agc gcg      1248
   Phe Lys Arg His Asn Leu Pro Tyr Tyr Asp Leu Pro Tyr Thr Ser Ala
                   405                 410                 415
   gtc tcg acc acc ttt gcc aat ctt tat tcc gtc ggc cac tcg gtc ggc      1296
   Val Ser Thr Thr Phe Ala Asn Leu Tyr Ser Val Gly His Ser Val Gly
               420                 425                 430
   gcc gac acc aag aag cag gac tga                                      1320
   Ala Asp Thr Lys Lys Gln Asp
           435                 440
<210> 52
   <211> 439
   <212> PRT
   <213> Thraustochytrium ssp.
<400> 52
   Met Gly Lys Gly Ser Glu Gly Arg Ser Ala Ala Arg Glu Met Thr Ala
   1               5                   10                  15
   Glu Ala Asn Gly Asp Lys Arg Lys Thr Ile Leu Ile Glu Gly Val Leu
               20                  25                  30
   Tyr Asp Ala Thr Asn Phe Lys His Pro Gly Gly Ser Ile Ile Asn Phe
           35                  40                  45
   Leu Thr Glu Gly Glu Ala Gly Val Asp Ala Thr Gln Ala Tyr Arg Glu
       50                  55                  60
   Phe His Gln Arg Ser Gly Lys Ala Asp Lys Tyr Leu Lys Ser Leu Pro
   65                  70                  75                  80
   Lys Leu Asp Ala Ser Lys Val Glu Ser Arg Phe Ser Ala Lys Glu Gln
                   85                  90                  95
   Ala Arg Arg Asp Ala Met Thr Arg Asp Tyr Ala Ala Phe Arg Glu Glu
               100                 105                 110
   Leu Val Ala Glu Gly Tyr Phe Asp Pro Ser Ile Pro His Met Ile Tyr
           115                 120                 125
   Arg Val Val Glu Ile Val Ala Leu Phe Ala Leu Ser Phe Trp Leu Met
       130                 135                 140
   Ser Lys Ala Ser Pro Thr Ser Leu Val Leu Gly Val Val Met Asn Gly
   145                 150                 155                 160
   Ile Ala Gln Gly Arg Cys Gly Trp Val Met His Glu Met Gly His Gly
                   165                 170                 175
   Ser Phe Thr Gly Val Ile Trp Leu Asp Asp Arg Met Cys Glu Phe Phe
               180                 185                 190
   Tyr Gly Val Gly Cys Gly Met Ser Gly His Tyr Trp Lys Asn Gln His
           195                 200                 205
   Ser Lys His His Ala Ala Pro Asn Arg Leu Glu His Asp Val Asp Leu
       210                 215                 220
   Asn Thr Leu Pro Leu Val Ala Phe Asn Glu Arg Val Val Arg Lys Val
   225                 230                 235                 240
   Lys Pro Gly Ser Leu Leu Ala Leu Trp Leu Arg Val Gln Ala Tyr Leu
                   245                 250                 255
   Phe Ala Pro Val Ser Cys Leu Leu Ile Gly Leu Gly Trp Thr Leu Tyr
               260                 265                 270
   Leu His Pro Arg Tyr Met Leu Arg Thr Lys Arg His Met Glu Phe Val
           275                 280                 285
   Trp Ile Phe Ala Arg Tyr Ile Gly Trp Phe Ser Leu Met Gly Ala Leu
       290                 295                 300
   Gly Tyr Ser Pro Gly Thr Ser Val Gly Met Tyr Leu Cys Ser Phe Gly
   305                 310                 315                 320
   Leu Gly Cys Ile Tyr Ile Phe Leu Gln Phe Ala Val Ser His Thr His
                   325                 330                 335
   Leu Pro Val Thr Asn Pro Glu Asp Gln Leu His Trp Leu Glu Tyr Ala
               340                 345                 350
   Ala Asp His Thr Val Asn Ile Ser Thr Lys Ser Trp Leu Val Thr Trp
           355                 360                 365
   Trp Met Ser Asn Leu Asn Phe Gln Ile Glu His His Leu Phe Pro Thr
       370                 375                 380
   Ala Pro Gln Phe Arg Phe Lys Glu Ile Ser Pro Arg Val Glu Ala Leu
   385                 390                 395                 400
   Phe Lys Arg His Asn Leu Pro Tyr Tyr Asp Leu Pro Tyr Thr Ser Ala
                   405                 410                 415
   Val Ser Thr Thr Phe Ala Asn Leu Tyr Ser Val Gly His Ser Val Gly
               420                 425                 430
   Ala Asp Thr Lys Lys Gln Asp
           435
<210> 53
   <211> 1371
   <212> DNA
   <213> Ostreococcus tauri
<220>
   <221> CDS
   <222> (1)..(1371)
<400> 53
   atg tgc gtg gag acg gaa aat aac gat ggg atc ccc acg gtg gag atc      48
   Met Cys Val Glu Thr Glu Asn Asn Asp Gly Ile Pro Thr Val Glu Ile
   1               5                   10                  15
   gcg ttc gac ggt gag cgc gag cgg gcg gag gca aac gtg aag ctg tcc      96
   Ala Phe Asp Gly Glu Arg Glu Arg Ala Glu Ala Asn Val Lys Leu Ser
               20                  25                  30
   gcg gag aag atg gag ccg gcg gcg ctg gcg aag acg ttc gcg agg cgg      144
   Ala Glu Lys Met Glu Pro Ala Ala Leu Ala Lys Thr Phe Ala Arg Arg
           35                  40                  45
   tac gtc gtg atc gag ggg gtg gag tac gat gtg acg gat ttt aag cac      192
   Tyr Val Val Ile Glu Gly Val Glu Tyr Asp Val Thr Asp Phe Lys His
       50                  55                  60
   ccg gga gga acg gtt att ttc tat gcg ttg tca aac acc ggg gcg gac      240
   Pro Gly Gly Thr Val Ile Phe Tyr Ala Leu Ser Asn Thr Gly Ala Asp
   65                  70                  75                  80
   gcg acg gaa gcg ttc aag gag ttt cat cat cgg tcg aga aag gcg agg      288
   Ala Thr Glu Ala Phe Lys Glu Phe His His Arg Ser Arg Lys Ala Arg
                   85                  90                  95
   aaa gcc ttg gcg gcg ctc ccg tct cga ccg gcc aag acg gcc aag gtg      336
   Lys Ala Leu Ala Ala Leu Pro Ser Arg Pro Ala Lys Thr Ala Lys Val
               100                 105                 110
   gac gac gcg gag atg ctc caa gat ttc gcc aag tgg cgg aaa gaa ttg      384
   Asp Asp Ala Glu Met Leu Gln Asp Phe Ala Lys Trp Arg Lys Glu Leu
           115                 120                 125
   gag aga gat gga ttc ttc aag ccc tct ccg gcg cac gtg gcg tat cgc      432
   Glu Arg Asp Gly Phe Phe Lys Pro Ser Pro Ala His Val Ala Tyr Arg
       130                 135                 140
   ttc gcc gag ctc gcg gcg atg tac gct ctc ggg acg tac ctg atg tac      480
   Phe Ala Glu Leu Ala Ala Met Tyr Ala Leu Gly Thr Tyr Leu Met Tyr
   145                 150                 155                 160
   gct cga tac gtc gtc tcc tcg gtg ctc gtg tac gct tgc ttt ttc ggc      528
   Ala Arg Tyr Val Val Ser Ser Val Leu Val Tyr Ala Cys Phe Phe Gly
                   165                 170                 175
   gcc cga tgc ggt tgg gtg cag cac gag ggc gga cac agc tcg ctg acg      576
   Ala Arg Cys Gly Trp Val Gln His Glu Gly Gly His Ser Ser Leu Thr
               180                 185                 190
   ggc aac att tgg tgg gac aag cgc atc cag gcc ttc aca gcc ggg ttc      624
   Gly Asn Ile Trp Trp Asp Lys Arg Ile Gln Ala Phe Thr Ala Gly Phe
           195                 200                 205
   ggt ctc gcc ggt agc ggc gac atg tgg aac tcg atg cac aac aag cat      672
   Gly Leu Ala Gly Ser Gly Asp Met Trp Asn Ser Met His Asn Lys His
       210                 215                 220
   cac gcg acg cct caa aag gtt cgt cac gac atg gat ctg gac acc acc        720
   His Ala Thr Pro Gln Lys Val Arg His Asp Met Asp Leu Asp Thr Thr
   225                 230                 235                 240
   ccc gcg gtg gcg ttc ttc aac acc gcg gtg gaa gac aat cgt ccc cgt        768
   Pro Ala Val Ala Phe Phe Asn Thr Ala Val Glu Asp Asn Arg Pro Arg
                   245                 250                 255
   ggc ttt agc aag tac tgg ttg cgc ctt cag gcg tgg acc ttc atc ccc        816
   Gly Phe Ser Lys Tyr Trp Leu Arg Leu Gln Ala Trp Thr Phe Ile Pro
               260                 265                 270
   gtg acg tcc ggc ttg gtg ctc ctt ttc tgg atg ttt ttc ctc cac ccc        864
   Val Thr Ser Gly Leu Val Leu Leu Phe Trp Met Phe Phe Leu His Pro
           275                 280                 285
   tcc aag gct ttg aag ggt ggc aag tac gaa gag ttg gtg tgg atg ctc        912
   Ser Lys Ala Leu Lys Gly Gly Lys Tyr Glu Glu Leu Val Trp Met Leu
       290                 295                 300
   gcc gcg cac gtc atc cgc acg tgg acg atc aag gcg gtg acc gga ttc        960
   Ala Ala His Val Ile Arg Thr Trp Thr Ile Lys Ala Val Thr Gly Phe
   305                 310                 315                 320
   acc gcg atg cag tcc tac ggc tta ttt ttg gcg acg agc tgg gtg agc       1008
   Thr Ala Met Gln Ser Tyr Gly Leu Phe Leu Ala Thr Ser Trp Val Ser
                   325                 330                 335
   ggc tgc tat ctg ttt gca cac ttc tcc acg tcg cac acg cac ctg gat       1056
   Gly Cys Tyr Leu Phe Ala His Phe Ser Thr Ser His Thr His Leu Asp
               340                 345                 350
   gtg gtg ccc gcg gac gag cat ctc tcc tgg gtt cga tac gcc gtc gat       1104
   Val Val Pro Ala Asp Glu His Leu Ser Trp Val Arg Tyr Ala Val Asp
           355                 360                 365
   cac acg atc gac atc gat ccg agt caa ggt tgg gtg aac tgg ttg atg       1152
   His Thr Ile Asp Ile Asp Pro Ser Gln Gly Trp Val Asn Trp Leu Met
       370                 375                 380
   ggc tac ctc aac tgc caa gtc atc cac cac ctc ttt ccg agc atg ccg       1200
   Gly Tyr Leu Asn Cys Gln Val Ile His His Leu Phe Pro Ser Met Pro
   385                 390                 395                 400
   cag ttc cgc cag ccc gag gta tct cgc cgc ttc gtc gcc ttt gcg aaa       1248
   Gln Phe Arg Gln Pro Glu Val Ser Arg Arg Phe Val Ala Phe Ala Lys
                   405                 410                 415
   aag tgg aac ctc aac tac aag gtc atg acc tac gcc ggt gcg tgg aag       1296
   Lys Trp Asn Leu Asn Tyr Lys Val Met Thr Tyr Ala Gly Ala Trp Lys
               420                 425                 430
   gca acg ctc gga aac ctc gac aac gtg ggt aag cac tac tac gtg cac       1344
   Ala Thr Leu Gly Asn Leu Asp Asn Val Gly Lys His Tyr Tyr Val His
           435                 440                 445
   ggc caa cac tcc gga aag acg gcg taa                                   1371
   Gly Gln His Ser Gly Lys Thr Ala
       450                 455
<210> 54
   <211> 456
   <212> PRT
   <213> Ostreococcus tauri
<400> 54
   Met Cys Val Glu Thr Glu Asn Asn Asp Gly Ile Pro Thr Val Glu Ile
   1               5                   10                  15
   Ala Phe Asp Gly Glu Arg Glu Arg Ala Glu Ala Asn Val Lys Leu Ser
               20                  25                  30
   Ala Glu Lys Met Glu Pro Ala Ala Leu Ala Lys Thr Phe Ala Arg Arg
           35                  40                  45
   Tyr Val Val Ile Glu Gly Val Glu Tyr Asp Val Thr Asp Phe Lys His
       50                  55                  60
   Pro Gly Gly Thr Val Ile Phe Tyr Ala Leu Ser Asn Thr Gly Ala Asp
   65                  70                  75                  80
   Ala Thr Glu Ala Phe Lys Glu Phe His His Arg Ser Arg Lys Ala Arg
                   85                  90                  95
   Lys Ala Leu Ala Ala Leu Pro Ser Arg Pro Ala Lys Thr Ala Lys Val
               100                 105                 110
   Asp Asp Ala Glu Met Leu Gln Asp Phe Ala Lys Trp Arg Lys Glu Leu
           115                 120                 125
   Glu Arg Asp Gly Phe Phe Lys Pro Ser Pro Ala His Val Ala Tyr Arg
       130                 135                 140
   Phe Ala Glu Leu Ala Ala Met Tyr Ala Leu Gly Thr Tyr Leu Met Tyr
   145                 150                 155                 160
   Ala Arg Tyr Val Val Ser Ser Val Leu Val Tyr Ala Cys Phe Phe Gly
                   165                 170                 175
   Ala Arg Cys Gly Trp Val Gln His Glu Gly Gly His Ser Ser Leu Thr
               180                 185                 190
   Gly Asn Ile Trp Trp Asp Lys Arg Ile Gln Ala Phe Thr Ala Gly Phe
           195                 200                 205
   Gly Leu Ala Gly Ser Gly Asp Met Trp Asn Ser Met His Asn Lys His
       210                 215                 220
   His Ala Thr Pro Gln Lys Val Arg His Asp Met Asp Leu Asp Thr Thr
   225                 230                 235                 240
   Pro Ala Val Ala Phe Phe Asn Thr Ala Val Glu Asp Asn Arg Pro Arg
                   245                 250                 255
   Gly Phe Ser Lys Tyr Trp Leu Arg Leu Gln Ala Trp Thr Phe Ile Pro
               260                 265                 270
   Val Thr Ser Gly Leu Val Leu Leu Phe Trp Met Phe Phe Leu His Pro
           275                 280                 285
   Ser Lys Ala Leu Lys Gly Gly Lys Tyr Glu Glu Leu Val Trp Met Leu
       290                 295                 300
   Ala Ala His Val Ile Arg Thr Trp Thr Ile Lys Ala Val Thr Gly Phe
   305                 310                 315                 320
   Thr Ala Met Gln Ser Tyr Gly Leu Phe Leu Ala Thr Ser Trp Val Ser
                   325                 330                 335
   Gly Cys Tyr Leu Phe Ala His Phe Ser Thr Ser His Thr His Leu Asp
               340                 345                 350
   Val Val Pro Ala Asp Glu His Leu Ser Trp Val Arg Tyr Ala Val Asp
           355                 360                 365
   His Thr Ile Asp Ile Asp Pro Ser Gln Gly Trp Val Asn Trp Leu Met
       370                 375                 380
   Gly Tyr Leu Asn Cys Gln Val Ile His His Leu Phe Pro Ser Met Pro
   385                 390                 395                 400
   Gln Phe Arg Gln Pro Glu Val Ser Arg Arg Phe Val Ala Phe Ala Lys
                   405                 410                 415
   Lys Trp Asn Leu Asn Tyr Lys Val Met Thr Tyr Ala Gly Ala Trp Lys
               420                 425                 430
   Ala Thr Leu Gly Asn Leu Asp Asn Val Gly Lys His Tyr Tyr Val His
           435                 440                 445
   Gly Gln His Ser Gly Lys Thr Ala
       450                 455
<210> 55
   <211> 1254
   <212> DNA
   <213> Leishmania major
<220>
   <221> CDS
   <222> (1)..(1254)
<400> 55
   atg gcc ctc gac aat gtc cgt ccg cac caa ccg aac gag gtg ctg atc       48
   Met Ala Leu Asp Asn Val Arg Pro His Gln Pro Asn Glu Val Leu Ile
   1               5                   10                  15
   gat ggc gtc ctt tac gac tgc acc gat ttc cgg cat ccg ggt ggc agc       96
   Asp Gly Val Leu Tyr Asp Cys Thr Asp Phe Arg His Pro Gly Gly Ser
               20                  25                  30
   att ctg aaa tac tac ctc ggc agc ggc gac gcc acc gag acg tac caa       144
   Ile Leu Lys Tyr Tyr Leu Gly Ser Gly Asp Ala Thr Glu Thr Tyr Gln
           35                  40                  45
   cag ttc cac ttg aag ctg ccc agg gcg gac aag tat ctc aag cgg ctg       192
   Gln Phe His Leu Lys Leu Pro Arg Ala Asp Lys Tyr Leu Lys Arg Leu
       50                  55                  60
   ccg aat cgc ccg gcg ccg cca cag cac agc gtc aac gtg gat gag cag       240
   Pro Asn Arg Pro Ala Pro Pro Gln His Ser Val Asn Val Asp Glu Gln
   65                  70                  75                  80
   aag cga ttg gag aag ctc tcg cgg gac ttc aag gcg ctg cag gat gcg 288
   Lys Arg Leu Glu Lys Leu Ser Arg Asp Phe Lys Ala Leu Gln Asp Ala
   85 90 95
   tgc gta gag gag ggc ctt ttt aac gcc agc tgg ccg cac atc gtc tac 336
   Cys Val Glu Glu Gly Leu Phe Asn Ala Ser Trp Pro His Ile Val Tyr
   100 105 110
   cgg ttt tct gag ctg atc ctg atg cac gcc atc ggt ctt tac atg ctc 384
   Arg Phe Ser Glu Leu Ile Leu Met His Ala Ile Gly Leu Tyr Met Leu
   115 120 125
   ttc cgt ctt ccg atc ctg tgg ccc gtc gcg ctg gtg atc ctt gga gtg 432
   Phe Arg Leu Pro Ile Leu Trp Pro Val Ala Leu Val Ile Leu Gly Val
   130 135 140
   gcg gag ggg cga tgt ggc tgg tgg atg cac gag gcc ggt cac tac agc 480
   Ala Glu Gly Arg Cys Gly Trp Trp Met His Glu Ala Gly His Tyr Ser
   145 150 155 160
   gtc aca ggc att ccg tgg ttg gac att aaa ata cag gag gta ctc tac 528
   Val Thr Gly Ile Pro Trp Leu Asp Ile Lys Ile Gln Glu Val Leu Tyr
   165 170 175
   gga ctt ggc gat gga atg agc gcg tcg tgg tgg cgg tcg cag cat aac 576
   Gly Leu Gly Asp Gly Met Ser Ala Ser Trp Trp Arg Ser Gln His Asn
   180 185 190
   aag cat cac gct act ccg cag aag cac cgg cac gac gtg gac ctt gag 624
   Lys His His Ala Thr Pro Gln Lys His Arg His Asp Val Asp Leu Glu
   195 200 205
   acg ctg cct ctc gtc gcc ttc aac aag atc atc gcg cgc cgc ggc aag 672
   Thr Leu Pro Leu Val Ala Phe Asn Lys Ile Ile Ala Arg Arg Gly Lys
   210 215 220
   agg aac gcg agc att cgc cgc tgg atc tcc ctg cag atg ttc ctc ttc 720
   Arg Asn Ala Ser Ile Arg Arg Trp Ile Ser Leu Gln Met Phe Leu Phe
   225 230 235 240
   ggc ccc gtc acc tgc tcc ctt gtc gcc ctc tac tgg cag ctc ttc cta 768
   Gly Pro Val Thr Cys Ser Leu Val Ala Leu Tyr Trp Gln Leu Phe Leu
   245 250 255
   cac gtc cgc cac gcc atg cgc act cag cgt tac aca gag ggc tct gcc 816
   His Val Arg His Ala Met Arg Thr Gln Arg Tyr Thr Glu Gly Ser Ala
   260 265 270
   atc ctg tgc cgc tgg atc gtg gtc ggc gtt atc tgt cac cag ctg cag 864
   Ile Leu Cys Arg Trp Ile Val Val Gly Val Ile Cys His Gln Leu Gln
   275 280 285
   gtc tcg ttc tgg caa ggc ctc ggc ggc gtt ctc ttc tcc cag gcc ttc 912
   Val Ser Phe Trp Gln Gly Leu Gly Gly Val Leu Phe Ser Gln Ala Phe
   290 295 300
   agc gcc gcc tac atc ttc ata aac ttc gcc ctc aac cac tct cac ctg 960
   Ser Ala Ala Tyr Ile Phe Ile Asn Phe Ala Leu Asn His Ser His Leu
   305 310 315 320
   ccg atg ctt ccg gaa gac gaa cac gcg cac ttc gtc gag tac gcg gcc 1008
   Pro Met Leu Pro Glu Asp Glu His Ala His Phe Val Glu Tyr Ala Ala
   325 330 335
   atc tac acc atg aac gtg aca ccg tcg tgg ttc gtg acg tgg ttc atg 1056
   Ile Tyr Thr Met Asn Val Thr Pro Ser Trp Phe Val Thr Trp Phe Met
   340 345 350
   ggc tac ctt aac tac cag gtg gaa cac cac ctc ttc cct acc atg cca 1104
   Gly Tyr Leu Asn Tyr Gln Val Glu His His Leu Phe Pro Thr Met Pro
   355 360 365
   cag ttc cgc ttc gtc caa ctg gcg ccg cga gtg cgg aaa ctt ttt gag 1152
   Gln Phe Arg Phe Val Gln Leu Ala Pro Arg Val Arg Lys Leu Phe Glu
   370 375 380
   gaa aac ggc ctc aag tac gat tcg cgt ccg tac atg gag tcg ctc cag 1200
   Glu Asn Gly Leu Lys Tyr Asp Ser Arg Pro Tyr Met Glu Ser Leu Gln
   385 390 395 400
   aaa acc ttc aag aac ctc ggc gac gtg gcc gag ttc atc gtt gct ggg 1248
   Lys Thr Phe Lys Asn Leu Gly Asp Val Ala Glu Phe Ile Val Ala Gly
   405 410 415
   aac taa 1254
   Asn
<210> 56
   <211> 417
   <212> PRT
   <213> Leishmania major
<400> 56
   Met Ala Leu Asp Asn Val Arg Pro His Gln Pro Asn Glu Val Leu Ile
   1               5                   10                  15
   Asp Gly Val Leu Tyr Asp Cys Thr Asp Phe Arg His Pro Gly Gly Ser
               20                  25                  30
   Ile Leu Lys Tyr Tyr Leu Gly Ser Gly Asp Ala Thr Glu Thr Tyr Gln
           35                  40                  45
   Gln Phe His Leu Lys Leu Pro Arg Ala Asp Lys Tyr Leu Lys Arg Leu
       50                  55                  60
   Pro Asn Arg Pro Ala Pro Pro Gln His Ser Val Asn Val Asp Glu Gln
   65                  70                  75                  80
   Lys Arg Leu Glu Lys Leu Ser Arg Asp Phe Lys Ala Leu Gln Asp Ala
                   85                  90                  95
   Cys Val Glu Glu Gly Leu Phe Asn Ala Ser Trp Pro His Ile Val Tyr
               100                 105                 110
   Arg Phe Ser Glu Leu Ile Leu Met His Ala Ile Gly Leu Tyr Met Leu
           115                 120                 125
   Phe Arg Leu Pro Ile Leu Trp Pro Val Ala Leu Val Ile Leu Gly Val
       130                 135                 140
   Ala Glu Gly Arg Cys Gly Trp Trp Met His Glu Ala Gly His Tyr Ser
   145                 150                 155                 160
   Val Thr Gly Ile Pro Trp Leu Asp Ile Lys Ile Gln Glu Val Leu Tyr
                   165                 170                 175
   Gly Leu Gly Asp Gly Met Ser Ala Ser Trp Trp Arg Ser Gln His Asn
               180                 185                 190
   Lys His His Ala Thr Pro Gln Lys His Arg His Asp Val Asp Leu Glu
           195                 200                 205
   Thr Leu Pro Leu Val Ala Phe Asn Lys Ile Ile Ala Arg Arg Gly Lys
       210                 215                 220
   Arg Asn Ala Ser Ile Arg Arg Trp Ile Ser Leu Gln Met Phe Leu Phe
   225                 230                 235                 240
   Gly Pro Val Thr Cys Ser Leu Val Ala Leu Tyr Trp Gln Leu Phe Leu
                   245                 250                 255
   His Val Arg His Ala Met Arg Thr Gln Arg Tyr Thr Glu Gly Ser Ala
               260                 265                 270
   Ile Leu Cys Arg Trp Ile Val Val Gly Val Ile Cys His Gln Leu Gln
           275                 280                 285
   Val Ser Phe Trp Gln Gly Leu Gly Gly Val Leu Phe Ser Gln Ala Phe
       290                 295                 300
   Ser Ala Ala Tyr Ile Phe Ile Asn Phe Ala Leu Asn His Ser His Leu
   305                 310                 315                 320
   Pro Met Leu Pro Glu Asp Glu His Ala His Phe Val Glu Tyr Ala Ala
                   325                 330                 335
   Ile Tyr Thr Met Asn Val Thr Pro Ser Trp Phe Val Thr Trp Phe Met
               340                 345                 350
   Gly Tyr Leu Asn Tyr Gln Val Glu His His Leu Phe Pro Thr Met Pro
           355                 360                 365
   Gln Phe Arg Phe Val Gln Leu Ala Pro Arg Val Arg Lys Leu Phe Glu
       370                 375                 380
   Glu Asn Gly Leu Lys Tyr Asp Ser Arg Pro Tyr Met Glu Ser Leu Gln
   385                 390                 395                 400
   Lys Thr Phe Lys Asn Leu Gly Asp Val Ala Glu Phe Ile Val Ala Gly
                   405                 410                 415
   Asn
<210> 57
   <211> 401
   <212> PRT
   <213> Artificial sequence
<220>
   <221> Variant
   <222> (2)..(2)
   <223> xaa in position 2 is any amino acid
<220>
   <221> Variant
   <222> (5)..(5)
   <223> xaa in position 5 is any amino acid
<220>
   <221> Variant
   <222> (8)..(8)
   <223> xaa in position 8 is any amino acid
<220>
   <221> Variant
   <222> (10)..(10)
   <223> xaa in position 10 is any amino acid
<220>
   <221> Variant
   <222> (12)..(12)
   <223> xaa in position 12 is any amino acid
<220>
   <221> Variant
   <222> (17)..(26)
   <223> xaa in position 17 to 26 is any amino acid
<220>
   <221> Variant
   <222> (27)..(29)
   <223> xaa in position 27 to 29 is any or no amino acid
<220>
   <221> Variant
   <222> (33)..(37)
   <223> xaa in position 33 to 37 is any amino acid
<220>
   <221> Variant
   <222> (40)..(44)
   <223> xaa in position 40 to 44 is any amino acid
<220>
   <221> Variant
   <222> (46)..(46)
   <223> xaa in position 46 is any amino acid
<220>
   <221> Variant
   <222> (48)..(48)
   <223> xaa in position 48 is any amino acid
<220>
   <221> Variant
   <222> (50)..(51)
   <223> xaa in position 50 to 51 is any amino acid
<220>
   <221> Variant
   <222> (54)..(56)
   <223> xaa in position 54 to 56 is any amino acid
<220>
   <221> Variant
   <222> (58)..(69)
   <223> xaa in position 58 to 69 is any amino acid
<220>
   <221> Variant
   <222> (70)..(77)
   <223> xaa in position 70 to 77 is any or no amino acid
<220>
   <221> Variant
   <222> (79)..(89)
   <223> xaa in position 79 to 89 is any amino acid
<220>
   <221> Variant
   <222> (91)..(91)
   <223> xaa in position 91 is any amino acid
<220>
   <221> Variant
   <222> (93)..(94)
   <223> xaa in position 93 to 94 is any amino acid
<220>
   <221> Variant
   <222> (96)..(97)
   <223> xaa in position 96 to 97 is any amino acid
<220>
   <221> Variant
   <222> (99)..(100)
   <223> xaa in position 99 to 100 is any amino acid
<220>
   <221> Variant
   <222> (103)..(104)
   <223> xaa in position 103 to 104 is any amino acid
<220>
   <221> Variant
   <222> (106)..(110)
   <223> xaa in position 106 to 110 is any amino acid
<220>
   <221> Variant
   <222> (112)..(127)
   <223> xaa in position 112 to 127 is any amino acid
<220>
   <221> Variant
   <222> (128)..(129)
   <223> xaa in position 128 to 129 is any or no amino acid
<220>
   <221> Variant
   <222> (131)..(137)
   <223> xaa in position 131 to 137 is any amino acid
<220>
   <221> Variant
   <222> (142)..(143)
   <223> xaa in position 142 to 143 is any amino acid
<220>
   <221> Variant
   <222> (146)..(146)
   <223> xaa in position 146 is any amino acid
<220>
   <221> Variant
   <222> (149)..(149)
   <223> xaa in position 149 is any amino acid
<220>
   <221> Variant
   <222> (151)..(151)
   <223> xaa in position 151 is any amino acid
<220>
   <221> Variant
   <222> (154)..(155)
   <223> xaa in position 154 to 155 is any amino acid
<220>
   <221> Variant
   <222> (157)..(157)
   <223> xaa in position 157 is any amino acid
<220>
   <221> Variant
   <222> (159)..(166)
   <223> xaa in position 159 to 166 is any amino acid
<220>
   <221> Variant
   <222> (168)..(168)
   <223> xaa in position 168 is any amino acid
<220>
   <221> Variant
   <222> (170)..(172)
   <223> xaa in position 170 to 172 is any amino acid
<220>
   <221> Variant
   <222> (174)..(176)
   <223> xaa in position 174 to 176 is any amino acid
<220>
   <221> Variant
   <222> (178)..(180)
   <223> xaa in position 178 to 180 is any amino acid
<220>
   <221> Variant
   <222> (182)..(182)
   <223> xaa in position 182 is any amino acid
<220>
   <221> Variant
   <222> (187)..(187)
   <223> xaa in position 187 is any amino acid
<220>
   <221> Variant
   <222> (189)..(192)
   <223> xaa in position 189 to 192 is any amino acid
<220>
   <221> Variant
   <222> (195)..(195)
   <223> xaa in position 195 is any amino acid
<220>
   <221> Variant
   <222> (198)..(198)
   <223> xaa in position 198 is any amino acid
<220>
   <221> Variant
   <222> (200)..(200)
   <223> xaa in position 200 is any amino acid
<220>
   <221> Variant
   <222> (202)..(202)
   <223> xaa in position 202 is any amino acid
<220>
   <221> Variant
   <222> (206)..(221)
   <223> xaa in position 206 to 221 is any amino acid
<220>
   <221> Variant
   <222> (223)..(225)
   <223> xaa in position 223 to 225 is any amino acid
<220>
   <221> Variant
   <222> (227)..(229)
   <223> xaa in position 227 to 229 is any amino acid
<220>
   <221> Variant
   <222> (231)..(231)
   <223> xaa in position 231 is any amino acid
<220>
   <221> Variant
   <222> (234)..(236)
   <223> xaa in position 234 to 236 is any amino acid
<220>
   <221> variant
   <222> (238)..(239)
   <223> xaa in position 238 to 239 is any amino acid
<220>
   <221> variant
   <222> (241)..(241)
   <223> xaa in position 241 is any amino acid
<220>
   <221> Variant
   <222> (243)..(245)
   <223> xaa in position 243 to 245 is any amino acid
<220>
   <221> Variant
   <222> (248)..(258)
   <223> xaa in position 248 to 258 is any amino acid
<220>
   <221> Variant
   <222> (260)..(285)
   <223> xaa in position 260 to 285 is any amino acid
<220>
   <221> Variant
   <222> (286)..(286)
   <223> xaa in position 286 is any or no amino acid
<220>
   <221> Variant
   <222> (288)..(289)
   <223> xaa in position 288 to 289 is any amino acid
<220>
   <221> Variant
   <222> (291)..(298)
   <223> xaa in position 291 to 298 is any amino acid
<220>
   <221> Variant
   <222> (300)..(300)
   <223> xaa in position 300 is any amino acid
<220>
   <221> Variant
   <222> (302)..(303)
   <223> xaa in position 302 to 303 is any amino acid
<220>
   <221> Variant
   <222> (305)..(307)
   <223> xaa in position 305 to 307 is any amino acid
<220>
   <221> Variant
   <222> (309)..(309)
   <223> xaa in position 309 is any amino acid
<220>
   <221> Variant
   <222> (312)..(324)
   <223> xaa in position 312 to 324 is any amino acid
<220>
   <221> Variant
   <222> (327)..(329)
   <223> xaa in position 327 to 329 is any amino acid
<220>
   <221> Variant
   <222> (331)..(338)
   <223> xaa in position 331 to 338 is any amino acid
<220>
   <221> Variant
   <222> (339)..(339)
   <223> xaa in position 339 is any or no amino acid
<220>
   <221> variant
   <222> (341)..(341)
   <223> xaa in position 341 is any amino acid
<220>
   <221> Variant
   <222> (343)..(343)
   <223> xaa in position 343 is any amino acid
<220>
   <221> Variant
   <222> (345)..(346)
   <223> xaa in position 345 to 346 is any amino acid
<220>
   <221> variant
   <222> (349)..(349)
   <223> xaa in position 349 is any amino acid
<220>
   <221> Variant
   <222> (351)..(352)
   <223> xaa in position 351 to 352 is any amino acid
<220>
   <221> Variant
   <222> (358)..(359)
   <223> xaa in position 358 to 359 is any amino acid
<220>
   <221> variant
   <222> (364)..(369)
   <223> xaa in position 364 to 369 is any amino acid
<220>
   <221> Variant
   <222> (371)..(379)
   <223> xaa in position 371 to 379 is any amino acid
<220>
   <221> Variant
   <222> (381)..(381)
   <223> xaa in position 381 is any amino acid
<220>
   <221> Variant
   <222> (383)..(386)
   <223> xaa in position 383 to 386 is any amino acid
<220>
   <221> Variant
   <222> (388)..(393)
   <223> xaa in position 388 to 393 is any amino acid
<220>
   <221> Variant
   <222> (395)..(396)
   <223> xaa in position 395 to 396 is any amino acid
<220>
   <221> variant
   <222> (399)..(400)
   <223> xaa in position 399 to 400 is any amino acid
<400> 57
<210> 58
   <211> 59
   <212> PRT
   <213> Artificial sequence
<220>
   <221> Variant
   <222> (3)..(4)
   <223> xaa in position 3 to 4 is any amino acid
<220>
   <221> Variant
   <222> (7)..(7)
   <223> xaa in position 7 is any amino acid
<220>
   <221> Variant
   <222> (10)..(10)
   <223> xaa in position 10 is any amino acid
<220>
   <221> Variant
   <222> (12)..(12)
   <223> xaa in position 12 is Phe, Leu or Val
<220>
   <221> variant
   <222> (15)..(15)
   <223> xaa in position 15 is any or no amino acid
<220>
   <221> variant
   <222> (17)..(17)
   <223> xaa in position 17 is any or no amino acid
<220>
   <221> Variant
   <222> (19)..(19)
   <223> xaa in position 19 is any amino acid
<220>
   <221> Variant
   <222> (21)..(21)
   <223> xaa in position 21 is any amino acid
<220>
   <221> Variant
   <222> (22)..(22)
   <223> xaa in position 22 is Lys or Arg
<220>
   <221> Variant
   <222> (23)..(23)
   <223> xaa in position 23 is Ile or Met
<220>
   <221> Variant
   <222> (24)..(24)
   <223> xaa in position 24 is any amino acid

<220>
   <221> Variant
   <222> (25)..(25)
   <223> xaa in position 25 is Ala or Glu
<220>
   <221> Variant
   <222> (26)..(26)
   <223> xaa in position 26 is Phe or Val
<220>
   <221> Variant
   <222> (27)..(28)
   <223> xaa in position 27 to 28 is any amino acid
<220>
   <221> Variant
   <222> (30)..(30)
   <223> xaa in position 30 is Phe, Leu or Val
<220>
   <221> Variant
   <222> (32)..(32)
   <223> xaa in position 32 is any amino acid
<220>
   <221> Variant
   <222> (33)..(33)
   <223> xaa in position 33 is Ala or Gly
<220>
   <221> Variant
   <222> (34)..(34)
   <223> xaa in position 34 is any amino acid
<220>
   <221> variant
   <222> (36)..(36)
   <223> xaa in position 36 is Ala or Gly
<220>
   <221> Variant
   <222> (37)..(38)
   <223> xaa in position 37 to 38 is any amino acid
<220>
   <221> Variant
   <222> (40)..(40)
   <223> xaa in position 40 is any amino acid
<220>
   <221> Variant
   <222> (41)..(41)
   <223> xaa in position 41 is Asn or Ser
<220>
   <221> Variant
   <222> (42)..(42)
   <223> xaa in position 42 is any amino acid
<220>
   <221> Variant
   <222> (44)..(44)
   <223> xaa in position 44 is Asn or Ser
<220>
   <221> Variant
   <222> (49)..(49)
   <223> xaa in position 49 is Ala or Thr
<220>
   <221> Variant
   <222> (51)..(51)
   <223> xaa in position 51 is Asn or Gln
<220>
   <221> Variant
   <222> (52)..(52)
   <223> xaa in position 52 is Lys or Arg
<220>
   <221> Variant
   <222> (53)..(53)
   <223> xaa in position 53 is any amino acid
<220>
   <221> Variant
   <222> (54)..(54)
   <223> xaa in position 54 is Glu or Arg
<220>
   <221> Variant
   <222> (57)..(57)
   <223> xaa in position 57 is Met or Val
<400> 58
<210> 59
   <211> 50
   <212> PRT
   <213> Artificial sequence
<220>
   <221> Variant
   <222> (2)..(2)
   <223> xaa in position 2 is Asn or Thr
<220>
   <221> Variant
   <222> (4)..(4)
   <223> xaa in position 4 is any amino acid
<220>
   <221> Variant
   <222> (6)..(6)
   <223> xaa in position 6 is any amino acid
<220>
   <221> Variant
   <222> (7)..(8)
   <223> xaa in position 7 to 8 is any or no amino acid
<220>
   <221> Variant
   <222> (10)..(10)
   <223> xaa in position 10 is any amino acid
<220>
   <221> variant
   <222> (11)..(12)
   <223> xaa in position 11 to 12 is any or no amino acid
<220>
   <221> Variant
   <222> (14)..(14)
   <223> xaa in position 14 is Ile or Val
<220>
   <221> Variant
   <222> (15)..(15)
   <223> xaa in position 15 is any amino acid
<220>
   <221> Variant
   <222> (21)..(21)
   <223> xaa in position 21 is Ser or Thr
<220>
   <221> Variant
   <222> (22)..(22)
   <223> xaa in position 22 is any amino acid
<220>
   <221> Variant
   <222> (27)..(28)
   <223> xaa in position 27 to 28 is any amino acid
<220>
   <221> Variant
   <222> (29)..(29)
   <223> xaa in position 29 is Glu or Gln
<220>
   <221> Variant
   <222> (30)..(30)
   <223> xaa in position 30 is Ile, Leu or Val
<220>
   <221> Variant
   <222> (31)..(31)
   <223> xaa in position 31 is Ala or Ser
<220>
   <221> Variant
   <222> (32)..(32)
   <223> xaa in position 32 is any amino acid
<220>
   <221> Variant
   <222> (34)..(34)
   <223> xaa in position 34 is Phe or val
<220>
   <221> Variant
   <222> (35)..(36)
   <223> xaa in position 35 to 36 is any amino acid
<220>
   <221> Variant
   <222> (37)..(37)
   <223> xaa in position 37 is Phe or Leu
<220>
   <221> Variant
   <222> (38)..(38)
   <223> xaa in position 38 is any amino acid
<220>
   <221> Variant
   <222> (39)..(39)
   <223> xaa in position 39 is Glu or Lys
<220>
   <221> Variant
   <222> (40)..(40)
   <223> xaa in position 40 is Glu, Lys or Arg
<220>
   <221> Variant
   <222> (41)..(41)
   <223> xaa in position 41 is any amino acid
<220>
   <221> Variant
   <222> (42)..(42)
   <223> xaa in position 42 is Gly or Asn
<220>
   <221> Variant
   <222> (44)..(44)
   <223> xaa in position 44 is any amino acid
<220>
   <221> Variant
   <222> (46)..(46)
   <223> xaa in position 46 is any amino acid
<220>
   <221> Variant
   <222> (47)..(47)
   <223> xaa in position 47 is Asp, Ser or Val
<220>
   <221> Variant
   <222> (48)..(48)
   <223> xaa in position 48 is any amino acid
<220>
   <221> Variant
   <222> (49)..(49)
   <223> xaa in position 49 is Pro or Thr
<400> 59
<210> 60
   <211> 20
   <212> PRT
   <213> Artificial sequence
<220>
   <221> Variant
   <222> (2)..(2)
   <223> xaa in position 2 is Asp or Glu
<220>
   <221> Variant
   <222> (5)..(5)
   <223> xaa in position 5 is any amino acid
<220>
   <221> Variant
   <222> (8)..(8)
   <223> xaa in position 8 is Ala, Cys or Val
<220>
   <221> Variant
   <222> (10)..(10)
   <223> xaa in position 10 is Asp or Asn
<220>
   <221> Variant
   <222> (12)..(12)
   <223> xaa in position 12 is Cys or Arg
<220>
   <221> Variant
   <222> (17)..(17)
   <223> xaa in position 17 is any amino acid
<220>
   <221> Variant
   <222> (18)..(18)
   <223> xaa in position 18 is any or no amino acid
<220>
   <221> Variant
   <222> (20)..(20)
   <223> xaa in position 20 is Phe, Ile or Leu
<400> 60
<210> 61
   <211> 61
   <212> PRT
   <213> Artificial sequence
<220>
   <221> Variant
   <222> (2)..(2)
   <223> xaa in position 2 is any amino acid
<220>
   <221> Variant
   <222> (4)..(4)
   <223> xaa in position 4 is Ala or Leu
<220>
   <221> Variant
   <222> (8)..(10)
   <223> xaa in position 8 to 10 is any amino acid
<220>
   <221> Variant
   <222> (11)..(11)
   <223> xaa in position 11 is Ala, Ile or Val
<220>
   <221> Variant
   <222> (12)..(12)
   <223> xaa in position 12 is Ala or Val
<220>
   <221> Variant
   <222> (13)..(13)
   <223> xaa in position 13 is Glu or Arg
<220>
   <221> Variant
   <222> (14)..(14)
   <223> xaa in position 14 is Asp, Lys or Arg
<220>
   <221> Variant
   <222> (15)..(15)
   <223> xaa in position 15 is Gly, Asn or Val
<220>
   <221> Variant
   <222> (16)..(16)
   <223> xaa in position 16 is Lys or Arg
<220>
   <221> Variant
   <222> (17)..(18)
   <223> xaa in position 17 to 18 is any amino acid
<220>
   <221> Variant
   <222> (19)..(19)
   <223> xaa in position 19 is Ala, Gly or Ser
<220>
   <221> Variant
   <222> (20)..(23)
   <223> xaa in position 20 to 23 is any amino acid
<220>
   <221> variant
   <222> (25)..(26)
   <223> xaa in position 25 to 26 is any or no amino acid
<220>
   <221> Variant
   <222> (28)..(29)
   <223> xaa in position 28 to 29 is any or no amino acid
<220>
   <221> Variant
   <222> (31)..(31)
   <223> xaa in position 31 is any amino acid
<220>
   <221> Variant
   <222> (32)..(32)
   <223> xaa in position 32 is Phe, Trp or Tyr
<220>
   <221> Variant
   <222> (33)..(33)
   <223> xaa in position 33 is any amino acid
<220>
   <221> Variant
   <222> (35)..(35)
   <223> xaa in position 35 is Ala, Gly or Ile
<220>
   <221> Variant
   <222> (38)..(38)
   <223> xaa in position 38 is Ser or Thr
<220>
   <221> Variant
   <222> (39)..(39)
   <223> xaa in position 39 is Cys or Ser
<220>
   <221> Variant
   <222> (40)..(40)
   <223> xaa in position 40 is any amino acid
<220>
   <221> Variant
   <222> (42)..(42)
   <223> xaa in position 42 is Ile or Val
<220>
   <221> Variant
   <222> (43)..(43)
   <223> xaa in position 43 is Ala, Gly or Leu
<220>
   <221> Variant
   <222> (45)..(45)
   <223> xaa in position 45 is any amino acid
<220>
   <221> Variant
   <222> (47)..(47)
   <223> xaa in position 47 is any amino acid
<220>
   <221> Variant
   <222> (48)..(48)
   <223> xaa in position 48 is Phe or Leu
<220>
   <221> Variant
   <222> (49)..(49)
   <223> xaa in position 49 is Phe or Tyr
<220>
   <221> Variant
   <222> (52)..(52)
   <223> xaa in position 52 is Pro or Val
<220>
   <221> Variant
   <222> (53)..(53)
   <223> xaa in position 53 is Arg or Ser
<220>
   <221> Variant
   <222> (54)..(55)
   <223> xaa in position 54 to 55 is any amino acid
<220>
   <221> Variant
   <222> (56)..(56)
   <223> xaa in position 56 is Leu or Met
<220>
   <221> Variant
   <222> (57)..(57)
   <223> xaa in position 57 is Lys or Arg
<220>
   <221> Variant
   <222> (58)..(58)
   <223> xaa in position 58 is Gly or Thr
<220>
   <221> Variant
   <222> (59)..(59)
   <223> xaa in position 59 is any amino acid
<220>
   <221> Variant
   <222> (60)..(60)
   <223> xaa in position 60 is Lys or Arg
<220>
   <221> variant
   <222> (61)..(61)
   <223> xaa in position 61 is His or Tyr
<400> 61
<210> 62
   <211> 34
   <212> PRT
   <213> Artificial sequence
<220>
   <221> variant
   <222> (2)..(5)
   <223> xaa in position 2 to 5 is any amino acid
<220>
   <221> Variant
   <222> (6)..(6)
   <223> xaa in position 6 is any or no amino acid
<220>
   <221> Variant
   <222> (8)..(9)
   <223> xaa in position 8 to 9 is any or no amino acid
<220>
   <221> Variant
   <222> (11)..(11)
   <223> xaa in position 11 is Asp or Pro
<220>
   <221> Variant
   <222> (12)..(12)
   <223> xaa in position 12 is Met or val
<220>
   <221> Variant
   <222> (13)..(13)
   <223> xaa in position 13 is any amino acid
<220>
   <221> Variant
   <222> (14)..(14)
   <223> xaa in position 14 is Asn or Pro
<220>
   <221> Variant
   <222> (15)..(15)
   <223> xaa in position 15 is any amino acid
<220>
   <221> Variant
   <222> (16)..(17)
   <223> xaa in position 16 to 17 is Asp or Glu
<220>
   <221> Variant
   <222> (18)..(18)
   <223> xaa in position 18 is any amino acid
<220>
   <221> Variant
   <222> (19)..(19)
   <223> xaa in position 19 is Ala or Leu
<220>
   <221> variant
   <222> (20)..(20)
   <223> xaa in position 20 is any amino acid
<220>
   <221> Variant
   <222> (21)..(21)
   <223> xaa in position 21 is Phe or Trp
<220>
   <221> Variant
   <222> (22)..(22)
   <223> xaa in position 22 is Leu or Val
<220>
   <221> Variant
   <222> (23)..(23)
   <223> xaa in position 23 is Glu or Arg
<220>
   <221> variant
   <222> (26)..(26)
   <223> xaa in position 26 is Ala or Val
<220>
   <221> variant
   <222> (27)..(27)
   <223> xaa in position 27 is any amino acid
<220>
   <221> Variant
   <222> (28)..(28)
   <223> xaa in position 28 is His or Tyr
<220>
   <221> Variant
   <222> (30)..(30)
   <223> xaa in position 30 is Ile, Met or Val
<220>
   <221> Variant
   <222> (31)..(31)
   <223> xaa in position 31 is Asp or Asn
<220>
   <221> Variant
   <222> (32)..(32)
   <223> xaa in position 32 is Ile or Val
<220>
   <221> variant
   <222> (33)..(33)
   <223> xaa in position 33 is Asp, Ser or Thr
<220>
   <221> variant
   <222> (34)..(34)
   <223> xaa in position 34 is Pro or Thr
<400> 62
<210> 63
   <211> 28
   <212> PRT
   <213> Artificial sequence
<220>
   <221> Variant
   <222> (4)..(4)
   <223> xaa in position 4 is Glu or Gln
<220>
   <221> Variant
   <222> (5)..(5)
   <223> xaa in position 5 is Ala or Thr
<220>
   <221> Variant
   <222> (6)..(6)
   <223> xaa in position 6 is Phe or Tyr
<220>
   <221> Variant
   <222> (7)..(7)
   <223> xaa in position 7 is any amino acid
<220>
   <221> Variant
   <222> (8)..(8)
   <223> xaa in position 8 is Glu or Gln
<220>
   <221> Variant
   <222> (11)..(11)
   <223> xaa in position 11 is any amino acid
<220>
   <221> Variant
   <222> (12)..(12)
   <223> xaa in position 12 is Lys or Arg
<220>
   <221> Variant
   <222> (13)..(14)
   <223> xaa in position 13 to 14 is any amino acid
<220>
   <221> Variant
   <222> (15)..(15)
   <223> xaa in position 15 is Cys or Arg
<220>
   <221> Variant
   <222> (17)..(17)
   <223> xaa in position 17 is Asp or Arg
<220>
   <221> Variant
   <222> (19)..(19)
   <223> xaa in position 19 is any amino acid
<220>
   <221> Variant
   <222> (21)..(22)
   <223> xaa in position 21 to 22 is any amino acid
<220>
   <221> Variant
   <222> (25)..(26)
   <223> xaa in position 25 to 26 is any amino acid
<220>
   <221> Variant
   <222> (27)..(27)
   <223> xaa in position 27 is Asp or Pro
<400> 63
<210> 64
   <211> 903
   <212> DNA
   <213> Ostreococcus tauri
<220>
   <221> CDS
   <222> (1)..(903)
<400> 64
   atg tct gct tct gga gct ttg ttg cct gct att gct ttc gct gct tac      48
   Met Ser Ala Ser Gly Ala Leu Leu Pro Ala Ile Ala Phe Ala Ala Tyr
   1               5                   10                  15
   gct tac gct acc tac gct tat gct ttc gag tgg tct cat gct aac gga      96
   Ala Tyr Ala Thr Tyr Ala Tyr Ala Phe Glu Trp Ser His Ala Asn Gly
               20                  25                  30
   atc gat aac gtg gat gct aga gag tgg att gga gct ttg tct ttg aga     144
   Ile Asp Asn Val Asp Ala Arg Glu Trp Ile Gly Ala Leu Ser Leu Arg
               35                  40                  45
   ctc cct gca att gct acc acc atg tac ctc ttg ttc tgc ctt gtg gga     192
   Leu Pro Ala Ile Ala Thr Thr Met Tyr Leu Leu Phe Cys Leu Val Gly
       50                  55                  60
   cct aga ttg atg gct aag agg gag gct ttt gat cct aag gga ttc atg     240
   Pro Arg Leu Met Ala Lys Arg Glu Ala Phe Asp Pro Lys Gly Phe Met
   65                  70                  75                  80
   ctc gct tac aac gct tac caa acc gct ttc aac gtt gtg gtg ctc gga     288
   Leu Ala Tyr Asn Ala Tyr Gln Thr Ala Phe Asn Val Val Val Leu Gly
                   85                  90                  95
   atg ttc gct aga gag atc tct gga ttg gga caa cct gtt tgg gga tct     336
   Met Phe Ala Arg Glu Ile Ser Gly Leu Gly Gln Pro Val Trp Gly Ser
               100                 105                 110
   act atg cct tgg agc gat agg aag tcc ttc aag att ttg ttg gga gtg     384
   Thr Met Pro Trp Ser Asp Arg Lys Ser Phe Lys Ile Leu Leu Gly Val
           115                 120                 125
   tgg ctc cat tac aac aat aag tac ctc gag ttg ttg gat act gtg ttc     432
   Trp Leu His Tyr Asn Asn Lys Tyr Leu Glu Leu Leu Asp Thr Val Phe
       130                 135                 140
   atg gtg gct agg aaa aag acc aag cag ctc tct ttc ttg cat gtg tac     480
   Met Val Ala Arg Lys Lys Thr Lys Gln Leu Ser Phe Leu His Val Tyr
   145                 150                 155                 160
   cat cat gct ttg ttg att tgg gct tgg tgg ctt gtt tgt cat ctc atg     528
   His His Ala Leu Leu Ile Trp Ala Trp Trp Leu Val Cys His Leu Met
                   165                 170                 175
   gct acc aac gat tgc atc gat gct tat ttc gga gct gct tgc aac tct     576
   Ala Thr Asn Asp Cys Ile Asp Ala Tyr Phe Gly Ala Ala Cys Asn Ser
               180                 185                 190
   ttc atc cac atc gtg atg tac tcc tac tac ctc atg tct gct ttg gga     624
   Phe Ile His Ile Val Met Tyr Ser Tyr Tyr Leu Met Ser Ala Leu Gly
           195                 200                 205
   att aga tgc cct tgg aag aga tat atc acc cag gct cag atg ttg caa     672
   Ile Arg Cys Pro Trp Lys Arg Tyr Ile Thr Gln Ala Gln Met Leu Gln
       210                 215                 220
   ttc gtg atc gtg ttc gct cat gct gtt ttc gtg ctc aga caa aag cac     720
   Phe Val Ile Val Phe Ala His Ala Val Phe Val Leu Arg Gln Lys His
   225                 230                 235                 240
   tgc cct gtt act ttg cct tgg gca caa atg ttc gtg atg aca aat atg     768
   Cys Pro Val Thr Leu Pro Trp Ala Gln Met Phe Val Met Thr Asn Met
                   245                 250                 255
   ttg gtg ctc ttc gga aac ttc tac ctc aag gct tac tct aac aag tct     816
   Leu Val Leu Phe Gly Asn Phe Tyr Leu Lys Ala Tyr Ser Asn Lys Ser
               260                 265                 270
   agg gga gat gga gct tct tct gtt aag cct gct gag act act aga gca     864
   Arg Gly Asp Gly Ala Ser Ser Val Lys Pro Ala Glu Thr Thr Arg Ala
           275                 280                 285
   cct tct gtg aga aga acc agg tcc agg aag atc gat tga                 903
   Pro Ser Val Arg Arg Thr Arg Ser Arg Lys Ile Asp
       290                 295                 300
<210> 65
   <211> 300
   <212> PRT
   <213> Ostreococcus tauri
<400> 65
   Met Ser Ala Ser Gly Ala Leu Leu Pro Ala Ile Ala Phe Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Lys Tyr Leu Glu Leu Leu Asp Thr Val Phe
       130                 135                 140
   Met Val Ala Arg Lys Lys Thr Lys Gln Leu Ser Phe Leu His Val Tyr
   145                 150                 155                 160
   His His Ala Leu Leu Ile Trp Ala Trp Trp Leu Val Cys His Leu Met
                   165                 170                 175
   Ala Thr Asn Asp Cys Ile Asp Ala Tyr Phe Gly Ala Ala Cys Asn Ser
               180                 185                 190
   Phe Ile His Ile Val Met Tyr Ser Tyr Tyr Leu Met Ser Ala Leu Gly
           195                 200                 205
   Ile Arg Cys Pro Trp Lys Arg Tyr Ile Thr Gln Ala Gln Met Leu Gln
       210                 215                 220
   Phe Val Ile Val Phe Ala His Ala Val Phe Val Leu Arg Gln Lys His
   225                 230                 235                 240
   Cys Pro Val Thr Leu Pro Trp Ala Gln Met Phe Val Met Thr Asn Met
                   245                 250                 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 66
   <211> 834
   <212> DNA
   <213> Pavlova sp
<220>
   <221> CDS
   <222> (1)..(834)
<400> 66
   atg atg ttg gcc gca ggc tat ctt cta gtg ctc tcg gcc gct cgc cag      48
   Met Met Leu Ala Ala Gly Tyr Leu Leu Val Leu Ser Ala Ala Arg Gln
   1               5                   10                  15
   agc ttc cag cag gac att gac aac ccc aac ggg gcc tac tcg acc tcg      96
   Ser Phe Gln Gln Asp Ile Asp Asn Pro Asn Gly Ala Tyr Ser Thr Ser
               20                  25                  30
   tgg act ggc ctg ccc att gtg atg tct gtg gtc tat ctc agc ggt gtg      144
   Trp Thr Gly Leu Pro Ile Val Met Ser Val Val Tyr Leu Ser Gly Val
           35                  40                  45
   ttt ggg ctc aca aag tac ttc gag aac cgg aag ccc atg acg ggg ctg      192
   Phe Gly Leu Thr Lys Tyr Phe Glu Asn Arg Lys Pro Met Thr Gly Leu
       50                  55                  60
   aag gac tac atg ttc act tac aat ctc tac cag gtg atc atc aac gtg      240
   Lys Asp Tyr Met Phe Thr Tyr Asn Leu Tyr Gln Val Ile Ile Asn Val
   65                  70                  75                  80
   tgg tgc gtg gtg gcc ttt ctc ctg gag gtg cgg cgt gcg ggc atg tca      288
   Trp Cys Val Val Ala Phe Leu Leu Glu Val Arg Arg Ala Gly Met Ser
                   85                  90                  95
   ctc atc ggc aat aag gtg gac ctt ggg ccc aac tcc ttc agg ctc ggc      336
   Leu Ile Gly Asn Lys Val Asp Leu Gly Pro Asn Ser Phe Arg Leu Gly
               100                 105                 110
   ttc gtc acg tgg gtg cac tac aac aac aag tac gtg gag ctc ctc gac      384
   Phe Val Thr Trp Val His Tyr Asn Asn Lys Tyr Val Glu Leu Leu Asp
           115                 120                 125
   acc cta tgg atg gtg ctg cgc aag aag acg cag cag gtc tcc ttc ctc      432
   Thr Leu Trp Met Val Leu Arg Lys Lys Thr Gln Gln Val Ser Phe Leu
       130                 135                 140
   cac gtc tat cat cac gtg ctt ctg atg tgg gcc tgg ttc gtt gtc gtc      480
   His Val Tyr His His Val Leu Leu Met Trp Ala Trp Phe Val Val Val
   145                 150                 155                 160
   aag ctc ggc aat ggt ggt gac gca tat ttt ggc ggt ctc atg aac tcg      528
   Lys Leu Gly Asn Gly Gly Asp Ala Tyr Phe Gly Gly Leu Met Asn Ser
                   165                 170                 175
   atc atc cac gtg atg atg tat tcc tac tac acc atg gcg ctc ctg ggc      576
   Ile Ile His Val Met Met Tyr Ser Tyr Tyr Thr Met Ala Leu Leu Gly
               180                 185                 190
   tgg tca tgc ccc tgg aag cgc tac ctc acg cag gca cag ctc gtg cag      624
   Trp Ser Cys Pro Trp Lys Arg Tyr Leu Thr Gln Ala Gln Leu Val Gln
           195                 200                 205
   ttt tgc atc tgc ctc gcc cac tcc aca tgg gcg gca gta acg ggt gcc      672
   Phe Cys Ile Cys Leu Ala His Ser Thr Trp Ala Ala Val Thr Gly Ala
       210                 215                 220
   tac ccg tgg cga att tgc ttg gtg gag gtg tgg gtg atg gtg tcc atg      720
   Tyr Pro Trp Arg Ile Cys Leu Val Glu Val Trp Val Met Val Ser Met
   225                 230                 235                 240
   ctg gtg ctc ttc aca cgc ttc tac cgc cag gcc tat gcc aag gag gcg      768
   Leu Val Leu Phe Thr Arg Phe Tyr Arg Gln Ala Tyr Ala Lys Glu Ala
                   245                 250                 255
   aag gcc aag gag gcg aaa aag ctc gca cag gag gca tca cag gcc aag      816
   Lys Ala Lys Glu Ala Lys Lys Leu Ala Gln Glu Ala Ser Gln Ala Lys
               260                 265                 270
   gcg gtc aag gcg gag taa                                              834
   Ala Val Lys Ala Glu
           275
<210> 67
   <211> 277
   <212> PRT
   <213> Pavlova sp
<400> 67
   Met Met Leu Ala Ala Gly Tyr Leu Leu Val Leu Ser Ala Ala Arg Gln
   1               5                   10                  15
   Ser Phe Gln Gln Asp Ile Asp Asn Pro Asn Gly Ala Tyr Ser Thr Ser
               20                  25                  30
   Trp Thr Gly Leu Pro Ile Val Met Ser Val Val Tyr Leu Ser Gly Val
           35                  40                  45
   Phe Gly Leu Thr Lys Tyr Phe Glu Asn Arg Lys Pro Met Thr Gly Leu
       50                  55                  60
   Lys Asp Tyr Met Phe Thr Tyr Asn Leu Tyr Gln Val Ile Ile Asn Val
   65                  70                  75                  80
   Trp Cys Val Val Ala Phe Leu Leu Glu Val Arg Arg Ala Gly Met Ser
                   85                  90                  95
   Leu Ile Gly Asn Lys Val Asp Leu Gly Pro Asn Ser Phe Arg Leu Gly
               100                 105                 110
   Phe Val Thr Trp Val His Tyr Asn Asn Lys Tyr Val Glu Leu Leu Asp
           115                 120                 125
   Thr Leu Trp Met Val Leu Arg Lys Lys Thr Gln Gln Val Ser Phe Leu
       130                 135                 140
   His Val Tyr His His Val Leu Leu Met Trp Ala Trp Phe Val Val Val
   145                 150                 155                 160
   Lys Leu Gly Asn Gly Gly Asp Ala Tyr Phe Gly Gly Leu Met Asn Ser
                   165                 170                 175
   Ile Ile His Val Met Met Tyr Ser Tyr Tyr Thr Met Ala Leu Leu Gly
               180                 185                 190
   Trp Ser Cys Pro Trp Lys Arg Tyr Leu Thr Gln Ala Gln Leu Val Gln
           195                 200                 205
   Phe Cys Ile Cys Leu Ala His Ser Thr Trp Ala Ala Val Thr Gly Ala
       210                 215                 220
   Tyr Pro Trp Arg Ile Cys Leu Val Glu Val Trp Val Met Val Ser Met
   225                 230                 235                 240
   Leu Val Leu Phe Thr Arg Phe Tyr Arg Gln Ala Tyr Ala Lys Glu Ala
                   245                 250                 255
   Lys Ala Lys Glu Ala Lys Lys Leu Ala Gln Glu Ala Ser Gln Ala Lys
               260                 265                 270
   Ala Val Lys Ala Glu
           275
<210> 68
   <211> 1077
   <212> DNA
   <213> Thalassiosira pseudonana
<220>
   <221> CDS
   <222> (1)..(1077)
<400> 68
   atg tgc tca tca ccg ccg tca caa tcc aaa aca aca tcc ctc cta gca      48
   Met Cys Ser Ser Pro Pro Ser Gln Ser Lys Thr Thr Ser Leu Leu Ala
   1               5                   10                  15
   cgg tac acc acc gcc gcc ctc ctc ctc ctc acc ctc aca aca tgg tgc      96
   Arg Tyr Thr Thr Ala Ala Leu Leu Leu Leu Thr Leu Thr Thr Trp Cys
               20                  25                  30
   cac ttc gcc ttc cca gcc gcc acc gcc aca ccc ggc ctc acc gcc gaa      144
   His Phe Ala Phe Pro Ala Ala Thr Ala Thr Pro Gly Leu Thr Ala Glu
           35                  40                  45
   atg cac tcc tac aaa gtc cca ctc ggt ctc acc gta ttc tac ctg ctg      192
   Met His Ser Tyr Lys Val Pro Leu Gly Leu Thr Val Phe Tyr Leu Leu
       50                  55                  60
   agt cta ccg tca cta aag tac gtt acg gac aac tac ctt gcc aaa aag      240
   Ser Leu Pro Ser Leu Lys Tyr Val Thr Asp Asn Tyr Leu Ala Lys Lys
   65                  70                  75                  80
   tat gat atg aag tca ctc cta acg gaa tca atg gtg ttg tac aat gtg      288
   Tyr Asp Met Lys Ser Leu Leu Thr Glu Ser Met Val Leu Tyr Asn Val
                   85                  90                  95
   gcg caa gtg ctg ctc aat ggg tgg acg gtg tat gcg att gtg gat gcg      336
   Ala Gln Val Leu Leu Asn Gly Trp Thr Val Tyr Ala Ile Val Asp Ala
               100                 105                 110
   gtg atg aat aga gac cat ccg ttt att gga agt aga agt ttg gtt ggg      384
   Val Met Asn Arg Asp His Pro Phe Ile Gly Ser Arg Ser Leu Val Gly
           115                 120                 125
   gcg gcg ttg cat agt ggg agc tcg tat gcg gtg tgg gtt cat tat tgt      432
   Ala Ala Leu His Ser Gly Ser Ser Tyr Ala Val Trp Val His Tyr Cys
       130                 135                 140
   gat aag tat ttg gag ttc ttt gat acg tat ttt atg gtg ttg agg ggg      480
   Asp Lys Tyr Leu Glu Phe Phe Asp Thr Tyr Phe Met Val Leu Arg Gly
   145                 150                 155                 160
   aaa atg gac cag gtc tcc ttc ctc cac atc tac cac cac acg acc ata      528
   Lys Met Asp Gln Val Ser Phe Leu His Ile Tyr His His Thr Thr Ile
                   165                 170                 175
   gcg tgg gca tgg tgg atc gcc ctc cgc ttc tcc ccc ggt gga gac att      576
   Ala Trp Ala Trp Trp Ile Ala Leu Arg Phe Ser Pro Gly Gly Asp Ile
               180                 185                 190
   tac ttc ggg gca ctc ctc aac tcc atc atc cac gtc ctc atg tat tcc      624
   Tyr Phe Gly Ala Leu Leu Asn Ser Ile Ile His Val Leu Met Tyr Ser
           195                 200                 205
   tac tac gcc ctt gcc cta ctc aag gtc agt tgt cca tgg aaa cga tac      672
   Tyr Tyr Ala Leu Ala Leu Leu Lys Val Ser Cys Pro Trp Lys Arg Tyr
       210                 215                 220
   ctg act caa gct caa tta ttg caa ttc aca agt gtg gtg gtt tat acg      720
   Leu Thr Gln Ala Gln Leu Leu Gln Phe Thr Ser Val Val Val Tyr Thr
   225                 230                 235                 240
   ggg tgt acg ggt tat act cat tac tat cat acg aag cat gga gcg gat      768
   Gly Cys Thr Gly Tyr Thr His Tyr Tyr His Thr Lys His Gly Ala Asp
                   245                 250                 255
   gag aca cag cct agt tta gga acg tat tat ttc tgt tgt gga gtg cag      816
   Glu Thr Gln Pro Ser Leu Gly Thr Tyr Tyr Phe Cys Cys Gly Val Gln
               260                 265                 270
   gtg ttt gag atg gtt agt ttg ttt gta ctc ttt tcc atc ttt tat aaa      864
   Val Phe Glu Met Val Ser Leu Phe Val Leu Phe Ser Ile Phe Tyr Lys
           275                 280                 285
   cga tcc tat tcg aag aag aac aag tca gga gga aag gat agc aag aag      912
   Arg Ser Tyr Ser Lys Lys Asn Lys Ser Gly Gly Lys Asp Ser Lys Lys
       290                 295                 300
   aat gat gat ggg aat aat gag gat caa tgt cac aag gct atg aag gat      960
   Asn Asp Asp Gly Asn Asn Glu Asp Gln Cys His Lys Ala Met Lys Asp
   305                 310                 315                 320
   ata tcg gag ggt gcg aag gag gtt gtg ggg cat gca gcg aag gat gct      1008
   Ile Ser Glu Gly Ala Lys Glu Val Val Gly His Ala Ala Lys Asp Ala
                   325                 330                 335
   gga aag ttg gtg gct acg gcg agt aag gct gta aag agg aag gga act      1056
   Gly Lys Leu Val Ala Thr Ala Ser Lys Ala Val Lys Arg Lys Gly Thr
               340                 345                 350
   cgt gtt act ggt gcc atg tag                                          1077
   Arg Val Thr Gly Ala Met
           355
<210> 69
   <211> 358
   <212> PRT
   <213> Thalassiosira pseudonana
<400> 69
   Met Cys Ser Ser Pro Pro Ser Gln Ser Lys Thr Thr Ser Leu Leu Ala
   1               5                   10                  15
   Arg Tyr Thr Thr Ala Ala Leu Leu Leu Leu Thr Leu Thr Thr Trp Cys
               20                  25                  30
   His Phe Ala Phe Pro Ala Ala Thr Ala Thr Pro Gly Leu Thr Ala Glu
           35                  40                  45
   Met His Ser Tyr Lys Val Pro Leu Gly Leu Thr Val Phe Tyr Leu Leu
       50                  55                  60
   Ser Leu Pro Ser Leu Lys Tyr Val Thr Asp Asn Tyr Leu Ala Lys Lys
   65                  70                  75                  80
   Tyr Asp Met Lys Ser Leu Leu Thr Glu Ser Met Val Leu Tyr Asn Val
                   85                  90                  95
   Ala Gln Val Leu Leu Asn Gly Trp Thr Val Tyr Ala Ile Val Asp Ala
               100                 105                 110
   Val Met Asn Arg Asp His Pro Phe Ile Gly Ser Arg Ser Leu Val Gly
           115                 120                 125
   Ala Ala Leu His Ser Gly Ser Ser Tyr Ala Val Trp Val His Tyr Cys
       130                 135                 140
   Asp Lys Tyr Leu Glu Phe Phe Asp Thr Tyr Phe Met Val Leu Arg Gly
   145                 150                 155                 160
   Lys Met Asp Gln Val Ser Phe Leu His Ile Tyr His His Thr Thr Ile
                   165                 170                 175
   Ala Trp Ala Trp Trp Ile Ala Leu Arg Phe Ser Pro Gly Gly Asp Ile
               180                 185                 190
   Tyr Phe Gly Ala Leu Leu Asn Ser Ile Ile His Val Leu Met Tyr Ser
           195                 200                 205
   Tyr Tyr Ala Leu Ala Leu Leu Lys Val Ser Cys Pro Trp Lys Arg Tyr
       210                 215                 220
   Leu Thr Gln Ala Gln Leu Leu Gln Phe Thr Ser Val Val Val Tyr Thr
   225                 230                 235                 240
   Gly Cys Thr Gly Tyr Thr His Tyr Tyr His Thr Lys His Gly Ala Asp
                   245                 250                 255
   Glu Thr Gln Pro Ser Leu Gly Thr Tyr Tyr Phe Cys Cys Gly Val Gln
               260                 265                 270
   Val Phe Glu Met Val Ser Leu Phe Val Leu Phe Ser Ile Phe Tyr Lys
           275                 280                 285
   Arg Ser Tyr Ser Lys Lys Asn Lys Ser Gly Gly Lys Asp Ser Lys Lys
       290                 295                 300
   Asn Asp Asp Gly Asn Asn Glu Asp Gln Cys His Lys Ala Met Lys Asp
   305                 310                 315                 320
   Ile Ser Glu Gly Ala Lys Glu Val Val Gly His Ala Ala Lys Asp Ala
                   325                 330                 335
   Gly Lys Leu Val Ala Thr Ala Ser Lys Ala Val Lys Arg Lys Gly Thr
               340                 345                 350
   Arg Val Thr Gly Ala Met
           355
<210> 70
   <211> 903
   <212> DNA
   <213> Ostreococcus tauri
<220>
   <221> CDS
   <222> (1)..(903)
<400> 70
   atg agc gcc tcc ggt gcg ctg ctg ccc gcg atc gcg tcc gcc gcg tac      48
   Met Ser Ala Ser Gly Ala Leu Leu Pro Ala Ile Ala Ser Ala Ala Tyr
   1               5                   10                  15
   gcg tac gcg acg tac gcc tac gcc ttt gag tgg tcg cac gcg aat ggc      96
   Ala Tyr Ala Thr Tyr Ala Tyr Ala Phe Glu Trp Ser His Ala Asn Gly
               20                  25                  30
   atc gac aac gtc gac gcg cgc gag tgg atc ggt gcg ctg tcg ttg agg      144
   Ile Asp Asn Val Asp Ala Arg Glu Trp Ile Gly Ala Leu Ser Leu Arg
           35                  40                  45
   ctc ccg gcg atc gcg acg acg atg tac ctg ttg ttc tgc ctg gtc gga      192
   Leu Pro Ala Ile Ala Thr Thr Met Tyr Leu Leu Phe Cys Leu Val Gly
       50                  55                  60
   ccg agg ttg atg gcg aag cgc gag gcg ttc gac ccg aag ggg ttc atg      240
   Pro Arg Leu Met Ala Lys Arg Glu Ala Phe Asp Pro Lys Gly Phe Met
   65                  70                  75                  80
   ctg gcg tac aat gcg tat cag acg gcg ttc aac gtc gtc gtg ctc ggg      288
   Leu Ala Tyr Asn Ala Tyr Gln Thr Ala Phe Asn Val Val Val Leu Gly
                   85                  90                  95
   atg ttc gcg cga gag atc tcg ggg ctg ggg cag ccc gtg tgg ggg tca      336
   Met Phe Ala Arg Glu Ile Ser Gly Leu Gly Gln Pro Val Trp Gly Ser
               100                 105                 110
   acc atg ccg tgg agc gat aga aaa tcg ttt aag atc ctc ctc ggg gtg      384
   Thr Met Pro Trp Ser Asp Arg Lys Ser Phe Lys Ile Leu Leu Gly Val
           115                 120                 125
   tgg ttg cac tac aac aac aaa tat ttg gag cta ttg gac act gtg ttc      432
   Trp Leu His Tyr Asn Asn Lys Tyr Leu Glu Leu Leu Asp Thr Val Phe
       130                 135                 140
   atg gtt gcg cgc aag aag acg aag cag ttg agc ttc ttg cac gtt tat      480
   Met Val Ala Arg Lys Lys Thr Lys Gln Leu Ser Phe Leu His Val Tyr
   145                 150                 155                 160
   cat cac gcc ctg ttg atc tgg gcg tgg tgg ttg gtg tgt cac ttg atg      528
   His His Ala Leu Leu Ile Trp Ala Trp Trp Leu Val Cys His Leu Met
                   165                 170                 175
   gcc acg aac gat tgt atc gat gcc tac ttc ggc gcg gcg tgc aac tcg      576
   Ala Thr Asn Asp Cys Ile Asp Ala Tyr Phe Gly Ala Ala Cys Asn Ser
               180                 185                 190
   ttc att cac atc gtg atg tac tcg tat tat ctc atg tcg gcg ctc ggc      624
   Phe Ile His Ile Val Met Tyr Ser Tyr Tyr Leu Met Ser Ala Leu Gly
           195                 200                 205
   att cga tgc ccg tgg aag cga tac atc acc cag gct caa atg ctc caa      672
   Ile Arg Cys Pro Trp Lys Arg Tyr Ile Thr Gln Ala Gln Met Leu Gln
       210                 215                 220
   ttc gtc att gtc ttc gcg cac gcc gtg ttc gtg ctg cgt cag aag cac      720
   Phe Val Ile Val Phe Ala His Ala Val Phe Val Leu Arg Gln Lys His
   225                 230                 235                 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg      816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg      864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                  903
                   Pro Ser Val Arg Arg Thr Arg Ser Arg Lys Ile Asp
                       290                 295                 300
<210> 71
   <211> 300
   <212> PRT
   <213> Ostreococcus tauri
<400> 71
   Met Ser Ala Ser Gly Ala Leu Leu Pro Ala Ile Ala Ser Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Lys Tyr Leu Glu Leu Leu Asp Thr Val Phe
       130                 135                 140
   Met Val Ala Arg Lys Lys Thr Lys Gln Leu Ser Phe Leu His Val Tyr
   145                 150                 155                 160
   His His Ala Leu Leu Ile Trp Ala Trp Trp Leu Val Cys His Leu Met
                   165                 170                 175
   Ala Thr Asn Asp Cys Ile Asp Ala Tyr Phe Gly Ala Ala Cys Asn Ser
               180                 185                 190
   Phe Ile His Ile Val Met Tyr Ser Tyr Tyr Leu Met Ser Ala Leu Gly
           195                 200                 205
   Ile Arg Cys Pro Trp Lys Arg Tyr Ile Thr Gln Ala Gln Met Leu Gln
       210                 215                 220
   Phe Val Ile Val Phe Ala His Ala Val Phe Val Leu Arg Gln Lys His
   225                 230                 235                 240
   Cys Pro Val Thr Leu Pro Trp Ala Gln Met Phe Val Met Thr Asn Met
                   245                 250                 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 72
   <211> 339
   <212> PRT
   <213> Artificial sequence
<220>
   <221> Variant
   <222> (3)..(29)
   <223> xaa in position 3 to 29 is any amino acid
<220>
   <221> Variant
   <222> (30)..(43)
   <223> xaa in position 30 to 43 is any or no amino acid
<220>
   <221> Variant
   <222> (45)..(50)
   <223> xaa in position 45 to 50 is any amino acid
<220>
   <221> Variant
   <222> (53)..(73)
   <223> xaa in position 53 to 73 is any amino acid
<220>
   <221> Variant
   <222> (74)..(79)
   <223> xaa in position 74 to 79 is any or no amino acid
<220>
   <221> Variant
   <222> (81)..(82)
   <223> xaa in position 81 to 82 is any amino acid
<220>
   <221> Variant
   <222> (85)..(86)
   <223> xaa in position 85 to 86 is any amino acid
<220>
   <221> Variant
   <222> (88)..(90)
   <223> xaa in position 88 to 90 is any amino acid
<220>
   <221> Variant
   <222> (92)..(110)
   <223> xaa in position 92 to 110 is any amino acid
<220>
   <221> Variant
   <222> (112)..(127)
   <223> xaa in position 112 to 127 is any amino acid
<220>
   <221> Variant
   <222> (128)..(128)
   <223> xaa in position 128 is any or no amino acid
<220>
   <221> Variant
   <222> (130)..(130)
   <223> xaa in position 130 is any amino acid
<220>
   <221> Variant
   <222> (133)..(134)
   <223> xaa in position 133 to 134 is any amino acid
<220>
   <221> Variant
   <222> (137)..(137)
   <223> xaa in position 137 is any amino acid
<220>
   <221> Variant
   <222> (139)..(140)
   <223> xaa in position 139 to 140 is any amino acid
<220>
   <221> Variant
   <222> (143)..(144)
   <223> xaa in position 143 to 144 is any amino acid
<220>
   <221> Variant
   <222> (147)..(147)
   <223> xaa in position 147 is any amino acid
<220>
   <221> Variant
   <222> (149)..(149)
   <223> xaa in position 149 is any amino acid
<220>
   <221> Variant
   <222> (151)..(152)
   <223> xaa in position 151 to 152 is any amino acid
<220>
   <221> Variant
   <222> (154)..(154)
   <223> xaa in position 154 is any amino acid
<220>
   <221> Variant
   <222> (159)..(159)
   <223> xaa in position 159 is any amino acid
<220>
   <221> Variant
   <222> (163)..(166)
   <223> xaa in position 163 to 166 is any amino acid
<220>
   <221> Variant
   <222> (170)..(179)
   <223> xaa in position 170 to 179 is any amino acid
<220>
   <221> Variant
   <222> (180)..(182)
   <223> xaa in position 180 to 182 is any or no amino acid
<220>
   <221> Variant
   <222> (184)..(184)
   <223> xaa in position 184 is any amino acid
<220>
   <221> Variant
   <222> (188)..(190)
   <223> xaa in position 188 to 190 is any amino acid
<220>
   <221> Variant
   <222> (193)..(193)
   <223> xaa in position 193 is any amino acid
<220>
   <221> Variant
   <222> (196)..(197)
   <223> xaa in position 196 to 197 is any amino acid
<220>
   <221> Variant
   <222> (203)..(206)
   <223> xaa in position 203 to 206 is any amino acid
<220>
   <221> Variant
   <222> (208)..(210)
   <223> xaa in position 208 to 210 is any amino acid
<220>
   <221> Variant
   <222> (217)..(217)
   <223> xaa in position 217 is any amino acid
<220>
   <221> Variant
   <222> (222)..(223)
   <223> xaa in position 222 to 223 is any amino acid
<220>
   <221> Variant
   <222> (226)..(252)
   <223> xaa in position 226 to 252 is any amino acid
<220>
   <221> Variant
   <222> (253)..(267)
   <223> xaa in position 253 to 267 is any or no amino acid
<220>
   <221> Variant
   <222> (269)..(272)
   <223> xaa in position 269 to 272 is any amino acid
<220>
   <221> Variant
   <222> (276)..(277)
   <223> xaa in position 276 to 277 is any amino acid
<220>
   <221> Variant
   <222> (280)..(282)
   <223> xaa in position 280 to 282 is any amino acid
<220>
   <221> Variant
   <222> (284)..(291)
   <223> xaa in position 284 to 291 is any amino acid
<220>
   <221> Variant
   <222> (292)..(324)
   <223> xaa in position 292 to 324 is any or no amino acid
<220>
   <221> Variant
   <222> (326)..(337)
   <223> xaa in position 326 to 337 is any amino acid
<220>
   <221> Variant
   <222> (338)..(338)
   <223> xaa in position 338 is any or no amino acid
<400> 72
<210> 73
   <211> 45
   <212> PRT
   <213> Artificial sequence
<220>
   <221> Variant
   <222> (4)..(4)
   <223> xaa in position 4 is Ala or Gly
<220>
   <221> Variant
   <222> (5)..(5)
   <223> xaa in position 5 is Ala or Leu
<220>
   <221> Variant
   <222> (6)..(6)
   <223> xaa in position 6 is any amino acid
<220>
   <221> Variant
   <222> (9)..(9)
   <223> xaa in position 9 is Phe or Ile
<220>
   <221> Variant
   <222> (12)..(12)
   <223> xaa in position 12 is any or no amino acid
<220>
   <221> Variant
   <222> (14)..(14)
   <223> xaa in position 14 is any or no amino acid
<220>
   <221> Variant
   <222> (20)..(20)
   <223> xaa in position 20 is any amino acid
<220>
   <221> Variant
   <222> (21)..(21)
   <223> xaa in position 21 is Leu or Met
<220>
   <221> Variant
   <222> (22)..(22)
   <223> xaa in position 22 is Ala or Ser
<220>
   <221> Variant
   <222> (23)..(23)
   <223> xaa in position 23 ins Ala or Leu
<220>
   <221> Variant
   <222> (25)..(26)
   <223> xaa in position 25 to 26 is any amino acid
<220>
   <221> Variant
   <222> (27)..(27)
   <223> xaa in position 27 is Arg or Ser
<220>
   <221> Variant
   <222> (34)..(34)
   <223> xaa in position 34 is Ile or Leu
<220>
   <221> Variant
   <222> (39)..(39)
   <223> xaa in position 39 is Leu or Met
<220>
   <221> Variant
   <222> (40)..(40)
   <223> xaa in position 40 is Leu or Val
<220>
   <221> Variant
   <222> (43)..(43)
   <223> xaa in position 43 is Cys, Thr or Val
<220>
   <221> Variant
   <222> (44)..(44)
   <223> xaa in position 44 is any amino acid
<220>
   <221> Variant
   <222> (45)..(45)
   <223> xaa in position 45 is Cys or Val

<400> 73
   Tyr Phe Gly xaa xaa xaa Asn Ser xaa Ile His xaa Val xaa Met Tyr
    1               5                  10                  15
   Ser Tyr Tyr xaa xaa xaa xaa Leu xaa xaa xaa Cys Pro Trp Lys Arg
               20                  25                  30
   Tyr xaa Thr Gln Ala Gln xaa xaa Gln Phe xaa xaa xaa
           35                  40                  45
<210> 74
   <211> 43
   <212> PRT
   <213> Artificial sequence
<220>
   <221> Variant
   <222> (2)..(2)
   <223> xaa in position 2 is Leu or Val
<220>
   <221> variant
   <222> (5)..(5)
   <223> xaa in position 5 is Cys or Asn
<220>
   <221> Variant
   <222> (6)..(6)
   <223> xaa in position 6 is Asp or Asn
<220>
   <221> Variant
   <222> (9)..(10)
   <223> xaa in position 9 to 10 is any or no amino acid
<220>
   <221> variant
   <222> (12)..(12)
   <223> xaa in position 12 is any amino acid
<220>
   <221> Variant
   <222> (13)..(14)
   <223> xaa in position 13 to 14 is any or no amino acid
<220>
   <221> Variant
   <222> (17)..(17)
   <223> xaa in position 17 is any amino acid
<220>
   <221> Variant
   <222> (18)..(18)
   <223> xaa in position 18 is Phe or Trp
<220>
   <221> Variant
   <222> (21)..(21)
   <223> xaa in position 21 is Ala or Leu
<220>
   <221> Variant
   <222> (23)..(23)
   <223> xaa in position 23 is any amino acid
<220>
   <221> Variant
   <222> (25)..(26)
   <223> xaa in position 25 to 26 is any amino acid
<220>
   <221> Variant
   <222> (28)..(28)
   <223> xaa in position 28 is Leu or Val
<220>
   <221> Variant
   <222> (33)..(33)
   <223> xaa in position 33 is Ile or Val
<220>
   <221> Variant
   <222> (37)..(37)
   <223> xaa in position 37 is Ala, Thr or Val
<220>
   <221> Variant
   <222> (38)..(38)
   <223> xaa in position 38 is any amino acid
<220>
   <221> Variant
   <222> (39)..(39)
   <223> xaa in position 39 is Ile or Leu
<220>
   <221> Variant
   <222> (40)..(40)
   <223> xaa in position 40 is any amino acid
<400> 74
<210> 75
   <211> 37
   <212> PRT
   <213> Artificial sequence
<220>
   <221> Variant
   <222> (2)..(3)
   <223> xaa in position 2 to 3 is any or no amino acid
<220>
   <221> Variant
   <222> (5)..(5)
   <223> xaa in position 5 is any or no amino acid
<220>
   <221> Variant
   <222> (9)..(9)
   <223> xaa in position 9 is Gly, Ser or Thr
<220>
   <221> Variant
   <222> (10)..(10)
   <223> xaa in position 10 is any amino acid
<220>
   <221> variant
   <222> (13)..(14)
   <223> xaa in position 13 to 14 is any amino acid
<220>
   <221> Variant
   <222> (15)..(15)
   <223> xaa in position 15 is Ala or Ser
<220>
   <221> Variant
   <222> (17)..(17)
   <223> xaa in position 17 is Ala or Ser
<220>
   <221> Variant
   <222> (18)..(18)
   <223> xaa in position 18 is any amino acid
<220>
   <221> Variant
   <222> (19)..(19)
   <223> xaa in position 19 is Glu or Lys
<220>
   <221> Variant
   <222> (20)..(20)
   <223> xaa in position 20 is Ala, Asn or Ser
<220>
   <221> Variant
   <222> (21)..(21)
   <223> xaa in position 21 is Lys or Arg
<220>
   <221> Variant
   <222> (22)..(22)
   <223> xaa in position 22 is Ala, Gly or Ser
<220>
   <221> Variant
   <222> (23)..(23)
   <223> xaa in position 23 is any amino acid
<220>
   <221> Variant
   <222> (24)..(24)
   <223> xaa in position 24 is Glu or Gly
<220>
   <221> Variant
   <222> (25)..(29)
   <223> xaa in position 25 to 29 is any amino acid
<220>
   <221> Variant
   <222> (30)..(30)
   <223> xaa in position 30 is Asn, Pro or Gln
<220>
   <221> Variant
   <222> (31)..(32)
   <223> xaa in position 31 to 32 is Ala, Asp or Glu
<220>
   <221> Variant
   <222> (33)..(33)
   <223> xaa in position 33 is Gly, Ser or Thr
<220>
   <221> Variant
   <222> (34)..(34)
   <223> xaa in position 34 is Asn, Gln or Thr
<220>
   <221> Variant
   <222> (35)..(36)
   <223> xaa in position 35 to 36 is any amino acid
<220>
   <221> Variant
   <222> (37)..(37)
   <223> xaa in position 37 is Ala, Asp or Pro
<400> 75
<210> 76
   <211> 19
   <212> PRT
   <213> Artificial sequence
<220>
   <221> Variant
   <222> (2)..(3)
   <223> xaa in position 2 to 3 is any amino acid
<220>
   <221> Variant
   <222> (4)..(7)
   <223> xaa in position 4 to 7 is any or no amino acid
<220>
   <221> Variant
   <222> (9)..(9)
   <223> xaa in position 9 is Phe, Leu or Val
<220>
   <221> Variant
   <222> (10)..(10)
   <223> xaa in position 10 is any amino acid
<220>
   <221> Variant
   <222> (13)..(13)
   <223> xaa in position 13 is Ala, Leu or Val
<220>
   <221> Variant
   <222> (14)..(14)
   <223> xaa in position 14 is any amino acid
<220>
   <221> Variant
   <222> (16)..(16)
   <223> xaa in position 16 is Thr or Val
<220>
   <221> variant
   <222> (17)..(17)
   <223> xaa in position 17 is Ala, Ile or Leu
<220>
   <221> variant
   <222> (18)..(18)
   <223> xaa in position 18 is Phe, Ile or Leu
<400> 76
<210> 77
   <211> 1560
   <212> DNA
   <213> Traustochytrium sp.
<220>
   <221> CDS
   <222> (1)..(1560)
<400> 77
   atg acg gtc ggc tac gac gag gag atc ccg ttc gag cag gtc cgc gcg      48
   Met Thr Val Gly Tyr Asp Glu Glu Ile Pro Phe Glu Gln Val Arg Ala
   1               5                   10                  15
   cac aac aag ccg gat gac gcc tgg tgc gcg atc cac ggg cac gtg tac      96
   His Asn Lys Pro Asp Asp Ala Trp Cys Ala Ile His Gly His Val Tyr
               20                  25                  30
   gat gtg acc aag ttc gcg agc gtg cac ccg ggc ggc gac att atc ctg      144
   Asp Val Thr Lys Phe Ala Ser Val His Pro Gly Gly Asp Ile Ile Leu
           35                  40                  45
   ctg gcc gca ggc aag gag gcc acc gtg ctg tac gag act tac cat gtg      192
   Leu Ala Ala Gly Lys Glu Ala Thr Val Leu Tyr Glu Thr Tyr His Val
       50                  55                  60
   cgg ggc gtc tcg gac gcg gtg ctg cgc aag tac cgc atc ggc aag ctg      240
   Arg Gly Val Ser Asp Ala Val Leu Arg Lys Tyr Arg Ile Gly Lys Leu
   65                  70                  75                  80
   ccg gac ggc caa ggc ggc gcg aac gag aag gaa aag cgg acg ctc tcg      288
   Pro Asp Gly Gln Gly Gly Ala Asn Glu Lys Glu Lys Arg Thr Leu Ser
                   85                  90                  95
   ggc ctc tcg tcg gcc tcg tac tac acg tgg aac agc gac ttt tac agg      336
   Gly Leu Ser Ser Ala Ser Tyr Tyr Thr Trp Asn Ser Asp Phe Tyr Arg
   100 105 110
   gta atg cgc gag cgc gtc gtg gct cgg ctc aag gag cgc ggc aag gcc 384
   Val Met Arg Glu Arg Val Val Ala Arg Leu Lys Glu Arg Gly Lys Ala
   115 120 125
   cgc cgc gga ggc tac gag ctc tgg atc aag gcg ttc ctg ctg ctc gtc 432
   Arg Arg Gly Gly Tyr Glu Leu Trp Ile Lys Ala Phe Leu Leu Leu Val
   130 135 140
   ggc ttc tgg agc tcg ctg tac tgg atg tgc acg ctg gac ccc tcg ttc 480
   Gly Phe Trp Ser Ser Leu Tyr Trp Met Cys Thr Leu Asp Pro Ser Phe
   145 150 155 160
   ggg gcc atc ctg gcc gcc atg tcg ctg ggc gtc ttt gcc gcc ttt gtg 528
   Gly Ala Ile Leu Ala Ala Met Ser Leu Gly Val Phe Ala Ala Phe Val
   165 170 175
   ggc acg tgc atc cag cac gac ggc aac cac ggc gcc ttt gcc cag tcg 576
   Gly Thr Cys Ile Gln His Asp Gly Asn His Gly Ala Phe Ala Gln Ser
   180 185 190
   cga tgg gtc aac aag gtt gcc ggg tgg acg ctc gac atg atc ggc gcc 624
   Arg Trp Val Asn Lys Val Ala Gly Trp Thr Leu Asp Met Ile Gly Ala
   195 200 205
   agc ggc atg acg tgg gag ttc cag cac gtc ctg ggc cac cat ccg tac 672
   Ser Gly Met Thr Trp Glu Phe Gln His Val Leu Gly His His Pro Tyr
   210 215 220
   acg aac ctg atc gag gag gag aac ggc ctg caa aag gtg agc ggc aag 720
   Thr Asn Leu Ile Glu Glu Glu Asn Gly Leu Gln Lys Val Ser Gly Lys
   225 230 235 240
   aag atg gac acc aag ctg gcc gac cag gag agc gat ccg gac gtc ttt 768
   Lys Met Asp Thr Lys Leu Ala Asp Gln Glu Ser Asp Pro Asp Val Phe
   245 250 255
   tcc acg tac ccg atg atg cgc ctg cac ccg tgg cac cag aag cgc tgg 816
   Ser Thr Tyr Pro Met Met Arg Leu His Pro Trp His Gln Lys Arg Trp
   260 265 270
   tac cac cgt ttc cag cac att tac ggc ccc ttc atc ttt ggc ttc atg 864
   Tyr His Arg Phe Gln His Ile Tyr Gly Pro Phe Ile Phe Gly Phe Met
   275 280 285
   acc atc aac aag gtg gtc acg cag gac gtc ggt gtg gtg ctc cgc aag 912
   Thr Ile Asn Lys Val Val Thr Gln Asp Val Gly Val Val Leu Arg Lys
   290 295 300
   cgg ctc ttc cag att gac gcc gag tgc cgg tac gcg agc cca atg tac 960
   Arg Leu Phe Gln Ile Asp Ala Glu Cys Arg Tyr Ala Ser Pro Met Tyr
   305 310 315 320
   gtg gcg cgt ttc tgg atc atg aag gcg ctc acg gtg ctc tac atg gtg 1008
   Val Ala Arg Phe Trp Ile Met Lys Ala Leu Thr Val Leu Tyr Met Val
   325 330 335
   gcc ctg ccg tgc tac atg cag ggc ccg tgg cac ggc ctc aag ctg ttc 1056
   Ala Leu Pro Cys Tyr Met Gln Gly Pro Trp His Gly Leu Lys Leu Phe
   340 345 350
   gcg atc gcg cac ttt acg tgc ggc gag gtg ctc gca acc atg ttc att 1104
   Ala Ile Ala His Phe Thr Cys Gly Glu Val Leu Ala Thr Met Phe Ile
   355 360 365
   gtg aac cac atc atc gag ggc gtc tcg tac gct tcc aag gac gcg gtc 1152
   Val Asn His Ile Ile Glu Gly Val Ser Tyr Ala Ser Lys Asp Ala Val
   370 375 380
   aag ggc acg atg gcg ccg ccg aag acg atg cac ggc gtg acg ccc atg 1200
   Lys Gly Thr Met Ala Pro Pro Lys Thr Met His Gly Val Thr Pro Met
   385 390 395 400
   aac aac acg cgc aag gag gtg gag gcg gag gcg tcc aag tct ggc gcc 1248
   Asn Asn Thr Arg Lys Glu Val Glu Ala Glu Ala Ser Lys Ser Gly Ala
   405 410 415
   gtg gtc aag tca gtc ccg ctc gac gac tgg gcc gcc gtc cag tgc cag 1296
   Val Val Lys Ser Val Pro Leu Asp Asp Trp Ala Ala Val Gln Cys Gln
   420 425 430
   acc tcg gtg aac tgg agc gtc ggc tcg tgg ttc tgg aat cac ttt tcc 1344
   Thr Ser Val Asn Trp Ser Val Gly Ser Trp Phe Trp Asn His Phe Ser
   435 440 445
   ggc ggc ctc aac cac cag att gag cac cac ctg ttc ccc ggg ctc agc 1392
   Gly Gly Leu Asn His Gln Ile Glu His His Leu Phe Pro Gly Leu Ser
   450 455 460
   cac gag acg tac tac cac atc cag gac gtc gtt cag tcc acc tgc gcc        1440
   His Glu Thr Tyr Tyr His Ile Gln Asp Val Val Gln Ser Thr Cys Ala
   465                 470                 475                 480
   gag tac ggc gtc ccg tac cag cac gag cct tcg ctc tgg acc gcg tac        1488
   Glu Tyr Gly Val Pro Tyr Gln His Glu Pro Ser Leu Trp Thr Ala Tyr
                   485                 490                 495
   tgg aag atg ctc gag cac ctc cgt cag ctc ggc aat gag gag acc cac        1536
   Trp Lys Met Leu Glu His Leu Arg Gln Leu Gly Asn Glu Glu Thr His
               500                 505                 510
   gag tcc tgg cag cgc gct gcc tga                                        1560
   Glu Ser Trp Gln Arg Ala Ala
           515                 520
<210> 78
   <211> 519
   <212> PRT
   <213> Traustochytrium sp.
<400> 78
   Met Thr Val Gly Tyr Asp Glu Glu Ile Pro Phe Glu Gln Val Arg Ala
   1               5                   10                  15
   His Asn Lys Pro Asp Asp Ala Trp Cys Ala Ile His Gly His Val Tyr
               20                  25                  30
   Asp Val Thr Lys Phe Ala Ser Val His Pro Gly Gly Asp Ile Ile Leu
           35                  40                  45
   Leu Ala Ala Gly Lys Glu Ala Thr Val Leu Tyr Glu Thr Tyr His Val
       50                  55                  60
   Arg Gly Val Ser Asp Ala Val Leu Arg Lys Tyr Arg Ile Gly Lys Leu
   65                  70                  75                  80
   Pro Asp Gly Gln Gly Gly Ala Asn Glu Lys Glu Lys Arg Thr Leu Ser
                   85                  90                  95
   Gly Leu Ser Ser Ala Ser Tyr Tyr Thr Trp Asn Ser Asp Phe Tyr Arg
               100                 105                 110
   Val Met Arg Glu Arg Val Val Ala Arg Leu Lys Glu Arg Gly Lys Ala
           115                 120                 125
   Arg Arg Gly Gly Tyr Glu Leu Trp Ile Lys Ala Phe Leu Leu Leu Val
       130                 135                 140
   Gly Phe Trp Ser Ser Leu Tyr Trp Met Cys Thr Leu Asp Pro Ser Phe
   145                 150                 155                 160
   Gly Ala Ile Leu Ala Ala Met Ser Leu Gly Val Phe Ala Ala Phe Val
                   165                 170                 175
   Gly Thr Cys Ile Gln His Asp Gly Asn His Gly Ala Phe Ala Gln Ser
               180                 185                 190
   Arg Trp Val Asn Lys Val Ala Gly Trp Thr Leu Asp Met Ile Gly Ala
           195                 200                 205
   Ser Gly Met Thr Trp Glu Phe Gln His Val Leu Gly His His Pro Tyr
       210                 215                 220
   Thr Asn Leu Ile Glu Glu Glu Asn Gly Leu Gln Lys Val Ser Gly Lys
   225                 230                 235                 240
   Lys Met Asp Thr Lys Leu Ala Asp Gln Glu Ser Asp Pro Asp Val Phe
                   245                 250                 255
   Ser Thr Tyr Pro Met Met Arg Leu His Pro Trp His Gln Lys Arg Trp
               260                 265                 270
   Tyr His Arg Phe Gln His Ile Tyr Gly Pro Phe Ile Phe Gly Phe Met
           275                 280                 285
   Thr Ile Asn Lys Val Val Thr Gln Asp Val Gly Val Val Leu Arg Lys
       290                 295                 300
   Arg Leu Phe Gln Ile Asp Ala Glu Cys Arg Tyr Ala Ser Pro Met Tyr
   305                 310                 315                 320
   Val Ala Arg Phe Trp Ile Met Lys Ala Leu Thr Val Leu Tyr Met Val
                   325                 330                 335
   Ala Leu Pro Cys Tyr Met Gln Gly Pro Trp His Gly Leu Lys Leu Phe
               340                 345                 350
   Ala Ile Ala His Phe Thr Cys Gly Glu Val Leu Ala Thr Met Phe Ile
           355                 360                 365
   Val Asn His Ile Ile Glu Gly Val Ser Tyr Ala Ser Lys Asp Ala Val
       370                 375                 380
   Lys Gly Thr Met Ala Pro Pro Lys Thr Met His Gly Val Thr Pro Met
   385                 390                 395                 400
   Asn Asn Thr Arg Lys Glu Val Glu Ala Glu Ala Ser Lys Ser Gly Ala
                   405                 410                 415
   Val Val Lys Ser Val Pro Leu Asp Asp Trp Ala Ala Val Gln Cys Gln
               420                 425                 430
   Thr Ser Val Asn Trp Ser Val Gly Ser Trp Phe Trp Asn His Phe Ser
           435                 440                 445
   Gly Gly Leu Asn His Gln Ile Glu His His Leu Phe Pro Gly Leu Ser
       450                 455                 460
   His Glu Thr Tyr Tyr His Ile Gln Asp Val Val Gln Ser Thr Cys Ala
   465                 470                 475                 480
   Glu Tyr Gly Val Pro Tyr Gln His Glu Pro Ser Leu Trp Thr Ala Tyr
                   485                 490                 495
   Trp Lys Met Leu Glu His Leu Arg Gln Leu Gly Asn Glu Glu Thr His
               500                 505                 510
   Glu Ser Trp Gln Arg Ala Ala
           515
<210> 79
   <211> 1560
   <212> DNA
   <213> Thraustochytrium sp
<220>
   <221> CDS
   <222> (1)..(1560)
<400> 79
   atg acg gtc ggc tac gac ggg gag atc ccg ttc gag cag gtc cgc gcg      48
   Met Thr Val Gly Tyr Asp Gly Glu Ile Pro Phe Glu Gln Val Arg Ala
   1               5                   10                  15
   cac aac aag ccg gat gac gcc tgg tgc gcg atc cac ggg cac gtg tac      96
   His Asn Lys Pro Asp Asp Ala Trp Cys Ala Ile His Gly His Val Tyr
               20                  25                  30
   gat gtg acc aag ttc gcg agc gtg cac ccg ggc ggc gac att atc ctg     144
   Asp Val Thr Lys Phe Ala Ser Val His Pro Gly Gly Asp Ile Ile Leu
           35                  40                  45
   ctg gcc gca ggc aag gat gcc acc gtg ctg tac gag act tac cat gtg     192
   Leu Ala Ala Gly Lys Asp Ala Thr Val Leu Tyr Glu Thr Tyr His Val
       50                  55                  60
   cgg ggc gtc tcg gac gcg gtg ctg cgc aag tac cgc atc ggc aag ctg     240
   Arg Gly Val Ser Asp Ala Val Leu Arg Lys Tyr Arg Ile Gly Lys Leu
   65                  70                  75                  80
   ccg gac ggc caa ggc ggc gcg aac gag aag gaa aag cgg acg ctc tcg     288
   Pro Asp Gly Gln Gly Gly Ala Asn Glu Lys Glu Lys Arg Thr Leu Ser
                   85                  90                  95
   ggc ctc tcg tcg gcc tcg tac tac acg tgg aac agc gac ttt tac agg     336
   Gly Leu Ser Ser Ala Ser Tyr Tyr Thr Trp Asn Ser Asp Phe Tyr Arg
               100                 105                 110
   gta atg cgc gag cgc gtc gtg gct cgg ctc aag gag cgc ggc aag gcc     384
   Val Met Arg Glu Arg Val Val Ala Arg Leu Lys Glu Arg Gly Lys Ala
           115                 120                 125
   cgc cgc gga ggc tac gag ctc tgg atc aag gcg ctc ctg ctg ctc gtc     432
   Arg Arg Gly Gly Tyr Glu Leu Trp Ile Lys Ala Leu Leu Leu Leu Val
       130                 135                 140
   ggc ttc tgg agc tcg ctg tgc tgg atg tgc acg ctg gac ccc tcg ttc     480
   Gly Phe Trp Ser Ser Leu Cys Trp Met Cys Thr Leu Asp Pro Ser Phe
   145                 150                 155                 160
   ggg gcc atc ctg gcc gcc atg tcg ctg ggc gtc ttt gcc gcc ttt gtg     528
   Gly Ala Ile Leu Ala Ala Met Ser Leu Gly Val Phe Ala Ala Phe Val
                   165                 170                 175
   ggc acg tgc atc cag cac gac ggc aac cac ggc gcc ttt gcc cag tcg     576
   Gly Thr Cys Ile Gln His Asp Gly Asn His Gly Ala Phe Ala Gln Ser
               180                 185                 190
   cga tgg gtc aac aag gtt gcc ggg tgg acg ctc gac atg atc ggc gcc     624
   Arg Trp Val Asn Lys Val Ala Gly Trp Thr Leu Asp Met Ile Gly Ala
           195                 200                 205
   agc ggc atg acg tgg gag ttc cag cac gcc ctg ggc cac cat ccg tac     672
   Ser Gly Met Thr Trp Glu Phe Gln His Ala Leu Gly His His Pro Tyr
       210                 215                 220
   acg aac ctg atc gag gag gag aac ggc ctg caa aag gtg agc ggc aag      720
   Thr Asn Leu Ile Glu Glu Glu Asn Gly Leu Gln Lys Val Ser Gly Lys
   225                 230                 235                 240
   aag atg gac acc aag ctg gcc gac cag gag agc gat ccg gac gtc ttt      768
   Lys Met Asp Thr Lys Leu Ala Asp Gln Glu Ser Asp Pro Asp Val Phe
                   245                 250                 255
   tcc acg tac ccg atg atg cgc ctg cac ccg tgg cac cag aag cgc tgg      816
   Ser Thr Tyr Pro Met Met Arg Leu His Pro Trp His Gln Lys Arg Trp
               260                 265                 270
   tac cac cgt ttc cag cac att tac ggc ccc ttc atc ttt ggc ttc atg      864
   Tyr His Arg Phe Gln His Ile Tyr Gly Pro Phe Ile Phe Gly Phe Met
           275                 280                 285
   acc atc aac aag gtg gtc acg cag gac gtc ggt gtg gtg ttc cgc aag      912
   Thr Ile Asn Lys Val Val Thr Gln Asp Val Gly Val Val Phe Arg Lys
       290                 295                 300
   cgg ctc ttc cag att gac gcc gag tgc cgg tac gcg agc cca atg tac      960
   Arg Leu Phe Gln Ile Asp Ala Glu Cys Arg Tyr Ala Ser Pro Met Tyr
   305                 310                 315                 320
   gtg gcg cgt ttc tgg atc atg aag gcg ctc acg gtg ctc tac atg gtg     1008
   Val Ala Arg Phe Trp Ile Met Lys Ala Leu Thr Val Leu Tyr Met Val
                   325                 330                 335
   gcc ctg ccg tgc tac atg cag ggc ccg tgg cac ggc ctc aag ctg ttc     1056
   Ala Leu Pro Cys Tyr Met Gln Gly Pro Trp His Gly Leu Lys Leu Phe
               340                 345                 350
   gcg atc gcg cac ttt acg tgc ggc gag gtg ctc gca acc atg ttc att     1104
   Ala Ile Ala His Phe Thr Cys Gly Glu Val Leu Ala Thr Met Phe Ile
           355                 360                 365
   gtg aac cac gtc atc gag ggc gtc tcg tac gct tcc aag gac gcg gtc     1152
   Val Asn His Val Ile Glu Gly Val Ser Tyr Ala Ser Lys Asp Ala Val
       370                 375                 380
   aag ggc acg atg gcg ccg ccg aag acg atg cac ggc gtg acg ccc atg     1200
   Lys Gly Thr Met Ala Pro Pro Lys Thr Met His Gly Val Thr Pro Met
   385                 390                 395                 400
   aac aac acg cgc aag gag gtg gag gcg gag gcg tcc aag tct ggc gcc     1248
   Asn Asn Thr Arg Lys Glu Val Glu Ala Glu Ala Ser Lys Ser Gly Ala
                   405                 410                 415
   gtg gtc aag tca gtc ccg ctc gac gac tgg gcc gcc gtc cag tgc cag     1296
   Val Val Lys Ser Val Pro Leu Asp Asp Trp Ala Ala Val Gln Cys Gln
               420                 425                 430
   acc tcg gcg aac tgg agc gtc ggc tcg tgg ttc tgg aac cac ttt tcc     1344
   Thr Ser Ala Asn Trp Ser Val Gly Ser Trp Phe Trp Asn His Phe Ser
           435                 440                 445
   ggc ggt ctc aac cac cag att gag cac cac ctg ttc ccc ggg ctc agc     1392
   Gly Gly Leu Asn His Gln Ile Glu His His Leu Phe Pro Gly Leu Ser
       450                 455                 460
   cac gag acg tac tac cac atc cag gac gtc gtt cag tcc acc tgc gcc     1440
   His Glu Thr Tyr Tyr His Ile Gln Asp Val Val Gln Ser Thr Cys Ala
   465                 470                 475                 480
   gag tac ggc gtc ccg tac cag cac gag cct tcg ctc tgg acc gcg tac     1488
   Glu Tyr Gly Val Pro Tyr Gln His Glu Pro Ser Leu Trp Thr Ala Tyr
                   485                 490                 495
   tgg aag atg ctc gag cac ctc cgt cgg ctc ggc aat gag gag acc cac     1536
   Trp Lys Met Leu Glu His Leu Arg Arg Leu Gly Asn Glu Glu Thr His
               500                 505                 510
   gag tcc tgg cag cgc gct gcc tga                                     1560
   Glu Ser Trp Gln Arg Ala Ala
           515
<210> 80
   <211> 519
   <212> PRT
   <213> Thraustochytrium sp
<400> 80
   Met Thr Val Gly Tyr Asp Gly Glu Ile Pro Phe Glu Gln Val Arg Ala
   1               5                   10                  15
   His Asn Lys Pro Asp Asp Ala Trp Cys Ala Ile His Gly His Val Tyr
               20                  25                  30
   Asp Val Thr Lys Phe Ala Ser Val His Pro Gly Gly Asp Ile Ile Leu
           35                  40                  45
   Leu Ala Ala Gly Lys Asp Ala Thr Val Leu Tyr Glu Thr Tyr His Val
       50                  55                  60
   Arg Gly Val Ser Asp Ala Val Leu Arg Lys Tyr Arg Ile Gly Lys Leu
   65                  70                  75                  80
   Pro Asp Gly Gln Gly Gly Ala Asn Glu Lys Glu Lys Arg Thr Leu Ser
                   85                  90                  95
   Gly Leu Ser Ser Ala Ser Tyr Tyr Thr Trp Asn Ser Asp Phe Tyr Arg
               100                 105                 110
   Val Met Arg Glu Arg Val Val Ala Arg Leu Lys Glu Arg Gly Lys Ala
           115                 120                 125
   Arg Arg Gly Gly Tyr Glu Leu Trp Ile Lys Ala Leu Leu Leu Leu Val
       130                 135                 140
   Gly Phe Trp Ser Ser Leu Cys Trp Met Cys Thr Leu Asp Pro Ser Phe
   145                 150                 155                 160
   Gly Ala Ile Leu Ala Ala Met Ser Leu Gly Val Phe Ala Ala Phe Val
                   165                 170                 175
   Gly Thr Cys Ile Gln His Asp Gly Asn His Gly Ala Phe Ala Gln Ser
               180                 185                 190
   Arg Trp Val Asn Lys Val Ala Gly Trp Thr Leu Asp Met Ile Gly Ala
           195                 200                 205
   Ser Gly Met Thr Trp Glu Phe Gln His Ala Leu Gly His His Pro Tyr
       210                 215                 220
   Thr Asn Leu Ile Glu Glu Glu Asn Gly Leu Gln Lys Val Ser Gly Lys
   225                 230                 235                 240
   Lys Met Asp Thr Lys Leu Ala Asp Gln Glu Ser Asp Pro Asp Val Phe
                   245                 250                 255
   Ser Thr Tyr Pro Met Met Arg Leu His Pro Trp His Gln Lys Arg Trp
               260                 265                 270
   Tyr His Arg Phe Gln His Ile Tyr Gly Pro Phe Ile Phe Gly Phe Met
           275                 280                 285
   Thr Ile Asn Lys Val Val Thr Gln Asp Val Gly Val Val Phe Arg Lys
       290                 295                 300
   Arg Leu Phe Gln Ile Asp Ala Glu Cys Arg Tyr Ala Ser Pro Met Tyr
   305                 310                 315                 320
   Val Ala Arg Phe Trp Ile Met Lys Ala Leu Thr Val Leu Tyr Met Val
                   325                 330                 335
   Ala Leu Pro Cys Tyr Met Gln Gly Pro Trp His Gly Leu Lys Leu Phe
               340                 345                 350
   Ala Ile Ala His Phe Thr Cys Gly Glu Val Leu Ala Thr Met Phe Ile
           355                 360                 365
   Val Asn His Val Ile Glu Gly Val Ser Tyr Ala Ser Lys Asp Ala Val
       370                 375                 380
   Lys Gly Thr Met Ala Pro Pro Lys Thr Met His Gly Val Thr Pro Met
   385                 390                 395                 400
   Asn Asn Thr Arg Lys Glu Val Glu Ala Glu Ala Ser Lys Ser Gly Ala
                   405                 410                 415
   Val Val Lys Ser Val Pro Leu Asp Asp Trp Ala Ala Val Gln Cys Gln
               420                 425                 430
   Thr Ser Ala Asn Trp Ser Val Gly Ser Trp Phe Trp Asn His Phe Ser
           435                 440                 445
   Gly Gly Leu Asn His Gln Ile Glu His His Leu Phe Pro Gly Leu Ser
       450                 455                 460
   His Glu Thr Tyr Tyr His Ile Gln Asp Val Val Gln Ser Thr Cys Ala
   465                 470                 475                 480
   Glu Tyr Gly Val Pro Tyr Gln His Glu Pro Ser Leu Trp Thr Ala Tyr
                   485                 490                 495
   Trp Lys Met Leu Glu His Leu Arg Arg Leu Gly Asn Glu Glu Thr His
               500                 505                 510
   Glu Ser Trp Gln Arg Ala Ala
           515
<210> 81
   <211> 1653
   <212> DNA
   <213> Thalassiosira pseudonana
<220>
   <221> CDS
   <222> (1)..(1653)
<400> 81
   atg ggc aac ggc aac ctc cca gca tcc acc gca cag ctc aag tcc acc      48
   Met Gly Asn Gly Asn Leu Pro Ala Ser Thr Ala Gln Leu Lys Ser Thr
   1               5                   10                  15
   tcg aag ccc cag cag caa cat gag cat cgc acc atc tcc aag tcc gag      96
   Ser Lys Pro Gln Gln Gln His Glu His Arg Thr Ile Ser Lys Ser Glu
               20                  25                  30
   ctc gcc caa cac aac acg ccc aaa tca gca tgg tgt gcc gtc cac tcc      144
   Leu Ala Gln His Asn Thr Pro Lys Ser Ala Trp Cys Ala Val His Ser
           35                  40                  45
   act ccc gcc acc gac cca tcc cac tcc aac aac aaa caa cac gca cac      192
   Thr Pro Ala Thr Asp Pro Ser His Ser Asn Asn Lys Gln His Ala His
       50                  55                  60
   cta gtc ctc gac att acc gac ttt gcg tcc cgc cat cca ggg gga gac      240
   Leu Val Leu Asp Ile Thr Asp Phe Ala Ser Arg His Pro Gly Gly Asp
   65                  70                  75                  80
   ctc atc ctc ctc gct tcc ggc aaa gac gcc tcg gtg ctg ttt gaa aca      288
   Leu Ile Leu Leu Ala Ser Gly Lys Asp Ala Ser Val Leu Phe Glu Thr
                   85                  90                  95
   tac cat cca cgt gga gtt ccg acg tct ctc att caa aag ctg cag att      336
   Tyr His Pro Arg Gly Val Pro Thr Ser Leu Ile Gln Lys Leu Gln Ile
               100                 105                 110
   gga gtg atg gag gag gag gcg ttt cgg gat tcg ttt tac agt tgg act      384
   Gly Val Met Glu Glu Glu Ala Phe Arg Asp Ser Phe Tyr Ser Trp Thr
           115                 120                 125
   gat tct gac ttt tat act gtg ttg aag agg agg gtt gtg gag cgg ttg      432
   Asp Ser Asp Phe Tyr Thr Val Leu Lys Arg Arg Val Val Glu Arg Leu
       130                 135                 140
   gag gag agg ggg ttg gac agg agg gga tcg aaa gag att tgg atc aag      480
   Glu Glu Arg Gly Leu Asp Arg Arg Gly Ser Lys Glu Ile Trp Ile Lys
   145                 150                 155                 160
   gct ttg ttc ttg ttg gtt gga ttt tgg tac tgt ttg tac aag atg tat      528
   Ala Leu Phe Leu Leu Val Gly Phe Trp Tyr Cys Leu Tyr Lys Met Tyr
                   165                 170                 175
   act acg tcg gat att gat cag tac ggt att gcc att gcc tat tct att      576
   Thr Thr Ser Asp Ile Asp Gln Tyr Gly Ile Ala Ile Ala Tyr Ser Ile
               180                 185                 190
   gga atg gga acc ttt gcg gca ttc atc ggc acg tgt att caa cac gat      624
   Gly Met Gly Thr Phe Ala Ala Phe Ile Gly Thr Cys Ile Gln His Asp
           195                 200                 205
   gga aat cac ggt gca ttc gct cag aac aag tta ctc aac aag ttg gct      672
   Gly Asn His Gly Ala Phe Ala Gln Asn Lys Leu Leu Asn Lys Leu Ala
       210                 215                 220
   ggg tgg acg ttg gat atg att ggt gcg agt gcg ttt acg tgg gag ctt      720
   Gly Trp Thr Leu Asp Met Ile Gly Ala Ser Ala Phe Thr Trp Glu Leu
   225                 230                 235                 240
   cag cac atg ctg ggg cat cat cca tat acg aat gtg ttg gat ggg gtg      768
   Gln His Met Leu Gly His His Pro Tyr Thr Asn Val Leu Asp Gly Val
                   245                 250                 255
   gag gag gag agg aag gag agg ggg gag gat gtt gct ttg gaa gaa aag      816
   Glu Glu Glu Arg Lys Glu Arg Gly Glu Asp Val Ala Leu Glu Glu Lys
               260                 265                 270
   gat cag gaa tca gat cca gac gta ttc tcc tcc ttc cct ctc atg aga      864
   Asp Gln Glu Ser Asp Pro Asp Val Phe Ser Ser Phe Pro Leu Met Arg
           275                 280                 285
   atg cat ccc cac cat aca acc tca tgg tat cat aaa tac caa cac ctc      912
   Met His Pro His His Thr Thr Ser Trp Tyr His Lys Tyr Gln His Leu
       290                 295                 300
   tac gct cca ccc ctc ttt gca ttg atg aca ctt gcc aaa gta ttc caa      960
   Tyr Ala Pro Pro Leu Phe Ala Leu Met Thr Leu Ala Lys Val Phe Gln
   305                 310                 315                 320
   cag gat ttt gaa gtt gcc aca tcc gga cga tta tat cat att gat gcc      1008
   Gln Asp Phe Glu Val Ala Thr Ser Gly Arg Leu Tyr His Ile Asp Ala
                   325                 330                 335
   aat gta cgt tat ggt tcg gta tgg aat gtc atg agg ttt tgg gct atg      1056
   Asn Val Arg Tyr Gly Ser Val Trp Asn Val Met Arg Phe Trp Ala Met
               340                 345                 350
   aag gtc att acg atg gga tat atg atg gga tta cca atc tac ttt cat      1104
   Lys Val Ile Thr Met Gly Tyr Met Met Gly Leu Pro Ile Tyr Phe His
           355                 360                 365
   gga gta ctg agg gga gtt gga ttg ttt gtt att ggg cat ttg gcg tgt      1152
   Gly Val Leu Arg Gly Val Gly Leu Phe Val Ile Gly His Leu Ala Cys
       370                 375                 380
   gga gag ttg ttg gcg acg atg ttt att gtg aat cac gtc att gag ggt      1200
   Gly Glu Leu Leu Ala Thr Met Phe Ile Val Asn His Val Ile Glu Gly
   385                 390                 395                 400
   gtg agt tat gga acg aag gat ttg gtt ggt ggt gcg agt cat gga gat      1248
   Val Ser Tyr Gly Thr Lys Asp Leu Val Gly Gly Ala Ser His Gly Asp
                   405                 410                 415
   gag aag aag att gtc aag cca acg act gta ttg gga gat aca cca atg      1296
   Glu Lys Lys Ile Val Lys Pro Thr Thr Val Leu Gly Asp Thr Pro Met
               420                 425                 430
   gaa aag act cgc gag gag gca ttg aaa agc aac agc aat aac aac aag      1344
   Glu Lys Thr Arg Glu Glu Ala Leu Lys Ser Asn Ser Asn Asn Asn Lys
           435                 440                 445
   aag aag gga gag aag aac tcg gta cca tcc gtt cca ttc aac gac tgg      1392
   Lys Lys Gly Glu Lys Asn Ser Val Pro Ser Val Pro Phe Asn Asp Trp
       450                 455                 460
   gca gca gtc caa tgc cag acc tcc gtg aat tgg tct cca ggc tca tgg      1440
   Ala Ala Val Gln Cys Gln Thr Ser Val Asn Trp Ser Pro Gly Ser Trp
   465                 470                 475                 480
   ttc tgg aat cac ttt tct ggg gga ctc tct cat cag att gag cat cac      1488
   Phe Trp Asn His Phe Ser Gly Gly Leu Ser His Gln Ile Glu His His
                   485                 490                 495
   ttg ttc ccc agc att tgt cat aca aac tac tgt cat atc cag gat gtt      1536
   Leu Phe Pro Ser Ile Cys His Thr Asn Tyr Cys His Ile Gln Asp Val
               500                 505                 510
   gtg gag agt acg tgt gct gag tac gga gtt ccg tat cag agt gag agt      1584
   Val Glu Ser Thr Cys Ala Glu Tyr Gly Val Pro Tyr Gln Ser Glu Ser
           515                 520                 525
   aat ttg ttt gtt gct tat gga aag atg att agt cat ttg aag ttt ttg      1632
   Asn Leu Phe Val Ala Tyr Gly Lys Met Ile Ser His Leu Lys Phe Leu
       530                 535                 540
   ggt aaa gcc aag tgt gag tag                                          1653
   Gly Lys Ala Lys Cys Glu
   545                 550
<210> 82
   <211> 550
   <212> PRT
   <213> Thalassiosira pseudonana
<400> 82
   Met Gly Asn Gly Asn Leu Pro Ala Ser Thr Ala Gln Leu Lys Ser Thr
   1               5                   10                  15
   Ser Lys Pro Gln Gln Gln His Glu His Arg Thr Ile Ser Lys Ser Glu
               20                  25                  30
   Leu Ala Gln His Asn Thr Pro Lys Ser Ala Trp Cys Ala Val His Ser
           35                  40                  45
   Thr Pro Ala Thr Asp Pro Ser His Ser Asn Asn Lys Gln His Ala His
       50                  55                  60
   Leu Val Leu Asp Ile Thr Asp Phe Ala Ser Arg His Pro Gly Gly Asp
   65                  70                  75                  80
   Leu Ile Leu Leu Ala Ser Gly Lys Asp Ala Ser Val Leu Phe Glu Thr
                   85                  90                  95
   Tyr His Pro Arg Gly Val Pro Thr Ser Leu Ile Gln Lys Leu Gln Ile
               100                 105                 110
   Gly Val Met Glu Glu Glu Ala Phe Arg Asp Ser Phe Tyr Ser Trp Thr
           115                 120                 125
   Asp Ser Asp Phe Tyr Thr Val Leu Lys Arg Arg Val Val Glu Arg Leu
       130                                135                                140
   Glu Glu Arg Gly Leu Asp Arg Arg Gly Ser Lys Glu Ile Trp Ile Lys
   145                     150                     155                     160
   Ala Leu Phe Leu Leu Val Gly Phe Trp Tyr Cys Leu Tyr Lys Met Tyr
                   165                     170                     175
   Thr Thr Ser Asp Ile Asp Gln Tyr Gly Ile Ala Ile Ala Tyr Ser Ile
               180                     185                     190
   Gly Met Gly Thr Phe Ala Ala Phe Ile Gly Thr Cys Ile Gln His Asp
           195                     200                     205
   Gly Asn His Gly Ala Phe Ala Gln Asn Lys Leu Leu Asn Lys Leu Ala
       210                     215                     220
   Gly Trp Thr Leu Asp Met Ile Gly Ala Ser Ala Phe Thr Trp Glu Leu
   225                     230                     235                     240
   Gln His Met Leu Gly His His Pro Tyr Thr Asn Val Leu Asp Gly Val
                   245                     250                     255
   Glu Glu Glu Arg Lys Glu Arg Gly Glu Asp Val Ala Leu Glu Glu Lys
               260                     265                     270
   Asp Gln Glu Ser Asp Pro Asp Val Phe Ser Ser Phe Pro Leu Met Arg
           275                     280                     285
   Met His Pro His His Thr Thr Ser Trp Tyr His Lys Tyr Gln His Leu
       290                     295                     300
   Tyr Ala Pro Pro Leu Phe Ala Leu Met Thr Leu Ala Lys Val Phe Gln
   305                     310                     315                     320
   Gln Asp Phe Glu Val Ala Thr Ser Gly Arg Leu Tyr His Ile Asp Ala
                   325                     330                     335
   Asn Val Arg Tyr Gly Ser Val Trp Asn Val Met Arg Phe Trp Ala Met
               340                     345                     350
   Lys Val Ile Thr Met Gly Tyr Met Met Gly Leu Pro Ile Tyr Phe His
           355                     360                     365
   Gly Val Leu Arg Gly Val Gly Leu Phe Val Ile Gly His Leu Ala Cys
       370                     375                     380
   Gly Glu Leu Leu Ala Thr Met Phe Ile Val Asn His Val Ile Glu Gly
   385                     390                     395                     400
   Val Ser Tyr Gly Thr Lys Asp Leu Val Gly Gly Ala Ser His Gly Asp
                   405                     410                     415
   Glu Lys Lys Ile Val Lys Pro Thr Thr Val Leu Gly Asp Thr Pro Met
               420                     425                     430
   Glu Lys Thr Arg Glu Glu Ala Leu Lys Ser Asn Ser Asn Asn Asn Lys
           435                     440                     445
   Lys Lys Gly Glu Lys Asn Ser Val Pro Ser Val Pro Phe Asn Asp Trp
       450                     455                     460
   Ala Ala Val Gln Cys Gln Thr Ser Val Asn Trp Ser Pro Gly Ser Trp
   465                     470                     475                     480
   Phe Trp Asn His Phe Ser Gly Gly Leu Ser His Gln Ile Glu His His
                   485                     490                     495
   Leu Phe Pro Ser Ile Cys His Thr Asn Tyr Cys His Ile Gln Asp Val
               500                     505                     510
   Val Glu Ser Thr Cys Ala Glu Tyr Gly Val Pro Tyr Gln Ser Glu Ser
           515                     520                     525
   Asn Leu Phe Val Ala Tyr Gly Lys Met Ile Ser His Leu Lys Phe Leu
       530                     535                     540
   Gly Lys Ala Lys Cys Glu
   545                     550
<210> 83
   <211> 1626
   <212> DNA
   <213> Euglena gracilis
<220>
   <221> CDS
   <222> (1)..(1626)
<400> 83
   atg ttg gtg ctg ttt ggc aat ttc tat gtc aag caa tac tcc caa aag      48
   Met Leu Val Leu Phe Gly Asn Phe Tyr Val Lys Gln Tyr Ser Gln Lys
   1               5                   10                  15
   aac ggc aag ccg gag aac gga gcc acc cct gag aac gga gcg aag ccg      96
   Asn Gly Lys Pro Glu Asn Gly Ala Thr Pro Glu Asn Gly Ala Lys Pro
               20                  25                  30
   caa cct tgc gag aac ggc acg gtg gaa aag cga gag aat gac acc gcc      144
   Gln Pro Cys Glu Asn Gly Thr Val Glu Lys Arg Glu Asn Asp Thr Ala
           35                  40                  45
   aac gtt cgg ccc acc cgt cca gct gga ccc ccg ccg gcc acg tac tac      192
   Asn Val Arg Pro Thr Arg Pro Ala Gly Pro Pro Pro Ala Thr Tyr Tyr
       50                  55                  60
   gac tcc ctg gca gtg tcg ggg cag ggc aag gag cgg ctg ttc acc acc      240
   Asp Ser Leu Ala Val Ser Gly Gln Gly Lys Glu Arg Leu Phe Thr Thr
   65                  70                  75                  80
   gat gag gtg agg cgg cac atc ctc ccc acc gat ggc tgg ctg acg tgc      288
   Asp Glu Val Arg Arg His Ile Leu Pro Thr Asp Gly Trp Leu Thr Cys
                   85                  90                  95
   cac gaa gga gtc tac gat gtc act gat ttc ctt gcc aag cac cct ggt      336
   His Glu Gly Val Tyr Asp Val Thr Asp Phe Leu Ala Lys His Pro Gly
               100                 105                 110
   ggc ggt gtc atc acg ctg ggc ctt gga agg gac tgc aca atc ctc atc      384
   Gly Gly Val Ile Thr Leu Gly Leu Gly Arg Asp Cys Thr Ile Leu Ile
           115                 120                 125
   gag tca tac cac cct gct ggg cgc ccg gac aag gtg atg gag aag tac      432
   Glu Ser Tyr His Pro Ala Gly Arg Pro Asp Lys Val Met Glu Lys Tyr
       130                 135                 140
   cgc att ggt acg ctg cag gac ccc aag acg ttc tat gct tgg gga gag      480
   Arg Ile Gly Thr Leu Gln Asp Pro Lys Thr Phe Tyr Ala Trp Gly Glu
   145                 150                 155                 160
   tcc gat ttc tac cct gag ttg aag cgc cgg gcc ctt gca agg ctg aag      528
   Ser Asp Phe Tyr Pro Glu Leu Lys Arg Arg Ala Leu Ala Arg Leu Lys
                   165                 170                 175
   gag gct ggt cag gcg cgg cgc ggc ggc ctt ggg gtg aag gcc ctc ctg      576
   Glu Ala Gly Gln Ala Arg Arg Gly Gly Leu Gly Val Lys Ala Leu Leu
               180                 185                 190
   gtg ctc acc ctc ttc ttc gtg tcg tgg tac atg tgg gtg gcc cac aag      624
   Val Leu Thr Leu Phe Phe Val Ser Trp Tyr Met Trp Val Ala His Lys
           195                 200                 205
   tcc ttc ctc tgg gcc gcc gtc tgg ggc ttc gcc ggc tcc cac gtc ggg      672
   Ser Phe Leu Trp Ala Ala Val Trp Gly Phe Ala Gly Ser His Val Gly
       210                 215                 220
   ctg agc atc cag cac gat ggc aac cac ggc gcg ttc agc cgc aac aca      720
   Leu Ser Ile Gln His Asp Gly Asn His Gly Ala Phe Ser Arg Asn Thr
   225                 230                 235                 240
   ctg gtg aac cgc ctg gcg ggg tgg ggc atg gac ttg atc ggc gcg tcg      768
   Leu Val Asn Arg Leu Ala Gly Trp Gly Met Asp Leu Ile Gly Ala Ser
                   245                 250                 255
   tcc acg gtg tgg gag tac cag cac gtc atc ggc cac cac cag tac acc      816
   Ser Thr Val Trp Glu Tyr Gln His Val Ile Gly His His Gln Tyr Thr
               260                 265                 270
   aac ctc gtg tcg gac acg cta ttc agt ctg cct gag aac gat ccg gac      864
   Asn Leu Val Ser Asp Thr Leu Phe Ser Leu Pro Glu Asn Asp Pro Asp
           275                 280                 285
   gtc ttc tcc agc tac ccg ctg atg cgc atg cac ccg gat acg gcg tgg      912
   Val Phe Ser Ser Tyr Pro Leu Met Arg Met His Pro Asp Thr Ala Trp
       290                 295                 300
   cag ccg cac cac cgc ttc cag cac ctg ttc gcg ttc cca ctg ttc gcc      960
   Gln Pro His His Arg Phe Gln His Leu Phe Ala Phe Pro Leu Phe Ala
   305                 310                 315                 320
   ctg atg aca atc agc aag gtg ctg acc agc gat ttc gct gtc tgc ctc     1008
   Leu Met Thr Ile Ser Lys Val Leu Thr Ser Asp Phe Ala Val Cys Leu
                   325                 330                 335
   agc atg aag aag ggg tcc atc gac tgc tcc tcc agg ctc gtc cca ctg     1056
   Ser Met Lys Lys Gly Ser Ile Asp Cys Ser Ser Arg Leu Val Pro Leu
               340                 345                 350
   gag ggg cag ctg ctg ttc tgg ggg gcc aag ctg gcg aac ttc ctg ttg     1104
   Glu Gly Gln Leu Leu Phe Trp Gly Ala Lys Leu Ala Asn Phe Leu Leu
           355                 360                 365
   cag att gtg ttg cca tgc tac ctc cac ggg aca gct atg ggc ctg gcc     1152
   Gln Ile Val Leu Pro Cys Tyr Leu His Gly Thr Ala Met Gly Leu Ala
       370                 375                 380
   ctc ttc tct gtt gct cac ctt gtg tcg ggg gag tac ctc gcg atc tgc      1200
   Leu Phe Ser Val Ala His Leu Val Ser Gly Glu Tyr Leu Ala Ile Cys
   385                 390                 395                 400
   ttc atc atc aac cac atc agc gag tct tgt gag ttt atg aat aca agc      1248
   Phe Ile Ile Asn His Ile Ser Glu Ser Cys Glu Phe Met Asn Thr Ser
                   405                 410                 415
   ttt caa acc gcc gcc cgg agg aca gag atg ctt cag gca gca cat cag      1296
   Phe Gln Thr Ala Ala Arg Arg Thr Glu Met Leu Gln Ala Ala His Gln
               420                 425                 430
   gca gcg gag gcc aag aag gtg aag ccc acc cct cca ccg aac gat tgg      1344
   Ala Ala Glu Ala Lys Lys Val Lys Pro Thr Pro Pro Pro Asn Asp Trp
           435                 440                 445
   gct gtg aca cag gtc caa tgc tgc gtg aat tgg aga tca ggt ggc gtg      1392
   Ala Val Thr Gln Val Gln Cys Cys Val Asn Trp Arg Ser Gly Gly Val
       450                 455                 460
   ttg gcc aat cac ctc tct gga ggc ttg aac cac cag atc gag cat cat      1440
   Leu Ala Asn His Leu Ser Gly Gly Leu Asn His Gln Ile Glu His His
   465                 470                 475                 480
   ctg ttc ccc agc atc tcg cat gcc aac tac ccc acc atc gcc cct gtt      1488
   Leu Phe Pro Ser Ile Ser His Ala Asn Tyr Pro Thr Ile Ala Pro Val
                   485                 490                 495
   gtg aag gag gtg tgc gag gag tac ggg ttg ccg tac aag aat tac gtc      1536
   Val Lys Glu Val Cys Glu Glu Tyr Gly Leu Pro Tyr Lys Asn Tyr Val
               500                 505                 510
   acg ttc tgg gat gca gtc tgt ggc atg gtt cag cac ctc cgg ttg atg      1584
   Thr Phe Trp Asp Ala Val Cys Gly Met Val Gln His Leu Arg Leu Met
           515                 520                 525
   ggt gct cca ccg gtg cca acg aac ggg gac aaa aag tca taa              1626
   Gly Ala Pro Pro Val Pro Thr Asn Gly Asp Lys Lys Ser
       530                 535                 540
<210> 84
   <211> 541
   <212> PRT
   <213> Euglena gracilis
<400> 84
   Met Leu Val Leu Phe Gly Asn Phe Tyr Val Lys Gln Tyr Ser Gln Lys
   1 5 10 15
   Asn Gly Lys Pro Glu Asn Gly Ala Thr Pro Glu Asn Gly Ala Lys Pro
   20 25 30
   Gln Pro Cys Glu Asn Gly Thr Val Glu Lys Arg Glu Asn Asp Thr Ala
   35 40 45
   Asn Val Arg Pro Thr Arg Pro Ala Gly Pro Pro Pro Ala Thr Tyr Tyr
   50 55 60
   Asp Ser Leu Ala Val Ser Gly Gln Gly Lys Glu Arg Leu Phe Thr Thr
   65 70 75 80
   Asp Glu Val Arg Arg His Ile Leu Pro Thr Asp Gly Trp Leu Thr Cys
   85 90 95
   His Glu Gly Val Tyr Asp Val Thr Asp Phe Leu Ala Lys His Pro Gly
   100 105 110
   Gly Gly Val Ile Thr Leu Gly Leu Gly Arg Asp Cys Thr Ile Leu Ile
   115 120 125
   Glu Ser Tyr His Pro Ala Gly Arg Pro Asp Lys Val Met Glu Lys Tyr
   130 135 140
   Arg Ile Gly Thr Leu Gln Asp Pro Lys Thr Phe Tyr Ala Trp Gly Glu
   145 150 155 160
   Ser Asp Phe Tyr Pro Glu Leu Lys Arg Arg Ala Leu Ala Arg Leu Lys
   165 170 175
   Glu Ala Gly Gln Ala Arg Arg Gly Gly Leu Gly Val Lys Ala Leu Leu
   180 185 190
   Val Leu Thr Leu Phe Phe Val Ser Trp Tyr Met Trp Val Ala His Lys
   195 200 205
   Ser Phe Leu Trp Ala Ala Val Trp Gly Phe Ala Gly Ser His Val Gly
   210 215 220
   Leu Ser Ile Gln His Asp Gly Asn His Gly Ala Phe Ser Arg Asn Thr
   225 230 235 240
   Leu Val Asn Arg Leu Ala Gly Trp Gly Met Asp Leu Ile Gly Ala Ser
                       245                     250                     255
   Ser Thr Val Trp Glu Tyr Gln His Val Ile Gly His His Gln Tyr Thr
                   260                 265                 270
   Asn Leu Val Ser Asp Thr Leu Phe Ser Leu Pro Glu Asn Asp Pro Asp
               275                 280                 285
   Val Phe Ser Ser Tyr Pro Leu Met Arg Met His Pro Asp Thr Ala Trp
       290                 295                 300
   Gln Pro His His Arg Phe Gln His Leu Phe Ala Phe Pro Leu Phe Ala
   305                 310                 315                 320
   Leu Met Thr Ile Ser Lys Val Leu Thr Ser Asp Phe Ala Val Cys Leu
                   325                 330                 335
   Ser Met Lys Lys Gly Ser Ile Asp Cys Ser Ser Arg Leu Val Pro Leu
               340                 345                 350
   Glu Gly Gln Leu Leu Phe Trp Gly Ala Lys Leu Ala Asn Phe Leu Leu
           355                 360                 365
   Gln Ile Val Leu Pro Cys Tyr Leu His Gly Thr Ala Met Gly Leu Ala
       370                 375                 380
   Leu Phe Ser Val Ala His Leu Val Ser Gly Glu Tyr Leu Ala Ile Cys
   385                 390                 395                 400
   Phe Ile Ile Asn His Ile Ser Glu Ser Cys Glu Phe Met Asn Thr Ser
                   405                 410                 415
   Phe Gln Thr Ala Ala Arg Arg Thr Glu Met Leu Gln Ala Ala His Gln
               420                 425                 430
   Ala Ala Glu Ala Lys Lys Val Lys Pro Thr Pro Pro Pro Asn Asp Trp
           435                 440                 445
   Ala Val Thr Gln Val Gln Cys Cys Val Asn Trp Arg Ser Gly Gly Val
       450                 455                 460
   Leu Ala Asn His Leu Ser Gly Gly Leu Asn His Gln Ile Glu His His
   465                 470                 475                 480
   Leu Phe Pro Ser Ile Ser His Ala Asn Tyr Pro Thr Ile Ala Pro Val
                   485                 490                 495
   Val Lys Glu Val Cys Glu Glu Tyr Gly Leu Pro Tyr Lys Asn Tyr Val
               500                 505                 510
   Thr Phe Trp Asp Ala Val Cys Gly Met Val Gln His Leu Arg Leu Met
           515                 520                 525
   Gly Ala Pro Pro Val Pro Thr Asn Gly Asp Lys Lys Ser
       530                 535                 540
<210> 85
   <211> 1548
   <212> DNA
   <213> Thraustochytrium aureum
<220>
   <221> CDS
   <222> (1)..(1548)
<400> 85
   atg acg gtc ggg ttt gac gaa acg gtg act atg gac acg gtc cgc aac        48
   Met Thr Val Gly Phe Asp Glu Thr Val Thr Met Asp Thr Val Arg Asn
   1               5                   10                  15
   cac aac atg ccg gac gac gcc tgg tgc gcg atc cac ggc acc gtg tac        96
   His Asn Met Pro Asp Asp Ala Trp Cys Ala Ile His Gly Thr Val Tyr
               20                  25                  30
   gac atc acc aag ttc agc aag gtg cac ccc ggc ggg gac atc atc atg       144
   Asp Ile Thr Lys Phe Ser Lys Val His Pro Gly Gly Asp Ile Ile Met
           35                  40                  45
   ctg gcc gct ggc aag gag gcc acc atc ctg ttc gag acc tac cac atc       192
   Leu Ala Ala Gly Lys Glu Ala Thr Ile Leu Phe Glu Thr Tyr His Ile
       50                  55                  60
   aag ggc gtc ccg gac gcg gtg ctg cgc aag tac aag gtc ggc aag ctc       240
   Lys Gly Val Pro Asp Ala Val Leu Arg Lys Tyr Lys Val Gly Lys Leu
   65                  70                  75                  80
   ccc cag ggc aag aag ggc gaa acg agc cac atg ccc acc ggg ctc gac       288
   Pro Gln Gly Lys Lys Gly Glu Thr Ser His Met Pro Thr Gly Leu Asp
                   85                  90                  95
   tcg gcc tcc tac tac tcg tgg gac agc gag ttt tac agg gtg ctc cgc       336
   Ser Ala Ser Tyr Tyr Ser Trp Asp Ser Glu Phe Tyr Arg Val Leu Arg
                   100                     105                     110
   gag cgc gtc gcc aag aag ctg gcc gag ccc ggc ctc atg cag cgc gcg        384
   Glu Arg Val Ala Lys Lys Leu Ala Glu Pro Gly Leu Met Gln Arg Ala
               115                     120                     125
   cgc atg gag ctc tgg gcc aag gcg atc ttc ctc ctg gca ggt ttc tgg        432
   Arg Met Glu Leu Trp Ala Lys Ala Ile Phe Leu Leu Ala Gly Phe Trp
           130                     135                     140
   ggc tcc ctt tac gcc atg tgc gtg cta gac ccg cac ggc ggt gcc atg        480
   Gly Ser Leu Tyr Ala Met Cys Val Leu Asp Pro His Gly Gly Ala Met
       145                 150                     155                     160
   gta gcc gcc gtt acg ctc ggc gtg ttc gct gcc ttt gtc gga act tgc        528
   Val Ala Ala Val Thr Leu Gly Val Phe Ala Ala Phe Val Gly Thr Cys
                   165                     170                     175
   atc cag cac gac ggc agc cac ggc gcc ttc tcc aag tcg cga ttc atg        576
   Ile Gln His Asp Gly Ser His Gly Ala Phe Ser Lys Ser Arg Phe Met
               180                     185                     190
   aac aag gcg gcg ggc tgg acc ctc gac atg atc ggc gcg agt gcg atg        624
   Asn Lys Ala Ala Gly Trp Thr Leu Asp Met Ile Gly Ala Ser Ala Met
           195                     200                     205
   acc tgg gag atg cag cac gtt ctt ggc cac cac ccg tac acc aac ctc        672
   Thr Trp Glu Met Gln His Val Leu Gly His His Pro Tyr Thr Asn Leu
       210                     215                     220
   atc gag atg gag aac ggt ttg gcc aag gtc aag ggc gcc gac gtc gac        720
   Ile Glu Met Glu Asn Gly Leu Ala Lys Val Lys Gly Ala Asp Val Asp
   225                 230                     235                     240
   ccg aag aag gtc gac cag gag agc gac ccg gac gtc ttc agt acg tac        768
   Pro Lys Lys Val Asp Gln Glu Ser Asp Pro Asp Val Phe Ser Thr Tyr
                   245                     250                     255
   ccg atg ctt cgc ctg cac ccg tgg cac cgc cag cgg ttt tac cac aag        816
   Pro Met Leu Arg Leu His Pro Trp His Arg Gln Arg Phe Tyr His Lys
               260                     265                     270
   ttc cag cac ctg tac gcc ccg ttt atc ttt ggg tct atg acg att aac        864
   Phe Gln His Leu Tyr Ala Pro Phe Ile Phe Gly Ser Met Thr Ile Asn
           275                     280                     285
   aag gtg att tcc cag gat gtc ggg gtt gtg ctg cgc aag cgc ctg ttc        912
   Lys Val Ile Ser Gln Asp Val Gly Val Val Leu Arg Lys Arg Leu Phe
       290                     295                     300
   cag atc gac gcc aac tgc cgg tat ggc agc ccc tgg tac gtg gcc cgc        960
   Gln Ile Asp Ala Asn Cys Arg Tyr Gly Ser Pro Trp Tyr Val Ala Arg
   305                 310                     315                     320
   ttc tgg atc atg aag ctc ctc acc acg ctc tac atg gtg gcg ctt ccc       1008
   Phe Trp Ile Met Lys Leu Leu Thr Thr Leu Tyr Met Val Ala Leu Pro
                   325                     330                     335
   atg tac atg cag ggg cct gct cag ggc ttg aag ctt ttc ttc atg gcc       1056
   Met Tyr Met Gln Gly Pro Ala Gln Gly Leu Lys Leu Phe Phe Met Ala
               340                     345                     350
   cac ttc acc tgc gga gag gtc ctc gcc acc atg ttt att gtc aac cac       1104
   His Phe Thr Cys Gly Glu Val Leu Ala Thr Met Phe Ile Val Asn His
           355                     360                     365
   atc atc gag ggc gtc agc tac gct tcc aag gac gcg gtc aag ggc gtc       1152
   Ile Ile Glu Gly Val Ser Tyr Ala Ser Lys Asp Ala Val Lys Gly Val
       370                     375                     380
   atg gct ccg ccg cgc act gtg cac ggt gtc acc ccg atg cag gtg acg       1200
   Met Ala Pro Pro Arg Thr Val His Gly Val Thr Pro Met Gln Val Thr
   385                 390                     395                     400
   caa aag gcg ctc agt gcg gcc gag tcg gcc aag tcg gac gcc gac aag       1248
   Gln Lys Ala Leu Ser Ala Ala Glu Ser Ala Lys Ser Asp Ala Asp Lys
                   405                     410                     415
   acg acc atg atc ccc ctc aac gac tgg gcc gct gtg cag tgc cag acc       1296
   Thr Thr Met Ile Pro Leu Asn Asp Trp Ala Ala Val Gln Cys Gln Thr
               420                     425                     430
   tct gtg aac tgg gct gtc ggg tcg tgg ttt tgg aac cac ttt tcg ggc       1344
   Ser Val Asn Trp Ala Val Gly Ser Trp Phe Trp Asn His Phe Ser Gly
           435                     440                     445
   ggc ctc aac cac cag att gag cac cac tgc ttc ccc caa aac ccc cac       1392
   Gly Leu Asn His Gln Ile Glu His His Cys Phe Pro Gln Asn Pro His
       450                     455                     460
   acg gtc aac gtc tac atc tcg ggc atc gtc aag gag acc tgc gaa gaa       1440
   Thr Val Asn Val Tyr Ile Ser Gly Ile Val Lys Glu Thr Cys Glu Glu
   465                 470                 475                 480
   tac ggc gtg ccg tac cag gct gag atc agc ctc ttc tct gcc tat ttc        1488
   Tyr Gly Val Pro Tyr Gln Ala Glu Ile Ser Leu Phe Ser Ala Tyr Phe
                   485                 490                 495
   aag atg ctg tcg cac ctc cgc acg ctc ggc aac gag gac ctc acg gcc        1536
   Lys Met Leu Ser His Leu Arg Thr Leu Gly Asn Glu Asp Leu Thr Ala
               500                 505                 510
   tgg tcc acg tga                                                        1548
   Trp Ser Thr
           515
<210> 86
   <211> 515
   <212> PRT
   <213> Thraustochytrium aureum
<400> 86
   Met Thr Val Gly Phe Asp Glu Thr Val Thr Met Asp Thr Val Arg Asn
   1 5 10 15
   His Asn Met Pro Asp Asp Ala Trp Cys Ala Ile His Gly Thr Val Tyr
   20 25 30
   Asp Ile Thr Lys Phe Ser Lys Val His Pro Gly Gly Asp Ile Ile Met
   35 40 45
   Leu Ala Ala Gly Lys Glu Ala Thr Ile Leu Phe Glu Thr Tyr His Ile
   50 55 60
   Lys Gly Val Pro Asp Ala Val Leu Arg Lys Tyr Lys Val Gly Lys Leu
   65 70 75 80
   Pro Gln Gly Lys Lys Gly Glu Thr Ser His Met Pro Thr Gly Leu Asp
   85 90 95
   Ser Ala Ser Tyr Tyr Ser Trp Asp Ser Glu Phe Tyr Arg Val Leu Arg
   100 105 110
   Glu Arg Val Ala Lys Lys Leu Ala Glu Pro Gly Leu Met Gln Arg Ala
   115 120 125
   Arg Met Glu Leu Trp Ala Lys Ala Ile Phe Leu Leu Ala Gly Phe Trp
   130 135 140
   Gly Ser Leu Tyr Ala Met Cys Val Leu Asp Pro His Gly Gly Ala Met
   145 150 155 160
   Val Ala Ala Val Thr Leu Gly Val Phe Ala Ala Phe Val Gly Thr Cys
   165 170 175
   Ile Gln His Asp Gly Ser His Gly Ala Phe Ser Lys Ser Arg Phe Met
   180 185 190
   Asn Lys Ala Ala Gly Trp Thr Leu Asp Met Ile Gly Ala Ser Ala Met
   195 200 205
   Thr Trp Glu Met Gln His Val Leu Gly His His Pro Tyr Thr Asn Leu
   210 215 220
   Ile Glu Met Glu Asn Gly Leu Ala Lys Val Lys Gly Ala Asp Val Asp
   225 230 235 240
   Pro Lys Lys Val Asp Gln Glu Ser Asp Pro Asp Val Phe Ser Thr Tyr
   245 250 255
   Pro Met Leu Arg Leu His Pro Trp His Arg Gln Arg Phe Tyr His Lys
   260 265 270
   Phe Gln His Leu Tyr Ala Pro Phe Ile Phe Gly Ser Met Thr Ile Asn
   275 280 285
   Lys Val Ile Ser Gln Asp Val Gly Val Val Leu Arg Lys Arg Leu Phe
   290 295 300
   Gln Ile Asp Ala Asn Cys Arg Tyr Gly Ser Pro Trp Tyr Val Ala Arg
   305 310 315 320
   Phe Trp Ile Met Lys Leu Leu Thr Thr Leu Tyr Met Val Ala Leu Pro
   325 330 335
   Met Tyr Met Gln Gly Pro Ala Gln Gly Leu Lys Leu Phe Phe Met Ala
   340 345 350
   His Phe Thr Cys Gly Glu Val Leu Ala Thr Met Phe Ile Val Asn His
   355 360 365
   Ile Ile Glu Gly Val Ser Tyr Ala Ser Lys Asp Ala Val Lys Gly Val
   370 375 380
   Met Ala Pro Pro Arg Thr Val His Gly Val Thr Pro Met Gln Val Thr
   385 390 395 400
   Gln Lys Ala Leu Ser Ala Ala Glu Ser Ala Lys Ser Asp Ala Asp Lys
   405 410 415
   Thr Thr Met Ile Pro Leu Asn Asp Trp Ala Ala Val Gln Cys Gln Thr
   420 425 430
   Ser Val Asn Trp Ala Val Gly Ser Trp Phe Trp Asn His Phe Ser Gly
   435 440 445
   Gly Leu Asn His Gln Ile Glu His His Cys Phe Pro Gln Asn Pro His
   450 455 460
   Thr Val Asn Val Tyr Ile Ser Gly Ile Val Lys Glu Thr Cys Glu Glu
   465 470 475 480
   Tyr Gly Val Pro Tyr Gln Ala Glu Ile Ser Leu Phe Ser Ala Tyr Phe
   485 490 495
   Lys Met Leu Ser His Leu Arg Thr Leu Gly Asn Glu Asp Leu Thr Ala
   500 505 510
   Trp Ser Thr
   515
<210> 87
   <211> 1548
   <212> DNA
   <213> Thraustochytrium aureum
<220>
   <221> CDS
   <222> (1)..(1548)
<400> 87
   atg acg gtc ggg ttt gac gaa acg gtg act atg gac acg gtc cgc aac      48
   Met Thr Val Gly Phe Asp Glu Thr Val Thr Met Asp Thr Val Arg Asn
   1               5                   10                  15
   cac aac atg ccg gac gac gcc tgg tgc gcg atc cac ggc acc gtg tac      96
   His Asn Met Pro Asp Asp Ala Trp Cys Ala Ile His Gly Thr Val Tyr
               20                  25                  30
   gac atc acc aag ttc agc aag gtg cac ccc ggc ggg gac atc atc atg      144
   Asp Ile Thr Lys Phe Ser Lys Val His Pro Gly Gly Asp Ile Ile Met
           35                  40                  45
   ctg gcc gct ggc aag gag gcc acc atc ctg ttc gag acg tac cac atc      192
   Leu Ala Ala Gly Lys Glu Ala Thr Ile Leu Phe Glu Thr Tyr His Ile
       50                  55                  60
   aag ggc gtt ccg gac gcg gtg ctg cgc aag tac aag gtc ggc aag ctc      240
   Lys Gly Val Pro Asp Ala Val Leu Arg Lys Tyr Lys Val Gly Lys Leu
   65                  70                  75                  80
   ccc cag ggc aag aag ggc gaa acg agc cac atg ccc acc ggg ctc gac      288
   Pro Gln Gly Lys Lys Gly Glu Thr Ser His Met Pro Thr Gly Leu Asp
                   85                  90                  95
   tcg gcc ttc tac tac tcg tgg gac agc gag ttt tac agg gtg ctc cgc      336
   Ser Ala Phe Tyr Tyr Ser Trp Asp Ser Glu Phe Tyr Arg Val Leu Arg
               100                 105                 110
   gag cgc gtc gcc aag aag ctg gcc gag ccc ggc ctc atg cag cgc gcg      384
   Glu Arg Val Ala Lys Lys Leu Ala Glu Pro Gly Leu Met Gln Arg Ala
           115                 120                 125
   cgc atg gag ctc tgg gcc aag gcg atc ttc ctc ctg gca ggt ttc tgg      432
   Arg Met Glu Leu Trp Ala Lys Ala Ile Phe Leu Leu Ala Gly Phe Trp
       130                 135                 140
   ggc tcc ctt tac gcc atg tgc gtg cta gac ccg cac ggc ggt gcc atg      480
   Gly Ser Leu Tyr Ala Met Cys Val Leu Asp Pro His Gly Gly Ala Met
   145                 150                 155                 160
   gta gcc gcc gtt acg ctc ggc gtg ttc gct gcc ttt gtc gga act tgc      528
   Val Ala Ala Val Thr Leu Gly Val Phe Ala Ala Phe Val Gly Thr Cys
                   165                 170                 175
   atc cag cac gac ggc agc cac ggc gcc ttc tcc aag tcg cga ttc atg      576
   Ile Gln His Asp Gly Ser His Gly Ala Phe Ser Lys Ser Arg Phe Met
               180                 185                 190
   aac aag gcg gcg ggc tgg acc ctc gac atg atc ggc gcg agc gcg atg      624
   Asn Lys Ala Ala Gly Trp Thr Leu Asp Met Ile Gly Ala Ser Ala Met
           195                 200                 205
   acc tgg gag atg cag cac gtt ctt ggc cac cac ccg tac acc aac ctc      672
   Thr Trp Glu Met Gln His Val Leu Gly His His Pro Tyr Thr Asn Leu
               210                           215                           220
   atc gag atg gag aac ggt ttg gcc aag gtc aag ggc gcc gac gtc gac         720
   Ile Glu Met Glu Asn Gly Leu Ala Lys Val Lys Gly Ala Asp Val Asp
   225                     230                     235                     240
   ccg aag aag gtc gac cag gag agc gac ccg gac gtc ttc agt acg tac         768
   Pro Lys Lys Val Asp Gln Glu Ser Asp Pro Asp Val Phe Ser Thr Tyr
                   245                     250                     255
   ccg atg ctt cgc ctg cac ccg tgg cac cgc cag cgg ttt tac cac aag         816
   Pro Met Leu Arg Leu His Pro Trp His Arg Gln Arg Phe Tyr His Lys
               260                     265                     270
   ttc cag cac ctg tac gcc ccg ttt atc ttt ggg ttt atg acg att aac         864
   Phe Gln His Leu Tyr Ala Pro Phe Ile Phe Gly Phe Met Thr Ile Asn
           275                     280                     285
   aag gtg att tcc cag gat gtc ggg gtt gtg ctg cgc aag cgc ctg ttc         912
   Lys Val Ile Ser Gln Asp Val Gly Val Val Leu Arg Lys Arg Leu Phe
       290                     295                     300
   cag atc gac gcc aac tgc cgg tat ggc agc ccc tgg tac gtg gcc cgc         960
   Gln Ile Asp Ala Asn Cys Arg Tyr Gly Ser Pro Trp Tyr Val Ala Arg
   305                     310                     315                     320
   ttc tgg atc atg aag ctc ctc acc acg ctc tac atg gtg gcg ctt ccc        1008
   Phe Trp Ile Met Lys Leu Leu Thr Thr Leu Tyr Met Val Ala Leu Pro
                   325                     330                     335
   atg tac atg cag ggg cct gct cag ggc ttg aag ctt ttc ttc atg gcc        1056
   Met Tyr Met Gln Gly Pro Ala Gln Gly Leu Lys Leu Phe Phe Met Ala
               340                     345                     350
   cac ttc acc tgc gga gag gtc ctc gcc acc atg ttt att gtc aac cac        1104
   His Phe Thr Cys Gly Glu Val Leu Ala Thr Met Phe Ile Val Asn His
           355                     360                     365
   atc atc gag ggc gtc agc tac gct tcc aag gac gcg gtc aag ggc gtc        1152
   Ile Ile Glu Gly Val Ser Tyr Ala Ser Lys Asp Ala Val Lys Gly Val
       370                     375                     380
   atg gct ccg ccg cgc act gtg cac ggt gtc acc ccg atg cag gtg acg        1200
   Met Ala Pro Pro Arg Thr Val His Gly Val Thr Pro Met Gln Val Thr
   385                     390                     395                     400
   caa aag gcg ctc agt gcg gcc gag tcg acc aag tcg gac gcc gac aag        1248
   Gln Lys Ala Leu Ser Ala Ala Glu Ser Thr Lys Ser Asp Ala Asp Lys
                   405                     410                     415
   acg acc atg atc ccc ctc aac gac tgg gcc gct gtg cag tgc cag acc        1296
   Thr Thr Met Ile Pro Leu Asn Asp Trp Ala Ala Val Gln Cys Gln Thr
               420                     425                     430
   tct gtg aac tgg gct gtc ggg tcg tgg ttt tgg aac cac ttt tcg ggc        1344
   Ser Val Asn Trp Ala Val Gly Ser Trp Phe Trp Asn His Phe Ser Gly
           435                     440                     445
   ggc ctc aac cac cag att gag cac cac tgc ttc ccc caa aac ccc cac        1392
   Gly Leu Asn His Gln Ile Glu His His Cys Phe Pro Gln Asn Pro His
       450                     455                     460
   acg gtc aac gtc tac atc tca ggc atc gtc aag gag acc tgc gaa gaa        1440
   Thr Val Asn Val Tyr Ile Ser Gly Ile Val Lys Glu Thr Cys Glu Glu
   465                     470                     475                     480
   tac ggc gtg ccg tac cag gct gag atc agc ctc ttc tct gcc tat ttc        1488
   Tyr Gly Val Pro Tyr Gln Ala Glu Ile Ser Leu Phe Ser Ala Tyr Phe
                   485                     490                     495
   aag atg ctg tcg cac ctc cgc acg ctc ggc aac gag gac ctc acg gcc        1536
   Lys Met Leu Ser His Leu Arg Thr Leu Gly Asn Glu Asp Leu Thr Ala
               500                     505                     510
   tgg tcc acg tga                                                        1548
   Trp Ser Thr
           515
<210> 88
   <211> 515
   <212> PRT
   <213> Thraustochytrium aureum
<400> 88
   Met Thr Val Gly Phe Asp Glu Thr Val Thr Met Asp Thr Val Arg Asn
   1               5                   10                  15
   His Asn Met Pro Asp Asp Ala Trp Cys Ala Ile His Gly Thr Val Tyr
               20                  25                  30
   Asp Ile Thr Lys Phe Ser Lys Val His Pro Gly Gly Asp Ile Ile Met
           35                  40                  45
   Leu Ala Ala Gly Lys Glu Ala Thr Ile Leu Phe Glu Thr Tyr His Ile
       50                  55                  60
   Lys Gly Val Pro Asp Ala Val Leu Arg Lys Tyr Lys Val Gly Lys Leu
   65                  70                  75                  80
   Pro Gln Gly Lys Lys Gly Glu Thr Ser His Met Pro Thr Gly Leu Asp
                   85                  90                  95
   Ser Ala Phe Tyr Tyr Ser Trp Asp Ser Glu Phe Tyr Arg Val Leu Arg
               100                 105                 110
   Glu Arg Val Ala Lys Lys Leu Ala Glu Pro Gly Leu Met Gln Arg Ala
           115                 120                 125
   Arg Met Glu Leu Trp Ala Lys Ala Ile Phe Leu Leu Ala Gly Phe Trp
       130                 135                 140
   Gly Ser Leu Tyr Ala Met Cys Val Leu Asp Pro His Gly Gly Ala Met
   145                 150                 155                 160
   Val Ala Ala Val Thr Leu Gly Val Phe Ala Ala Phe Val Gly Thr Cys
                   165                 170                 175
   Ile Gln His Asp Gly Ser His Gly Ala Phe Ser Lys Ser Arg Phe Met
               180                 185                 190
   Asn Lys Ala Ala Gly Trp Thr Leu Asp Met Ile Gly Ala Ser Ala Met
           195                 200                 205
   Thr Trp Glu Met Gln His Val Leu Gly His His Pro Tyr Thr Asn Leu
       210                 215                 220
   Ile Glu Met Glu Asn Gly Leu Ala Lys Val Lys Gly Ala Asp Val Asp
   225                 230                 235                 240
   Pro Lys Lys Val Asp Gln Glu Ser Asp Pro Asp Val Phe Ser Thr Tyr
                   245                 250                 255
   Pro Met Leu Arg Leu His Pro Trp His Arg Gln Arg Phe Tyr His Lys
               260                 265                 270
   Phe Gln His Leu Tyr Ala Pro Phe Ile Phe Gly Phe Met Thr Ile Asn
           275                 280                 285
   Lys Val Ile Ser Gln Asp Val Gly Val Val Leu Arg Lys Arg Leu Phe
       290                 295                 300
   Gln Ile Asp Ala Asn Cys Arg Tyr Gly Ser Pro Trp Tyr Val Ala Arg
   305                 310                 315                 320
   Phe Trp Ile Met Lys Leu Leu Thr Thr Leu Tyr Met Val Ala Leu Pro
                   325                 330                 335
   Met Tyr Met Gln Gly Pro Ala Gln Gly Leu Lys Leu Phe Phe Met Ala
               340                 345                 350
   His Phe Thr Cys Gly Glu Val Leu Ala Thr Met Phe Ile Val Asn His
           355                 360                 365
   Ile Ile Glu Gly Val Ser Tyr Ala Ser Lys Asp Ala Val Lys Gly Val
       370                 375                 380
   Met Ala Pro Pro Arg Thr Val His Gly Val Thr Pro Met Gln Val Thr
   385                 390                 395                 400
   Gln Lys Ala Leu Ser Ala Ala Glu Ser Thr Lys Ser Asp Ala Asp Lys
                   405                 410                 415
   Thr Thr Met Ile Pro Leu Asn Asp Trp Ala Ala Val Gln Cys Gln Thr
               420                 425                 430
   Ser Val Asn Trp Ala Val Gly Ser Trp Phe Trp Asn His Phe Ser Gly
           435                 440                 445
   Gly Leu Asn His Gln Ile Glu His His Cys Phe Pro Gln Asn Pro His
       450                 455                 460
   Thr Val Asn Val Tyr Ile Ser Gly Ile Val Lys Glu Thr Cys Glu Glu
   465                 470                 475                 480
   Tyr Gly Val Pro Tyr Gln Ala Glu Ile Ser Leu Phe Ser Ala Tyr Phe
                   485                 490                 495
   Lys Met Leu Ser His Leu Arg Thr Leu Gly Asn Glu Asp Leu Thr Ala
               500                 505                 510
   Trp Ser Thr
           515
<210> 89
   <211> 1548
   <212> DNA
   <213> Thraustochytrium aureum
<220>
   <221> CDS
   <222> (1)..(1548)
<400> 89
   atg acg gtc ggg ttt gac gaa acg gtg act atg gac acg gtc cgc aac        48
   Met Thr Val Gly Phe Asp Glu Thr Val Thr Met Asp Thr Val Arg Asn
   1               5                   10                  15
   cac aac atg ccg gac gac gcc tgg tgc gcg atc cac ggc acc gtg tac        96
   His Asn Met Pro Asp Asp Ala Trp Cys Ala Ile His Gly Thr Val Tyr
               20                  25                  30
   gac atc acc aag ttc agc aag gtg cac ccc ggc ggg gac atc atc atg        144
   Asp Ile Thr Lys Phe Ser Lys Val His Pro Gly Gly Asp Ile Ile Met
           35                  40                  45
   ctg gcc gct ggc aag gag gcc acc atc ctg ttc gag acg tac cac atc        192
   Leu Ala Ala Gly Lys Glu Ala Thr Ile Leu Phe Glu Thr Tyr His Ile
       50                  55                  60
   aag ggc gtc ccg gac gcg gtg ctg cgc aag tac aag gtc ggc aag ctc        240
   Lys Gly Val Pro Asp Ala Val Leu Arg Lys Tyr Lys Val Gly Lys Leu
   65                  70                  75                  80
   ccc cag ggc aag aag ggc gaa acg agc cac atg ccc acc ggg ctc gac        288
   Pro Gln Gly Lys Lys Gly Glu Thr Ser His Met Pro Thr Gly Leu Asp
                   85                  90                  95
   tcg gcc tcc tac tac tcg tgg gac agc gag ttt tac agg gtg ctc cgc        336
   Ser Ala Ser Tyr Tyr Ser Trp Asp Ser Glu Phe Tyr Arg Val Leu Arg
               100                 105                 110
   gag cgc gtc gcc aag aag ctg gcc gag ccc ggc ctc atg cag cgc gcg        384
   Glu Arg Val Ala Lys Lys Leu Ala Glu Pro Gly Leu Met Gln Arg Ala
           115                 120                 125
   cgc atg gag ctc tgg gcc aag gcg atc ttc ctc ctg gca ggt ttc tgg        432
   Arg Met Glu Leu Trp Ala Lys Ala Ile Phe Leu Leu Ala Gly Phe Trp
       130                 135                 140
   ggc tcc ctt tac gcc atg tgc gtg cta gac ccg cac ggc ggt gcc atg        480
   Gly Ser Leu Tyr Ala Met Cys Val Leu Asp Pro His Gly Gly Ala Met
   145                 150                 155                 160
   gta gcc gcc gtt acg ctc ggc gtg ttc gct gcc ttt gtc gga act tgc        528
   Val Ala Ala Val Thr Leu Gly Val Phe Ala Ala Phe Val Gly Thr Cys
                   165                 170                 175
   atc cag cac gac ggc agc cac ggc gcc ttc tcc aag tcg cga ttc atg        576
   Ile Gln His Asp Gly Ser His Gly Ala Phe Ser Lys Ser Arg Phe Met
               180                 185                 190
   aac aag gcg gcg ggc tgg acc ctc gac atg atc ggc gcg agc gcg atg        624
   Asn Lys Ala Ala Gly Trp Thr Leu Asp Met Ile Gly Ala Ser Ala Met
           195                 200                 205
   acc tgg gag atg cag cac gtt ctt ggc cac cac ccg tac acc aac ctc        672
   Thr Trp Glu Met Gln His Val Leu Gly His His Pro Tyr Thr Asn Leu
       210                 215                 220
   atc gag atg gag aac ggt ttg gcc aag gtc aag ggc gcc gac gtc gac        720
   Ile Glu Met Glu Asn Gly Leu Ala Lys Val Lys Gly Ala Asp Val Asp
   225                 230                 235                 240
   ccg aag aag gtc gac cag gag agc gac ccg gac gtc ttc agt acg tac        768
   Pro Lys Lys Val Asp Gln Glu Ser Asp Pro Asp Val Phe Ser Thr Tyr
                   245                 250                 255
   ccg atg ctt cgc ctg cac ccg tgg cac cgc cag cgg ttt tac cac aag        816
   Pro Met Leu Arg Leu His Pro Trp His Arg Gln Arg Phe Tyr His Lys
               260                 265                 270
   ttc cag cac ctg tac gcc ccg ttt atc ttt ggg ttt atg acg att aac        864
   Phe Gln His Leu Tyr Ala Pro Phe Ile Phe Gly Phe Met Thr Ile Asn
           275                 280                 285
   aag gtg att tcc cag gat gtc ggg gtt gtg ctg cgc aag cgc ctg ttc        912
   Lys Val Ile Ser Gln Asp Val Gly Val Val Leu Arg Lys Arg Leu Phe
       290                 295                 300
   cag atc gac gcc aac tgc cgg tat ggc agc ccc tgg tac gtg gcc cgc        960
   Gln Ile Asp Ala Asn Cys Arg Tyr Gly Ser Pro Trp Tyr Val Ala Arg
   305                 310                 315                 320
   ttc tgg atc atg aag ctc ctc acc acg ctc tac acg gtg gcg ctt ccc        1008
   Phe Trp Ile Met Lys Leu Leu Thr Thr Leu Tyr Thr Val Ala Leu Pro
                       325                     330                     335
   atg tac atg cag ggg cct gct cag ggc ttg aag ctt ttc ttc atg gcc      1056
   Met Tyr Met Gln Gly Pro Ala Gln Gly Leu Lys Leu Phe Phe Met Ala
               340                     345                     350
   cac ttc acc tgc gga gag gtc ctc gcc acc atg ttt att gtc aac cac      1104
   His Phe Thr Cys Gly Glu Val Leu Ala Thr Met Phe Ile Val Asn His
           355                     360                     365
   atc atc gag ggc gtc agc tac gct tcc aag gac gcg gtc aag ggc gtc      1152
   Ile Ile Glu Gly Val Ser Tyr Ala Ser Lys Asp Ala Val Lys Gly Val
       370                     375                     380
   atg gct ccg ccg cgc act gtg cac ggt gtc acc ccg atg cag gtg acg      1200
   Met Ala Pro Pro Arg Thr Val His Gly Val Thr Pro Met Gln Val Thr
   385                     390                     395                     400
   caa aag gcg ctc agt gcg gcc gag tcg acc aag tcg gac gcc gac aag      1248
   Gln Lys Ala Leu Ser Ala Ala Glu Ser Thr Lys Ser Asp Ala Asp Lys
                   405                     410                     415
   acg acc atg atc ccc ctc aac gac tgg gcc gct gtg cag tgc cag acc      1296
   Thr Thr Met Ile Pro Leu Asn Asp Trp Ala Ala Val Gln Cys Gln Thr
               420                     425                     430
   tct gtg aac tgg gct gtc ggg tcg tgg ttt tgg aac cac ttt tcg ggc      1344
   Ser Val Asn Trp Ala Val Gly Ser Trp Phe Trp Asn His Phe Ser Gly
           435                     440                     445
   ggc ctc aac cac cag att gag cac cac tgc ttc ccc caa aac ccc cac      1392
   Gly Leu Asn His Gln Ile Glu His His Cys Phe Pro Gln Asn Pro His
       450                     455                     460
   acg gtc aac gtc tac atc tcg ggc atc gtc aag gag acc tgc gaa gaa      1440
   Thr Val Asn Val Tyr Ile Ser Gly Ile Val Lys Glu Thr Cys Glu Glu
   465                     470                     475                     480
   tac ggc gtg ccg tac cag gct gag atc agc ctc ttc tct gcc tat ttc      1488
   Tyr Gly Val Pro Tyr Gln Ala Glu Ile Ser Leu Phe Ser Ala Tyr Phe
                   485                     490                     495
   aag atg ctg tcg cac ctc cgc acg ctc ggc aac gag gac ctc acg gcc      1536
   Lys Met Leu Ser His Leu Arg Thr Leu Gly Asn Glu Asp Leu Thr Ala
               500                     505                     510
   agg tcc acg tga                                                      1548
   Arg Ser Thr
           515

<210> 90
   <211> 515
   <212> PRT
   <213> Thraustochytrium aureum
<400> 90
   Met Thr Val Gly Phe Asp Glu Thr Val Thr Met Asp Thr Val Arg Asn
   1               5                   10                  15
   His Asn Met Pro Asp Asp Ala Trp Cys Ala Ile His Gly Thr Val Tyr
               20                  25                  30
   Asp Ile Thr Lys Phe Ser Lys Val His Pro Gly Gly Asp Ile Ile Met
           35                  40                  45
   Leu Ala Ala Gly Lys Glu Ala Thr Ile Leu Phe Glu Thr Tyr His Ile
       50                  55                  60
   Lys Gly Val Pro Asp Ala Val Leu Arg Lys Tyr Lys Val Gly Lys Leu
   65                  70                  75                  80
   Pro Gln Gly Lys Lys Gly Glu Thr Ser His Met Pro Thr Gly Leu Asp
                   85                  90                  95
   Ser Ala Ser Tyr Tyr Ser Trp Asp Ser Glu Phe Tyr Arg Val Leu Arg
               100                 105                 110
   Glu Arg Val Ala Lys Lys Leu Ala Glu Pro Gly Leu Met Gln Arg Ala
           115                 120                 125
   Arg Met Glu Leu Trp Ala Lys Ala Ile Phe Leu Leu Ala Gly Phe Trp
       130                 135                 140
   Gly Ser Leu Tyr Ala Met Cys Val Leu Asp Pro His Gly Gly Ala Met
   145                 150                 155                 160
   Val Ala Ala Val Thr Leu Gly Val Phe Ala Ala Phe Val Gly Thr Cys
                   165                 170                 175
   Ile Gln His Asp Gly Ser His Gly Ala Phe Ser Lys Ser Arg Phe Met
               180                 185                 190
   Asn Lys Ala Ala Gly Trp Thr Leu Asp Met Ile Gly Ala Ser Ala Met
           195                 200                 205
   Thr Trp Glu Met Gln His Val Leu Gly His His Pro Tyr Thr Asn Leu
       210                 215                 220
   Ile Glu Met Glu Asn Gly Leu Ala Lys Val Lys Gly Ala Asp Val Asp
   225                 230                 235                 240
   Pro Lys Lys Val Asp Gln Glu Ser Asp Pro Asp Val Phe Ser Thr Tyr
                   245                 250                 255
   Pro Met Leu Arg Leu His Pro Trp His Arg Gln Arg Phe Tyr His Lys
               260                 265                 270
   Phe Gln His Leu Tyr Ala Pro Phe Ile Phe Gly Phe Met Thr Ile Asn
           275                 280                 285
   Lys Val Ile Ser Gln Asp Val Gly Val Val Leu Arg Lys Arg Leu Phe
       290                 295                 300
   Gln Ile Asp Ala Asn Cys Arg Tyr Gly Ser Pro Trp Tyr Val Ala Arg
   305                 310                 315                 320
   Phe Trp Ile Met Lys Leu Leu Thr Thr Leu Tyr Thr Val Ala Leu Pro
                   325                 330                 335
   Met Tyr Met Gln Gly Pro Ala Gln Gly Leu Lys Leu Phe Phe Met Ala
               340                 345                 350
   His Phe Thr Cys Gly Glu Val Leu Ala Thr Met Phe Ile Val Asn His
           355                 360                 365
   Ile Ile Glu Gly Val Ser Tyr Ala Ser Lys Asp Ala Val Lys Gly Val
       370                 375                 380
   Met Ala Pro Pro Arg Thr Val His Gly Val Thr Pro Met Gln Val Thr
   385                 390                 395                 400
   Gln Lys Ala Leu Ser Ala Ala Glu Ser Thr Lys Ser Asp Ala Asp Lys
                   405                 410                 415
   Thr Thr Met Ile Pro Leu Asn Asp Trp Ala Ala Val Gln Cys Gln Thr
               420                 425                 430
   Ser Val Asn Trp Ala Val Gly Ser Trp Phe Trp Asn His Phe Ser Gly
           435                 440                 445
   Gly Leu Asn His Gln Ile Glu His His Cys Phe Pro Gln Asn Pro His
       450                 455                 460
   Thr Val Asn Val Tyr Ile Ser Gly Ile Val Lys Glu Thr Cys Glu Glu
   465                 470                 475                 480
   Tyr Gly Val Pro Tyr Gln Ala Glu Ile Ser Leu Phe Ser Ala Tyr Phe
                   485                 490                 495
   Lys Met Leu Ser His Leu Arg Thr Leu Gly Asn Glu Asp Leu Thr Ala
               500                 505                 510
   Arg Ser Thr
           515
<210> 91
   <211> 1548
   <212> DNA
   <213> Thraustochytrium aureum
<220>
   <221> CDS
   <222> (1)..(1548)
<400> 91
   atg acg gtc ggg ttt gac gaa acg gtg act atg gac acg gtc cgc aac        48
   Met Thr Val Gly Phe Asp Glu Thr Val Thr Met Asp Thr Val Arg Asn
   1               5                   10                  15
   cac aac atg ccg gac gac gcc tgg tgc gcg atc cac ggc acc gtg tac        96
   His Asn Met Pro Asp Asp Ala Trp Cys Ala Ile His Gly Thr Val Tyr
               20                  25                  30
   gac atc acc aag ttc agc aag gtg cac ccc ggc ggg gac atc atc atg        144
   Asp Ile Thr Lys Phe Ser Lys Val His Pro Gly Gly Asp Ile Ile Met
           35                  40                  45
   ctg gcc gct ggc aag gag gcc acc atc ctg ttc gag acc tac cac atc        192
   Leu Ala Ala Gly Lys Glu Ala Thr Ile Leu Phe Glu Thr Tyr His Ile
       50                  55                  60
   aag ggc gtc ccg gac gcg gtg ctg cgc aag tac aag gtc ggc aag ctc        240
   Lys Gly Val Pro Asp Ala Val Leu Arg Lys Tyr Lys Val Gly Lys Leu
   65                  70                  75                  80
   ccc cag ggc aag aag ggc gaa acg agc cac atg ccc acc ggg ctc gac 288
   Pro Gln Gly Lys Lys Gly Glu Thr Ser His Met Pro Thr Gly Leu Asp
   85 90 95
   tcg gcc tcc tac tac tcg tgg gac agc gag ttt tac agg gtg ctc cgc 336
   Ser Ala Ser Tyr Tyr Ser Trp Asp Ser Glu Phe Tyr Arg Val Leu Arg
   100 105 110
   gag cgc gtc gcc aag aag ctg gcc gag ccc ggc ctc atg cag cgc gcg 384
   Glu Arg Val Ala Lys Lys Leu Ala Glu Pro Gly Leu Met Gln Arg Ala
   115 120 125
   cgc atg gag ctc tgg gcc aag gcg atc ttc ctc ctg gca ggt ttc tgg 432
   Arg Met Glu Leu Trp Ala Lys Ala Ile Phe Leu Leu Ala Gly Phe Trp
   130 135 140
   ggc tcc ctt tac gcc atg tgc gtg cta gac ccg cac ggc ggt gcc atg 480
   Gly Ser Leu Tyr Ala Met Cys Val Leu Asp Pro His Gly Gly Ala Met
   145 150 155 160
   gta gcc gcc gtt acg ctc ggc gtg ttc gct gcc ttt gtc gga act tgc 528
   Val Ala Ala Val Thr Leu Gly Val Phe Ala Ala Phe Val Gly Thr Cys
   165 170 175
   atc cag cac gac ggc agc cac ggc gcc ttc tcc aag tcg cga ttc atg 576
   Ile Gln His Asp Gly Ser His Gly Ala Phe Ser Lys Ser Arg Phe Met
   180 185 190
   aac aag gcg gcg ggc tgg acc ctc gac atg atc ggc gcg agc gcg atg 624
   Asn Lys Ala Ala Gly Trp Thr Leu Asp Met Ile Gly Ala Ser Ala Met
   195 200 205
   acc tgg gag atg cag cac gtt ctt ggt cac cac ccg tac acc aac ctc 672
   Thr Trp Glu Met Gln His Val Leu Gly His His Pro Tyr Thr Asn Leu
   210 215 220
   atc gag atg gag aac ggt ttg gcc aag gtc aag ggc gcc gac gtc gac 720
   Ile Glu Met Glu Asn Gly Leu Ala Lys Val Lys Gly Ala Asp Val Asp
   225 230 235 240
   ccg aag aag gtc gac cag gag agc gac ccg gac gtc ttc agt acg tac 768
   Pro Lys Lys Val Asp Gln Glu Ser Asp Pro Asp Val Phe Ser Thr Tyr
   245 250 255
   ccg atg ctt cgc ctg cac ccg tgg cac cgc cag cgg ttt tac cac aag 816
   Pro Met Leu Arg Leu His Pro Trp His Arg Gln Arg Phe Tyr His Lys
   260 265 270
   ttc cag cac ctg tac gcc ccg ctt atc ttt ggg ttt atg acg att aac 864
   Phe Gln His Leu Tyr Ala Pro Leu Ile Phe Gly Phe Met Thr Ile Asn
   275 280 285
   aag gtg att tcc cag gat gtc ggg gtt gtg ctg cgc aag cgc ctg ttc 912
   Lys Val Ile Ser Gln Asp Val Gly Val Val Leu Arg Lys Arg Leu Phe
   290 295 300
   cag atc gac gcc aac tgc cgg tat ggc agc ccc tgg aac gtg gcc cgc 960
   Gln Ile Asp Ala Asn Cys Arg Tyr Gly Ser Pro Trp Asn Val Ala Arg
   305 310 315 320
   ttc tgg atc atg aag ctc ctc acc acg ctc tac atg gtg gcg ctt ccc 1008
   Phe Trp Ile Met Lys Leu Leu Thr Thr Leu Tyr Met Val Ala Leu Pro
   325 330 335
   atg tac atg cag ggg cct gct cag ggc ttg aag ctt ttc ttc atg gcc 1056
   Met Tyr Met Gln Gly Pro Ala Gln Gly Leu Lys Leu Phe Phe Met Ala
   340 345 350
   cac ttc acc tgc gga gag gtc ctc gcc acc atg ttt att gtc aac cac 1104
   His Phe Thr Cys Gly Glu Val Leu Ala Thr Met Phe Ile Val Asn His
   355 360 365
   atc atc gag ggc gtc agc tac gct tcc aag gac gcg gtc aag ggc gtc 1152
   Ile Ile Glu Gly Val Ser Tyr Ala Ser Lys Asp Ala Val Lys Gly Val
   370 375 380
   atg gct ccg ccg cgc act gtg cac ggt gtc acc ccg atg cag gtg acg 1200
   Met Ala Pro Pro Arg Thr Val His Gly Val Thr Pro Met Gln Val Thr
   385 390 395 400
   caa aag gcg ctc agt gcg gcc gag tcg acc aag tcg gac gcc gac aag 1248
   Gln Lys Ala Leu Ser Ala Ala Glu Ser Thr Lys Ser Asp Ala Asp Lys
   405 410 415
   acg acc atg atc ccc ctc aac gac tgg gcc gct gtg cag tgc cag acc 1296
   Thr Thr Met Ile Pro Leu Asn Asp Trp Ala Ala Val Gln Cys Gln Thr
   420 425 430
   tct gtg aac tgg gct gtc ggg tcg tgg ttt tgg aac cac ttt tcg ggc 1344
   Ser Val Asn Trp Ala Val Gly Ser Trp Phe Trp Asn His Phe Ser Gly
               435                 440                 445
   ggc cta aac cac cag att gag cac cac tgc ttc ccc caa aac ccc cac      1392
   Gly Leu Asn His Gln Ile Glu His His Cys Phe Pro Gln Asn Pro His
           450                 455                 460
   acg gtc aac gtc tac atc tcg ggc atc gtc aag gag acc tgc gaa gaa      1440
   Thr Val Asn Val Tyr Ile Ser Gly Ile Val Lys Glu Thr Cys Glu Glu
   465                 470                 475                 480
   tac ggc gtg ccg tac cag gct gag atc agc ctc ttc tct gcc tat ttc      1488
   Tyr Gly Val Pro Tyr Gln Ala Glu Ile Ser Leu Phe Ser Ala Tyr Phe
                   485                 490                 495
   aag atg ctg tcg cac ctc cgc acg ctc ggc aac gag gac ctc acg gcc      1536
   Lys Met Leu Ser His Leu Arg Thr Leu Gly Asn Glu Asp Leu Thr Ala
               500                 505                 510
   tgg tcc acg tga                                                      1548
   Trp Ser Thr
           515
<210> 92
   <211> 515
   <212> PRT
   <213> Thraustochytrium aureum
<400> 92
   Met Thr Val Gly Phe Asp Glu Thr Val Thr Met Asp Thr Val Arg Asn
   1               5                   10                  15
   His Asn Met Pro Asp Asp Ala Trp Cys Ala Ile His Gly Thr Val Tyr
               20                  25                  30
   Asp Ile Thr Lys Phe Ser Lys Val His Pro Gly Gly Asp Ile Ile Met
           35                  40                  45
   Leu Ala Ala Gly Lys Glu Ala Thr Ile Leu Phe Glu Thr Tyr His Ile
       50                  55                  60
   Lys Gly Val Pro Asp Ala Val Leu Arg Lys Tyr Lys Val Gly Lys Leu
   65                  70                  75                  80
   Pro Gln Gly Lys Lys Gly Glu Thr Ser His Met Pro Thr Gly Leu Asp
                   85                  90                  95
   Ser Ala Ser Tyr Tyr Ser Trp Asp Ser Glu Phe Tyr Arg Val Leu Arg
               100                 105                 110
   Glu Arg Val Ala Lys Lys Leu Ala Glu Pro Gly Leu Met Gln Arg Ala
           115                 120                 125
   Arg Met Glu Leu Trp Ala Lys Ala Ile Phe Leu Leu Ala Gly Phe Trp
       130                 135                 140
   Gly Ser Leu Tyr Ala Met Cys Val Leu Asp Pro His Gly Gly Ala Met
   145                 150                 155                 160
   Val Ala Ala Val Thr Leu Gly Val Phe Ala Ala Phe Val Gly Thr Cys
                   165                 170                 175
   Ile Gln His Asp Gly Ser His Gly Ala Phe Ser Lys Ser Arg Phe Met
               180                 185                 190
   Asn Lys Ala Ala Gly Trp Thr Leu Asp Met Ile Gly Ala Ser Ala Met
           195                 200                 205
   Thr Trp Glu Met Gln His Val Leu Gly His His Pro Tyr Thr Asn Leu
       210                 215                 220
   Ile Glu Met Glu Asn Gly Leu Ala Lys Val Lys Gly Ala Asp Val Asp
   225                 230                 235                 240
   Pro Lys Lys Val Asp Gln Glu Ser Asp Pro Asp Val Phe Ser Thr Tyr
                   245                 250                 255
   Pro Met Leu Arg Leu His Pro Trp His Arg Gln Arg Phe Tyr His Lys
               260                 265                 270
   Phe Gln His Leu Tyr Ala Pro Leu Ile Phe Gly Phe Met Thr Ile Asn
           275                 280                 285
   Lys Val Ile Ser Gln Asp Val Gly Val Val Leu Arg Lys Arg Leu Phe
       290                 295                 300
   Gln Ile Asp Ala Asn Cys Arg Tyr Gly Ser Pro Trp Asn Val Ala Arg
   305                 310                 315                 320
   Phe Trp Ile Met Lys Leu Leu Thr Thr Leu Tyr Met Val Ala Leu Pro
                   325                 330                 335
   Met Tyr Met Gln Gly Pro Ala Gln Gly Leu Lys Leu Phe Phe Met Ala
               340                 345                 350
   His Phe Thr Cys Gly Glu Val Leu Ala Thr Met Phe Ile Val Asn His
                   355                         360                         365
   Ile Ile Glu Gly Val Ser Tyr Ala Ser Lys Asp Ala Val Lys Gly Val
       370                     375                     380
   Met Ala Pro Pro Arg Thr Val His Gly Val Thr Pro Met Gln Val Thr
   385                     390                     395                     400
   Gln Lys Ala Leu Ser Ala Ala Glu Ser Thr Lys Ser Asp Ala Asp Lys
                   405                     410                     415
   Thr Thr Met Ile Pro Leu Asn Asp Trp Ala Ala Val Gln Cys Gln Thr
               420                     425                     430
   Ser Val Asn Trp Ala Val Gly Ser Trp Phe Trp Asn His Phe Ser Gly
           435                     440                     445
   Gly Leu Asn His Gln Ile Glu His His Cys Phe Pro Gln Asn Pro His
       450                     455                     460
   Thr Val Asn Val Tyr Ile Ser Gly Ile Val Lys Glu Thr Cys Glu Glu
   465                     470                     475                     480
   Tyr Gly Val Pro Tyr Gln Ala Glu Ile Ser Leu Phe Ser Ala Tyr Phe
                   485                     490                     495
   Lys Met Leu Ser His Leu Arg Thr Leu Gly Asn Glu Asp Leu Thr Ala
               500                     505                     510
   Trp Ser Thr
           515
<210> 93
   <211> 1560
   <212> DNA
   <213> Thraustochytrium sp
<220>
   <221> CDS
   <222> (1)..(1560)
<400> 93
   atg acg gtc ggc tac gac gag gag atc ccg ttc gag cag gtc cgc gcg      48
   Met Thr Val Gly Tyr Asp Glu Glu Ile Pro Phe Glu Gln Val Arg Ala
   1               5                   10                  15
   cac aac aag ccg gat gac gcc tgg tgc gcg atc cac ggg cac gtg tac      96
   His Asn Lys Pro Asp Asp Ala Trp Cys Ala Ile His Gly His Val Tyr
               20                  25                  30
   gat gtg acc aag ttc gcg agc gtg cac ccg ggc ggc gac att atc ctg      144
   Asp Val Thr Lys Phe Ala Ser Val His Pro Gly Gly Asp Ile Ile Leu
           35                  40                  45
   ctg gcc gca ggc aag gag gcc acc gtg ctg tac gag act tac cat gtg      192
   Leu Ala Ala Gly Lys Glu Ala Thr Val Leu Tyr Glu Thr Tyr His Val
       50                  55                  60
   cgg ggc gtc tcg gac gcg gtg ctg cgc aag tac cgc atc ggc aag ctg      240
   Arg Gly Val Ser Asp Ala Val Leu Arg Lys Tyr Arg Ile Gly Lys Leu
   65                  70                  75                  80
   ccg gac ggc caa ggc ggc gcg aac gag aag gaa aag cgg acg ctc tcg      288
   Pro Asp Gly Gln Gly Gly Ala Asn Glu Lys Glu Lys Arg Thr Leu Ser
                   85                  90                  95
   ggc ctc tcg tcg gcc tcg tac tac acg tgg aac agc gac ttt tac agg      336
   Gly Leu Ser Ser Ala Ser Tyr Tyr Thr Trp Asn Ser Asp Phe Tyr Arg
               100                 105                 110
   gta atg cgc gag cgc gtc gtg gct cgg ctc aag gag cgc ggc aag gcc      384
   Val Met Arg Glu Arg Val Val Ala Arg Leu Lys Glu Arg Gly Lys Ala
           115                 120                 125
   cgc cgc gga ggc tac gag ctc tgg atc aag gcg ttc ctg ctg ctc gtc      432
   Arg Arg Gly Gly Tyr Glu Leu Trp Ile Lys Ala Phe Leu Leu Leu Val
       130                 135                 140
   ggc ttc tgg agc tcg ctg tac tgg atg tgc acg ctg gac ccc tcg ttc      480
   Gly Phe Trp Ser Ser Leu Tyr Trp Met Cys Thr Leu Asp Pro Ser Phe
   145                 150                 155                 160
   ggg gcc atc ctg gcc gcc atg tcg ctg ggc gtc ttt gcc gcc ttt gtg      528
   Gly Ala Ile Leu Ala Ala Met Ser Leu Gly Val Phe Ala Ala Phe Val
                   165                 170                 175
   ggc acg tgc atc cag cac gac ggc aac cac ggc gcc ttt gcc cag tcg      576
   Gly Thr Cys Ile Gln His Asp Gly Asn His Gly Ala Phe Ala Gln Ser
               180                 185                 190
   cga tgg gtc aac aag gtt gcc ggg tgg acg ctc gac atg atc ggc gcc      624
   Arg Trp Val Asn Lys Val Ala Gly Trp Thr Leu Asp Met Ile Gly Ala
           195                 200                 205
   agc ggc atg acg tgg gag ttc cag cac gtc ctg ggc cac cat ccg tac      672
   Ser Gly Met Thr Trp Glu Phe Gln His Val Leu Gly His His Pro Tyr
       210                 215                 220
   acg aac ctg atc gag gag gag aac ggc ctg caa aag gtg agc ggc aag      720
   Thr Asn Leu Ile Glu Glu Glu Asn Gly Leu Gln Lys Val Ser Gly Lys
   225                 230                 235                 240
   aag atg gac acc aag ctg gcc gac cag gag agc gat ccg gac gtc ttt      768
   Lys Met Asp Thr Lys Leu Ala Asp Gln Glu Ser Asp Pro Asp Val Phe
                   245                 250                 255
   tcc acg tac ccg atg atg cgc ctg cac ccg tgg cac cag aag cgc tgg      816
   Ser Thr Tyr Pro Met Met Arg Leu His Pro Trp His Gln Lys Arg Trp
               260                 265                 270
   tac cac cgt ttc cag cac att tac ggc ccc ttc atc ttt ggc ttc atg      864
   Tyr His Arg Phe Gln His Ile Tyr Gly Pro Phe Ile Phe Gly Phe Met
           275                 280                 285
   acc atc aac aag gtg gtc acg cag gac gtc ggt gtg gtg ctc cgc aag      912
   Thr Ile Asn Lys Val Val Thr Gln Asp Val Gly Val Val Leu Arg Lys
       290                 295                 300
   cgg ctc ttc cag att gac gcc gag tgc cgg tac gcg agc cca atg tac      960
   Arg Leu Phe Gln Ile Asp Ala Glu Cys Arg Tyr Ala Ser Pro Met Tyr
   305                 310                 315                 320
   gtg gcg cgt ttc tgg atc atg aag gcg ctc acg gtg ctc tac atg gtg      1008
   Val Ala Arg Phe Trp Ile Met Lys Ala Leu Thr Val Leu Tyr Met Val
                   325                 330                 335
   gcc ctg ccg tgc tac atg cag ggc ccg tgg cac ggc ctc aag ctg ttc      1056
   Ala Leu Pro Cys Tyr Met Gln Gly Pro Trp His Gly Leu Lys Leu Phe
               340                 345                 350
   gcg atc gcg cac ttt acg tgc ggc gag gtg ctc gca acc atg ttc att      1104
   Ala Ile Ala His Phe Thr Cys Gly Glu Val Leu Ala Thr Met Phe Ile
           355                 360                 365
   gtg aac cac atc atc gag ggc gtc tcg tac gct tcc aag gac gcg gtc      1152
   Val Asn His Ile Ile Glu Gly Val Ser Tyr Ala Ser Lys Asp Ala Val
       370                 375                 380
   aag ggc acg atg gcg ccg ccg aag acg atg cac ggc gtg acg ccc atg      1200
   Lys Gly Thr Met Ala Pro Pro Lys Thr Met His Gly Val Thr Pro Met
   385                 390                 395                 400
   aac aac acg cgc aag gag gtg gag gcg gag gcg tcc aag tct ggc gcc      1248
   Asn Asn Thr Arg Lys Glu Val Glu Ala Glu Ala Ser Lys Ser Gly Ala
                   405                 410                 415
   gtg gtc aag tca gtc ccg ctc gac gac tgg gcc gtc gtc cag tgc cag      1296
   Val Val Lys Ser Val Pro Leu Asp Asp Trp Ala Val Val Gln Cys Gln
               420                 425                 430
   acc tcg gtg aac tgg agc gtc ggc tcg tgg ttc tgg aat cac ttt tcc      1344
   Thr Ser Val Asn Trp Ser Val Gly Ser Trp Phe Trp Asn His Phe Ser
           435                 440                 445
   ggc ggc ctc aac cac cag att gag cac cac ctg ttc ccc ggr ctc agc      1392
   Gly Gly Leu Asn His Gln Ile Glu His His Leu Phe Pro Gly Leu Ser
       450                 455                 460
   cac gag acg tac tac cac att cag gac gtc ttt cag tcc acc tgc gcc      1440
   His Glu Thr Tyr Tyr His Ile Gln Asp Val Phe Gln Ser Thr Cys Ala
   465                 470                 475                 480
   gag tac ggc gtc ccg tac cag cac gag cct tcg ctc tgg acc gcg tac      1488
   Glu Tyr Gly Val Pro Tyr Gln His Glu Pro Ser Leu Trp Thr Ala Tyr
                   485                 490                 495
   tgg aag atg ctc gag cac ctc cgt cag ctc ggc aat gag gag acc cac      1536
   Trp Lys Met Leu Glu His Leu Arg Gln Leu Gly Asn Glu Glu Thr His
               500                 505                 510
   gag tcc tgg cag cgc gct gcc tga                                      1560
   Glu Ser Trp Gln Arg Ala Ala
           515
<210> 94
   <211> 519
   <212> PRT
   <213> Thraustochytrium sp
<400> 94
   Met Thr Val Gly Tyr Asp Glu Glu Ile Pro Phe Glu Gln Val Arg Ala
   1               5                   10                  15
   His Asn Lys Pro Asp Asp Ala Trp Cys Ala Ile His Gly His Val Tyr
               20                  25                  30
   Asp Val Thr Lys Phe Ala Ser Val His Pro Gly Gly Asp Ile Ile Leu
           35                  40                  45
   Leu Ala Ala Gly Lys Glu Ala Thr Val Leu Tyr Glu Thr Tyr His Val
       50                  55                  60
   Arg Gly Val Ser Asp Ala Val Leu Arg Lys Tyr Arg Ile Gly Lys Leu
   65                  70                  75                  80
   Pro Asp Gly Gln Gly Gly Ala Asn Glu Lys Glu Lys Arg Thr Leu Ser
                   85                  90                  95
   Gly Leu Ser Ser Ala Ser Tyr Tyr Thr Trp Asn Ser Asp Phe Tyr Arg
               100                 105                 110
   Val Met Arg Glu Arg Val Val Ala Arg Leu Lys Glu Arg Gly Lys Ala
           115                 120                 125
   Arg Arg Gly Gly Tyr Glu Leu Trp Ile Lys Ala Phe Leu Leu Leu Val
       130                 135                 140
   Gly Phe Trp Ser Ser Leu Tyr Trp Met Cys Thr Leu Asp Pro Ser Phe
   145                 150                 155                 160
   Gly Ala Ile Leu Ala Ala Met Ser Leu Gly Val Phe Ala Ala Phe Val
                   165                 170                 175
   Gly Thr Cys Ile Gln His Asp Gly Asn His Gly Ala Phe Ala Gln Ser
               180                 185                 190
   Arg Trp Val Asn Lys Val Ala Gly Trp Thr Leu Asp Met Ile Gly Ala
           195                 200                 205
   Ser Gly Met Thr Trp Glu Phe Gln His Val Leu Gly His His Pro Tyr
       210                 215                 220
   Thr Asn Leu Ile Glu Glu Glu Asn Gly Leu Gln Lys Val Ser Gly Lys
   225                 230                 235                 240
   Lys Met Asp Thr Lys Leu Ala Asp Gln Glu Ser Asp Pro Asp Val Phe
                   245                 250                 255
   Ser Thr Tyr Pro Met Met Arg Leu His Pro Trp His Gln Lys Arg Trp
               260                 265                 270
   Tyr His Arg Phe Gln His Ile Tyr Gly Pro Phe Ile Phe Gly Phe Met
           275                 280                 285
   Thr Ile Asn Lys Val Val Thr Gln Asp Val Gly Val Val Leu Arg Lys
       290                 295                 300
   Arg Leu Phe Gln Ile Asp Ala Glu Cys Arg Tyr Ala Ser Pro Met Tyr
   305                 310                 315                 320
   Val Ala Arg Phe Trp Ile Met Lys Ala Leu Thr Val Leu Tyr Met Val
                   325                 330                 335
   Ala Leu Pro Cys Tyr Met Gln Gly Pro Trp His Gly Leu Lys Leu Phe
               340                 345                 350
   Ala Ile Ala His Phe Thr Cys Gly Glu Val Leu Ala Thr Met Phe Ile
           355                 360                 365
   Val Asn His Ile Ile Glu Gly Val Ser Tyr Ala Ser Lys Asp Ala Val
       370                 375                 380
   Lys Gly Thr Met Ala Pro Pro Lys Thr Met His Gly Val Thr Pro Met
   385                 390                 395                 400
   Asn Asn Thr Arg Lys Glu Val Glu Ala Glu Ala Ser Lys Ser Gly Ala
                   405                 410                 415
   Val Val Lys Ser Val Pro Leu Asp Asp Trp Ala Val Val Gln Cys Gln
               420                 425                 430
   Thr Ser Val Asn Trp Ser Val Gly Ser Trp Phe Trp Asn His Phe Ser
           435                 440                 445
   Gly Gly Leu Asn His Gln Ile Glu His His Leu Phe Pro Gly Leu Ser
       450                 455                 460
   His Glu Thr Tyr Tyr His Ile Gln Asp Val Phe Gln Ser Thr Cys Ala
   465                 470                 475                 480
   Glu Tyr Gly Val Pro Tyr Gln His Glu Pro Ser Leu Trp Thr Ala Tyr
                   485                 490                 495
   Trp Lys Met Leu Glu His Leu Arg Gln Leu Gly Asn Glu Glu Thr His
               500                 505                 510
   Glu Ser Trp Gln Arg Ala Ala
           515
<210> 95
   <211> 575
   <212> PRT
   <213> Artificial sequence
<220>
   <221> Variant
   <222> (2)..(3)
   <223> xaa in position 2 to 3 is any amino acid
<220>
   <221> Variant
   <222> (5)..(13)
   <223> xaa in position 5 to 13 is any amino acid
<220>
   <221> Variant
   <222> (14)..(48)
   <223> xaa in position 14 to 48 is any or no amino acid
<220>
   <221> Variant
   <222> (51)..(51)
   <223> xaa in position 51 is any amino acid
<220>
   <221> Variant
   <222> (54)..(54)
   <223> xaa in position 54 is any amino acid
<220>
   <221> Variant
   <222> (56)..(56)
   <223> xaa in position 56 is any amino acid
<220>
   <221> Variant
   <222> (62)..(62)
   <223> xaa in position 62 is any amino acid
<220>
   <221> Variant
   <222> (64)..(65)
   <223> xaa in position 64 to 65 is any amino acid
<220>
   <221> Variant
   <222> (66)..(81)
   <223> xaa in position 66 to 81 is any or no amino acid
<220>
   <221> variant
   <222> (85)..(85)
   <223> xaa in position 85 is any amino acid
<220>
   <221> Variant
   <222> (87)..(87)
   <223> xaa in position 87 is any amino acid
<220>
   <221> Variant
   <222> (89)..(91)
   <223> xaa in position 89 to 91 is any amino acid
<220>
   <221> Variant
   <222> (97)..(97)
   <223> xaa in position 97 is any amino acid
<220>
   <221> Variant
   <222> (99)..(99)
   <223> xaa in position 99 is any amino acid
<220>
   <221> Variant
   <222> (102)..(102)
   <223> xaa in position 102 is any amino acid
<220>
   <221> Variant
   <222> (105)..(105)
   <223> xaa in position 105 is any amino acid
<220>
   <221> Variant
   <222> (108)..(108)
   <223> xaa in position 108 is any amino acid
<220>
   <221> Variant
   <222> (110)..(110)
   <223> xaa in position 110 is any amino acid
<220>
   <221> Variant
   <222> (115)..(116)
   <223> xaa in position 115 to 116 is any amino acid
<220>
   <221> Variant
   <222> (119)..(119)
   <223> xaa in position 119 is any amino acid
<220>
   <221> Variant
   <222> (121)..(121)
   <223> xaa in position 121 is any amino acid
<220>
   <221> Variant
   <222> (123)..(124)
   <223> xaa in position 123 to 124 is any amino acid
<220>
   <221> Variant
   <222> (127)..(128)
   <223> xaa in position 127 to 128 is any amino acid
<220>
   <221> variant
   <222> (130)..(130)
   <223> xaa in position 130 is any amino acid
<220>
   <221> Variant
   <222> (132)..(137)
   <223> xaa in position 132 to 137 is any amino acid
<220>
   <221> Variant
   <222> (138)..(154)
   <223> xaa in position 138 to 154 is any or no amino acid
<220>
   <221> Variant
   <222> (156)..(156)
   <223> xaa in position 156 is any amino acid
<220>
   <221> Variant
   <222> (158)..(158)
   <223> xaa in position 158 is any amino acid
<220>
   <221> Variant
   <222> (159)..(159)
   <223> xaa in position 159 is any or no amino acid
<220>
   <221> Variant
   <222> (161)..(161)
   <223> xaa in position 161 is any amino acid
<220>
   <221> Variant
   <222> (164)..(164)
   <223> xaa in position 164 is any amino acid
<220>
   <221> Variant
   <222> (166)..(168)
   <223> xaa in position 166 to 168 is any amino acid
<220>
   <221> variant
   <222> (171)..(173)
   <223> xaa in position 171 to 173 is any amino acid
<220>
   <221> Variant
   <222> (175)..(175)
   <223> xaa in position 175 is any amino acid
<220>
   <221> Variant
   <222> (177)..(177)
   <223> xaa in position 177 is any amino acid
<220>
   <221> Variant
   <222> (179)..(181)
   <223> xaa in position 179 to 181 is any amino acid
<220>
   <221> Variant
   <222> (183)..(184)
   <223> xaa in position 183 to 184 is any amino acid
<220>
   <221> Variant
   <222> (185)..(185)
   <223> xaa in position 185 is any or no amino acid
<220>
   <221> Variant
   <222> (189)..(189)
   <223> xaa in position 189 is any amino acid
<220>
   <221> Variant
   <222> (192)..(193)
   <223> xaa in position 192 to 193 is any amino acid
<220>
   <221> Variant
   <222> (196)..(196)
   <223> xaa in position 196 is any amino acid
<220>
   <221> Variant
   <222> (200)..(200)
   <223> xaa in position 200 is any amino acid
<220>
   <221> Variant
   <222> (204)..(204)
   <223> xaa in position 204 is any amino acid
<220>
   <221> Variant
   <222> (206)..(207)
   <223> xaa in position 206 to 207 is any amino acid
<220>
   <221> Variant
   <222> (208)..(211)
   <223> xaa in position 208 to 211 is any or no amino acid
<220>
   <221> Variant
   <222> (213)..(214)
   <223> xaa in position 213 to 214 is any amino acid
<220>
   <221> Variant
   <222> (215)..(216)
   <223> xaa in position 215 to 216 is any or no amino acid
<220>
   <221> Variant
   <222> (218)..(220)
   <223> xaa in position 218 to 220 is any amino acid
<220>
   <221> Variant
   <222> (222)..(224)
   <223> xaa in position 222 to 224 is any amino acid
<220>
   <221> Variant
   <222> (226)..(226)
   <223> xaa in position 226 is any amino acid
<220>
   <221> variant
   <222> (240)..(240)
   <223> xaa in position 240 is any amino acid
<220>
   <221> Variant
   <222> (245)..(250)
   <223> xaa in position 245 to 250 is any amino acid
<220>
   <221> Variant
   <222> (253)..(253)
   <223> xaa in position 253 is any amino acid
<220>
   <221> Variant
   <222> (265)..(266)
   <223> xaa in position 265 to 266 is any amino acid
<220>
   <221> variant
   <222> (270)..(270)
   <223> xaa in position 270 is any amino acid
<220>
   <221> Variant
   <222> (273)..(273)
   <223> xaa in position 273 is any amino acid
<220>
   <221> Variant
   <222> (283)..(284)
   <223> xaa in position 283 to 284 is any amino acid
<220>
   <221> Variant
   <222> (285)..(290)
   <223> xaa in position 285 to 290 is any or no amino acid
<220>
   <221> variant
   <222> (292)..(293)
   <223> xaa in position 292 to 293 is any amino acid
<220>
   <221> Variant
   <222> (295)..(297)
   <223> xaa in position 295 to 297 is any amino acid
<220>
   <221> Variant
   <222> (299)..(302)
   <223> xaa in position 299 to 302 is any amino acid
<220>
   <221> variant
   <222> (313)..(313)
   <223> xaa in position 313 is any amino acid
<220>
   <221> variant
   <222> (316)..(317)
   <223> xaa in position 316 to 317 is any amino acid
<220>
   <221> Variant
   <222> (319)..(319)
   <223> xaa in position 319 is any amino acid
<220>
   <221> Variant
   <222> (322)..(322)
   <223> xaa in position 322 is any amino acid
<220>
   <221> Variant
   <222> (324)..(327)
   <223> xaa in position 324 to 327 is any amino acid
<220>
   <221> Variant
   <222> (330)..(330)
   <223> xaa in position 330 is any amino acid
<220>
   <221> Variant
   <222> (334)..(334)
   <223> xaa in position 334 is any amino acid
<220>
   <221> Variant
   <222> (336)..(336)
   <223> xaa in position 336 is any amino acid
<220>
   <221> Variant
   <222> (338)..(339)
   <223> xaa in position 338 to 339 is any amino acid
<220>
   <221> Variant
   <222> (341)..(342)
   <223> xaa in position 341 to 342 is any amino acid
<220>
   <221> Variant
   <222> (346)..(346)
   <223> xaa in position 346 is any amino acid
<220>
   <221> Variant
   <222> (349)..(350)
   <223> xaa in position 349 to 350 is any amino acid
<220>
   <221> Variant
   <222> (353)..(354)
   <223> xaa in position 353 to 354 is any amino acid
<220>
   <221> Variant
   <222> (356)..(359)
   <223> xaa in position 356 to 359 is any amino acid
<220>
   <221> Variant
   <222> (362)..(363)
   <223> xaa in position 362 to 363 is any amino acid
<220>
   <221> Variant
   <222> (367)..(368)
   <223> xaa in position 367 to 368 is any amino acid
<220>
   <221> Variant
   <222> (371)..(371)
   <223> xaa in position 371 is any amino acid
<220>
   <221> Variant
   <222> (373)..(375)
   <223> xaa in position 373 to 375 is any amino acid
<220>
   <221> Variant
   <222> (377)..(377)
   <223> xaa in position 377 is any amino acid
<220>
   <221> Variant
   <222> (381)..(381)
   <223> xaa in position 381 is any amino acid
<220>
   <221> Variant
   <222> (384)..(385)
   <223> xaa in position 384 to 385 is any amino acid
<220>
   <221> Variant
   <222> (387)..(387)
   <223> xaa in position 387 is any amino acid
<220>
   <221> Variant
   <222> (390)..(392)
   <223> xaa in position 390 to 392 is any amino acid
<220>
   <221> Variant
   <222> (395)..(395)
   <223> xaa in position 395 is any amino acid
<220>
   <221> Variant
   <222> (397)..(398)
   <223> xaa in position 397 to 398 is any amino acid
<220>
   <221> Variant
   <222> (400)..(402)
   <223> xaa in position 400 to 402 is any amino acid
<220>
   <221> Variant
   <222> (405)..(405)
   <223> xaa in position 405 is any amino acid
<220>
   <221> Variant
   <222> (408)..(409)
   <223> xaa in position 408 to 409 is any amino acid
<220>
   <221> Variant
   <222> (412)..(413)
   <223> xaa in position 412 to 413 is any amino acid
<220>
   <221> variant
   <222> (417)..(417)
   <223> xaa in position 417 is any amino acid
<220>
   <221> Variant
   <222> (427)..(427)
   <223> xaa in position 427 is any amino acid
<220>
   <221> Variant
   <222> (434)..(435)
   <223> xaa in position 434 to 435 is any amino acid
<220>
   <221> Variant
   <222> (438)..(438)
   <223> xaa in position 438 is any amino acid
<220>
   <221> variant
   <222> (440)..(440)
   <223> xaa in position 440 is any amino acid
<220>
   <221> Variant
   <222> (442)..(443)
   <223> xaa in position 442 to 443 is any amino acid
<220>
   <221> Variant
   <222> (444)..(452)
   <223> xaa in position 444 to 452 is any or no amino acid
<220>
   <221> Variant
   <222> (454)..(454)
   <223> xaa in position 454 is any amino acid
<220>
   <221> Variant
   <222> (456)..(457)
   <223> xaa in position 456 to 457 is any amino acid
<220>
   <221> Variant
   <222> (459)..(459)
   <223> xaa in position 459 is any amino acid
<220>
   <221> Variant
   <222> (463)..(464)
   <223> xaa in position 463 to 464 is any amino acid
<220>
   <221> Variant
   <222> (466)..(466)
   <223> xaa in position 466 is any amino acid
<220>
   <221> Variant
   <222> (468)..(472)
   <223> xaa in position 468 to 472 is any amino acid
<220>
   <221> Variant
   <222> (473)..(473)
   <223> xaa in position 473 is any or no amino acid
<220>
   <221> Variant
   <222> (475)..(483)
   <223> xaa in position 475 to 483 is any amino acid
<220>
   <221> Variant
   <222> (484)..(489)
   <223> xaa in position 484 to 489 is any or no amino acid
<220>
   <221> Variant
   <222> (491)..(492)
   <223> xaa in position 491 to 492 is any amino acid
<220>
   <221> Variant
   <222> (496)..(496)
   <223> xaa in position 496 is any amino acid
<220>
   <221> Variant
   <222> (506)..(507)
   <223> xaa in position 506 to 507 is any amino acid
<220>
   <221> Variant
   <222> (527)..(527)
   <223> xaa in position 527 is any amino acid
<220>
   <221> Variant
   <222> (530)..(532)
   <223> xaa in position 530 to 532 is any amino acid
<220>
   <221> Variant
   <222> (534)..(538)
   <223> xaa in position 534 to 538 is any amino acid
<220>
   <221> Variant
   <222> (540)..(542)
   <223> xaa in position 540 to 542 is any amino acid
<220>
   <221> Variant
   <222> (544)..(545)
   <223> xaa in position 544 to 545 is any amino acid
<220>
   <221> Variant
   <222> (548)..(548)
   <223> xaa in position 548 is any amino acid
<220>
   <221> Variant
   <222> (556)..(556)
   <223> xaa in position 556 is any amino acid
<220>
   <221> Variant
   <222> (558)..(559)
   <223> xaa in position 558 to 559 is any amino acid
<220>
   <221> Variant
   <222> (561)..(562)
   <223> xaa in position 561 to 562 is any amino acid
<220>
   <221> Variant
   <222> (565)..(565)
   <223> xaa in position 565 is any amino acid
<220>
   <221> Variant
   <222> (568)..(569)
   <223> xaa in position 568 to 569 is any amino acid
<220>
   <221> Variant
   <222> (573)..(573)
   <223> xaa in position 573 is any amino acid
<400> 95
   Met Xaa Xaa Gly Xaa Xaa Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Val Arg Xaa His Asn Xaa Pro Xaa Asp Ala Trp Cys Ala Xaa His Xaa
       50                  55                  60
   Xaa Xaa Xaa Xaa Xaa Xaa Xaa Xaa Xaa Xaa Xaa Xaa Xaa Xaa Xaa Xaa
   65                  70                  75                  80
   Xaa Val Tyr Asp Xaa Thr Xaa Phe Xaa Xaa Xaa His Pro Gly Gly Asp
                   85                  90                  95
   Xaa Ile Xaa Leu Ala Xaa Gly Lys Xaa Ala Thr Xaa Leu Xaa Glu Thr
               100                 105                 110
   Tyr His Xaa Xaa Gly Val Xaa Asp Xaa Val Xaa Xaa Lys Tyr Xaa Xaa
           115                 120                 125
   Gly Xaa Leu Xaa Xaa Xaa Xaa Xaa Xaa Xaa Xaa Xaa Xaa Xaa Xaa Xaa
       130                 135                 140
   Xaa Xaa Xaa Xaa Xaa Xaa Xaa Xaa Xaa Xaa Tyr Xaa Trp Xaa Xaa Ser
   145                 150                 155                 160
   Xaa Phe Tyr Xaa Val Xaa Xaa Xaa Arg Val Xaa Xaa Xaa Leu Xaa Glu
                   165                 170                 175
   Xaa Gly Xaa Xaa Xaa Arg Xaa Xaa Xaa Glu Leu Trp Xaa Lys Ala Xaa
               180                 185                 190
   Xaa Leu Leu Xaa Gly Phe Trp Xaa Ser Leu Tyr Xaa Met Xaa Xaa Xaa
           195                 200                 205
   Xaa Xaa Xaa Asp Xaa Xaa Xaa Xaa Ala Xaa Xaa Xaa Ala Xaa Xaa Xaa
       210                 215                 220
   Gly Xaa Phe Ala Ala Phe Val Gly Thr Cys Ile Gln His Asp Gly Xaa
   225                 230                 235                 240
   His Gly Ala Phe Xaa Xaa Xaa Xaa Xaa Xaa Asn Lys Xaa Ala Gly Trp
                   245                 250                 255
   Thr Leu Asp Met Ile Gly Ala Ser Xaa Xaa Thr Trp Glu Xaa Gln His
               260                 265                 270
   Xaa Leu Gly His His Pro Tyr Thr Asn Leu Xaa Xaa Xaa Xaa Xaa Xaa
           275                 280                 285
   Xaa Xaa Lys Xaa Xaa Gly Xaa Xaa Xaa Asp Xaa Xaa Xaa Xaa Asp Gln
       290                 295                 300
   Glu Ser Asp Pro Asp Val Phe Ser Xaa Tyr Pro Xaa Xaa Arg Xaa His
   305                 310                 315                 320
   Pro Xaa His Xaa Xaa Xaa Xaa Tyr His Xaa Phe Gln His Xaa Tyr Xaa
                   325                 330                 335
   Pro Xaa Xaa Phe Xaa Xaa Met Thr Ile Xaa Lys Val Xaa Xaa Gln Asp
               340                 345                 350
   Xaa Xaa Val Xaa Xaa Xaa Xaa Arg Leu Xaa Xaa Ile Asp Ala Xaa Xaa
           355                 360                 365
   Arg Tyr Xaa Ser Xaa Xaa Xaa Val Xaa Arg Phe Trp Xaa Met Lys Xaa
       370                 375                 380
   Xaa Thr Xaa Leu Tyr Xaa Xaa Xaa Leu Pro Xaa Tyr Xaa Xaa Gly Xaa
   385                 390                 395                 400
   Xaa Xaa Gly Leu Xaa Leu Phe Xaa Xaa Ala His Xaa Xaa Cys Gly Glu
                   405                 410                 415
   Xaa Leu Ala Thr Met Phe Ile Val Asn His Xaa Ile Glu Gly Val Ser
               420                 425                 430
   Tyr Xaa Xaa Lys Asp Xaa Val Xaa Gly Xaa Xaa Xaa Xaa Xaa Xaa Xaa
           435                 440                 445
   Xaa Xaa Xaa Xaa Pro Xaa Thr Xaa Xaa Gly Xaa Thr Pro Met Xaa Xaa
       450                 455                 460
   Thr Xaa Lys Xaa Xaa Xaa Xaa Xaa Xaa Ser Xaa Xaa Xaa Xaa Xaa Xaa
   465                 470                 475                 480
   Xaa Xaa Xaa Xaa Xaa Xaa Xaa Xaa Xaa Pro Xaa Xaa Asp Trp Ala Xaa
                   485                 490                 495
   Val Gln Cys Gln Thr Ser Val Asn Trp Xaa Xaa Gly Ser Trp Phe Trp
               500                 505                 510
   Asn His Phe Ser Gly Gly Leu Asn His Gln Ile Glu His His Xaa Phe
           515                 520                 525
   Pro Xaa Xaa Xaa His Xaa Xaa Xaa Xaa Xaa Ile Xaa Xaa Xaa Val Xaa
       530                 535                 540
   Xaa Thr Cys Xaa Glu Tyr Gly Val Pro Tyr Gln Xaa Glu Xaa Xaa Leu
   545                 550                 555                 560
   Xaa Xaa Ala Tyr Xaa Lys Met Xaa Xaa His Leu Arg Xaa Leu Gly
                   565                 570                 575
<210> 96
   <211> 58
   <212> PRT
   <213> Artificial sequence
<220>
   <221> variant
   <222> (2)..(4)
   <223> xaa in position 2 to 4 is any amino acid
<220>
   <221> Variant
   <222> (10)..(10)
   <223> xaa in position 10 is Ala or Val
<220>
   <221> Variant
   <222> (13)..(13)
   <223> xaa in position 13 is Ala or Ser
<220>
   <221> Variant
   <222> (14)..(14)
   <223> xaa in position 14 is Pro or Val
<220>
   <221> Variant
   <222> (27)..(27)
   <223> xaa in position 27 is Asn or Ser
<220>
   <221> Variant
   <222> (34)..(34)
   <223> xaa in position 34 is any amino acid
<220>
   <221> Variant
   <222> (37)..(37)
   <223> xaa in position 37 is Gly, Gln or Ser
<220>
   <221> Variant
   <222> (38)..(39)
   <223> xaa in position 38 to 39 is any amino acid
<220>
   <221> Variant
   <222> (41)..(41)
   <223> xaa in position 41 is Glu or Thr
<220>
   <221> Variant
   <222> (42)..(42)
   <223> xaa in position 42 is Asn, Thr or Val
<220>
   <221> Variant
   <222> (43)..(44)
   <223> xaa in position 43 to 44 is any amino acid
<220>
   <221> variant
   <222> (45)..(45)
   <223> xaa in position 45 is His or Tyr
<220>
   <221> Variant
   <222> (47)..(47)
   <223> xaa in position 47 is Gln or Ser
<220>
   <221> Variant
   <222> (48)..(48)
   <223> xaa in position 48 is Asp or Gly
<220>
   <221> Variant
   <222> (49)..(49)
   <223> xaa in position 49 is Ile or Val
<220>
   <221> Variant
   <222> (50)..(50)
   <223> xaa in position 50 is Phe or Val
<220>
   <221> Variant
   <222> (51)..(51)
   <223> xaa in position 51 is any amino acid
<220>
   <221> Variant
   <222> (52)..(52)
   <223> xaa in position 52 is Glu or Ser
<220>
   <221> Variant
   <222> (55)..(55)
   <223> xaa in position 55 is Ala or Glu
<400> 96
<210> 97
   <211> 61
   <212> PRT
   <213> Artificial sequence
<220>
   <221> Variant
   <222> (2)..(2)
   <223> xaa in position 2 is any amino acid
<220>
   <221> Variant
   <222> (7)..(7)
   <223> xaa in position 7 is Ile or Val
<220>
   <221> Variant
   <222> (16)..(16)
   <223> xaa in position 16 is Asn or Ser
<220>
   <221> Variant
   <222> (21)..(21)
   <223> xaa in position 21 is Ala or Ser
<220>
   <221> Variant
   <222> (22)..(22)
   <223> xaa in position 22 is any amino acid
<220>
   <221> Variant
   <222> (23)..(23)
   <223> xaa in position 23 is Asn or Ser
<220>
   <221> Variant
   <222> (24)..(24)
   <223> xaa in position 24 is Lys or Arg
<220>
   <221> Variant
   <222> (25)..(25)
   <223> xaa in position 25 is any amino acid
<220>
   <221> Variant
   <222> (26)..(26)
   <223> xaa in position 26 is Leu, Met or Val
<220>
   <221> Variant
   <222> (29)..(29)
   <223> xaa in position 29 is any or no amino acid
<220>
   <221> Variant
   <222> (31)..(31)
   <223> xaa in position 31 is any or no amino acid
<220>
   <221> Variant
   <222> (42)..(42)
   <223> xaa in position 42 is Ala or Gly
<220>
   <221> Variant
   <222> (43)..(43)
   <223> xaa in position 43 is Phe or Met
<220>
   <221> Variant
   <222> (47)..(47)
   <223> xaa in position 47 is Phe, Leu or Met
<220>
   <221> Variant
   <222> (50)..(50)
   <223> xaa in position 50 is any amino acid
<220>
   <221> Variant
   <222> (59)..(59)
   <223> xaa in position 59 is Leu or Val
<220>
   <221> Variant
   <222> (60)..(60)
   <223> xaa in position 60 is Ile or Leu
<220>
   <221> Variant
   <222> (61)..(61)
   <223> xaa in position 61 is Asp or Glu
<400> 97
   Gly xaa Phe Ala Ala Phe xaa Gly Thr Cys Ile Gln His Asp Gly xaa
    1               5                   10                  15
   His Gly Ala Phe xaa xaa xaa xaa xaa xaa Asn Lys xaa Ala xaa Gly
               20                  25                  30
   Trp Thr Leu Asp Met Ile Gly Ala Ser xaa xaa Thr Trp Glu xaa Gln
           35                  40                  45
   His xaa Leu Gly His His Pro Tyr Thr Asn xaa xaa xaa
       50                  55                  60
<210> 98
   <211> 40
   <212> PRT
   <213> Artificial sequence
<220>
   <221> Variant
   <222> (3)..(3)
   <223> xaa in position 3 is Lys or Arg
<220>
   <221> Variant
   <222> (4)..(4)
   <223> xaa in position 4 is Phe or Tyr
<220>
   <221> Variant
   <222> (7)..(7)
   <223> xaa in position 7 is Ile or Leu
<220>
   <221> Variant
   <222> (9)..(9)
   <223> xaa in position 9 is Ala or Gly
<220>
   <221> Variant
   <222> (11)..(12)
   <223> xaa in position 11 to 12 is any or no amino acid
<220>
   <221> Variant
   <222> (14)..(15)
   <223> xaa in position 14 to 15 is any amino acid
<220>
   <221> Variant
   <222> (16)..(17)
   <223> xaa in position 16 to 17 is any or no amino acid
<220>
   <221> Variant
   <222> (20)..(20)
   <223> xaa in position 20 is Ile or Leu
<220>
   <221> Variant
   <222> (21)..(21)
   <223> xaa in position 21 is Ala or Asn
<220>
   <221> Variant
   <222> (24)..(24)
   <223> xaa in position 24 is Phe, Ile or Val
<220>
   <221> Variant
   <222> (25)..(25)
   <223> xaa in position 25 is Gln, Ser or Thr
<220>
   <221> Variant
   <222> (28)..(28)
   <223> xaa in position 28 is Phe or Val
<220>
   <221> Variant
   <222> (29)..(29)
   <223> xaa in position 29 is Glu or Gly
<220>
   <221> Variant
   <222> (31)..(31)
   <223> xaa in position 31 is Ala or Val
<220>
   <221> Variant
   <222> (32)..(32)
   <223> xaa in position 32 is any amino acid
<220>
   <221> Variant
   <222> (33)..(33)
   <223> xaa in position 33 is Arg or Ser

<220>
   <221> Variant
   <222> (34)..(34)
   <223> xaa in position 34 is any amino acid
<220>
   <221> Variant
   <222> (37)..(37)
   <223> xaa in position 37 is Phe or Tyr
<220>
   <221> Variant
   <222> (38)..(38)
   <223> xaa in position 38 is any amino acid
<400> 98
<210> 99
   <211> 59
   <212> PRT
   <213> Artificial sequence
<220>
   <221> variant
   <222> (3)..(3)
   <223> xaa in position 3 is Ala or Ile
<220>
   <221> Variant
   <222> (6)..(6)
   <223> xaa in position 6 is Ala, Leu or Val
<220>
   <221> Variant
   <222> (7)..(7)
   <223> xaa in position 7 is Ile or Leu
<220>
   <221> variant
   <222> (9)..(9)
   <223> xaa in position 9 is any amino acid
<220>
   <221> Variant
   <222> (10)..(10)
   <223> xaa in position 10 is Gly or Leu
<220>
   <221> Variant
   <222> (12)..(12)
   <223> xaa in position 12 is any amino acid
<220>
   <221> Variant
   <222> (13)..(13)
   <223> xaa in position 13 is Met or Val
<220>
   <221> Variant
   <222> (14)..(14)
   <223> xaa in position 14 is Ala or Gly
<220>
   <221> Variant
   <222> (17)..(17)
   <223> xaa in position 17 is any amino acid
<220>
   <221> Variant
   <222> (19)..(19)
   <223> xaa in position 19 is Phe or Met
<220>
   <221> Variant
   <222> (20)..(20)
   <223> xaa in position 20 is any amino acid
<220>
   <221> variant
   <222> (22)..(22)
   <223> xaa in position 22 is Pro or Val
<220>
   <221> Variant
   <222> (23)..(24)
   <223> xaa in position 23 to 24 is any amino acid
<220>
   <221> Variant
   <222> (26)..(26)
   <223> xaa in position 26 is Leu or Val
<220>
   <221> Variant
   <222> (27)..(27)
   <223> xaa in position 27 is any amino acid
<220>
   <221> Variant
   <222> (30)..(30)
   <223> xaa in position 30 is any amino acid
<220>
   <221> Variant
   <222> (31)..(31)
   <223> xaa in position 31 is Ile or Met
<220>
   <221> Variant
   <222> (32)..(32)
   <223> xaa in position 32 is Ala or Gly
<220>
   <221> Variant
   <222> (34)..(34)
   <223> xaa in position 34 is Phe or Leu
<220>
   <221> Variant
   <222> (35)..(35)
   <223> xaa in position 35 is Ala or Thr
<220>
   <221> Variant
   <222> (39)..(39)
   <223> xaa in position 39 is Leu or Val
<220>
   <221> Variant
   <222> (49)..(49)
   <223> xaa in position 49 is Ile or Val
<220>
   <221> Variant
   <222> (56)..(56)
   <223> xaa in position 56 is Ala or Gly
<220>
   <221> Variant
   <222> (57)..(57)
   <223> xaa in position 57 is Ser or Thr
<400> 99
<210> 100
   <211> 45
   <212> PRT
   <213> Artificial sequence
<220>
   <221> Variant
   <222> (3)..(3)
   <223> xaa in position 3 is Ile or Val
<220>
   <221> Variant
   <222> (5)..(5)
   <223> xaa in position 5 is Asp or Lys
<220>
   <221> Variant
   <222> (7)..(9)
   <223> xaa in position 7 to 9 is any amino acid
<220>
   <221> Variant
   <222> (14)..(14)
   <223> xaa in position 14 is Asp or Gly
<220>
   <221> Variant
   <222> (15)..(15)
   <223> xaa in position 15 is Ile or Val
<220>
   <221> Variant
   <222> (17)..(17)
   <223> xaa in position 17 is any amino acid
<220>
   <221> variant
   <222> (19)..(19)
   <223> xaa in position 19 is Ala or Gly
<220>
   <221> Variant
   <222> (20)..(20)
   <223> xaa in position 20 is Ala or Leu
<220>
   <221> Variant
   <222> (22)..(22)
   <223> xaa in position 22 is Cys or Arg
<220>
   <221> variant
   <222> (23)..(23)
   <223> xaa in position 23 is Asp or Glu
<220>
   <221> Variant
   <222> (24)..(24)
   <223> xaa in position 24 is Ala or Cys
<220>
   <221> Variant
   <222> (26)..(26)
   <223> xaa in position 26 is Ile or Val
<220>
   <221> Variant
   <222> (28)..(28)
   <223> xaa in position 28 is any amino acid
<220>
   <221> Variant
   <222> (30)..(30)
   <223> xaa in position 30 is Ser or Thr
<220>
   <221> variant
   <222> (33)..(33)
   <223> xaa in position 33 is Ile, Pro or Val
<220>
   <221> Variant
   <222> (34)..(34)
   <223> xaa in position 34 is any amino acid
<220>
   <221> Variant
   <222> (36)..(36)
   <223> xaa in position 36 is any amino acid
<220>
   <221> Variant
   <222> (37)..(37)
   <223> xaa in position 37 is Pro or ser
<220>
   <221> Variant
   <222> (39)..(39)
   <223> xaa in position 39 is any amino acid
<220>
   <221> Variant
   <222> (41)..(41)
   <223> xaa in position 41 is Leu or Met
<220>
   <221> Variant
   <222> (42)..(42)
   <223> xaa in position 42 is Glu or Arg
<220>
   <221> Variant
   <222> (45)..(45)
   <223> xaa in position 45 is Lys or Arg
<400> 100
<210> 101
   <211> 24
   <212> PRT
   <213> Artificial sequence
<220>
   <221> Variant
   <222> (2)..(6)
   <223> xaa in position 2 to 6 is any or no amino acid
<220>
   <221> Variant
   <222> (16)..(16)
   <223> xaa in position 16 is Ser or Thr
<220>
   <221> Variant
   <222> (17)..(17)
   <223> xaa in position 17 is Phe or Tyr
<220>
   <221> variant
   <222> (19)..(20)
   <223> xaa in position 19 to 20 is Leu or Met
<220>
   <221> Variant
   <222> (22)..(22)
   <223> xaa in position 22 is Leu or Met
<400> 101
   Asp Xaa Xaa Xaa Xaa Xaa Gln Glu Ser Asp Pro Asp Val Phe Ser Xaa
   1               5                   10                  15
   Xaa Pro Xaa Xaa Arg Xaa His Pro
               20
<210> 102
   <211> 27
   <212> PRT
   <213> Artificial sequence
<220>
   <221> Variant
   <222> (2)..(2)
   <223> xaa in position 2 is Asp or Glu
<220>
   <221> Variant
   <222> (5)..(5)
   <223> xaa in position 5 is any amino acid
<220>
   <221> Variant
   <222> (7)..(7)
   <223> xaa in position 7 is Leu or Met
<220>
   <221> Variant
   <222> (8)..(8)
   <223> xaa in position 8 is Lys or Arg
<220>
   <221> Variant
   <222> (9)..(9)
   <223> xaa in position 9 is Glu or Arg
<220>
   <221> Variant
   <222> (12)..(12)
   <223> xaa in position 12 is Ala or Val
<220>
   <221> Variant
   <222> (13)..(13)
   <223> xaa in position 13 is any amino acid
<220>
   <221> Variant
   <222> (14)..(14)
   <223> xaa in position 14 is Cys or Arg
<220>
   <221> variant
   <222> (16)..(16)
   <223> xaa in position 16 is any amino acid
<220>
   <221> Variant
   <222> (18)..(18)
   <223> xaa in position 18 is any amino acid
<220>
   <221> Variant
   <222> (20)..(21)
   <223> xaa in position 20 to 21 is any amino acid
<220>
   <221> Variant
   <222> (22)..(22)
   <223> xaa in position 22 is Gln or Arg
<220>
   <221> variant
   <222> (24)..(24)
   <223> xaa in position 24 is Ala or Gly
<220>
   <221> Variant
   <222> (25)..(26)
   <223> xaa in position 25 to 26 is any amino acid
<400> 102
   Ser xaa Phe Tyr xaa Val xaa xaa xaa Arg Val xaa xaa xaa Leu xaa
    1               5                   10                  15
   Glu xaa Gly xaa xaa xaa Arg xaa xaa xaa Glu
                20                  25
<210> 103
   <211> 28
   <212> PRT
   <213> Artificial sequence
<220>
   <221> Variant
   <222> (2)..(3)
   <223> xaa in position 2 to 3 is any amino acid
<220>
   <221> Variant
   <222> (4)..(4)
   <223> xaa in position 4 is any or no amino acid
<220>
   <221> Variant
   <222> (6)..(10)
   <223> xaa in position 6 to 10 is any amino acid
<220>
   <221> Variant
   <222> (11)..(12)
   <223> xaa in position 11 to 12 is any or no amino acid
<220>
   <221> Variant
   <222> (15)..(15)
   <223> xaa in position 15 is any amino acid
<220>
   <221> Variant
   <222> (17)..(18)
   <223> xaa in position 17 to 18 is any amino acid
<220>
   <221> Variant
   <222> (20)..(20)
   <223> xaa in position 20 is Asp or Thr
<220>
   <221> Variant
   <222> (22)..(22)
   <223> xaa in position 22 is Ala or Gly
<220>
   <221> Variant
   <222> (24)..(24)
   <223> xaa in position 24 is any amino acid
<220>
   <221> Variant
   <222> (25)..(25)
   <223> xaa in position 25 is Ala or Thr
<220>
   <221> Variant
   <222> (26)..(26)
   <223> xaa in position 26 is any amino acid
<220>
   <221> Variant
   <222> (28)..(28)
   <223> xaa in position 28 is Glu or Gly
<400> 103
<210> 104
   <211> 28
   <212> PRT
   <213> Artificial sequence
<220>
   <221> Variant
   <222> (5)..(5)
   <223> xaa in position 5 is any amino acid
<220>
   <221> Variant
   <222> (7)..(7)
   <223> xaa in position 7 is any or no amino acid
<220>
   <221> Variant
   <222> (9)..(9)
   <223> xaa in position 9 is any or no amino acid
<220>
   <221> Variant
   <222> (11)..(11)
   <223> xaa in position 11 is Phe or Trp
<220>
   <221> Variant
   <222> (12)..(12)
   <223> xaa in position 12 is Ser, Thr or Val
<220>
   <221> variant
   <222> (15)..(15)
   <223> xaa in position 15 is any amino acid
<220>
   <221> Variant
   <222> (18)..(18)
   <223> xaa in position 18 is Ile or Leu
<220>
   <221> Variant
   <222> (19)..(19)
   <223> xaa in position 19 is Glu or Ser
<220>
   <221> Variant
   <222> (22)..(22)
   <223> xaa in position 22 is Cys or Arg
<220>
   <221> Variant
   <222> (23)..(23)
   <223> xaa in position 23 is any amino acid
<220>
   <221> Variant
   <222> (26)..(26)
   <223> xaa in position 26 is any amino acid
<220>
   <221> variant
   <222> (27)..(27)
   <223> xaa in position 27 is Ala or Glu
<220>
   <221> Variant
   <222> (28)..(28)
   <223> xaa in position 28 is Asp, Glu or Lys
<400> 104
   Val Pro Tyr Gln xaa Glu xaa Ser xaa Leu xaa xaa Ala Tyr xaa Lys
   1               5                   10                  15
   Met xaa xaa His Leu xaa xaa Leu Gly xaa xaa xaa
               20                  25
<210> 105
   <211> 15
   <212> PRT
   <213> Artificial sequence
<220>
   <221> Variant
   <222> (4)..(4)
   <223> xaa in position 4 is any amino acid
<220>
   <221> Variant
   <222> (5)..(5)
   <223> xaa in position 5 is Cys or Ser
<220>
   <221> Variant
   <222> (7)..(8)
   <223> xaa in position 7 to 8 is any amino acid
<220>
   <221> Variant
   <222> (10)..(12)
   <223> xaa in position 10 to 12 is any amino acid
<220>
   <221> Variant
   <222> (13)..(13)
   <223> xaa in position 13 is any or no amino acid
<220>
   <221> Variant
   <222> (15)..(15)
   <223> xaa in position 15 is Ile or Pro
<400> 105
   Gly Phe Trp xaa xaa Leu xaa xaa Met xaa xaa xaa xaa Asp xaa
   1               5                   10                  15
<210> 106
   <211> 29
   <212> PRT
   <213> Artificial sequence
<220>
   <221> Variant
   <222> (2)..(7)
   <223> xaa in position 2 to 7 is any amino acid
<220>
   <221> Variant
   <222> (8)..(12)
   <223> xaa in position 8 to 12 is any or no amino acid
<220>
   <221> Variant
   <222> (14)..(14)
   <223> xaa in position 14 is Asp or Val
<220>
   <221> Variant
   <222> (18)..(18)
   <223> xaa in position 18 is Glu, Asn or Gln
<220>
   <221> Variant
   <222> (19)..(19)
   <223> xaa in position 19 is any amino acid
<220>
   <221> Variant
   <222> (21)..(21)
   <223> xaa in position 21 is Gln or Arg
<220>
   <221> Variant
   <222> (22)..(22)
   <223> xaa in position 22 is Glu or Lys
<220>
   <221> Variant
   <222> (23)..(23)
   <223> xaa in position 23 is Ala or Glu
<220>
   <221> Variant
   <222> (24)..(24)
   <223> xaa in position 24 is Ala, Leu or Val
<220>
   <221> variant
   <222> (25)..(26)
   <223> xaa in position 25 to 26 is any amino acid
<220>
   <221> Variant
   <222> (27)..(27)
   <223> xaa in position 27 is Ala, Glu or Ser
<220>
   <221> Variant
   <222> (28)..(28)
   <223> xaa in position 28 is Ala, Glu or Asn
<400> 106
   Val xaa xaa xaa xaa xaa xaa xaa xaa xaa xaa xaa Gly xaa Thr Pro
     1               5                  10                  15
   Met xaa xaa Thr xaa xaa xaa xaa xaa xaa xaa xaa Ser
               20                  25
<210> 107
   <211> 11
   <212> PRT
   <213> Artificial sequence
<220>
   <221> Variant
   <222> (2)..(2)
   <223> xaa in position 2 is Glu or Asn
<220>
   <221> Variant
   <222> (3)..(3)
   <223> xaa in position 3 is Cys or Val
<220>
   <221> variant
   <222> (6)..(6)
   <223> xaa in position 6 is Ala or Gly
<220>
   <221> Variant
   <222> (8)..(8)
   <223> xaa in position 8 is Pro or Val
<220>
   <221> Variant
   <222> (9)..(10)
   <223> xaa in position 9 to 10 is any amino acid
<400> 107
   Ala xaa xaa Arg Tyr xaa Ser xaa xaa xaa Val
   1               5                   10
<210> 108
   <211> 8
   <212> PRT
   <213> Artificial sequence
<220>
   <221> Variant
   <222> (2)..(2)
   <223> xaa in position 2 is Ala or Ile
<220>
   <221> Variant
   <222> (5)..(5)
   <223> xaa in position 5 is Phe, Ile or Leu
<220>
   <221> Variant
   <222> (6)..(6)
   <223> xaa in position 6 is Phe or Leu
<400> 108
   Trp Xaa Lys Ala Xaa Xaa Leu Leu
    1               5
<210> 109
   <211> 903
   <212> DNA
   <213> Ostreococcus tauri
<220>
   <221> CDS
   <222> (1)..(903)
<400> 109
   atg agc gcc tcc ggt gcg ctg ctg ccc gcg atc gcg ttc gcc gcg tac      48
   Met Ser Ala Ser Gly Ala Leu Leu Pro Ala Ile Ala Phe Ala Ala Tyr
   1               5                   10                  15
   gcg tac gcg acg tac gcc tac gcc ttt gag tgg tcg cac gcg aat ggc      96
   Ala Tyr Ala Thr Tyr Ala Tyr Ala Phe Glu Trp Ser His Ala Asn Gly
               20                  25                  30
   atc gac aac gtc gac gcg cgc gag tgg atc ggt gcg ctg tcg ttg agg      144
   Ile Asp Asn Val Asp Ala Arg Glu Trp Ile Gly Ala Leu Ser Leu Arg
           35                  40                  45
   ctc ccg gcg atc gcg acg acg atg tac ctg ttg ttc tgc ctg gtc gga      192
   Leu Pro Ala Ile Ala Thr Thr Met Tyr Leu Leu Phe Cys Leu Val Gly
       50                  55                  60
   ccg agg ttg atg gcg aag cgc gag gcg ttc gac ccg aag ggg ttc atg      240
   Pro Arg Leu Met Ala Lys Arg Glu Ala Phe Asp Pro Lys Gly Phe Met
   65                  70                  75                  80
   ctg gcg tac aat gcg tat cag acg gcg ttc aac gtc gtc gtg ctc ggg      288
   Leu Ala Tyr Asn Ala Tyr Gln Thr Ala Phe Asn Val Val Val Leu Gly
                   85                  90                  95
   atg ttc gcg cga gag atc tcg ggg ctg ggg cag ccc gtg tgg ggg tca      336
   Met Phe Ala Arg Glu Ile Ser Gly Leu Gly Gln Pro Val Trp Gly Ser
               100                 105                 110
   acc atg ccg tgg agc gat aga aaa tcg ttt aag atc ctc ctc ggg gtg      384
   Thr Met Pro Trp Ser Asp Arg Lys Ser Phe Lys Ile Leu Leu Gly Val
           115                 120                 125
   tgg ttg cac tac aac aac aaa tat ttg gag cta ttg gac act gtg ttc      432
   Trp Leu His Tyr Asn Asn Lys Tyr Leu Glu Leu Leu Asp Thr Val Phe
       130                 135                 140
   atg gtt gcg cgc aag aag acg aag cag ttg agc ttc ttg cac gtt tat      480
   Met Val Ala Arg Lys Lys Thr Lys Gln Leu Ser Phe Leu His Val Tyr
   145                 150                 155                 160
   cat cac gcc ctg ttg atc tgg gcg tgg tgg ttg gtg tgt cac ttg atg      528
   His His Ala Leu Leu Ile Trp Ala Trp Trp Leu Val Cys His Leu Met
                   165                 170                 175
   gcc acg aac gat tgt atc gat gcc tac ttc ggc gcg gcg tgc aac tcg      576
   Ala Thr Asn Asp Cys Ile Asp Ala Tyr Phe Gly Ala Ala Cys Asn Ser
               180                 185                 190
   ttc att cac atc gtg atg tac tcg tat tat ctc atg tcg gcg ctc ggc      624
   Phe Ile His Ile Val Met Tyr Ser Tyr Tyr Leu Met Ser Ala Leu Gly
           195                 200                 205
   att cga tgc ccg tgg aag cga tac atc acc cag gct caa atg ctc caa      672
   Ile Arg Cys Pro Trp Lys Arg Tyr Ile Thr Gln Ala Gln Met Leu Gln
       210                 215                 220
   ttc gtc att gtc ttc gcg cac gcc gtg ttc gtg ctg cgt cag aag cac      720
   Phe Val Ile Val Phe Ala His Ala Val Phe Val Leu Arg Gln Lys His
   225                 230                 235                 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg      816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg      864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                  903
   Pro Ser Val Arg Arg Thr Arg Ser Arg Lys Ile Asp
       290                 295                 300
<210> 110
   <211> 300
   <212> PRT
   <213> Ostreococcus tauri
<400> 110
   Met Ser Ala Ser Gly Ala Leu Leu Pro Ala Ile Ala Phe Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Lys Tyr Leu Glu Leu Leu Asp Thr Val Phe
       130                 135                 140
   Met Val Ala Arg Lys Lys Thr Lys Gln Leu Ser Phe Leu His Val Tyr
   145                 150                 155                 160
   His His Ala Leu Leu Ile Trp Ala Trp Trp Leu Val Cys His Leu Met
                   165                 170                 175
   Ala Thr Asn Asp Cys Ile Asp Ala Tyr Phe Gly Ala Ala Cys Asn Ser
               180                 185                 190
   Phe Ile His Ile Val Met Tyr Ser Tyr Tyr Leu Met Ser Ala Leu Gly
           195                 200                 205
   Ile Arg Cys Pro Trp Lys Arg Tyr Ile Thr Gln Ala Gln Met Leu Gln
       210                 215                 220
   Phe Val Ile Val Phe Ala His Ala Val Phe Val Leu Arg Gln Lys His
   225                 230                 235                 240
   Cys Pro Val Thr Leu Pro Trp Ala Gln Met Phe Val Met Thr Asn Met
                   245                 250                 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 111
   <211> 834
   <212> DNA
   <213> Pavlova sp
<220>
   <221> CDS
   <222> (1)..(834)
<400> 111
   atg atg ttg gcc gca ggc tat ctt cta gtg ctc tcg gcc gct cgc cag      48
   Met Met Leu Ala Ala Gly Tyr Leu Leu Val Leu Ser Ala Ala Arg Gln
   1               5                   10                  15
   agc ttc cag cag gac att gac aac ccc aac ggg gcc tac tcg acc tcg      96
   Ser Phe Gln Gln Asp Ile Asp Asn Pro Asn Gly Ala Tyr Ser Thr Ser
               20                  25                  30
   tgg act ggc ctg ccc att gtg atg tct gtg gtc tat ctc agc ggt gtg      144
   Trp Thr Gly Leu Pro Ile Val Met Ser Val Val Tyr Leu Ser Gly Val
                   35                      40                      45
   ttt ggg ctc aca aag tac ttc gag aac cgg aag ccc atg acg ggg ctg      192
   Phe Gly Leu Thr Lys Tyr Phe Glu Asn Arg Lys Pro Met Thr Gly Leu
       50                      55                      60
   aag gac tac atg ttc act tac aat ctc tac cag gtg atc atc aac gtg      240
   Lys Asp Tyr Met Phe Thr Tyr Asn Leu Tyr Gln Val Ile Ile Asn Val
   65                      70                      75                  80
   tgg tgc gtg gtg gcc ttt ctc ctg gag gtg cgg cgt gcg ggc atg tca      288
   Trp Cys Val Val Ala Phe Leu Leu Glu Val Arg Arg Ala Gly Met Ser
                       85                      90                  95
   ctc atc ggc aat aag gtg gac ctt ggg ccc aac tcc ttc agg ctc ggc      336
   Leu Ile Gly Asn Lys Val Asp Leu Gly Pro Asn Ser Phe Arg Leu Gly
                   100                     105                     110
   ttc gtc acg tgg gtg cac tac aac aac aag tac gtg gag ctc ctc gac      384
   Phe Val Thr Trp Val His Tyr Asn Asn Lys Tyr Val Glu Leu Leu Asp
               115                     120                     125
   acc cta tgg atg gtg ctg cgc aag aag acg cag cag gtc tcc ttc ctc      432
   Thr Leu Trp Met Val Leu Arg Lys Lys Thr Gln Gln Val Ser Phe Leu
       130                     135                     140
   cac gtc tat cat cac gtg ctt ctg atg tgg gcc tgg ttc gtt gtc gtc      480
   His Val Tyr His His Val Leu Leu Met Trp Ala Trp Phe Val Val Val
   145                     150                     155                 160
   aag ctc ggc aat ggt ggt gac gca tat ttt ggc ggt ctc atg aac tcg      528
   Lys Leu Gly Asn Gly Gly Asp Ala Tyr Phe Gly Gly Leu Met Asn Ser
                   165                     170                     175
   atc atc cac gtg atg atg tat tcc tac tac acc atg gcg ctc ctg ggc      576
   Ile Ile His Val Met Met Tyr Ser Tyr Tyr Thr Met Ala Leu Leu Gly
               180                     185                     190
   tgg tca tgc ccc tgg aag cgc tac ctc acg cag gca cag ctc gtg cag      624
   Trp Ser Cys Pro Trp Lys Arg Tyr Leu Thr Gln Ala Gln Leu Val Gln
           195                     200                     205
   ttt tgc atc tgc ctc gcc cac tcc aca tgg gcg gca gta acg ggt gcc      672
   Phe Cys Ile Cys Leu Ala His Ser Thr Trp Ala Ala Val Thr Gly Ala
       210                     215                     220
   tac ccg tgg cga att tgc ttg gtg gag gtg tgg gtg atg gtg tcc atg      720
   Tyr Pro Trp Arg Ile Cys Leu Val Glu Val Trp Val Met Val Ser Met
   225                     230                     235                 240
   ctg gtg ctc ttc aca cgc ttc tac cgc cag gcc tat gcc aag gag gcg      768
   Leu Val Leu Phe Thr Arg Phe Tyr Arg Gln Ala Tyr Ala Lys Glu Ala
                   245                     250                     255
   aag gcc aag gag gcg aaa aag ctc gca cag gag gca tca cag gcc aag      816
   Lys Ala Lys Glu Ala Lys Lys Leu Ala Gln Glu Ala Ser Gln Ala Lys
               260                     265                     270
   gcg gtc aag gcg gag taa                                              834
   Ala Val Lys Ala Glu
           275
<210> 112
   <211> 277
   <212> PRT
   <213> Pavlova sp
<400> 112
   Met Met Leu Ala Ala Gly Tyr Leu Leu Val Leu Ser Ala Ala Arg Gln
   1               5                   10                  15
   Ser Phe Gln Gln Asp Ile Asp Asn Pro Asn Gly Ala Tyr Ser Thr Ser
               20                  25                  30
   Trp Thr Gly Leu Pro Ile Val Met Ser Val Val Tyr Leu Ser Gly Val
           35                  40                  45
   Phe Gly Leu Thr Lys Tyr Phe Glu Asn Arg Lys Pro Met Thr Gly Leu
       50                  55                  60
   Lys Asp Tyr Met Phe Thr Tyr Asn Leu Tyr Gln Val Ile Ile Asn Val
   65                  70                  75                  80
   Trp Cys Val Val Ala Phe Leu Leu Glu Val Arg Arg Ala Gly Met Ser
                   85                  90                  95
   Leu Ile Gly Asn Lys Val Asp Leu Gly Pro Asn Ser Phe Arg Leu Gly
               100                 105                 110
   Phe Val Thr Trp Val His Tyr Asn Asn Lys Tyr Val Glu Leu Leu Asp
           115                 120                 125
   Thr Leu Trp Met Val Leu Arg Lys Lys Thr Gln Gln Val Ser Phe Leu
       130                 135                 140
   His Val Tyr His His Val Leu Leu Met Trp Ala Trp Phe Val Val Val
   145                 150                 155                 160
   Lys Leu Gly Asn Gly Gly Asp Ala Tyr Phe Gly Gly Leu Met Asn Ser
                   165                 170                 175
   Ile Ile His Val Met Met Tyr Ser Tyr Tyr Thr Met Ala Leu Leu Gly
               180                 185                 190
   Trp Ser Cys Pro Trp Lys Arg Tyr Leu Thr Gln Ala Gln Leu Val Gln
           195                 200                 205
   Phe Cys Ile Cys Leu Ala His Ser Thr Trp Ala Ala Val Thr Gly Ala
       210                 215                 220
   Tyr Pro Trp Arg Ile Cys Leu Val Glu Val Trp Val Met Val Ser Met
   225                 230                 235                 240
   Leu Val Leu Phe Thr Arg Phe Tyr Arg Gln Ala Tyr Ala Lys Glu Ala
                   245                 250                 255
   Lys Ala Lys Glu Ala Lys Lys Leu Ala Gln Glu Ala Ser Gln Ala Lys
               260                 265                 270
   Ala Val Lys Ala Glu
           275
<210> 113
   <211> 1077
   <212> DNA
   <213> Thalassiosira pseudonana
<220>
   <221> CDS
   <222> (1)..(1077)
<400> 113
   atg tgc tca tca ccg ccg tca caa tcc aaa aca aca tcc ctc cta gca      48
   Met Cys Ser Ser Pro Pro Ser Gln Ser Lys Thr Thr Ser Leu Leu Ala
   1               5                   10                  15
   cgg tac acc acc gcc gcc ctc ctc ctc ctc acc ctc aca aca tgg tgc      96
   Arg Tyr Thr Thr Ala Ala Leu Leu Leu Leu Thr Leu Thr Thr Trp Cys
               20                  25                  30
   cac ttc gcc ttc cca gcc gcc acc gcc aca ccc ggc ctc acc gcc gaa      144
   His Phe Ala Phe Pro Ala Ala Thr Ala Thr Pro Gly Leu Thr Ala Glu
           35                  40                  45
   atg cac tcc tac aaa gtc cca ctc ggt ctc acc gta ttc tac ctg ctg      192
   Met His Ser Tyr Lys Val Pro Leu Gly Leu Thr Val Phe Tyr Leu Leu
       50                  55                  60
   agt cta ccg tca cta aag tac gtt acg gac aac tac ctt gcc aaa aag      240
   Ser Leu Pro Ser Leu Lys Tyr Val Thr Asp Asn Tyr Leu Ala Lys Lys
   65                  70                  75                  80
   tat gat atg aag tca ctc cta acg gaa tca atg gtg ttg tac aat gtg      288
   Tyr Asp Met Lys Ser Leu Leu Thr Glu Ser Met Val Leu Tyr Asn Val
                   85                  90                  95
   gcg caa gtg ctg ctc aat ggg tgg acg gtg tat gcg att gtg gat gcg      336
   Ala Gln Val Leu Leu Asn Gly Trp Thr Val Tyr Ala Ile Val Asp Ala
               100                 105                 110
   gtg atg aat aga gac cat ccg ttt att gga agt aga agt ttg gtt ggg      384
   Val Met Asn Arg Asp His Pro Phe Ile Gly Ser Arg Ser Leu Val Gly
           115                 120                 125
   gcg gcg ttg cat agt ggg agc tcg tat gcg gtg tgg gtt cat tat tgt      432
   Ala Ala Leu His Ser Gly Ser Ser Tyr Ala Val Trp Val His Tyr Cys
       130                 135                 140
   gat aag tat ttg gag ttc ttt gat acg tat ttt atg gtg ttg agg ggg      480
   Asp Lys Tyr Leu Glu Phe Phe Asp Thr Tyr Phe Met Val Leu Arg Gly
   145                 150                 155                 160
   aaa atg gac cag gtc tcc ttc ctc cac atc tac cac cac acg acc ata      528
   Lys Met Asp Gln Val Ser Phe Leu His Ile Tyr His His Thr Thr Ile
                   165                 170                 175
   gcg tgg gca tgg tgg atc gcc ctc cgc ttc tcc ccc ggt gga gac att      576
   Ala Trp Ala Trp Trp Ile Ala Leu Arg Phe Ser Pro Gly Gly Asp Ile
               180                 185                 190
   tac ttc ggg gca ctc ctc aac tcc atc atc cac gtc ctc atg tat tcc      624
   Tyr Phe Gly Ala Leu Leu Asn Ser Ile Ile His Val Leu Met Tyr Ser
           195                 200                 205
   tac tac gcc ctt gcc cta ctc aag gtc agt tgt cca tgg aaa cga tac      672
   Tyr Tyr Ala Leu Ala Leu Leu Lys Val Ser Cys Pro Trp Lys Arg Tyr
       210                 215                 220
   ctg act caa gct caa tta ttg caa ttc aca agt gtg gtg gtt tat acg      720
   Leu Thr Gln Ala Gln Leu Leu Gln Phe Thr Ser Val Val Val Tyr Thr
   225                 230                 235                 240
   ggg tgt acg ggt tat act cat tac tat cat acg aag cat gga gcg gat      768
   Gly Cys Thr Gly Tyr Thr His Tyr Tyr His Thr Lys His Gly Ala Asp
                   245                 250                 255
   gag aca cag cct agt tta gga acg tat tat ttc tgt tgt gga gtg cag      816
   Glu Thr Gln Pro Ser Leu Gly Thr Tyr Tyr Phe Cys Cys Gly Val Gln
               260                 265                 270
   gtg ttt gag atg gtt agt ttg ttt gta ctc ttt tcc atc ttt tat aaa      864
   Val Phe Glu Met Val Ser Leu Phe Val Leu Phe Ser Ile Phe Tyr Lys
           275                 280                 285
   cga tcc tat tcg aag aag aac aag tca gga gga aag gat agc aag aag      912
   Arg Ser Tyr Ser Lys Lys Asn Lys Ser Gly Gly Lys Asp Ser Lys Lys
       290                 295                 300
   aat gat gat ggg aat aat gag gat caa tgt cac aag gct atg aag gat      960
   Asn Asp Asp Gly Asn Asn Glu Asp Gln Cys His Lys Ala Met Lys Asp
   305                 310                 315                 320
   ata tcg gag ggt gcg aag gag gtt gtg ggg cat gca gcg aag gat gct      1008
   Ile Ser Glu Gly Ala Lys Glu Val Val Gly His Ala Ala Lys Asp Ala
                   325                 330                 335
   gga aag ttg gtg gct acg gcg agt aag gct gta aag agg aag gga act      1056
   Gly Lys Leu Val Ala Thr Ala Ser Lys Ala Val Lys Arg Lys Gly Thr
               340                 345                 350
   cgt gtt act ggt gcc atg tag                                          1077
   Arg Val Thr Gly Ala Met
           355
<210> 114
   <211> 358
   <212> PRT
   <213> Thalassiosira pseudonana
<400> 114
   Met Cys Ser Ser Pro Pro Ser Gln Ser Lys Thr Thr Ser Leu Leu Ala
   1               5                   10                  15
   Arg Tyr Thr Thr Ala Ala Leu Leu Leu Leu Thr Leu Thr Thr Trp Cys
               20                  25                  30
   His Phe Ala Phe Pro Ala Ala Thr Ala Thr Pro Gly Leu Thr Ala Glu
           35                  40                  45
   Met His Ser Tyr Lys Val Pro Leu Gly Leu Thr Val Phe Tyr Leu Leu
       50                  55                  60
   Ser Leu Pro Ser Leu Lys Tyr Val Thr Asp Asn Tyr Leu Ala Lys Lys
   65                  70                  75                  80
   Tyr Asp Met Lys Ser Leu Leu Thr Glu Ser Met Val Leu Tyr Asn Val
                   85                  90                  95
   Ala Gln Val Leu Leu Asn Gly Trp Thr Val Tyr Ala Ile Val Asp Ala
               100                 105                 110
   Val Met Asn Arg Asp His Pro Phe Ile Gly Ser Arg Ser Leu Val Gly
           115                 120                 125
   Ala Ala Leu His Ser Gly Ser Ser Tyr Ala Val Trp Val His Tyr Cys
       130                 135                 140
   Asp Lys Tyr Leu Glu Phe Phe Asp Thr Tyr Phe Met Val Leu Arg Gly
   145                 150                 155                 160
   Lys Met Asp Gln Val Ser Phe Leu His Ile Tyr His His Thr Thr Ile
                   165                 170                 175
   Ala Trp Ala Trp Trp Ile Ala Leu Arg Phe Ser Pro Gly Gly Asp Ile
               180                 185                 190
   Tyr Phe Gly Ala Leu Leu Asn Ser Ile Ile His Val Leu Met Tyr Ser
           195                 200                 205
   Tyr Tyr Ala Leu Ala Leu Leu Lys Val Ser Cys Pro Trp Lys Arg Tyr
       210                 215                 220
   Leu Thr Gln Ala Gln Leu Leu Gln Phe Thr Ser Val Val Val Tyr Thr
   225                 230                 235                 240
   Gly Cys Thr Gly Tyr Thr His Tyr Tyr His Thr Lys His Gly Ala Asp
                   245                 250                 255
   Glu Thr Gln Pro Ser Leu Gly Thr Tyr Tyr Phe Cys Cys Gly Val Gln
               260                 265                 270
   Val Phe Glu Met Val Ser Leu Phe Val Leu Phe Ser Ile Phe Tyr Lys
           275                 280                 285
   Arg Ser Tyr Ser Lys Lys Asn Lys Ser Gly Gly Lys Asp Ser Lys Lys
       290                 295                 300
   Asn Asp Asp Gly Asn Asn Glu Asp Gln Cys His Lys Ala Met Lys Asp
   305                 310                 315                 320
   Ile Ser Glu Gly Ala Lys Glu Val Val Gly His Ala Ala Lys Asp Ala
                   325                 330                 335
   Gly Lys Leu Val Ala Thr Ala Ser Lys Ala Val Lys Arg Lys Gly Thr
               340                 345                 350
   Arg Val Thr Gly Ala Met
           355
<210> 115
   <211> 903
   <212> DNA
   <213> Ostreococcus tauri
<220>
   <221> CDS
   <222> (1)..(903)
<400> 115
   atg agc gcc tcc ggt gcg ctg ctg ccc gcg atc gcg tcc gcc gcg tac      48
   Met Ser Ala Ser Gly Ala Leu Leu Pro Ala Ile Ala Ser Ala Ala Tyr
   1               5                   10                  15
   gcg tac gcg acg tac gcc tac gcc ttt gag tgg tcg cac gcg aat ggc      96
   Ala Tyr Ala Thr Tyr Ala Tyr Ala Phe Glu Trp Ser His Ala Asn Gly
               20                  25                  30
   atc gac aac gtc gac gcg cgc gag tgg atc ggt gcg ctg tcg ttg agg      144
   Ile Asp Asn Val Asp Ala Arg Glu Trp Ile Gly Ala Leu Ser Leu Arg
           35                  40                  45
   ctc ccg gcg atc gcg acg acg atg tac ctg ttg ttc tgc ctg gtc gga      192
   Leu Pro Ala Ile Ala Thr Thr Met Tyr Leu Leu Phe Cys Leu Val Gly
       50                  55                  60
   ccg agg ttg atg gcg aag cgc gag gcg ttc gac ccg aag ggg ttc atg      240
   Pro Arg Leu Met Ala Lys Arg Glu Ala Phe Asp Pro Lys Gly Phe Met
   65                  70                  75                  80
   ctg gcg tac aat gcg tat cag acg gcg ttc aac gtc gtc gtg ctc ggg      288
   Leu Ala Tyr Asn Ala Tyr Gln Thr Ala Phe Asn Val Val Val Leu Gly
                   85                  90                  95
   atg ttc gcg cga gag atc tcg ggg ctg ggg cag ccc gtg tgg ggg tca      336
   Met Phe Ala Arg Glu Ile Ser Gly Leu Gly Gln Pro Val Trp Gly Ser
               100                 105                 110
   acc atg ccg tgg agc gat aga aaa tcg ttt aag atc ctc ctc ggg gtg      384
   Thr Met Pro Trp Ser Asp Arg Lys Ser Phe Lys Ile Leu Leu Gly Val
           115                 120                 125
   tgg ttg cac tac aac aac aaa tat ttg gag cta ttg gac act gtg ttc      432
   Trp Leu His Tyr Asn Asn Lys Tyr Leu Glu Leu Leu Asp Thr Val Phe
       130                 135                 140
   atg gtt gcg cgc aag aag acg aag cag ttg agc ttc ttg cac gtt tat      480
   Met Val Ala Arg Lys Lys Thr Lys Gln Leu Ser Phe Leu His Val Tyr
   145                 150                 155                 160
   cat cac gcc ctg ttg atc tgg gcg tgg tgg ttg gtg tgt cac ttg atg      528
   His His Ala Leu Leu Ile Trp Ala Trp Trp Leu Val Cys His Leu Met
                   165                 170                 175
   gcc acg aac gat tgt atc gat gcc tac ttc ggc gcg gcg tgc aac tcg      576
   Ala Thr Asn Asp Cys Ile Asp Ala Tyr Phe Gly Ala Ala Cys Asn Ser
               180                 185                 190
   ttc att cac atc gtg atg tac tcg tat tat ctc atg tcg gcg ctc ggc      624
   Phe Ile His Ile Val Met Tyr Ser Tyr Tyr Leu Met Ser Ala Leu Gly
           195                 200                 205
   att cga tgc ccg tgg aag cga tac atc acc cag gct caa atg ctc caa      672
   Ile Arg Cys Pro Trp Lys Arg Tyr Ile Thr Gln Ala Gln Met Leu Gln
       210                 215                 220
   ttc gtc att gtc ttc gcg cac gcc gtg ttc gtg ctg cgt cag aag cac      720
   Phe Val Ile Val Phe Ala His Ala Val Phe Val Leu Arg Gln Lys His
   225                 230                 235                 240
   tgc ccg gtc acc ctt cct tgg gcg caa atg ttc gtc atg acg aac atg      768
   Cys Pro Val Thr Leu Pro Trp Ala Gln Met Phe Val Met Thr Asn Met
                   245                 250                 255
   ctc gtg ctc ttc ggg aac ttc tac ctc aag gcg tac tcg aac aag tcg      816
   Leu Val Leu Phe Gly Asn Phe Tyr Leu Lys Ala Tyr Ser Asn Lys Ser
               260                 265                 270
   cgc ggc gac ggc gcg agt tcc gtg aaa cca gcc gag acc acg cgc gcg      864
   Arg Gly Asp Gly Ala Ser Ser Val Lys Pro Ala Glu Thr Thr Arg Ala
           275                 280                 285
   ccc agc gtg cga cgc acg cga tct cga aaa att gac taa                  903
   Pro Ser Val Arg Arg Thr Arg Ser Arg Lys Ile Asp
       290                 295                 300
<210> 116
   <211> 300
   <212> PRT
   <213> Ostreococcus tauri
<400> 116
   Met Ser Ala Ser Gly Ala Leu Leu Pro Ala Ile Ala Ser Ala Ala Tyr
   1               5                   10                  15
   Ala Tyr Ala Thr Tyr Ala Tyr Ala Phe Glu Trp Ser His Ala Asn Gly
               20                  25                  30
   Ile Asp Asn Val Asp Ala Arg Glu Trp Ile Gly Ala Leu Ser Leu Arg
           35                  40                  45
   Leu Pro Ala Ile Ala Thr Thr Met Tyr Leu Leu Phe Cys Leu Val Gly
       50                  55                  60
   Pro Arg Leu Met Ala Lys Arg Glu Ala Phe Asp Pro Lys Gly Phe Met
   65                  70                  75                  80
   Leu Ala Tyr Asn Ala Tyr Gln Thr Ala Phe Asn Val Val Val Leu Gly
                   85                  90                  95
   Met Phe Ala Arg Glu Ile Ser Gly Leu Gly Gln Pro Val Trp Gly Ser
               100                 105                 110
   Thr Met Pro Trp Ser Asp Arg Lys Ser Phe Lys Ile Leu Leu Gly Val
           115                 120                 125
   Trp Leu His Tyr Asn Asn Lys Tyr Leu Glu Leu Leu Asp Thr Val Phe
       130                 135                 140
   Met Val Ala Arg Lys Lys Thr Lys Gln Leu Ser Phe Leu His Val Tyr
   145                 150                 155                 160
   His His Ala Leu Leu Ile Trp Ala Trp Trp Leu Val Cys His Leu Met
                   165                 170                 175
   Ala Thr Asn Asp Cys Ile Asp Ala Tyr Phe Gly Ala Ala Cys Asn Ser
               180                 185                 190
   Phe Ile His Ile Val Met Tyr Ser Tyr Tyr Leu Met Ser Ala Leu Gly
           195                 200                 205
   Ile Arg Cys Pro Trp Lys Arg Tyr Ile Thr Gln Ala Gln Met Leu Gln
       210                 215                 220
   Phe Val Ile Val Phe Ala His Ala Val Phe Val Leu Arg Gln Lys His
   225                 230                 235                 240
   Cys Pro Val Thr Leu Pro Trp Ala Gln Met Phe Val Met Thr Asn Met
                   245                 250                 255
   Leu Val Leu Phe Gly Asn Phe Tyr Leu Lys Ala Tyr Ser Asn Lys Ser
               260                 265                 270
   Arg Gly Asp Gly Ala Ser Ser Val Lys Pro Ala Glu Thr Thr Arg Ala
           275                 280                 285
   Pro Ser Val Arg Arg Thr Arg Ser Arg Lys Ile Asp
       290                 295                 300
<210> 117
   <211> 339
   <212> PRT
   <213> Artificial sequence
<220>
   <221> Variant
   <222> (3)..(29)
   <223> xaa in position 3 to 29 is any amino acid
<220>
   <221> Variant
   <222> (30)..(43)
   <223> xaa in position 30 to 43 is any or no amino acid
<220>
   <221> Variant
   <222> (45)..(50)
   <223> xaa in position 45 to 50 is any amino acid
<220>
   <221> Variant
   <222> (53)..(73)
   <223> xaa in position 53 to 73 is any amino acid
<220>
   <221> Variant
   <222> (74)..(79)
   <223> xaa in position 74 to 79 is any or no amino acid
<220>
   <221> Variant
   <222> (81)..(82)
   <223> xaa in position 81 to 82 is any amino acid
<220>
   <221> Variant
   <222> (85)..(86)
   <223> xaa in position 85 to 86 is any amino acid
<220>
   <221> Variant
   <222> (88)..(90)
   <223> xaa in position 88 to 90 is any amino acid
<220>
   <221> Variant
   <222> (92)..(110)
   <223> xaa in position 92 to 110 is any amino acid
<220>
   <221> Variant
   <222> (112)..(127)
   <223> xaa in position 112 to 127 is any amino acid
<220>
   <221> Variant
   <222> (128)..(128)
   <223> xaa in position 128 is any or no amino acid
<220>
   <221> Variant
   <222> (130)..(130)
   <223> xaa in position 130 is any amino acid
<220>
   <221> Variant
   <222> (133)..(134)
   <223> xaa in position 133 to 134 is any amino acid
<220>
   <221> Variant
   <222> (137)..(137)
   <223> xaa in position 137 is any amino acid
<220>
   <221> Variant
   <222> (139)..(140)
   <223> xaa in position 139 to 140 is any amino acid
<220>
   <221> Variant
   <222> (143)..(144)
   <223> xaa in position 143 to 144 is any amino acid
<220>
   <221> Variant
   <222> (147)..(147)
   <223> xaa in position 147 is any amino acid
<220>
   <221> Variant
   <222> (149)..(149)
   <223> xaa in position 149 is any amino acid
<220>
   <221> Variant
   <222> (151)..(152)
   <223> xaa in position 151 to 152 is any amino acid
<220>
   <221> Variant
   <222> (154)..(154)
   <223> xaa in position 154 is any amino acid
<220>
   <221> Variant
   <222> (159)..(159)
   <223> xaa in position 159 is any amino acid
<220>
   <221> Variant
   <222> (163)..(166)
   <223> xaa in position 163 to 166 is any amino acid
<220>
   <221> variant
   <222> (170)..(179)
   <223> xaa in position 170 to 179 is any amino acid
<220>
   <221> Variant
   <222> (180)..(182)
   <223> xaa in position 180 to 182 is any or no amino acid
<220>
   <221> Variant
   <222> (184)..(184)
   <223> xaa in position 184 is any amino acid
<220>
   <221> Variant
   <222> (188)..(190)
   <223> xaa in position 188 to 190 is any amino acid
<220>
   <221> Variant
   <222> (193)..(193)
   <223> xaa in position 193 is any amino acid
<220>
   <221> Variant
   <222> (196)..(197)
   <223> xaa in position 196 to 197 is any amino acid
<220>
   <221> Variant
   <222> (203)..(206)
   <223> xaa in position 203 to 206 is any amino acid
<220>
   <221> Variant
   <222> (208)..(210)
   <223> xaa in position 208 to 210 is any amino acid
<220>
   <221> Variant
   <222> (217)..(217)
   <223> xaa in position 217 is any amino acid
<220>
   <221> Variant
   <222> (222)..(223)
   <223> xaa in position 222 to 223 is any amino acid
<220>
   <221> Variant
   <222> (226)..(252)
   <223> xaa in position 226 to 252 is any amino acid
<220>
   <221> Variant
   <222> (253)..(267)
   <223> xaa in position 253 to 267 is any or no amino acid
<220>
   <221> Variant
   <222> (269)..(272)
   <223> xaa in position 269 to 272 is any amino acid
<220>
   <221> Variant
   <222> (276)..(277)
   <223> xaa in position 276 to 277 is any amino acid
<220>
   <221> Variant
   <222> (280)..(282)
   <223> xaa in position 280 to 282 is any amino acid
<220>
   <221> Variant
   <222> (284)..(291)
   <223> xaa in position 284 to 291 is any amino acid
<220>
   <221> Variant
   <222> (292)..(324)
   <223> xaa in position 292 to 324 is any or no amino acid
<220>
   <221> Variant
   <222> (326)..(337)
   <223> xaa in position 326 to 337 is any amino acid
<220>
   <221> Variant
   <222> (338)..(338)
   <223> xaa in position 338 is any or no amino acid
<400> 117
<210> 118
   <211> 45
   <212> PRT
   <213> Artificial sequence
<220>
   <221> Variant
   <222> (4)..(4)
   <223> xaa in position 4 is Ala or Gly
<220>
   <221> Variant
   <222> (5)..(5)
   <223> xaa in position 5 is Ala or Leu
<220>
   <221> Variant
   <222> (6)..(6)
   <223> xaa in position 6 is any amino acid
<220>
   <221> Variant
   <222> (9)..(9)
   <223> xaa in position 9 is Phe or Ile
<220>
   <221> Variant
   <222> (12)..(12)
   <223> xaa in position 12 is any or no amino acid
<220>
   <221> Variant
   <222> (14)..(14)
   <223> xaa in position 14 is any or no amino acid
<220>
   <221> Variant
   <222> (20)..(20)
   <223> xaa in position 20 is any amino acid
<220>
   <221> Variant
   <222> (21)..(21)
   <223> xaa in position 21 is Leu or Met
<220>
   <221> Variant
   <222> (22)..(22)
   <223> xaa in position 22 is Ala or Ser
<220>
   <221> Variant
   <222> (23)..(23)
   <223> xaa in position 23 is Ala or Leu
<220>
   <221> Variant
   <222> (25)..(26)
   <223> xaa in position 25 to 26 is any amino acid
<220>
   <221> Variant
   <222> (27)..(27)
   <223> xaa in position 27 is Arg or Ser
<220>
   <221> Variant
   <222> (34)..(34)
   <223> xaa in position 34 is Ile or Leu
<220>
   <221> Variant
   <222> (39)..(39)
   <223> xaa in position 39 is Leu or Met

<220>
   <221> Variant
   <222> (40)..(40)
   <223> xaa in position 40 is Leu or Val
<220>
   <221> Variant
   <222> (43)..(43)
   <223> xaa in position 43 is Cys, Thr or Val
<220>
   <221> Variant
   <222> (44)..(44)
   <223> xaa in position 44 is any amino acid
<220>
   <221> Variant
   <222> (45)..(45)
   <223> xaa in position 45 is Cys or Val
<400> 118
   Tyr Phe Gly xaa xaa xaa Asn Ser xaa Ile His xaa Val xaa Met Tyr
    1               5                  10                  15
   Ser Tyr Tyr xaa xaa xaa xaa Leu xaa xaa xaa Cys Pro Trp Lys Arg
               20                  25                  30
   Tyr xaa Thr Gln Ala Gln xaa xaa Gln Phe xaa xaa xaa
           35                  40                  45
<210> 119
   <211> 43
   <212> PRT
   <213> Artificial sequence
<220>
   <221> Variant
   <222> (2)..(2)
   <223> xaa in position 2 is Leu or Val
<220>
   <221> Variant
   <222> (5)..(5)
   <223> xaa in position 5 is Cys or Asn
<220>
   <221> Variant
   <222> (6)..(6)
   <223> xaa in position 6 is Asp or Asn
<220>
   <221> Variant
   <222> (9)..(10)
   <223> xaa in position 9 to 10 is any or no amino acid
<220>
   <221> Variant
   <222> (12)..(12)
   <223> xaa in position 12 is any amino acid
<220>
   <221> Variant
   <222> (13)..(14)
   <223> xaa in position 13 to 14 is any or no amino acid
<220>
   <221> Variant
   <222> (17)..(17)
   <223> xaa in position 17 is any amino acid
<220>
   <221> Variant
   <222> (18)..(18)
   <223> xaa in position 18 is Phe or Trp
<220>
   <221> Variant
   <222> (21)..(21)
   <223> xaa in position 21 is Ala or Leu
<220>
   <221> Variant
   <222> (23)..(23)
   <223> xaa in position 23 is any amino acid
<220>
   <221> variant
   <222> (25)..(26)
   <223> xaa in position 25 to 26 is any amino acid
<220>
   <221> Variant
   <222> (28)..(28)
   <223> xaa in position 28 is Leu or Val
<220>
   <221> Variant
   <222> (33)..(33)
   <223> xaa in position 33 is Ile or Val
<220>
   <221> Variant
   <222> (37)..(37)
   <223> xaa in position 37 is Ala, Thr or Val
<220>
   <221> Variant
   <222> (38)..(38)
   <223> xaa in position 38 is any amino acid
<220>
   <221> Variant
   <222> (39)..(39)
   <223> xaa in position 39 is Ile or Leu
<220>
   <221> Variant
   <222> (40)..(40)
   <223> xaa in position 40 is any amino acid
<400> 119
<210> 120
   <211> 37
   <212> PRT
   <213> Artificial sequence
<220>
   <221> Variant
   <222> (2)..(3)
   <223> xaa in position 2 to 3 is any or no amino acid
<220>
   <221> Variant
   <222> (5)..(5)
   <223> xaa in position 5 is any or no amino acid
<220>
   <221> Variant
   <222> (9)..(9)
   <223> xaa in position 9 is Gly, Ser or Thr
<220>
   <221> Variant
   <222> (10)..(10)
   <223> xaa in position 10 is any amino acid
<220>
   <221> Variant
   <222> (13)..(14)
   <223> xaa in position 13 to 14 is any amino acid
<220>
   <221> Variant
   <222> (15)..(15)
   <223> xaa in position 15 is Ala or Ser
<220>
   <221> Variant
   <222> (17)..(17)
   <223> xaa in position 17 is Ala or Ser
<220>
   <221> Variant
   <222> (18)..(18)
   <223> xaa in position 18 is any amino acid
<220>
   <221> Variant
   <222> (19)..(19)
   <223> xaa in position 19 is Glu or Lys
<220>
   <221> Variant
   <222> (20)..(20)
   <223> xaa in position 20 is Ala, Asn or Ser
<220>
   <221> Variant
   <222> (21)..(21)
   <223> xaa in position 21 is Lys or Arg
<220>
   <221> Variant
   <222> (22)..(22)
   <223> xaa in position 22 is Ala, Gly or Ser
<220>
   <221> Variant
   <222> (23)..(23)
   <223> xaa in position 23 is any amino acid
<220>
   <221> Variant
   <222> (24)..(24)
   <223> xaa in position 24 is Glu or Gly
<220>
   <221> Variant
   <222> (25)..(29)
   <223> xaa in position 25 to 29 is any amino acid
<220>
   <221> variant
   <222> (30)..(30)
   <223> xaa in position 30 is Asn, Pro or Gln
<220>
   <221> Variant
   <222> (31)..(32)
   <223> xaa in position 31 to 32 is Ala, Asp or Glu
<220>
   <221> Variant
   <222> (33)..(33)
   <223> xaa in position 33 is Gly, Ser or Thr
<220>
   <221> Variant
   <222> (34)..(34)
   <223> xaa in position 34 is Asn, Gln or Thr
<220>
   <221> variant
   <222> (35)..(36)
   <223> xaa in position 35 to 36 is any amino acid
<220>
   <221> Variant
   <222> (37)..(37)
   <223> xaa in position 37 is Ala, Asp or Pro
<400> 120
<210> 121
   <211> 19
   <212> PRT
   <213> Artificial sequence
<220>
   <221> Variant
   <222> (2)..(3)
   <223> xaa in position 2 to 3 is any amino acid
<220>
   <221> Variant
   <222> (4)..(7)
   <223> xaa in position 4 to 7 is any or no amino acid
<220>
   <221> Variant
   <222> (9)..(9)
   <223> xaa in position 9 is Phe, Leu or Val
<220>
   <221> Variant
   <222> (10)..(10)
   <223> xaa in position 10 is any amino acid
<220>
   <221> Variant
   <222> (13)..(13)
   <223> xaa in position 13 is Ala, Leu or Val
<220>
   <221> Variant
   <222> (14)..(14)
   <223> xaa in position 14 is any amino acid
<220>
   <221> Variant
   <222> (16)..(16)
   <223> xaa in position 16 is Thr or Val
<220>
   <221> Variant
   <222> (17)..(17)
   <223> xaa in position 17 is Ala, Ile or Leu
<220>
   <221> Variant
   <222> (18)..(18)
   <223> xaa in position 18 is Phe, Ile or Leu
<400> 121
<210> 122
   <211> 879
   <212> DNA
   <213> Ostreococcus tauri
<220>
   <221> CDS
   <222> (1)..(879)
<400> 122
   atg tct gga ttg agg gct cct aac ttc ttg cat agg ttc tgg acc aag       48
   Met Ser Gly Leu Arg Ala Pro Asn Phe Leu His Arg Phe Trp Thr Lys
   1               5                   10                  15
   tgg gat tac gct atc tct aag gtg gtg ttc act tgc gct gat tct ttc        96
   Trp Asp Tyr Ala Ile Ser Lys Val Val Phe Thr Cys Ala Asp Ser Phe
               20                  25                  30
   cag tgg gat atc gga cct gtt tct tct tct acc gct cat ttg cct gct       144
   Gln Trp Asp Ile Gly Pro Val Ser Ser Ser Thr Ala His Leu Pro Ala
           35                  40                  45
   att gag tct cct act cct ttg gtg acc tct ttg ctc ttc tac ttg gtg       192
   Ile Glu Ser Pro Thr Pro Leu Val Thr Ser Leu Leu Phe Tyr Leu Val
       50                  55                  60
   act gtg ttc ttg tgg tac gga aga ttg acc aga tcc tcc gat aag aag       240
   Thr Val Phe Leu Trp Tyr Gly Arg Leu Thr Arg Ser Ser Asp Lys Lys
   65                  70                  75                  80
   atc aga gag cct acc tgg ttg agg aga ttc atc atc tgc cac aac gct       288
   Ile Arg Glu Pro Thr Trp Leu Arg Arg Phe Ile Ile Cys His Asn Ala
                   85                  90                  95
   ttc ttg att gtg ctc tcc ttg tac atg tgt ttg gga tgc gtt gct caa       336
   Phe Leu Ile Val Leu Ser Leu Tyr Met Cys Leu Gly Cys Val Ala Gln
               100                 105                 110
   gct tac caa aac gga tac acc ttg tgg gga aac gag ttc aag gct act       384
   Ala Tyr Gln Asn Gly Tyr Thr Leu Trp Gly Asn Glu Phe Lys Ala Thr
           115                 120                 125
   gag acc caa ttg gct ctc tac atc tac atc ttc tac gtg tcc aag atc       432
   Glu Thr Gln Leu Ala Leu Tyr Ile Tyr Ile Phe Tyr Val Ser Lys Ile
       130                 135                 140
   tac gag ttc gtg gat acc tac atc atg ctc ctc aag aac aac ctc agg       480
   Tyr Glu Phe Val Asp Thr Tyr Ile Met Leu Leu Lys Asn Asn Leu Arg
   145                 150                 155                 160
   caa gtg tct ttc ttg cac atc tac cac cac tct acc atc tct ttc atc       528
   Gln Val Ser Phe Leu His Ile Tyr His His Ser Thr Ile Ser Phe Ile
                   165                 170                 175
   tgg tgg atc atc gct aga aga gca cct gga gga gat gct tat ttc tcc       576
   Trp Trp Ile Ile Ala Arg Arg Ala Pro Gly Gly Asp Ala Tyr Phe Ser
               180                 185                 190
   gct gct ctc aac tct tgg gtt cat gtg tgc atg tac act tac tac ctc       624
   Ala Ala Leu Asn Ser Trp Val His Val Cys Met Tyr Thr Tyr Tyr Leu
           195                 200                 205
   ctc tct acc ttg att gga aag gaa gat cct aag agg tct aac tac ctc       672
   Leu Ser Thr Leu Ile Gly Lys Glu Asp Pro Lys Arg Ser Asn Tyr Leu
       210                 215                 220
   tgg tgg gga agg cat ttg acc caa atg caa atg ctc cag ttc ttc ttc       720
   Trp Trp Gly Arg His Leu Thr Gln Met Gln Met Leu Gln Phe Phe Phe
   225                 230                 235                 240
   aac gtg ctc caa gct ctt tat tgc gct tcc ttc tcc act tac cct aag       768
   Asn Val Leu Gln Ala Leu Tyr Cys Ala Ser Phe Ser Thr Tyr Pro Lys
                   245                 250                 255
   ttc ctc tcc aag atc ttg ctc gtg tac atg atg tct ttg ctc gga ctt       816
   Phe Leu Ser Lys Ile Leu Leu Val Tyr Met Met Ser Leu Leu Gly Leu
               260                 265                 270
   ttc gga cac ttc tac tac tct aag cac atc gct gct gct aag ttg caa       864
   Phe Gly His Phe Tyr Tyr Ser Lys His Ile Ala Ala Ala Lys Leu Gln
           275                 280                 285
   aag aag cag cag tga                                                   879
   Lys Lys Gln Gln
       290
<210> 123
   <211> 292
   <212> PRT
   <213> Ostreococcus tauri
<400> 123
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Ser Phe Leu His Ile Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
           275                 280                 285
   Lys Lys Gln Gln
       290
<210> 124
   <211> 1047
   <212> DNA
   <213> Marchantia polymorpha
<220>
   <221> CDS
   <222> (1)..(1047)
<400> 124
   atg gcg acg aag agc ggg agc gga ttg ctc gag tgg ata gca gta gcg      48
   Met Ala Thr Lys Ser Gly Ser Gly Leu Leu Glu Trp Ile Ala Val Ala
   1               5                   10                  15
   gcg aag atg aag caa gct cgg agc agc ccc gag ggt gag atc gtg ggt      96
   Ala Lys Met Lys Gln Ala Arg Ser Ser Pro Glu Gly Glu Ile Val Gly
               20                  25                  30
   ggg aat agg atg ggc tct gga aac gga gct gag tgg acc acg agt ctg      144
   Gly Asn Arg Met Gly Ser Gly Asn Gly Ala Glu Trp Thr Thr Ser Leu
           35                  40                  45
   att cat gca ttt ttg aat gcc acg aat ggg aag agc ggc ggt gct tcg      192
   Ile His Ala Phe Leu Asn Ala Thr Asn Gly Lys Ser Gly Gly Ala Ser
       50                  55                  60
   aaa gtg agg cct ctc gag gag aga atc ggg gag gcg gtg ttc aga gtt      240
   Lys Val Arg Pro Leu Glu Glu Arg Ile Gly Glu Ala Val Phe Arg Val
   65                  70                  75                  80
   ctt gaa gat gtc gtg ggc gtg gat att agg aag ccg aat cct gtc acg      288
   Leu Glu Asp Val Val Gly Val Asp Ile Arg Lys Pro Asn Pro Val Thr
                   85                  90                  95
   aag gac ctt ccg atg gtc gag agt ccc gtg ccc gtg ttg gcc tgc att      336
   Lys Asp Leu Pro Met Val Glu Ser Pro Val Pro Val Leu Ala Cys Ile
               100                 105                 110
   tct ctg tac ttg ctc gtg gtg tgg ctt tgg tct tct cac att aag gcg      384
   Ser Leu Tyr Leu Leu Val Val Trp Leu Trp Ser Ser His Ile Lys Ala
           115                 120                 125
   tct ggc caa aag ccc agg aag gag gac ccg ctc gcc ctg cgt tgc ctt      432
   Ser Gly Gln Lys Pro Arg Lys Glu Asp Pro Leu Ala Leu Arg Cys Leu
       130                     135                     140
   gtg att gcc cac aat ctg ttc ctg tgt tgc ttg agc ttg ttc atg tgc      480
   Val Ile Ala His Asn Leu Phe Leu Cys Cys Leu Ser Leu Phe Met Cys
   145                     150                     155                 160
   gtc ggt ctc att gcc gca gct cga cat tac ggg tat agt gta tgg ggg      528
   Val Gly Leu Ile Ala Ala Ala Arg His Tyr Gly Tyr Ser Val Trp Gly
                   165                     170                     175
   aac tac tac aga gaa aga gaa ccc gca atg aat ttg ctc att tac gtg      576
   Asn Tyr Tyr Arg Glu Arg Glu Pro Ala Met Asn Leu Leu Ile Tyr Val
               180                     185                     190
   ttc tac atg tcg aag ctg tac gaa ttt atg gac acg gcc att atg tta      624
   Phe Tyr Met Ser Lys Leu Tyr Glu Phe Met Asp Thr Ala Ile Met Leu
           195                     200                     205
   ttc aga aga aat ctg cga caa gtc acg tac ttg cat gta tat cac cac      672
   Phe Arg Arg Asn Leu Arg Gln Val Thr Tyr Leu His Val Tyr His His
       210                     215                     220
   gca agc atc gca atg att tgg tgg ata att tgc tat cgg ttt cca gga      720
   Ala Ser Ile Ala Met Ile Trp Trp Ile Ile Cys Tyr Arg Phe Pro Gly
   225                     230                     235                 240
   gct gat tcg tat ttc tcc gca gca ttc aat tcc tgt atc cat gta gcg      768
   Ala Asp Ser Tyr Phe Ser Ala Ala Phe Asn Ser Cys Ile His Val Ala
                   245                     250                     255
   atg tac ctg tat tat cta ctc gcg gca acc gtc gcc aga gac gaa aag      816
   Met Tyr Leu Tyr Tyr Leu Leu Ala Ala Thr Val Ala Arg Asp Glu Lys
               260                     265                     270
   cgg aga cgc aaa tat ctc ttc tgg gga aag tat ctg acc atc ata caa      864
   Arg Arg Arg Lys Tyr Leu Phe Trp Gly Lys Tyr Leu Thr Ile Ile Gln
           275                     280                     285
   atg ctt cag ttt ttg tcc ttc att ggg cag gcg att tat gca atg tgg      912
   Met Leu Gln Phe Leu Ser Phe Ile Gly Gln Ala Ile Tyr Ala Met Trp
       290                     295                     300
   aag ttt gaa tac tat ccc aag ggc ttt ggc agg atg ttg ttc ttt tac      960
   Lys Phe Glu Tyr Tyr Pro Lys Gly Phe Gly Arg Met Leu Phe Phe Tyr
   305                     310                     315                 320
   tct gta tca ttg ttg gca ttt ttc ggc aac ttc ttt gtc aaa aag tat     1008
   Ser Val Ser Leu Leu Ala Phe Phe Gly Asn Phe Phe Val Lys Lys Tyr
                   325                     330                     335
   tcg aac gct tca cag cct aag aca gtt aaa gtg gag tga                 1047
   Ser Asn Ala Ser Gln Pro Lys Thr Val Lys Val Glu
               340                     345
<210> 125
   <211> 348
   <212> PRT
   <213> Marchantia polymorpha
<400> 125
   Met Ala Thr Lys Ser Gly Ser Gly Leu Leu Glu Trp Ile Ala Val Ala
   1               5                   10                  15
   Ala Lys Met Lys Gln Ala Arg Ser Ser Pro Glu Gly Glu Ile Val Gly
               20                  25                  30
   Gly Asn Arg Met Gly Ser Gly Asn Gly Ala Glu Trp Thr Thr Ser Leu
           35                  40                  45
   Ile His Ala Phe Leu Asn Ala Thr Asn Gly Lys Ser Gly Gly Ala Ser
       50                  55                  60
   Lys Val Arg Pro Leu Glu Glu Arg Ile Gly Glu Ala Val Phe Arg Val
   65                  70                  75                  80
   Leu Glu Asp Val Val Gly Val Asp Ile Arg Lys Pro Asn Pro Val Thr
                   85                  90                  95
   Lys Asp Leu Pro Met Val Glu Ser Pro Val Pro Val Leu Ala Cys Ile
               100                 105                 110
   Ser Leu Tyr Leu Leu Val Val Trp Leu Trp Ser Ser His Ile Lys Ala
           115                 120                 125
   Ser Gly Gln Lys Pro Arg Lys Glu Asp Pro Leu Ala Leu Arg Cys Leu
       130                 135                 140
   Val Ile Ala His Asn Leu Phe Leu Cys Cys Leu Ser Leu Phe Met Cys
   145                 150                 155                 160
   Val Gly Leu Ile Ala Ala Ala Arg His Tyr Gly Tyr Ser Val Trp Gly
                   165                 170                 175
   Asn Tyr Tyr Arg Glu Arg Glu Pro Ala Met Asn Leu Leu Ile Tyr Val
               180                 185                 190
   Phe Tyr Met Ser Lys Leu Tyr Glu Phe Met Asp Thr Ala Ile Met Leu
           195                 200                 205
   Phe Arg Arg Asn Leu Arg Gln Val Thr Tyr Leu His Val Tyr His His
       210                 215                 220
   Ala Ser Ile Ala Met Ile Trp Trp Ile Ile Cys Tyr Arg Phe Pro Gly
   225                 230                 235                 240
   Ala Asp Ser Tyr Phe Ser Ala Ala Phe Asn Ser Cys Ile His Val Ala
                   245                 250                 255
   Met Tyr Leu Tyr Tyr Leu Leu Ala Ala Thr Val Ala Arg Asp Glu Lys
               260                 265                 270
   Arg Arg Arg Lys Tyr Leu Phe Trp Gly Lys Tyr Leu Thr Ile Ile Gln
           275                 280                 285
   Met Leu Gln Phe Leu Ser Phe Ile Gly Gln Ala Ile Tyr Ala Met Trp
       290                 295                 300
   Lys Phe Glu Tyr Tyr Pro Lys Gly Phe Gly Arg Met Leu Phe Phe Tyr
   305                 310                 315                 320
   Ser Val Ser Leu Leu Ala Phe Phe Gly Asn Phe Phe Val Lys Lys Tyr
                   325                 330                 335
   Ser Asn Ala Ser Gln Pro Lys Thr Val Lys Val Glu
               340                 345
<210> 126
   <211> 831
   <212> DNA
   <213> Thraustochytrium sp
<220>
   <221> CDS
   <222> (1)..(831)
<400> 126
   atg gat gtc gtc gag cag caa tgg cgc cgc ttc gtg gac gcc gtg gac        48
   Met Asp Val Val Glu Gln Gln Trp Arg Arg Phe Val Asp Ala Val Asp
   1               5                   10                  15
   aac gga atc gtg gag ttc atg gag cat gag gag ccc aac aag ctg aac        96
   Asn Gly Ile Val Glu Phe Met Glu His Glu Glu Pro Asn Lys Leu Asn
               20                  25                  30
   gag ggc aag ctc tcc acc tcg acc gag gag atg atg gcg ctt atc gtc        144
   Glu Gly Lys Leu Ser Thr Ser Thr Glu Glu Met Met Ala Leu Ile Val
           35                  40                  45
   ggc tac ctg gcg ttc gtg gtc ctc ggg tcc gcc ttc atg aag gcc ttt        192
   Gly Tyr Leu Ala Phe Val Val Leu Gly Ser Ala Phe Met Lys Ala Phe
       50                  55                  60
   gtc gat aag cct ttc gag ctc aag ttc ctc aag ctc gtg cac aac atc        240
   Val Asp Lys Pro Phe Glu Leu Lys Phe Leu Lys Leu Val His Asn Ile
   65                  70                  75                  80
   ttc ctc acc ggt ctg tcc atg tac atg gcc acc gag tgc gcg cgc cag        288
   Phe Leu Thr Gly Leu Ser Met Tyr Met Ala Thr Glu Cys Ala Arg Gln
                   85                  90                  95
   gca tac ctc ggc ggc tac aag ctc ttt ggc aac ccg atg gag aag ggc        336
   Ala Tyr Leu Gly Gly Tyr Lys Leu Phe Gly Asn Pro Met Glu Lys Gly
               100                 105                 110
   acc gag tcg cac gcc ccg ggc atg gcc aac atc atc tac atc ttc tac        384
   Thr Glu Ser His Ala Pro Gly Met Ala Asn Ile Ile Tyr Ile Phe Tyr
           115                 120                 125
   gtg agc aag ttc ctc gaa ttc ctc gac acc gtc ttc atg atc ctc ggc        432
   Val Ser Lys Phe Leu Glu Phe Leu Asp Thr Val Phe Met Ile Leu Gly
       130                 135                 140
   aag aag tgg aag cag ctc agc ttt ctc cac gtc tac cac cac gcg agc        480
   Lys Lys Trp Lys Gln Leu Ser Phe Leu His Val Tyr His His Ala Ser
   145                 150                 155                 160
   atc agc ttc atc tgg ggc atc atc gcc cgc ttc gcg ccc ggt ggc gac        528
   Ile Ser Phe Ile Trp Gly Ile Ile Ala Arg Phe Ala Pro Gly Gly Asp
                   165                 170                 175
   gcc tac ttc tct acc atc ctc aac agc agc gtg cat gtc gtg ctc tac        576
   Ala Tyr Phe Ser Thr Ile Leu Asn Ser Ser Val His Val Val Leu Tyr
               180                 185                 190
   ggc tac tac gcc tcg acc acc ctc ggc tac acc ttc atg cgc ccg ctg        624
   Gly Tyr Tyr Ala Ser Thr Thr Leu Gly Tyr Thr Phe Met Arg Pro Leu
           195                 200                 205
   cgc ccg tac att acc acc att cag ctc acg cag ttc atg gcc atg gtc        672
   Arg Pro Tyr Ile Thr Thr Ile Gln Leu Thr Gln Phe Met Ala Met Val
       210                 215                 220
   gtc cag tcc gtc tat gac tac tac aac ccc tgc gac tac ccg cag ccc        720
   Val Gln Ser Val Tyr Asp Tyr Tyr Asn Pro Cys Asp Tyr Pro Gln Pro
   225                 230                 235                 240
   ctc gtc aag ctg ctc ttc tgg tac atg ctc acc atg ctc ggc ctc ttc        768
   Leu Val Lys Leu Leu Phe Trp Tyr Met Leu Thr Met Leu Gly Leu Phe
                   245                 250                 255
   ggc aac ttc ttc gtg cag cag tac ctc aag ccc aag gcg ccc aag aag        816
   Gly Asn Phe Phe Val Gln Gln Tyr Leu Lys Pro Lys Ala Pro Lys Lys
               260                 265                 270
   cag aag acc atc taa                                                    831
   Gln Lys Thr Ile
           275
<210> 127
   <211> 276
   <212> PRT
   <213> Thraustochytrium sp
<400> 127
   Met Asp Val Val Glu Gln Gln Trp Arg Arg Phe Val Asp Ala Val Asp
   1               5                   10                  15
   Asn Gly Ile Val Glu Phe Met Glu His Glu Glu Pro Asn Lys Leu Asn
               20                  25                  30
   Glu Gly Lys Leu Ser Thr Ser Thr Glu Glu Met Met Ala Leu Ile Val
           35                  40                  45
   Gly Tyr Leu Ala Phe Val Val Leu Gly Ser Ala Phe Met Lys Ala Phe
       50                  55                  60
   Val Asp Lys Pro Phe Glu Leu Lys Phe Leu Lys Leu Val His Asn Ile
   65                  70                  75                  80
   Phe Leu Thr Gly Leu Ser Met Tyr Met Ala Thr Glu Cys Ala Arg Gln
                   85                  90                  95
   Ala Tyr Leu Gly Gly Tyr Lys Leu Phe Gly Asn Pro Met Glu Lys Gly
               100                 105                 110
   Thr Glu Ser His Ala Pro Gly Met Ala Asn Ile Ile Tyr Ile Phe Tyr
           115                 120                 125
   Val Ser Lys Phe Leu Glu Phe Leu Asp Thr Val Phe Met Ile Leu Gly
       130                 135                 140
   Lys Lys Trp Lys Gln Leu Ser Phe Leu His Val Tyr His His Ala Ser
   145                 150                 155                 160
   Ile Ser Phe Ile Trp Gly Ile Ile Ala Arg Phe Ala Pro Gly Gly Asp
                   165                 170                 175
   Ala Tyr Phe Ser Thr Ile Leu Asn Ser Ser Val His Val Val Leu Tyr
               180                 185                 190
   Gly Tyr Tyr Ala Ser Thr Thr Leu Gly Tyr Thr Phe Met Arg Pro Leu
           195                 200                 205
   Arg Pro Tyr Ile Thr Thr Ile Gln Leu Thr Gln Phe Met Ala Met Val
       210                 215                 220
   Val Gln Ser Val Tyr Asp Tyr Tyr Asn Pro Cys Asp Tyr Pro Gln Pro
   225                 230                 235                 240
   Leu Val Lys Leu Leu Phe Trp Tyr Met Leu Thr Met Leu Gly Leu Phe
                   245                 250                 255
   Gly Asn Phe Phe Val Gln Gln Tyr Leu Lys Pro Lys Ala Pro Lys Lys
               260                 265                 270
   Gln Lys Thr Ile
           275
<210> 128
   <211> 1146
   <212> DNA
   <213> Leishmania major
<220>
   <221> CDS
   <222> (1)..(1146)
<400> 128
   atg gtc tct ctg gag cag gcc gaa cag atc gcg gcc gcg atc gag gta 48
   Met Val Ser Leu Glu Gln Ala Glu Gln Ile Ala Ala Ala Ile Glu Val
   1 5 10 15
   cct gac tgg gtc ttg aca aag tct gcg gcg ctg gtg tac agc tgc ttc 96
   Pro Asp Trp Val Leu Thr Lys Ser Ala Ala Leu Val Tyr Ser Cys Phe
   20 25 30
   ggc tcc gcc gcc aat gct ttt gaa agc agt atc aag atc aac ttc ccg 144
   Gly Ser Ala Ala Asn Ala Phe Glu Ser Ser Ile Lys Ile Asn Phe Pro
   35 40 45
   gcg cag cat gcg ttc gtg gag gcg tgg atg cgc gcg cgc tcc cac cct 192
   Ala Gln His Ala Phe Val Glu Ala Trp Met Arg Ala Arg Ser His Pro
   50 55 60
   ttt gcg gag cgc ctg ccg tac ctg aat ccg tgg cac gtt atc gcc tcg 240
   Phe Ala Glu Arg Leu Pro Tyr Leu Asn Pro Trp His Val Ile Ala Ser
   65 70 75 80
   ata ctg gcc tac ctc tcc ttg att gtc acc ttg cgc ctg ttg cat cgt 288
   Ile Leu Ala Tyr Leu Ser Leu Ile Val Thr Leu Arg Leu Leu His Arg
   85 90 95
   gta ctc ggt aag ttc tcg tgc cgc act ctc gga ttg gtg cac aac ctc 336
   Val Leu Gly Lys Phe Ser Cys Arg Thr Leu Gly Leu Val His Asn Leu
   100 105 110
   ggt ctc cat ctt ctc tcg ttg tac atg agc ctt ggt ctc atg atc agc 384
   Gly Leu His Leu Leu Ser Leu Tyr Met Ser Leu Gly Leu Met Ile Ser
   115 120 125
   gcg cgc gcc gcg ggg tac tcg ctc tgg aac aac gcg gtc ggc acc tcc 432
   Ala Arg Ala Ala Gly Tyr Ser Leu Trp Asn Asn Ala Val Gly Thr Ser
   130 135 140
   ccg gct gag tgg cgc att gcg aag ctg atc tgg ctc ttc tat gtc tcg 480
   Pro Ala Glu Trp Arg Ile Ala Lys Leu Ile Trp Leu Phe Tyr Val Ser
   145 150 155 160
   aag gtg gtg gaa tgg gtg gac acg gta att atg tta tta aag cag aac 528
   Lys Val Val Glu Trp Val Asp Thr Val Ile Met Leu Leu Lys Gln Asn
   165 170 175
   tac cac cag gtc acc ttc ctg cac gtg tat cac cac acg acg gtt ttt 576
   Tyr His Gln Val Thr Phe Leu His Val Tyr His His Thr Thr Val Phe
   180 185 190
   gtg ctg tgg tgg ctg gcg ttg ctg gtc gct cct ggc ggc gag tcg tac 624
   Val Leu Trp Trp Leu Ala Leu Leu Val Ala Pro Gly Gly Glu Ser Tyr
   195 200 205
   tac agc gcc atg gtg aac tct ggc gtc cac gtt ttc atg tac ggg tac 672
   Tyr Ser Ala Met Val Asn Ser Gly Val His Val Phe Met Tyr Gly Tyr
   210 215 220
   tac ttt ctc acg ctg ctc ttc cca tcc ggc atc gtg cgc gac gtc ttg 720
   Tyr Phe Leu Thr Leu Leu Phe Pro Ser Gly Ile Val Arg Asp Val Leu
   225 230 235 240
   agc aag ttc aag ttt gcc att acg aag ggc cag atg tgg cag ttc gtc 768
   Ser Lys Phe Lys Phe Ala Ile Thr Lys Gly Gln Met Trp Gln Phe Val
   245 250 255
   ttc aac tgc cta cag tcc gcg tac gac ctc gtg tgg gtg ccg cgg gaa 816
   Phe Asn Cys Leu Gln Ser Ala Tyr Asp Leu Val Trp Val Pro Arg Glu
   260 265 270
   gag ctc aag tac agc gcg gag ctg ctg cag atc ctc ttc tgg tac atg 864
   Glu Leu Lys Tyr Ser Ala Glu Leu Leu Gln Ile Leu Phe Trp Tyr Met
   275 280 285
   atc tcc ctc ttg gcg ctc ttt ggc aac ttc ttg gtg aag aac aag aag 912
   Ile Ser Leu Leu Ala Leu Phe Gly Asn Phe Leu Val Lys Asn Lys Lys
   290 295 300
   ttc tcg cac cgc cgc tgc gtt gat gcc gcg act gct tcg ggc gcg aag 960
   Phe Ser His Arg Arg Cys Val Asp Ala Ala Thr Ala Ser Gly Ala Lys
   305 310 315 320
   gag gac acg gcg gcg agg tcc cac ggc gac cgc acc cac aga acc cgt 1008
   Glu Asp Thr Ala Ala Arg Ser His Gly Asp Arg Thr His Arg Thr Arg
                       325                     330                     335
   gtg aag gct ggc atg acc aac atg caa ctg gag agg ctg aag aat gag        1056
   Val Lys Ala Gly Met Thr Asn Met Gln Leu Glu Arg Leu Lys Asn Glu
               340                     345                     350
   aag tcc acg gag atg aag ctg ctg atg cgc aag aac ggc aac ggc aac        1104
   Lys Ser Thr Glu Met Lys Leu Leu Met Arg Lys Asn Gly Asn Gly Asn
           355                     360                     365
   gga caa aaa gcg tcg ctc cag gcc atg gca ggc agt cga tga                1146
   Gly Gln Lys Ala Ser Leu Gln Ala Met Ala Gly Ser Arg
       370                     375                     380
<210> 129
   <211> 381
   <212> PRT
   <213> Leishmania major
<400> 129
   Met Val Ser Leu Glu Gln Ala Glu Gln Ile Ala Ala Ala Ile Glu Val
   1               5                   10                  15
   Pro Asp Trp Val Leu Thr Lys Ser Ala Ala Leu Val Tyr Ser Cys Phe
               20                  25                  30
   Gly Ser Ala Ala Asn Ala Phe Glu Ser Ser Ile Lys Ile Asn Phe Pro
           35                  40                  45
   Ala Gln His Ala Phe Val Glu Ala Trp Met Arg Ala Arg Ser His Pro
       50                  55                  60
   Phe Ala Glu Arg Leu Pro Tyr Leu Asn Pro Trp His Val Ile Ala Ser
   65                  70                  75                  80
   Ile Leu Ala Tyr Leu Ser Leu Ile Val Thr Leu Arg Leu Leu His Arg
                   85                  90                  95
   Val Leu Gly Lys Phe Ser Cys Arg Thr Leu Gly Leu Val His Asn Leu
               100                 105                 110
   Gly Leu His Leu Leu Ser Leu Tyr Met Ser Leu Gly Leu Met Ile Ser
           115                 120                 125
   Ala Arg Ala Ala Gly Tyr Ser Leu Trp Asn Asn Ala Val Gly Thr Ser
       130                 135                 140
   Pro Ala Glu Trp Arg Ile Ala Lys Leu Ile Trp Leu Phe Tyr Val Ser
   145                 150                 155                 160
   Lys Val Val Glu Trp Val Asp Thr Val Ile Met Leu Leu Lys Gln Asn
                   165                 170                 175
   Tyr His Gln Val Thr Phe Leu His Val Tyr His His Thr Thr Val Phe
               180                 185                 190
   Val Leu Trp Trp Leu Ala Leu Leu Val Ala Pro Gly Gly Glu Ser Tyr
           195                 200                 205
   Tyr Ser Ala Met Val Asn Ser Gly Val His Val Phe Met Tyr Gly Tyr
       210                 215                 220
   Tyr Phe Leu Thr Leu Leu Phe Pro Ser Gly Ile Val Arg Asp Val Leu
   225                 230                 235                 240
   Ser Lys Phe Lys Phe Ala Ile Thr Lys Gly Gln Met Trp Gln Phe Val
                   245                 250                 255
   Phe Asn Cys Leu Gln Ser Ala Tyr Asp Leu Val Trp Val Pro Arg Glu
               260                 265                 270
   Glu Leu Lys Tyr Ser Ala Glu Leu Leu Gln Ile Leu Phe Trp Tyr Met
           275                 280                 285
   Ile Ser Leu Leu Ala Leu Phe Gly Asn Phe Leu Val Lys Asn Lys Lys
       290                 295                 300
   Phe Ser His Arg Arg Cys Val Asp Ala Ala Thr Ala Ser Gly Ala Lys
   305                 310                 315                 320
   Glu Asp Thr Ala Ala Arg Ser His Gly Asp Arg Thr His Arg Thr Arg
                   325                 330                 335
   Val Lys Ala Gly Met Thr Asn Met Gln Leu Glu Arg Leu Lys Asn Glu
               340                 345                 350
   Lys Ser Thr Glu Met Lys Leu Leu Met Arg Lys Asn Gly Asn Gly Asn
           355                 360                 365
   Gly Gln Lys Ala Ser Leu Gln Ala Met Ala Gly Ser Arg
       370                 375                 380
<210> 130
   <211> 879
   <212> DNA
   <213> Ostreococcus tauri
<220>
   <221> CDS
   <222> (1)..(879)
<400> 130
   atg agt ggc tta cgt gca ccc aac ttt tta cac aga ttc tgg aca aag       48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt       96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc       144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc       192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa       240
   Thr Val Phe Leu Trp Tyr Gly Arg Leu Thr Arg Ser Ser Asp Lys Lys
   65                  70                  75                  80
   att aga gag cct acg tgg tta aga aga ttc ata ata tgt cat aat gcg       288
   Ile Arg Glu Pro Thr Trp Leu Arg Arg Phe Ile Ile Cys His Asn Ala
                   85                  90                  95
   ttc ttg ata gtc ctc agt ctt tac atg tgc ctt ggt tgt gtg gcc caa       336
   Phe Leu Ile Val Leu Ser Leu Tyr Met Cys Leu Gly Cys Val Ala Gln
               100                 105                 110
   gcg tat cag aat gga tat act tta tgg ggt aat gaa ttc aag gcc acg       384
   Ala Tyr Gln Asn Gly Tyr Thr Leu Trp Gly Asn Glu Phe Lys Ala Thr
           115                 120                 125
   gaa act cag ctt gct ctc tac att tac att ttt tac gta agt aaa ata       432
   Glu Thr Gln Leu Ala Leu Tyr Ile Tyr Ile Phe Tyr Val Ser Lys Ile
       130                 135                 140
   tac gag ttt gta gat act tac att atg ctt ctc aag aat aac ttg cgg       480
   Tyr Glu Phe Val Asp Thr Tyr Ile Met Leu Leu Lys Asn Asn Leu Arg
   145                 150                 155                 160
   caa gta agt ttc cta cac att tat cac cac agc acg att tcc ttt att       528
   Gln Val Ser Phe Leu His Ile Tyr His His Ser Thr Ile Ser Phe Ile
                   165                 170                 175
   tgg tgg atc att gct cgg agg gct ccg ggt ggt gat gct tac ttc agc       576
   Trp Trp Ile Ile Ala Arg Arg Ala Pro Gly Gly Asp Ala Tyr Phe Ser
               180                 185                 190
   gcg gcc ttg aac tca tgg gta cac gtg tgc atg tac acc tat tat cta       624
   Ala Ala Leu Asn Ser Trp Val His Val Cys Met Tyr Thr Tyr Tyr Leu
           195                 200                 205
   tta tca acc ctt att gga aaa gaa gat cct aag cgt tcc aac tac ctt       672
   Leu Ser Thr Leu Ile Gly Lys Glu Asp Pro Lys Arg Ser Asn Tyr Leu
       210                 215                 220
   tgg tgg ggt cgc cac cta acg caa atg cag atg ctt cag ttt ttc ttc       720
   Trp Trp Gly Arg His Leu Thr Gln Met Gln Met Leu Gln Phe Phe Phe
   225                 230                 235                 240
   aac gta ctt caa gcg ttg tac tgc gct tcg ttc tct acg tat ccc aag       768
   Asn Val Leu Gln Ala Leu Tyr Cys Ala Ser Phe Ser Thr Tyr Pro Lys
                   245                 250                 255
   ttt ttg tcc aaa att ctg ctc gtc tat atg atg agc ctt ctc ggc ttg       816
   Phe Leu Ser Lys Ile Leu Leu Val Tyr Met Met Ser Leu Leu Gly Leu
               260                 265                 270
   ttt ggg cat ttc tac tat tcc aag cac ata gca gca gct aag ctc cag       864
   Phe Gly His Phe Tyr Tyr Ser Lys His Ile Ala Ala Ala Lys Leu Gln
           275                 280                 285
   aaa aaa cag cag tga                                                   879
   Lys Lys Gln Gln
       290
<210> 131
   <211> 292
   <212> PRT
   <213> Ostreococcus tauri
<400> 131
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Ser Phe Leu His Ile Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
           275                 280                 285
   Lys Lys Gln Gln
       290
<210> 132
   <211> 873
   <212> DNA
   <213> Marchantia polymorpha
<220>
   <221> CDS
   <222> (1)..(873)
<400> 132
   atg gag gcg tac gag atg gtg gat agt ttt gtg tcg aag acg gtt ttc      48
   Met Glu Ala Tyr Glu Met Val Asp Ser Phe Val Ser Lys Thr Val Phe
   1               5                   10                  15
   gaa acg ctg cag aga ctg agg ggc gga gtc gtg ttg acg gaa tct gcg      96
   Glu Thr Leu Gln Arg Leu Arg Gly Gly Val Val Leu Thr Glu Ser Ala
               20                  25                  30
   atc acc aaa ggt ttg cca tgc gtc gat agc ccg acg ccg atc gtt ctt      144
   Ile Thr Lys Gly Leu Pro Cys Val Asp Ser Pro Thr Pro Ile Val Leu
           35                  40                  45
   ggg ttg tcg tcc tac ttg aca ttc gtg ttt ctc ggg ctc att gtc atc      192
   Gly Leu Ser Ser Tyr Leu Thr Phe Val Phe Leu Gly Leu Ile Val Ile
       50                  55                  60
   aag agc ctg gat ctt aag ccc cgc tcc aag gag ccc gcc att ttg aac      240
   Lys Ser Leu Asp Leu Lys Pro Arg Ser Lys Glu Pro Ala Ile Leu Asn
   65                  70                  75                  80
   ctg ttt gtg atc ttc cac aac ttc gtc tgc ttc gca ctc agt ctg tac      288
   Leu Phe Val Ile Phe His Asn Phe Val Cys Phe Ala Leu Ser Leu Tyr
                   85                  90                  95
   atg tgc gtg gga att gtc cgt caa gct atc ctc aac agg tac tct ctg      336
   Met Cys Val Gly Ile Val Arg Gln Ala Ile Leu Asn Arg Tyr Ser Leu
               100                 105                 110
   tgg ggc aat gcg tac aat ccc aaa gaa gtt caa atg ggc cac ctg ctc      384
   Trp Gly Asn Ala Tyr Asn Pro Lys Glu Val Gln Met Gly His Leu Leu
           115                 120                 125
   tac att ttc tac atg tca aag tac atc gag ttt atg gac acg gtc att      432
   Tyr Ile Phe Tyr Met Ser Lys Tyr Ile Glu Phe Met Asp Thr Val Ile
       130                 135                 140
   atg att ttg aag cgc aac acg cgc cag atc act gtg ttg cat gtg tac      480
   Met Ile Leu Lys Arg Asn Thr Arg Gln Ile Thr Val Leu His Val Tyr
   145                 150                 155                 160
   cac cac gca tcc atc tcc ttc atc tgg tgg atc atc gcc tac cat gct      528
   His His Ala Ser Ile Ser Phe Ile Trp Trp Ile Ile Ala Tyr His Ala
                   165                 170                 175
   cct ggc ggt gaa gct tat ttc tct gcc gca ttg aac tcc gga gta cat      576
   Pro Gly Gly Glu Ala Tyr Phe Ser Ala Ala Leu Asn Ser Gly Val His
               180                 185                 190
   gtg ctc atg tac ctc tac tac ctt ttg gca gca act ctg gga aag aac      624
   Val Leu Met Tyr Leu Tyr Tyr Leu Leu Ala Ala Thr Leu Gly Lys Asn
           195                 200                 205
   gag aaa gct cgc cgc aag tac cta tgg tgg gga aaa tac ttg aca cag      672
   Glu Lys Ala Arg Arg Lys Tyr Leu Trp Trp Gly Lys Tyr Leu Thr Gln
       210                 215                 220
   ctg cag atg ttc cag ttt gtc ctt aac atg att cag gct tac tac gat      720
   Leu Gln Met Phe Gln Phe Val Leu Asn Met Ile Gln Ala Tyr Tyr Asp
   225                 230                 235                 240
   att aag aac aac tcg cct tac cca caa ttt ttg atc cag att ttg ttc      768
   Ile Lys Asn Asn Ser Pro Tyr Pro Gln Phe Leu Ile Gln Ile Leu Phe
                   245                 250                 255
   tac tac atg atc tcg ctt tta gcg cta ttt gga aac ttt tac gtt cac      816
   Tyr Tyr Met Ile Ser Leu Leu Ala Leu Phe Gly Asn Phe Tyr Val His
               260                 265                 270
   aaa tac gta tca gcg ccc gca aaa cct gcg aag atc aag agc aaa aag      864
   Lys Tyr Val Ser Ala Pro Ala Lys Pro Ala Lys Ile Lys Ser Lys Lys
           275                 280                 285
   gca gaa taa                                                          873
   Ala Glu
       290
<210> 133
   <211> 290
   <212> PRT
   <213> Marchantia polymorpha
<400> 133
   Met Glu Ala Tyr Glu Met Val Asp Ser Phe Val Ser Lys Thr Val Phe
   1               5                   10                  15
   Glu Thr Leu Gln Arg Leu Arg Gly Gly Val Val Leu Thr Glu Ser Ala
               20                  25                  30
   Ile Thr Lys Gly Leu Pro Cys Val Asp Ser Pro Thr Pro Ile Val Leu
           35                  40                  45
   Gly Leu Ser Ser Tyr Leu Thr Phe Val Phe Leu Gly Leu Ile Val Ile
       50                  55                  60
   Lys Ser Leu Asp Leu Lys Pro Arg Ser Lys Glu Pro Ala Ile Leu Asn
   65                  70                  75                  80
   Leu Phe Val Ile Phe His Asn Phe Val Cys Phe Ala Leu Ser Leu Tyr
                   85                  90                  95
   Met Cys Val Gly Ile Val Arg Gln Ala Ile Leu Asn Arg Tyr Ser Leu
               100                 105                 110
   Trp Gly Asn Ala Tyr Asn Pro Lys Glu Val Gln Met Gly His Leu Leu
           115                 120                 125
   Tyr Ile Phe Tyr Met Ser Lys Tyr Ile Glu Phe Met Asp Thr Val Ile
       130                 135                 140
   Met Ile Leu Lys Arg Asn Thr Arg Gln Ile Thr Val Leu His Val Tyr
   145                 150                 155                 160
   His His Ala Ser Ile Ser Phe Ile Trp Trp Ile Ile Ala Tyr His Ala
                   165                 170                 175
   Pro Gly Gly Glu Ala Tyr Phe Ser Ala Ala Leu Asn Ser Gly Val His
               180                 185                 190
   Val Leu Met Tyr Leu Tyr Tyr Leu Leu Ala Ala Thr Leu Gly Lys Asn
           195                 200                 205
   Glu Lys Ala Arg Arg Lys Tyr Leu Trp Trp Gly Lys Tyr Leu Thr Gln
       210                 215                 220
   Leu Gln Met Phe Gln Phe Val Leu Asn Met Ile Gln Ala Tyr Tyr Asp
   225                 230                 235                 240
   Ile Lys Asn Asn Ser Pro Tyr Pro Gln Phe Leu Ile Gln Ile Leu Phe
                   245                 250                 255
   Tyr Tyr Met Ile Ser Leu Leu Ala Leu Phe Gly Asn Phe Tyr Val His
               260                 265                 270
   Lys Tyr Val Ser Ala Pro Ala Lys Pro Ala Lys Ile Lys Ser Lys Lys
           275                 280                 285
   Ala Glu
       290
<210> 134
   <211> 873
   <212> DNA
   <213> Physcomitrella patens
<220>
   <221> CDS
   <222> (1)..(873)
<400> 134
   atg gag gtc gtg gag aga ttc tac ggt gag ttg gat ggg aag gtc tcg        48
   Met Glu Val Val Glu Arg Phe Tyr Gly Glu Leu Asp Gly Lys Val Ser
   1               5                   10                  15
   cag ggc gtg aat gca ttg ctg ggt agt ttt ggg gtg gag ttg acg gat        96
   Gln Gly Val Asn Ala Leu Leu Gly Ser Phe Gly Val Glu Leu Thr Asp
               20                  25                  30
   acg ccc act acc aaa ggc ttg ccc ctc gtt gac agt ccc aca ccc atc       144
   Thr Pro Thr Thr Lys Gly Leu Pro Leu Val Asp Ser Pro Thr Pro Ile
           35                  40                  45
   gtc ctc ggt gtt tct gta tac ttg act att gtc att gga ggg ctt ttg       192
   Val Leu Gly Val Ser Val Tyr Leu Thr Ile Val Ile Gly Gly Leu Leu
       50                  55                  60
   tgg ata aag gcc agg gat ctg aaa ccg cgc gcc tcg gag cca ttt ttg       240
   Trp Ile Lys Ala Arg Asp Leu Lys Pro Arg Ala Ser Glu Pro Phe Leu
   65                  70                  75                  80
   ctc caa gct ttg gtg ctt gtg cac aac ctg ttc tgt ttt gcg ctc agt       288
   Leu Gln Ala Leu Val Leu Val His Asn Leu Phe Cys Phe Ala Leu Ser
                   85                  90                  95
   ctg tat atg tgc gtg ggc atc gct tat cag gct att acc tgg cgg tac       336
   Leu Tyr Met Cys Val Gly Ile Ala Tyr Gln Ala Ile Thr Trp Arg Tyr
               100                 105                 110
   tct ctc tgg ggc aat gca tac aat cct aaa cat aaa gag atg gcg att       384
   Ser Leu Trp Gly Asn Ala Tyr Asn Pro Lys His Lys Glu Met Ala Ile
           115                 120                 125
   ctg gta tac ttg ttc tac atg tct aag tac gtg gaa ttc atg gat acc       432
   Leu Val Tyr Leu Phe Tyr Met Ser Lys Tyr Val Glu Phe Met Asp Thr
       130                 135                 140
   gtt atc atg ata ctg aag cgc agc acc agg caa ata agc ttc ctc cac       480
   Val Ile Met Ile Leu Lys Arg Ser Thr Arg Gln Ile Ser Phe Leu His
   145                 150                 155                 160
   gtt tat cat cat tct tca att tcc ctc att tgg tgg gct att gct cat       528
   Val Tyr His His Ser Ser Ile Ser Leu Ile Trp Trp Ala Ile Ala His
                   165                 170                 175
   cac gct cct ggc ggt gaa gca tat tgg tct gcg gct ctg aac tca gga       576
   His Ala Pro Gly Gly Glu Ala Tyr Trp Ser Ala Ala Leu Asn Ser Gly
               180                 185                 190
   gtg cat gtt ctc atg tat gcg tat tac ttc ttg gct gcc tgc ctt cga       624
   Val His Val Leu Met Tyr Ala Tyr Tyr Phe Leu Ala Ala Cys Leu Arg
           195                 200                 205
   agt agc cca aag tta aaa aat aag tac ctt ttt tgg ggc agg tac ttg       672
   Ser Ser Pro Lys Leu Lys Asn Lys Tyr Leu Phe Trp Gly Arg Tyr Leu
       210                 215                 220
   aca caa ttc caa atg ttc cag ttt atg ctg aac tta gtg cag gct tac       720
   Thr Gln Phe Gln Met Phe Gln Phe Met Leu Asn Leu Val Gln Ala Tyr
   225                 230                 235                 240
   tac gac atg aaa acg aat gcg cca tat cca caa tgg ctg atc aag att       768
   Tyr Asp Met Lys Thr Asn Ala Pro Tyr Pro Gln Trp Leu Ile Lys Ile
                   245                 250                 255
   ttg ttc tac tac atg atc tcg ttg ctg ttt ctt ttc ggc aat ttt tac       816
   Leu Phe Tyr Tyr Met Ile Ser Leu Leu Phe Leu Phe Gly Asn Phe Tyr
               260                 265                 270
   gta caa aaa tac atc aaa ccc tct gac gga aag caa aag gga gct aaa       864
   Val Gln Lys Tyr Ile Lys Pro Ser Asp Gly Lys Gln Lys Gly Ala Lys
           275                 280                 285
   act gag tga                                                           873
   Thr Glu
       290
<210> 135
   <211> 290
   <212> PRT
   <213> Physcomitrella patens
<400> 135
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145                 150                 155                 160
   Val Tyr His His Ser Ser Ile Ser Leu Ile Trp Trp Ala Ile Ala His
                   165                 170                 175
   His Ala Pro Gly Gly Glu Ala Tyr Trp Ser Ala Ala Leu Asn Ser Gly
               180                 185                 190
   Val His Val Leu Met Tyr Ala Tyr Tyr Phe Leu Ala Ala Cys Leu Arg
           195                 200                 205
   Ser Ser Pro Lys Leu Lys Asn Lys Tyr Leu Phe Trp Gly Arg Tyr Leu
       210                 215                 220
   Thr Gln Phe Gln Met Phe Gln Phe Met Leu Asn Leu Val Gln Ala Tyr
   225                 230                 235                 240
   Tyr Asp Met Lys Thr Asn Ala Pro Tyr Pro Gln Trp Leu Ile Lys Ile
                   245                 250                 255
   Leu Phe Tyr Tyr Met Ile Ser Leu Leu Phe Leu Phe Gly Asn Phe Tyr
               260                 265                 270
   Val Gln Lys Tyr Ile Lys Pro Ser Asp Gly Lys Gln Lys Gly Ala Lys
           275                 280                 285
   Thr Glu
       290
<210> 136
   <211> 957
   <212> DNA
   <213> Mortierella alpina
<220>
   <221> CDS
   <222> (1)..(957)
<400> 136
   atg gag tcg att gcg cca ttc ctc cca tca aag atg ccg caa gat ctg      48
   Met Glu Ser Ile Ala Pro Phe Leu Pro Ser Lys Met Pro Gln Asp Leu
   1               5                   10                  15
   ttt atg gac ctt gcc acc gct atc ggt gtc cgg gcc gcg ccc tat gtc      96
   Phe Met Asp Leu Ala Thr Ala Ile Gly Val Arg Ala Ala Pro Tyr Val
               20                  25                  30
   gat cct ctc gag gcc gcg ctg gtg gcc cag gcc gag aag tac atc ccc     144
   Asp Pro Leu Glu Ala Ala Leu Val Ala Gln Ala Glu Lys Tyr Ile Pro
           35                  40                  45
   acg att gtc cat cac acg cgt ggg ttc ctg gtc gcg gtg gag tcg cct     192
   Thr Ile Val His His Thr Arg Gly Phe Leu Val Ala Val Glu Ser Pro
       50                  55                  60
   ttg gcc cgt gag ctg ccg ttg atg aac ccg ttc cac gtg ctg ttg atc     240
   Leu Ala Arg Glu Leu Pro Leu Met Asn Pro Phe His Val Leu Leu Ile
   65                  70                  75                  80
   gtg ctc gct tat ttg gtc acg gtc ttt gtg ggc atg cag atc atg aag     288
   Val Leu Ala Tyr Leu Val Thr Val Phe Val Gly Met Gln Ile Met Lys
                   85                  90                  95
   aac ttt gag cgg ttc gag gtc aag acg ttt tcg ctc ctg cac aac ttt     336
   Asn Phe Glu Arg Phe Glu Val Lys Thr Phe Ser Leu Leu His Asn Phe
               100                 105                 110
   tgt ctg gtc tcg atc agc gcc tac atg tgc ggt ggg atc ctg tac gag     384
   Cys Leu Val Ser Ile Ser Ala Tyr Met Cys Gly Gly Ile Leu Tyr Glu
           115                 120                 125
   gct tat cag gcc aac tat gga ctg ttt gag aac gct gct gat cat acc     432
   Ala Tyr Gln Ala Asn Tyr Gly Leu Phe Glu Asn Ala Ala Asp His Thr
       130                 135                 140
   ttc aag ggt ctt cct atg gcc aag atg atc tgg ctc ttc tac ttc tcc     480
   Phe Lys Gly Leu Pro Met Ala Lys Met Ile Trp Leu Phe Tyr Phe Ser
   145                 150                 155                 160
   aag atc atg gag ttt gtc gac acc atg atc atg gtc ctc aag aag aac     528
   Lys Ile Met Glu Phe Val Asp Thr Met Ile Met Val Leu Lys Lys Asn
                   165                 170                 175
   aac cgc cag atc tcc ttc ttg cac gtt tac cac cac agc tcc atc ttc     576
   Asn Arg Gln Ile Ser Phe Leu His Val Tyr His His Ser Ser Ile Phe
               180                 185                 190
   acc atc tgg tgg ttg gtc acc ttt gtt gca ccc aac ggt gaa gcc tac     624
   Thr Ile Trp Trp Leu Val Thr Phe Val Ala Pro Asn Gly Glu Ala Tyr
           195                 200                 205
   ttc tct gct gcg ttg aac tcg ttc atc cat gtg atc atg tac ggc tac     672
   Phe Ser Ala Ala Leu Asn Ser Phe Ile His Val Ile Met Tyr Gly Tyr
       210                 215                 220
   tac ttc ttg tcg gcc ttg ggc ttc aag cag gtg tcg ttc atc aag ttc     720
   Tyr Phe Leu Ser Ala Leu Gly Phe Lys Gln Val Ser Phe Ile Lys Phe
   225                 230                 235                 240
   tac atc acg cgc tcg cag atg aca cag ttc tgc atg atg tcg gtc cag     768
   Tyr Ile Thr Arg Ser Gln Met Thr Gln Phe Cys Met Met Ser Val Gln
                   245                 250                 255
   tct tcc tgg gac atg tac gcc atg aag gtc ctt ggc cgc ccc gga tac     816
   Ser Ser Trp Asp Met Tyr Ala Met Lys Val Leu Gly Arg Pro Gly Tyr
               260                 265                 270
   ccc ttc ttc atc acg gct ctg ctt tgg ttc tac atg tgg acc atg ctc     864
   Pro Phe Phe Ile Thr Ala Leu Leu Trp Phe Tyr Met Trp Thr Met Leu
           275                 280                 285
   ggt ctc ttc tac aac ttt tac aga aag aac gcc aag ttg gcc aag cag     912
   Gly Leu Phe Tyr Asn Phe Tyr Arg Lys Asn Ala Lys Leu Ala Lys Gln
       290                 295                 300
   gcc aag gcc gac gct gcc aag gag aag gca agg aag ttg cag taa         957
   Ala Lys Ala Asp Ala Ala Lys Glu Lys Ala Arg Lys Leu Gln
   305                 310                 315
<210> 137
   <211> 318
   <212> PRT
   <213> Mortierella alpina
<400> 137
   Met Glu Ser Ile Ala Pro Phe Leu Pro Ser Lys Met Pro Gln Asp Leu
   1               5                   10                  15
   Phe Met Asp Leu Ala Thr Ala Ile Gly Val Arg Ala Ala Pro Tyr Val
               20                  25                  30
   Asp Pro Leu Glu Ala Ala Leu Val Ala Gln Ala Glu Lys Tyr Ile Pro
           35                  40                  45
   Thr Ile Val His His Thr Arg Gly Phe Leu Val Ala Val Glu Ser Pro
       50                  55                  60
   Leu Ala Arg Glu Leu Pro Leu Met Asn Pro Phe His Val Leu Leu Ile
   65                  70                  75                  80
   Val Leu Ala Tyr Leu Val Thr Val Phe Val Gly Met Gln Ile Met Lys
                   85                  90                  95
   Asn Phe Glu Arg Phe Glu Val Lys Thr Phe Ser Leu Leu His Asn Phe
               100                 105                 110
   Cys Leu Val Ser Ile Ser Ala Tyr Met Cys Gly Gly Ile Leu Tyr Glu
           115                 120                 125
   Ala Tyr Gln Ala Asn Tyr Gly Leu Phe Glu Asn Ala Ala Asp His Thr
       130                 135                 140
   Phe Lys Gly Leu Pro Met Ala Lys Met Ile Trp Leu Phe Tyr Phe Ser
   145                 150                 155                 160
   Lys Ile Met Glu Phe Val Asp Thr Met Ile Met Val Leu Lys Lys Asn
                   165                 170                 175
   Asn Arg Gln Ile Ser Phe Leu His Val Tyr His His Ser Ser Ile Phe
               180                 185                 190
   Thr Ile Trp Trp Leu Val Thr Phe Val Ala Pro Asn Gly Glu Ala Tyr
           195                 200                 205
   Phe Ser Ala Ala Leu Asn Ser Phe Ile His Val Ile Met Tyr Gly Tyr
       210                 215                 220
   Tyr Phe Leu Ser Ala Leu Gly Phe Lys Gln Val Ser Phe Ile Lys Phe
   225                 230                 235                 240
   Tyr Ile Thr Arg Ser Gln Met Thr Gln Phe Cys Met Met Ser Val Gln
                   245                 250                 255
   Ser Ser Trp Asp Met Tyr Ala Met Lys Val Leu Gly Arg Pro Gly Tyr
               260                 265                 270
   Pro Phe Phe Ile Thr Ala Leu Leu Trp Phe Tyr Met Trp Thr Met Leu
           275                 280                 285
   Gly Leu Phe Tyr Asn Phe Tyr Arg Lys Asn Ala Lys Leu Ala Lys Gln
       290                 295                 300
   Ala Lys Ala Asp Ala Ala Lys Glu Lys Ala Arg Lys Leu Gln
   305                 310                 315
<210> 138
   <211> 242
   <212> PRT
   <213> Artificial sequence
<220>
   <221> variant
   <222> (3)..(5)
   <223> xaa in position 3 to 5 is any amino acid
<220>
   <221> Variant
   <222> (6)..(6)
   <223> xaa in position 6 is any or no amino acid
<220>
   <221> Variant
   <222> (8)..(16)
   <223> xaa in position 8 to 16 is any amino acid
<220>
   <221> Variant
   <222> (19)..(20)
   <223> xaa in position 19 to 20 is any amino acid
<220>
   <221> Variant
   <222> (22)..(42)
   <223> xaa in position 22 to 42 is any amino acid
<220>
   <221> Variant
   <222> (43)..(49)
   <223> xaa in position 43 to 49 is any or no amino acid
<220>
   <221> Variant
   <222> (52)..(56)
   <223> xaa in position 52 to 56 is any amino acid
<220>
   <221> Variant
   <222> (59)..(59)
   <223> xaa in position 59 is any amino acid
<220>
   <221> Variant
   <222> (62)..(63)
   <223> xaa in position 62 to 63 is any amino acid
<220>
   <221> Variant
   <222> (65)..(68)
   <223> xaa in position 65 to 68 is any amino acid
<220>
   <221> Variant
   <222> (70)..(73)
   <223> xaa in position 70 to 73 is any amino acid
<220>
   <221> variant
   <222> (75)..(75)
   <223> xaa in position 75 is any amino acid
<220>
   <221> Variant
   <222> (77)..(78)
   <223> xaa in position 77 to 78 is any amino acid
<220>
   <221> Variant
   <222> (80)..(94)
   <223> xaa in position 80 to 94 is any amino acid
<220>
   <221> Variant
   <222> (95)..(98)
   <223> xaa in position 95 to 98 is any or no amino acid
<220>
   <221> variant
   <222> (101)..(101)
   <223> xaa in position 101 is any amino acid
<220>
   <221> Variant
   <222> (104)..(105)
   <223> xaa in position 104 to 105 is any amino acid
<220>
   <221> Variant
   <222> (108)..(108)
   <223> xaa in position 108 is any amino acid
<220>
   <221> Variant
   <222> (111)..(111)
   <223> xaa in position 111 is any amino acid
<220>
   <221> Variant
   <222> (114)..(114)
   <223> xaa in position 114 is any amino acid
<220>
   <221> Variant
   <222> (116)..(120)
   <223> xaa in position 116 to 120 is any amino acid
<220>
   <221> Variant
   <222> (122)..(124)
   <223> xaa in position 122 to 124 is any amino acid
<220>
   <221> Variant
   <222> (127)..(127)
   <223> xaa in position 127 is any amino acid
<220>
   <221> Variant
   <222> (131)..(132)
   <223> xaa in position 131 to 132 is any amino acid
<220>
   <221> Variant
   <222> (134)..(135)
   <223> xaa in position 134 to 135 is any amino acid
<220>
   <221> variant
   <222> (139)..(143)
   <223> xaa in position 139 to 143 is any amino acid
<220>
   <221> Variant
   <222> (148)..(149)
   <223> xaa in position 148 to 149 is any amino acid
<220>
   <221> Variant
   <222> (151)..(151)
   <223> xaa in position 151 is any amino acid
<220>
   <221> variant
   <222> (154)..(155)
   <223> xaa in position 154 to 155 is any amino acid
<220>
   <221> Variant
   <222> (158)..(159)
   <223> xaa in position 158 to 159 is any amino acid
<220>
   <221> Variant
   <222> (162)..(162)
   <223> xaa in position 162 is any amino acid
<220>
   <221> variant
   <222> (165)..(165)
   <223> xaa in position 165 is any amino acid
<220>
   <221> Variant
   <222> (168)..(168)
   <223> xaa in position 168 is any amino acid
<220>
   <221> Variant
   <222> (170)..(184)
   <223> xaa in position 170 to 184 is any amino acid
<220>
   <221> Variant
   <222> (185)..(190)
   <223> xaa in position 185 to 190 is any or no amino acid
<220>
   <221> Variant
   <222> (192)..(193)
   <223> xaa in position 192 to 193 is any amino acid
<220>
   <221> variant
   <222> (196)..(196)
   <223> xaa in position 196 is any amino acid
<220>
   <221> Variant
   <222> (199)..(203)
   <223> xaa in position 199 to 203 is any amino acid

<220>
   <221> Variant
   <222> (205)..(206)
   <223> xaa in position 205 to 206 is any amino acid
<220>
   <221> Variant
   <222> (208)..(213)
   <223> xaa in position 208 to 213 is any amino acid
<220>
   <221> Variant
   <222> (214)..(219)
   <223> xaa in position 214 to 219 is any or no amino acid
<220>
   <221> Variant
   <222> (222)..(227)
   <223> xaa in position 222 to 227 is any amino acid
<220>
   <221> Variant
   <222> (229)..(230)
   <223> xaa in position 229 to 230 is any amino acid
<220>
   <221> Variant
   <222> (233)..(235)
   <223> xaa in position 233 to 235 is any amino acid
<220>
   <221> Variant
   <222> (237)..(237)
   <223> xaa in position 237 is any amino acid
<220>
   <221> Variant
   <222> (241)..(241)
   <223> xaa in position 241 is any amino acid
<400> 138
   Leu Pro Xaa Xaa Xaa Xaa Pro Xaa Xaa Xaa Xaa Xaa Xaa Xaa Xaa Xaa
   1               5                   10                  15
   Tyr Leu Xaa Xaa Val Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa Xaa Xaa Xaa Xaa Xaa
           35                  40                  45
   Xaa His Asn Xaa Xaa Xaa Xaa Xaa Leu Ser Xaa Tyr Met Xaa Xaa Gly
       50                  55                  60
   Xaa Xaa Xaa Xaa Ala Xaa Xaa Xaa Xaa Tyr Xaa Leu Xaa Xaa Asn Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Phe Tyr Xaa Ser Lys Xaa Xaa Glu Phe Xaa Asp Thr Xaa Ile
               100                 105                 110
   Met Xaa Leu Xaa Xaa Xaa Xaa Xaa Gln Xaa Xaa Xaa Leu His Xaa Tyr
           115                 120                 125
   His His Xaa Xaa Ile Xaa Xaa Ile Trp Trp Xaa Xaa Xaa Xaa Xaa Ala
       130                 135                 140
   Pro Gly Gly Xaa Xaa Tyr Xaa Ser Ala Xaa Xaa Asn Ser Xaa Xaa His
   145                 150                 155                 160
   Val Xaa Met Tyr Xaa Tyr Tyr Xaa Leu Xaa Xaa Xaa Xaa Xaa Xaa Xaa
                   165                 170                 175
   Xaa Xaa Xaa Xaa Xaa Xaa Xaa Xaa Xaa Xaa Xaa Xaa Xaa Xaa Thr Xaa
               180                 185                 190
   Xaa Gln Met Xaa Gln Phe Xaa Xaa Xaa Xaa Xaa Gln Xaa Xaa Tyr Xaa
           195                 200                 205
   Xaa Xaa Xaa Xaa Xaa Xaa Xaa Xaa Xaa Xaa Xaa Tyr Pro Xaa Xaa Xaa
       210                 215                 220
   Xaa Xaa Xaa Leu Xaa Xaa Tyr Met Xaa Xaa Xaa Leu Xaa Leu Phe Gly
   225                 230                 235                 240
   Xaa Phe
<210> 139
   <211> 60
   <212> PRT
   <213> Artificial sequence
<220>
   <221> Variant
   <222> (2)..(2)
   <223> xaa in position 2 is Phe or Trp
<220>
   <221> Variant
   <222> (3)..(3)
   <223> xaa in position 3 is Leu, Met or Val
<220>
   <221> Variant
   <222> (6)..(6)
   <223> xaa in position 6 is any amino acid
<220>
   <221> Variant
   <222> (7)..(7)
   <223> xaa in position 7 is Phe or Ile
<220>
   <221> Variant
   <222> (9)..(9)
   <223> xaa in position 9 is any or no amino acid
<220>
   <221> Variant
   <222> (11)..(11)
   <223> xaa in position 11 is any or no amino acid
<220>
   <221> Variant
   <222> (13)..(15)
   <223> xaa in position 13 to 15 is any amino acid
<220>
   <221> Variant
   <222> (16)..(16)
   <223> xaa in position 16 is any or no amino acid
<220>
   <221> Variant
   <222> (18)..(18)
   <223> xaa in position 18 is Ile, Leu or Val
<220>
   <221> Variant
   <222> (19)..(19)
   <223> xaa in position 19 is Ser or Thr
<220>
   <221> Variant
   <222> (20)..(20)
   <223> xaa in position 20 is Phe or Val
<220>
   <221> Variant
   <222> (23)..(23)
   <223> xaa in position 23 is Ile or Val
<220>
   <221> Variant
   <222> (27)..(27)
   <223> xaa in position 27 is Ala, Ser or Thr
<220>
   <221> Variant
   <222> (28)..(28)
   <223> xaa in position 28 is Ser or Thr
<220>
   <221> Variant
   <222> (29)..(29)
   <223> xaa in position 29 is Ile or Val
<220>
   <221> Variant
   <222> (30)..(31)
   <223> xaa in position 30 to 31 is any amino acid
<220>
   <221> variant
   <222> (32)..(32)
   <223> xaa in position 32 is Ile or Leu
<220>
   <221> Variant
   <222> (34)..(34)
   <223> xaa in position 34 is any amino acid
<220>
   <221> Variant
   <222> (35)..(35)
   <223> xaa in position 35 is Ala, Ile or Leu
<220>
   <221> Variant
   <222> (36)..(36)
   <223> xaa in position 36 is Ala, Ile or val
<220>
   <221> Variant
   <222> (37)..(39)
   <223> xaa in position 37 to 39 is any amino acid
<220>
   <221> Variant
   <222> (42)..(42)
   <223> xaa in position 42 is Gly or Asn
<220>
   <221> Variant
   <222> (44)..(44)
   <223> xaa in position 44 is Asp or Glu
<220>
   <221> Variant
   <222> (45)..(45)
   <223> xaa in position 45 is Ala or Ser
<220>
   <221> variant
   <222> (47)..(47)
   <223> xaa in position 47 is Phe, Trp or Tyr
<220>
   <221> Variant
   <222> (49)..(49)
   <223> xaa in position 49 is Ala or Thr
<220>
   <221> variant
   <222> (50)..(50)
   <223> xaa in position 50 is any amino acid
<220>
   <221> Variant
   <222> (51)..(51)
   <223> xaa in position 51 is Leu or val
<220>
   <221> Variant
   <222> (54)..(54)
   <223> xaa in position 54 is any amino acid
<220>
   <221> Variant
   <222> (55)..(55)
   <223> xaa in position 55 is Ile or val
<220>
   <221> Variant
   <222> (58)..(58)
   <223> xaa in position 58 is any amino acid
<220>
   <221> variant
   <222> (59)..(59)
   <223> xaa in position 59 is Leu or Met
<400> 139
<210> 140
   <211> 22
   <212> PRT
   <213> Artificial sequence
<220>
   <221> Variant
   <222> (2)..(2)
   <223> xaa in position 2 is Ala or Leu
<220>
   <221> Variant
   <222> (3)..(3)
   <223> xaa in position 3 is Phe or Tyr
<220>
   <221> Variant
   <222> (5)..(5)
   <223> xaa in position 5 is Cys or ser
<220>
   <221> Variant
   <222> (6)..(6)
   <223> xaa in position 6 is Gly, Leu or val
<220>
   <221> Variant
   <222> (8)..(9)
   <223> xaa in position 8 to 9 is any amino acid
<220>
   <221> Variant
   <222> (10)..(11)
   <223> xaa in position 10 to 11 is any or no amino acid
<220>
   <221> variant
   <222> (13)..(14)
   <223> xaa in position 13 to 14 is any amino acid
<220>
   <221> Variant
   <222> (15)..(16)
   <223> xaa in position 15 to 16 is any or no amino acid
<220>
   <221> Variant
   <222> (18)..(18)
   <223> xaa in position 18 is Gly, Ser or Thr
<220>
   <221> Variant
   <222> (19)..(19)
   <223> xaa in position 19 is Leu or val
<220>
   <221> variant
   <222> (20)..(20)
   <223> xaa in position 20 is Phe or Trp
<220>
   <221> Variant
   <222> (21)..(21)
   <223> xaa in position 21 is Glu, Gly or Asn
<400> 140
   Ser xaa xaa Met xaa xaa Gly xaa xaa xaa xaa Ala xaa xaa xaa xaa
   1               5                   10                  15
   Tyr xaa xaa xaa xaa Asn
                                       20
<210> 141
   <211> 18
   <212> PRT
   <213> Artificial sequence
<220>
   <221> Variant
   <222> (2)..(3)
   <223> xaa in position 2 to 3 is any amino acid
<220>
   <221> Variant
   <222> (6)..(6)
   <223> xaa in position 6 is any amino acid
<220>
   <221> Variant
   <222> (9)..(12)
   <223> xaa in position 9 to 12 is any amino acid
<220>
   <221> Variant
   <222> (13)..(13)
   <223> xaa in position 13 is Gly, Ile, Leu or val
<220>
   <221> Variant
   <222> (15)..(15)
   <223> xaa in position 15 is Ala or Ser
<220>
   <221> Variant
   <222> (16)..(16)
   <223> xaa in position 16 is any amino acid
<220>
   <221> Variant
   <222> (17)..(17)
   <223> xaa in position 17 is Trp or Tyr
<220>
   <221> Variant
   <222> (18)..(18)
   <223> xaa in position 18 is Ala, Cys or Asp
<400> 141
<210> 142
   <211> 25
   <212> PRT
   <213> Artificial sequence
<220>
   <221> Variant
   <222> (2)..(2)
   <223> xaa in position 2 is any or no amino acid
<220>
   <221> Variant
   <222> (4)..(5)
   <223> xaa in position 4 to 5 is any amino acid
<220>
   <221> Variant
   <222> (6)..(6)
   <223> xaa in position 6 is Phe, Ile or Leu
<220>
   <221> Variant
   <222> (7)..(8)
   <223> xaa in position 7 to 8 is any amino acid
<220>
   <221> Variant
   <222> (9)..(9)
   <223> xaa in position 9 is Ile, Leu or Met
<220>
   <221> Variant
   <222> (11)..(12)
   <223> xaa in position 11 to 12 is any amino acid
<220>
   <221> Variant
   <222> (14)..(15)
   <223> xaa in position 14 to 15 is any amino acid
<220>
   <221> Variant
   <222> (16)..(16)
   <223> xaa in position 16 is Ser or Thr
<220>
   <221> Variant
   <222> (17)..(17)
   <223> xaa in position 17 is Leu or Met
<220>
   <221> Variant
   <222> (19)..(19)
   <223> xaa in position 19 is any amino acid
<220>
   <221> Variant
   <222> (20)..(20)
   <223> xaa in position 20 is Phe or Leu
<220>
   <221> variant
   <222> (22)..(23)
   <223> xaa in position 22 to 23 is any amino acid
<220>
   <221> Variant
   <222> (25)..(25)
   <223> xaa in position 25 is Phe or Tyr
<400> 142
<210> 143
   <211> 879
   <212> DNA
   <213> Ostreococcus tauri
<220>
   <221> CDS
   <222> (1)..(879)
<400> 143
   atg agt ggc tta cgt gca ccc aac ttt tta cac aga ttc tgg aca aag        48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt        96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc       144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc       192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa       240
   Thr Val Phe Leu Trp Tyr Gly Arg Leu Thr Arg Ser Ser Asp Lys Lys
   65                  70                       75                       80
   att aga gag cct acg tgg tta aga aga ttc ata ata tgt cat aat gcg       288
   Ile Arg Glu Pro Thr Trp Leu Arg Arg Phe Ile Ile Cys His Asn Ala
                   85                       90                       95
   ttc ttg ata gtc ctc agt ctt tac atg tgc ctt ggt tgt gtg gcc caa       336
   Phe Leu Ile Val Leu Ser Leu Tyr Met Cys Leu Gly Cys Val Ala Gln
               100                      105                      110
   gcg tat cag aat gga tat act tta tgg ggt aat gaa ttc aag gcc acg       384
   Ala Tyr Gln Asn Gly Tyr Thr Leu Trp Gly Asn Glu Phe Lys Ala Thr
           115                      120                      125
   gaa act cag ctt gct ctc tac att tac att ttt tac gta agt aaa ata       432
   Glu Thr Gln Leu Ala Leu Tyr Ile Tyr Ile Phe Tyr Val Ser Lys Ile
       130                      135                      140
   tac gag ttt gta gat act tac att atg ctt ctc aag aat aac ttg cgg       480
   Tyr Glu Phe Val Asp Thr Tyr Ile Met Leu Leu Lys Asn Asn Leu Arg
   145                     150                      155                      160
   caa gta agt ttc cta cac att tat cac cac agc acg att tcc ttt att       528
   Gln Val Ser Phe Leu His Ile Tyr His His Ser Thr Ile Ser Phe Ile
                   165                      170                      175
   tgg tgg atc att gct cgg agg gct ccg ggt ggt gat gct tac ttc agc       576
   Trp Trp Ile Ile Ala Arg Arg Ala Pro Gly Gly Asp Ala Tyr Phe Ser
               180                      185                      190
   gcg gcc ttg aac tca tgg gta cac gtg tgc atg tac acc tat tat cta       624
   Ala Ala Leu Asn Ser Trp Val His Val Cys Met Tyr Thr Tyr Tyr Leu
           195                      200                      205
   tta tca acc ctt att gga aaa gaa gat cct aag cgt tcc aac tac ctt       672
   Leu Ser Thr Leu Ile Gly Lys Glu Asp Pro Lys Arg Ser Asn Tyr Leu
       210                      215                      220
   tgg tgg ggt cgc cac cta acg caa atg cag atg ctt cag ttt ttc ttc       720
   Trp Trp Gly Arg His Leu Thr Gln Met Gln Met Leu Gln Phe Phe Phe
   225                     230                      235                      240
   aac gta ctt caa gcg ttg tac tgc gct tcg ttc tct acg tat ccc aag       768
   Asn Val Leu Gln Ala Leu Tyr Cys Ala Ser Phe Ser Thr Tyr Pro Lys
                   245                      250                      255
   ttt ttg tcc aaa att ctg ctc gtc tat atg atg agc ctt ctc ggc ttg       816
   Phe Leu Ser Lys Ile Leu Leu Val Tyr Met Met Ser Leu Leu Gly Leu
               260                      265                      270
   ttt ggg cat ttc tac tat tcc aag cac ata gca gca gct aag ctc cag       864
   Phe Gly His Phe Tyr Tyr Ser Lys His Ile Ala Ala Ala Lys Leu Gln
           275                      280                      285
   aaa aaa cag cag tga                                                   879
   Lys Lys Gln Gln
       290
<210> 144
   <211> 292
   <212> PRT
   <213> Ostreococcus tauri
<400> 144
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Ser Phe Leu His Ile Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
           275                 280                 285
   Lys Lys Gln Gln
       290
<210> 145
   <211> 831
   <212> DNA
   <213> Thraustochytrium sp
<220>
   <221> CDS
   <222> (1)..(831)
<400> 145
   atg gat gtc gtc gag cag caa tgg cgc cgc ttc gtg gac gcc gtg gac      48
   Met Asp Val Val Glu Gln Gln Trp Arg Arg Phe Val Asp Ala Val Asp
   1               5                   10                  15
   aac gga atc gtg gag ttc atg gag cat gag gag ccc aac aag ctg aac      96
   Asn Gly Ile Val Glu Phe Met Glu His Glu Glu Pro Asn Lys Leu Asn
               20                  25                  30
   gag ggc aag ctc tcc acc tcg acc gag gag atg atg gcg ctt atc gtc      144
   Glu Gly Lys Leu Ser Thr Ser Thr Glu Glu Met Met Ala Leu Ile Val
           35                  40                  45
   ggc tac ctg gcg ttc gtg gtc ctc ggg tcc gcc ttc atg aag gcc ttt      192
   Gly Tyr Leu Ala Phe Val Val Leu Gly Ser Ala Phe Met Lys Ala Phe
       50                  55                  60
   gtc gat aag cct ttc gag ctc aag ttc ctc aag ctc gtg cac aac atc      240
   Val Asp Lys Pro Phe Glu Leu Lys Phe Leu Lys Leu Val His Asn Ile
   65                  70                  75                  80
   ttc ctc acc ggt ctg tcc atg tac atg gcc acc gag tgc gcg cgc cag      288
   Phe Leu Thr Gly Leu Ser Met Tyr Met Ala Thr Glu Cys Ala Arg Gln
                   85                  90                  95
   gca tac ctc ggc ggc tac aag ctc ttt ggc aac ccg atg gag aag ggc      336
   Ala Tyr Leu Gly Gly Tyr Lys Leu Phe Gly Asn Pro Met Glu Lys Gly
               100                 105                 110
   acc gag tcg cac gcc ccg ggc atg gcc aac atc atc tac atc ttc tac      384
   Thr Glu Ser His Ala Pro Gly Met Ala Asn Ile Ile Tyr Ile Phe Tyr
           115                 120                 125
   gtg agc aag ttc ctc gaa ttc ctc gac acc gtc ttc atg atc ctc ggc      432
   Val Ser Lys Phe Leu Glu Phe Leu Asp Thr Val Phe Met Ile Leu Gly
       130                 135                 140
   aag aag tgg aag cag ctc agc ttt ctc cac gtc tac cac cac gcg agc      480
   Lys Lys Trp Lys Gln Leu Ser Phe Leu His Val Tyr His His Ala Ser
   145                 150                 155                 160
   atc agc ttc atc tgg ggc atc atc gcc cgc ttc gcg ccc ggt ggc gac      528
   Ile Ser Phe Ile Trp Gly Ile Ile Ala Arg Phe Ala Pro Gly Gly Asp
                   165                 170                 175
   gcc tac ttc tct acc atc ctc aac agc agc gtg cat gtc gtg ctc tac      576
   Ala Tyr Phe Ser Thr Ile Leu Asn Ser Ser Val His Val Val Leu Tyr
               180                 185                 190
   ggc tac tac gcc tcg acc acc ctc ggc tac acc ttc atg cgc ccg ctg      624
   Gly Tyr Tyr Ala Ser Thr Thr Leu Gly Tyr Thr Phe Met Arg Pro Leu
           195                 200                 205
   cgc ccg tac att acc acc att cag ctc acg cag ttc atg gcc atg gtc      672
   Arg Pro Tyr Ile Thr Thr Ile Gln Leu Thr Gln Phe Met Ala Met Val
       210                 215                 220
   gtc cag tcc gtc tat gac tac tac aac ccc tgc gac tac ccg cag ccc      720
   Val Gln Ser Val Tyr Asp Tyr Tyr Asn Pro Cys Asp Tyr Pro Gln Pro
   225                 230                 235                 240
   ctc gtc aag ctg ctc ttc tgg tac atg ctc acc atg ctc ggc ctc ttc      768
   Leu Val Lys Leu Leu Phe Trp Tyr Met Leu Thr Met Leu Gly Leu Phe
                   245                 250                 255
   ggc aac ttc ttc gtg cag cag tac ctc aag ccc aag gcg ccc aag aag      816
   Gly Asn Phe Phe Val Gln Gln Tyr Leu Lys Pro Lys Ala Pro Lys Lys
               260                 265                 270
   cag aag acc atc taa                                                  831
   Gln Lys Thr Ile
           275
<210> 146
   <211> 276
   <212> PRT
   <213> Thraustochytrium sp
<400> 146
   Met Asp Val Val Glu Gln Gln Trp Arg Arg Phe Val Asp Ala Val Asp
   1               5                   10                  15
   Asn Gly Ile Val Glu Phe Met Glu His Glu Glu Pro Asn Lys Leu Asn
               20                  25                  30
   Glu Gly Lys Leu Ser Thr Ser Thr Glu Glu Met Met Ala Leu Ile Val
           35                  40                  45
   Gly Tyr Leu Ala Phe Val Val Leu Gly Ser Ala Phe Met Lys Ala Phe
       50                  55                  60
   Val Asp Lys Pro Phe Glu Leu Lys Phe Leu Lys Leu Val His Asn Ile
   65                  70                  75                  80
   Phe Leu Thr Gly Leu Ser Met Tyr Met Ala Thr Glu Cys Ala Arg Gln
                   85                  90                  95
   Ala Tyr Leu Gly Gly Tyr Lys Leu Phe Gly Asn Pro Met Glu Lys Gly
               100                 105                 110
   Thr Glu Ser His Ala Pro Gly Met Ala Asn Ile Ile Tyr Ile Phe Tyr
           115                 120                 125
   Val Ser Lys Phe Leu Glu Phe Leu Asp Thr Val Phe Met Ile Leu Gly
       130                 135                 140
   Lys Lys Trp Lys Gln Leu Ser Phe Leu His Val Tyr His His Ala Ser
   145                 150                 155                 160
   Ile Ser Phe Ile Trp Gly Ile Ile Ala Arg Phe Ala Pro Gly Gly Asp
                   165                 170                 175
   Ala Tyr Phe Ser Thr Ile Leu Asn Ser Ser Val His Val Val Leu Tyr
               180                 185                 190
   Gly Tyr Tyr Ala Ser Thr Thr Leu Gly Tyr Thr Phe Met Arg Pro Leu
           195                 200                 205
   Arg Pro Tyr Ile Thr Thr Ile Gln Leu Thr Gln Phe Met Ala Met Val
       210                 215                 220
   Val Gln Ser Val Tyr Asp Tyr Tyr Asn Pro Cys Asp Tyr Pro Gln Pro
   225                 230                 235                 240
   Leu Val Lys Leu Leu Phe Trp Tyr Met Leu Thr Met Leu Gly Leu Phe
                   245                 250                 255
   Gly Asn Phe Phe Val Gln Gln Tyr Leu Lys Pro Lys Ala Pro Lys Lys
               260                 265                 270
   Gln Lys Thr Ile
           275
<210> 147
   <211> 1146
   <212> DNA
   <213> Leishmania major
<220>
   <221> CDS
   <222> (1)..(1146)
<400> 147
   atg gtc tct ctg gag cag gcc gaa cag atc gcg gcc gcg atc gag gta        48
   Met Val Ser Leu Glu Gln Ala Glu Gln Ile Ala Ala Ala Ile Glu Val
   1               5                   10                  15
   cct gac tgg gtc ttg aca aag tct gcg gcg ctg gtg tac agc tgc ttc        96
   Pro Asp Trp Val Leu Thr Lys Ser Ala Ala Leu Val Tyr Ser Cys Phe
               20                  25                  30
   ggc tcc gcc gcc aat gct ttt gaa agc agt atc aag atc aac ttc ccg       144
   Gly Ser Ala Ala Asn Ala Phe Glu Ser Ser Ile Lys Ile Asn Phe Pro
           35                  40                  45
   gcg cag cat gcg ttc gtg gag gcg tgg atg cgc gcg cgc tcc cac cct       192
   Ala Gln His Ala Phe Val Glu Ala Trp Met Arg Ala Arg Ser His Pro
       50                  55                  60
   ttt gcg gag cgc ctg ccg tac ctg aat ccg tgg cac gtt atc gcc tcg       240
   Phe Ala Glu Arg Leu Pro Tyr Leu Asn Pro Trp His Val Ile Ala Ser
   65                  70                  75                  80
   ata ctg gcc tac ctc tcc ttg att gtc acc ttg cgc ctg ttg cat cgt       288
   Ile Leu Ala Tyr Leu Ser Leu Ile Val Thr Leu Arg Leu Leu His Arg
                   85                  90                  95
   gta ctc ggt aag ttc tcg tgc cgc act ctc gga ttg gtg cac aac ctc       336
   Val Leu Gly Lys Phe Ser Cys Arg Thr Leu Gly Leu Val His Asn Leu
               100                 105                 110
   ggt ctc cat ctt ctc tcg ttg tac atg agc ctt ggt ctc atg atc agc       384
   Gly Leu His Leu Leu Ser Leu Tyr Met Ser Leu Gly Leu Met Ile Ser
           115                 120                 125
   gcg cgc gcc gcg ggg tac tcg ctc tgg aac aac gcg gtc ggc acc tcc       432
   Ala Arg Ala Ala Gly Tyr Ser Leu Trp Asn Asn Ala Val Gly Thr Ser
       130                 135                 140
   ccg gct gag tgg cgc att gcg aag ctg atc tgg ctc ttc tat gtc tcg       480
   Pro Ala Glu Trp Arg Ile Ala Lys Leu Ile Trp Leu Phe Tyr Val Ser
   145                 150                 155                 160
   aag gtg gtg gaa tgg gtg gac acg gta att atg tta tta aag cag aac       528
   Lys Val Val Glu Trp Val Asp Thr Val Ile Met Leu Leu Lys Gln Asn
                   165                 170                 175
   tac cac cag gtc acc ttc ctg cac gtg tat cac cac acg acg gtt ttt       576
   Tyr His Gln Val Thr Phe Leu His Val Tyr His His Thr Thr Val Phe
               180                 185                 190
   gtg ctg tgg tgg ctg gcg ttg ctg gtc gct cct ggc ggc gag tcg tac       624
   Val Leu Trp Trp Leu Ala Leu Leu Val Ala Pro Gly Gly Glu Ser Tyr
           195                 200                 205
   tac agc gcc atg gtg aac tct ggc gtc cac gtt ttc atg tac ggg tac       672
   Tyr Ser Ala Met Val Asn Ser Gly Val His Val Phe Met Tyr Gly Tyr
       210                 215                 220
   tac ttt ctc acg ctg ctc ttc cca tcc ggc atc gtg cgc gac gtc ttg       720
   Tyr Phe Leu Thr Leu Leu Phe Pro Ser Gly Ile Val Arg Asp Val Leu
   225                 230                 235                 240
   agc aag ttc aag ttt gcc att acg aag ggc cag atg tgg cag ttc gtc       768
   Ser Lys Phe Lys Phe Ala Ile Thr Lys Gly Gln Met Trp Gln Phe Val
                   245                 250                 255
   ttc aac tgc cta cag tcc gcg tac gac ctc gtg tgg gtg ccg cgg gaa       816
   Phe Asn Cys Leu Gln Ser Ala Tyr Asp Leu Val Trp Val Pro Arg Glu
               260                 265                 270
   gag ctc aag tac agc gcg gag ctg ctg cag atc ctc ttc tgg tac atg       864
   Glu Leu Lys Tyr Ser Ala Glu Leu Leu Gln Ile Leu Phe Trp Tyr Met
           275                 280                 285
   atc tcc ctc ttg gcg ctc ttt ggc aac ttc ttg gtg aag aac aag aag       912
   Ile Ser Leu Leu Ala Leu Phe Gly Asn Phe Leu Val Lys Asn Lys Lys
       290                 295                 300
   ttc tcg cac cgc cgc tgc gtt gat gcc gcg act gct tcg ggc gcg aag       960
   Phe Ser His Arg Arg Cys Val Asp Ala Ala Thr Ala Ser Gly Ala Lys
   305                 310                 315                 320
   gag gac acg gcg gcg agg tcc cac ggc gac cgc acc cac aga acc cgt      1008
   Glu Asp Thr Ala Ala Arg Ser His Gly Asp Arg Thr His Arg Thr Arg
                   325                 330                 335
   gtg aag gct ggc atg acc aac atg caa ctg gag agg ctg aag aat gag      1056
   Val Lys Ala Gly Met Thr Asn Met Gln Leu Glu Arg Leu Lys Asn Glu
               340                     345                     350
   aag tcc acg gag atg aag ctg ctg atg cgc aag aac ggc aac ggc aac        1104
   Lys Ser Thr Glu Met Lys Leu Leu Met Arg Lys Asn Gly Asn Gly Asn
           355                     360                     365
   gga caa aaa gcg tcg ctc cag gcc atg gca ggc agt cga tga                1146
   Gly Gln Lys Ala Ser Leu Gln Ala Met Ala Gly Ser Arg
       370                     375                     380
<210> 148
   <211> 381
   <212> PRT
   <213> Leishmania major
<400> 148
   Met Val Ser Leu Glu Gln Ala Glu Gln Ile Ala Ala Ala Ile Glu Val
   1               5                   10                  15
   Pro Asp Trp Val Leu Thr Lys Ser Ala Ala Leu Val Tyr Ser Cys Phe
               20                  25                  30
   Gly Ser Ala Ala Asn Ala Phe Glu Ser Ser Ile Lys Ile Asn Phe Pro
           35                  40                  45
   Ala Gln His Ala Phe Val Glu Ala Trp Met Arg Ala Arg Ser His Pro
       50                  55                  60
   Phe Ala Glu Arg Leu Pro Tyr Leu Asn Pro Trp His Val Ile Ala Ser
   65                  70                  75                  80
   Ile Leu Ala Tyr Leu Ser Leu Ile Val Thr Leu Arg Leu Leu His Arg
                   85                  90                  95
   Val Leu Gly Lys Phe Ser Cys Arg Thr Leu Gly Leu Val His Asn Leu
               100                 105                 110
   Gly Leu His Leu Leu Ser Leu Tyr Met Ser Leu Gly Leu Met Ile Ser
           115                 120                 125
   Ala Arg Ala Ala Gly Tyr Ser Leu Trp Asn Asn Ala Val Gly Thr Ser
       130                 135                 140
   Pro Ala Glu Trp Arg Ile Ala Lys Leu Ile Trp Leu Phe Tyr Val Ser
   145                 150                 155                 160
   Lys Val Val Glu Trp Val Asp Thr Val Ile Met Leu Leu Lys Gln Asn
                   165                 170                 175
   Tyr His Gln Val Thr Phe Leu His Val Tyr His His Thr Thr Val Phe
               180                 185                 190
   Val Leu Trp Trp Leu Ala Leu Leu Val Ala Pro Gly Gly Glu Ser Tyr
           195                 200                 205
   Tyr Ser Ala Met Val Asn Ser Gly Val His Val Phe Met Tyr Gly Tyr
       210                 215                 220
   Tyr Phe Leu Thr Leu Leu Phe Pro Ser Gly Ile Val Arg Asp Val Leu
   225                 230                 235                 240
   Ser Lys Phe Lys Phe Ala Ile Thr Lys Gly Gln Met Trp Gln Phe Val
                   245                 250                 255
   Phe Asn Cys Leu Gln Ser Ala Tyr Asp Leu Val Trp Val Pro Arg Glu
               260                 265                 270
   Glu Leu Lys Tyr Ser Ala Glu Leu Leu Gln Ile Leu Phe Trp Tyr Met
           275                 280                 285
   Ile Ser Leu Leu Ala Leu Phe Gly Asn Phe Leu Val Lys Asn Lys Lys
       290                 295                 300
   Phe Ser His Arg Arg Cys Val Asp Ala Ala Thr Ala Ser Gly Ala Lys
   305                 310                 315                 320
   Glu Asp Thr Ala Ala Arg Ser His Gly Asp Arg Thr His Arg Thr Arg
                   325                 330                 335
   Val Lys Ala Gly Met Thr Asn Met Gln Leu Glu Arg Leu Lys Asn Glu
               340                 345                 350
   Lys Ser Thr Glu Met Lys Leu Leu Met Arg Lys Asn Gly Asn Gly Asn
           355                 360                 365
   Gly Gln Lys Ala Ser Leu Gln Ala Met Ala Gly Ser Arg
       370                 375                 380
<210> 149
   <211> 879
   <212> DNA
   <213> Ostreococcus tauri
<220>
   <221> CDS
   <222> (1)..(879)
<400> 149
   atg agt ggc tta cgt gca ccc aac ttt tta cac aga ttc tgg aca aag        48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt        96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc        144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc        192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa        240
   Thr Val Phe Leu Trp Tyr Gly Arg Leu Thr Arg Ser Ser Asp Lys Lys
   65                  70                  75                  80
   att aga gag cct acg tgg tta aga aga ttc ata ata tgt cat aat gcg        288
   Ile Arg Glu Pro Thr Trp Leu Arg Arg Phe Ile Ile Cys His Asn Ala
                   85                  90                  95
   ttc ttg ata gtc ctc agt ctt tac atg tgc ctt ggt tgt gtg gcc caa        336
   Phe Leu Ile Val Leu Ser Leu Tyr Met Cys Leu Gly Cys Val Ala Gln
               100                 105                 110
   gcg tat cag aat gga tat act tta tgg ggt aat gaa ttc aag gcc acg        384
   Ala Tyr Gln Asn Gly Tyr Thr Leu Trp Gly Asn Glu Phe Lys Ala Thr
           115                 120                 125
   gaa act cag ctt gct ctc tac att tac att ttt tac gta agt aaa ata        432
   Glu Thr Gln Leu Ala Leu Tyr Ile Tyr Ile Phe Tyr Val Ser Lys Ile
       130                 135                 140
   tac gag ttt gta gat act tac att atg ctt ctc aag aat aac ttg cgg        480
   Tyr Glu Phe Val Asp Thr Tyr Ile Met Leu Leu Lys Asn Asn Leu Arg
   145                 150                 155                 160
   caa gta agt ttc cta cac att tat cac cac agc acg att tcc ttt att        528
   Gln Val Ser Phe Leu His Ile Tyr His His Ser Thr Ile Ser Phe Ile
                   165                 170                 175
   tgg tgg atc att gct cgg agg gct ccg ggt ggt gat gct tac ttc agc        576
   Trp Trp Ile Ile Ala Arg Arg Ala Pro Gly Gly Asp Ala Tyr Phe Ser
               180                 185                 190
   gcg gcc ttg aac tca tgg gta cac gtg tgc atg tac acc tat tat cta        624
   Ala Ala Leu Asn Ser Trp Val His Val Cys Met Tyr Thr Tyr Tyr Leu
           195                 200                 205
   tta tca acc ctt att gga aaa gaa gat cct aag cgt tcc aac tac ctt        672
   Leu Ser Thr Leu Ile Gly Lys Glu Asp Pro Lys Arg Ser Asn Tyr Leu
       210                 215                 220
   tgg tgg ggt cgc cac cta acg caa atg cag atg ctt cag ttt ttc ttc        720
   Trp Trp Gly Arg His Leu Thr Gln Met Gln Met Leu Gln Phe Phe Phe
   225                 230                 235                 240
   aac gta ctt caa gcg ttg tac tgc gct tcg ttc tct acg tat ccc aag        768
   Asn Val Leu Gln Ala Leu Tyr Cys Ala Ser Phe Ser Thr Tyr Pro Lys
                   245                 250                 255
   ttt ttg tcc aaa att ctg ctc gtc tat atg atg agc ctt ctc ggc ttg        816
   Phe Leu Ser Lys Ile Leu Leu Val Tyr Met Met Ser Leu Leu Gly Leu
               260                 265                 270
   ttt ggg cat ttc tac tat tcc aag cac ata gca gca gct aag ctc cag        864
   Phe Gly His Phe Tyr Tyr Ser Lys His Ile Ala Ala Ala Lys Leu Gln
           275                 280                 285
   aaa aaa cag cag tga                                                    879
   Lys Lys Gln Gln
       290
<210> 150
   <211> 292
   <212> PRT
   <213> Ostreococcus tauri
<400> 150
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Ser Phe Leu His Ile Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
           275                 280                 285
   Lys Lys Gln Gln
       290
<210> 151
   <211> 873
   <212> DNA
   <213> Physcomitrella patens
<220>
   <221> CDS
   <222> (1)..(873)
<400> 151
   atg gag gtc gtg gag aga ttc tac ggt gag ttg gat ggg aag gtc tcg        48
   Met Glu Val Val Glu Arg Phe Tyr Gly Glu Leu Asp Gly Lys Val Ser
   1               5                   10                  15
   cag ggc gtg aat gca ttg ctg ggt agt ttt ggg gtg gag ttg acg gat        96
   Gln Gly Val Asn Ala Leu Leu Gly Ser Phe Gly Val Glu Leu Thr Asp
               20                  25                  30
   acg ccc act acc aaa ggc ttg ccc ctc gtt gac agt ccc aca ccc atc        144
   Thr Pro Thr Thr Lys Gly Leu Pro Leu Val Asp Ser Pro Thr Pro Ile
           35                  40                  45
   gtc ctc ggt gtt tct gta tac ttg act att gtc att gga ggg ctt ttg        192
   Val Leu Gly Val Ser Val Tyr Leu Thr Ile Val Ile Gly Gly Leu Leu
       50                  55                  60
   tgg ata aag gcc agg gat ctg aaa ccg cgc gcc tcg gag cca ttt ttg        240
   Trp Ile Lys Ala Arg Asp Leu Lys Pro Arg Ala Ser Glu Pro Phe Leu
   65                  70                  75                  80
   ctc caa gct ttg gtg ctt gtg cac aac ctg ttc tgt ttt gcg ctc agt        288
   Leu Gln Ala Leu Val Leu Val His Asn Leu Phe Cys Phe Ala Leu Ser
                   85                  90                  95
   ctg tat atg tgc gtg ggc atc gct tat cag gct att acc tgg cgg tac        336
   Leu Tyr Met Cys Val Gly Ile Ala Tyr Gln Ala Ile Thr Trp Arg Tyr
               100                 105                 110
   tct ctc tgg ggc aat gca tac aat cct aaa cat aaa gag atg gcg att        384
   Ser Leu Trp Gly Asn Ala Tyr Asn Pro Lys His Lys Glu Met Ala Ile
           115                 120                 125
   ctg gta tac ttg ttc tac atg tct aag tac gtg gaa ttc atg gat acc        432
   Leu Val Tyr Leu Phe Tyr Met Ser Lys Tyr Val Glu Phe Met Asp Thr
       130                 135                 140
   gtt atc atg ata ctg aag cgc agc acc agg caa ata agc ttc ctc cac        480
   Val Ile Met Ile Leu Lys Arg Ser Thr Arg Gln Ile Ser Phe Leu His
   145                 150                 155                 160
   gtt tat cat cat tct tca att tcc ctc att tgg tgg gct att gct cat        528
   Val Tyr His His Ser Ser Ile Ser Leu Ile Trp Trp Ala Ile Ala His
                   165                 170                 175
   cac gct cct ggc ggt gaa gca tat tgg tct gcg gct ctg aac tca gga        576
   His Ala Pro Gly Gly Glu Ala Tyr Trp Ser Ala Ala Leu Asn Ser Gly
               180                 185                 190
   gtg cat gtt ctc atg tat gcg tat tac ttc ttg gct gcc tgc ctt cga        624
   Val His Val Leu Met Tyr Ala Tyr Tyr Phe Leu Ala Ala Cys Leu Arg
           195                 200                 205
   agt agc cca aag tta aaa aat aag tac ctt ttt tgg ggc agg tac ttg        672
   Ser Ser Pro Lys Leu Lys Asn Lys Tyr Leu Phe Trp Gly Arg Tyr Leu
       210                 215                 220
   aca caa ttc caa atg ttc cag ttt atg ctg aac tta gtg cag gct tac        720
   Thr Gln Phe Gln Met Phe Gln Phe Met Leu Asn Leu Val Gln Ala Tyr
   225                 230                 235                 240
   tac gac atg aaa acg aat gcg cca tat cca caa tgg ctg atc aag att        768
   Tyr Asp Met Lys Thr Asn Ala Pro Tyr Pro Gln Trp Leu Ile Lys Ile
                   245                 250                 255
   ttg ttc tac tac atg atc tcg ttg ctg ttt ctt ttc ggc aat ttt tac        816
   Leu Phe Tyr Tyr Met Ile Ser Leu Leu Phe Leu Phe Gly Asn Phe Tyr
               260                 265                 270
   gta caa aaa tac atc aaa ccc tct gac gga aag caa aag gga gct aaa        864
   Val Gln Lys Tyr Ile Lys Pro Ser Asp Gly Lys Gln Lys Gly Ala Lys
           275                 280                 285
   act gag tga                                                            873
   Thr Glu
       290
<210> 152
   <211> 290
   <212> PRT
   <213> Physcomitrella patens
<400> 152
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145                 150                 155                 160
   Val Tyr His His Ser Ser Ile Ser Leu Ile Trp Trp Ala Ile Ala His
                   165                 170                 175
   His Ala Pro Gly Gly Glu Ala Tyr Trp Ser Ala Ala Leu Asn Ser Gly
               180                 185                 190
   Val His Val Leu Met Tyr Ala Tyr Tyr Phe Leu Ala Ala Cys Leu Arg
           195                 200                 205
   Ser Ser Pro Lys Leu Lys Asn Lys Tyr Leu Phe Trp Gly Arg Tyr Leu
       210                 215                 220
   Thr Gln Phe Gln Met Phe Gln Phe Met Leu Asn Leu Val Gln Ala Tyr
   225                 230                 235                 240
   Tyr Asp Met Lys Thr Asn Ala Pro Tyr Pro Gln Trp Leu Ile Lys Ile
                   245                 250                 255
   Leu Phe Tyr Tyr Met Ile Ser Leu Leu Phe Leu Phe Gly Asn Phe Tyr
               260                 265                 270
   Val Gln Lys Tyr Ile Lys Pro Ser Asp Gly Lys Gln Lys Gly Ala Lys
           275                 280                 285
   Thr Glu
       290
<210> 153
   <211> 957
   <212> DNA
   <213> Mortierella alpina
<220>
   <221> CDS
   <222> (1)..(957)
<400> 153
   atg gag tcg att gcg cca ttc ctc cca tca aag atg ccg caa gat ctg      48
   Met Glu Ser Ile Ala Pro Phe Leu Pro Ser Lys Met Pro Gln Asp Leu
   1               5                   10                  15
   ttt atg gac ctt gcc acc gct atc ggt gtc cgg gcc gcg ccc tat gtc      96
   Phe Met Asp Leu Ala Thr Ala Ile Gly Val Arg Ala Ala Pro Tyr Val
               20                  25                  30
   gat cct ctc gag gcc gcg ctg gtg gcc cag gcc gag aag tac atc ccc     144
   Asp Pro Leu Glu Ala Ala Leu Val Ala Gln Ala Glu Lys Tyr Ile Pro
           35                  40                  45
   acg att gtc cat cac acg cgt ggg ttc ctg gtc gcg gtg gag tcg cct     192
   Thr Ile Val His His Thr Arg Gly Phe Leu Val Ala Val Glu Ser Pro
       50                  55                  60
   ttg gcc cgt gag ctg ccg ttg atg aac ccg ttc cac gtg ctg ttg atc     240
   Leu Ala Arg Glu Leu Pro Leu Met Asn Pro Phe His Val Leu Leu Ile
   65                  70                  75                  80
   gtg ctc gct tat ttg gtc acg gtc ttt gtg ggc atg cag atc atg aag     288
   Val Leu Ala Tyr Leu Val Thr Val Phe Val Gly Met Gln Ile Met Lys
                   85                  90                  95
   aac ttt gag cgg ttc gag gtc aag acg ttt tcg ctc ctg cac aac ttt     336
   Asn Phe Glu Arg Phe Glu Val Lys Thr Phe Ser Leu Leu His Asn Phe
               100                 105                 110
   tgt ctg gtc tcg atc agc gcc tac atg tgc ggt ggg atc ctg tac gag     384
   Cys Leu Val Ser Ile Ser Ala Tyr Met Cys Gly Gly Ile Leu Tyr Glu
           115                 120                 125
   gct tat cag gcc aac tat gga ctg ttt gag aac gct gct gat cat acc     432
   Ala Tyr Gln Ala Asn Tyr Gly Leu Phe Glu Asn Ala Ala Asp His Thr
       130                 135                 140
   ttc aag ggt ctt cct atg gcc aag atg atc tgg ctc ttc tac ttc tcc     480
   Phe Lys Gly Leu Pro Met Ala Lys Met Ile Trp Leu Phe Tyr Phe Ser
   145                 150                 155                 160
   aag atc atg gag ttt gtc gac acc atg atc atg gtc ctc aag aag aac     528
   Lys Ile Met Glu Phe Val Asp Thr Met Ile Met Val Leu Lys Lys Asn
                   165                 170                 175
   aac cgc cag atc tcc ttc ttg cac gtt tac cac cac agc tcc atc ttc     576
   Asn Arg Gln Ile Ser Phe Leu His Val Tyr His His Ser Ser Ile Phe
               180                 185                 190
   acc atc tgg tgg ttg gtc acc ttt gtt gca ccc aac ggt gaa gcc tac     624
   Thr Ile Trp Trp Leu Val Thr Phe Val Ala Pro Asn Gly Glu Ala Tyr
           195                 200                 205
   ttc tct gct gcg ttg aac tcg ttc atc cat gtg atc atg tac ggc tac     672
   Phe Ser Ala Ala Leu Asn Ser Phe Ile His Val Ile Met Tyr Gly Tyr
       210                 215                 220
   tac ttc ttg tcg gcc ttg ggc ttc aag cag gtg tcg ttc atc aag ttc     720
   Tyr Phe Leu Ser Ala Leu Gly Phe Lys Gln Val Ser Phe Ile Lys Phe
   225                 230                 235                 240
   tac atc acg cgc tcg cag atg aca cag ttc tgc atg atg tcg gtc cag     768
   Tyr Ile Thr Arg Ser Gln Met Thr Gln Phe Cys Met Met Ser Val Gln
                   245                 250                 255
   tct tcc tgg gac atg tac gcc atg aag gtc ctt ggc cgc ccc gga tac     816
   Ser Ser Trp Asp Met Tyr Ala Met Lys Val Leu Gly Arg Pro Gly Tyr
               260                 265                 270
   ccc ttc ttc atc acg gct ctg ctt tgg ttc tac atg tgg acc atg ctc     864
   Pro Phe Phe Ile Thr Ala Leu Leu Trp Phe Tyr Met Trp Thr Met Leu
           275                 280                 285
   ggt ctc ttc tac aac ttt tac aga aag aac gcc aag ttg gcc aag cag     912
   Gly Leu Phe Tyr Asn Phe Tyr Arg Lys Asn Ala Lys Leu Ala Lys Gln
       290                 295                 300
   gcc aag gcc gac gct gcc aag gag aag gca agg aag ttg cag taa         957
   Ala Lys Ala Asp Ala Ala Lys Glu Lys Ala Arg Lys Leu Gln
   305                 310                 315
<210> 154
   <211> 318
   <212> PRT
   <213> Mortierella alpina
<400> 154
   Met Glu Ser Ile Ala Pro Phe Leu Pro Ser Lys Met Pro Gln Asp Leu
   1 5 10 15
   Phe Met Asp Leu Ala Thr Ala Ile Gly Val Arg Ala Ala Pro Tyr Val
   20 25 30
   Asp Pro Leu Glu Ala Ala Leu Val Ala Gln Ala Glu Lys Tyr Ile Pro
   35 40 45
   Thr Ile Val His His Thr Arg Gly Phe Leu Val Ala Val Glu Ser Pro
   50 55 60
   Leu Ala Arg Glu Leu Pro Leu Met Asn Pro Phe His Val Leu Leu Ile
   65 70 75 80
   Val Leu Ala Tyr Leu Val Thr Val Phe Val Gly Met Gln Ile Met Lys
   85 90 95
   Asn Phe Glu Arg Phe Glu Val Lys Thr Phe Ser Leu Leu His Asn Phe
   100 105 110
   Cys Leu Val Ser Ile Ser Ala Tyr Met Cys Gly Gly Ile Leu Tyr Glu
   115 120 125
   Ala Tyr Gln Ala Asn Tyr Gly Leu Phe Glu Asn Ala Ala Asp His Thr
   130 135 140
   Phe Lys Gly Leu Pro Met Ala Lys Met Ile Trp Leu Phe Tyr Phe Ser
   145 150 155 160
   Lys Ile Met Glu Phe Val Asp Thr Met Ile Met Val Leu Lys Lys Asn
   165 170 175
   Asn Arg Gln Ile Ser Phe Leu His Val Tyr His His Ser Ser Ile Phe
   180 185 190
   Thr Ile Trp Trp Leu Val Thr Phe Val Ala Pro Asn Gly Glu Ala Tyr
   195 200 205
   Phe Ser Ala Ala Leu Asn Ser Phe Ile His Val Ile Met Tyr Gly Tyr
   210 215 220
   Tyr Phe Leu Ser Ala Leu Gly Phe Lys Gln Val Ser Phe Ile Lys Phe
   225 230 235 240
   Tyr Ile Thr Arg Ser Gln Met Thr Gln Phe Cys Met Met Ser Val Gln
   245 250 255
   Ser Ser Trp Asp Met Tyr Ala Met Lys Val Leu Gly Arg Pro Gly Tyr
   260 265 270
   Pro Phe Phe Ile Thr Ala Leu Leu Trp Phe Tyr Met Trp Thr Met Leu
   275 280 285
   Gly Leu Phe Tyr Asn Phe Tyr Arg Lys Asn Ala Lys Leu Ala Lys Gln
   290 295 300
   Ala Lys Ala Asp Ala Ala Lys Glu Lys Ala Arg Lys Leu Gln
   305 310 315
<210> 155
   <211> 236
   <212> PRT
   <213> Artificial sequence
<220>
   <221> Variant
   <222> (2)..(8)
   <223> xaa in position 2 to 8 is any amino acid
<220>
   <221> Variant
   <222> (10)..(10)
   <223> xaa in position 10 is any amino acid
<220>
   <221> Variant
   <222> (13)..(14)
   <223> xaa in position 13 to 14 is any amino acid
<220>
   <221> Variant
   <222> (16)..(36)
   <223> xaa in position 16 to 36 is any amino acid
<220>
   <221> Variant
   <222> (37)..(43)
   <223> xaa in position 37 to 43 is any or no amino acid
<220>
   <221> Variant
   <222> (46)..(47)
   <223> xaa in position 46 to 47 is any amino acid
<220>
   <221> Variant
   <222> (49)..(50)
   <223> xaa in position 49 to 50 is any amino acid
<220>
   <221> Variant
   <222> (53)..(53)
   <223> xaa in position 53 is any amino acid
<220>
   <221> Variant
   <222> (56)..(57)
   <223> xaa in position 56 to 57 is any amino acid
<220>
   <221> Variant
   <222> (59)..(62)
   <223> xaa in position 59 to 62 is any amino acid
<220>
   <221> Variant
   <222> (64)..(67)
   <223> xaa in position 64 to 67 is any amino acid
<220>
   <221> Variant
   <222> (69)..(69)
   <223> xaa in position 69 is any amino acid
<220>
   <221> Variant
   <222> (71)..(72)
   <223> xaa in position 71 to 72 is any amino acid
<220>
   <221> Variant
   <222> (74)..(82)
   <223> xaa in position 74 to 82 is any amino acid
<220>
   <221> Variant
   <222> (83)..(86)
   <223> xaa in position 83 to 86 is any or no amino acid
<220>
   <221> Variant
   <222> (88)..(89)
   <223> xaa in position 88 to 89 is any amino acid
<220>
   <221> Variant
   <222> (91)..<92)
   <223> xaa in position 91 to 92 is any amino acid
<220>
   <221> Variant
   <222> (95)..(95)
   <223> xaa in position 95 is any amino acid
<220>
   <221> Variant
   <222> (98)..(99)
   <223> xaa in position 98 to 99 is any amino acid
<220>
   <221> Variant
   <222> (102)..(102)
   <223> xaa in position 102 is any amino acid
<220>
   <221> Variant
   <222> (105)..(105)
   <223> xaa in position 105 is any amino acid
<220>
   <221> Variant
   <222> (108)..(108)
   <223> xaa in position 108 is any amino acid
<220>
   <221> Variant
   <222> (111)..(114)
   <223> xaa in position 111 to 114 is any amino acid
<220>
   <221> Variant
   <222> (116)..(116)
   <223> xaa in position 116 is any amino acid
<220>
   <221> Variant
   <222> (121)..(121)
   <223> xaa in position 121 is any amino acid
<220>
   <221> Variant
   <222> (125)..(126)
   <223> xaa in position 125 to 126 is any amino acid
<220>
   <221> Variant
   <222> (128)..(129)
   <223> xaa in position 128 to 129 is any amino acid
<220>
   <221> Variant
   <222> (133)..(137)
   <223> xaa in position 133 to 137 is any amino acid
<220>
   <221> Variant
   <222> (142)..(142)
   <223> xaa in position 142 is any amino acid
<220>
   <221> Variant
   <222> (145)..(145)
   <223> xaa in position 145 is any amino acid
<220>
   <221> Variant
   <222> (148)..(148)
   <223> xaa in position 148 is any amino acid
<220>
   <221> Variant
   <222> (152)..(152)
   <223> xaa in position 152 is any amino acid
<220>
   <221> Variant
   <222> (156)..(156)
   <223> xaa in position 156 is any amino acid
<220>
   <221> Variant
   <222> (159)..(159)
   <223> xaa in position 159 is any amino acid
<220>
   <221> Variant
   <222> (162)..(162)
   <223> xaa in position 162 is any amino acid
<220>
   <221> Variant
   <222> (164)..(165)
   <223> xaa in position 164 to 165 is any amino acid
<220>
   <221> Variant
   <222> (167)..(178)
   <223> xaa in position 167 to 178 is any amino acid
<220>
   <221> Variant
   <222> (179)..(184)
   <223> xaa in position 179 to 184 is any or no amino acid
<220>
   <221> Variant
   <222> (186)..(187)
   <223> xaa in position 186 to 187 is any amino acid
<220>
   <221> variant
   <222> (190)..(190)
   <223> xaa in position 190 is any amino acid
<220>
   <221> variant
   <222> (193)..(197)
   <223> xaa in position 193 to 197 is any amino acid
<220>
   <221> Variant
   <222> (199)..(200)
   <223> xaa in position 199 to 200 is any amino acid
<220>
   <221> variant
   <222> (202)..(207)
   <223> xaa in position 202 to 207 is any amino acid
<220>
   <221> Variant
   <222> (208)..(213)
   <223> xaa in position 208 to 213 is any or no amino acid
<220>
   <221> Variant
   <222> (216)..(217)
   <223> xaa in position 216 to 217 is any amino acid
<220>
   <221> Variant
   <222> (219)..(221)
   <223> xaa in position 219 to 221 is any amino acid
<220>
   <221> variant
   <222> (223)..(224)
   <223> xaa in position 223 to 224 is any amino acid
<220>
   <221> Variant
   <222> (227)..(229)
   <223> xaa in position 227 to 229 is any amino acid
<220>
   <221> Variant
   <222> (231)..(231)
   <223> xaa in position 231 is any amino acid
<220>
   <221> Variant
   <222> (235)..(235)
   <223> xaa in position 235 is any amino acid
<400> 155
   Pro Xaa Xaa Xaa Xaa Xaa Xaa Xaa Leu Xaa Tyr Leu Xaa Xaa Val Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Xaa Xaa His Asn Xaa Xaa Leu
           35                  40                  45
   Xaa Xaa Leu Ser Xaa Tyr Met Xaa Xaa Gly Xaa Xaa Xaa Xaa Ala Xaa
       50                  55                  60
   Xaa Xaa Xaa Tyr Xaa Leu Xaa Xaa Asn Xaa Xaa Xaa Xaa Xaa Xaa Xaa
   65                  70                  75                  80
   Xaa Xaa Xaa Xaa Xaa Xaa Ala Xaa Xaa Ile Xaa Xaa Phe Tyr Xaa Ser
                   85                  90                  95
   Lys Xaa Xaa Glu Phe Xaa Asp Thr Xaa Ile Met Xaa Leu Lys Xaa Xaa
               100                 105                 110
   Xaa Xaa Gln Xaa Ser Phe Leu His Xaa Tyr His His Xaa Xaa Ile Xaa
           115                 120                 125
   Xaa Ile Trp Trp Xaa Xaa Xaa Xaa Xaa Ala Pro Gly Gly Xaa Ala Tyr
       130                 135                 140
   Xaa Ser Ala Xaa Leu Asn Ser Xaa Val His Val Xaa Met Tyr Xaa Tyr
   145                 150                 155                 160
   Tyr Xaa Leu Xaa Xaa Leu Xaa Xaa Xaa Xaa Xaa Xaa Xaa Xaa Xaa Xaa
                   165                 170                 175
   Xaa Xaa Xaa Xaa Xaa Xaa Xaa Xaa Thr Xaa Xaa Gln Met Xaa Gln Phe
               180                 185                 190
   Xaa Xaa Xaa Xaa Xaa Gln Xaa Xaa Tyr Xaa Xaa Xaa Xaa Xaa Xaa Xaa
           195                 200                 205
   Xaa Xaa Xaa Xaa Xaa Tyr Pro Xaa Xaa Leu Xaa Xaa Xaa Leu Xaa Xaa
       210                 215                 220
   Tyr Met Xaa Xaa Xaa Leu Xaa Leu Phe Gly Xaa Phe
   225                 230                 235
<210> 156
   <211> 59
   <212> PRT
   <213> Artificial sequence
<220>
   <221> Variant
   <222> (2)..(2)
   <223> xaa in position 2 is any amino acid
<220>
   <221> Variant
   <222> (5)..(5)
   <223> xaa in position 5 is any amino acid
<220>
   <221> variant
   <222> (6)..(6)
   <223> xaa in position 6 is Phe or Ile
<220>
   <221> Variant
   <222> (8)..(8)
   <223> xaa in position 8 is Ile, Leu or Val
<220>
   <221> variant
   <222> (10)..(13)
   <223> xaa in position 10 to 13 is any amino acid
<220>
   <221> variant
   <222> (14)..(14)
   <223> xaa in position 14 is Cys or Arg
<220>
   <221> Variant
   <222> (16)..(16)
   <223> xaa in position 16 is Ile, Leu or Val
<220>
   <221> variant
   <222> (21)..(21)
   <223> xaa in position 21 is Ile or Val

<220>
   <221> Variant
   <222> (25)..(25)
   <223> xaa in position 25 is Ala or Ser
<220>
   <221> Variant
   <222> (26)..(26)
   <223> xaa in position 26 is Ser or Thr
<220>
   <221> Variant
   <222> (28)..(29)
   <223> xaa in position 28 to 29 is any amino acid
<220>
   <221> Variant
   <222> (32)..(32)
   <223> xaa in position 32 is any amino acid
<220>
   <221> Variant
   <222> (33)..(33)
   <223> xaa in position 33 is Ala, Ile or Leu
<220>
   <221> Variant
   <222> (34)..(34)
   <223> xaa in position 34 is Ile or Val
<220>
   <221> Variant
   <222> (35)...(35)
   <223> xaa in position 35 is Ala or Thr
<220>
   <221> Variant
   <222> (36)..(37)
   <223> xaa in position 36 to 37 is any amino acid
<220>
   <221> Variant
   <222> (40)..(40)
   <223> xaa in position 40 is Gly or Asn
<220>
   <221> variant
   <222> (42)..(42)
   <223> xaa in position 42 is Asp or Glu
<220>
   <221> Variant
   <222> (45)..(45)
   <223> xaa in position 45 is Phe or Trp
<220>
   <221> Variant
   <222> (47)..(47)
   <223> xaa in position 47 is Ala or Thr
<220>
   <221> variant
   <222> (48)..(48)
   <223> xaa in position 48 is Ala or Ile
<220>
   <221> Variant
   <222> (52)..(52)
   <223> xaa in position 52 is any amino acid
<220>
   <221> Variant
   <222> (53)..(53)
   <223> xaa in position 53 is Ile or Val
<220>
   <221> variant
   <222> (56)..(56)
   <223> xaa in position 56 is any amino acid
<220>
   <221> Variant
   <222> (57)..(57)
   <223> xaa in position 57 is Leu or Met
<220>
   <221> Variant
   <222> (59)..(59)
   <223> xaa in position 59 is Ala, Gly or Thr
<400> 156
<210> 157
   <211> 22
   <212> PRT
   <213> Artificial sequence
<220>
   <221> Variant
   <222> (2)..(2)
   <223> xaa in position 2 is Leu or Met
<220>
   <221> Variant
   <222> (5)..(5)
   <223> xaa in position 5 is Ala, Cys or Ser
<220>
   <221> Variant
   <222> (6)..(6)
   <223> xaa in position 6 is any amino acid
<220>
   <221> Variant
   <222> (7)..(7)
   <223> xaa in position 7 is Glu or Gly
<220>
   <221> Variant
   <222> (8)..(10)
   <223> xaa in position 8 to 10 is any amino acid
<220>
   <221> variant
   <222> (11) .. (11)
   <223> xaa in position 11 is Gln or Ser
<220>
   <221> variant
   <222> (13)..(16)
   <223> xaa in position 13 to 16 is any amino acid
<220>
   <221> Variant
   <222> (18)..(18)
   <223> xaa in position 18 is any amino acid
<220>
   <221> Variant
   <222> (20)..(20)
   <223> xaa in position 20 is Phe or Trp
<220>
   <221> variant
   <222> (21)..(21)
   <223> xaa in position 21 is Gly or Asn
<400> 157
   Ser xaa Tyr Met xaa xaa xaa xaa xaa xaa xaa Ala xaa xaa xaa xaa
   1 5 10 15
   Tyr xaa Leu xaa xaa Asn
   20
<210> 158
   <211> 16
   <212> PRT
   <213> Artificial sequence
<220>
   <221> Variant
   <222> (2)..(2)
   <223> xaa in position 2 is Leu or Met
<220>
   <221> Variant
   <222> (3)..(3)
   <223> xaa in position 3 is any amino acid
<220>
   <221> Variant
   <222> (6)..(8)
   <223> xaa in position 6 to 8 is any amino acid
<220>
   <221> variant
   <222> (9)..(9)
   <223> xaa in position 9 is any or no amino acid
<220>
   <221> Variant
   <222> (11)..(11)
   <223> xaa in position 11 is any or no amino acid
<220>
   <221> Variant
   <222> (13)..(13)
   <223> xaa in position 13 is Ala or Ser
<220>
   <221> variant
   <222> (14)..(14)
   <223> xaa in position 14 is any amino acid
<220>
   <221> Variant
   <222> (15)..(15)
   <223> xaa in position 15 is Trp or Tyr
<220>
   <221> Variant
   <222> (16)..(16)
   <223> xaa in position 16 is Cys or Asp
<400> 158
   Gln xaa xaa Gln Phe xaa xaa xaa xaa Val xaa Gln xaa xaa xaa xaa
   1 5 10 15
<210> 159
   <211> 24
   <212> PRT
   <213> Artificial sequence
<220>
   <221> Variant
   <222> (3)..(4)
   <223> xaa in position 3 to 4 is any amino acid
<220>
   <221> Variant
   <222> (5)..(5)
   <223> xaa in position 5 is Ile or Leu
<220>
   <221> Variant
   <222> (6)..(7)
   <223> xaa in position 6 to 7 is any amino acid
<220>
   <221> Variant
   <222> (8)..(8)
   <223> xaa in position 8 is Ile or Leu
<220>
   <221> Variant
   <222> (10)..(11)
   <223> xaa in position 10 to 11 is any amino acid
<220>
   <221> Variant
   <222> (14)..(14)
   <223> xaa in position 14 is any amino acid
<220>
   <221> Variant
   <222> (15)..(15)
   <223> xaa in position 15 is Ser or Thr
<220>
   <221> Variant
   <222> (16)..(16)
   <223> xaa in position 16 is Leu or Met
<220>
   <221> Variant
   <222> (18)..(18)
   <223> xaa in position 18 is any amino acid
<220>
   <221> Variant
   <222> (21)..(22)
   <223> xaa in position 21 to 22 is any amino acid
<220>
   <221> Variant
   <222> (24)..(24)
   <223> xaa in position 24 is Phe or Tyr
<400> 159
   Tyr Pro xaa xaa xaa xaa xaa xaa Leu xaa xaa Tyr Met xaa xaa xaa
    1               5                  10                  15
   Leu xaa Leu Phe xaa xaa Phe xaa
               20
<210> 160
   <211> 8
   <212> PRT
   <213> Artificial sequence
<220>
   <221> Variant
   <222> (2)..(2)
   <223> xaa in position 2 is Trp or Tyr
<220>
   <221> Variant
   <222> (3)..(3)
   <223> xaa in position 3 is Ile or Leu
<220>
   <221> Variant
   <222> (6)..(6)
   <223> xaa in position 6 is Phe or Val
<400> 160
   Ile xaa xaa Phe Tyr xaa Ser Lys
    1               5
<210> 161
   <211> 755
   <212> DNA
   <213> Cauliflower mosaic virus
<400> 161
   cctgcaggtc aacatggtgg agcacgacac acttgtctac tccaaaaata tcaaagatac     60
   agtctcagaa gaccaaaggg caattgagac ttttcaacaa agggtaatat ccggaaacct    120
   cctcggattc cattgcccag ctatctgtca ctttattgtg aagatagtgg aaaaggaagg    180
   tggctcctac aaatgccatc attgcgataa aggaaaggcc atcgttgaag atgcctctgc    240
   cgacagtggt cccaaagatg gacccccacc cacgaggagc atcgtggaaa aagaagacgt    300
   tccaaccacg tcttcaaagc aagtggattg atgtgataac atggtggagc acgacacact    360
   tgtctactcc aaaaatatca aagatacagt ctcagaagac caaagggcaa ttgagacttt    420
   tcaacaaagg gtaatatccg gaaacctcct cggattccat tgcccagcta tctgtcactt    480
   tattgtgaag atagtggaaa aggaaggtgg ctcctacaaa tgccatcatt gcgataaagg    540
   aaaggccatc gttgaagatg cctctgccga cagtggtccc aaagatggac ccccacccac    600
   gaggagcatc gtggaaaaag aagacgttcc aaccacgtct tcaaagcaag tggattgatg    660
   tgatatctcc actgacgtaa gggatgacgc acaatcccac tatccttcgc aagacccttc    720
   ctctatataa ggaagttcat ttcatttgga gagga                               755
<210> 162
   <211> 211
   <212> DNA
   <213> Cauliflower mosaic virus
<400> 162
   agtccgcaaa aatcaccagt ctctctctac aaatctatct ctctctattt ttctccagaa      60
   taatgtgtga gtagttccca gataagggaa ttagggttct tatagggttt cgctcatgtg     120
   ttgagcatat aagaaaccct tagtatgtat ttgtatttgt aaaatacttc tatcaataaa     180
   atttctaatt cctaaaacca aaatccagtg a                                    211
<210> 163
   <211> 819
   <212> DNA
   <213> Thalassiosira pseudonana
<220>
   <221> CDS
   <222> (1)..(819)
<400> 163
   atg gac gcc tac aac gct gca atg gat aag atc ggt gcc gcc atc atc        48
   Met Asp Ala Tyr Asn Ala Ala Met Asp Lys Ile Gly Ala Ala Ile Ile
   1               5                   10                  15
   gat tgg tct gat ccc gat gga aag ttc cgt gcc gat aga gag gac tgg        96
   Asp Trp Ser Asp Pro Asp Gly Lys Phe Arg Ala Asp Arg Glu Asp Trp
               20                  25                  30
   tgg ctc tgc gac ttc cgt agc gcc atc acc atc gcc ctc atc tac atc       144
   Trp Leu Cys Asp Phe Arg Ser Ala Ile Thr Ile Ala Leu Ile Tyr Ile
           35                  40                  45
   gcc ttc gtc atc ctc ggt tcc gcc gtc atg caa tcc ctc ccc gca atg       192
   Ala Phe Val Ile Leu Gly Ser Ala Val Met Gln Ser Leu Pro Ala Met
       50                  55                  60
   gat ccc tac ccc atc aaa ttc ctc tac aac gtc tcc caa atc ttc ctt       240
   Asp Pro Tyr Pro Ile Lys Phe Leu Tyr Asn Val Ser Gln Ile Phe Leu
   65                  70                  75                  80
   tgt gcc tac atg act gtc gag gcg gga ttt ttg gcc tac cgc aat gga       288
   Cys Ala Tyr Met Thr Val Glu Ala Gly Phe Leu Ala Tyr Arg Asn Gly
                   85                  90                  95
   tat acc gtc atg cct tgc aat cat ttc aat gtg aat gat cct ccc gtg       336
   Tyr Thr Val Met Pro Cys Asn His Phe Asn Val Asn Asp Pro Pro Val
               100                 105                 110
   gcg aat ctt ctt tgg ttg ttt tat att tcc aag gtg tgg gac ttt tgg       384
   Ala Asn Leu Leu Trp Leu Phe Tyr Ile Ser Lys Val Trp Asp Phe Trp
           115                 120                 125
   gat acc att ttc att gtg ttg ggg aag aag tgg cgt caa tta tct ttc       432
   Asp Thr Ile Phe Ile Val Leu Gly Lys Lys Trp Arg Gln Leu Ser Phe
       130                 135                 140
   ttg cat gta tac cat cac acc acc atc ttt cta ttc tat tgg ctg aat       480
   Leu His Val Tyr His His Thr Thr Ile Phe Leu Phe Tyr Trp Leu Asn
   145                 150                 155                 160
   gcc aat gtc ttg tac gat ggt gac atc ttc ctt acc atc ttg ctc aat       528
   Ala Asn Val Leu Tyr Asp Gly Asp Ile Phe Leu Thr Ile Leu Leu Asn
                   165                 170                 175
   gga ttc atc cac acg gtg atg tac acg tat tac ttc atc tgt atg cat      576
   Gly Phe Ile His Thr Val Met Tyr Thr Tyr Tyr Phe Ile Cys Met His
               180                 185                 190
   acc aaa gat tcc aag acg ggc aag agt ctt cct ata tgg tgg aag tcg      624
   Thr Lys Asp Ser Lys Thr Gly Lys Ser Leu Pro Ile Trp Trp Lys Ser
           195                 200                 205
   agt ttg acg gcg ttt cag ttg ttg caa ttc act atc atg atg agt cag      672
   Ser Leu Thr Ala Phe Gln Leu Leu Gln Phe Thr Ile Met Met Ser Gln
       210                 215                 220
   gct acc tac ctt gtc ttc cac ggg tgt gat aag gtg tcg ctt cgt atc      720
   Ala Thr Tyr Leu Val Phe His Gly Cys Asp Lys Val Ser Leu Arg Ile
   225                 230                 235                 240
   acg att gtg tac ttt gtg tcc ctt ttg agt ttg ttc ttc ctt ttt gct      768
   Thr Ile Val Tyr Phe Val Ser Leu Leu Ser Leu Phe Phe Leu Phe Ala
                   245                 250                 255
   cag ttc ttt gtg caa tca tac atg gca ccc aaa aag aag aag agt gct      816
   Gln Phe Phe Val Gln Ser Tyr Met Ala Pro Lys Lys Lys Lys Ser Ala
               260                 265                 270
   tag                                                                  819
<210> 164
   <211> 272
   <212> PRT
   <213> Thalassiosira pseudonana
<400> 164
   Met Asp Ala Tyr Asn Ala Ala Met Asp Lys Ile Gly Ala Ala Ile Ile
   1               5                   10                  15
   Asp Trp Ser Asp Pro Asp Gly Lys Phe Arg Ala Asp Arg Glu Asp Trp
               20                  25                  30
   Trp Leu Cys Asp Phe Arg Ser Ala Ile Thr Ile Ala Leu Ile Tyr Ile
           35                  40                  45
   Ala Phe Val Ile Leu Gly Ser Ala Val Met Gln Ser Leu Pro Ala Met
       50                  55                  60
   Asp Pro Tyr Pro Ile Lys Phe Leu Tyr Asn Val Ser Gln Ile Phe Leu
   65                  70                  75                  80
   Cys Ala Tyr Met Thr Val Glu Ala Gly Phe Leu Ala Tyr Arg Asn Gly
                   85                  90                  95
   Tyr Thr Val Met Pro Cys Asn His Phe Asn Val Asn Asp Pro Pro Val
               100                 105                 110
   Ala Asn Leu Leu Trp Leu Phe Tyr Ile Ser Lys Val Trp Asp Phe Trp
           115                 120                 125
   Asp Thr Ile Phe Ile Val Leu Gly Lys Lys Trp Arg Gln Leu Ser Phe
       130                 135                 140
   Leu His Val Tyr His His Thr Thr Ile Phe Leu Phe Tyr Trp Leu Asn
   145                 150                 155                 160
   Ala Asn Val Leu Tyr Asp Gly Asp Ile Phe Leu Thr Ile Leu Leu Asn
                   165                 170                 175
   Gly Phe Ile His Thr Val Met Tyr Thr Tyr Tyr Phe Ile Cys Met His
               180                 185                 190
   Thr Lys Asp Ser Lys Thr Gly Lys Ser Leu Pro Ile Trp Trp Lys Ser
           195                 200                 205
   Ser Leu Thr Ala Phe Gln Leu Leu Gln Phe Thr Ile Met Met Ser Gln
       210                 215                 220
   Ala Thr Tyr Leu Val Phe His Gly Cys Asp Lys Val Ser Leu Arg Ile
   225                 230                 235                 240
   Thr Ile Val Tyr Phe Val Ser Leu Leu Ser Leu Phe Phe Leu Phe Ala
                   245                 250                 255
   Gln Phe Phe Val Gln Ser Tyr Met Ala Pro Lys Lys Lys Lys Ser Ala
               260                 265                 270
<210> 165
   <211> 837
   <212> DNA
   <213> Phaeodactylum tricornutum
<220>
   <221> CDS
   <222> (1)..(837)
<400> 165
   atg atg gta cct tca agt tat gac gag tat atc gtc atg gtc aac gac      48
   Met Met Val Pro Ser Ser Tyr Asp Glu Tyr Ile Val Met Val Asn Asp
   1               5                   10                  15
   ctt ggc gac tct att ctg agc tgg gcc gac cct gat cac tat cgt gga      96
   Leu Gly Asp Ser Ile Leu Ser Trp Ala Asp Pro Asp His Tyr Arg Gly
               20                  25                  30
   cat acc gag gga tgg gag ttc act gac ttt tct gct gct ttt agc att      144
   His Thr Glu Gly Trp Glu Phe Thr Asp Phe Ser Ala Ala Phe Ser Ile
           35                  40                  45
   gcc gtc gcg tac ctc ctg ttt gtc ttt gtt gga tct ctc att atg agt      192
   Ala Val Ala Tyr Leu Leu Phe Val Phe Val Gly Ser Leu Ile Met Ser
       50                  55                  60
   atg gga gtc ccc gca att gac cct tat ccg ctc aag ttt gtc tac aat      240
   Met Gly Val Pro Ala Ile Asp Pro Tyr Pro Leu Lys Phe Val Tyr Asn
   65                  70                  75                  80
   gtt tca cag att atg ctt tgt gct tac atg acc att gaa gcc agt ctt      288
   Val Ser Gln Ile Met Leu Cys Ala Tyr Met Thr Ile Glu Ala Ser Leu
                   85                  90                  95
   cta gct tat cgt aac ggc tac aca ttc tgg cct tgc aac gat tgg gac      336
   Leu Ala Tyr Arg Asn Gly Tyr Thr Phe Trp Pro Cys Asn Asp Trp Asp
               100                 105                 110
   ttt gaa aag ccg cct atc gct aag ctc ctc tgg ctc ttt tac gtt tcc      384
   Phe Glu Lys Pro Pro Ile Ala Lys Leu Leu Trp Leu Phe Tyr Val Ser
           115                 120                 125
   aaa att tgg gat ttt tgg gac acc atc ttt att gtt ctc ggg aag aag      432
   Lys Ile Trp Asp Phe Trp Asp Thr Ile Phe Ile Val Leu Gly Lys Lys
       130                 135                 140
   tgg cgt caa ctt tcc ttc ctg cac gtc tac cat cac acc acc atc ttt      480
   Trp Arg Gln Leu Ser Phe Leu His Val Tyr His His Thr Thr Ile Phe
   145                 150                 155                 160
   ctc ttc tac tgg ttg aat gca cat gta aac ttt gat ggt gat att ttc      528
   Leu Phe Tyr Trp Leu Asn Ala His Val Asn Phe Asp Gly Asp Ile Phe
                   165                 170                 175
   ctc acc atc gtc ttg aac ggt ttc atc cac acc gtc atg tac acg tac      576
   Leu Thr Ile Val Leu Asn Gly Phe Ile His Thr Val Met Tyr Thr Tyr
               180                 185                 190
   tac ttc att tgc atg cac acc aag gtc cca gag acc ggc aaa tcc ttg      624
   Tyr Phe Ile Cys Met His Thr Lys Val Pro Glu Thr Gly Lys Ser Leu
           195                 200                 205
   ccc att tgg tgg aaa tct agt ttg aca agc atg cag ctg gtg cag ttc      672
   Pro Ile Trp Trp Lys Ser Ser Leu Thr Ser Met Gln Leu Val Gln Phe
       210                 215                 220
   atc acg atg atg acg cag gct atc atg atc ttg tac aag ggc tgt gct      720
   Ile Thr Met Met Thr Gln Ala Ile Met Ile Leu Tyr Lys Gly Cys Ala
   225                 230                 235                 240
   gct ccc cat agc cgg gtg gtg aca tcg tac ttg gtt tac att ttg tcg      768
   Ala Pro His Ser Arg Val Val Thr Ser Tyr Leu Val Tyr Ile Leu Ser
                   245                 250                 255
   ctc ttt att ttg ttc gcc cag ttc ttt gtc agc tca tac ctc aag ccg      816
   Leu Phe Ile Leu Phe Ala Gln Phe Phe Val Ser Ser Tyr Leu Lys Pro
               260                 265                 270
   aag aag aag aag aca gct taa                                          837
   Lys Lys Lys Lys Thr Ala
           275
<210> 166
   <211> 278
   <212> PRT
   <213> Phaeodactylum tricornutum
<400> 166
   Met Met Val Pro Ser Ser Tyr Asp Glu Tyr Ile Val Met Val Asn Asp
   1               5                   10                  15
   Leu Gly Asp Ser Ile Leu Ser Trp Ala Asp Pro Asp His Tyr Arg Gly
               20                  25                  30
   His Thr Glu Gly Trp Glu Phe Thr Asp Phe Ser Ala Ala Phe Ser Ile
           35                  40                  45
   Ala Val Ala Tyr Leu Leu Phe Val Phe Val Gly Ser Leu Ile Met Ser
       50                  55                  60
   Met Gly Val Pro Ala Ile Asp Pro Tyr Pro Leu Lys Phe Val Tyr Asn
   65                  70                  75                  80
   Val Ser Gln Ile Met Leu Cys Ala Tyr Met Thr Ile Glu Ala Ser Leu
                   85                  90                  95
   Leu Ala Tyr Arg Asn Gly Tyr Thr Phe Trp Pro Cys Asn Asp Trp Asp
               100                 105                 110
   Phe Glu Lys Pro Pro Ile Ala Lys Leu Leu Trp Leu Phe Tyr Val Ser
           115                 120                 125
   Lys Ile Trp Asp Phe Trp Asp Thr Ile Phe Ile Val Leu Gly Lys Lys
       130                 135                 140
   Trp Arg Gln Leu Ser Phe Leu His Val Tyr His His Thr Thr Ile Phe
   145                 150                 155                 160
   Leu Phe Tyr Trp Leu Asn Ala His Val Asn Phe Asp Gly Asp Ile Phe
                   165                 170                 175
   Leu Thr Ile Val Leu Asn Gly Phe Ile His Thr Val Met Tyr Thr Tyr
               180                 185                 190
   Tyr Phe Ile Cys Met His Thr Lys Val Pro Glu Thr Gly Lys Ser Leu
           195                 200                 205
   Pro Ile Trp Trp Lys Ser Ser Leu Thr Ser Met Gln Leu Val Gln Phe
       210                 215                 220
   Ile Thr Met Met Thr Gln Ala Ile Met Ile Leu Tyr Lys Gly Cys Ala
   225                 230                 235                 240
   Ala Pro His Ser Arg Val Val Thr Ser Tyr Leu Val Tyr Ile Leu Ser
                   245                 250                 255
   Leu Phe Ile Leu Phe Ala Gln Phe Phe Val Ser Ser Tyr Leu Lys Pro
               260                 265                 270
   Lys Lys Lys Lys Thr Ala
           275
<210> 167
   <211> 272
   <212> PRT
   <213> Artificial sequence
<220>
   <221> Variant
   <222> (2)..(2)
   <223> xaa in position 2 is any amino acid
<220>
   <221> Variant
   <222> (4)..(10)
   <223> xaa in position 4 to 10 is any amino acid
<220>
   <221> Variant
   <222> (12)..(13)
   <223> xaa in position 12 to 13 is any amino acid
<220>
   <221> Variant
   <222> (15)..(16)
   <223> xaa in position 15 to 16 is any amino acid
<220>
   <221> Variant
   <222> (18)..(18)
   <223> xaa in position 18 is any amino acid
<220>
   <221> Variant
   <222> (22)..(23)
   <223> xaa in position 22 to 23 is any amino acid
<220>
   <221> Variant
   <222> (24)..(24)
   <223> xaa in position 24 is any or no amino acid
<220>
   <221> Variant
   <222> (26)..(28)
   <223> xaa in position 26 to 28 is any amino acid
<220>
   <221> Variant
   <222> (30)..(30)
   <223> xaa in position 30 is any amino acid
<220>
   <221> Variant
   <222> (32)..(34)
   <223> xaa in position 32 to 34 is any amino acid
<220>
   <221> variant
   <222> (37)..(38)
   <223> xaa in position 37 to 38 is any amino acid
<220>
   <221> Variant
   <222> (40)..(41)
   <223> xaa in position 40 to 41 is any amino acid
<220>
   <221> variant
   <222> (44)..(45)
   <223> xaa in position 44 to 45 is any amino acid
<220>
   <221> Variant
   <222> (47)..(48)
   <223> xaa in position 47 to 48 is any amino acid
<220>
   <221> Variant
   <222> (51)..(52)
   <223> xaa in position 51 to 52 is any amino acid
<220>
   <221> Variant
   <222> (55)..(56)
   <223> xaa in position 55 to 56 is any amino acid
<220>
   <221> Variant
   <222> (58)..(60)
   <223> xaa in position 58 to 60 is any amino acid
<220>
   <221> Variant
   <222> (61)..(61)
   <223> xaa in position 61 is any or no amino acid
<220>
   <221> Variant
   <222> (64)..(64)
   <223> xaa in position 64 is any amino acid
<220>
   <221> Variant
   <222> (69)..(69)
   <223> xaa in position 69 is any amino acid
<220>
   <221> variant
   <222> (72)..(72)
   <223> xaa in position 72 is any amino acid
<220>
   <221> Variant
   <222> (79)..(79)
   <223> xaa in position 79 is any amino acid
<220>
   <221> Variant
   <222> (86)..(86)
   <223> xaa in position 86 is any amino acid
<220>
   <221> Variant
   <222> (89)..(90)
   <223> xaa in position 89 to 90 is any amino acid
<220>
   <221> variant
   <222> (99)..(100)
   <223> xaa in position 99 to 100 is any amino acid
<220>
   <221> Variant
   <222> (104)..(109)
   <223> xaa in position 104 to 109 is any amino acid
<220>
   <221> Variant
   <222> (112)..(112)
   <223> xaa in position 112 is any amino acid
<220>
   <221> Variant
   <222> (114)..(114)
   <223> xaa in position 114 is any amino acid
<220>
   <221> Variant
   <222> (121)..(121)
   <223> xaa in position 121 is any amino acid
<220>
   <221> Variant
   <222> (124)..(124)
   <223> xaa in position 124 is any amino acid
<220>
   <221> Variant
   <222> (162)..(162)
   <223> xaa in position 162 is any amino acid
<220>
   <221> Variant
   <222> (164)..(165)
   <223> xaa in position 164 to 165 is any amino acid
<220>
   <221> Variant
   <222> (174)..(174)
   <223> xaa in position 174 is any amino acid
<220>
   <221> Variant
   <222> (195)..(197)
   <223> xaa in position 195 to 197 is any amino acid
<220>
   <221> Variant
   <222> (212)..(213)
   <223> xaa in position 212 to 213 is any amino acid
<220>
   <221> Variant
   <222> (216)..(216)
   <223> xaa in position 216 is any amino acid
<220>
   <221> Variant
   <222> (219)..(220)
   <223> xaa in position 219 to 220 is any amino acid
<220>
   <221> Variant
   <222> (223)..(223)
   <223> xaa in position 223 is any amino acid
<220>
   <221> Variant
   <222> (226)..(231)
   <223> xaa in position 226 to 231 is any amino acid
<220>
   <221> Variant
   <222> (234)..(238)
   <223> xaa in position 234 to 238 is any amino acid
<220>
   <221> Variant
   <222> (240)..(243)
   <223> xaa in position 240 to 243 is any amino acid
<220>
   <221> Variant
   <222> (245)..(245)
   <223> xaa in position 245 is any amino acid
<220>
   <221> Variant
   <222> (247)..(248)
   <223> xaa in position 247 to 248 is any amino acid
<220>
   <221> Variant
   <222> (253)..(253)
   <223> xaa in position 253 is any amino acid
<220>
   <221> Variant
   <222> (261)..(261)
   <223> xaa in position 261 is any amino acid
<220>
   <221> Variant
   <222> (264)..(265)
   <223> xaa in position 264 to 265 is any amino acid
<220>
   <221> Variant
   <222> (271)..(271)
   <223> xaa in position 271 is any amino acid
<400> 167
   Asp Xaa Tyr Xaa Xaa Xaa Xaa Xaa Xaa Xaa Gly Xaa Xaa Ile Xaa Xaa
   1               5                   10                  15
   Trp Xaa Asp Pro Asp Xaa Xaa Xaa Arg Xaa Xaa Xaa Glu Xaa Trp Xaa
               20                  25                  30
   Xaa Xaa Asp Phe Xaa Xaa Ala Xaa Xaa Ile Ala Xaa Xaa Tyr Xaa Xaa
           35                  40                  45
   Phe Val Xaa Xaa Gly Ser Xaa Xaa Met Xaa Xaa Xaa Xaa Pro Ala Xaa
       50                  55                  60
   Asp Pro Tyr Pro Xaa Lys Phe Xaa Tyr Asn Val Ser Gln Ile Xaa Leu
   65                  70                  75                  80
   Cys Ala Tyr Met Thr Xaa Glu Ala Xaa Xaa Leu Ala Tyr Arg Asn Gly
                   85                  90                  95
   Tyr Thr Xaa Xaa Pro Cys Asn Xaa Xaa Xaa Xaa Xaa Xaa Pro Pro Xaa
               100                 105                 110
   Ala Xaa Leu Leu Trp Leu Phe Tyr Xaa Ser Lys Xaa Trp Asp Phe Trp
           115                 120                 125
   Asp Thr Ile Phe Ile Val Leu Gly Lys Lys Trp Arg Gln Leu Ser Phe
       130                 135                 140
   Leu His Val Tyr His His Thr Thr Ile Phe Leu Phe Tyr Trp Leu Asn
   145                 150                 155                 160
   Ala Xaa Val Xaa Xaa Asp Gly Asp Ile Phe Leu Thr Ile Xaa Leu Asn
                   165                 170                 175
   Gly Phe Ile His Thr Val Met Tyr Thr Tyr Tyr Phe Ile Cys Met His
               180                 185                 190
   Thr Lys Xaa Xaa Xaa Thr Gly Lys Ser Leu Pro Ile Trp Trp Lys Ser
           195                 200                 205
   Ser Leu Thr Xaa Xaa Gln Leu Xaa Gln Phe Xaa Xaa Met Met Xaa Gln
       210                 215                 220
   Ala Xaa Xaa Xaa Xaa Xaa Xaa Gly Cys Xaa Xaa Xaa Xaa Xaa Arg Xaa
   225                 230                 235                 240
   Xaa Xaa Xaa Tyr Xaa Val Xaa Xaa Leu Ser Leu Phe Xaa Leu Phe Ala
                   245                 250                 255
   Gln Phe Phe Val Xaa Ser Tyr Xaa Xaa Pro Lys Lys Lys Lys Xaa Ala
               260                 265                 270
<210> 168
   <211> 59
   <212> PRT
   <213> Artificial sequence
<220>
   <221> Variant
   <222> (27)..(27)
   <223> xaa in position 27 is any amino acid
<220>
   <221> Variant
   <222> (29)..(29)
   <223> xaa in position 29 is any amino acid
<220>
   <221> Variant
   <222> (30)..(34)
   <223> xaa in position 30 to 34 is any or no amino acid
<220>
   <221> Variant
   <222> (39)..(39)
   <223> xaa in position 39 is Leu or val
<400> 168
<210> 169
   <211> 60
   <212> PRT
   <213> Artificial sequence
<220>
   <221> Variant
   <222> (7)..(7)
   <223> xaa in position 7 is Phe or Met
<220>
   <221> variant
   <222> (14)..(14)
   <223> xaa in position 14 is Ile or Val
<220>
   <221> Variant
   <222> (17)..(17)
   <223> xaa in position 17 is any amino acid
<220>
   <221> Variant
   <222> (18)..(18)
   <223> xaa in position 18 is any or no amino acid
<220>
   <221> Variant
   <222> (20)..(20)
   <223> xaa in position 20 is any or no amino acid
<220>
   <221> Variant
   <222> (28)..(28)
   <223> xaa in position 28 is Phe or Val
<220>
   <221> Variant
   <222> (29)..(29)
   <223> xaa in position 29 is any amino acid
<220>
   <221> Variant
   <222> (33)..(33)
   <223> xaa in position 33 is Asp or His
<220>
   <221> Variant
   <222> (34)..(34)
   <223> xaa in position 34 is Phe or Trp
<220>
   <221> Variant
   <222> (35)..(35)
   <223> xaa in position 35 is Asp or Asn
<220>
   <221> Variant
   <222> (36)..(36)
   <223> xaa in position 36 is Phe or Val
<220>
   <221> Variant
   <222> (37)..(37)
   <223> xaa in position 37 is Glu or Asn
<220>
   <221> variant
   <222> (38)..(38)
   <223> xaa in position 38 is Asp or Lys
<220>
   <221> Variant
   <222> (41)..(41)
   <223> xaa in position 41 is Ile or Val
<220>
   <221> Variant
   <222> (43)..(43)
   <223> xaa in position 43 is any amino acid
<220>
   <221> Variant
   <222> (50)..(50)
   <223> xaa in position 50 is Ile or Val
<220>
   <221> Variant
   <222> (53)..(53)
   <223> xaa in position 53 is Ile or Val
<400> 169
<210> 170
   <211> 39
   <212> PRT
   <213> Artificial sequence
<220>
   <221> Variant
   <222> (11)..(11)
   <223> xaa in position 11 is Ala or Ser
<220>
   <221> Variant
   <222> (12)..(12)
   <223> xaa in position 12 is Phe or Met
<220>
   <221> Variant
   <222> (15)..(15)
   <223> xaa in position 15 is Leu or Val
<220>
   <221> Variant
   <222> (18)..(18)
   <223> xaa in position 18 is any or no amino acid
<220>
   <221> Variant
   <222> (20)..(20)
   <223> xaa in position 20 is any amino acid
<220>
   <221> Variant
   <222> (22)..(22)
   <223> xaa in position 22 is any amino acid
<220>
   <221> Variant
   <222> (23)..(23)
   <223> xaa in position 23 is any or no amino acid
<220>
   <221> Variant
   <222> (26)..(27)
   <223> xaa in position 26 to 27 is any amino acid
<220>
   <221> Variant
   <222> (28)..(28)
   <223> xaa in position 28 is Ile or Leu
<220>
   <221> Variant
   <222> (29)..(29)
   <223> xaa in position 29 is Leu or Val
<220>
   <221> Variant
   <222> (30)..(30)
   <223> xaa in position 30 is Phe or Tyr
<220>
   <221> Variant
   <222> (31)..(31)
   <223> xaa in position 31 is His or Lys
<220>
   <221> Variant
   <222> (34)..(34)
   <223> xaa in position 34 is Ala or Asp
<220>
   <221> Variant
   <222> (35)..(35)
   <223> xaa in position 35 is any amino acid
<220>
   <221> Variant
   <222> (36)..(36)
   <223> xaa in position 36 is Pro or Val
<220>
   <221> Variant
   <222> (37)..(38)
   <223> xaa in position 37 to 38 is any amino acid
<400> 170
   Leu Pro Ile Trp Trp Lys Ser Ser Leu Thr xaa xaa Gln Leu xaa Gln
     1               5                  10                  15
   Phe xaa Ile xaa Met xaa xaa Gln Ala xaa xaa xaa xaa xaa xaa Gly
               20                  25                  30
   Cys xaa xaa xaa xaa xaa Arg
           35
<210> 171
   <211> 831
   <212> DNA
   <213> Traustochytrium sp.
<220>
   <221> CDS
   <222> (1)..(831)
<400> 171
   atg gac gtc gtc gag cag caa tgg cgc cgc ttc gtg gac gcc gtg gac      48
   Met Asp Val Val Glu Gln Gln Trp Arg Arg Phe Val Asp Ala Val Asp
   1               5                   10                  15
   aac gga atc gtg gag ttc atg gag cat gag aag ccc aac aag ctg aac      96
   Asn Gly Ile Val Glu Phe Met Glu His Glu Lys Pro Asn Lys Leu Asn
               20                  25                  30
   gag ggc aag ctc ttc acc tcg acc gag gag atg atg gcg ctt atc gtc      144
   Glu Gly Lys Leu Phe Thr Ser Thr Glu Glu Met Met Ala Leu Ile Val
           35                  40                  45
   ggc tac ctg gcg ttc gtg gtc ctc ggg tcc gcc ttc atg aag gcc ttt      192
   Gly Tyr Leu Ala Phe Val Val Leu Gly Ser Ala Phe Met Lys Ala Phe
       50                  55                  60
   gtc gat aag cct ttc gag ctc aag ttc ctc aag ctc gtg cac aac atc      240
   Val Asp Lys Pro Phe Glu Leu Lys Phe Leu Lys Leu Val His Asn Ile
   65                  70                  75                  80
   ttc ctc acc ggt ctg tcc atg tac atg gcc acc gag tgc gcg cgc cag      288
   Phe Leu Thr Gly Leu Ser Met Tyr Met Ala Thr Glu Cys Ala Arg Gln
                   85                  90                  95
   gca tac ctc ggc ggc tac aag ctc ttt ggc aac ccg atg gag aag ggc      336
   Ala Tyr Leu Gly Gly Tyr Lys Leu Phe Gly Asn Pro Met Glu Lys Gly
               100                 105                 110
   acc gag tcg cac gcc ccg ggc atg gcc aac atc atc tac atc ttc tac      384
   Thr Glu Ser His Ala Pro Gly Met Ala Asn Ile Ile Tyr Ile Phe Tyr
           115                 120                 125
   gtg agc aag ttc ctc gaa ttc ctc gac acc gtc ttc atg atc ctc ggc      432
   Val Ser Lys Phe Leu Glu Phe Leu Asp Thr Val Phe Met Ile Leu Gly
       130                 135                 140
   aag aag tgg aag cag ctc agc ttt ctc cac gtc tac cac cac gcg agc      480
   Lys Lys Trp Lys Gln Leu Ser Phe Leu His Val Tyr His His Ala Ser
   145                 150                 155                 160
   atc agc ttc atc tgg ggc atc atc gcc cgc ttc gcg ccc ggt ggc gac      528
   Ile Ser Phe Ile Trp Gly Ile Ile Ala Arg Phe Ala Pro Gly Gly Asp
                   165                 170                 175
   gcc tac ttc tct acc atc ctc aac agc agc gtg cat gtc gtg ctc tac      576
   Ala Tyr Phe Ser Thr Ile Leu Asn Ser Ser Val His Val Val Leu Tyr
               180                 185                 190
   ggc tac tac gcc tcg acc acc ctc ggc tac acc ttc atg cgc ccg ctg      624
   Gly Tyr Tyr Ala Ser Thr Thr Leu Gly Tyr Thr Phe Met Arg Pro Leu
           195                 200                 205
   cgc ccg tac att acc acc att cag ctc acg cag ttc atg gcc atg gtc      672
   Arg Pro Tyr Ile Thr Thr Ile Gln Leu Thr Gln Phe Met Ala Met Val
       210                 215                 220
   gtc cag tcc gtc tat gac tac tac aac ccc tgc gac tac ccg cag ccc      720
   Val Gln Ser Val Tyr Asp Tyr Tyr Asn Pro Cys Asp Tyr Pro Gln Pro
   225                 230                 235                 240
   ctc gtc aag ctg ctc ttc tgg tac atg ctc acc atg ctc ggc ctc ttc      768
   Leu Val Lys Leu Leu Phe Trp Tyr Met Leu Thr Met Leu Gly Leu Phe
                   245                 250                 255
   ggc aac ttc ttc gtg cag cag tac ctc aag ccc aag gcg ccc aag aag      816
   Gly Asn Phe Phe Val Gln Gln Tyr Leu Lys Pro Lys Ala Pro Lys Lys
               260                 265                 270
   cag aag acc atc taa                                                  831
   Gln Lys Thr Ile
           275
<210> 172
   <211> 276
   <212> PRT
   <213> Traustochytrium sp.
<400> 172
   Met Asp Val Val Glu Gln Gln Trp Arg Arg Phe Val Asp Ala Val Asp
   1               5                   10                  15
   Asn Gly Ile Val Glu Phe Met Glu His Glu Lys Pro Asn Lys Leu Asn
               20                  25                  30
   Glu Gly Lys Leu Phe Thr Ser Thr Glu Glu Met Met Ala Leu Ile Val
           35                  40                  45
   Gly Tyr Leu Ala Phe Val Val Leu Gly Ser Ala Phe Met Lys Ala Phe
       50                  55                  60
   Val Asp Lys Pro Phe Glu Leu Lys Phe Leu Lys Leu Val His Asn Ile
   65                  70                  75                  80
   Phe Leu Thr Gly Leu Ser Met Tyr Met Ala Thr Glu Cys Ala Arg Gln
                   85                  90                  95
   Ala Tyr Leu Gly Gly Tyr Lys Leu Phe Gly Asn Pro Met Glu Lys Gly
               100                 105                 110
   Thr Glu Ser His Ala Pro Gly Met Ala Asn Ile Ile Tyr Ile Phe Tyr
           115                 120                 125
   Val Ser Lys Phe Leu Glu Phe Leu Asp Thr Val Phe Met Ile Leu Gly
       130                 135                 140
   Lys Lys Trp Lys Gln Leu Ser Phe Leu His Val Tyr His His Ala Ser
   145                 150                 155                 160
   Ile Ser Phe Ile Trp Gly Ile Ile Ala Arg Phe Ala Pro Gly Gly Asp
                   165                 170                 175
   Ala Tyr Phe Ser Thr Ile Leu Asn Ser Ser Val His Val Val Leu Tyr
               180                 185                 190
   Gly Tyr Tyr Ala Ser Thr Thr Leu Gly Tyr Thr Phe Met Arg Pro Leu
           195                 200                 205
   Arg Pro Tyr Ile Thr Thr Ile Gln Leu Thr Gln Phe Met Ala Met Val
       210                 215                 220
   Val Gln Ser Val Tyr Asp Tyr Tyr Asn Pro Cys Asp Tyr Pro Gln Pro
   225                 230                 235                 240
   Leu Val Lys Leu Leu Phe Trp Tyr Met Leu Thr Met Leu Gly Leu Phe
                   245                 250                 255
   Gly Asn Phe Phe Val Gln Gln Tyr Leu Lys Pro Lys Ala Pro Lys Lys
               260                 265                 270
   Gln Lys Thr Ile
           275
<210> 173
   <211> 1047
   <212> DNA
   <213> Marchantia polymorpha
<220>
   <221> CDS
   <222> (1)..(1047)
<400> 173
   atg gcg acg aag agc ggg agc gga ttg ctc gag tgg ata gca gta gcg        48
   Met Ala Thr Lys Ser Gly Ser Gly Leu Leu Glu Trp Ile Ala Val Ala
   1               5                   10                  15
   gcg aag atg aag caa gct cgg agc agc ccc gag ggt gag atc gtg ggt        96
   Ala Lys Met Lys Gln Ala Arg Ser Ser Pro Glu Gly Glu Ile Val Gly
               20                  25                  30
   ggg aat agg atg ggc tct gga aac gga gct gag tgg acc acg agt ctg       144
   Gly Asn Arg Met Gly Ser Gly Asn Gly Ala Glu Trp Thr Thr Ser Leu
           35                  40                  45
   att cat gca ttt ttg aat gcc acg aat ggg aag agc ggc ggt gct tcg       192
   Ile His Ala Phe Leu Asn Ala Thr Asn Gly Lys Ser Gly Gly Ala Ser
       50                  55                  60
   aaa gtg agg cct ctc gag gag aga atc ggg gag gcg gtg ttc aga gtt       240
   Lys Val Arg Pro Leu Glu Glu Arg Ile Gly Glu Ala Val Phe Arg Val
   65                  70                  75                  80
   ctt gaa gat gtc gtg ggc gtg gat att agg aag ccg aat cct gtc acg       288
   Leu Glu Asp Val Val Gly Val Asp Ile Arg Lys Pro Asn Pro Val Thr
                   85                  90                  95
   aag gac ctt ccg atg gtc gag agt ccc gtg ccc gtg ttg gcc tgc att       336
   Lys Asp Leu Pro Met Val Glu Ser Pro Val Pro Val Leu Ala Cys Ile
               100                 105                 110
   tct ctg tac ttg ctc gtg gtg tgg ctt tgg tct tct cac att aag gcg       384
   Ser Leu Tyr Leu Leu Val Val Trp Leu Trp Ser Ser His Ile Lys Ala
           115                 120                 125
   tct ggc caa aag ccc agg aag gag gac ccg ctc gcc ctg cgt tgc ctt       432
   Ser Gly Gln Lys Pro Arg Lys Glu Asp Pro Leu Ala Leu Arg Cys Leu
       130                 135                 140
   gtg att gcc cac aat ctg ttc ctg tgt tgc ttg agc ttg ttc atg tgc       480
   Val Ile Ala His Asn Leu Phe Leu Cys Cys Leu Ser Leu Phe Met Cys
   145                 150                 155                 160
   gtc ggt ctc att gcc gca gct cga cat tac ggg tat agt gta tgg ggg      528
   Val Gly Leu Ile Ala Ala Ala Arg His Tyr Gly Tyr Ser Val Trp Gly
                   165                 170                 175
   aac tac tac aga gaa aga gaa ccc gca atg aat ttg ctc att tac gtg      576
   Asn Tyr Tyr Arg Glu Arg Glu Pro Ala Met Asn Leu Leu Ile Tyr Val
               180                 185                 190
   ttc tac atg tcg aag ctg tac gaa ttt atg gac acg gcc att atg tta      624
   Phe Tyr Met Ser Lys Leu Tyr Glu Phe Met Asp Thr Ala Ile Met Leu
           195                 200                 205
   ttc aga aga aat ctg cga caa gtc acg tac ttg cat gta tat cac cac      672
   Phe Arg Arg Asn Leu Arg Gln Val Thr Tyr Leu His Val Tyr His His
       210                 215                 220
   gca agc atc gca atg att tgg tgg ata att tgc tat cgg ttt cca gga      720
   Ala Ser Ile Ala Met Ile Trp Trp Ile Ile Cys Tyr Arg Phe Pro Gly
   225                 230                 235                 240
   gct gat tcg tat ttc tcc gca gca ttc aat tcc tgt atc cat gta gcg      768
   Ala Asp Ser Tyr Phe Ser Ala Ala Phe Asn Ser Cys Ile His Val Ala
                   245                 250                 255
   atg tac ctg tat tat cta ctc gcg gca acc gtc gcc aga gac gaa aag      816
   Met Tyr Leu Tyr Tyr Leu Leu Ala Ala Thr Val Ala Arg Asp Glu Lys
               260                 265                 270
   cgg aga cgc aaa tat ctc ttc tgg gga aag tat ctg acc atc ata caa      864
   Arg Arg Arg Lys Tyr Leu Phe Trp Gly Lys Tyr Leu Thr Ile Ile Gln
           275                 280                 285
   atg ctt cag ttt ttg tcc ttc att ggg cag gcg att tat gca atg tgg      912
   Met Leu Gln Phe Leu Ser Phe Ile Gly Gln Ala Ile Tyr Ala Met Trp
       290                 295                 300
   aag ttt gaa tac tat ccc aag ggc ttt ggc agg atg ttg ttc ttt tac      960
   Lys Phe Glu Tyr Tyr Pro Lys Gly Phe Gly Arg Met Leu Phe Phe Tyr
   305                 310                 315                 320
   tct gta tca ttg ttg gca ttt ttc ggc aac ttc ttt gtc aaa aag tat     1008
   Ser Val Ser Leu Leu Ala Phe Phe Gly Asn Phe Phe Val Lys Lys Tyr
                   325                 330                 335
   tcg aac gct tca cag cct aag aca gtt aaa gtg gag tga                 1047
   Ser Asn Ala Ser Gln Pro Lys Thr Val Lys Val Glu
               340                 345
<210> 174
   <211> 348
   <212> PRT
   <213> Marchantia polymorpha
<400> 174
   Met Ala Thr Lys Ser Gly Ser Gly Leu Leu Glu Trp Ile Ala Val Ala
   1               5                   10                  15
   Ala Lys Met Lys Gln Ala Arg Ser Ser Pro Glu Gly Glu Ile Val Gly
               20                  25                  30
   Gly Asn Arg Met Gly Ser Gly Asn Gly Ala Glu Trp Thr Thr Ser Leu
           35                  40                  45
   Ile His Ala Phe Leu Asn Ala Thr Asn Gly Lys Ser Gly Gly Ala Ser
       50                  55                  60
   Lys Val Arg Pro Leu Glu Glu Arg Ile Gly Glu Ala Val Phe Arg Val
   65                  70                  75                  80
   Leu Glu Asp Val Val Gly Val Asp Ile Arg Lys Pro Asn Pro Val Thr
                   85                  90                  95
   Lys Asp Leu Pro Met Val Glu Ser Pro Val Pro Val Leu Ala Cys Ile
               100                 105                 110
   Ser Leu Tyr Leu Leu Val Val Trp Leu Trp Ser Ser His Ile Lys Ala
           115                 120                 125
   Ser Gly Gln Lys Pro Arg Lys Glu Asp Pro Leu Ala Leu Arg Cys Leu
       130                 135                 140
   Val Ile Ala His Asn Leu Phe Leu Cys Cys Leu Ser Leu Phe Met Cys
   145                 150                 155                 160
   Val Gly Leu Ile Ala Ala Ala Arg His Tyr Gly Tyr Ser Val Trp Gly
                   165                 170                 175
   Asn Tyr Tyr Arg Glu Arg Glu Pro Ala Met Asn Leu Leu Ile Tyr Val
               180                 185                 190
   Phe Tyr Met Ser Lys Leu Tyr Glu Phe Met Asp Thr Ala Ile Met Leu
           195                 200                 205
   Phe Arg Arg Asn Leu Arg Gln Val Thr Tyr Leu His Val Tyr His His
       210                 215                 220
   Ala Ser Ile Ala Met Ile Trp Trp Ile Ile Cys Tyr Arg Phe Pro Gly
   225                 230                 235                 240
   Ala Asp Ser Tyr Phe Ser Ala Ala Phe Asn Ser Cys Ile His Val Ala
                   245                 250                 255
   Met Tyr Leu Tyr Tyr Leu Leu Ala Ala Thr Val Ala Arg Asp Glu Lys
               260                 265                 270
   Arg Arg Arg Lys Tyr Leu Phe Trp Gly Lys Tyr Leu Thr Ile Ile Gln
           275                 280                 285
   Met Leu Gln Phe Leu Ser Phe Ile Gly Gln Ala Ile Tyr Ala Met Trp
       290                 295                 300
   Lys Phe Glu Tyr Tyr Pro Lys Gly Phe Gly Arg Met Leu Phe Phe Tyr
   305                 310                 315                 320
   Ser Val Ser Leu Leu Ala Phe Phe Gly Asn Phe Phe Val Lys Lys Tyr
                   325                 330                 335
   Ser Asn Ala Ser Gln Pro Lys Thr Val Lys Val Glu
               340                 345
<210> 175
   <211> 831
   <212> DNA
   <213> Thraustochytrium sp
<220>
   <221> CDS
   <222> (1)..(831)
<400> 175
   atg gat gtc gtc gag cag caa tgg cgc cgc ttc gtg gac gcc gtg gac      48
   Met Asp Val Val Glu Gln Gln Trp Arg Arg Phe Val Asp Ala Val Asp
   1               5                   10                  15
   aac gga atc gtg gag ttc atg gag cat gag gag ccc aac aag ctg aac      96
   Asn Gly Ile Val Glu Phe Met Glu His Glu Glu Pro Asn Lys Leu Asn
               20                  25                  30
   gag ggc aag ctc tcc acc tcg acc gag gag atg atg gcg ctt atc gtc     144
   Glu Gly Lys Leu Ser Thr Ser Thr Glu Glu Met Met Ala Leu Ile Val
           35                  40                  45
   ggc tac ctg gcg ttc gtg gtc ctc ggg tcc gcc ttc atg aag gcc ttt     192
   Gly Tyr Leu Ala Phe Val Val Leu Gly Ser Ala Phe Met Lys Ala Phe
       50                  55                  60
   gtc gat aag cct ttc gag ctc aag ttc ctc aag ctc gtg cac aac atc     240
   Val Asp Lys Pro Phe Glu Leu Lys Phe Leu Lys Leu Val His Asn Ile
   65                  70                  75                  80
   ttc ctc acc ggt ctg tcc atg tac atg gcc acc gag tgc gcg cgc cag     288
   Phe Leu Thr Gly Leu Ser Met Tyr Met Ala Thr Glu Cys Ala Arg Gln
                   85                  90                  95
   gca tac ctc ggc ggc tac aag ctc ttt ggc aac ccg atg gag aag ggc     336
   Ala Tyr Leu Gly Gly Tyr Lys Leu Phe Gly Asn Pro Met Glu Lys Gly
               100                 105                 110
   acc gag tcg cac gcc ccg ggc atg gcc aac atc atc tac atc ttc tac     384
   Thr Glu Ser His Ala Pro Gly Met Ala Asn Ile Ile Tyr Ile Phe Tyr
           115                 120                 125
   gtg agc aag ttc ctc gaa ttc ctc gac acc gtc ttc atg atc ctc ggc     432
   Val Ser Lys Phe Leu Glu Phe Leu Asp Thr Val Phe Met Ile Leu Gly
       130                 135                 140
   aag aag tgg aag cag ctc agc ttt ctc cac gtc tac cac cac gcg agc     480
   Lys Lys Trp Lys Gln Leu Ser Phe Leu His Val Tyr His His Ala Ser
   145                 150                 155                 160
   atc agc ttc atc tgg ggc atc atc gcc cgc ttc gcg ccc ggt ggc gac     528
   Ile Ser Phe Ile Trp Gly Ile Ile Ala Arg Phe Ala Pro Gly Gly Asp
                   165                 170                 175
   gcc tac ttc tct acc atc ctc aac agc agc gtg cat gtc gtg ctc tac     576
   Ala Tyr Phe Ser Thr Ile Leu Asn Ser Ser Val His Val Val Leu Tyr
               180                 185                 190
   ggc tac tac gcc tcg acc acc ctc ggc tac acc ttc atg cgc ccg ctg     624
   Gly Tyr Tyr Ala Ser Thr Thr Leu Gly Tyr Thr Phe Met Arg Pro Leu
           195                 200                 205
   cgc ccg tac att acc acc att cag ctc acg cag ttc atg gcc atg gtc     672
   Arg Pro Tyr Ile Thr Thr Ile Gln Leu Thr Gln Phe Met Ala Met Val
       210                 215                 220
   gtc cag tcc gtc tat gac tac tac aac ccc tgc gac tac ccg cag ccc     720
   Val Gln Ser Val Tyr Asp Tyr Tyr Asn Pro Cys Asp Tyr Pro Gln Pro
   225                 230                 235                 240
   ctc gtc aag ctg ctc ttc tgg tac atg ctc acc atg ctc ggc ctc ttc     768
   Leu Val Lys Leu Leu Phe Trp Tyr Met Leu Thr Met Leu Gly Leu Phe
                   245                 250                 255
   ggc aac ttc ttc gtg cag cag tac ctc aag ccc aag gcg ccc aag aag     816
   Gly Asn Phe Phe Val Gln Gln Tyr Leu Lys Pro Lys Ala Pro Lys Lys
               260                 265                 270
   cag aag acc atc taa                                                 831
   Gln Lys Thr Ile
           275
<210> 176
   <211> 276
   <212> PRT
   <213> Thraustochytrium sp
<400> 176
   Met Asp Val Val Glu Gln Gln Trp Arg Arg Phe Val Asp Ala Val Asp
   1               5                   10                  15
   Asn Gly Ile Val Glu Phe Met Glu His Glu Glu Pro Asn Lys Leu Asn
               20                  25                  30
   Glu Gly Lys Leu Ser Thr Ser Thr Glu Glu Met Met Ala Leu Ile Val
           35                  40                  45
   Gly Tyr Leu Ala Phe Val Val Leu Gly Ser Ala Phe Met Lys Ala Phe
       50                  55                  60
   Val Asp Lys Pro Phe Glu Leu Lys Phe Leu Lys Leu Val His Asn Ile
   65                  70                  75                  80
   Phe Leu Thr Gly Leu Ser Met Tyr Met Ala Thr Glu Cys Ala Arg Gln
                   85                  90                  95
   Ala Tyr Leu Gly Gly Tyr Lys Leu Phe Gly Asn Pro Met Glu Lys Gly
               100                 105                 110
   Thr Glu Ser His Ala Pro Gly Met Ala Asn Ile Ile Tyr Ile Phe Tyr
           115                 120                 125
   Val Ser Lys Phe Leu Glu Phe Leu Asp Thr Val Phe Met Ile Leu Gly
       130                 135                 140
   Lys Lys Trp Lys Gln Leu Ser Phe Leu His Val Tyr His His Ala Ser
   145                 150                 155                 160
   Ile Ser Phe Ile Trp Gly Ile Ile Ala Arg Phe Ala Pro Gly Gly Asp
                   165                 170                 175
   Ala Tyr Phe Ser Thr Ile Leu Asn Ser Ser Val His Val Val Leu Tyr
               180                 185                 190
   Gly Tyr Tyr Ala Ser Thr Thr Leu Gly Tyr Thr Phe Met Arg Pro Leu
           195                 200                 205
   Arg Pro Tyr Ile Thr Thr Ile Gln Leu Thr Gln Phe Met Ala Met Val
       210                 215                 220
   Val Gln Ser Val Tyr Asp Tyr Tyr Asn Pro Cys Asp Tyr Pro Gln Pro
   225                 230                 235                 240
   Leu Val Lys Leu Leu Phe Trp Tyr Met Leu Thr Met Leu Gly Leu Phe
                   245                 250                 255
   Gly Asn Phe Phe Val Gln Gln Tyr Leu Lys Pro Lys Ala Pro Lys Lys
               260                 265                 270
   Gln Lys Thr Ile
           275
<210> 177
   <211> 879
   <212> DNA
   <213> Ostreococcus tauri
<220>
   <221> CDS
   <222> (1)..(879)
<400> 177
   atg agt ggc tta cgt gca ccc aac ttt tta cac aga ttc tgg aca aag     48
   Met Ser Gly Leu Arg Ala Pro Asn Phe Leu His Arg Phe Trp Thr Lys
   1               5                   10                  15
   tgg gac tac gcg att tcc aaa gtc gtc ttc acg tgt gcc gac agt ttt     96
   Trp Asp Tyr Ala Ile Ser Lys Val Val Phe Thr Cys Ala Asp Ser Phe
               20                  25                  30
   cag tgg gac atc ggg cca gtg agt tcg agt acg gcg cat tta ccc gcc    144
   Gln Trp Asp Ile Gly Pro Val Ser Ser Ser Thr Ala His Leu Pro Ala
           35                  40                  45
   att gaa tcc cct acc cca ctg gtg act agc ctc ttg ttc tac tta gtc    192
   Ile Glu Ser Pro Thr Pro Leu Val Thr Ser Leu Leu Phe Tyr Leu Val
       50                  55                  60
   aca gtt ttc ttg tgg tat ggt cgt tta acc agg agt tca gac aag aaa    240
   Thr Val Phe Leu Trp Tyr Gly Arg Leu Thr Arg Ser Ser Asp Lys Lys
   65                  70                  75                  80
   att aga gag cct acg tgg tta aga aga ttc ata ata tgt cat aat gcg    288
   Ile Arg Glu Pro Thr Trp Leu Arg Arg Phe Ile Ile Cys His Asn Ala
                   85                  90                  95
   ttc ttg ata gtc ctc agt ctt tac atg tgc ctt ggt tgt gtg gcc caa    336
   Phe Leu Ile Val Leu Ser Leu Tyr Met Cys Leu Gly Cys Val Ala Gln
               100                 105                 110
   gcg tat cag aat gga tat act tta tgg ggt aat gaa ttc aag gcc acg    384
   Ala Tyr Gln Asn Gly Tyr Thr Leu Trp Gly Asn Glu Phe Lys Ala Thr
           115                 120                 125
   gaa act cag ctt gct ctc tac att tac att ttt tac gta agt aaa ata    432
   Glu Thr Gln Leu Ala Leu Tyr Ile Tyr Ile Phe Tyr Val Ser Lys Ile
       130                 135                 140
   tac gag ttt gta gat act tac att atg ctt ctc aag aat aac ttg cgg    480
   Tyr Glu Phe Val Asp Thr Tyr Ile Met Leu Leu Lys Asn Asn Leu Arg
   145                 150                 155                 160
   caa gta agt ttc cta cac att tat cac cac agc acg att tcc ttt att    528
   Gln Val Ser Phe Leu His Ile Tyr His His Ser Thr Ile Ser Phe Ile
                   165                 170                 175
   tgg tgg atc att gct cgg agg gct ccg ggt ggt gat gct tac ttc agc    576
   Trp Trp Ile Ile Ala Arg Arg Ala Pro Gly Gly Asp Ala Tyr Phe Ser
               180                 185                 190
   gcg gcc ttg aac tca tgg gta cac gtg tgc atg tac acc tat tat cta    624
   Ala Ala Leu Asn Ser Trp Val His Val Cys Met Tyr Thr Tyr Tyr Leu
           195                 200                 205
   tta tca acc ctt att gga aaa gaa gat cct aag cgt tcc aac tac ctt    672
   Leu Ser Thr Leu Ile Gly Lys Glu Asp Pro Lys Arg Ser Asn Tyr Leu
       210                 215                 220
   tgg tgg ggt cgc cac cta acg caa atg cag atg ctt cag ttt ttc ttc    720
   Trp Trp Gly Arg His Leu Thr Gln Met Gln Met Leu Gln Phe Phe Phe
   225                 230                 235                 240
   aac gta ctt caa gcg ttg tac tgc gct tcg ttc tct acg tat ccc aag    768
   Asn Val Leu Gln Ala Leu Tyr Cys Ala Ser Phe Ser Thr Tyr Pro Lys
                   245                 250                 255
   ttt ttg tcc aaa att ctg ctc gtc tat atg atg agc ctt ctc ggc ttg    816
   Phe Leu Ser Lys Ile Leu Leu Val Tyr Met Met Ser Leu Leu Gly Leu
               260                 265                 270
   ttt ggg cat ttc tac tat tcc aag cac ata gca gca gct aag ctc cag    864
   Phe Gly His Phe Tyr Tyr Ser Lys His Ile Ala Ala Ala Lys Leu Gln
           275                 280                 285
   aaa aaa cag cag tga                                                879
   Lys Lys Gln Gln
       290
<210> 178
   <211> 292
   <212> PRT
   <213> Ostreococcus tauri
<400> 178
   Met Ser Gly Leu Arg Ala Pro Asn Phe Leu His Arg Phe Trp Thr Lys
   1               5                   10                  15
   Trp Asp Tyr Ala Ile Ser Lys Val Val Phe Thr Cys Ala Asp Ser Phe
               20                  25                  30
   Gln Trp Asp Ile Gly Pro Val Ser Ser Ser Thr Ala His Leu Pro Ala
           35                  40                  45
   Ile Glu Ser Pro Thr Pro Leu Val Thr Ser Leu Leu Phe Tyr Leu Val
       50                  55                  60
   Thr Val Phe Leu Trp Tyr Gly Arg Leu Thr Arg Ser Ser Asp Lys Lys
   65                  70                  75                  80
   Ile Arg Glu Pro Thr Trp Leu Arg Arg Phe Ile Ile Cys His Asn Ala
                   85                  90                  95
   Phe Leu Ile Val Leu Ser Leu Tyr Met Cys Leu Gly Cys Val Ala Gln
               100                 105                 110
   Ala Tyr Gln Asn Gly Tyr Thr Leu Trp Gly Asn Glu Phe Lys Ala Thr
           115                 120                 125
   Glu Thr Gln Leu Ala Leu Tyr Ile Tyr Ile Phe Tyr Val Ser Lys Ile
       130                 135                 140
   Tyr Glu Phe Val Asp Thr Tyr Ile Met Leu Leu Lys Asn Asn Leu Arg
   145                 150                 155                 160
   Gln Val Ser Phe Leu His Ile Tyr His His Ser Thr Ile Ser Phe Ile
                   165                 170                 175
   Trp Trp Ile Ile Ala Arg Arg Ala Pro Gly Gly Asp Ala Tyr Phe Ser
               180                 185                 190
   Ala Ala Leu Asn Ser Trp Val His Val Cys Met Tyr Thr Tyr Tyr Leu
           195                 200                 205
   Leu Ser Thr Leu Ile Gly Lys Glu Asp Pro Lys Arg Ser Asn Tyr Leu
       210                 215                 220
   Trp Trp Gly Arg His Leu Thr Gln Met Gln Met Leu Gln Phe Phe Phe
   225                 230                 235                 240
   Asn Val Leu Gln Ala Leu Tyr Cys Ala Ser Phe Ser Thr Tyr Pro Lys
                   245                 250                 255
   Phe Leu Ser Lys Ile Leu Leu Val Tyr Met Met Ser Leu Leu Gly Leu
               260                 265                 270
   Phe Gly His Phe Tyr Tyr Ser Lys His Ile Ala Ala Ala Lys Leu Gln
           275                 280                 285
   Lys Lys Gln Gln
       290

<210> 179
   <211> 873
   <212> DNA
   <213> Marchantia polymorpha
<220>
   <221> CDS
   <222> (1)..(873)
<400> 179
   atg gag gcg tac gag atg gtg gat agt ttt gtg tcg aag acg gtt ttc      48
   Met Glu Ala Tyr Glu Met Val Asp Ser Phe Val Ser Lys Thr Val Phe
   1               5                   10                  15
   gaa acg ctg cag aga ctg agg ggc gga gtc gtg ttg acg gaa tct gcg      96
   Glu Thr Leu Gln Arg Leu Arg Gly Gly Val Val Leu Thr Glu Ser Ala
               20                  25                  30
   atc acc aaa ggt ttg cca tgc gtc gat agc ccg acg ccg atc gtt ctt      144
   Ile Thr Lys Gly Leu Pro Cys Val Asp Ser Pro Thr Pro Ile Val Leu
           35                  40                  45
   ggg ttg tcg tcc tac ttg aca ttc gtg ttt ctc ggg ctc att gtc atc      192
   Gly Leu Ser Ser Tyr Leu Thr Phe Val Phe Leu Gly Leu Ile Val Ile
       50                  55                  60
   aag agc ctg gat ctt aag ccc cgc tcc aag gag ccc gcc att ttg aac      240
   Lys Ser Leu Asp Leu Lys Pro Arg Ser Lys Glu Pro Ala Ile Leu Asn
   65                  70                  75                  80
   ctg ttt gtg atc ttc cac aac ttc gtc tgc ttc gca ctc agt ctg tac      288
   Leu Phe Val Ile Phe His Asn Phe Val Cys Phe Ala Leu Ser Leu Tyr
                   85                  90                  95
   atg tgc gtg gga att gtc cgt caa gct atc ctc aac agg tac tct ctg      336
   Met Cys Val Gly Ile Val Arg Gln Ala Ile Leu Asn Arg Tyr Ser Leu
               100                 105                 110
   tgg ggc aat gcg tac aat ccc aaa gaa gtt caa atg ggc cac ctg ctc      384
   Trp Gly Asn Ala Tyr Asn Pro Lys Glu Val Gln Met Gly His Leu Leu
           115                 120                 125
   tac att ttc tac atg tca aag tac atc gag ttt atg gac acg gtc att      432
   Tyr Ile Phe Tyr Met Ser Lys Tyr Ile Glu Phe Met Asp Thr Val Ile
       130                 135                 140
   atg att ttg aag cgc aac acg cgc cag atc act gtg ttg cat gtg tac      480
   Met Ile Leu Lys Arg Asn Thr Arg Gln Ile Thr Val Leu His Val Tyr
   145                 150                 155                 160
   cac cac gca tcc atc tcc ttc atc tgg tgg atc atc gcc tac cat gct      528
   His His Ala Ser Ile Ser Phe Ile Trp Trp Ile Ile Ala Tyr His Ala
                   165                 170                 175
   cct ggc ggt gaa gct tat ttc tct gcc gca ttg aac tcc gga gta cat      576
   Pro Gly Gly Glu Ala Tyr Phe Ser Ala Ala Leu Asn Ser Gly Val His
               180                 185                 190
   gtg ctc atg tac ctc tac tac ctt ttg gca gca act ctg gga aag aac      624
   Val Leu Met Tyr Leu Tyr Tyr Leu Leu Ala Ala Thr Leu Gly Lys Asn
           195                 200                 205
   gag aaa gct cgc cgc aag tac cta tgg tgg gga aaa tac ttg aca cag      672
   Glu Lys Ala Arg Arg Lys Tyr Leu Trp Trp Gly Lys Tyr Leu Thr Gln
       210                 215                 220
   ctg cag atg ttc cag ttt gtc ctt aac atg att cag gct tac tac gat      720
   Leu Gln Met Phe Gln Phe Val Leu Asn Met Ile Gln Ala Tyr Tyr Asp
   225                 230                 235                 240
   att aag aac aac tcg cct tac cca caa ttt ttg atc cag att ttg ttc      768
   Ile Lys Asn Asn Ser Pro Tyr Pro Gln Phe Leu Ile Gln Ile Leu Phe
                   245                 250                 255
   tac tac atg atc tcg ctt tta gcg cta ttt gga aac ttt tac gtt cac      816
   Tyr Tyr Met Ile Ser Leu Leu Ala Leu Phe Gly Asn Phe Tyr Val His
               260                 265                 270
   aaa tac gta tca gcg ccc gca aaa cct gcg aag atc aag agc aaa aag      864
   Lys Tyr Val Ser Ala Pro Ala Lys Pro Ala Lys Ile Lys Ser Lys Lys
           275                 280                 285
   gca gaa taa                                                          873
   Ala Glu
       290
<210> 180
   <211> 290
   <212> PRT
   <213> Marthantia polymorpha
<400> 180
   Met Glu Ala Tyr Glu Met Val Asp Ser Phe Val Ser Lys Thr Val Phe
   1 5 10 15
   Glu Thr Leu Gln Arg Leu Arg Gly Gly Val Val Leu Thr Glu Ser Ala
   20 25 30
   Ile Thr Lys Gly Leu Pro Cys Val Asp Ser Pro Thr Pro Ile Val Leu
   35 40 45
   Gly Leu Ser Ser Tyr Leu Thr Phe Val Phe Leu Gly Leu Ile Val Ile
   50 55 60
   Lys Ser Leu Asp Leu Lys Pro Arg Ser Lys Glu Pro Ala Ile Leu Asn
   65 70 75 80
   Leu Phe Val Ile Phe His Asn Phe Val Cys Phe Ala Leu Ser Leu Tyr
   85 90 95
   Met Cys Val Gly Ile Val Arg Gln Ala Ile Leu Asn Arg Tyr Ser Leu
   100 105 110
   Trp Gly Asn Ala Tyr Asn Pro Lys Glu Val Gln Met Gly His Leu Leu
   115 120 125
   Tyr Ile Phe Tyr Met Ser Lys Tyr Ile Glu Phe Met Asp Thr Val Ile
   130 135 140
   Met Ile Leu Lys Arg Asn Thr Arg Gln Ile Thr Val Leu His Val Tyr
   145 150 155 160
   His His Ala Ser Ile Ser Phe Ile Trp Trp Ile Ile Ala Tyr His Ala
   165 170 175
   Pro Gly Gly Glu Ala Tyr Phe Ser Ala Ala Leu Asn Ser Gly Val His
   180 185 190
   Val Leu Met Tyr Leu Tyr Tyr Leu Leu Ala Ala Thr Leu Gly Lys Asn
   195 200 205
   Glu Lys Ala Arg Arg Lys Tyr Leu Trp Trp Gly Lys Tyr Leu Thr Gln
   210 215 220
   Leu Gln Met Phe Gln Phe Val Leu Asn Met Ile Gln Ala Tyr Tyr Asp
   225 230 235 240
   Ile Lys Asn Asn Ser Pro Tyr Pro Gln Phe Leu Ile Gln Ile Leu Phe
   245 250 255
   Tyr Tyr Met Ile Ser Leu Leu Ala Leu Phe Gly Asn Phe Tyr Val His
   260 265 270
   Lys Tyr Val Ser Ala Pro Ala Lys Pro Ala Lys Ile Lys Ser Lys Lys
   275 280 285
   Ala Glu
   290
<210> 181
   <211> 873
   <212> DNA
   <213> Physcomitrella patens
<220>
   <221> CDS
   <222> (1)..(873)
<400> 181
   atg gag gtc gtg gag aga ttc tac ggt gag ttg gat ggg aag gtc tcg      48
   Met Glu Val Val Glu Arg Phe Tyr Gly Glu Leu Asp Gly Lys Val Ser
   1               5                   10                  15
   cag ggc gtg aat gca ttg ctg ggt agt ttt ggg gtg gag ttg acg gat      96
   Gln Gly Val Asn Ala Leu Leu Gly Ser Phe Gly Val Glu Leu Thr Asp
               20                  25                  30
   acg ccc act acc aaa ggc ttg ccc ctc gtt gac agt ccc aca ccc atc      144
   Thr Pro Thr Thr Lys Gly Leu Pro Leu Val Asp Ser Pro Thr Pro Ile
           35                  40                  45
   gtc ctc ggt gtt tct gta tac ttg act att gtc att gga ggg ctt ttg      192
   Val Leu Gly Val Ser Val Tyr Leu Thr Ile Val Ile Gly Gly Leu Leu
       50                  55                  60
   tgg ata aag gcc agg gat ctg aaa ccg cgc gcc tcg gag cca ttt ttg      240
   Trp Ile Lys Ala Arg Asp Leu Lys Pro Arg Ala Ser Glu Pro Phe Leu
   65                  70                  75                  80
   ctc caa gct ttg gtg ctt gtg cac aac ctg ttc tgt ttt gcg ctc agt      288
   Leu Gln Ala Leu Val Leu Val His Asn Leu Phe Cys Phe Ala Leu Ser
                   85                  90                  95
   ctg tat atg tgc gtg ggc atc gct tat cag gct att acc tgg cgg tac      336
   Leu Tyr Met Cys Val Gly Ile Ala Tyr Gln Ala Ile Thr Trp Arg Tyr
               100                 105                 110
   tct ctc tgg ggc aat gca tac aat cct aaa cat aaa gag atg gcg att      384
   Ser Leu Trp Gly Asn Ala Tyr Asn Pro Lys His Lys Glu Met Ala Ile
           115                 120                 125
   ctg gta tac ttg ttc tac atg tct aag tac gtg gaa ttc atg gat acc      432
   Leu Val Tyr Leu Phe Tyr Met Ser Lys Tyr Val Glu Phe Met Asp Thr
       130                 135                 140
   gtt atc atg ata ctg aag cgc agc acc agg caa ata agc ttc ctc cac      480
   Val Ile Met Ile Leu Lys Arg Ser Thr Arg Gln Ile Ser Phe Leu His
   145                 150                 155                 160
   gtt tat cat cat tct tca att tcc ctc att tgg tgg gct att gct cat      528
   Val Tyr His His Ser Ser Ile Ser Leu Ile Trp Trp Ala Ile Ala His
                   165                 170                 175
   cac gct cct ggc ggt gaa gca tat tgg tct gcg gct ctg aac tca gga      576
   His Ala Pro Gly Gly Glu Ala Tyr Trp Ser Ala Ala Leu Asn Ser Gly
               180                 185                 190
   gtg cat gtt ctc atg tat gcg tat tac ttc ttg gct gcc tgc ctt cga      624
   Val His Val Leu Met Tyr Ala Tyr Tyr Phe Leu Ala Ala Cys Leu Arg
           195                 200                 205
   agt agc cca aag tta aaa aat aag tac ctt ttt tgg ggc agg tac ttg      672
   Ser Ser Pro Lys Leu Lys Asn Lys Tyr Leu Phe Trp Gly Arg Tyr Leu
       210                 215                 220
   aca caa ttc caa atg ttc cag ttt atg ctg aac tta gtg cag gct tac      720
   Thr Gln Phe Gln Met Phe Gln Phe Met Leu Asn Leu Val Gln Ala Tyr
   225                 230                 235                 240
   tac gac atg aaa acg aat gcg cca tat cca caa tgg ctg atc aag att      768
   Tyr Asp Met Lys Thr Asn Ala Pro Tyr Pro Gln Trp Leu Ile Lys Ile
                   245                 250                 255
   ttg ttc tac tac atg atc tcg ttg ctg ttt ctt ttc ggc aat ttt tac      816
   Leu Phe Tyr Tyr Met Ile Ser Leu Leu Phe Leu Phe Gly Asn Phe Tyr
               260                 265                 270
   gta caa aaa tac atc aaa ccc tct gac gga aag caa aag gga gct aaa      864
   Val Gln Lys Tyr Ile Lys Pro Ser Asp Gly Lys Gln Lys Gly Ala Lys
           275                 280                 285
   act gag tga                                                          873
   Thr Glu
       290
<210> 182
   <211> 290
   <212> PRT
   <213> Physcomitrella patens
<400> 182
   Met Glu Val Val Glu Arg Phe Tyr Gly Glu Leu Asp Gly Lys Val Ser
   1               5                   10                  15
   Gln Gly Val Asn Ala Leu Leu Gly Ser Phe Gly Val Glu Leu Thr Asp
               20                  25                  30
   Thr Pro Thr Thr Lys Gly Leu Pro Leu Val Asp Ser Pro Thr Pro Ile
           35                  40                  45
   Val Leu Gly Val Ser Val Tyr Leu Thr Ile Val Ile Gly Gly Leu Leu
       50                  55                  60
   Trp Ile Lys Ala Arg Asp Leu Lys Pro Arg Ala Ser Glu Pro Phe Leu
   65                  70                  75                  80
   Leu Gln Ala Leu Val Leu Val His Asn Leu Phe Cys Phe Ala Leu Ser
                   85                  90                  95
   Leu Tyr Met Cys Val Gly Ile Ala Tyr Gln Ala Ile Thr Trp Arg Tyr
               100                 105                 110
   Ser Leu Trp Gly Asn Ala Tyr Asn Pro Lys His Lys Glu Met Ala Ile
           115                 120                 125
   Leu Val Tyr Leu Phe Tyr Met Ser Lys Tyr Val Glu Phe Met Asp Thr
       130                 135                 140
   Val Ile Met Ile Leu Lys Arg Ser Thr Arg Gln Ile Ser Phe Leu His
   145                 150                 155                 160
   Val Tyr His His Ser Ser Ile Ser Leu Ile Trp Trp Ala Ile Ala His
                   165                 170                 175
   His Ala Pro Gly Gly Glu Ala Tyr Trp Ser Ala Ala Leu Asn Ser Gly
               180                 185                 190
   Val His Val Leu Met Tyr Ala Tyr Tyr Phe Leu Ala Ala Cys Leu Arg
           195                 200                 205
   Ser Ser Pro Lys Leu Lys Asn Lys Tyr Leu Phe Trp Gly Arg Tyr Leu
       210                 215                 220
   Thr Gln Phe Gln Met Phe Gln Phe Met Leu Asn Leu Val Gln Ala Tyr
   225                 230                 235                 240
   Tyr Asp Met Lys Thr Asn Ala Pro Tyr Pro Gln Trp Leu Ile Lys Ile
                   245                 250                 255
   Leu Phe Tyr Tyr Met Ile Ser Leu Leu Phe Leu Phe Gly Asn Phe Tyr
               260                 265                 270
   Val Gln Lys Tyr Ile Lys Pro Ser Asp Gly Lys Gln Lys Gly Ala Lys
           275                 280                 285
   Thr Glu
       290
<210> 183
   <211> 957
   <212> DNA
   <213> Mortierella alpina
<220>
   <221> CDS
   <222> (1)..(957)
<400> 183
   atg gag tcg att gcg cca ttc ctc cca tca aag atg ccg caa gat ctg      48
   Met Glu Ser Ile Ala Pro Phe Leu Pro Ser Lys Met Pro Gln Asp Leu
   1               5                   10                  15
   ttt atg gac ctt gcc acc gct atc ggt gtc cgg gcc gcg ccc tat gtc      96
   Phe Met Asp Leu Ala Thr Ala Ile Gly Val Arg Ala Ala Pro Tyr Val
               20                  25                  30
   gat cct ctc gag gcc gcg ctg gtg gcc cag gcc gag aag tac atc ccc     144
   Asp Pro Leu Glu Ala Ala Leu Val Ala Gln Ala Glu Lys Tyr Ile Pro
           35                  40                  45
   acg att gtc cat cac acg cgt ggg ttc ctg gtc gcg gtg gag tcg cct     192
   Thr Ile Val His His Thr Arg Gly Phe Leu Val Ala Val Glu Ser Pro
       50                  55                  60
   ttg gcc cgt gag ctg ccg ttg atg aac ccg ttc cac gtg ctg ttg atc     240
   Leu Ala Arg Glu Leu Pro Leu Met Asn Pro Phe His Val Leu Leu Ile
   65                  70                  75                  80
   gtg ctc gct tat ttg gtc acg gtc ttt gtg ggc atg cag atc atg aag     288
   Val Leu Ala Tyr Leu Val Thr Val Phe Val Gly Met Gln Ile Met Lys
                   85                  90                  95
   aac ttt gag cgg ttc gag gtc aag acg ttt tcg ctc ctg cac aac ttt     336
   Asn Phe Glu Arg Phe Glu Val Lys Thr Phe Ser Leu Leu His Asn Phe
               100                 105                 110
   tgt ctg gtc tcg atc agc gcc tac atg tgc ggt ggg atc ctg tac gag     384
   Cys Leu Val Ser Ile Ser Ala Tyr Met Cys Gly Gly Ile Leu Tyr Glu
           115                 120                 125
   gct tat cag gcc aac tat gga ctg ttt gag aac gct gct gat cat acc     432
   Ala Tyr Gln Ala Asn Tyr Gly Leu Phe Glu Asn Ala Ala Asp His Thr
       130                 135                 140
   ttc aag ggt ctt cct atg gcc aag atg atc tgg ctc ttc tac ttc tcc     480
   Phe Lys Gly Leu Pro Met Ala Lys Met Ile Trp Leu Phe Tyr Phe Ser
   145                 150                 155                 160
   aag atc atg gag ttt gtc gac acc atg atc atg gtc ctc aag aag aac     528
   Lys Ile Met Glu Phe Val Asp Thr Met Ile Met Val Leu Lys Lys Asn
                   165                 170                 175
   aac cgc cag atc tcc ttc ttg cac gtt tac cac cac agc tcc atc ttc     576
   Asn Arg Gln Ile Ser Phe Leu His Val Tyr His His Ser Ser Ile Phe
               180                 185                 190
   acc atc tgg tgg ttg gtc acc ttt gtt gca ccc aac ggt gaa gcc tac     624
   Thr Ile Trp Trp Leu Val Thr Phe Val Ala Pro Asn Gly Glu Ala Tyr
           195                 200                 205
   ttc tct gct gcg ttg aac tcg ttc atc cat gtg atc atg tac ggc tac     672
   Phe Ser Ala Ala Leu Asn Ser Phe Ile His Val Ile Met Tyr Gly Tyr
       210                 215                 220
   tac ttc ttg tcg gcc ttg ggc ttc aag cag gtg tcg ttc atc aag ttc     720
   Tyr Phe Leu Ser Ala Leu Gly Phe Lys Gln Val Ser Phe Ile Lys Phe
   225                 230                 235                 240
   tac atc acg cgc tcg cag atg aca cag ttc tgc atg atg tcg gtc cag     768
   Tyr Ile Thr Arg Ser Gln Met Thr Gln Phe Cys Met Met Ser Val Gln
                   245                 250                 255
   tct tcc tgg gac atg tac gcc atg aag gtc ctt ggc cgc ccc gga tac     816
   Ser Ser Trp Asp Met Tyr Ala Met Lys Val Leu Gly Arg Pro Gly Tyr
               260                 265                 270
   ccc ttc ttc atc acg gct ctg ctt tgg ttc tac atg tgg acc atg ctc     864
   Pro Phe Phe Ile Thr Ala Leu Leu Trp Phe Tyr Met Trp Thr Met Leu
           275                 280                 285
   ggt ctc ttc tac aac ttt tac aga aag aac gcc aag ttg gcc aag cag     912
   Gly Leu Phe Tyr Asn Phe Tyr Arg Lys Asn Ala Lys Leu Ala Lys Gln
       290                 295                 300
   gcc aag gcc gac gct gcc aag gag aag gca agg aag ttg cag taa         957
   Ala Lys Ala Asp Ala Ala Lys Glu Lys Ala Arg Lys Leu Gln
   305                 310                 315
<210> 184
   <211> 318
   <212> PRT
   <213> Mortierella alpina
<400> 184
   Met Glu Ser Ile Ala Pro Phe Leu Pro Ser Lys Met Pro Gln Asp Leu
   1               5                   10                  15
   Phe Met Asp Leu Ala Thr Ala Ile Gly Val Arg Ala Ala Pro Tyr Val
               20                  25                  30
   Asp Pro Leu Glu Ala Ala Leu Val Ala Gln Ala Glu Lys Tyr Ile Pro
           35                  40                  45
   Thr Ile Val His His Thr Arg Gly Phe Leu Val Ala Val Glu Ser Pro
       50                  55                  60
   Leu Ala Arg Glu Leu Pro Leu Met Asn Pro Phe His Val Leu Leu Ile
   65                  70                  75                  80
   Val Leu Ala Tyr Leu Val Thr Val Phe Val Gly Met Gln Ile Met Lys
                   85                  90                  95
   Asn Phe Glu Arg Phe Glu Val Lys Thr Phe Ser Leu Leu His Asn Phe
               100                 105                 110
   Cys Leu Val Ser Ile Ser Ala Tyr Met Cys Gly Gly Ile Leu Tyr Glu
           115                 120                 125
   Ala Tyr Gln Ala Asn Tyr Gly Leu Phe Glu Asn Ala Ala Asp His Thr
       130                 135                 140
   Phe Lys Gly Leu Pro Met Ala Lys Met Ile Trp Leu Phe Tyr Phe Ser
   145                 150                 155                 160
   Lys Ile Met Glu Phe Val Asp Thr Met Ile Met Val Leu Lys Lys Asn
                   165                 170                 175
   Asn Arg Gln Ile Ser Phe Leu His Val Tyr His His Ser Ser Ile Phe
               180                 185                 190
   Thr Ile Trp Trp Leu Val Thr Phe Val Ala Pro Asn Gly Glu Ala Tyr
           195                 200                 205
   Phe Ser Ala Ala Leu Asn Ser Phe Ile His Val Ile Met Tyr Gly Tyr
       210                 215                 220
   Tyr Phe Leu Ser Ala Leu Gly Phe Lys Gln Val Ser Phe Ile Lys Phe
   225                 230                 235                 240
   Tyr Ile Thr Arg Ser Gln Met Thr Gln Phe Cys Met Met Ser Val Gln
                   245                 250                 255
   Ser Ser Trp Asp Met Tyr Ala Met Lys Val Leu Gly Arg Pro Gly Tyr
               260                 265                 270
   Pro Phe Phe Ile Thr Ala Leu Leu Trp Phe Tyr Met Trp Thr Met Leu
           275                 280                 285
   Gly Leu Phe Tyr Asn Phe Tyr Arg Lys Asn Ala Lys Leu Ala Lys Gln
       290                 295                 300
   Ala Lys Ala Asp Ala Ala Lys Glu Lys Ala Arg Lys Leu Gln
   305                 310                 315
<210> 185
   <211> 251
   <212> PRT
   <213> Artificial sequence
<220>
   <221> Variant
   <222> (2)..(11)
   <223> xaa in position 2 to 11 is any amino acid
<220>
   <221> Variant
   <222> (14)..(15)
   <223> xaa in position 14 to 15 is any amino acid
<220>
   <221> variant
   <222> (17)..(23)
   <223> xaa in position 17 to 23 is any amino acid
<220>
   <221> Variant
   <222> (25)..(25)
   <223> xaa in position 25 is any amino acid
<220>
   <221> Variant
   <222> (26)..(28)
   <223> xaa in position 26 to 28 is any or no amino acid
<220>
   <221> Variant
   <222> (30)..(31)
   <223> xaa in position 30 to 31 is any amino acid
<220>
   <221> Variant
   <222> (32)..(34)
   <223> xaa in position 32 to 34 is any or no amino acid
<220>
   <221> Variant
   <222> (36)..(37)
   <223> xaa in position 36 to 37 is any amino acid
<220>
   <221> Variant
   <222> (39)..(44)
   <223> xaa in position 39 to 44 is any amino acid
<220>
   <221> Variant
   <222> (47)..(51)
   <223> xaa in position 47 to 51 is any amino acid
<220>
   <221> Variant
   <222> (54)..(54)
   <223> xaa in position 54 is any amino acid
<220>
   <221> Variant
   <222> (57)..(63)
   <223> xaa in position 57 to 63 is any amino acid
<220>
   <221> Variant
   <222> (65)..(68)
   <223> xaa in position 65 to 68 is any amino acid
<220>
   <221> Variant
   <222> (70)..(70)
   <223> xaa in position 70 is any amino acid
<220>
   <221> Variant
   <222> (72)..(72)
   <223> xaa in position 72 is any amino acid
<220>
   <221> Variant
   <222> (75)..(82)
   <223> xaa in position 75 to 82 is any amino acid
<220>
   <221> Variant
   <222> (83)..(86)
   <223> xaa in position 83 to 86 is any or no amino acid
<220>
   <221> Variant
   <222> (88)..(91)
   <223> xaa in position 88 to 91 is any amino acid
<220>
   <221> Variant
   <222> (93)..(93)
   <223> xaa in position 93 is any amino acid
<220>
   <221> Variant
   <222> (96)..(96)
   <223> xaa in position 96 is any amino acid
<220>
   <221> Variant
   <222> (99)..(100)
   <223> xaa in position 99 to 100 is any amino acid
<220>
   <221> Variant
   <222> (103)..(103)
   <223> xaa in position 103 is any amino acid
<220>
   <221> Variant
   <222> (106)..(107)
   <223> xaa in position 106 to 107 is any amino acid
<220>
   <221> Variant
   <222> (109)..(109)
   <223> xaa in position 109 is any amino acid
<220>
   <221> Variant
   <222> (111)..(115)
   <223> xaa in position 111 to 115 is any amino acid
<220>
   <221> Variant
   <222> (117)..(119)
   <223> xaa in position 117 to 119 is any amino acid
<220>
   <221> Variant
   <222> (126)..(126)
   <223> xaa in position 126 is any amino acid
<220>
   <221> Variant
   <222> (129)..(130)
   <223> xaa in position 129 to 130 is any amino acid
<220>
   <221> Variant
   <222> (133)..(134)
   <223> xaa in position 133 to 134 is any amino acid
<220>
   <221> Variant
   <222> (136)..(138)
   <223> xaa in position 136 to 138 is any amino acid
<220>
   <221> Variant
   <222> (143)..(143)
   <223> xaa in position 143 is any amino acid
<220>
   <221> Variant
   <222> (148)..(149)
   <223> xaa in position 148 to 149 is any amino acid
<220>
   <221> Variant
   <222> (153)..(154)
   <223> xaa in position 153 to 154 is any amino acid
<220>
   <221> Variant
   <222> (157)..(158)
   <223> xaa in position 157 to 158 is any amino acid
<220>
   <221> variant
   <222> (160)..(160)
   <223> xaa in position 160 is any amino acid
<220>
   <221> variant
   <222> (163)..(179)
   <223> xaa in position 163 to 179 is any amino acid
<220>
   <221> Variant
   <222> (180)..(185)
   <223> xaa in position 180 to 185 is any or no amino acid
<220>
   <221> Variant
   <222> (187)..(188)
   <223> xaa in position 187 to 188 is any amino acid
<220>
   <221> Variant
   <222> (190)..(191)
   <223> xaa in position 190 to 191 is any amino acid
<220>
   <221> Variant
   <222> (194)..(198)
   <223> xaa in position 194 to 198 is any amino acid
<220>
   <221> Variant
   <222> (200)..(201)
   <223> xaa in position 200 to 201 is any amino acid
<220>
   <221> Variant
   <222> (203)..(208)
   <223> xaa in position 203 to 208 is any amino acid
<220>
   <221> Variant
   <222> (209)..(209)
   <223> xaa in position 209 is any or no amino acid
<220>
   <221> Variant
   <222> (212)..(217)
   <223> xaa in position 212 to 217 is any amino acid
<220>
   <221> Variant
   <222> (219)..(220)
   <223> xaa in position 219 to 220 is any amino acid
<220>
   <221> Variant
   <222> (223)..(225)
   <223> xaa in position 223 to 225 is any amino acid
<220>
   <221> Variant
   <222> (227)..(227)
   <223> xaa in position 227 is any amino acid
<220>
   <221> variant
   <222> (233)..(246)
   <223> xaa in position 233 to 246 is any amino acid
<220>
   <221> Variant
   <222> (247)..(250)
   <223> xaa in position 247 to 250 is any or no amino acid
<400> 185
   Ser Xaa Xaa Xaa Xaa Xaa Xaa Xaa Xaa Xaa Xaa Tyr Leu Xaa Xaa Val
   1               5                   10                  15
   Xaa Xaa Xaa Xaa Xaa Xaa Xaa Lys Xaa Xaa Xaa Xaa Lys Xaa Xaa Xaa
               20                  25                  30
   Xaa Xaa Pro Xaa Xaa Leu Xaa Xaa Xaa Xaa Xaa Xaa His Asn Xaa Xaa
           35                  40                  45
   Xaa Xaa Xaa Leu Ser Xaa Tyr Met Xaa Xaa Xaa Xaa Xaa Xaa Xaa Ala
       50                  55                  60
   Xaa Xaa Xaa Xaa Tyr Xaa Leu Xaa Gly Asn Xaa Xaa Xaa Xaa Xaa Xaa
   65                  70                  75                  80
   Xaa Xaa Xaa Xaa Xaa Xaa Met Xaa Xaa Xaa Xaa Tyr Xaa Phe Tyr Xaa
                   85                  90                  95
   Ser Lys Xaa Xaa Glu Phe Xaa Asp Thr Xaa Xaa Met Xaa Leu Xaa Xaa
               100                 105                 110
   Xaa Xaa Xaa Gln Xaa Xaa Xaa Leu His Val Tyr His His Xaa Ser Ile
           115                 120                 125
   Xaa Xaa Ile Trp Xaa Xaa Ile Xaa Xaa Xaa Ala Pro Gly Gly Xaa Ala
       130                 135                 140
   Tyr Phe Ser Xaa Xaa Leu Asn Ser Xaa Xaa His Val Xaa Xaa Tyr Xaa
   145                 150                 155                 160
   Tyr Tyr Xaa Xaa Xaa Xaa Xaa Xaa Xaa Xaa Xaa Xaa Xaa Xaa Xaa Xaa
                   165                 170                 175
   Xaa Xaa Xaa Xaa Xaa Xaa Xaa Xaa Xaa Thr Xaa Xaa Gln Xaa Xaa Gln
               180                 185                 190
   Phe Xaa Xaa Xaa Xaa Xaa Gln Xaa Xaa Tyr Xaa Xaa Xaa Xaa Xaa Xaa
           195                 200                 205
   Xaa Tyr Pro Xaa Xaa Xaa Xaa Xaa Xaa Leu Xaa Xaa Tyr Met Xaa Xaa
       210                 215                 220
   Xaa Leu Xaa Leu Phe Gly Asn Phe Xaa Xaa Xaa Xaa Xaa Xaa Xaa Xaa
   225                 230                 235                 240
   Xaa Xaa Xaa Xaa Xaa Xaa Xaa Xaa Xaa Xaa Lys
                   245                 250
<210> 186
   <211> 60
   <212> PRT
   <213> Artificial sequence
<220>
   <221> Variant
   <222> (3)..(4)
   <223> xaa in position 3 to 4 is any amino acid
<220>
   <221> Variant
   <222> (7)..(7)
   <223> xaa in position 7 is Leu, Met or Val
<220>
   <221> Variant
   <222> (10)..(10)
   <223> xaa in position 10 is any amino acid
<220>
   <221> Variant
   <222> (11)..(11)
   <223> xaa in position 11 is Phe or Ile
<220>
   <221> Variant
   <222> (13)..(13)
   <223> xaa in position 13 is Ile, Leu or Val
<220>
   <221> Variant
   <222> (15)..(18)
   <223> xaa in position 15 to 18 is any amino acid
<220>
   <221> Variant
   <222> (19)..(19)
   <223> xaa in position 19 is Lys or Arg
<220>
   <221> Variant
   <222> (21)..(21)
   <223> xaa in position 21 is Ile, Leu or Val
<220>
   <221> Variant
   <222> (22)..(22)
   <223> xaa in position 22 is Ser or Thr
<220>
   <221> variant
   <222> (23)..(23)
   <223> xaa in position 23 is Phe or Val
<220>
   <221> Variant
   <222> (26)..(26)
   <223> xaa in position 26 is Ile or Val
<220>
   <221> Variant
   <222> (30)..(30)
   <223> xaa in position 30 is Ala or Ser
<220>
   <221> Variant
   <222> (31)..(31)
   <223> xaa in position 31 is Ser or Thr
<220>
   <221> variant
   <222> (33)..(34)
   <223> xaa in position 33 to 34 is any amino acid
<220>
   <221> Variant
   <222> (37)..(37)
   <223> xaa in position 37 is any amino acid
<220>
   <221> Variant
   <222> (38)..(38)
   <223> xaa in position 38 is Ala, Ile or Leu
<220>
   <221> Variant
   <222> (39)..(39)
   <223> xaa in position 39 is Ile or Val
<220>
   <221> Variant
   <222> (40)..(40)
   <223> xaa in position 40 is Ala or Thr
<220>
   <221> Variant
   <222> (41)..(42)
   <223> xaa in position 41 to 42 is any amino acid
<220>
   <221> Variant
   <222> (45)..(45)
   <223> xaa in position 45 is Gly or Asn
<220>
   <221> Variant
   <222> (47)..(47)
   <223> xaa in position 47 is Asp or Glu
<220>
   <221> Variant
   <222> (50)..(50)
   <223> xaa in position 50 is Phe or Trp
<220>
   <221> Variant
   <222> (52)..(52)
   <223> xaa in position 52 is Ala or Thr
<220>
   <221> Variant
   <222> (53)..(53)
   <223> xaa in position 53 is Ala or Ile
<220>
   <221> Variant
   <222> (57)..(57)
   <223> xaa in position 57 is any amino acid
<220>
   <221> variant
   <222> (58)..(58)
   <223> xaa in position 58 is Ile or Val
<400> 186
<210> 187
   <211> 23
   <212> PRT
   <213> Artificial sequence
<220>
   <221> Variant
   <222> (3)..(3)
   <223> xaa in position 3 is Leu or Met
<220>
   <221> Variant
   <222> (4)..(4)
   <223> xaa in position 4 is Phe or Tyr
<220>
   <221> Variant
   <222> (6)..(6)
   <223> xaa in position 6 is Ala or Cys
<220>
   <221> Variant
   <222> (7)..(7)
   <223> xaa in position 7 is any amino acid
<220>
   <221> Variant
   <222> (8)..(8)
   <223> xaa in position 8 is Glu or Gly
<220>
   <221> Variant
   <222> (9)..(9)
   <223> xaa in position 9 is any amino acid
<220>
   <221> Variant
   <222> (10)..(10)
   <223> xaa in position 10 is Ala, Ile or Val
<220>
   <221> Variant
   <222> (11)..(11)
   <223> xaa in position 11 is any amino acid
<220>
   <221> Variant
   <222> (12)..(12)
   <223> xaa in position 12 is Ala or Gln
<220>
   <221> Variant
   <222> (14)..(17)
   <223> xaa in position 14 to 17 is any amino acid
<220>
   <221> Variant
   <222> (19)..(19)
   <223> xaa in position 19 is any amino acid
<220>
   <221> Variant
   <222> (20)..(20)
   <223> xaa in position 20 is Leu or Val
<220>
   <221> Variant
   <222> (21)..(21)
   <223> xaa in position 21 is Phe or Trp
<400> 187
<210> 188
   <211> 24
   <212> PRT
   <213> Artificial sequence
<220>
   <221> Variant
   <222> (3)..(4)
   <223> xaa in position 3 to 4 is any amino acid
<220>
   <221> Variant
   <222> (5)..(5)
   <223> xaa in position 5 is Ile or Leu
<220>
   <221> Variant
   <222> (6)..(7)
   <223> xaa in position 6 to 7 is any amino acid
<220>
   <221> Variant
   <222> (8)..(8)
   <223> xaa in position 8 is Ile or Leu
<220>
   <221> Variant
   <222> (10)..(11)
   <223> xaa in position 10 to 11 is any amino acid
<220>
   <221> Variant
   <222> (14)..(14)
   <223> xaa in position 14 is any amino acid
<220>
   <221> Variant
   <222> (15)..(15)
   <223> xaa in position 15 is Ser or Thr
<220>
   <221> Variant
   <222> (16)..(16)
   <223> xaa in position 16 is Leu or Met
<220>
   <221> Variant
   <222> (18)..(18)
   <223> xaa in position 18 is any amino acid
<220>
   <221> variant
   <222> (21)..(22)
   <223> xaa in position 21 to 22 is any amino acid
<220>
   <221> Variant
   <222> (24)..(24)
   <223> xaa in position 24 is Phe or Tyr
<400> 188
   Tyr Pro xaa xaa xaa xaa xaa xaa Leu xaa xaa Tyr Met xaa xaa xaa
     1               5                  10                  15
   Leu xaa Leu Phe xaa xaa Phe xaa
                20
<210> 189
   <211> 20
   <212> PRT
   <213> Artificial sequence
<220>
   <221> Variant
   <222> (2)..(2)
   <223> xaa in position 2 is Ile or Leu
<220>
   <221> Variant
   <222> (4)..(5)
   <223> xaa in position 4 to 5 is any amino acid
<220>
   <221> variant
   <222> (7)..(7)
   <223> xaa in position 7 is Leu or Met
<220>
   <221> Variant
   <222> (8)..(8)
   <223> xaa in position 8 is any amino acid
<220>
   <221> variant
   <222> (11)..(14)
   <223> xaa in position 11 to 14 is any amino acid
<220>
   <221> Variant
   <222> (15)..(15)
   <223> xaa in position 15 is Gly, Ile or Val
<220>
   <221> Variant
   <222> (17)..(17)
   <223> xaa in position 17 is Ala or Ser
<220>
   <221> Variant
   <222> (18)..(18)
   <223> xaa in position 18 is any amino acid
<220>
   <221> Variant
   <222> (19)..(19)
   <223> xaa in position 19 is Trp or Tyr
<220>
   <221> Variant
   <222> (20)..(20)
   <223> xaa in position 20 is Ala or Asp
<400> 189
<210> 190
   <211> 18
   <212> PRT
   <213> Artificial sequence
<220>
   <221> Variant
   <222> (2)..(5)
   <223> xaa in position 2 to 5 is any amino acid
<220>
   <221> Variant
   <222> (6)..(6)
   <223> xaa in position 6 is any or no amino acid
<220>
   <221> Variant
   <222> (8)..(9)
   <223> xaa in position 8 to 9 is any amino acid
<220>
   <221> Variant
   <222> (11)..(12)
   <223> xaa in position 11 to 12 is any amino acid
<220>
   <221> variant
   <222> (13)..(13)
   <223> xaa in position 13 is Phe or Leu
<220>
   <221> Variant
   <222> (14)..(14)
   <223> xaa in position 14 is any amino acid
<220>
   <221> Variant
   <222> (15)..(15)
   <223> xaa in position 15 is Ile or _{L}eu
<220>
   <221> Variant
   <222> (16)..(16)
   <223> xaa in position 16 is any amino acid
<400> 190
   Lys xaa xaa xaa xaa xaa Pro xaa xaa Leu xaa xaa xaa xaa xaa xaa
   1               5                   10                  15
   His Asn
<210> 191
   <211> 18
   <212> PRT
   <213> Artificial sequence
<220>
   <221> Variant
   <222> (2)..(2)
   <223> xaa in position 2 is Pro or Thr
<220>
   <221> Variant
   <222> (3)..(3)
   <223> xaa in position 3 is any amino acid
<220>
   <221> Variant
   <222> (4)..(4)
   <223> xaa in position 4 is Glu or Pro
<220>
   <221> Variant
   <222> (5)..(5)
   <223> xaa in position 5 is Ile, Leu, Met or Val
<220>
   <221> variant
   <222> (6)..(6)
   <223> xaa in position 6 is Leu, Met or Val
<220>
   <221> Variant
   <222> (7)..(8)
   <223> xaa in position 7 to 8 is any amino acid
<220>
   <221> Variant
   <222> (9)..(9)
   <223> xaa in position 9 is Ile, Leu or Val
<220>
   <221> variant
   <222> (10)..(11)
   <223> xaa in position 10 to 11 is any amino acid
<220>
   <221> variant
   <222> (14)..(15)
   <223> xaa in position 14 to 15 is any amino acid
<220>
   <221> Variant
   <222> (17)..(17)
   <223> xaa in position 17 is any amino acid
<220>
   <221> Variant
   <222> (18)..(18)
   <223> xaa in position 18 is Gly or Leu
<400> 191
<210> 192
   <211> 10
   <212> PRT
   <213> Artificial sequence
<220>
   <221> variant
   <222> (2)..(2)
   <223> xaa in position 2 is Ala, Gly or Asn
<220>
   <221> Variant
   <222> (3)..(3)
   <223> xaa in position 3 is any amino acid
<220>
   <221> Variant
   <222> (4)..(4)
   <223> xaa in position 4 is Ile, Leu or Met
<220>
   <221> variant
   <222> (5)..(5)
   <223> xaa in position 5 is Ile, Leu or Val
<220>
   <221> Variant
   <222> (6)..(6)
   <223> xaa in position 6 is Trp or Tyr
<220>
   <221> Variant
   <222> (7)..(7)
   <223> xaa in position 7 is Ile, Leu or Val
<220>
   <221> Variant
   <222> (10)..(10)
   <223> xaa in position 10 is Phe, Met or Val
<400> 192
   Met xaa xaa xaa xaa xaa xaa Phe Tyr xaa
    1               5                  10
<210> 193
   <211> 1086
   <212> DNA
   <213> Phytophthora infestans
<220>
   <221> CDS
   <222> (1)..(1086)
<400> 193
   atg gcg acg aag gag gcg tat gtg ttc ccc act ctg acg gag atc aag      48
   Met Ala Thr Lys Glu Ala Tyr Val Phe Pro Thr Leu Thr Glu Ile Lys
   1               5                   10                  15
   cgg tcg cta cct aaa gac tgt ttc gag gct tcg gtg cct ctg tcg ctc      96
   Arg Ser Leu Pro Lys Asp Cys Phe Glu Ala Ser Val Pro Leu Ser Leu
               20                  25                  30
   tac tac acc gtg cgt tgt ctg gtg atc gcg gtg gct cta acc ttc ggt      144
   Tyr Tyr Thr Val Arg Cys Leu Val Ile Ala Val Ala Leu Thr Phe Gly
           35                  40                  45
   ctc aac tac gct cgc gct ctg ccc gag gtc gag agc ttc tgg gct ctg      192
   Leu Asn Tyr Ala Arg Ala Leu Pro Glu Val Glu Ser Phe Trp Ala Leu
       50                  55                  60
   gac gcc gca ctc tgc acg ggc tac atc ttg ctg cag ggc atc gtg ttc      240
   Asp Ala Ala Leu Cys Thr Gly Tyr Ile Leu Leu Gln Gly Ile Val Phe
   65                  70                  75                  80
   tgg ggc ttc ttc acg gtg ggc cac gat gcc ggc cac ggc gcc ttc tcg      288
   Trp Gly Phe Phe Thr Val Gly His Asp Ala Gly His Gly Ala Phe Ser
                   85                  90                  95
   cgc tac cac ctg ctt aac ttc gtg gtg ggc act ttc atg cac tcg ctc      336
   Arg Tyr His Leu Leu Asn Phe Val Val Gly Thr Phe Met His Ser Leu
               100                 105                 110
   atc ctc acg ccc ttc gag tcg tgg aag ctc acg cac cgt cac cac cac      384
   Ile Leu Thr Pro Phe Glu Ser Trp Lys Leu Thr His Arg His His His
           115                 120                 125
   aag aac acg ggc aac att gac cgt gac gag gtc ttc tac ccg caa cgc      432
   Lys Asn Thr Gly Asn Ile Asp Arg Asp Glu Val Phe Tyr Pro Gln Arg
       130                 135                 140
   aag gcc gac gac cac ccg ctg tct cgc aac ctg att ctg gcg ctc ggg      480
   Lys Ala Asp Asp His Pro Leu Ser Arg Asn Leu Ile Leu Ala Leu Gly
   145                 150                 155                 160
   gca gcg tgg ctc gcc tat ttg gtc gag ggc ttc cct cct cgt aag gtc      528
   Ala Ala Trp Leu Ala Tyr Leu Val Glu Gly Phe Pro Pro Arg Lys Val
                   165                 170                 175
   aac cac ttc aac ccg ttc gag cct ctg ttc gtg cgt cag gtg tca gct      576
   Asn His Phe Asn Pro Phe Glu Pro Leu Phe Val Arg Gln Val Ser Ala
               180                 185                 190
   gtg gta atc tct ctt ctc gcc cac ttc ttc gtg gcc gga ctc tcc atc      624
   Val Val Ile Ser Leu Leu Ala His Phe Phe Val Ala Gly Leu Ser Ile
           195                 200                 205
   tat ctg agc ctc cag ctg ggc ctt aag acg atg gca atc tac tac tat      672
   Tyr Leu Ser Leu Gln Leu Gly Leu Lys Thr Met Ala Ile Tyr Tyr Tyr
       210                 215                 220
   gga cct gtt ttt gtg ttc ggc agc atg ctg gtc att acc acc ttc cta      720
   Gly Pro Val Phe Val Phe Gly Ser Met Leu Val Ile Thr Thr Phe Leu
   225                 230                 235                 240
   cac cac aat gat gag gag acc cca tgg tac gcc gac tcg gag tgg acg      768
   His His Asn Asp Glu Glu Thr Pro Trp Tyr Ala Asp Ser Glu Trp Thr
                   245                 250                 255
   tac gtc aag ggc aac ctc tcg tcc gtg gac cga tcg tac ggc gcg ctc      816
   Tyr Val Lys Gly Asn Leu Ser Ser Val Asp Arg Ser Tyr Gly Ala Leu
               260                 265                 270
   att gac aac ctg agc cac aac atc ggc acg cac cag atc cac cac ctt      864
   Ile Asp Asn Leu Ser His Asn Ile Gly Thr His Gln Ile His His Leu
           275                 280                 285
   ttc cct atc att ccg cac tac aaa ctc aag aaa gcc act gcg gcc ttc      912
   Phe Pro Ile Ile Pro His Tyr Lys Leu Lys Lys Ala Thr Ala Ala Phe
       290                 295                 300
   cac cag gct ttc cct gag ctc gtg cgc aag agc gac gag cca att atc      960
   His Gln Ala Phe Pro Glu Leu Val Arg Lys Ser Asp Glu Pro Ile Ile
   305                 310                 315                 320
   aag gct ttc ttc cgg gtt gga cgt ctc tac gca aac tac ggc gtt gtg      1008
   Lys Ala Phe Phe Arg Val Gly Arg Leu Tyr Ala Asn Tyr Gly Val Val
                   325                 330                 335
   gac cag gag gcg aag ctc ttc acg cta aag gaa gcc aag gcg gcg acc      1056
   Asp Gln Glu Ala Lys Leu Phe Thr Leu Lys Glu Ala Lys Ala Ala Thr
               340                 345                 350
   gag gcg gcg gcc aag acc aag tcc acg taa                              1086
   Glu Ala Ala Ala Lys Thr Lys Ser Thr
           355                 360
<210> 194
   <211> 361
   <212> PRT
   <213> Phytophthora infestans
<400> 194
   Met Ala Thr Lys Glu Ala Tyr Val Phe Pro Thr Leu Thr Glu Ile Lys
   1               5                   10                  15
   Arg Ser Leu Pro Lys Asp Cys Phe Glu Ala Ser Val Pro Leu Ser Leu
               20                  25                  30
   Tyr Tyr Thr Val Arg Cys Leu Val Ile Ala Val Ala Leu Thr Phe Gly
           35                  40                  45
   Leu Asn Tyr Ala Arg Ala Leu Pro Glu Val Glu Ser Phe Trp Ala Leu
       50                  55                  60
   Asp Ala Ala Leu Cys Thr Gly Tyr Ile Leu Leu Gln Gly Ile Val Phe
   65                  70                  75                  80
   Trp Gly Phe Phe Thr Val Gly His Asp Ala Gly His Gly Ala Phe Ser
                   85                  90                  95
   Arg Tyr His Leu Leu Asn Phe Val Val Gly Thr Phe Met His Ser Leu
               100                 105                 110
   Ile Leu Thr Pro Phe Glu Ser Trp Lys Leu Thr His Arg His His His
           115                 120                 125
   Lys Asn Thr Gly Asn Ile Asp Arg Asp Glu Val Phe Tyr Pro Gln Arg
       130                 135                 140
   Lys Ala Asp Asp His Pro Leu Ser Arg Asn Leu Ile Leu Ala Leu Gly
   145                 150                 155                 160
   Ala Ala Trp Leu Ala Tyr Leu Val Glu Gly Phe Pro Pro Arg Lys Val
                   165                 170                 175
   Asn His Phe Asn Pro Phe Glu Pro Leu Phe Val Arg Gln Val Ser Ala
               180                 185                 190
   Val Val Ile Ser Leu Leu Ala His Phe Phe Val Ala Gly Leu Ser Ile
           195                 200                 205
   Tyr Leu Ser Leu Gln Leu Gly Leu Lys Thr Met Ala Ile Tyr Tyr Tyr
       210                 215                 220
   Gly Pro Val Phe Val Phe Gly Ser Met Leu Val Ile Thr Thr Phe Leu
   225                 230                 235                 240
   His His Asn Asp Glu Glu Thr Pro Trp Tyr Ala Asp Ser Glu Trp Thr
                   245                 250                 255
   Tyr Val Lys Gly Asn Leu Ser Ser Val Asp Arg Ser Tyr Gly Ala Leu
               260                 265                 270
   Ile Asp Asn Leu Ser His Asn Ile Gly Thr His Gln Ile His His Leu
           275                 280                 285
   Phe Pro Ile Ile Pro His Tyr Lys Leu Lys Lys Ala Thr Ala Ala Phe
       290                 295                 300
   His Gln Ala Phe Pro Glu Leu Val Arg Lys Ser Asp Glu Pro Ile Ile
   305                 310                 315                 320
   Lys Ala Phe Phe Arg Val Gly Arg Leu Tyr Ala Asn Tyr Gly Val Val
                   325                 330                 335
   Asp Gln Glu Ala Lys Leu Phe Thr Leu Lys Glu Ala Lys Ala Ala Thr
               340                 345                 350
   Glu Ala Ala Ala Lys Thr Lys Ser Thr
           355                 360
<210> 195
   <211> 1077
   <212> DNA
   <213> Saprolegnia diclina
<220>
   <221> CDS
   <222> (1)..(1077)
<400> 195
   atg act gag gat aag acg aag gtc gag ttc ccg acg ctc acg gag ctc      48
   Met Thr Glu Asp Lys Thr Lys Val Glu Phe Pro Thr Leu Thr Glu Leu
   1               5                   10                  15
   aag cac tcg atc ccg aac gcg tgc ttt gag tcg aac ctc ggc ctc tcg      96
   Lys His Ser Ile Pro Asn Ala Cys Phe Glu Ser Asn Leu Gly Leu Ser
               20                  25                  30
   ctc tac tac acg gcc cgc gcg atc ttc aac gcg tcg gcc tcg gcg gcg      144
   Leu Tyr Tyr Thr Ala Arg Ala Ile Phe Asn Ala Ser Ala Ser Ala Ala
           35                  40                  45
   ctg ctc tac gcg gcg cgc tcg acg ccg ttc att gcc gat aac gtt ctg      192
   Leu Leu Tyr Ala Ala Arg Ser Thr Pro Phe Ile Ala Asp Asn Val Leu
       50                  55                  60
   ctc cac gcg ctc gtt tgc gcc acc tac atc tac gtg cag ggc gtc atc      240
   Leu His Ala Leu Val Cys Ala Thr Tyr Ile Tyr Val Gln Gly Val Ile
   65                  70                  75                  80
   ttc tgg ggc ttc ttc acg gtc ggc cac gac tgc ggc cac tcg gcc ttc      288
   Phe Trp Gly Phe Phe Thr Val Gly His Asp Cys Gly His Ser Ala Phe
                   85                  90                  95
   tcg cgc tac cac agc gtc aac ttt atc atc ggc tgc atc atg cac tct      336
   Ser Arg Tyr His Ser Val Asn Phe Ile Ile Gly Cys Ile Met His Ser
               100                 105                 110
   gcg att ttg acg ccg ttc gag agc tgg cgc gtg acg cac cgc cac cac      384
   Ala Ile Leu Thr Pro Phe Glu Ser Trp Arg Val Thr His Arg His His
           115                 120                 125
   cac aag aac acg ggc aac att gat aag gac gag atc ttt tac ccg cac      432
   His Lys Asn Thr Gly Asn Ile Asp Lys Asp Glu Ile Phe Tyr Pro His
       130                 135                 140
   cgg tcg gtc aag gac ctc cag gac gtg cgc caa tgg gtc tac acg ctc      480
   Arg Ser Val Lys Asp Leu Gln Asp Val Arg Gln Trp Val Tyr Thr Leu
   145                 150                 155                 160
   ggc ggt gcg tgg ttt gtc tac ttg aag gtc ggg tat gcc ccg cgc acg      528
   Gly Gly Ala Trp Phe Val Tyr Leu Lys Val Gly Tyr Ala Pro Arg Thr
                   165                 170                 175
   atg agc cac ttt gac ccg tgg gac ccg ctc ctc ctt cgc cgc gcg tcg      576
   Met Ser His Phe Asp Pro Trp Asp Pro Leu Leu Leu Arg Arg Ala Ser
               180                 185                 190
   gcc gtc atc gtg tcg ctc ggc gtc tgg gcc gcc ttc ttc gcc gcg tac      624
   Ala Val Ile Val Ser Leu Gly Val Trp Ala Ala Phe Phe Ala Ala Tyr
           195                 200                 205
   gcg tac ctc aca tac tcg ctc ggc ttt gcc gtc atg ggc ctc tac tac      672
   Ala Tyr Leu Thr Tyr Ser Leu Gly Phe Ala Val Met Gly Leu Tyr Tyr
       210                 215                 220
   tat gcg ccg ctc ttt gtc ttt gct tcg ttc ctc gtc att acg acc ttc      720
   Tyr Ala Pro Leu Phe Val Phe Ala Ser Phe Leu Val Ile Thr Thr Phe
   225                 230                 235                 240
   ttg cac cac aac gac gaa gcg acg ccg tgg tac ggc gac tcg gag tgg      768
   Leu His His Asn Asp Glu Ala Thr Pro Trp Tyr Gly Asp Ser Glu Trp
                   245                 250                 255
   acg tac gtc aag ggc aac ctc tcg agc gtc gac cgc tcg tac ggc gcg      816
   Thr Tyr Val Lys Gly Asn Leu Ser Ser Val Asp Arg Ser Tyr Gly Ala
               260                 265                 270
   ttc gtg gac aac ctg agc cac cac att ggc acg cac cag gtc cac cac      864
   Phe Val Asp Asn Leu Ser His His Ile Gly Thr His Gln Val His His
           275                 280                 285
   ttg ttc ccg atc att ccg cac tac aag ctc aac gaa gcc acc aag cac      912
   Leu Phe Pro Ile Ile Pro His Tyr Lys Leu Asn Glu Ala Thr Lys His
       290                 295                 300
   ttt gcg gcc gcg tac ccg cac ctc gtg cgc agg aac gac gag ccc atc      960
   Phe Ala Ala Ala Tyr Pro His Leu Val Arg Arg Asn Asp Glu Pro Ile
   305                 310                 315                 320
   atc acg gcc ttc ttc aag acc gcg cac ctc ttt gtc aac tac ggc gct      1008
   Ile Thr Ala Phe Phe Lys Thr Ala His Leu Phe Val Asn Tyr Gly Ala
                   325                 330                 335
   gtg ccc gag acg gcg cag atc ttc acg ctc aaa gag tcg gcc gcg gcc      1056
   Val Pro Glu Thr Ala Gln Ile Phe Thr Leu Lys Glu Ser Ala Ala Ala
               340                 345                 350
   gcc aag gcc aag tcg gac taa                                          1077
   Ala Lys Ala Lys Ser Asp
           355
<210> 196
   <211> 358
   <212> PRT
   <213> Saprolegnia diclina
<400> 196
   Met Thr Glu Asp Lys Thr Lys Val Glu Phe Pro Thr Leu Thr Glu Leu
   1               5                   10                  15
   Lys His Ser Ile Pro Asn Ala Cys Phe Glu Ser Asn Leu Gly Leu Ser
               20                  25                  30
   Leu Tyr Tyr Thr Ala Arg Ala Ile Phe Asn Ala Ser Ala Ser Ala Ala
           35                  40                  45
   Leu Leu Tyr Ala Ala Arg Ser Thr Pro Phe Ile Ala Asp Asn Val Leu
       50                  55                  60
   Leu His Ala Leu Val Cys Ala Thr Tyr Ile Tyr Val Gln Gly Val Ile
   65                  70                  75                  80
   Phe Trp Gly Phe Phe Thr Val Gly His Asp Cys Gly His Ser Ala Phe
                   85                  90                  95
   Ser Arg Tyr His Ser Val Asn Phe Ile Ile Gly Cys Ile Met His Ser
               100                 105                 110
   Ala Ile Leu Thr Pro Phe Glu Ser Trp Arg Val Thr His Arg His His
           115                 120                 125
   His Lys Asn Thr Gly Asn Ile Asp Lys Asp Glu Ile Phe Tyr Pro His
       130                 135                 140
   Arg Ser Val Lys Asp Leu Gln Asp Val Arg Gln Trp Val Tyr Thr Leu
   145                 150                 155                 160
   Gly Gly Ala Trp Phe Val Tyr Leu Lys Val Gly Tyr Ala Pro Arg Thr
                   165                 170                 175
   Met Ser His Phe Asp Pro Trp Asp Pro Leu Leu Leu Arg Arg Ala Ser
               180                 185                 190
   Ala Val Ile Val Ser Leu Gly Val Trp Ala Ala Phe Phe Ala Ala Tyr
           195                 200                 205
   Ala Tyr Leu Thr Tyr Ser Leu Gly Phe Ala Val Met Gly Leu Tyr Tyr
       210                 215                 220
   Tyr Ala Pro Leu Phe Val Phe Ala Ser Phe Leu Val Ile Thr Thr Phe
   225                 230                 235                 240
   Leu His His Asn Asp Glu Ala Thr Pro Trp Tyr Gly Asp Ser Glu Trp
                   245                 250                 255
   Thr Tyr Val Lys Gly Asn Leu Ser Ser Val Asp Arg Ser Tyr Gly Ala
               260                 265                 270
   Phe Val Asp Asn Leu Ser His His Ile Gly Thr His Gln Val His His
           275                 280                 285
   Leu Phe Pro Ile Ile Pro His Tyr Lys Leu Asn Glu Ala Thr Lys His
       290                 295                 300
   Phe Ala Ala Ala Tyr Pro His Leu Val Arg Arg Asn Asp Glu Pro Ile
   305                 310                 315                 320
   Ile Thr Ala Phe Phe Lys Thr Ala His Leu Phe Val Asn Tyr Gly Ala
                   325                 330                 335
   Val Pro Glu Thr Ala Gln Ile Phe Thr Leu Lys Glu Ser Ala Ala Ala
               340                 345                 350
   Ala Lys Ala Lys Ser Asp
           355
<210> 197
   <211> 359
   <212> PRT
   <213> Artificial sequence
<220>
   <221> Variant
   <222> (2)..(5)
   <223> xaa in position 2 to 5 is any amino acid
<220>
   <221> Variant
   <222> (12)..(12)
   <223> xaa in position 12 is any amino acid
<220>
   <221> variant
   <222> (14)..(14)
   <223> xaa in position 14 is any amino acid
<220>
   <221> Variant
   <222> (16)..(16)
   <223> xaa in position 16 is any amino acid
<220>
   <221> Variant
   <222> (18)..(19)
   <223> xaa in position 18 to 19 is any amino acid
<220>
   <221> Variant
   <222> (23)..(26)
   <223> xaa in position 23 to 26 is any amino acid
<220>
   <221> Variant
   <222> (33)..(33)
   <223> xaa in position 33 is any amino acid
<220>
   <221> Variant
   <222> (35)..(38)
   <223> xaa in position 35 to 38 is any amino acid

<220>
   <221> Variant
   <222> (40)..(40)
   <223> xaa in position 40 is any amino acid
<220>
   <221> Variant
   <222> (42)..(45)
   <223> xaa in position 42 to 45 is any amino acid
<220>
   <221> Variant
   <222> (47)..(48)
   <223> xaa in position 47 to 48 is any amino acid
<220>
   <221> Variant
   <222> (51)..(52)
   <223> xaa in position 51 to 52 is any amino acid
<220>
   <221> Variant
   <222> (54)..(60)
   <223> xaa in position 54 to 60 is any amino acid
<220>
   <221> Variant
   <222> (62)..(62)
   <223> xaa in position 62 is any amino acid
<220>
   <221> variant
   <222> (64)..(65)
   <223> xaa in position 64 to 65 is any amino acid
<220>
   <221> Variant
   <222> (67)..(68)
   <223> xaa in position 67 to 68 is any amino acid
<220>
   <221> Variant
   <222> (71)..(72)
   <223> xaa in position 71 to 72 is any amino acid
<220>
   <221> Variant
   <222> (75)..(76)
   <223> xaa in position 75 to 76 is any amino acid
<220>
   <221> Variant
   <222> (87)..(87)
   <223> xaa in position 87 is any amino acid
<220>
   <221> Variant
   <222> (90)..(90)
   <223> xaa in position 90 is any amino acid
<220>
   <221> Variant
   <222> (97)..(98)
   <223> xaa in position 97 to 98 is any amino acid
<220>
   <221> Variant
   <222> (101)..(102)
   <223> xaa in position 101 to 102 is any amino acid
<220>
   <221> Variant
   <222> (104)..(105)
   <223> xaa in position 104 to 105 is any amino acid
<220>
   <221> Variant
   <222> (109)..(109)
   <223> xaa in position 109 is any amino acid
<220>
   <221> Variant
   <222> (118)..(119)
   <223> xaa in position 118 to 119 is any amino acid
<220>
   <221> Variant
   <222> (133)..(133)
   <223> xaa in position 133 is any amino acid
<220>
   <221> Variant
   <222> (136)..(136)
   <223> xaa in position 136 is any amino acid
<220>
   <221> Variant
   <222> (140)..(140)
   <223> xaa in position 140 is any amino acid
<220>
   <221> Variant
   <222> (142)..(144)
   <223> xaa in position 142 to 144 is any amino acid
<220>
   <221> Variant
   <222> (146)..(149)
   <223> xaa in position 146 to 149 is any amino acid
<220>
   <221> Variant
   <222> (151)..(155)
   <223> xaa in position 151 to 155 is any amino acid
<220>
   <221> Variant
   <222> (158)..(158)
   <223> xaa in position 158 is any amino acid
<220>
   <221> Variant
   <222> (161)..(162)
   <223> xaa in position 161 to 162 is any amino acid
<220>
   <221> Variant
   <222> (165)..(166)
   <223> xaa in position 165 to 166 is any amino acid
<220>
   <221> Variant
   <222> (168):.(169)
   <223> xaa in position 168 to 169 is any amino acid
<220>
   <221> Variant
   <222> (172)..(174)
   <223> xaa in position 172 to 174 is any amino acid
<220>
   <221> Variant
   <222> (177)..(177)
   <223> xaa in position 177 is any amino acid
<220>
   <221> Variant
   <222> (179)..(180)
   <223> xaa in position 179 to 180 is any amino acid
<220>
   <221> Variant
   <222> (183)..(184)
   <223> xaa in position 183 to 184 is any amino acid
<220>
   <221> Variant
   <222> (186)..(187)
   <223> xaa in position 186 to 187 is any amino acid
<220>
   <221> Variant
   <222> (191)..(192)
   <223> xaa in position 191 to 192 is any amino acid
<220>
   <221> Variant
   <222> (195)..(195)
   <223> xaa in position 195 is any amino acid
<220>
   <221> Variant
   <222> (196)..(197)
   <223> xaa in position 196 to 197 is any or no amino acid
<220>
   <221> Variant
   <222> (199)..(199)
   <223> xaa in position 199 is any amino acid
<220>
   <221> Variant
   <222> (202)..(202)
   <223> xaa in position 202 is any amino acid
<220>
   <221> Variant
   <222> (204)..(205)
   <223> xaa in position 204 to 205 is any amino acid
<220>
   <221> Variant
   <222> (206)..(207)
   <223> xaa in position 206 to 207 is any or no amino acid
<220>
   <221> Variant
   <222> (210)..(212)
   <223> xaa in position 210 to 212 is any amino acid
<220>
   <221> variant
   <222> (215)..(217)
   <223> xaa in position 215 to 217 is any amino acid
<220>
   <221> Variant
   <222> (219)..(220)
   <223> xaa in position 219 to 220 is any amino acid
<220>
   <221> Variant
   <222> (224)..(224)
   <223> xaa in position 224 is any amino acid
<220>
   <221> Variant
   <222> (226)..(226)
   <223> xaa in position 226 is any amino acid
<220>
   <221> variant
   <222> (230)..(230)
   <223> xaa in position 230 is any amino acid
<220>
   <221> Variant
   <222> (232)..(232)
   <223> xaa in position 232 is any amino acid
<220>
   <221> Variant
   <222> (245)..(245)
   <223> xaa in position 245 is any amino acid
<220>
   <221> Variant
   <222> (250)..(250)
   <223> xaa in position 250 is any amino acid
<220>
   <221> Variant
   <222> (271)..(272)
   <223> xaa in position 271 to 272 is any amino acid
<220>
   <221> Variant
   <222> (278)..(278)
   <223> xaa in position 278 is any amino acid
<220>
   <221> Variant
   <222> (284)..(284)
   <223> xaa in position 284 is any amino acid
<220>
   <221> Variant
   <222> (297)..(298)
   <223> xaa in position 297 to 298 is any amino acid
<220>
   <221> Variant
   <222> (301)..(302)
   <223> xaa in position 301 to 302 is any amino acid
<220>
   <221> Variant
   <222> (304)..(305)
   <223> xaa in position 304 to 305 is any amino acid
<220>
   <221> Variant
   <222> (307)..(307)
   <223> xaa in position 307 is any amino acid
<220>
   <221> Variant
   <222> (309)..(309)
   <223> xaa in position 309 is any amino acid
<220>
   <221> Variant
   <222> (313)..(314)
   <223> xaa in position 313 to 314 is any amino acid
<220>
   <221> Variant
   <222> (320)..(320)
   <223> xaa in position 320 is any amino acid
<220>
   <221> Variant
   <222> (324)..(327)
   <223> xaa in position 324 to 327 is any amino acid
<220>
   <221> Variant
   <222> (329)..(330)
   <223> xaa in position 329 to 330 is any amino acid
<220>
   <221> Variant
   <222> (334)..(334)
   <223> xaa in position 334 is any amino acid
<220>
   <221> Variant
   <222> (336)..(338)
   <223> xaa in position 336 to 338 is any amino acid
<220>
   <221> Variant
   <222> (340)..(341)
   <223> xaa in position 340 to 341 is any amino acid
<220>
   <221> Variant
   <222> (347)..(348)
   <223> xaa in position 347 to 348 is any amino acid
<220>
   <221> Variant
   <222> (349)..(352)
   <223> xaa in position 349 to 352 is any or no amino acid
<220>
   <221> Variant
   <222> (357)..(357)
   <223> xaa in position 357 is any amino acid
<400> 197
<210> 198
   <211> 60
   <212> PRT
   <213> Artificial sequence
<220>
   <221> Variant
   <222> (10)..(10)
   <223> xaa in position 10 is Ala or Glu
<220>
   <221> Variant
   <222> (15)..(15)
   <223> xaa in position 15 is Ala or Gly
<220>
   <221> Variant
   <222> (36)..(36)
   <223> xaa in position 36 is Phe or Leu
<220>
   <221> Variant
   <222> (37)..(37)
   <223> xaa in position 37 is Ile or Val
<220>
   <221> Variant
   <222> (43)..(43)
   <223> xaa in position 43 is any amino acid
<220>
   <221> Variant
   <222> (49)..(49)
   <223> xaa in position 49 is Ile or Val
<400> 198
<210> 199
   <211> 60
   <212> PRT
   <213> Artificial sequence
<220>
   <221> Variant
   <222> (6)..(6)
   <223> xaa in position 6 is Ala or Cys
<220>
   <221> Variant
   <222> (9)..(9)
   <223> xaa in position 9 is Gly or Ser
<220>
   <221> Variant
   <222> (16)..(16)
   <223> xaa in position 16 is any amino acid
<220>
   <221> Variant
   <222> (17)..(17)
   <223> xaa in position 17 is Leu or Val
<220>
   <221> Variant
   <222> (20)..(21)
   <223> xaa in position 20 to 21 is Ile or Val
<220>
   <221> Variant
   <222> (23)..(23)
   <223> xaa in position 23 is Cys or Thr
<220>
   <221> Variant
   <222> (24)..(24)
   <223> xaa in position 24 is Phe or Ile
<220>
   <221> variant
   <222> (28)..(28)
   <223> xaa in position 28 is Ala or Leu
<220>
   <221> Variant
   <222> (37)..(37)
   <223> xaa in position 37 is Lys or Arg
<220>
   <221> Variant
   <222> (38)..(38)
   <223> xaa in position 38 is Leu or Val
<220>
   <221> Variant
   <222> (52)..(52)
   <223> xaa in position 52 is Lys or Arg
<220>
   <221> Variant
   <222> (55)..(55)
   <223> xaa in position 55 is Ile or Val
<220>
   <221> variant
   <222> (59)..(59)
   <223> xaa in position 59 is any amino acid
<400> 199

## Beschreibung

Die vorliegende Erfindung betrifft ein Verfahren zur Herstellung von Eicosapentaensäure, Docosapentaensäure und/oder Docosahexaensäure in transgenen Pflanzen, indem in der Pflanze bereitgestellt werden.
● mindestens eine Nukleinsäuresequenz, welche für ein Polypeptid oder einen Teil davon mit einer Δ6-Desaturase-Aktivität kodiert mit einer zu mindestens 90% zu SEQ ID NO: 2 identischen Aminosäuresequenz;
● mindestens eine Nukleinsäuresequenz, welche für ein Polypeptid oder einen Teil davon mit einer Δ6-Elongase-Aktivität kodiert mit einer zu mindestens 90% zu SEQ ID NO: 172 identischen Aminosäuresequenz;
● mindestens eine Nukleinsäuresequenz, welche für ein Polypeptid oder einen Teil davon mit einer Δ5-Desaturase-Aktivität kodiert mit einer zu mindestens 90% zu SEQ ID NO: 52 identischen Aminosäuresequenz; und
● mindestens eine Nukleinsäuresequenz, welche für ein Polypeptid oder einen Teil davon mit einer Δ5-Elongase-Aktivität mit einer zu mindestens 90% zu SEQ ID NO: 65 identischen Aminosäuresequenz; und
● soweit das Verfahren die Produktion von Docosahexaensäure (DHA) bezweckt, mindestens eine Nukleinsäure, welche für ein Polypeptid oder einen Teil davon mit einer Δ4-Desaturase-Aktivität kodiert mit einer zu mindestens 90% zu SEQ ID NO: 78 identischen Aminosäuresequenz,
wobei die Nukleinsäuresequenz, welche für ein Polypeptid mit einer Δ5-Elongase-Aktivität kodiert, gegenüber der Nukleinsäuresequenz in dem Organismus, aus dem die Sequenz stammt, dadurch verändert ist, dass sie an die Kodonverwendung in einer oder mehreren Pflanzenarten angepasst ist, und wobei in den transgenen Pflanzen eine Steigerung der Ausbeute an langkettigen mehrfach ungesättigten Fettsäuren (LCPUFAs) von mindestens 50% gegenüber nicht-transgenen Pflanzen erreicht wird.

In einer bevorzugten Ausführungsform werden zusätzlich weitere Nukleinsäuresequenzen, die für ein Polypeptid mit der Aktivität einer ω3-Desaturase und/oder einer Δ4-Desaturase kodieren, in der Pflanze bereitgestellt.

In einer weiteren bevorzugten Ausfühzungsform werden weitere Nukleinsäuresequenzen, die für Acyl-CoA-Dehydrogenase(n), Acyl-ACP(= acyl carrier protein)-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) kodieren, in der Pflanze bereitgestellt.

Die Erfindung betrifft weiterhin rekombinante Nukleinsäuremoleküle, umfassend
a) eine oder mehrere Kopien von mindestens einem in Pflanzenzellen aktiven Promotor,
b) mindestens eine Nukleinsäuresequenz, kodierend für ein Polypeptid mit einer Δ6-Desaturase-Aktivität, wobei das Polypeptid eine zu mindestens 90% zu SEQ ID NO. 2 identische Aminosäuresequenz aufweist,
c) mindestens eine Nukleinsäuresequenz, kodierend für ein Polypeptid mit einer Δ5-Desaturase-Aktivität, wobei das Polypeptid eine zu mindestens 90% zu SEQ ID NO. 52 identische Aminosäuresequenz aufweist,
d) mindestens eine Nukleinsäuresequenz, kodierend für ein Polypeptid mit einer Δ6-Elongase-Aktivität, wobei das Polypeptid eine zu mindestens 90% zu SEQ ID NO. 172 identische Aminosäuresequenz aufweist,
e) mindestens eine Nukleinsäuresequenz, kodierend für ein Polypeptid mit einer Δ5-Elongase-Aktivität, wobei das Polypeptid eine zu mindestens 90% zu SEQ ID NO. 65 identische Aminosäuresequenz aufweist und die Nukleinsäuresequenz gegenüber der Nukleinsäuresequenz in dem Organismus, aus dem die Sequenz stammt, dadurch verändert ist, dass sie an die Kodonverwendung in einer oder mehreren Pflanzenarten angepasst ist, und
f) optional eine Nukleinsäuresequenz, kodierend für ein Polypeptid mit einer Δ4-Desaturase-Aktivität, wobei das Polypeptid eine zu mindestens 90% zu SEQ ID NO. 78 identische Aminosäuresequenz aufweist, und
g) eine oder mehrere Kopien von mindestens einer Terminatorsequenz.

Ein weiterer Teil der Erfindung betrifft Öle, Lipide und/oder Fettsäuren, die nach dem erfindungsgemäßen Verfahren hergestellt wurden. Ein weiterer Teil der Offenbarung betrifft deren Verwendung.

Schließlich betrifft die Erfindung auch transgene Pflanzen, die nach dem erfindungsgemäßen Verfahren hergestellt wurden oder die ein erfindungsgemäßes rekombinantes Nukleinsäuremolekül enthalten, wobei in den transgenen Pflanzen eine Steigerung der Ausbente an langfkettigen mehrfach ungesättigten Fettsäuren von mindestens 50% gegenüber nicht-transgenen Pflanzen erreicht wird, und deren Verwendung als Nahrungs- oder Futtermittel.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend für die weiteren Elongationen aus den Phospholipiden wieder in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Eine Übersicht über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung geben einschließlich der Literaturstellen die folgenden Artikel: Kinney (1997) Genetic Engeneering, Hrsgb.: JK Setlow, 19:149-166; Ohlrogge und Browse (1995) Plant Cell 7:957-970; Shanklin und Cahoon (1998) Annu. Rev. Plant Physiol. Plant Mol. Biol. 49:611-641; Voelker (1996) Genetic Engeneering, Hrsgb.: JK Setlow, 18:111-13; Gerhardt (1992) Prog. Lipid R. 31:397-417; Gühnemann-Schäfer & Kindl (1995) Biochim. Biophys Acta 1256:181-186; Kunau et al. (1995) Prog. Lipid Res. 34:267-342; Stymne et al. (1993) in: Biochemistry and Molecular Biology of Membrane and Storage Lipids of Plants, Hrsgb.: Murata und Somerville, Rockville, American Society of Plant Physiologists, 150-158; Murphy & Ross (1998) Plant Journal. 13(1):1-16.

Die mehrfach ungesättigten Fettsäuren können entsprechend ihrem Desaturierungsmuster in zwei große Klassen, die ω-6- und die ω-3-Fettsäuren, eingeteilt werden, die metabolisch und funktionell unterschiedliche Aktivitäten haben.

Im Folgenden werden mehrfach ungesättigte Fettsäuren als PUFA, PUFAs; LCPUFA oder LCPUFAs bezeichnet (poly unsaturated fatty acids, PUFA, mehrfach ungesättigte Fettsäuren; long chain poly unsaturated fatty acids, LCPUFA, langkettige mehrfach ungesättigte Fettsäuren).

Als Ausgangsstoff für den ω-6-Stoffwechselweg fungiert die Fettsäure Linolsäure (18:2^{Δ9,12}), während der ω-3-Weg über Linolensäure (18:3^{Δ9,12,15}) abläuft. Linolensäure wird dabei durch die Aktivität einer ω-3-Desaturase aus Linolsäure gebildet (Tocher et al. (1998) Prog. Lipid Res. 37: 73-117 ; Domergue et al. (2002) Eur. J. Biochem. 269: 4105-4113).

Säugetiere und damit auch der Mensch verfügen über keine entsprechende Desaturaseaktivität (Δ-12- und ω-3-Desaturase) zur Bildung dieser Ausgangsstoffe und müssen daher diese Fettsäuren (essentielle Fettsäuren) über die Nahrung aufnehmen. Über eine Abfolge von Desaturase- und Elongase-Reaktionen werden dann aus diesen Vorstufen die physiologisch wichtigen mehrfach ungesättigten Fettsäuren Arachidonsäure (= ARA, 20:4^{Δ5,8,11,14}) eine ω-6-Fettsäure und die beiden ω-3-Fettsäuren Eicosapentaen- (= EPA, 20:5^{Δ5,8,11,14,17}) und Docosahexaensäure (DHA, 22:6^{Δ4,7,10,13,17,19}) synthetisiert.

Die Verlängerung von Fettsäuren durch Elongasen um 2 bzw. 4 C-Atome ist für die Produktion von C₂₀- bzw. C₂₂-PUFAs von entscheidender Bedeutung. Dieser Prozess verläuft über 4 Stufen. Den ersten Schritt stellt die Kondensation von Malonyl-CoA an das Fettsäure-Acyl-CoA durch die Ketoacyl-CoA-Synthase (KCS, im weiteren Text als Elongase bezeichnet) dar. Es folgt dann ein Reduktionsschritt (Ketoacyl-CoA-Reduktase, KCR), ein Dehydratationsschritt (Dehydratase) und ein abschließender Reduktionsschritt (Enoyl-CoA-Reduktase). Es wurde postuliert, dass die Aktivität der Elongase die Spezifität und Geschwindigkeit des gesamten Prozesses beeintlusst (Millar and Kunst (1997) Plant Journal 12:121-131).

Für Fettsäuren und Triacylglyceride besteht eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. So werden z.B. in der humanen Ernährung Lipide mit ungesättigten, speziell mehrfach ungesättigten, Fettsäuren bevorzugt. Den mehrfach ungesättigten ω-3-Fettsäuren wird dabei ein positiver Effekt auf den Cholesterinspiegel im Blut und damit auf die Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden (Shimikawa (2001) World Rev. Nutr. Diet. 88: 100-108).

Auch entzündliche, speziell chronisch entzündliche, Prozesse im Rahmen immunologischer Erkrankungen wie rheumatoider Arthritis lassen sich durch ω-3-Fettsäuren positiv beeinflussen (Calder (2002) Proc. Nutr. Soc. 61: 345-358; Cleland und James (2000) J. Rheumatol. 27: 2305-2307). Sie werden deshalb Lebensmitteln, speziell diätetischen Lebensmitteln, zugegeben oder finden in Medikamenten Anwendung. ω-6-Fettsäuren wie Arachidonsäure üben bei diesen rheumatischen Erkrankungen eher einen negativen Effekt aus.

ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, und den Thromboxanen und Leukotrienen, die sich von der Arachidonsäure und der Eicosapentaensäure ableiten. Eicosanoide (sog. PG₂-Serie), die aus ω-6-Fettsäuren gebildet werden, fördern in der Regel Entzündungsreaktionen, während Eicosanoide (sog. PG₃-Serie) aus ω-3-Fettsäuren geringe oder keine entzündungsfördernde Wirkung haben.

Mehrfach ungesättigte langkettige ω-3-Fettsäuren wie Eicosapentaensäure (= EPA, C20:5^{Δ5,8,11,14,17}) oder Docosahexaensäure (=DHA, C22:6^{Δ4,7,10,13,16,19}) sind wichtige Komponenten der menschlichen Ernährung aufgrund ihrer verschiedenen Rollen in der Gesundheit, die Aspekte wie die Entwicklung des kindlichen Gehirns, die Funktionalität des Auges, die Synthese von Hormonen und anderer Signalstoffe, sowie die Vorbeugung von Herz-Kreislauf Beschwerden, Krebs und Diabetes umfassen (Poulos, A (1995) Lipids 30:1-14; Horrocks, LA und Yeo YK (1999) Pharmacol Res 40:211-225).

Aufgrund der heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, C22:6^{Δ4,7,10,13,16,19}) oder Eisosapentaensäure (= EPA, C20:5^{Δ5,8,11,14,17}) der Babynahrung zur Erhöhung des Nährwertes zugesetzt. Es besteht aus diesem Grund ein Bedarf an der Produktion mehrfach ungesättigter langkettiger Fettsäuren.

Hauptsächlich werden die verschiedenen Fettsäuren und Triglyceride aus Mikroorganismen wie Mortierella oder Schizochytrium oder aus Öl-produzierenden Pflanzen wie Soja, Raps und Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung der Triacylglyceride hergestellt. Sehr langkettige mehrfach ungesättigte Fettsäuren wie DHA, EPA, Arachidonsäure (ARA, C20:4^{Δ5,8,11,14}), Dihomo-γ-linolensäure (DHGL, C20:3^{Δ8,11,14}) oder Docosapentaensäure (DPA, C22:5^{Δ7,11,13,16,19}) werden in Ölfruchtpflanzen wie Raps, Soja, Sonnenblume, Färbersaflor jedoch nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch oder Algen.

Aufgrund der positiven Eigenschaften der mehrfach ungesättigten Fettsäuren hat es in der Vergangenheit nicht an Ansätzen gefehlt, Gene, die an der Synthese dieser Fettsäuren bzw. Triglyceride beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase und in WO 94/11516 eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al. (1990) J. Biol. Chem., 265: 20144-20149, Wada et al. (1990) Nature 347: 200-203 oder Huang et al. (1999) Lipids 34: 649-659 beschrieben. Die biochemische Charakterisierung der verschiedenen Desaturasen ist jedoch bisher nur unzureichend erfolgt; da die Enzyme als membrangebundene Proteine nur sehr schwer zu isolieren und zu charakterisieren sind (McKeon et al. (1981) Methods in Enzymol. 71: 12141-12147, Wang et al. (1988) Plant Physiol. Biochem., 26: 777-792).

In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, WO 96/21022, WO 00/21557 und WO 99/27111 beschrieben. Die Anwendung dieses Enzyms zur Produktion von Fettsäuren in transgenen Organismen wird in WO 98/46763, WO 98/46764 und WO 98/46765 beschrieben. Die Expression verschiedener Desaturasen und die Bildung mehrfach ungesättigter Fettsäuren wird auch in WO 99/64616 oder WO 98/46776 beschrieben und beansprucht. Bzgl. der Effektivität der Expression von Desaturasen und ihrem Einfluss auf die Bildung mehrfach ungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden.

In der Vergangenheit wurden zahlreiche Versuche unternommen, Elongase-Gene zu erhalten. Millar and Kunst (1997) Plant Journal 12:121-131 und Millar et al. (1999) Plant Cell 11:825-838 beschreiben die Charakterisierung von pflanzlichen Elongasen zur Synthese von einfach ungesättigten langkettigen Fettsäuren (C22:1) bzw. zur Synthese von sehr langkettigen Fettsäuren für die Wachsbildung in Pflanzen (C₂₈-C₃₂). Beschreibungen zur Synthese von Arachidonsäure und EPA finden sich beispielsweise in WO 01/59128, WO 00/12720, WO 02/077213 und WO 02/08401. Die Synthese von mehrfach ungesättigter C24-Fettsäuren ist beispielsweise in Tvrdik et al. (2000) J. Cell Biol. 149: 707-718 oder in WO 02/44320 beschrieben.

Besonders geeignete Mikroorganismen zur Herstellung von PUFAs sind Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moose wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Mit Hilfe der vorgenannten Mikroorganismen lassen sich zudem nur begrenzte Mengen der gewünschten mehrfach ungesättigten Fettsäuren wie DPA, EPA oder ARA herstellen, die noch dazu in der Regel als Fettsäuregemische anfallen. Deshalb werden, wann immer möglich, gentechnologische Verfahren bevorzugt.

Höhere Pflanzen enthalten mehrfach ungesättigte Fettsäuren wie Linolsäure (C18:2) und Linolensäure (C 18:3). ARA, EPA und DHA kommen im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Hierzu werden vorteilhafterweise über gentechnische Methoden Gene, die für Enzyme der Biosynthese von LCPUFAs kodieren, in Ölsaaten eingeführt und exprimiert. Dies sind Gene, die beispielsweise für Δ-6-Desaturasen, Δ-6-Elongasen, Δ-5-Desaturasen oder Δ-4-Desaturasen kodieren. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert werden.

Transgene Pflanzen, die für Enzyme der LCPUFA-Biosynthese kodierende Gene enthalten und exprimieren und als Folge dessen LCPUFAs produzieren, wurden beispielsweise in DE-A-102 19 203 (Verfahren zur Herstellung mehrfach ungesättigter Fettsäuren in Pflanzen) beschrieben. Diese Pflanzen produzieren allerdings LCPUFAs in Mengen, die für eine Aufarbeitung der in den Pflanzen enthaltenen Öle noch weiter optimiert werden müssen. So beträgt der Gehalt von ARA in den in DE-A-102 19 203 beschriebenen Pflanzen lediglich 0,4 bis 2% und der Gehalt von EPA lediglich 0,5 bis 1 %, jeweils bezogen auf den Gesamtlipidgehalt der Pflanze.

Um eine Anreicherung der Nahrung und des Futters mit mehrfach ungesättigten, langkettigen Fettsäuren zu ermöglichen, besteht daher ein großer Bedarf an einem einfachen, kostengünstigen Verfahren zur Herstellung von mehrfach ungesättigten, langkettigen Fettsäuren speziell in pflanzlichen Systemen.

Eine Aufgabe der Erfindung ist es daher, ein Verfahren bereitzustellen, mit dem langkettige, mehrfach ungesättigte Fettsäuren, insbesondere Eicosapentaensäure, Docosapentaensäure und/oder Docosahexaensäure, in transgenen Pflanzen in großer Menge preiswert hergestellt werden können.

Es wurde nun überraschenderweise herausgefunden, dass durch die Expression einer optimierten Δ5-Elongase-Sequenz in transgenen Pflanzen die Ausbeute an langkettigen, mehrfach ungesättigten Fettsäuren, insbesondere Eicosapentaensäure, Docosapentaensäure und/oder Docosahexaensäure, gesteigert werden kann.

Die durch das erfindungsgemäße Verfahren hergestellten PUFAs umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden können.

Entsprechend wird die Aufgabe der Erfindung gelöst durch das erfindungsgemäße Verfahren zur Herstellung von Eicosapentaensäure, Docosapentaensäure und/oder Docosahexaensäure in einer transgenen Pflanze, umfassend das Bereitstellen in der Pflanze von
● mindestens einer Nukleinsäuresequenz, welche für ein Polypeptid oder einen Teil davon mit einer Δ6-Desaturase-Aktivität kodiert mit einer zu mindestens 90% zu SEQ ID NO: 2 identischen Aminosäuresequenz;
● mindestens einer Nukleinsäuresequenz, welche für ein Polypeptid oder einen Teil davon mit einer Δ6-Elongase-Aktivität kodiert mit einer zu mindestens 90% zu SEQ ID NO: 172 identischen Aminosäuresequenz;
● mindestens einer Nukleinsäuresequenz, welche für ein Polypeptid oder einen Teil davon mit einer Δ5-Desaturase-Aktivität kodiert mit einer zu mindestens 90% zu SEQ ID NO: 52 identischen Aminosäuresequenz; und
● mindestens einer Nukleinsäuresequenz, welche für ein Polypeptid oder einen Teil davon mit einer Δ5-Elongase-Aktivität mit einer zu mindestens 90% zu SEQ ID NO: 65 identischen Aminosäuresequenz; und
● soweit das Verfahren die Produktion von Docosahexaensäure (DHA) bezweckt, mindestens einer Nukleinsäure, welche für ein Polypeptid oder einen Teil davon mit einer Δ4-Desaturase-Aktivität kodiert mit einer zu mindestens 90% zu SEQ ID NO: 78 identischen Aminosäuresequenz,
wobei die Nukleinsäuresequenz, welche für ein Polypeptid mit einer Δ5-Elongase-Aktivität kodiert, gegenüber der Nukleinsäuresequenz in dem Organismus, aus dem die Sequenz stammt, dadurch verändert ist, dass sie an die Kodonverwendung in einer oder mehreren Pflanzenarten angepasst ist, und wobei in den transgenen Pflanzen eine Steigerung der Ausbeute an langkettigen mehrfach ungesättigten Fettsäuren (LCPUFAs) von mindestens 50% gegenüber nicht-transgenen Pflanzen erreicht wird.

Das "Bereitstellen in der Pflanze" bedeutet im Sinne der vorliegenden Erfindung, dass Maßnahmen getroffen werden, so dass die Nukleinsäuresequenzen kodierend für ein Polypeptid mit einer Δ6-Desaturase-Aktivität, ein Polypeptid mit einer Δ6-Elongase-Aktivität, ein Polypeptid mit einer Δ5-Desaturase-Aktivität und ein Polypeptid mit einer Δ5-Elongase-Aktivität zusammen in einer Pflanze vorliegen. Das "Bereitstellen in der Pflanze" umfasst somit das Einbringen der Nukleinsäuresequenzen in die Pflanze sowohl durch Transformation einer Pflanze mit einem oder mehreren rekombinanten Nukleinsäuremolekülen, die die genannten Nukleinsäuresequenzen enthalten, als auch durch Verkreuzung von geeigneten Elternpflanzen, die eine oder mehrere der genannten Nukleinsäuresequenzen enthalten.

Die Nukleinsäuresequenz, die für ein Polypeptid mit einer Δ5-Elongase-Aktivität kodiert, ist erfindungsgemäß gegenüber der Nukleinsäuresequenz in dem Organismus, aus dem die Sequenz stammt, dadurch verändert, dass sie an die Kodonverwendung in einer oder mehreren Pflanzenarten angepasst ist. Dies bedeutet, dass die Nukleinsäuresequenz gezielt für die Zwecke der Erfindung optimiert wurde, ohne dass dadurch die von der Nukleinsäuresequenz kodierte Aminosäuresequenz verändert wurde.

Der genetische Code ist redundant, da er 61 Kodons verwendet, um 20 Aminosäuren zu spezifizieren. Daher werden die meisten der 20 proteinogenen Aminosäuren von mehreren Tripletts (Kodons) kodiert. Die synonymen Kodons, die eine einzelne Aminosäure spezifizieren, werden in einem bestimmten Organismus jedoch nicht mit gleicher Häufigkeit verwendet, sondern es gibt bevorzugte Kodons, die häufig verwendet werden und Kodons, die seltener verwendet werden. Diese Unterschiede in der Kodonverwendung werden zurückgeführt auf selektive evolutionäre Drücke und vor allem die. Effizienz der Translation. Ein Grund für die geringere Translationseffizienz von selten auftretenden Kodons könnte darin liegen, dass die entsprechenden Aminoacyl-tRNA-Pools erschöpft werden und damit nicht mehr zur Proteinsynthese zur Verfügung stehen.

Außerdem bevorzugen unterschiedliche Organismen unterschiedliche Kodons. Daher läuft beispielsweise die Expression einer rekombinanten DNA, die aus einer Säugerzelle stammt, in E. coli-Zellen häufig nur suboptimal ab. Deshalb kann der Austausch selten verwendeter Kodons gegen häufig verwendete Kodons in manchen Fällen die Expression erhöhen. Ohne an eine Hypothese gebunden sein zu wollen, wird angenommen, dass die kodonoptimierten DNA-Sequenzen eine effizientere Translation ermöglichen und die daraus gebildeten mRNAs möglicherweise eine höhere Halbwertszeit in der Zelle besitzen und daher häufiger für die Translation zur Verfügung stehen. Aus dem vorstehend gesagten folgt, dass eine Kodonoptimierung nur dann nötig ist, wenn der Organismus, in dem die Nukleinsäuresequenz exprimiert werden soll, ein anderer ist als der Organismus, aus dem die Nukleinsäuresequenz ursprünglich stammt.

Für viele Organismen, von denen die DNA-Sequenz einer größeren Zahl von Genen bekannt ist, gibt es Tabellen, denen man die Häufigkeit der Verwendung bestimmter Kodons in dem jeweiligen Organismus entnehmen kann. Mit Hilfe dieser Tabellen lassen sich Proteinsequenzen mit relativ großer Genauigkeit in eine DNA-Sequenz zurückübersetzen, die die im jeweiligen Organismus bevorzugten Kodons für die verschiedenen Aminosäuren des Proteins enthält. Tabellen zur Kodonverwendung können u.a. unter der folgenden Internet-Adresse aufgefunden werden:
http://www.kazusa.or.ip/Kodon/E.html. Darüber hinaus bieten mehrere Firmen Software für die Genoptimierung an, wie z.B. die Firma Entelechon (Software Leto) oder die Firma Geneart (Software GeneOptimizer).

Die Anpassung der Sequenzen an die Kodonverwendung in einem bestimmten Organismus kann unter Zuhilfenahme verschiedener Kriterien erfolgen. Zum einen kann für eine bestimmte Aminosäure immer das am häufigsten im ausgewählten Organismus vorkommende Kodon verwendet werden, zum anderen kann aber auch die natürliche Frequenz der verschiedenen Kodons berücksichtigt werden, so dass alle Kodons für eine bestimmte Aminosäure entsprechend ihrer natürlichen Häufigkeit in die optimierte Sequenz eingebaut werden. Dabei kann die Auswahl, an welcher Position welches Basen-Triplett verwendet wird, zufällig stattfinden. Erfindungsgemäß wurde die DNA-Sequenz unter Berücksichtigung der natürlichen Häufigkeit einzelner Kodons angepasst, wobei die Verwendung des am häufigsten im ausgewählten Organismus vorkommenden Kodons ebenfalls geeignet ist.

Besonders bevorzugt ist eine Nukleinsäuresequenz aus *Ostreococcus tauri,* die für ein Polypeptid mit einer Δ5-Elongase-Aktivität kodiert, wie beispielsweise das in SEQ ID No. 110 dargestellte Polypeptid, zumindest an die Kodonverwendung in Raps, Soja und/oder Lein angepasst. Bei der ursprünglich aus *Ostreococcus tauri* stammenden Nukleinsäuresequenz handelt es sich bevorzugt um die in SEQ ID No. 109 )dargestellte Sequenz. Die für die Δ5-Elongase kodierende DNA-Sequenz ist an mindestens 20% der Positionen, bevorzugt an mindestens 30% der Positionen, besonders bevorzugt an mindestens 40% Positionen und am meisten bevorzugt an mindestens 50% der Positionen an die Kodonverwendung in Raps, Soja und/oder Lein angepasst.

Am meisten bevorzugt handelt es sich bei der verwendeten Nukleinsäuresequenz um die in SEQ ID No. 64 angegebene Sequenz.

Es versteht sich, dass auch solche kodonoptimierten DNA-Sequenzen von der Erfindung erfasst sind, die für ein Polypeptid mit der Aktivität einer Δ5-Elongase kodieren, dessen Aminosäuresequenz an einer oder mehreren Positionen gegenüber der Wildtyp-Sequenz verändert ist, das aber noch im wesentlichen die gleiche Aktivität aufweist wie das Wildtyp-Protein.

Bevorzugt ist die Nukleinsäuresequenz, die für ein Polypeptid mit einer Δ6-Desaturase-Aktivität kodiert, ausgewählt aus der Gruppe bestehend aus:
a) Nukleinsäuresequenzen mit der in SEQ ID NO.
   1,3,5,7,9,11,13,15,17,19,21,23,25,27,29,31,33,35,37,39 oder 41, bevorzugt mit der in SEQ ID No. 1 dargestellten Sequenz,
b) Nukleinsäuresequenzen, die für die in SEQ ID No.
   2,4,6,8,10,12,14,16,18,20,22,24,26,28,30,32,34,36,38,40 oder 42, bevorzugt in SEQ ID No. 2 angegebene Aminosäuresequenz kodieren,
c) Nukleinsäuresequenzen, die mit dem komplementären Strang der a) oder b) oberhalb angegebenen Nukleinsäuresequenzen, insbesondere der in SEQ ID No. 1 angegebenen Nukleinsäuresequenz unter stringenten Bedingungen hybridisieren,
d) Nukleinsäuresequenzen, die zu den in a) oder b) oberhalb angegebenen Nukleinsäuresequenzen, insbeondere zur der in SEQ ID No. 1 angegebenen Sequenz zu mindestens 60%, 65%, 70%, 75% oder 80%, bevorzugt zu mindestens 81%, 82%, 83%, 84%, 85%, 86%, 87%, 88%, 89% oder 90%, besonders bevorzugt zu mindestens 91%, 92%, 93%, 94% oder 95% und insbesondere zu mindestens 96%, 97%, 98% oder 99% identisch sind, und
e) Nukleinsäuresequenzen, die für eine Aminosäuresequenz kodieren, die mindestens eines, beispielsweise 2, 3, 4, 5, 6, 7 oder 8, vorzugsweise alle der in SEQ ID No. 43,44,45,46,47,48,49 oder 50 angegebenen Aminosäurepattern aufweisen.

Unter Aminosäurepattern sind kurze Aminosäuresequenzen zu verstehen, die vorzugsweise weniger als 50, besonders bevorzugt weniger als 40 und insbesondere von 10 bis 40 und noch weiter bevorzugt von 10 bis 30 Aminosäuren umfassen.

Für die vorliegende Erfindung wird die Identität vorzugsweise über die Vollänge der erfindungsgemäßen Nukleotid-oder Aminosäuresequenzen ermittelt, beispielsweise für die in SEQ ID NO: 64 angegebene Nukleinsäuresequenz über die Vollänge von 903 Nukleotiden.

Bevorzugt ist die Nukleinsäuresequenz, die für ein Polypeptid mit einer Δ6-Elongasen-Aktivität kodiert, ausgewählt aus der Gruppe bestehend aus:
a) Nukleinsäuresequenzen mit der in SEQ ID No. 171, 173,175,177,179,181 oder 183, insbesondere mit der in SEQ ID No. 171 dargestellten Sequenz,
b) Nukleinsäuresequenzen, die für die in SEQ ID No. 172, 174,176,178,180,182 oder184, insbesondere für die in SEQ ID No. 172 angegebene Aminosäuresequenz kodieren,
c) Nukleinsäuresequenzen, die mit dem komplementären Strang der a) oder b) oberhalb angegebenen Nukleinsäuresequenzen, insbesondere der in SEQ ID No. 1 angegebenen Nukleinsäuresequenz unter stringenten Bedingungen hybridisieren,
d) Nukleinsäuresequenzen, die zu den in a) oder b) oberhalb angegebenen Nukleinsäuresequenzen, insbesondere zu der in SEQ ID No. 171 angegebenen Sequenz zu mindestens 60%, 65%, 70%, 75% oder 80%, bevorzugt zu mindestens 81%, 82%, 83%, 84%, 85%, 86%, 87%, 88%, 89% oder 90%, besonders bevorzugt zu mindestens 91%, 92%, 93%, 94% oder 95% und insbesondere zu mindestens 96%, 97%, 98% oder 99% identisch sind, und
e) Nukleinsäuresequenzen, die für eine Aminosäuresequenz kodieren, die mindestens eines, beispielsweise 2, 3, 4, 5, 6, 7 oder 8, vorzugsweise alle der in SEQ ID No. 185,186,187,188,189,190,191 oder192 angebebenen Aminosäurepattern aufweisen.

Insbesondere handelt es sich bei der Nukleinsäuresequenz, die für ein Polypeptid mit einer Δ6-Elongase-Aktivität kodiert ebenfalls um eine nach der vorliegenden Erfindung kodonoptimierten Sequenz, bevorzugt um die in der SEQ ID NO: 122 darstellte Nukleinsäuresequenz.

Bevorzugt ist die Nukleinsäuresequenz, die für ein Polypeptid mit einer Δ5-Desaturase-Aktivität kodiert, ausgewählt aus der Gruppe bestehend aus:
a) Nukleinsäuresequenzen mit der in SEQ ID No. 51, 53 oder 55, bevorzugt mit der in SEQ ID No. 51 dargestellten Sequenz,
b Nukleinsäuresequenzen, die für die in SEQ ID No. 52, 54 oder 56, bevorzugt mit der in SEQ ID No. 52 angegebenen Aminosäuresequenz kodieren,
c) Nukleinsäuresequenzen, die mit dem komplementären Strang der in a) oder b) oberhalb angegebenen Nukleinsäuresequenzen, insbesondere der in SEQ ID No. 51 angegebenen Nukleinsäuresequenz unter stringenten Bedingungen hybridisieren, und
d) Nukleinsäuresequenzen, die zu den in a) oder b) oberhalb angegebenen Nukleinsäuresequenzen, insbesondere zur der in SEQ ID No. 51 angegebenen Sequenz zu mindestens 60%, 65%, 70%, 75% oder 80%, bevorzugt zu mindestens 81%, 82%, 83%, 84%, 85%, 86%, 87%, 88%, 89% oder 90%, besonders bevorzugt zu mindestens 91%, 92%, 93%, 94% oder 95% und insbesondere zu mindestens 96%, 97%, 98% oder 99% identisch sind, und
e) Nukleinsäuresequenzen, die für eine Aminosäuresequenz kodieren, die mindestens eines, beispielsweise 2, 3, 4, 5, 6 oder 7 , vorzugsweise alle der in SEQ ID No. 57,58,59,60,61,62 oder 63 angebebenen Aminosäurepattern aufweisen.

Weitere geeignete Nukleinsäuresequenzen kann der Fachmann der Literatur bzw. den bekannten Genbanken wie z.B. http://www.ncbi.nlm.nih.gov entnehmen.

In einer weiteren bevorzugten Ausführungsform des Verfahrens werden zusätzlich eine oder mehrere Nukleinsäuresequenzen, die für ein Polypeptid mit der Aktivität einer ω-3-Desaturase und/oder einer Δ4-Desaturase kodieren, in die Pflanze eingebracht.

Bevorzugt ist die Nukleinsäuresequenz, die für ein Polypeptid mit einer ω-3-Desaturase-Aktivität kodiert, ausgewählt aus der Gruppe bestehend aus:
a) Nukleinsäuresequenzen mit der in SEQ ID No. 193 oder 195, vorzugsweise der in SEQ ID No. 193 dargestellten Sequenz,
b) Nukleinsäuresequenzen, die für die in SEQ ID No. 194 angegebene Aminosäuresequenz kodieren,
c) Nukleinsäuresequenzen, die mit dem komplementären Strang der in SEQ ID No. 193 oder 195 angegebenen Nukleinsäuresequenz unter stringenten Bedingungen hybridisieren, und
d) Nukleinsäuresequenzen, die zu der in SEQ ID No. 193 oder 195 angegebenen Sequenz zu mindestens 60%, 65%, 70%, 75% oder 80%, bevorzugt zu mindestens 81%, 82%, 83%, 84%, 85%, 86%, 87%, 88%, 89% oder 90%, besonders bevorzugt zu mindestens 91%, 92%, 93%, 94% oder 95% und insbesondere zu mindestens 96%, 97%, 98% oder 99% identisch sind.

Die im erfindungsgemäßen Verfahren vorteilhaft verwendete ω-3-Desaturase ermöglicht eine Verschiebung vom ω-6-Biosyntheseweg zum ω-3-Biosyntheseweg, was zu einer Verschiebung von C_{18:2}- zu C_{18:3}-Fettsäuren führt. Weiterhin ist vorteilhaft, dass die ω-3-Desaturase eine breite Palette von Phospholipiden wie Phosphatidylcholin (= PC), Phosphatidylinositol (= PIS) oder Phosphatidylethanolamin (= PE) umsetzt. Schließlich lassen sich auch Desaturierungsprodukte in den Neutrallipiden (= NL), das heißt in den Triglyceriden finden.

Bevorzugt ist die Nukleinsäuresequenz, die für ein Polypeptid mit einer Δ4-Desaturase-Aktivität kodiert, ausgewählt aus der Gruppe bestehend aus:
a) Nukleinsäuresequenzen mit der in SEQ ID No. 77, 79,81,83,85,87,89,91 oder 93, bevorzugt mit der in der SEQ ID No. 77 dargestellten Sequenz,
b) Nukleinsäuresequenzen, die für die in SEQ ID No. 78, 80,82,84,86,88,90,92 oder 94, bevorzugt für die in SEQ ID No. 78 angegebene Aminosäuresequenz kodieren,
c) Nukleinsäuresequenzen, die mit dem komplementären Strang der in a) oder b) oberhalb angegebenen Nukleinsäuresequenzen, insbesondere der in SEQ ID No. 77 angegebenen Nukleinsäuresequenz unter stringenten Bedingungen hybridisieren, und
d) Nukleinsäuresequenzen, die zu der in ID No. 77 angegebenen Sequenz zu mindestens 60%, 65%, 70%, 75% oder 80%, bevorzugt zu mindestens 81%, 82%, 83%, 84%, 85%, 86%, 87%, 88%, 89% oder 90%, besonders bevorzugt zu mindestens 91%, 92%, 93%, 94% oder 95% und insbesondere zu mindestens 96%, 97%, 98% oder 99% identisch sind, und
e) Nukleinsäuresequenzen, die für eine Aminosäuresequenz kodieren, die mindestens eines, beispielsweise 2, 3, 4, 5, 6, 7, 8, 9, 10, 11, 12, 13 oder 14, vorzugsweise alle der in SEQ ID No. 95,96,97,98,99,100,101,102,103,104,105,106,107 oder 108 angebebenen Aminosäurepattern aufweisen.

Die Δ4-Desaturase, die im erfindungsgemäßen Verfahren vorteilhaft verwendet wird, katalysiert die Einführung einer Doppelbindung in die Fettsäure Docosapentaensäure, was zur Bildung von Docosahexaensäure führt.

Für das erfindungsgemäße beschriebene Verfahren ist es vorteilhaft, in die Pflanzen zusätzlich zu den Nukleinsäuresequenzen, die für Polypeptide mit einer Δ6-Desaturase-Aktivität, einer Δ6-Elongase-Aktivität, einer Δ5-Desaturase-Aktivität und einer Δ5-Elongase-Aktivität kodieren, sowie den ggf. eingebrachten Nukleinsäuresequenzen, die für ein Polypeptid mit einer ω-3-Desaturase-Aktivität und/oder einer Δ4-Desaturase-Aktivität kodieren, zusätzlich weitere Nukleinsäuren einzubringen, die für Enzyme des Fettsäure- oder Lipidstoffwechsels kodieren.

Im Prinzip können alle Gene des Fettsäure- oder Lipidstoffwechsels in Kombination mit den im erfindungsgemäßen Verfahren verwendeten Nukleinsäuresequenzen verwendet werden; bevorzugt werden Gene des Fettsäure- oder Lipidstoffwechsels ausgewählt aus der Gruppe Acyl-CoA-Dehydrogenase(n), Acyl-ACP(= acyl carrier protein)-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in Kombination mit der Δ-6-Elongase, Δ-6-Desaturase, Δ-5-Desaturase und der Δ-5-Elongase sowie ggf. der ω-3-Desaturase und/oder der Δ-4-Desaturase verwendet, wobei einzelne Gene oder mehrere Gene in Kombination verwendet werden können.

Vorteilhaft werden die im erfindungsgemäßen Verfahren verwendeten Nukleinsäuren im vegetativen Gewebe (= somatischem Gewebe) exprimiert. Unter vegetativem Gewebe ist im Sinne dieser Erfindung ein Gewebe zu verstehen, das sich durch mitotische Teilungen vermehrt. Derartiges Gewebe entsteht auch durch asexuelle Fortpflanzung (= Apomixis) und Vermehrung. Von Vermehrung spricht man dann, wenn sich die Zahl der Individuen in aufeinander folgenden Generationen erhöht. Diese durch asexuelle Vermehrung entstandenen Individuen sind mit ihren Eltern weitestgehend identisch. Beispiele für derartige Gewebe sind Blatt, Blüte, Wurzel, Stengel, oberirdische oder unterirdische Ausläufer (Seitensprosse, Stolonen), Rhizome, Knospen, Knollen wie Wurzelknollen oder Ausläuferknollen, Zwiebel, Brutkörper, Brutknospen, Bulbillen oder Turione. Derartige Gewebe können auch durch unechte, echte oder durch den Mensch verursachte Viviparie entstehen. Aber auch Samen, die durch Agamospermie, wie sie für Asteraceae, Poaceae oder Rosaceae typisch sind, entstanden sind, gehören zu den vegetativen Geweben, in denen vorteilhaft die Expression stattfindet. Zu einem geringeren Teil oder gar nicht werden die im erfindungsgemäßen Verfahren verwendeten Nukleinsäuren im generativen Gewebe (Keimbahngewebe) exprimiert. Beispiele für derartige Gewebe sind Gewebe, die durch geschlechtliche Fortpflanzung, d.h. meiotische Zellteilungen entstehen, wie z.B. Samen, die durch geschlechtliche Prozesse entstanden sind.

Unter zu einem geringen Teil ist zu verstehen, dass im Vergleich zum vegetativen Gewebe die Expression gemessen auf RNA- und/oder Proteinebene weniger als 5 %, vorteilhaft weniger als 3 %, besonders vorteilhaft weniger als 2 %, am meisten bevorzugt weniger als 1; 0,5; 0,25 oder 0,125% beträgt.

Besonders bevorzugt werden die Nukleinsäuresequenzen in den Blättern der transgenen Pflanzen exprimiert. Dies hat den Vorteil, dass die erfindungsgemäß hergestellten LCPUFAs von Tieren und Menschen direkt durch den Verzehr der Blätter aufgenommen werden können und keine vorherige Aufarbeitung des Pflanzenmaterials erforderlich ist.

Die Expression der erfindungsgemäßen Nukleinsäuresequenzen im Blatt kann durch die Verwendung von konstitutiven oder blattspezifischen Promotoren erreicht werden.

"Konstitutive Promotoren" sind Promotoren, die die Expression in einer Vielzahl, vorzugsweise in allen, Geweben über einen wesentlichen Zeitraum während der pflanzlichen Entwicklung, bevorzugt während der gesamten pflanzlichen Entwicklung, ermöglichen. Bevorzugt wird ein Promotor aus einer Pflanze oder aus einem Pflanzenvirus verwendet. Bevorzugt sind der Promotor des CaMV (cauliflower mosaic virus) 35S-Transkripts (Franck et al. (1980) Cell 21: 285-294), der 19S CaMV-Promotor (US 5,352,605), der Aktinpromotor aus Reis (McElroy et al. (1990) Plant Cell 2: 163-171), der Legumin B-Promotor (GenBank Acc. No. X03677), der Promotor der Nopalinsynthase aus Agrobacterium, der TR duale Promotor, der Octopinsynthase-Promotor aus Agrobacterium, der Ubiquitin-Promotor (Holtorf et al. (1995) Plant Mol. Biol. 29: 637-649), der Smas-Promotor, der Cinnamoylalkoholdehydrogenase-Promotor (US 5,683,439), die Promotoren der vacuolaren ATPase-Untereinheiten, der pEMU-Promotor (Last et al. (1991) Theor. Appl. Genet. 81: 581-588), der MAS-Promotor (Velten et al. (1984) EMBO J. 3(12): 2723-2730), der Histon-H3-Promotor aus Mais (Lepetit et al. (1992) Mol. Gen. Genet. 231: 276-285), der Promotor des Nitrilase 1-Gens aus Arabidopsis (GenBank Acc. No. U38846, Nukleotide 3862-5325) und der Promotor eines Prolin-reichen Proteins aus Weizen (WO 91/13991) und weitere Promotoren, die konstitutive Genexpression vermitteln. Besonders bevorzugt ist der Promotor des CaMV 35S-Transkripts.

Es ist im Prinzip möglich, alle natürlich auftretenden konstitutiven Promotoren mit ihren Regulationssequenzen, wie die oben genannten, für das neue Verfahren zu verwenden. Es ist aber ebenfalls möglich, zusätzlich oder alleine synthetische Promotoren zu verwenden.

"Blattspezifische Promotoren" sind Promotoren, die eine hohe Aktivität im Blatt und keine oder nur eine geringe Aktivität in anderen Geweben zeigen. Unter "geringer Aktivität" wird im Rahmen der Erfindung verstanden, dass die Aktivität in anderen Geweben weniger als 20%, bevorzugt weniger als 10%, besonders bevorzugt weniger als 5% und am meisten bevorzugt weniger als 3, 2 oder 1 % der Aktivität im Blatt beträgt. Geeignete blattspezifische Promotoren sind z.B. die Promotoren der kleinen Untereinheit von Rubisco (Timko et al. (1985) Nature 318: 579-582) und des Chlorophyll a/b-bindenden Proteins (Simpson et al. (1985) EMBO J. 4: 2723-2729).

Dem Fachmann sind weitere blattspezifische Promotoren bekannt bzw. er kann mit bekannten Methoden weitere geeignete Promotoren zu isolieren. So kann der Fachmann mit Hilfe gängiger molekularbiologischer Methoden, z. B. Hybridisierungsexperimenten oder DNA-Protein-Bindungsstudien, Blatt-spezifische regulatorische Nukleinsäurelemente identifizieren. Dabei wird z. B. in einem ersten Schritt aus Blattgewebe des gewünschten Organismus, aus dem die regulatorischen Sequenzen isoliert werden sollen, die gesamte poly(A)⁺-RNA isoliert und eine cDNA-Bank angelegt. In einem zweiten Schritt werden mit Hilfe von cDNA-Klonen, die auf poly(A)⁺-RNA-Molekülen aus einem Nicht-Blattgewebe basieren, aus der ersten Bank mittels Hybridisierung diejenigen Klone identifiziert, deren korrespondierende poly(A)⁺-RNA-Moleküle lediglich im Blattgewebe akkumulieren. Anschließend werden mit Hilfe dieser so identifizierten cDNAs Promotoren isoliert, die über Blatt-spezifische regulatorische Elemente verfügen. Dem Fachmann stehen darüber hinaus weitere auf PCR basierende Methoden für die Isolierung geeigneter Blatt-spezifischer Promotoren zur Verfügung.

Selbstverständlich können die Nukleinsäuresequenzen der vorliegenden Erfindung auch in den Samen der transgenen Pflanzen exprimiert werden, indem samen-spezifische Promotoren verwendet werden, die im Embryo und/oder im Endosperm aktiv sind. Samen-spezifische Promotoren können prinzipiell sowohl aus dikotyledonen als auch aus monokotyledonen Pflanzen isoliert werden. Im Folgenden sind bevorzugte Promotoren aufgeführt: USP (= unknown seed protein) und Vicilin (Vicia faba) (Bäumlein et al. (1991) Mol. Gen Genet. 225(3): 459-467), Napin (Raps) (US 5,608,152), Conlinin (Lein) (WO 02/102970), Acyl-Carrier Protein (Raps) (US 5,315,001 und WO 92/18634), Oleosin (Arabidopsis thaliana) (WO 98/45461 und WO 93/20216), Phaseolin (Phaseolus vulgaris) (US 5,504,200), Bce4 (WO 91/13980), Leguminosen B4 (LegB4-Promotor) (Bäumlein et al. (1992) Plant J. 2(2): 233-239), Lpt2 und 1pt1(Gerste) (WO 95/15389 und WO95/23230), Samen-spezifische Promotoren aus Reis, Mais und Weizen (WO 99/16890), Amy32b, Amy 6-6 und Aleurain (US 5,677,474), Bce4 (Raps) (US 5,530,149), Glycinin (Soja) (EP 571 741), Phosphoenol-Pyruvatcarboxylase (Soja) (JP 06/62870), ADR12-2 (Soja) (WO 98/08962), Isocitratlyase (Raps) (US 5,689,040) oder α-Amylase (Gerste) (EP 781 849).

In einer besonders bevorzugten Ausführungsform der vorliegenden Erfindung werden die verwendeten Nukleinsäuresequenzen, insbesondere die für eine Δ-5 Elongase kodierende Nukleinsäuresequenz, welche gegenüber der Nukleinsäuresequenz in dem Organismus, aus dem die Sequenz stammt, dadurch verändert ist, dass sie an die Kodonverwendung in einer oder mehreren Pflanzenarten angepasst ist, bevorzugt die in SEQ ID NO: 64 beschriebene Nukleinsäuresequenz im generativen Gewebe, insbesondere im Samen exprimiert. Die spezifische Expression im Samen erfolgt vorteilhaft unter Verwendung eines der oben erwähnten samenspezifischen Promotoren, insbesondere unter Verwendung des Napin Promotors. In dieser besonders bevorzugten Ausführungsform beträgt der Gehalt an den hergestellten LCPUFAs, insbesondere an den C22 Fettsäuren im Samenöl mindestens 5 Gew.-%, vorteilhaft mindestens 6, 7, 8, 9 oder 10 Gen.-%, bevorzugt von mindestens 11, 12, 13, 14 oder 15 Gew.-%, besonders bevorzugt von mindestens 16, 17, 18, 19, oder 20 Gew.-%, ganz besonders bevorzugt von mindestens 25, 30, 35 oder 40 Gew.% des Samenölgehalt. In einer weiteren besonders bevorzugten Ausführungsform mit der in SEQ ID NO: 63 beschriebenen Nukleinsäuresequenz beträgt der Gehalt an C22-Fettsäuren im Samenöl mindestens 8 Gew.-% des Samenölgehalts.

In einer weiteren besonders bevorzugten Ausführungsform der vorliegenden Erfindung werden die verwendeten Nukleinsäuresequenzen, insbesondere die für eine Δ-5 Elongase kodierende Nukleinsäuresequenz, welche gegenüber der Nukleinsäuresequenz in dem Organismus, aus dem die Sequenz stammt, dadurch verändert ist, dass sie an die Kodonverwendung in einer oder mehreren Pflanzenarten angepasst ist, bevorzugt die in SEQ ID NO: 64 beschriebene Nukleinsäuresequenz im generativen Gewebe, insbesondere im Samen exprimiert. Die spezifische Expression im Samen erfolgt vorteilhaft unter Verwendung eines der oben erwähnten samenspezifischen Promotoren, insbesondere unter Verwendung des Napin-Promotors. In dieser besonders bevorzugten Ausführungsform beträgt der Gehalt an Docosahexaensäure im Samenöl mindestens 1 Gew.-%, bevorzugt mindestens 1,1, 1,2, 1,3, 1,4 oder 1,5 Gew.-%, besonders bevorzugt mindestens 1,6, 1,7, 1,8 oder 1,9 Gew.-%, insbesondere mindestens 2, 2,1, 2,2, 2,3, 2,4, 2,5, 2,6, 2,7, 2,8 oder 2,9 Gew.-%, weiterhin bevorzugt mindestens 3, 3,5 oder 4 Gew.-% des Samenölgehalts. In einer weiteren besonders bevorzugten Ausführungsform mit der in SEQ ID NO: 63 beschriebenen Nukleinsäuresequenz beträgt der Gehalt an Docosahexaensäure im Samenöl mindestens 1,9 Gew.-% des Samenölgehalts. Es ist dem Fachmann dabei bekannt, dass zur Herstellung von Docosahexaensäure zusätzlich eine oder mehrere Nukleinsäuresequenzen, die für ein Polypeptid mit der Aktivität einer Δ4-Desaturase-Aktivität kodiert, benötigt werden. Vorteilhaft wird eine Nukleinsäuresequenz, die für ein Polypeptid mit der Aktivität einer Δ4-Desaturase-Aktivität kodiert, ausgewählt aus der Gruppe bestehend aus Nukleinsäuresequenzen mit der in SEQ ID No. 77, 79,81,83,85,87,89,91 oder 93, bevorzugt mit der in der SEQ ID No. 77 dargestellten Sequenz.

In einer weiteren besonders bevorzugten Ausführungsform der vorliegenden Erfindung werden die verwendeten Nukleinsäuresequenzen, insbesondere die für eine Δ-5 Elongase kodierende Nukleinsäuresequenz, welche gegenüber der Nukleinsäuresequenz in dem Organismus, aus dem die Sequenz stammt, dadurch verändert ist, dass sie an die Kodonverwendung in einer oder mehreren Pflanzenarten angepasst ist, bevorzugt die in SEQ ID NO: 64 beschriebene Nukleinsäuresequenz im generativen Gewebe, insbesondere im Samen exprimiert. Die spezifische Expression im Samen erfolgt vorteilhaft unter Verwendung eines der oben erwähnten samenspezifischen Promotoren, insbesondere unter Verwendung des NapinPromotors. In dieser besonders bevorzugten Ausführungsform beträgt der Gehalt an Docosahexaensäure im Samenöl mindestens 1 Gew.-%, bevorzugt mindestens 1,1, 1,2, 1,3, 1,4 oder 1,5 Gew.-%, besonders bevorzugt mindestens 1,6, 1,7, 1,8 oder 1,9 Gew.-%, insbesondere mindestens 2, 2,1, 2,2, 2,5, 2,6, 2,7, 2,8 oder 2,9 Gew.-%, weiterhin bevorzugt mindestens 3, 3,5, oder 4 Gew.-% des Samenölgehalts. Dabei beträgt der Gehalt an den hergestellten LCPUFAs, insbesondere an den C22 Fettsäuren im Samenöl mindestens 5 Gew.-%, vorteilhaft mindestens 6, 7, 8, 9 oder 10 Gew.-%, bevorzugt von mindestens 11, 12, 13, 14 oder 15 Gew.-%, besonders bevorzugt von mindestens 16, 17, 18, 19, oder 20 Gew.-%, ganz besonders bevorzugt von mindestens 25, 30, 35 oder 40 Gew.-% des Samenölgehalt. In einer weiteren besonders bevorzugten Ausführungsform mit der in SEQ ID NO: 63 beschriebenen Nukleinsäuresequenz beträgt der Gehalt an Docosahexaensäure im Samenöl mindestens 1,9 Gew.-% des Samenölgehalts, wobei der Gehalt an C22-Fettsäuren im Samenöl mindestens 8 Gew.-% des Samenölgehalts beträgt.

Die Pflanzengenexpression lässt sich auch über einen chemisch induzierbaren Promotor erleichtern (siehe eine Übersicht in Gatz (1997) Annu. Rev. Plant Physiol. Plant Mol. Biol. 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, wie beispielsweise der pathogeninduzierte PRP1-Gen-Promotor (Ward et al. (1993) Plant. Mol. Biol. 22: 361-366), der hitzeinduzierbare hsp80-Promötor aus Tomate (US 5,187,267), der kälteinduzierbare Alpha-Amylase-Promotor aus Kartoffel (WO 96/12814) oder der durch Wunden induzierbare pinII-Promotor (EP-A-0 375 091).

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Es versteht sich, dass die erfindungsgemäß hergestellten mehrfach ungesättigten Fettsäuren nicht nur in intakten transgenen Pflanzen produziert werden können, sondern auch in pflanzlichen Zellkulturen oder in Kalluskulturen.

Die im Verfahren hergestellten mehrfach ungesättigten Fettsäuren sind vorteilhaft in Phospholipiden und/oder Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Phospholipide wie Phosphatidylglycerol, Phosphatidylcholin, Phosphatidylethanolamin und/oder Phosphatidylserin und/oder Triacylglyceriden, Monoacylglyceriden und/oder Diacylglyceriden vorliegen. Vorteilhaft liegen die im Verfahren hergestellten LCPUFAS EPA, DPA und DHA im Phosphatidylcholin und/oder Phosphatidylethanolamin und/oder in den Triacylglyceriden vor. Die Triacylglyceride können außerdem noch weitere Fettsäuren enthalten wie kurzkettige Fettsäuren mit 4 bis 6 C-Atomen, mittelkettige Fettsäuren mit 8 bis 12 C-Atomen oder langkettige Fettsäuren mit 14 bis 24 C-Atomen. Bevorzugt enthalten sie langkettige Fettsäuren, besonders bevorzugt C₂₀- oder C₂₂-Fettsäuren.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch aus verschiedenen Glyceriden verstanden. Vorteilhaft handelt es sich bei dem Glycerid um ein Triglycerid. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Unter Phospholipiden im Sinne der Erfindung sind zu verstehen Phosphatidylcholin, Phosphatidylethanolamin, Phosphatidylserin, Phosphatidylglycerin und/oder Phosphatidylinositol.

Die Fettsäureester mit mehrfach ungesättigten C₁₈-, C₂₀- und/oder C₂₂-Fettsäuremolekülen können aus den Nutzpflanzen, die für die Herstellung der Fettsäureester verwendet wurden, in Form eines Öls oder Lipids beispielsweise in Form von Verbindungen wie Sphingolipiden, Phosphoglyceriden, Lipiden, Glycolipiden wie Glycosphingolipiden, Phospholipiden wie Phosphatidylethanolamin, Phosphatidylcholin, Phosphatidylserin, Phosphatidylglycerol, Phosphatidylinositol oder Diphosphatidylglycerol, Monoacylglyceriden, Diacylglyceriden, Triacylglyceriden oder sonstigen Fettsäureestern wie den Acetyl-CoenzymA-Estern, die die mehrfach ungesättigten Fettsäuren mit mindestens zwei, drei oder vier, bevorzugt vier, fünf oder sechs Doppelbindungen enthalten, isoliert werden, vorteilhaft werden sie in der Form ihrer Diacylglyceride, Triacylglyceride und/oder in Form des Phosphatidylesters isoliert, besonders bevorzugt in der Form der Triacylglyceride, Phosphatidylcholin und/oder Phosphatidylethanolamin. Neben diesen Estern sind die mehrfach ungesättigten Fettsäuren auch als freie Fettsäuren oder gebunden in anderen Verbindungen in den Pflanzen enthalten. In der Regel liegen die verschiedenen vorgenannten Verbindungen (Fettsäureester und freie Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 4 Gew.-%, vorteilhaft von mindestens 5, 6, 7, 8, 9 oder 10 Gew.-%, bevorzugt von mindestens 11, 12, 13, 14 oder 15 Gew.-%, besonders bevorzugt von mindestens 16, 17, 18, 19, oder 20 Gew.-%, ganz besonders bevorzugt von mindestens 25, 30, 35 oder 40 Gew.-% bezogen auf die gesamten Fettsäuren in der transgenen Pflanze hergestellt. Dabei sind die im erfindungsgemäßen Verfahren hergestellten Fettsäuren EPA, DPA und/oder DHA mit einem Gehalt von jeweils mindestens 5 Gew.-%, bevorzugt von jeweils mindestens 6, 7, 8 oder 9 Gew.-%, besonders bevorzugt von jeweils mindestens 10, 11 oder 12 Gew.-%, am meisten bevorzugt von jeweils mindestens 13, 14, 15, 16, 17, 18, 19 oder 20 Gew.-%, bezogen auf die gesamten Fettsäuren in der transgenen Pflanze enthalten.

Vorteilhaft werden die Fettsäuren in gebundener Form hergestellt. Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triacylglyceride bringen. Vorteilhaft sind mindestens 11% der Triacylglyceride doppelt (das heißt an sn1- und sn2- oder sn2- und sn3-Position) substituiert. Auch dreifach substituierte Triacylglyceride sind nachweisbar. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA) oder Eicosapentaensäure (EPA) nicht als absolute Reinprodukte an, es sind immer auch Spuren oder größere Mengen der Vorstufen im Endprodukt enthalten. Sind in der Ausgangspflanze beispielsweise sowohl Linolsäure als auch Linolensäure vorhanden, so liegen die Endprodukte wie ARA oder EPA und/oder DPA und/oder DHA als Mischungen vor. Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweiligen Endprodukts betragen. Vorteilhaft werden in einer transgenen Pflanze als Endprodukte nur ARA oder EPA und/oder DPA und/oder DHA im erfindungsgemäßen Verfahren gebunden oder als freie Säuren hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren, jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureestern bzw. Fettsäuregemischen, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopentendodecansäure), Furan-Fettsäure (9,12-Epoxy-octadeca-9,11-dienonsäure), Vemonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf den Gesamtfettsäuregehalt zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Buttersäure, kein Cholesterin sowie keine Nisinsäure (Tetracosahexaensäure, C23:6^{Δ3,8,12,15,18,21}).

Durch die im erfindungsgemäßen Verfahren verwendeten Nukleinsäuresequenzen kann in den transgenen Pflanzen eine Steigerung der Ausbeute an LCPUFAs von mindestens 50%, vorteilhaft von mindestens 80%, besonders vorteilhaft von mindestens 100%, ganz besonders vorteilhaft von mindestens 150% gegenüber den nicht transgenen Pflanzen erreicht werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus den Pflanzen in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Für das erfindungsgemäße Verfahren sind prinzipiell alle dicotylen oder monokotylen Nutzpflanzen geeignet. Unter Nutzpflanzen sind Pflanzen zu verstehen, die der Nahrungsproduktion für Mensch und Tier, der Produktion von Genussmitteln, Fasern und Pharmazeutika dienen, wie Getreide, z.B. Mais, Reis, Weizen, Gerste, Hirse, Hafer, Roggen, Buchweizen; wie Knollen, z.B. Kartoffel, Maniok, Batate, Yams etc.; wie Zuckerpflanzen, z.B. Zuckerrohr oder Zuckerrübe; wie Hülsenfrüchte, z.B. Bohnen, Erbsen, Saubohne etc.; wie Öl- und Fettfrüchte, z.B. Sojabohne, Raps, Sonnenblume, Färberdiestel, Lein, Camelina etc., um nur einige zu nennen. Vorteilhafte Pflanzen sind ausgewählt aus der Gruppe der Pflanzenfamilien bestehend aus den Familien der Aceraceae, Actinidiaceae, Anacardiaceae, Apiaceae, Arecaceae, Asteraceae, Arecaceae, Betulaceae, Boraginaceae, Brassicaceae, Bromeliaceae, Cannabaceae, Cannaceae, Caprifoliaceae, Chenopodiaceae, Convolvulaceae, Cucurbitaceae, Dioscoreaceae, Elaeagnaceae, Ericaceae, Euphorbiaceae, Fabaceae, Fagaceae, Grossulariaceae, Juglandaceae, Lauraceae, Liliaceae, Linaceae, Malvaceae, Moraceae, Musaceae, Oleaceae, Oxalidaceae, Papaveraceae, Poaceae, Polygonaceae, Punicaceae, Rosaceae, Rubiaceae, Rutaceae, Scrophulariaceae, Solanaceae, Sterculiaceae und Valerianaceae.

Beispielhaft seien die folgenden Pflanzen genannt: Anacardiaceae wie die Gattungen Pistacia, Mangifera, Anacardium z.B. die Gattung und Arten Pistacia vera (Pistazie), Mangifer indica (Mango) oder Anacardium occidentale (Cashew), Asteraceae wie die Gattungen Calendula, Carthamus, Centaurea, Cichorium, Cynara, Helianthus, Lactuca, Locusta, Tagetes, Valeriana z.B. die Gattung und Arten Calendula officinalis (Garten-Ringelblume), Carthamus tinctorius (Färberdistel, safflower), Centaurea cyanus (Kornblume), Cichorium intybus (Wegwarte), Cynara scolymus (Artischocke), Helianthus annus (Sonnenblume), Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta (Salat), Tagetes lucida, Tagetes erecta oder Tagetes tenuifolia (Studentenblume), Apiaceae wie die Gattung Daucus z.B. die Gattung und Art Daucus carota (Karotte), Betulaceae wie die Gattung Corylus z.B. die Gattungen und Arten Corylus avellana oder Corylus colurna (Haselnuss), Boraginaceae wie die Gattung Borago z.B. die Gattung und Art Borago officinalis (Borretsch), Brassicaceae wie die Gattungen Brassica, Camelina, Melanosinapis, Sinapis, Arabadopsis z.B. die Gattungen und Arten Brassica napus, Brassica rapa ssp. (Raps), Sinapis arvensis Brassica juncea, Brassica juncea var. juncea, Brassica juncea var. crispifolia, Brassica juncea var. foliosa, Brassica nigra, Brassica sinapioides, Camelina sativa, Melanosinapis communis (Senf), Brassica oleracea (Futterrübe) oder Arabidopsis thaliana, Bromeliaceae wie die Gattungen Anana, Bromelia (Ananas) z.B. die Gattungen und Arten Anana comosus, Ananas ananas oder Bromelia comosa (Ananas), Caricaceae wie die Gattung Carica wie die Gattung und Art Carica papaya (Papaya), Cannabaceae wie die Gattung Cannabis wie die Gattung und Art Cannabis sative (Hanf), Convolvulaceae wie die Gattungen Ipomoea, Convolvulus z.B. die Gattungen und Arten Ipomoea batatus, Ipomoea pandurata, Convolvulus batatas, Convolvulus tiliaceus, Ipomoea fastigiata, Ipomoea tiliacea, Ipomoea triloba oder Convolvulus panduratus (Süßkartoffel, Batate), Chenopodiaceae wie die Gattung Beta wie die Gattungen und Arten Beta vulgaris, Beta vulgaris var. altissima, Beta vulgaris var. vulgaris, Beta maritima, Beta vulgaris var. perennis, Beta vulgaris var. conditiva oder Beta vulgaris var. esculenta (Zuckerrübe), Cucurbitaceae wie die Gattung Cucubita z.B. die Gattungen und Arten Cucurbita maxima, Cucurbita mixta, Cucurbita pepo oder Cucurbita moschata (Kürbis), Elaeagnaceae wie die Gattung Elaeagnus z.B. die Gattung und Art Olea europaea (Olive), Ericaceae wie die Gattung Kalmia z.B. die Gattungen und Arten Kalmia latifolia, Kalmia angustifolia, Kalmia microphylla, Kalmia polifolia, Kalmia occidentalis, Cistus chamaerhodendros oder Kalmia lucida (Berglorbeer), Euphorbiaceae wie die Gattungen Manihot, Janipha, Jatropha, Ricinus z.B. die Gattungen und Arten Manihot utilissima, Janipha manihot, Jatropha manihot., Manihot aipil, Manihot dulcis, Manihot manihot, Manihot melanobasis, Manihot esculenta (Manihot) oder Ricinus communis (Rizinus), Fabaceae wie die Gattungen Pisum, Albizia, Cathormion, Feuillea, Inga, Pithecolobium, Acacia, Mimosa, Medicajo, Glycine, Dolichos, Phaseolus, Soja z.B. die Gattungen und Arten Pisum sativum, Pisum arvense, Pisum humile (Erbse), Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa (Seidenbaum), Medicago sativa, Medicago falcata, Medicago varia (Alfalfa) Glycine max Dolichos soja, Glycine gracilis, Glycine hispida, Phaseolus max, Soja hispida oder Soja max (Sojabohne), Geraniaceae wie die Gattungen Pelargonium, Cocos, Oleum z.B. die Gattungen und Arten Cocos nucifera, Pelargonium grossularioides oder Oleum cocois (Kokusnuss), Gramineae wie die Gattung Saccharum z.B. die Gattung und Art Saccharum officinarum, Juglandaceae wie die Gattungen Juglans, Wallia z.B. die Gattungen und Arten Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra oder Wallia nigra (Walnuss), Lauraceae wie die Gattungen Persea, Laurus z.B. die Gattungen und Arten Laurus nobilis (Lorbeer), Persea americana, Persea gratissima oder Persea persea (Avocado), Leguminosae wie die Gattung Arachis z.B. die Gattung und Art Arachis hypogaea (Erdnuss), Linaceae wie die Gattungen Linum, Adenolinum z.B. die Gattungen und Arten Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense oder Linum trigynum (Lein), Lythrarieae wie die Gattung Punica z.B. die Gattung und Art Punica granatum (Granatapfel), Malvaceae wie die Gattung Gossypium z.B. die Gattungen und Arten Gossypium hirsutum, Gossypium arboreum, Gossypium barbadense, Gossypium herbaceum oder Gossypium thurberi (Baumwolle), Musaceae wie die Gattung Musa z.B. die Gattungen und Arten Musa nana, Musa acuminata, Musa paradisiaca, Musa spp. (Banane), Onagraceae wie die Gattungen Camissonia, Oenothera z.B. die Gattungen und Arten Oenothera biennis oder Camissonia brevipes (Nachtkerze), Palmae wie die Gattung Elaeis z.B., die Gattung und Art Elaeis guineensis (Ölpalme), Papaveraceae wie die Gattung Papaver z.B. die Gattungen und Arten Papaver orientale, Papaver rhoeas, Papaver dubium (Mohn), Pedaliaceae wie die Gattung Sesamum z.B. die Gattung und Art Sesamum indicum (Sesam), Piperaceae wie die Gattungen Piper, Artanthe, Peperomia, Steffensia z.B. die Gattungen und Arten Piper aduncum, Piper ämalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata (Cayennepfeffer), Poaceae wie die Gattungen Hordeum, Secale, Avena, Sorghum, Andropogon, Holcus, Panicum, Oryza, Zea (Mais), Triticum, z.B. die Gattungen und Arten Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum (Gerste), Secale cereale (Roggen), Avena sativa, Avena fatua, Avena byzantina, Avena fatua var. sativa, Avena hybrida (Hafer), Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cemuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum (Hirse), Oryza sativa, Oryza latifolia (Reis), Zea mays (Mais) Triticum aestivum, Triticum durum, Triticum turgidum, Triticum hybernum, Triticum macha, Triticum sativum oder Triticum vulgare (Weizen), Porphyridiaceae wie die Gattungen Chroothece, Flintiella, Petrovanella, Porphyridium, Rhodella, Rhodosorus, Vanhoeffenia z.B. die Gattung und Art Porphyridium cruentum, Proteaceae wie die Gattung Macadamia z.B. die Gattung und Art Macadamia intergrifolia (Macadamia), Rubiaceae wie die Gattung Coffea z.B. die Gattungen und Arten Cofea spp., Coffea arabica, Coffea canephora oder Coffea liberica (Kaffee), Scrophulariaceae wie die Gattung Verbascum z.B. die Gattungen und Arten Verbascum blattaria, Verbascum chaixii, Verbascum densiflorum, Verbascum lagurus, Verbascum longifolium, Verbascum lychnitis, Verbascum nigrum, Verbascum olympicuni, Verbascum phlomoides, Verbascum phoenicum, Verbascum pulverulentum oder Verbascum thapsus (Königskerze), Solanaceae wie die Gattungen Capsicum, Nicotiana, Solanum, Lycopersicon z.B. die Gattungen und Arten Capsicum annuum, Capsicum annuum var. glabriusculum, Capsicum frutescens (Pfeffer), Capsicum annuum (Paprika), Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris (Tabak), Solanum tuberosum (Kartoffel), Solanum melongena (Aubergine) Lycopersicon esculentum, Lycopersicon lycopersicum., Lycopersicon pyriforme, Solanum integrifolium oder Solanum lycopersicum (Tomate), Sterculiaceae wie die Gattung Theobroma z.B. die Gattung und Art Theobroma cacao (Kakao) oder Theaceae wie die Gattung Camellia z.B. die Gattung und Art Camellia sinensis (Tee).

In einer vorteilhaften Ausführungsform des Verfahren werden als Nutzpflanzen Ölfruchtpflanzen verwendet, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Puffer, Tagetes, Solanaceen-Pflanzen wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Vorteilhafte erfindungsgemäße Pflanzen sind Ölfruchtpflanzen, wie Erdnuss, Raps, Canola, Sonnenblume, Saflor , Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind Pflanzen, die reich an C 18:2- und/oder C18:3-Fettsäuren sind, wie Sonnenblume, Färberdistel, Tabak, Königskerze, Sesam, Baumwolle, Kürbis, Mohn, Nachtkerze, Walnuss, Lein, Hanf oder Distel. Ganz besonders bevorzugt sind Pflanzen wie Färberdistel, Sonnenblume, Mohn, Nachtkerze, Walnuss, Lein oder Hanf.

Es ist auch vorteilhaft, die erfindungsgemäßen Nukleinsäuresequenzen in den Blättern von Futter- oder Nahrungspflanzen zu exprimieren und dadurch den Gehalt der Blätter an Eicosapentaensäure, Docosapentaerisäure und/oder Docosahexaensäure zu steigern. Bevorzugte Futterpflanzen sind z.B. Kleearten wie Rotklee (Trifolium pratense), Weißklee (Trifolium repens), Bastardklee (Trifolium hybridum), Esparsette (Onobrychis viciifolia), Alexandrinerklee (Trifolium alexandrinum) und Perserklee (Trifolium resupinatum). Bevorzugte Nahrungspflanzen sind etwa Salatarten wie Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis und Valeriana locusta.

Durch die enzymatische Aktivität der im erfindungsgemäßen Verfahren verwendeten Nukleinsäuresequenzen, die für Polypeptide mit Δ-6-Elongase-, Δ-6-Desaturase-, Δ-5-Desaturase- und/oder Δ-5-Elongase-Aktivität kodieren, vorteilhaft in Kombination mit Nukleinsäuresequenzen, die für Polypeptide mit ω-3-Desaturase- und/oder Δ-4-Desaturase-Aktivität kodieren, sowie weiteren Nukleinsäuresequenzen, die für Polypeptide des Fettsäure- oder Lipidstoffwechsels wie weiteren Polypeptiden mit Δ-5-, Δ-6-, Δ-8-, Δ-12-Desaturase- oder Δ-5-, Δ-6-oder Δ-9-Elongaseaktivität kodieren, können unterschiedlichste mehrfach ungesättigte Fettsäuren im erfindungsgemäßen Verfahren hergestellt werden. Je nach Auswahl der für das erfindungsgemäße Verfahren verwendeten Nutzpflanzen lassen sich Mischungen der verschiedenen mehrfach ungesättigten Fettsäuren oder einzelne mehrfach ungesättigte Fettsäuren wie EPA, DPA oder DHA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder Fettsäuren, die sich von C 18:3-Fettsäuren ableiten, wie EPA, DPA oder DHA. Liegt in der für das Verfahren verwendeten Pflanze als ungesättigte Fettsäure nur Linolsäure (= LA, C18:2^{Δ9,12}) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:3^{Δ9,12,15})vorhanden, beispielsweise wie in Lein, so können als Produkte des Verfahrens nur SDA, ETA, EPA, DPA und/oder DHA entstehen, die wie oben beschrieben als freie Fettsäuren oder gebunden vorliegen können. Durch Modifikation der Aktivität der im Verfahren verwendeten und an der Synthese beteiligten Enzyme Δ-6-Elongasen, Δ-6-Desaturase, Δ-5-Desaturase und/oder Δ-6-Elongase vorteilhaft in Kombination mit weiteren Genen des Lipid- oder Fettsäurestoffwechsels lassen sich gezielt in den Pflanzen nur einzelne Produkte herstellen. Vorteilhaft werden nur EPA, DPA oder DHA oder deren Mischungen synthetisiert. Da die Fettsäuren in Biosyntheseketten synthetisiert werden, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf die Endprodukte EPA, DPA oder DHA oder deren Mischungen.

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft, die Menge an Ausgangsprodukt für die Fettsäuresynthese zu steigern. Dies kann beispielsweise durch das Einbringen einer Nukleinsäure, die für ein Polypeptid mit Δ-12-Desaturase kodiert, in den Organismus erreicht werden. Dies ist besonders vorteilhaft in Nutzpflanzen, wie Öl-produzierenden Pflanzen wie Pflanzen der Familie der Brassicaceae wie der Gattung Brassica z.B. Raps; der Familie der Elaeagnaceae wie die Gattung Elaeagnus z.B. die Gattung und Art Olea europaea oder der Familie Fabaceae wie der Gattung Glycine z.B. die Gattung und Art Glycine max, die einen hohen Ölsäuregehalt aufweisen. Da diese Organismen nur einen geringen Gehalt an Linolsäure aufweisen (Mikoklajczak et al. (1961) Journal of the American Oil Chemical Society 38: 678 - 681) ist die Verwendung der genannten Δ-12-Desaturasen zur Herstellung des Ausgangsprodukts Linolsäure aus Ölsäure vorteilhaft. Daneben können die Ausgangsfettsäuren auch von außen zugefüttert werden, was aber aus Kostengründen weniger bevorzugt ist.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden, während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einer Pflanze, wie einer Nutzpflanze wie einer Ölfruchtpflanze, beispielsweise Raps, Canola, Lein, Hanf, Soja, Sonnenblumen, Borretsch. Sie sind deshalb vorteilhaft im erfindungsgemäßen Verfahren verwendbar.

Im erfindungsgemäßen Verfahren verwendete Nukleinsäuren stammen vorteilhaft aus Pflanzen wie Algen, beispielsweise Algen der Familie der Prasinophyceae wie aus den Gattungen Heteromastix, Mammella, Mantoniella, Micromonas, Nephroselmis, Ostreococcus, Prasinocladus, Prasinococcus, Pseudoscourfielda, Pycnococcus, Pyramimonas, Scherffelia oder Tetraselmis wie den Gattungen und Arten Heteromastix longifillis, Mamiella gilva, Mantoniella squamata, Micromonas pusilla, Nephroselmis olivacea, Nephroselmis pyriformis, Nephroselmis rotunda, Ostreococcus tauri, Ostreococcus sp. Prasinocladus ascus, Prasinocladus lubricus, Pycnococcus provasolii, Pyramimonas amylifera, Pyramimonas disomata, Pyramimonas obovata, Pyramimonas orientalis, Pyramimonas parkeae, Pyramimonas spinifera, Pyramimonas sp., Tetraselmis apiculata, Tetraselmis carteriaformis, Tetraselmis chui, Tetraselmis convolutae, Tetraselmis desikacharyi, Tetraselmis gracilis, Tetraselmis hazeni, Tetraselmis impellucida, Tetraselmis inconspicua, Tetraselmis levis, Tetraselmis maculata, Tetraselmis marina, Tetraselmis striata, Tetraselmis subcordiformis, Tetraselmis suecica, Tetraselmis tetrabrachia, Tetraselmis tetrathele, Tetraselmis verrucosa, Tetraselmis verrucosa fo. rubens oder Tetraselmis sp. oder aus Algen der Familie Euglenaceae wie aus den Gattungen Ascoglena, Astasia, Colacium, Cyclidiopsis, Euglena, Euglenopsis, Hyalophacus, Khawkinea, Lepocinclis, Phacus, Strombomonas oder Trachelomonas wie die Gattungen und Art Euglena acus, Euglena geniculata, Euglena gracilis, Euglena mixocylindracea, Euglena rostrifera, Euglena viridis, Colacium stentorium, Trachelomonas cylindrica oder Trachelomonas volvocina.

Weitere vorteilhafte Pflanzen sind Algen wie Isochrysis oder Crypthecodinium, Algen/Diatomeen wie Thalassiosira oder Phaeodactylum, Moose wie Physcomitrella oder Ceratodon oder höheren Pflanzen wie den Primulaceae wie Aleuritia, Calendula stellata, Osteospermum spinescens oder Osteospermum hyoseroides, Mikroorganismen wie Pilzen wie Aspergillus, Thraustochytrium, Phytophthora, Entomophthora, Mucor oder Mortierella, Bakterien wie Shewanella, Hefen oder Tiere wie Nematoden wie Caenorhabditis, Insekten, Frösche, Seegurken oder Fische. Vorteilhaft stammen die erfindungsgemäßen isolierten Nukleinsäuresequenzen aus einem Tier aus der Ordnung der Vertebraten. Bevorzugt stammen die Nukleinsäuresequenzen aus der Klasse der Vertebrata; Euteleostomi, Actinopterygii; Neopterygii; Teleostei; Euteleostei, Protacanthopterygii, Salmoniformes; Salmonidae bzw. Oncorhynchus oder Vertebrata, Amphibia, Anura, Pipidae, Xenopus oder Evertebrata wie Protochordata, Tunicata; Holothuroidea, Cionidae wie Amaroucium constellatum, Botryllus schlosseri, Ciona intestinalis, Molgula citrina, Molgula manhattensis, Perophora viridis oder Styela partita. Besonders vorteilhaft stammen die Nukleinsäuren aus Pilzen, Tieren oder aus Pflanzen wie Algen oder Moosen, bevorzugt aus der Ordnung der Salmoniformes wie der Familie der Salmonidae wie der Gattung Salmo beispielsweise aus den Gattungen und Arten Oncorhynchus mykiss, Trutta trutta oder Salmo trutta fario, aus Algen wie den Gattungen Mantoniella oder Ostreococcus oder aus den Diatomeen wie den Gattungen Thalassiosira oder Phaeodactylum oder aus Algen wie Crypthecodinium.

Bei einer bevorzugten Ausführungsform umfasst das Verfahren ferner den Schritt des Gewinnens einer Zelle oder einer ganzen Pflanze, die die im Verfahren verwendeten Nukleinsäuresequenzen, die für eine Δ-6-Desaturase, Δ-6-Elongase, Δ-5-Desaturase und/oder Δ-5-Elongase sowie ggf. Nukleinsäuresequenzen, die für eine ω-3-Desaturase und/oder eine Δ-4-Desaturase kodieren, enthält, wobei die Zelle und/oder die Nutzpflanze noch weitere Nukleinsäuresequenzen des Lipid- oder Fettsäurestoffwechsels enthalten kann. Die im Verfahren bevorzugt verwendeten Nukleinsäuresequenzen werden zur Expression vorteilhaft in mindestens ein Genkonstrukt und/oder einen Vektor wie nachfolgend beschrieben, allein oder in Kombination mit weiteren Nukleinsäuresequenzen, die für Proteine des Fettsäure- oder Lipidstoffwechsels kodieren, eingebaut und schließlich in die Zelle oder Pflanze transformiert. Bei einer weiteren bevorzugten Ausführungsform umfasst dieses Verfahren ferner den Schritt des Gewinnens der Öle, Lipide oder freien Fettsäuren aus den Nutzpflanzen. Die so hergestellte Zelle oder die so hergestellte Nutzpflanze ist vorteilhaft eine Zelle einer Öl-produzierenden Pflanze, Gemüse-, Salat-, oder Zierpflanze oder die Pflanze selbst wie oben ausgeführt.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

"Transgen" bzw. "rekombinant" im Sinne der Erfindung bedeutet bezüglich zum Beispiel einer Nukleinsäuresequenz, einer Expressionskassette (= Genkonstrukt) oder einem Vektor enthaltend die im erfindungsgemäßen Verfahren verwendeten Nukleinsäuresequenzen oder einer mit den im erfindungsgemäßen Verfahren verwendeten Nukleinsäuresequenzen, Expressionskassette oder Vektor transformierten Pflanze alle solche durch gentechnische Methoden zustandegekommenen Konstruktionen, in denen sich entweder
a) die Nukleinsäuresequenz, oder
b) eine mit der Nukleinsäuresequenz funktionell verknüpfte genetische Kontrollsequenz, zum Beispiel ein Promotor, öder
c) (a) und (b)
nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der im erfindungsgemäßen Verfahren verwendeten Nukleinsäuresequenz mit der Nukleinsäuresequenz, die für Proteine mit entsprechender Δ-6-Desaturase-, Δ-6-Elongasen-, Δ-5-Desaturase- und Δ-5-Elongase-Aktivität kodiert, vorteilhaft in Kombination mit Nukleinsäuresequenzen, die für Proteine mit ω-3-Desaturase- und/oder Δ-4-Desaturase-Aktivität kodieren - wird zu einer transgenen Expressionskassette, wenn diese durch nichtnatürliche, synthetische ("künstliche") Verfahren wie beispielsweise einer Mutagenisierung geändert wird. Entsprechende Verfahren sind beispielsweise beschrieben in US 5,565,350 oder WO 00/15815.

Unter "transgener Pflanze" im Sinne der Erfindung ist wie vorgenannt zu verstehen, dass die im Verfahren verwendeten Nukleinsäuren nicht an ihrer natürlichen Stelle im Genom der Pflanze sind. Dabei können die Nukleinsäuresequenzen homolog oder heterolog exprimiert werden. Transgen bedeutet aber auch, dass die erfindungsgemäßen Nukleinsäuren an ihrem natürlichen Platz im Genom der Pflanze sind, dass jedoch die Sequenz gegenüber der natürlichen Sequenz verändert wurde und/oder dass die Regulationssequenzen der natürlichen Sequenzen verändert wurden. Bevorzugt ist unter transgen die Expression der im erfindungsgemäßen Verfahren verwendeten Nukleinsäuren an nicht-natürlicher Stelle im Genom zu verstehen, das heißt eine homologe oder bevorzugt heterologe Expression der Nukleinsäuresequenzen liegt vor.

Bevorzugte transgene Organismen sind Nutzpflanzen wie Öl-produzierende Pflanzen, Gemüse-, Salat- oder Zierpflanzen, die vorteilhaft ausgewählt sind aus der Gruppe der Pflanzenfamilien bestehend aus den Familien der Aceraceae, Actinidiaceae, Anacardiaceae, Apiaceae, Arecaceae, Asteraceae, Arecaceae, Betulaceae, Boraginaceae, Brassicaceae, Bromeliaceae, Cannabaceae, Cannaceae, Caprifoliaceae, Chenopodiaceae, Convolvulaceae, Cucurbitaceae, Dioscoreaceae, Elaeagnaceae, Ericaceae, Euphorbiaceae, Fabaceae, Fagaceae, Grossulariaceae, Juglandaceae, Lauraceae, Liliaceae, Linaceae, Malvaceae, Moraceae, Musaceae, Oleaceae, Oxalidaceae, Papaveraceae, Poaceae, Polygonaceae, Punicaceae, Rosaceae, Rubiaceae, Rutaceae, Scrophulariaceae, Solanaceae, Sterculiaceae und Valerianaceae.

Als Wirtspflanzen für die im erfindungsgemäßen Verfahren verwendeten Nukleinsäuren, die Expressionskassette oder den Vektor eignen sich prinzipiell vorteilhaft alle Nutzpflanzen, die in der Lage sind Fettsäuren, speziell ungesättigte Fettsäuren, zu synthetisieren bzw. die für die Expression rekombinanter Gene geeignet sind. Beispielhaft seien an dieser Stelle Pflanzen wie Arabidopsis, Asteraceae wie Calendula oder Nutzpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne genannt. Weitere vorteilhafte Pflanzen sind an anderer Stelle dieser Anmeldung aufgeführt.

Für die Herstellung der transgenen Nutzpflanze werden in der Regel als Zwischenwirte Mikroorganismen verwendet. Derartige nutzbare Zwischenwirtszellen werden in: Goeddel, Gene Expression Technology: Methods in Enzymology 185, Academic Press, San Diego, CA (1990) genannt.

Vorteilhaft verwendbare Expressionsstämme für diesen Zweck sind z.B. solche, die eine geringe Proteaseaktivität aufweisen. Sie werden z.B. in: Gottesman, S., Gene Expression Technology: Methods in Enzymology 185, Academic Press, San Diego, California (1990) 119-128 beschrieben.

Transgene Pflanzen, die die im erfindungsgemäßen Verfahren synthetisierten mehrfach ungesättigten, langekettigen Fettsäuren enthalten, können vorteilhaft direkt vermarktet werden, ohne dass die synthetisierten Öle, Lipide oder Fettsäuren isoliert werden müssen. Diese Form der Vermarktung ist besonders vorteilhaft.

Bei den "Pflanzen" im Sinne der vorliegenden Erfindung handelt es sich um ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze,ableiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe.

Die im erfindungsgemäßen Verfahren hergestellten Verbindungen können aber auch aus den Pflanzen in Form ihrer Öle, Fett, Lipide und/oder freien Fettsäuren isoliert werden. Durch das erfindungsgemäße, Verfahren hergestellte mehrfach ungesättigte Fettsäuren lassen sich durch Ernten der Pflanzen oder Pflanzenzellen entweder aus der Kultur, in der sie wachsen, oder vom Feld gewinnen. Dies kann über Pressen oder Extraktion der Pflanzenteile, bevorzugt der Pflanzensamen, erfolgen. Dabei können die Öle, Fette, Lipide und/oder freien Fettsäuren durch so genanntes Kaltschlagen oder Kaltpressen ohne Zuführung von Wärme durch Pressen gewonnen werden. Damit sich die Pflanzenteile, speziell die Samen, leichter aufschließen lassen, werden sie vorher zerkleinert, gedämpft oder geröstet. Die so vorbehandelten Samen können anschließend gepresst oder mit Lösungsmittel wie warmem Hexan extrahiert werden. Anschließend wird das Lösungsmittel wieder entfernt. Auf diese Weise können mehr als 96 % der im erfindungsgemäßen Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen, werden die Produkte einer Bleichung mit beispielsweise Bleicherde oder Aktivkohle unterzogen. Zum Schluss wird das Produkt noch beispielsweise mit Wasserdampf desodoriert.

Vorzugsweise sind die durch dieses Verfahren produzierten PUFAs bzw. LCPUFAs C₂₀- und/oder C₂₂-Fettsäuremoleküle mit mindestens vier Doppelbindungen im Fettsäuremolekül, vorzugsweise fünf oder sechs Doppelbindungen. Diese C₂₀- und/oder C₂₂-Fettsäuremoleküle lassen sich aus der Pflanze in Form eines Öls, Lipids oder einer freien Fettsäure isolieren. Geeignete transgene Pflanzen sind beispielsweise die vorstehend erwähnten.

Diese erfindungsgemäßen Öle, Lipide oder Fettsäuren enthalten wie oben beschrieben vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Pflanzen.

Als vorteilhafte mehrfach ungesättigte, langkettige Fettsäuren sind in den Fettsäureestern bzw. Fettsäuregemischen wie Phosphatidylfettsäureestern oder Triacylglyceridestern bevorzugt mindestens 10; 11; 12; 13; 14; 15; 16; 17; 18; 19 oder 20 Gew.-% bezogen auf den Gesamtfettsäuregehalt an Eicosapentaensäure und/oder mindestens 1; 2; 3; 4; 5 oder 6 Gew.-% bezogen auf den Gesamtfettsäuregehalt an Docosapentaensäure und/oder mindestens 1; 2; 3; bevorzugt mindestens 4; 5; 6; besonders bevorzugt mindestens 7 oder 8 und am meisten bevorzugt mindestens 9 oder 10 Gew.-% bezogen auf den Gesamtfettsäuregehalt an Docosahexaensäure enthalten.

Weiterhin enthalten die Fettsäureester bzw. Fettsäuregemische, die nach dem erfindungsgemäßen Verfahren hergestellt wurden, Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopentendodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadeeatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfmdungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Buttersäure, kein Cholesterin sowie keine Nisinsäure.

Eine weitere hier beschriebene Ausführungsform ist die Verwendung der Öle, Lipide, der Fettsäuren und/oder der Fettsäurezusammensetzung, die nach dem erfindungsgemäßen Verfahren hergestellt werden, in Futtermitteln, Nahrungsmitteln, Kosmetika oder Pharmazeutika. Die im erfindungsgemäßen Verfahren gewonnenen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese so hergestellten Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte und/oder gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, γ-Linolensäure, Dihomo-γ-linolensäure, Arachidonsäure, α-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, besonders bevorzugt ist ein Anteil von 50 %, am meisten bevorzugt ist ein Anteil von 60 %, 70 %, 80 % oder mehr. Der Anteil an Fettsäure kann nach Überführung der Fettsäuren in die Methylester durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangspflanze der Anteil der verschiedenen Fettsäuren in dem Öl oder Fett schwanken.

Bei den im Verfahren hergestellten mehrfach ungesättigte Fettsäuren mit vorteilhaft mindestens drei, vier; fünf oder sechs; besonders vorteilhaft mit fünf oder sechs Doppelbindungen, handelt es sich wie oben beschrieben vorteilhaft um Fettsäureester beispielsweise um Sphingolipidester, Phosphoglyceridester, Lipidester, Glycolipidester, Phospholipidester, Monoacylglycerinester, Diacylglycerinester, Triacylglycerinester oder sonstige Fettsäureester, bevorzugt handelt es sich um Phospholipidester und/oder Triacylglycerinester.

Aus den so im erfindungsgemäßen Verfahren hergestellten mehrfach ungesättigte Fettsäureestern mit vorteilhaft mindestens drei, vier, fünf oder sechs Doppelbindungen lassen sich die enthaltenen mehrfach ungesättigten Fettsäuren beispielsweise über eine Alkalibehandlung, beispielsweise mit wäßriger KOH oder NaOH, oder durch saure Hydrolyse vorteilhaft in Gegenwart eines Alkohols wie Methanol oder Ethanol oder über eine enzymatische Abspaltung freisetzen und isolieren über beispielsweise Phasentrennung und anschließende Ansäuerung mit z.B. H₂SO₄. Die Freisetzung der Fettsäuren kann auch direkt ohne die vorhergehend beschriebene Aufarbeitung erfolgen.

Als Substrate der im erfindungsgemäßen Verfahren verwendeten Nukleinsäuresequenzen, die für Polypeptide mit Δ-6-Desaturase-, Δ-6-Elongase-, Δ-5-Desaturase- und/oder Δ-5-Elongase-Aktivität sowie ggf. Nukleinsäuresequenzen, die für Polypeptide mit ω-3-Desaturase-und/oder Δ-4-Desaturase-Aktivität kodieren, und/oder den weiteren verwendeten Nukleinsäuren wie den Nukleinsäuresequenzen, die für Polypeptide des Fettsäure- oder Lipidstoffwechsels ausgewählt aus der Gruppe Acyl-CoA-Dehydrogenase(n), Acyl-ACP(= acyl carrier protein)-Desaturase(n), Acyl-ACP-Thioesterase(n), Fettsäure-AcylTransferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) kodieren, eignen sich vorteilhaft C₁₆-, C₁₈- oder C₂₀-Fettsäuren. Bevorzugt werden die im Verfahren als Substrate umgesetzten Fettsäuren in Form ihrer Acyl-CoA-Ester und/oder ihrer Phospholipid-Ester umgesetzt.

Zur Herstellung der erfindungsgemäßen langkettigen PUFAs müssen die gesättigten, einfach ungesättigten C₁₆-Fettsäuren und/oder mehrfach ungesättigten C₁₈-Fettsäuren zunächst je nach Substrat durch die enzymatische Aktivität einer Desaturase und/oder Elongase desaturiert und/oder elongiert oder nur desaturiert und anschließend über eine Elongase um mindestens zwei Kohlenstoffatome verlängert werden. Nach einer Elongationsrunde führt diese Enzymaktivität entweder ausgehend von C₁₆-Fettsäuren zu C₁₈-Fettsäuren oder ausgehend von C₁₈-Fettsäuren zu C₂₀-Fettsäuren, und nach zwei Elongationsrunden ausgehend von C₁₆-Fettsäuren zu C₂₀-Fettsäuren. Die Aktivität der im erfindungsgemäßen Verfahren verwendeten Desaturasen und Elongasen führt vorzugsweise zu C₂₀- und/oder C₂₂-Fettsäuren vorteilhaft mit mindestens zwei oder drei Doppelbindungen im Fettsäuremolekül, vorzugsweise mit vier, fünf oder sechs Doppelbindungen, besonders bevorzugt zu C₂₂-Fettsäuren mit mindestens fünf Doppelbindungen im Fettsäuremolekül. Besonders bevorzugt als Produkte des erfindungsgemäßen Verfahrens sind Eicosapentaensäure, Docosapentaensäure und/oder Docosahexaensäure. Die C₁₈-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann. Vorteilhaft findet die Synthese gemäß des erfinderischen Verfahrens im vegetativen (somatischen) Gewebe statt.

Durch das erfindungsgemäße Verfahren können die hergestellten mehrfach ungesättigten Fettsäuren in den im Verfahren verwendeten Pflanzen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Pflanzen erhöht, vorteilhaft in Form der Phosphatidylester und/oder Triacylester.

Die im erfindungsgemäßen Verfahren verwendeten Sequenzen werden einzeln in Expressionskonstrukte kloniert oder auf einem gemeinsamen rekombinanten Nukleinsäuremolekül bereitgestellt und zum Einbringen und zur Expression in Organismen verwendet. Diese Expressionskonstrukte ermöglichen eine optimale Synthese der im erfindungsgemäßen Verfahren produzierten mehrfach ungesättigten Fettsäuren.

Die im Verfahren verwendeten Nukleinsäuren können nach Einbringung in eine Pflanze oder Pflanzenzelle entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuresequenzen über Multiexpressionskassetten oder Konstrukte zur multiparallelen Expression in die Pflanzen gebracht, d.h. die Nukleinsäuresequenzen liegen in einer gemeinsamen Expressionseinheit vor.

Es kann im Nukleinsäurekonstrukt mehr als eine Nukleinsäuresequenz kodierend für ein Polypeptid mit der enzymatischen Aktivität einer Δ-12-Desaturase, Δ-4-Desaturase, Δ-5-Desaturase, Δ-6-Desaturase-, Δ-5-Elongasen, Δ-6-Elongase und/oder ω-3-Desaturase enthalten sein. Es können auch mehrere Kopien einer Nukleinsäuresequenz kodierend für ein Polypeptid mit der enzymatischen Aktivität einer Δ-12-Desaturase, Δ-4-Desaturase, Δ-5-Desaturase, Δ-6-Desaturase, Δ-5-Elongase, Δ-6-Elongase und/oder ω-3-Desaturase enthalten sein.

Zum Einbringen werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klönierung zur Verfügung. Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakteriumvermittelte Transformation benötigten vir-Gene sowie die T-DNA begrenzende Sequenzen (T-DNA-Border) beinhalten. Vorzugsweise umfassen diese Vektorsysteme auch weitere cisregulatorische Regionen wie Promotoren und Terminatoren und/oder Selektionsmarker, mit denen entsprechend transformierte Organismen identifiziert werden können. Während bei cointegrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E. coli als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pBI101, pBinAR, pGPTV und pCAMBIA. Eine Übersicht über binäre Vektoren und ihre Verwendung gibt Hellens et al. (2000) Trends in Plant Science 5: 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittene und erforderlichenfalls gereinigte Amplifikat mit ähnlich präparierten Vektorfragmenten unter Einsatz von Ligase kloniert. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere Escherichia coli und Agrobacterium tumefaciens, unter selektiven Bedingungen stabil propagieren und ermöglichen so einen Transfer von heterologer DNA in Pflanzen.

Unter der vorteilhaften Verwendung von Klonierungsvektoren können die im erfindungsgemäßen Verfahren verwendeten Nukleinsäuresequenzen und Nukleinsäurekonstrukte in Mikroorganismen und danach in Pflanzen eingebracht werden und damit bei der Pflanzentransformation verwendet werden, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. (1991) 42: 205-225. Die im Verfahren verwendeten Nukleinsäuren, Nukleinsäurekonstrukte und/oder Vektoren lassen sich damit zur gentechnologischen Veränderung eines breiten. Spektrums an Pflanzen verwenden, so dass diese bessere und/oder effizientere Produzenten von LCPUFAs werden.

Durch das Einbringen eines Δ-6-Desaturase-, Δ-6-Elongase-, Δ-5-Desaturase- und Δ-5-Elongase-Genes in eine Pflanze allein oder in Kombination mit anderen Genen kann nicht nur der Biosynthesefluss zum Endprodukt erhöht, sondern auch die entsprechende Triacylglycerin- und/oder Phosphatidylester-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im Folgenden beschrieben, weiter gesteigert wird. Durch Optimierung der Aktivität oder Erhöhung der Anzahl eines oder mehrerer der Δ-6-Desaturase-, Δ-6-Elongase-, Δ-5-Desaturase- und/oder Δ-5-Elongase,-Gene, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die im erfindungsgemäßen Verfahren verwendeten Nukleinsäuremoleküle kodieren für Proteine oder Teile von diesen, wobei die Proteine oder das einzelne Protein oder Teile davon eine Aminosäuresequenz enthält, die ausreichend homolog zu einer Aminosäuresequenz ist, die in den Sequenzen SEQ ID NO. 65, SEQ ID NO. 2, SEQ ID NO. 172 oder SEQ ID NO. 52 und ggf. SEQ ID NO. 194 oder SEQ ID NO. 78 dargestellt ist, so dass die Proteine oder Teile davon noch eine Δ-6-Desaturase-, Δ-6-Elongase-, Δ-5-Desaturase- und/oder Δ-5-Elongase-Aktivität sowie ggf. eine Δ-4-Desaturase- und/oder ω-3-Desaturase-Aktivität aufweisen. Vorzugsweise haben die Proteine oder Teile davon, die von dem Nukleinsäuremolekül/den Nukleinsäuremolekülen kodiert wird/werden, noch seine/ihre wesentliche enzymatische Aktivität und die Fähigkeit, am Stoffwechsel von zum Aufbau von Zellmembranen oder Lipidkörperchen in Organismen vorteilhaft in Pflanzen notwendigen Verbindungen oder am Transport von Molekülen über diese Membranen teilzunehmen. Die von den Nukleinsäuremolekülen kodierten Proteine sind zu mindestens etwa 90 %, 91 %, 92 %, 93 %, 94 %, 95 %, 96 %, 97 %, 98 %, 99 % oder mehr identisch zu den in SEQ ID NO. 65, SEQ ID NO. 2, SEQ ID NO. 172, SEQ ID NO. 52, SEQ ID NO. 194 oder SEQ ID NO. 78 dargestellten Aminosäuresequenzen. Im Sinne der Erfindung ist unter Homologie oder homolog, Identität oder identisch zu verstehen.

Die Homologie wurde über den gesamten Aminosäure- bzw. Nukleinsäuresequenzbereich berechnet. Für den Vergleich verschiedener Sequenzen stehen dem Fachmann eine Reihe von Programmen zur Verfügung, die auf verschiedenen Algorithmen beruhen. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution (1987) 25: 351-360; Higgins et al. (1989) CABIOS 5: 151-153) oder die Programme Gap und BestFit (Needleman and Wunsch (1970) J. Mol. Biol. 48: 443-453 und Smith and Waterman (1981) Adv. Appl. Math. 2: 482-489), die im GCG Software-Packet (Genetics Computer Group, 575 Science Drive, Madison, Wisconsin, USA 53711 (1991)) enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Diese Einstellungen wurden, falls nicht anders angegeben, immer als Standardeinstellungen für Sequenzvergleiche verwendet.

Unter wesentlicher enzymatischer Aktivität der im erfindungsgemäßen Verfahren verwendeten ω-3-Desaturase, Δ-6-Desaturase, Δ-6-Elongase, Δ-5-Elongase, Δ-4-Desaturase und/oder Δ-5-Desaturase ist zu verstehen, dass sie im Vergleich zu den durch die Sequenz mit der SEQ ID NO. 64, SEQ ID NO. 1, SEQ ID NO. 171, SEQ ID NO. 51, SEQ ID NO. 193 oder SEQ ID NO. 77 kodierten Proteinen/Enzymen noch eine enzymatische Aktivität von mindestens 10 %, bevorzugt von mindestens 20 %, besonders bevorzugt von mindestens 30 % und am meisten bevorzugt von mindestens 40, 50 oder 60 % aufweisen und damit am Stoffwechsel von zum Aufbau von Fettsäuren, vorteilhaft Fettsäureestern wie Phosphatidylestem und/oder Triacylglyceridestern, in einer Pflanze oder Pflanzenzelle notwendigen Verbindungen oder am Transport von Molekülen über Membranen teilnehmen können.

Vorteilhaft im Verfahren verwendbare Nukleinsäuren stammen aus Bakterien, Pilzen, Diatomeen, Tieren wie Caenorhabditis oder Oncorhynchus oder Pflanzen wie Algen oder Moosen wie den Gattungen Shewanella, Physcomitrella, Thraustochytrium, Fusarium, Phytophthora, Ceratodon, Pytium irregulare, Mantoniella, Ostreococcus, Isochrysis, Aleurita, Muscarioides, Mortierella, Borago, Phaeodactylum, Crypthecodinium, speziell aus den Gattungen und Arten Pytium irregulare, Oncorhynchus mykiss, Xenopus laevis, Ciona intestinalis, Thalassiosira pseudonona, Mantoniella squamata, Ostreococcus sp., Ostreococcus tauri, Euglena gracilis, Physcomitrella patens, Phytophtora infestans, Fusarium graminaeum, Cryptocodinium cohnii, Ceratodon purpureus, Isochrysis galbana, Aleurita farinosa, Thraustochytrium sp., Muscarioides viallii, Mortierella alpina, Borago officinalis, Phaeodactylum tricomutum, Caenorhabditis elegans oder besonders vorteilhaft aus Pytium irregulare, Thraustochytrium sp. und/oder Ostreococcus tauri.
Im erfindungsgemäßen Verfahren können zusätzlich Nukleotidsequenzen verwendet werden, die für eine Δ-12-Desaturase, Δ-9-Elongasen oder Δ-8-Desaturase kodieren. Die im Verfahren verwendeten Nukleinsäuresequenzen werden vorteilhaft in einer Expressionskassette, die die Expression der Nukleinsäuren in Pflanzen ermöglicht, eingebracht.

Die Nukleinsäuresequenzen, die für die Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase kodieren, werden mit einem oder mehreren Regulationssignalen zur Erhöhung der Genexpression funktionell verknüpft. Diese regulatorischen Sequenzen sollen die gezielte Expression der Gene ermöglichen. Dies kann beispielsweise je nach Pflanze bedeuten, dass das Gen erst nach Induktion exprimiert und/oder überexprimiert wird, oder dass es sofort exprimiert und/oder überexprimiert wird. Vorteilhaft werden Sequenzen für die Expression verwendet, die eine konstitutive Expression ermöglichen, wie der CaMV35S-, CaMV36S-, CaMV35Smas-, nos-, mas-, ubi-, stpt-, lea- oder Super-Promotor. Bevorzugt erfolgt die Expression im vegetativen Gewebe wie oben beschrieben. In einer anderen bevorzugten Ausführungsform erfolgt die Expression im Samen.

Beispielsweise handelt es sich bei diesen regulatorischen Sequenzen um Sequenzen, an die Induktoren oder Repressoren binden und so die Expression der Nukleinsäure regulieren. Zusätzlich zu den Regulationssequenzen, die in ihrem natürlichen Locus nicht mit den Nukleinsäuresequenzen verknüpft sind, oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht ist. Das Genkonstrukt kann außerdem vorteilhafterweise auch eine oder mehrere sogenannte "Enhancer-Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Vorteilhafte Terminatoren sind beispielweise virale Terminatoren wie der 35S-Terminator oder andere. Die im erfindungsgemäßen Verfahren verwendeten Nukleinsäuresequenzen können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können gleichzeitig oder nacheinander in die Pflanze eingebracht werden und zusammen im Wirtsorganismus exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene in die Pflanze, wenn die zu exprimierenden Gene zusammen in einem Genkonstrukt vorliegen. Es ist aber auch möglich, jeweils ein Genkonstrukt enthaltend eine Nukleinsäüresequenz in eine Pflanze einzuführen und die so erhaltenen Pflanzen miteinander zu verkreuzen, um Nachkommen zu erhalten, die alle Genkonstrukte gemeinsam enthalten.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Um eine stabile Integration der Biosynthesegene in die transgene Pflanze über mehrere Generation sicherzustellen, sollte jede der im Verfahren verwendeten Nukleinsäuren, die für die Δ-6-Desaturase, Δ-6-Elongase, Δ-5-Desaturase oder Δ-5-Elongase und ggf. für die ω-3-Desaturase oder Δ-4-Desaturase kodieren, unter der Kontrolle eines eigenen Promotors exprimiert werden. Dieser kann für jede der Sequenzen gleich oder unterschiedlich sein. Die Expressionskassette ist dabei vorteilhaft so aufgebaut, dass einem Promotor eine geeignete Schnittstelle zur Insertion der zu exprimierenden Nukleinsäure folgt, die vorteilhaft in einem Polylinker liegt. Hinter dem Polylinker kann ggf. ein Terminator liegen. Diese Abfolge wiederholt sich mehrfach, bevorzugt drei-, vier-, fünf- oder sechsmal, so dass bis zu sechs Gene in einem Konstrukt zusammengeführt werden und so zur Expression in die transgene Pflanze eingebracht werden können. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator. Es ist aber auch möglich, mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass durch die die Position die Expression wesentlich beeinflusst wird. Es können in der Expressionskassette in einer vorteilhaften Ausführungsform unterschiedliche Promotoren wie beispielsweise der USP-, LegB4 oder DC3-Promotor und unterschiedliche Terminatoren verwendet werden. In einer weiteren vorteilhaften Ausführungsform können auch identische Promotoren wie der CaMV35S-Promotor verwendet werden.

Wie oben beschrieben sollte die Transkription der eingebrachten Gene vorteilhaft durch geeignete Terminatoren am 3'-Ende der eingebrachten Biosynthesegene (hinter dem Stopcodon) abgebrochen werden. Verwendet werden kann hier z.B. der OCS1- oder der 35SCaMV-Terminator. Wie auch für die Promotoren, sollten hier für jedes Gen unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtspflanzen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure- oder Lipidstoffwechsels enthalten sein oder aber diese Gene können auf einem weiteren oder mehreren weiteren Nukleinsäurekonstrukten liegen. Vorteilhaft werden als Biosynthesegene des Fettsäure- oder Lipidstoffwechsels ein oder mehrere Gene ausgewählt aus der Gruppe Acyl-CoA-Dehydrogenase(n), Acyl-ACP(= acyl carrier prötein)-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) oder Kombinationen davon verwendet. Besonders vorteilhafte Nukleinsäuresequenzen sind Biosynthesegene des Fettsäure- oder Lipidstoffwechsels ausgewählt aus der Gruppe der Acyl-CoA:Lysophospholipid-Acyltransferase, Δ-8-Desaturase, Δ-9-Desaturase, Δ-12-Desaturase und/oder Δ-9-Elongase.

Dabei können die vorgenannten Nukleinsäuren bzw. Gene in Kombination mit anderen Elongasen und Desaturasen in Expressionskassetten, wie den vorgenannten, kloniert werden und zur Transformation von Pflanzen mit Hilfe von Agrobakterium eingesetzt werden.

Der in dieser Beschreibung verwendete Begriff "Vektor" steht für ein Nukleinsäuremolekül, das eine andere Nukleinsäure transportieren kann, an welche es gebunden ist. Ein Vektortyp ist ein "Plasmid", eine zirkuläre doppelsträngige DNA-Schleife, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch auch andere Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die im Verfahren verwendeten Nukleinsäuresequenzen oder das oben beschriebene Genkonstrukt in einer Form, die sich zur Expression der verwendeten Nukleinsäuren in einer Wirtszelle eignet, was bedeutet, dass die rekombinanten Expressionsvektoren eine oder mehrere Regulationssequenzen, die auf der Basis der zur Expression zu verwendenden Wirtszellen ausgewählt ist und die mit der zu exprimierenden Nukleinsäuresequenz funktionsfähig verbunden ist, umfasst. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz ermöglicht wird und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem gewünschten Ausmaß der Expression des Proteins, usw. abhängen kann.

Die verwendeten rekombinanten Expressionsvektoren können zur Expression der im Verfahren verwendeten Nukleinsäuresequenzen so gestaltet sein, dass sie in prokaryotische Zwischenwirte transformiert werden können und schließlich nach Einbringung in die Pflanzen die Expression der Gene in diesen ermöglichen. Dies ist vorteilhaft, da häufig Zwischenschritte der Vektorkonstruktion der Einfachheit halber in Mikroorganismen durchgeführt werden. Beispielsweise können die Δ-6-Desaturase-, Δ-6-Elongase-, Δ-5-Desaturase- und/oder Δ-5-Elongase-Gene in bakteriellen Zellen, Insektenzellen (unter Verwendung von Baculovirus-Expressionsvektoren), Hefe- und anderen Pilzzellen (siehe Romanos, M.A., et al. (1992) Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al. (1999) Marine Biotechnology.1: (3):239-251), Ciliaten, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus (1991) Annu. Rev. Plant Physiol. Plant Molec. Biol. 42: 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryonten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer koexprimierten viralen RNA-Polymerase (T7 gn1) vermittelt wird. Diese virale Polymerase wird von den Wirtsstämmen BL21 (DE3) oder HMS 174 (DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCI, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasecl (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi (J.W. Bennet & L.L. Lasure, Hrsgb., S. 396-428: Academic Press: San Diego). Weitere geeignete Hefevektoren sind beispielsweise pAG-1, YEp6, YEp13 oder pEMBLYe23.

Alternativ können die im erfindungsgemäßen Verfahren verwendeten Nukleinsäuresequenzen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Auch können die im Verfahren verwendeten Gen in einzelligen Pflanzenzellen (wie Algen), siehe Falciatore et al. (1999) Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) Plant Mol. Biol. 20:1195-1197; und Bevan, M.W. (1984) Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al. (1984) EMBO J. 3. 835ff.) oder funktionelle Äquivalente davon, aber auch alle anderen in Pflanzen funktionell aktiven Terminatoren sind geeignet.

Da die Regulation der Pflanzengenexpression sehr oft nicht auf die Transkriptionsebene beschränkt ist, enthält eine Pflanzen-Expressionskassette vorzugsweise andere funktionsfähig verbundene Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al. (1987) Nucl. Acids Research 15:8693-8711).

Die Pflanzengenexpression muss wie oben beschrieben funktionsfähig mit einem geeigneten Promotor verbunden sein, der die Genexpression steuert. Vorteilhaft nutzbare Promotoren sind konstitutive Promotoren (Benfey et al., EMBO J. (1989) 8: 2195-2202), wie diejenigen, die von Pflanzenviren stammen, wie 35S CAMV (Franck et al. (1980) Cell 21: 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen; notwendig sind (siehe eine Übersicht in Kermode (1996) Crit. Rev. Plant Sci. 15 (4): 285-423 und darin zitierte Literaturstellen).

Vektor-DNA lässt sich in prokaryotische oder eukaryotische Zellen über herkömmliche Transfomations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäuren (z.B. DNA) in eine Wirtszelle, einschließlich Calciumphosphat- oder Calciumchlorid-Kopräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartlarid und Davey, Humana Press, Totowa, New Jersey.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das im Verfahren vewendete isolierte Δ-6-Desaturase-, Δ-6-Elongase- oder Δ-5-Desaturase- sowie ggf. das ω-3-Desaturase- oder Δ-4-Desaturase-Molekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt, flankieren.

Die im Verfahren verwendeten Nukleinsäuremoleküle können unter Verwendung molekularbiologischer Standardtechniken und der hier bereitgestellten Sequenzinformation isoliert werden. Auch kann mit Hilfe von Vergleichsalgorithmen beispielsweise eine homologe Sequenz oder homologe, konservierte Sequenzbereiche auf DNA- oder Aminosäureebene identifiziert werden. Diese können als Hybridisierungssonde in Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lassen sich die im Verfahren verwendeten Nukleinsäuremoleküle oder Teile von diesen durch Polymerasekettenreaktion isolieren, wobei Oligonukleotidprimer auf der Basis dieser Sequenz oder von Teilen davon verwendet werden (z.B. kann ein Nukleinsäuremolekül umfassend die vollständige Sequenz oder einen Teil davon durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikägaku America, Inc., St.Petersburg, FL) herstellen. Synthetische Oligonukleotidprimer zur Amplifizierung mittels Polymerasekettenreaktion lassen sich auf der Basis einer der in SEQ ID NO. 64, SEQ ID NO. 1, SEQ ID NO. 171, SEQ ID NO. 51, SEQ ID NO. 193 oder SEQ ID NO. 77 gezeigten Sequenzen oder mit Hilfe der in SEQ ID NO. 65, SEQ ID NO. 2, SEQ ID NO. 172, SEQ ID NO. 52, SEQ ID NO. 194 oder SEQ ID NO. 78 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimem nach Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide können durch Standard-Syntheseverfahren, beilspielsweise mit einem automatischen DNA-Synthesegerät, hergestellt werden.

Homologe der verwendeten Δ-5-Elongase-, ω-3-Desaturase-, Δ-6-Desaturase-, Δ-6-Elongase-, Δ-4-Desaturase- oder Δ-5-Desaturase-Nukleinsäuresequenzen mit der Sequenz SEQ ID NO. 64, SEQ ID NO. 1, SEQ ID NO. 171, SEQ ID NO. 51, SEQ ID NO. 193 oder SEQ ID NO. 77 bedeutet beispielsweise allelische Varianten mit mindestens etwa 40, 50 oder 60 %, vorzugsweise mindestens etwa 60 oder 70 %, stärker bevorzugt mindestens etwa 70 oder 80 %, 90 % oder 95 % und noch stärker bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr Identität bzw. Homologie zu einer der in SEQ ID NO. 64, 66, 68 oder 70, zu einer der in SEQ ID NO. 1, 3, 5, 7, 9, 11, 13, 15, 17, 19, 21, 23, 25, 27, 29, 31, 33, 35, 37, 39 oder 41, zu einer der in SEQ ID NO. 171, 173, 175, 177, 179, 181 oder 183, zu einer der in SEQ ID NO. 51, 53 oder 55, zu einer der in SEQ ID NO. 193 oder 195 oder zu einer der in oder SEQ ID NO. 77, 79, 81, 83, 85, 87, 89, 91 oder 93, insbesondere der in SEQ ID NO. 64, SEQ ID NO. 1, SEQ ID NO: 171, SEQ ID NO. 51, SEQ ID NO. 193 oder SEQ ID NO. 77 gezeigten Nukleotidsequenzen oder ihren Homologen, Derivaten oder Analoga oder Teilen davon. Weiterhin sind isolierte Nukleinsäuremoleküle einer Nukleotidsequenz umfasst, die an eine der in SEQ ID NO. 64, SEQ ID NO. 1, SEQ ID NO. 171, SEQ ID NO. 51, SEQ ID NO. 193 oder SEQ ID NO. 77 gezeigten Nukleotidsequenzen oder einen Teil davon, z.B. unter stringenten Bedingungen hybridisieren. Unter einem Teil gemäß der Erfindung ist dabei zu verstehen, dass mindestens 25 Basenpaare (= bp), 50 bp, 75 bp, 100 bp, 125 bp oder 150 bp, bevorzugt mindestens 175 bp, 200 bp, 225 bp, 250 bp, 275 bp oder 300 bp, besonders bevorzugt 350 bp, 400 bp, 450 bp, 500 bp oder mehr Basenpaare für die Hybridisierung verwendet werden. Es kann auch vorteilhaft die Gesamtsequenz verwendet werden. Allelische Varianten umfassen insbesondere funktionelle Varianten, die sich durch Deletion, Insertion oder Substitution von Nukleotiden aus der in SEQ ID NO. 64, SEQ ID NO. 1, SEQ ID NO. 171, SEQ ID NO. 51, SEQ ID NO. 193 oder SEQ ID NO. 77 dargestellten Sequenz erhalten lassen, wobei aber die Enzymaktivität der davon kodierten Proteine für die Insertion im wesentlichen beibehalten wird.

Für das erfindungsgemäße Verfahren vorteilhafte Nukleinsäuremoleküle können auf der Grundlage ihrer Homologie zu den hier offenbarten ω-3-Desaturase-, Δ-6-Desaturase-, Δ-5-Desaturase-, Δ-5-Elongase-, Δ-4-Desaturase- und/oder Δ-6-Elongase-Nukleinsäuresequenzen unter Verwendung der Sequenzen oder eines Teils davon als Hybridisierungssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und unter stringenten Bedingungen mit den Nukleinsäuretriolekülen, die eine Nukleotidsequenz der SEQ ID NO. 64, SEQ ID NO. 1, SEQ ID NO. 171, SEQ ID NO. 51, SEQ ID NO. 193 oder SEQ ID No. 77 umfassen, hybridisieren. Es können auch Nukleinsäuremoleküle mit mindestens 25, 50, 100, 250 oder mehr Nukleotiden verwendet werden.

Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, bevorzugt mindestens etwa 70 % und besonders bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6. finden. Ein bevorzugtes, nicht einschränkendes Beispiel für stringente Hybridisierungsbedingungen sind Hybridisierungen in 6 x Natriumchlorid/Natriumcitrat (sodium chloride/sodium citrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur liegt beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel, zum Beispiel 50 % Formamid, im oben genannten Puffer vorliegt, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise 30°C bis 45°C. Vorzugsweise sind die Hybridisierungsbedingungen für DNA:RNA-Hybride zum Beispiel 0,1 x SSC und 30°C bis 55°C, vorzugsweise 45°C bis 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Zur Bestimmung der prozentualen Homologie (= Identität) von zwei Aminosäuresequenzen (z.B. einer der Sequenzen der SEQ ID NO. 65, SEQ ID NO. 2, SEQ ID NO. 172, SEQ ID NO. 52, SEQ ID NO. 194 oder SEQ ID NO. 78) oder von zwei Nukleinsäuren (z.B. SEQ ID NO. 64, SEQ ID NO. 1, SEQ ID NO. 171, SEQ ID NO. 51, SEQ ID NO. 193 oder SEQ ID NO. 77) werden die Sequenzen untereinander geschrieben, um sie optimal vergleichen zu können (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäureresten oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die zur Bestimmung der Homologie verwendeten Programme bzw. Algorithmen sind oben beschrieben.

Ein isoliertes Nukleinsäuremolekül, das für eine im Verfahren verwendete ω-3-Desaturase, Δ-6-Desaturase, Δ-5-Desaturase, Δ-5-Elongase, Δ-4-Desaturase und/oder Δ-6-Elongase kodiert, die zu einer Proteinsequenz der SEQ ID NO. 65, SEQ ID NO. 2, SEQ ID NO. 172, SEQ ID NO. 52, SEQ ID NO. 194 oder SEQ ID NO. 78 homolog ist, kann durch Einbringen einer oder mehrerer Nukleotidsubstitutionen, -additionen oder -deletionen in eine Nukleotidsequenz der SEQ ID NO. 64, SEQ ID NO. 1, SEQ ID NO. 171, SEQ ID NO. 51, SEQ ID NO. 193 oder SEQ ID NO. 77 erzeugt werden, so dass eine oder mehrere Aminosäuresubstitutionen,-additionen oder -deletionen in das kodierte Protein eingebracht werden. Mutationen können in eine der Sequenzen der SEQ ID NO. 64, SEQ ID NO. 1, SEQ ID NO. 171, SEQ ID NO. 51, SEQ ID NO. 193 oder SEQ ID NO. 77 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nichtessentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer ω-3-Desaturase, Δ-6-Desaturase, Δ-5-Desaturase, Δ-5-Elongase, Δ-4-Desaturase oder Δ-6-Elongase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der ω-3-Desaturase, Δ-6-Desaturase, Δ-5-Desaturase, Δ-5-Elongase, Δ-4-Desaturase oder Δ**-**6-Elongase kodierenden Sequenz eingebracht werden, z.B. durch Sättigungsmutagenese, und die resultierenden Mutanten können nach der hier beschriebenen ω-3-Desaturase-, Δ-6-Desaturase-, Δ-5-Desaturase-, Δ-5-Elongase-, Δ-4-Desaturase- oder Δ-6-Elongase-Aktivität durchmustert werden, um Mutanten zu identifizieren, die die ω-3-Desaturase-, Δ-6-Desaturase-, Δ-5-Desaturase-, Δ-5-Elongase-, Δ-4-Desaturase- oder Δ-6-Elongase-Aktivität beibehalten haben. Nach der Mutagenese kann das kodierte Protein rekombinant exprimiert werden, und die Aktivität des Proteins kann z.B. unter Verwendung der hier beschriebenen Tests bestimmt werden.

Diese Erfindung wird durch die nachstehenden Beispiele weiter veranschaulicht, die nicht als beschränkend aufgefasst werden sollten.

Die folgende Tabelle zeigt die Sequenzkennzahlen, wie sie in der Prioritätsanmeldung vom 21.02.2006 mit dem deutschen Anmeldeaktenzeichen 102006008030.0 verwendet wurden sowie den entsprechenden Sequenzkennzahlen in der vorliegenden Nachanmeldung. Die durch die SEQ ID No: 1 der Prioritätsanmeldung gekennzeichnete Nukleinsäuresequenz entspricht beispielsweise der durch die SEQ ID NO: 64 der Nachanmeldung gekennzeichneten Nukleinsäuresequenz.

Konkordanztabelle Sequenzkennzahlen der Prioritätsanmeldung und der Sequenzkennzahlen in der Nachanmeldung:

| **SEQ ID NO: Prioritätsanmeldung Deutsches Anmeldeaktenzeichen 102006008030.0** | **SEQ ID NO: vorliegende Nachanmeldung** | **Organism** |
|---|---|---|
| 1 | 64 | Ostreococcus tauri |
| 2 | 65 | Ostreococcus tauri |
| 3 | 1 | Phytium irregulare |
| 4 | 2 | Phytium irregulare |
| 5 | 171 | Traustochytrium sp. |
| 6 | 172 | Traustochytrium sp. |
| 7 | 51 | Thraustochytrium ssp. |
| 8 | 52 | Thraustochytrium ssp. |
| 9 | 193 | Phytophthora infestans |
| 10 | 194 | Phytophthora infestans |
| 11 | 77 | Traustochytrium sp. |
| 12 | 78 | Traustochytrium sp. |
| 13 | 109 | Ostreococcus tauri |
| n.a. | 110 | Ostreococcus tauri |
| 14 | 122 | Ostreococcus tauri |
| n.a. | 123 | Ostreococcus tauri |
| 15 | 143 | Ostreococcus tauri |
| 16 | 144 | Ostreococcus tauri |
| 17 | 161 | Cauliflower mosaic virus |
| 18 | 162 | Cauliflower mosaic virus |
| 19 | 163 | Thalassiosira pseudonana |
| 20 | 164 | Thalassiosira pseudonana |

### Beispiele

### Beispiel 1: Allgemeine Klonierungsverfahren

Die Klonierungsverfahren wie z.B. Restriktionsspaltungen, Agarose-Gelelektrophorese, Reinigung von DNA-Fragmenten, Transfer von Nukleinsäuren auf Nitrozellulose- und Nylon-Membranen, Verknüpfen von DNA-Fragmenteri, Transformation von Escherichia coli-Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA 74: 5463-5467). Fragmente resultierend aus einer PolymeraseKettenreaktion wurden zur Vermeidung von Polymerasefehlem in zu exprimierenden Konstrukten sequenziert und überprüft.

### Beispiel 3: Klonierung von Genen aus Ostreococcus tauri

In einer Ostreococcus tauri Sequenzdatenbank (genomische Sequenzen) konnte durch Suche nach konservierten Bereichen jeweils eine Sequenz kodierend für ein Protein mit Δ-5-Elongaseaktivität oder Δ-6-Elongase-Aktivität identifiziert werden. Es handelt sich dabei um die folgenden Sequenzen:

| Gen-Name | SEQ ID | Aminosäuren |
|---|---|---|
| OtELO1.1, (d-6-Elongase) | SEQ ID NO. 143 | 292 |
| | | |
| OtELO2.1, (Δ-5-Elongase) | SEQ ID NO. 109 | 300 |

OtElo2.1 weist die höchste Ähnlichkeit zu einer Elongase aus Danio rerio auf (GenBank AAN77156; ca. 26 % Identität), während OtElo1.1 die größte Ähnlichkeit zur Elongase aus Physcomitrella (PSE) (ca. 36 % Identität) aufweist (Alignments wurden mit dem tBLASTn-Aalgorithmus (Altschul et al. (1990) J. Mol. Biol. 215: 403 - 410) durchgeführt).

Die Klonierung der Elongasen wurde wie folgt durchgeführt:

40 ml einer Ostreococcus tauri Kultur in der stationären Phase wurden abzentrifugiert und in 100 µl Aqua bidest resuspendiert und bei -20°C gelagert. Mit Hilfe des PCR-Verfahrens wurden die entsprechenden genomischen DNAs amplifiziert. Die entsprechenden Primerpaare wurden so ausgewählt, dass sie die Hefe-Konsensus-Sequenz für hocheffiziente Translation (Kozak (1986) Cell 44: 283-292) neben dem Startcodon trugen. Die Amplifizierung der OtElo-DNAs wurde jeweils mit 1 µl aufgetauten Zellen, 200 µM dNTPs, 2,5 U Taq-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

### Beispiel 4: Optimierung von Elongase-Genen aus Ostreococcus tauri

Elongasen aus dem Organismus Ostreococcus tauri wurden isoliert wie in Beispiel 3 beschrieben. Um eine Steigerung des Gehalts an C22-Fettsäuren zu erreichen, wurden die Sequenzen SEQ ID No. 143 (Δ6-Elongase) und SEQ ID No. 109 (kodierend für ein durch die SEQ ID No. 110 gekennzeichnetes Protein)(Δ5-Elongase) an die Kodonverwendung in Raps, Lein und Soja angepasst. Dazu wurde die Aminosäuresequenz der Δ6-Elongase und der Δ5-Elongase (SEQ ID NO. 144 für die Δ6-Elongase; SEQ ID NO. 65 für die Δ5-Elongase) revers translatiert, wodurch degenerierte DNA-Sequenzen erhalten wurden. Diese DNA-Sequenzen wurden mit Hilfe des Programms GeneOptimizer (Fa. Geneart, Regensburg) an die Kodonverwendung in Raps, Soja und Lein angepasst, wobei die natürliche Häufigkeit einzelner Kodons berücksichtigt wurde. Die so erhaltenen optimierten Sequenzen, die in SEQ ID NO. 64 (Δ5-Elongase) und SEQ ID NO. 122 (kodierend für ein durch die SEQ ID NO. 123 gekennzeichnetes Protein)(Δ6-Elongase) angegeben sind, wurden in vitro synthetisiert.

### Beispiel 5: Klonierung von Expressionsplasmiden zur heterologen Expression in Hefen

Zur Charakterisierung der Funktion der optimierten Nukleinsäuresequenzen wurden die offenen Leserahmen der jeweiligen DNAs stromabwärts des Galactose-induzierbaren GAL1-Promotors von pYES2.1/V5-His-TOPO (Invitrogen) kloniert, wodurch die Plasmide pOTE1.2 (enthaltend die Δ6-Elongase-Sequenz) und pOTE2. 2 (enthaltend die Δ5-Elongase-Sequenz) erhalten wurden.

Übersicht zu den in den Hefe-Vektor pYES2.1/V5-His-TOPO klonierten Elongase-Sequenzen:

| Gen-Name | SEQ ID | Aminosäuren |
|---|---|---|
| pOTE1.1, (Δ-6-Elongase) | SEQ ID NO. 143 | 292 |
| pOTE1.2, (Δ-6-Elongase) | SEQ ID NO. 122 | 292, Kodon-optimiert |
| pOTE2.1, (Δ-5-Elongase) | SEQ ID NO. 109 | 300 |
| pOTE2.2, (Δ-5-Elongase) | SEQ ID NO. 64 | 300, Kodon-optimiert |

Der Saccharomyces cerevisiae-Stamm 334 wurde durch Elektroporation (1500 V) mit den Vektoren pOTE1.2 bzw. pOTE2.2 sowie den Vergleichskonstrukten pOTE1.1 und pOTE2.1, die die natürliche, für die Δ6-Elongase bzw. Δ5-Elongase kodierende Nukleinsäuresequenz enthalten, transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der Ot-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert. 5 ml CMdum-Flüssigmedium (ohne Uracil) mit 2% Raffinose wurden dann mit den Vorkulturen auf eine OD₆₀₀von 0,05 angeimpft. Dabei wurde der Hefekultur, die mit pOTE1.1 und pOTE1.2transformiert worden war, jeweils 0,2mM γ-Linolensäure (GLA) zugegeben. Ausgehend von der Aktivität von OtELO1.1 ist eine Elongation der γ-Linolensäure zur Fettsäure 20:3 zu erwarten. Der Hefekultur, die mit pOTE2.1 und pOTE2.2 transformiert worden war, wurden jeweils 0,2mM Arachidonsäure bzw. Eicosapenfaensäure zugegeben. Entsprechend der Aktivität von OtELO2.1 ist eine Elongation der Fettsäuren ARA bzw. EPA zu den Fettsäuren 22:4 bzw. 22:5 zu erwarten. Die Expression wurde durch die Zugabe von 2% (w/v) Galaktose induziert. Die Kulturen wurden für weitere 96 h bei 20°C inkubiert.

### Beispiel 6: Expression von OtELO2.2 (wie in SEQ ID NO: 64 dargestellt) und OtELO.1.2 (wie in SEQ ID NO: 122) in Hefen

Hefen, die wie in Beispiel 5 mit den Plasmiden pYES2, pOTE1.2, undpOTE2.1 transformiert wurden, wurden folgendermaßen analysiert:

Die Hefezellen aus den Hauptkulturen wurden durch Zentrifugation (100 x g, 5 min, 20°C) geerntet und mit 100 mM NaHCO₃, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM NaHCO₃, pH 8,0 und mit 2 ml Aqua dest. gewaschen. Anschließend wurden die PE-Phasen mit Na₂SO₄ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C (Halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson (2001) Lipids 36(8):761-766; Sayanova et al. (2001) Journal of Experimental Botany 52(360):1581-1585, Sperling et al. (2001) Arch. Biochem. Biophys. 388(2):293-298 und Michaelson et al. (1998) FEBS Letters 439(3):215-218. Die Ergebnisse der Analysen sind in Tabelle 1 dargestellt.

Sowohl für pOTE1.1/pOTE1.2 als auch für pOTE2.1/2.2 konnten die entsprechenden Aktivitäten bestätigt werden. In beiden Fällen zeigte die optimierte Sequenz (pOTE1.2 bzw. pOTE2.2) Aktivität. Dabei konnte die Synthese der γ-Linolensäure durch pOTE1.2 gegenüber der Wildtyp-Sequenz nur geringfügig gesteigert werden. Dagegen konnte für pOTE2.2 überraschenderweise sowohl eine Erhöhung der Aktivität als auch eine Veränderung der Spezifität beobachtet werden (Tabelle 1). Dabei hat sich die Aktivität zur Verlängerung von EPA nahezu verdoppelt, während die Verlängerung von ARA sich mehr als vervierfacht hat. Mit der Optimierung der Sequenz der Δ5-Elongase von Ostreococcus tauri konnte somit in Hefe bei gleicher Substratmenge die Ausbeute an den Vorstufen von DHA 6fach erhöht werden.

### Beispiel 7: Klonierung von Expressionsplasmiden zur Samen-spezifischen Expression in Pflanzen

Die folgenden beschriebenen allgemeinen Bedingungen gelten für alle nachfolgenden Versuche, wenn nicht anders beschrieben.

Erfindungsgemäß bevorzugt verwendet werden für die folgenden Beispiele Bin19, pBI101, pBinAR, pGPTV, pCAMBIA oder pSUN. Eine Übersicht über binäre Vektoren und ihre Verwendung gibt Hellens et al, Trends in Plant Science (2000) 5: 446-451. Verwendet wurde ein pGPTV-Derivat wie in DE10205607 beschrieben. Dieser Vektor unterscheidet sich von pGPTV durch eine zusätzlich eingefügte AscI-Restriktionsschnittstelle.

Ausgangspunkt der Klonierung war der Klonierungsvektor pUC19 (Maniatis et al.). Im ersten Schritt wurde das Conlinin-Promotor-Fragment mit folgenden Primem amplifiziert::
Cnl1 C 5': gaattcggcgcgccgagctcctcgagcaacggttccggcggtatagagttgggtaattcga
Cnl1 C 3': cccgggatcgatgccggcagatctccaccattttttggtggtgat

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym EcoRI und dann für 12 h bei 25°C mit dem Restriktionsenzym SmaI inkubiert. Der Klonierungsvektor pUC19 wurde in gleicher Weise inkubiert. Anschließend wurden das PCR-Produkt und der 2668 bp große, geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1-C wurde durch Sequenzierung verifiziert.

Im nächsten Schritt wurde der OCS-Terminator (Genbank Accession V00088; De Greve, H. et al. (1982) J. Mol. Appl. Genet. 1 (6): 499-511) aus dem Vektor pGPVT-USP/OCS (DE 102 05 607) mit den folgenden Primem amplifiziert:
OCS_C 5': aggcctccatggcctgctttaatgagatatgcgagacgcc
OCS_C 3': cccgggccggacaatcagtaaattgaacggag

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym StuI und dann für 12 h bei 25°C mit dem Restriktionsenzym SmaI inkubiert. Der Vektor pUC19-Cnl1-C wurde 12 h bei 25°C mit dem Restriktionsenzym SmaI inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1C_OCS wurde durch Sequenzierung verifiziert.

Im nächsten Schritt wurde der Cnl1-B Promotor durch PCR mittels folgender Primer amplifiziert:
Cnl1-B 5': aggcctcaacggttccggcggtatag
Cnl1-B 3': cccggggttaacgctagcgggcccgatatcggatcccattttttggtggtgattggttct

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym StuI und dann für 12 h bei 25°C mit dem Restriktionsenzym SmaI inkubiert. Der Vektor pUC19-Cnl1-C wurde 12 h bei 25°C mit dem Restriktionsenzym SmaI inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC 19-Cnl1C_Cnl1B_OCS wurde durch Sequenzierung verifiziert.

In einem weiteren Schritt wurde der OCS-Terminator für Cnl1B eingefügt. Dazu wurde die PCR mit folgenden Primer durchgeführt:
OCS2 5': aggcctcctgctttaatgagatatgcgagac
OCS2 3': cccgggcggacaatcagtaaattgaacggag

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym StuI und dann für 12 h bei 25°C mit dem Restriktionsenzym SmaI inkubiert. Der Vektor pUC19-Cnl1C_Cnl1B_OCS wurde für 12 h bei 25°C mit dem Restriktionsenzym SmaI inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1C_Cnl1B_OCS2 wurde durch Sequenzierung verifiziert.

Im nächsten Schritt wurde der Cnl1-A Promotor durch PCR mittels folgender Primer amplifiziert:
Cnl1-B 5': aggcctcaacggttccggcggtatagag
Cnll-B 3': aggccttctagactgcaggcggccgcccgcattttttggtggtgattggt

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde für 2 h bei 37°C mit dem Restriktionsenzym StuI inkubiert. Der Vektor pUC19-Cnl1-C wurde für 12 h bei 25°C mit dem Restriktionsenzym SmaI inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1C_Cnl1B_Cnl1A_OCS2 wurde durch Sequenzierung verifiziert.

In einem weiteren Schritt wurde der OCS-Terminator für Cnl1A eingefügt. Dazu wurde die PCR mit folgenden Primer durchgeführt:
OCS2 5': ggcctcctgctttaatgagatatgcga
OCS2 3': aagcttggcgcgccgagctcgtcgacggacaatcagtaaattgaacggaga

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym StuI und dann für 2 h bei 37°C mit dem Restriktionsenzym HindIII inkubiert. Der Vektor pUC19_Cnl1C_Cnl1B_Cnl1A_OCS2 wurde für 2 h bei 37°C mit dem Restriktionsenzym StuI und für 2 h bei 37°C mit dem Restriktionsenzym HindIII inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1C_Cnl1B_Cnl1A_OCS3 wurde durch Sequenzierung verifiziert.

Das Plasmid pUC19-Cnl1C_Cnl1B_Cnl1A_OCS3 wurde im nächsten Schritt zur Klonierung der Δ6-, Δ5-Desaturase und Δ6-Elongase verwendet. Dazu wurde die Δ6-Desaturase aus Phytium irregulare (WO02/26946) mit folgenden PCR-Primern amplifiziert:
D6Des(Pir) 5': agatctatggtggacctcaagcctggagtg
D6Des(Pir) 3': ccatggcccgggttacatcgctgggaactcggtgat

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym BgIII und dann für 2 h bei 37°C mit dem Restriktionsenzym NcoI inkubiert. Der Vektor pUC19-Cnl1C_Cnl1B_Cnl1A_OCS3 wurde für 2 h bei 37°C mit dem Restriktionsenzym BglII und für 2 h bei 37°C mit dem Restriktionsenzym NcoI inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC 19-Cnl1_d6Des(Pir) wurde durch Sequenzierung verifiziert.

Das Plasmid pUC19-Cnl1_d6Des(Pir) wurde im nächsten Schritt zur Klonierung der Δ5-Desaturase aus Thraustochytrium ssp. (WO02/26946) verwendet. Dazu wurde die Δ5-Desaturase aus Thraustochytrium ssp. mit folgenden PCR-Primern amplifiziert:
D5Des(Tc) 5': gggatccatgggcaagggcagcgagggccg
D5Des(Tc) 3': ggcgccgacaccaagaagcaggactgagatatc

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym BamHI und dann für 2 h bei 37°C mit dem Restriktionsenzym EcoRV inkubiert. Der Vektor pUC19-Cnl1_d6Des(Pir) wurde für 2 h bei 37°C mit dem Restriktionsenzym BamHI und für 2 h bei 37°C mit dem Restriktionsenzym EcoRV inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1_d6Des(Pir)_d5Des(Tc) wurde durch Sequenzierung verifiziert.

Das Plasmid pUC19-Cnl1_d6Des(Pir)_d5Des(Tc) wurde im nächsten Schritt zur Klonierung der Δ6-Elongase aus Physcomitrella patens (WO01/59128) verwendet, wozu diese mit folgenden PCR-Primern amplifiziert wurde:
D6Elo(Pp) 5': gcggccgcatggaggtcgtggagagattctacggtg
D6E1o(Pp) 3': gcaaaagggagctaaaactgagtgatctaga

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym NotI und dann für 2 h bei 37°C mit dem Restriktionsenzym XbaI inkubiert. Der Vektor pUC19-Cnl1_d6Dcs(Pir)_d5Des(Tc) wurde für 2 h bei 37°C mit dem Restriktionsenzym NotI und für 2 h bei 37°C mit dem Restriktionsenzym XbaI inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von-Roche verwendet. Das entstandene Plasmid pUC19-Cnl1_d6Des(Pir)_d5Des(Tc)_D6Elo(Pp) wurde durch Sequenzierung verifiziert.

Ausgehend von pUC19-Cnl1_d6Des(Pir)_d5Des(Tc)_D6Elo(Pp) wurde der binäre Vektor für die Pflanzentransformation hergestellt. Dazu wurde pUC19-Cnl1_d6Des(Pir)_d5Des(Tc)_D6Elo(Pp) für 2 h bei 37°C mit dem Restriktionsenzym AscI inkubiert. Der Vektor pGPTV wurde in gleicher Weise behandelt. Anschließend wurden das Fragment aus pUC19-Cnl1_d6Des(Pir)_d5Des(Tc)_D6Elo(Pp) und der geschnittene pGPTV-Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pGPTV- Cnl1_d6Des(Pir)_d5Des(Tc)_D6Elo(Pp) wurde durch Sequenzierung verifiziert.

Ein weiteres Konstrukt, pGPTV-Cnl1_d6Des(Pir)_d6Des(Tc)_D6Elo(Pp)_D12Des(Co), fand Verwendung. Dazu wurde ausgehend von pUC19-CnMC_OCS mit folgenden Primem amplifiziert:
Cnl1_OCS 5': gtcgatcaacggttccggcggtatagagttg
Cnl1_OCS 3': gtcgatcggacaatcagtaaattgaacggaga

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10 pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde für 2 h bei 37°C mit dem Restriktionsenzym SalI inkubiert. Der Vektor pUC 19 wurde für 2 h bei 37°C mit dem Restriktionsenzym SalI. inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1_OCS wurde durch Sequenzierung verifiziert.

In einem weiteren Schritt wurde das Δ12-Desaturase-Gen aus Calendula officinalis (WO01/85968) in pUC19-Cnl1_OCS kloniert. Dazu wurde d12Des(Co) mit folgenden Primer amplifiziert:
D12Des(Co) 5': agatctatgggtgcaggcggtcgaatgc
D12Des(Co) 3': ccatggttaaatcttattacgatacc

### Zusammensetzung des PCR-Ansatzes (50 µl):

5,00 µl Template cDNA
5,00 µl 10x Puffer (Advantage-Polymerase) + 25mM MgCl₂
5,00 µl 2mM dNTP
1,25 µl je Primer (10pmol/µl)
0,50 µl Advantage-Polymerase (Clontech)

### Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde für 2 h bei 37°C mit dem Restriktionsenzym BglII und anschließend für 2 h bei gleicher Temperatur mit NcoI inkubiert. Der Vektor pUC219-Cnl1_OCS wurde in gleicher Weise inkubiert. Anschließend wurden das PCR-Fragment und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1_D12Des(Co) wurde durch Sequenzierung verifiziert.

Das Plasmid pUC 19-Cnl1_D 12Des(Co), sowie das Plasmid pUC19-Cnl1_d6Des(Pir)_d5Des(Tc)_D6Elo(Pp) wurden für 2 h bei 37°C mit dem Restriktionsenzym SaII inkubiert. Anschließend wurde das Vektor-Fragment sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und Vektor-Fragment ligiert. Dazu wurde das Rapid Ligation Kit von Roche verwendet. Das entstandene Plasmid pUC19-Cnl1_d6Des(Pir)_d5Des(Tc)_D6Elo(Pp)_D12Des(Co) wurde durch Sequenzierung verifiziert.

Ausgehend von pUC19-Cnl1_d6Des(Pir)_d5Des(Tc)_D6Elo(Pp)_D12Des(Co) wurde der binäre Vektor für die Pflanzentransformation hergestellt. Dazu wurde pUC19_Cnl1_d6Des(Pir)_d5Des(Tc)_D6Elo(Pp)_D12Des(Co) für 2 h bei 37°C mit dem Restriktionsenzym AscI inkubiert. Der Vektor pGPTV wurde in gleicher Weise behandelt. Anschließend wurden das Fragment aus pUC19-Cnl1_d6Des(Pir)_d5Des(Tc)_D6Elo(Pp)_D12Des(Co) und der geschnittene pGPTV-Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pGPTV- Cnl1_d6Des(Pir)_d5Des(Tc)_D6Elo(Pp)_D12Des(Co) wurde durch Sequenzierung verifiziert.

Ein weiteres Beispiel für die Verwendung von samenspezifischen Expressionskonstrukten ist der Napin Promotor. In Wu et al. (2005) Nat. Biotech. 23:1013-1017 ist die Herstellung dieser Expressionskonstrukte in den Vektoren pGPTV oder pSUN beschrieben.

Ein weiterer für die Pflanzentransformation geeigneter Vektor ist pSUN2. Um die Zahl der im Vektor enthaltenen Expressionskassetten auf mehr als vier zu erhöhen wurde dieser Vektor in Kombination mit dem Gateway-System (Invitrogen, Karlsruhe) verwendet. Dazu wurde in den Vektor pSUN2 gemäß Herstellerangaben die Gateway-Kassette A wie folgendermaßen beschrieben, eingefügt:

Der pSUN2 Vektor (1 µg) wurde 1 h mit dem Restriktionsenzym EcoRV bei 37° inkubiert. Anschließend wurde die Gateway-Kassette A (Invitrogen, Karlsruhe) in den geschnittenen Vektor ligiert mittels des Rapid Ligation Kits von Roche, Mannheim. Das entstandene Plasmid wurde in E. coli DB3.1 Zellen (Invitrogen) transformiert. Das isolierte Plasmid pSUN-GW wurde anschließend durch Sequenzierung verifiziert.

Im zweiten Schritt wurde die Expressionskassette aus pUC19-Cnl1_d6Des(Pir)_d5Des(Tc)_D6Elo(Pp)_D12Des(Co) mittels AscI ausgeschnitten und in den in gleicherweise behandelten Vektor pSUN-GW ligiert. Das so entstandene Plasmid pSUN-4G wurde für weitere Genkonstrukte verwendet.

Dazu wurde zuerst gemäß Herstellerangaben (Invitrogen) ein pENTR-Klon modifiziert. Das Plasmid pENTR1A (Invitrogen) wurde 1 h bei 37° mit dem Restriktionsenzym EcoRI inkubiert, anschließend für 30 min mit Klenow-Enzym, sowie einem 1 µM dNTP-Mix behandelt und dann der AscI-Adapter (5'-ggcgcgcc; am 5'-Ende phosphoryliert, doppelsträngig) in den pENTRIA-Vektor liegiert. In diesen modifizierten wurde wie oben beschrieben schrittweise Gene in die Cnl-Kassette eingefügt und über AscI in den pENTR-Vektor übertragen, wodurch der pENTR-Cnl-Vektor entstand.

In einem weiteren Schritt wurde das Konstrukt pSUN-8G hergestellt. Dazu wurden 5',- und 3'-Primer für die Gene mit den SEQ ID NOs: 1, 3, 5 und 7 mit den oben beschriebenen Restriktionsschnittstellen sowie den ersten und jeweils letzten 20 Nukleotiden des offenen Leserahmens erstellt und mit den Standardbedingungen (siehe oben) amplifiziert und in den pENTR-Cnl-Vektor ligiert, der anschließend einer Rekombinationsreaktion nach Herstellerangaben mit dem Vektor pSUN-4G unterworfen wurde.

Dadurch wurde das Konstrukt pSUN-8G hergestellt, das in Brassicajuncea bzw. Brassica napus transformiert wurde. Die Samen der transgenen Pflanzen wurden durch Gaschromatographie analysiert.

Ein weiteres Konstrukt, dass für die Transformation von B. juncea und B. napus verwendet wurde, war das Konstrukt pSUN-9G. Dieses Konstrukt wurde entsprechend zu Wu et al. (2005) Nat. Biotech. 23:1013-1017 mit dem Napin Promotor dargestellt. In Modifikation zu Wu et al. 2005 wurde anstelle des Genes OmELO die kodierende Sequenz von OtELO2.2 in der beschriebenen Art eingesetzt. Das erhaltene Konstrukt pSUN-9G wurde dann in B. juncea bzw. B. napus transformiert.

### Beispiel 8: Lipidextraktion aus Pflanzenmaterial

Die Auswirkung der genetischen Modifikation in Pflanzen auf die Produktion einer gewünschten Verbindung (wie einer Fettsäure) kann bestimmt werden, indem die modifizierte Pflanze unter geeigneten Bedingungen (wie den vorstehend beschriebenen) gezüchtet wird und das Medium und/oder die zellulären Komponenten auf die erhöhte Produktion des gewünschten Produktes (d.h. der Lipide oder einer Fettsäure) untersucht werden. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon A. et al. (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A. et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22): 12935-12940, und Browse et al. (1986) Analytic Biochemistry 152:141-145 beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press. Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan für 1h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

### Beispiel 9: Verwendung der optimierten Δ5-Elongase (wie in SEQ ID NO: 64 dargestellt) aus Ostreococcus tauri für Konstrukte zur konstitutiven Expression

Für die Transformation von Pflanzen wurden Transformationsvektoren auf Basis von pGPTV-35S, eines Plasmids basierend auf pBIN19-35S (Bevan M. (1984) Nucl. Acids Res. 18:203) erzeugt. Dazu wurde zunächst in einem pUC-Vektor eine Expressionskassette bestehend aus dem Promotor-Element CaMV35S (SEQ ID No. 161) sowie dem 35S-Terminator (SEQ ID NO. 162; Franck, A. et al. (1980) Cell 21 (1): 285-294) zusammengesetzt. Dabei wurde der Promotor über die Restriktionsschnittstellen SalI/XbaI und der Terminator über die Restriktionsschnittstellen BamHI/SmaI eingesetzt. An den Terminator wurde außerdem ein Polylinker mit der XhoI Schnittstelle angehängt (,triple ligation'). Das entstandene Plasmid pUC19-35S wurde dann für die Klonierung von PUFA Genen eingesetzt. Es wurden parallel die offenen Leserahmen der Δ6-Desaturase- (SEQ ID NO. 1), der Δ5-Desaturase- (SEQ ID NO. 51) und Δ6-Elongasen- (SEQ ID NO. 171) Sequenzen über die EcoRV-Schnittstelle in pUC19-35S Vektoren eingefügt. Die entstandenen Plasmide pUC-D6, pUC-D5, pUC-E6(Tc) wurden für die Erstellung des binären Vektors pGPTV-35S_D6DSE6(Tc) verwendet. Dazu wurde der Vektor pGPTV mit dem Enzym SalI, das Plasmid pUC-D6 mit SalI/XhoI verdaut und die korrekten Fragmente ligiert. Das entstandene Plasmid pGPTV-D6 wurde anschließend mit SalI, das Plasmid pUC-D5 mit SalI/XhoI verdaut und die korrekten Fragmente ligiert. Das entstandene Plasmid pGPTV-D6-D5 wurde dann ein weiteres Mal mit SalI verdaut, das Plasmid pUC-E6(Tc) mit SalI/XhoI und die korrekten Fragmente ligiert. Aus diesen sequentiellen Klonierungsschritten entstand der binäre Vektor pGPTV-D6D5E6(Tc), der für die Transformation eingesetzt wurde.

In einer weiteren Ausführung wurde an Stelle der Sequenz d6Elo(Tc) die Sequenz von d6Elo(Tp) (SEQ ID NO. 163) in den Vektor pUC19-35S eingesetzt. Das entstandene Plasmid pUC- E6(Tp) wurde zur Herstellung des binären Vektors pGPTV-35S_D6D5E6(Tp) verwendet.

In einer weiteren Ausführung wurde der offene Leserahmen der ω3Des (SEQ ID NO. 193) in pUC19-35S kloniert. Das entstandene Plasmid pUC-ω3Pi wurde über SalI/XhoI in die binären Vektoren pGPTV-D6D5E6(Tc) und pGPTV-D6D5E6(Tp) übertragen. Die entstandenen Vektoren pGPTV-D6D5E6(Tc)ω3Pi und pGPTV-D6D5E6(Tp)ω3Pi wurden für die Pflanzentransformation eingesetzt.

In einer weiteren Ausführung wurde der offene Leserahmen der optimierten Δ5-Elongase aus Ostreococcus tauri (SEQ ID No. 64), sowie der offene Leserahmen der Δ4-Desaturase aus Thraustochytrium sp. (SEQ ID No. 77) in pUC19-35S kloniert. Die entstandenen Plasmide pUC-E5 und pUC-D4 wurden dann über SalI/XhoI entsprechend obiger Angaben in den Vektor pGPTV-D6D5E6(Tp)ω3Pi übertragen. Der entstandene Vektor pGPTV-D6D5E6(Tp)ω3PiE5D4 wurde für die Pflanzentransformation eingesetzt.

Alle binären Vektoren wurden in E. coli DH5α-Zellen (Invitrogen) nach Herstellerangaben transformiert. Positive Klone wurden durch PCR identifiziert und Plasmid-DNA isoliert (Qiagen Dneasy).

### Beispiel 10: Transformation der konstitutiven binären Vektoren in Pflanzen

a) Erzeugung transgene Brassica napus bzw. Brassica juncea Pflanzen Es wurde das Protokoll zur Transformation von Rapsptlanzen verwendet (verändert nach Moloney et al. (1992) Plant Cell Reports 8:238-242).

Zur Erzeugung transgener Pflanzen wurde der binäre Vektor pGPTV-D6D5E6(Tp)ω3PiE5D4 in Agrobacterium tumefaciens C58C1:pGV2260 transformiert (Deblaere et al. (1984) Nucl. Acids. Res. 13: 4777-4788). Zur Transformation von Orychophragmus violaceus wurde eine 1:50 Verdünnung einer Übemachtkultur einer positiv transformierten Agrobakterienkolonie in Murashige-Skoog Medium (Murashige und Skoog (1962) Physiol. Plant. 15: 473) mit 3 % Saccharose (3MS-Medium) verwendet. Petiolen oder Hypokotyledonen frisch gekeimter steriler Pflanzen (zu je ca. 1 cm²) wurden in einer Petrischale mit einer 1:50 Agrobakterienverdünnung für 5-10 Minuten inkubiert. Es folgt eine 3-tägige Koinkubation in Dunkelheit bei 25°C auf 3MS-Medium mit 0,8 % Bacto-Agar. Die Kultivierung wurde anschließend mit 16 Stunden Licht / 8 Stunden Dunkelheit und in wöchentlichem Rhythmus auf MS-Medium mit 500 mg/l Claforan (Cefotaxime-Natrium), 15 mg/l Kanamycin, 20 µM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, wurde dem Medium als Wachstumshormon 2-Indolbuttersäure zum Bewurzeln zugegeben.

Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und mittels Lipidanalysen auf Elongase-Expression wie Δ-6-Elongaseaktivität oder Δ-5- oder Δ-6-Desaturaseaktivität untersucht. Linien mit erhöhten Gehalten an mehrfach ungesättigten C20-und C22-Fettsäuren wurden so identifiziert.

### b) Erzeugung transgener Orychophragmus violaceus Pflanzen

Es wurde das Protokoll zur Transformation von Rapsptlanzen verwendet (verändert nach Moloney et al. (1992) Plant Cell Reports 8:238-242) wie unter a) beschrieben, angewendet.

Zur Erzeugung transgener Pflanzen wurde der binäre Vektor pGPTV-D6D5E6(Tp)ω3PiE5D4 in Agrobacterium tumefaciens C58C 1:pGV2260 transformiert (Deblaere et al. (1984) Nucl. Acids. Res. 13: 4777-4788). Zur Transformation von Orychophragmus violaceus wurde eine 1:50 Verdünnung einer Übernachtkultur einer positiv transformierten Agrobakterienkolonie in Murashige-Skoog Medium (Murashige und Skoog (1962) Physiol. Plant. 15: 473) mit 3 % Saccharose (3MS-Medium) verwendet. Petiolen oder Hypokotyledonen frisch gekeimter steriler Pflanzen (zu je ca. 1 cm²) wurden in einer Petrischale mit einer 1:50 Agrobakterienverdünnung für 5-10 Minuten inkubiert. Es folgt eine 3-tägige Koinkubation in Dunkelheit bei 25°C auf 3MS-Medium mit 0,8 % Bacto-Agar. Die Kultivierung wurde anschließend mit 16 Stunden Licht / 8 Stunden Dunkelheit und in wöchentlichem Rhythmus auf MS-Medium mit 500 mg/l Claforan (Cefotaxime-Natrium), 15 mg/l Kanamycin, 20 µM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, wurde dem Medium als Wachstumshormon 2-Indolbuttersäure zum Bewurzeln zugegeben.

Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und mittels Lipidanalysen auf Elongase-Expression wie Δ-6-Elongaseaktivität oder Δ-5- oder Δ-6-Desaturaseaktivität untersucht. Linien mit erhöhten Gehalten an mehrfach ungesättigten C20- und C22-Fettsäuren wurden so identifiziert.

### c) Transformation von Arabidopsis thaliana Pflanzen

Es wurde das Protokoll von Bechthold et al. (1993) C.R. Acad. Sci. Ser. III Sci. Vie. 316: 1194-1199 angewendet.
Zur Erzeugung transgener Pflanzen wurde der erzeugte binäre Vektor pGPTV-D6D5E6(Tp)ω3PiE5D4 in Agrobacterium tumefaciens C58Cl:pMP90 transformiert (Deblaere et al. (1984) Nucl. Acids. Res. 13: 4777-4788) und gemäß des Protokolls von Bechthold et al. (1993) Blüten von Arabidopsis thaliana cv. Columbia 0 in eine Agrobakterien-Lösung mit OD600=1,0 getaucht. Der Vorgang wurde zwei Tage später nochmals wiederholt. Samen aus diesen Blüten wurden dann auf Agar-Platten mit ½ MS, 2% Saccharose und 50mg/L Kanamycin ausgelegt. Grüne Keimlinge wurden dann auf Erde transferiert.

### Beispiel 11: Analyse von Pflanzenmaterial transgener Orychophragmus- bzw. Arabidopsis-Pflanzen

Die Extraktion von Blattmaterial transgener Orychophragmus violaceus und Arabidopsis thaliana Pflanzen tranformiert mit pGPTV-D6D5E6(Tp)ω3PiE5D4 sowie die gaschromatographische Analyse wurde wie in Beispiel 8 beschrieben durchgeführt. Tabelle 2 zeigt die Ergebnisse der Analysen. Die verschiedenen Fettsäuren sind in Gewichtsprozent angegeben. Für beide unterschiedliche Pflanzenarten konnte die Synthese von langkettigen, mehrfach ungesättigte Fettsäuren gezeigt werden. Überraschenderweise konnte mit der optimierten Sequenz der Δ5-Elongase (wie in SEQ ID NO: 64 dargestellt) aus Ostreococcus tauri eine deutlich höhere Ausbeute an DHA erhalten werden wie dies zum Beispiel von Robert et al. (2005) Functional Plant Biology 32:473-479 für Arabidopsis thaliana mit 1,5% DHA berichtet wird. Für Orychophragmus violaceus konnte zum ersten Mal eine Synthese von langkettigen, mehrfach ungesättigten Fettsäuren erzielt werden.

### Beispiel 12: Analyse von Samen von transgener Brassica juncea Linien

Die Extraktion von Samen von transgenen Brassica juncea Pflanzen tranformiert mit pSUN-9G sowie die gaschromatographische Analyse wurde wie in Beispiel 8 beschrieben durchgeführt. Tabelle 6 zeigt die Ergebnisse der Analysen. Die verschiedenen Fettsäuren sind in Flächenprozent angegeben. Wie in Wu et al. 2005 konnte die Synthese von langkettigen mehrfachungesättigen Fettsäuren (PUFA) gezeigt werden. Überraschenderweise resultierte die Verwendung der modifizierten Elongase-Sequenz OtELO2.2 wie in der durch die SEQ ID NO: 64 beschriebenen Nukleinsäuresequenz in einer dramatischen Erhöhung des Gehalts an C22 Fettsäuren. Insgesamt enthielt das Samenöl etwa 8% Gew.-% mehrfachungsättigte C22-Fettsäuren. Im speziellen war die Fettsäure Docosahexaensäure (DHA) zu 1,9% Gew.-% im Samenöl enthalten, was einer Steigerung um Faktor 10 im Vergleich zu Wu et al. 2005 darstellte.

### Beispiel 13: Detaillierte Analyse der Lipidklassen und Positionsanalyse von Blattmaterial von O. violaceus

Etwa 1 g Blattgewebe wurde für 10 Minuten bei 95°C in in 4 ml of Isopropanol erhitzt, durch Polytron homogenisiert und nach der Zugabe von 1,5 ml Chloroform geschüttelt. Die Proben wurden zentrifugiert, der Überstand gesammelt und das Pellet mit Isopropanol:Chloroform 1:1 (v/v) nochmals extrahiert. Die beiden Extrakte wurden vereinigt, getrocknet und in Chloroform gelöst. Der Lipidextrakt wurde an einer Silica PrepSep-Säule (Fisher Scientific, Nepean, Canada) in neutrale Lipide, Glykolipide und Phospholipide vorfraktioniert, wobei mit Chloroform:Essigsäure 100:1 (v/v), Aceton:Essigsäure 100:1 (v/v) bzw.

Methanol:Chloroform:Wasser 100:50:40 (v/v/v) eluiert wurde. Diese Fraktionen wurden auf Silica G-25 Dünnschichtchromatographieplatten (TLC; Macherey-Nagel, Düren, Germany) weiter aufgetrennt. Neutrale Lipide wurden mit Hexan:Diethylether:Essigsäure (70:30:1), Glykolipide mit Chloroform:Methanol:Ammoniak (65:25:4 v/v/v) und Phospholipide mit Chloroform:Methanol:Ammoniak:Wasser (70:30:4:1 v/v/v/v) entwickelt. Die einzelnen Lipidklassen wurden nach dem Besprühen mit Primulin unter UV-Licht identifiziert, durch Abkratzen von den Platten entfernt, und entweder für die direkte Transmethylierung verwendet oder durch ein geeignetes Lösungsmittel für die weitere Analyse extrahiert.

Entsprechend der beschriebenen Methoden konnten die verschiedenen Lipidklassen (Neutral-, Phospho- und Galaktolipide) aufgetrennt und separat analysiert werden. Desweiteren wurden die Glycolipide auf die Position der einzelnen Fettsäuren untersucht.

### a) Regiospezifische Analyse der Triacylglyceride (TAG)

Drei bis fünf mg der TLC-gereinigten TAG wurden unter Stickstoff in Glasröhrchen getrocknet, in wässrigem Puffer durch kurze Ultraschallbehandlung resuspendiert (1 M Tris pH 8; 2.2% CaCl₂ (w/v); 0.05% Gallensalze (w/v)) und für 4 Minuten bei 40° C inkubiert. Nach der Zugabe von 0.1 ml einer Lösung von Pankreaslipase (10 mg/ml in Wasser) wurden die Proben für 3 Minuten kräftig gevortext und der Verdau durch Zugabe von 1 ml Ethanol und 1,5 ml 4 M HCl abgebrochen. Die teilweise verdauten TAG wurden zweimal mit Diethylether extrahiert, mit Wasser gewaschen, getrocknet und in einem kleinen Volumen Chloroform gelöst. Monoacylglycerole (MAG) wurden von den freien Fettsäuren und unverdauten TAGs auf einer TLC-Platte getrennt wie oben für neutrale Lipide beschrieben. Der Punkt, der den MAGs entsprach, wurde durch GC analyert und stellte die sn-2-Position der TAG dar. Die Verteilung der Fettsäuren an den verbleibenden sn-1- and sn-3-Positionen wurde nach der folgenden Formel berechnet: sn-1 + sn-3 = (TAG x 3 - MAG)/ 2.

Diese Positionsanalyse der Triacylglyceride ergab dabei, dass EPA und DHA in den Positionen sn-2 und sn-1/3 in ähnlichen Konzentrationen vorhanden sind, während ARA insgesamt nur in geringen Mengen in den Triacylglyceriden zu finden ist und hier hauptsächlich in der Position sn-2 (Tab. 3).

### b) Stereospezifische Analyse von Phospholipiden

Aufgetrenntes und extrahiertes Phosphatidylglykol (PG), Phosphatidylethanolamin (PE) und Phosphatidylcholin (PC) wurden unter N₂ getrocknet und in 0.5 ml Boratpuffer (0.5M, pH 7.5, enthaltend 0.4 mM CaCl₂) resuspendiert. Nach einer kurzen Ultraschallbehandlung wurden 5U der Phospholipase A2 aus dem Gift von Naja mossambica (Sigma P-7778) und 2 ml Diethylether zugegeben und die Proben wurden für 2 Stunden bei Raumtemperatur gevortext. Die Etherphase wurde getrocknet, der Verdau mit 0,3 ml 1M HCl abgebrochen, und das Reaktionsgemisch wurde mit Chloroform:Methanol (2:1 v/v) extrahiert. Die verdauten Phospholipide wurden durch TLC in Chloroform:Methanol:Ammoniak:Wasser (70:30:4:2 v/v/v/v) getrennt und Punkte, die den freigesetzten freien Fettsäuren und Lysophosphölipiden entsprachen, wurden durch Kratzen entfernt und direkt transmethyliert.

Die Positionsanalyse der Phospholipide zeigte eine Akkumulation von EPA und DHA in der sn-2 Position von Phosphatidylcholin (PC), während DHA in Phosphatidylethanolamin (PE) in sn-1 und sn-2 Position ähnlich verteilt war. In beiden Phospholipiden war kein oder nur Spuren von ARA zu finden (Tab. 4). In Phosphatidylglycerol fanden sich geringere Konzentrationen an EPA und DHA als in den anderen untersuchten Phospholipiden, wobei auch in dieser Lipidklasse eine Akkumulation in der sn-2 Position zu beobachten war (Tab. 4, PG).

### c) Stereospezifische Analyse von Glykolipiden

Als weitere polare Lipidklasse wurden die Galaktolipide untersucht. Galaktolipide finden sich in den Membranen von Plastiden und bilden dort die Hauptkomponenten.

TLC-gereinigtes Monogalaktosyldiacylglycerol (MGDG) und Digalaktosyldiacylglycerol (DGDG) wurden unter Stickstoff getrocknet und in 0.5 ml Diethylether gelöst. Dann wurden 25 Einheiten der Lipase aus Rhizopus arrhizus (Sigma 62305), resuspendiert in 2 ml Boratepuffer (50 mM, pH 7.5 enthaltend 2 mM CaCl₂), zugegeben und die Proben für 2 Stunden bei Raumtemperatur gevortext. Die Etherphase wurde getrocknet und der Verdau durch die Zugabe von 0,3 ml 1M HCl abgebrochen und die Lipide wurden mit 4 ml Chloroform:Methanol (2:1 v/v) extrahiert. Nach dem Trocknen wurden die verdauten Galaktolipide in einem kleinen Volumen Chloroform:Methanol (2:1 v/v) und zweimal auf einer vorgefertigten Silica TLC-Platte entwickelt, zuerst mit Chloroform:Methanol:Ammoniak:Wasser (70:30:4:1 v/v/v/v) bis etwa zwei Drittel der Plattenhöhe, gefolgt von der vollständigen Entwicklung in Hexan:Diethylether:Essigsäure (70:30:1). Die Punkte, die den freigesetzten freien Fettsäuren und den Lysogalaktolipiden entsprachen, wurden nach dem Besprühen mit Primulin identifiziert, abgekratzt und direkt für die GC-Analyse transmethyliert.

Auch in diesen Lipiden konnten VLCPUFA gefunden werden, wobei eine Akkumulation von EPA in der sn-2 Position zu beobachten war. DHA war nur in den Digalakto-Diacylglycerolen (DGDG) zu finden und konnte nicht in den Monogalakto-Diacylglycerolen (MGDG) nachgewiesen werden (Tabelle 5). Die Verteilung von VLCPUFA in Galaktolipiden, einem Kompartiment, in dem diese Fettsäuren nicht erwartet wurden, zeigt die Dynamik der Synthese und den späteren Umbau. Ernährungstechnisch sind VLCPUFA in Polarlipiden besonders wertvoll, da diese im Darm von Säugetieren besser aufgenommen werden können als die Neutrallipide.

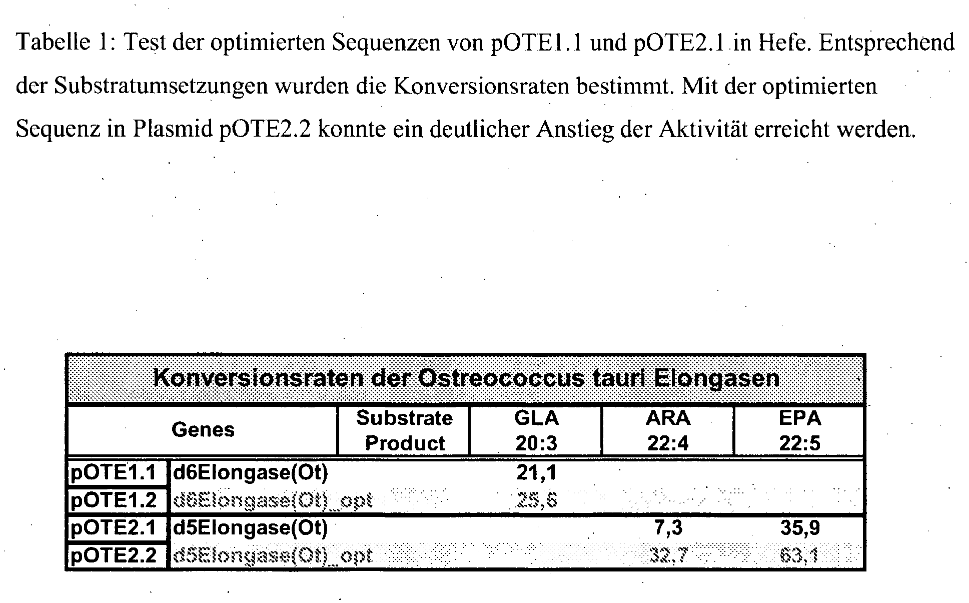

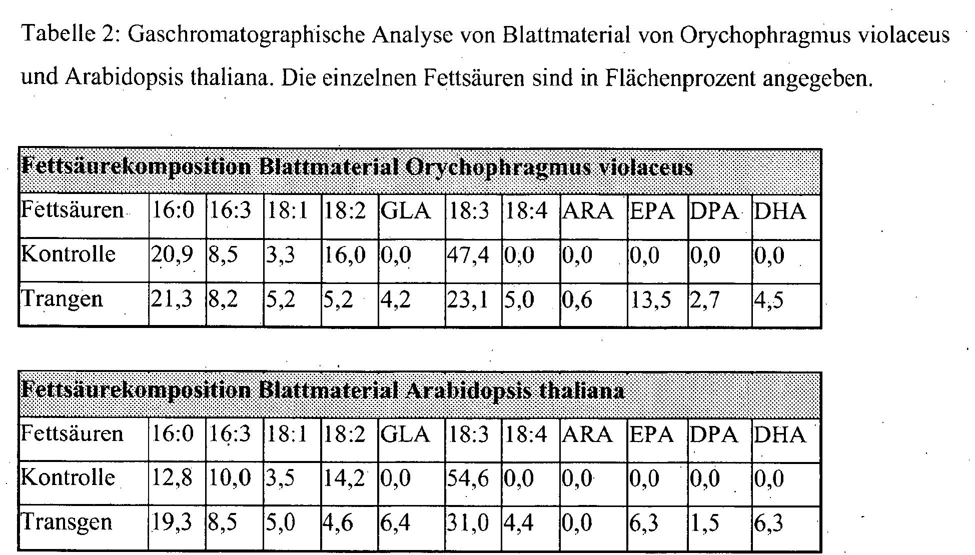

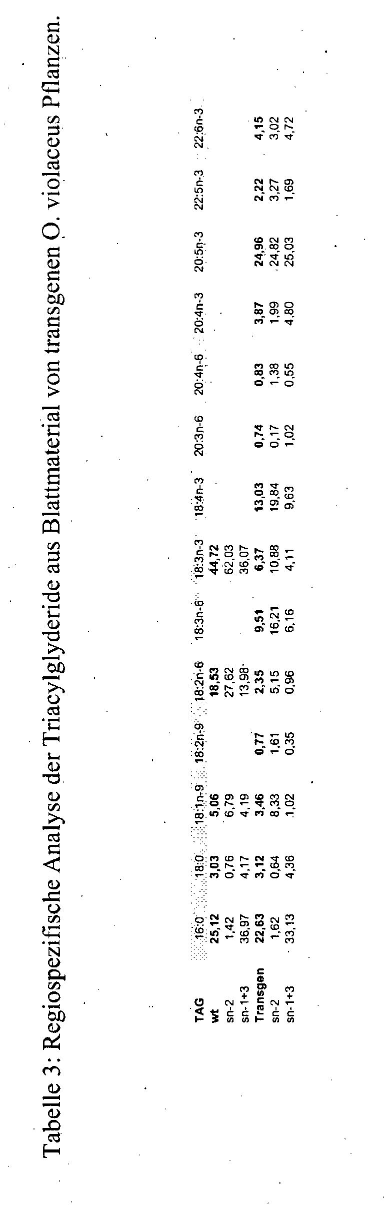

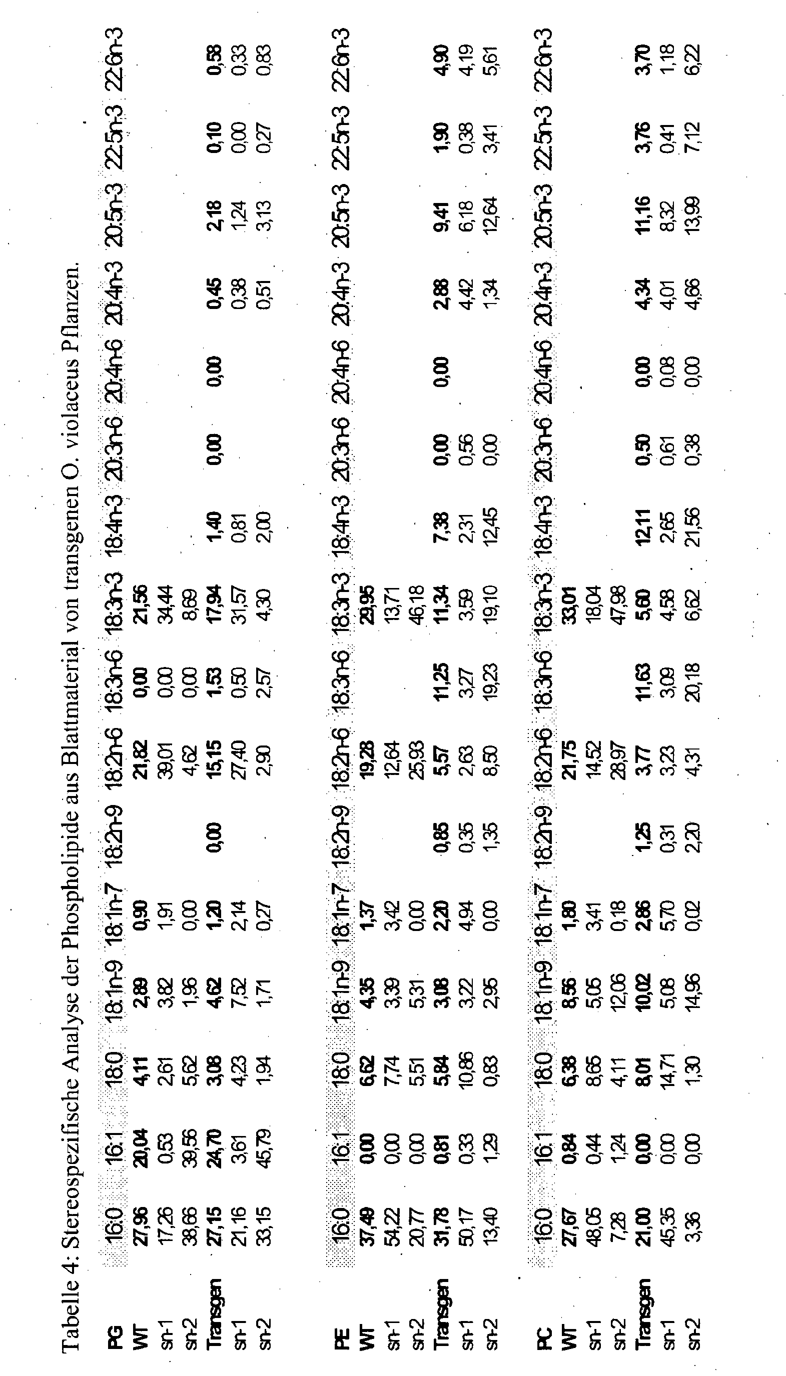

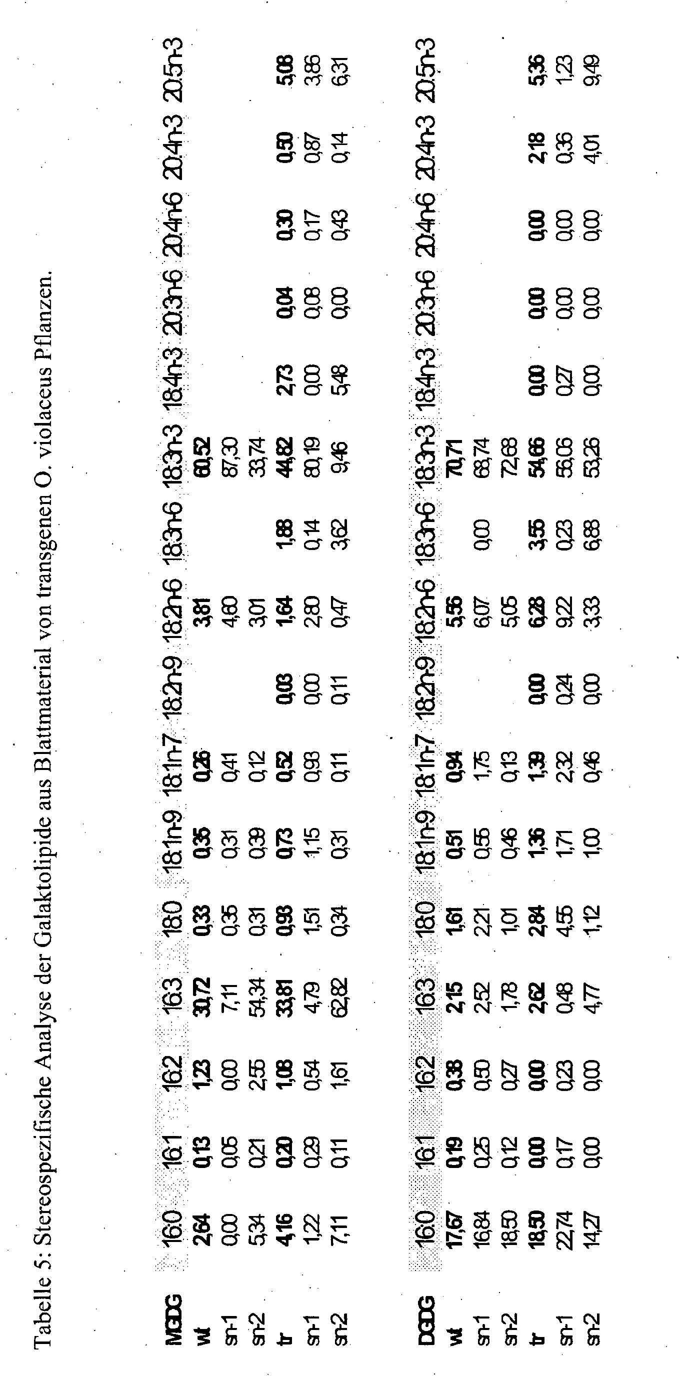

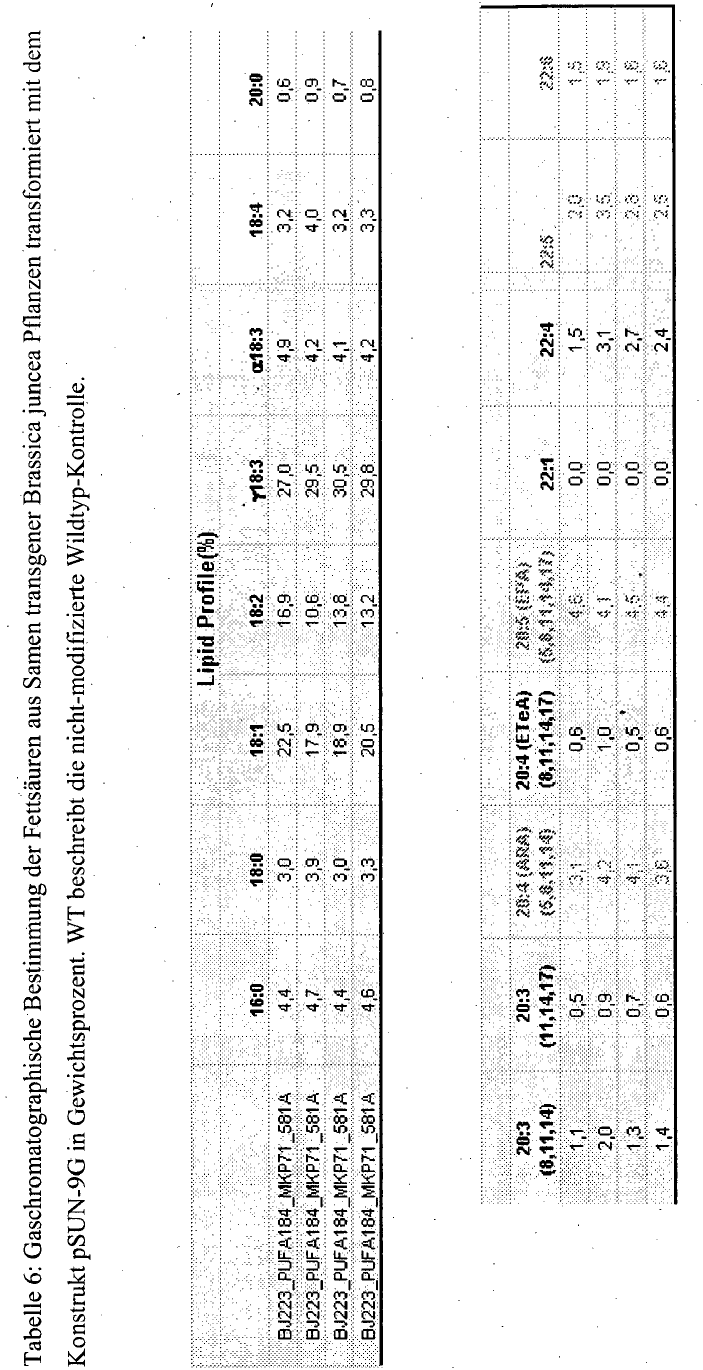

## Patentansprüche

1. Verfahren zur Herstellung von Eicosapentaensäure, Docosapentaensäure und/oder Docosahexaensäure in einer transgenen Pflanze, umfassend das Bereitstellen in der Pflanze von
• mindestens einer Nukleinsäuresequenz, welche für ein Polypeptid oder einem Teil davon mit einer Δ6-Desaturase-Aktivität kodiert mit einer zu mindestens 90% zu SEQ ID NO: 2 identischen Aminosäuresequenz;
• mindestens einer Nukleinsäuresequenz, welche für ein Polypeptid oder einem Teil davon mit einer Δ6-Elongase-Aktivität kodiert mit einer zu mindestens 90% zu SEQ ID NO: 172 identischen Aminosäuresequenz;
• mindestens einer Nukleinsäuresequenz, welche für ein Polypeptid oder einem Teil davon mit einer Δ5-Desaturase-Aktivität kodiert mit einer zu mindestens 90% zu SEQ ID NO: 52 identischen Aminosäuresequenz; und
• mindestens einer Nukleinsäuresequenz, welche für ein Polypeptid oder einem Teil davon mit einer Δ5-Elongase-Aktivität mit einer zu mindestens 90% zu SEQ ID NO: 65 identischen Aminosäuresequenz; und
• soweit das Verfahren die Produktion von Docosahexaensäure (DHA) bezweckt, mindestens einer Nukleinsäure, welche für ein Polypeptid oder einem Teil davon mit einer Δ4-Desaturase-Aktivität kodiert mit einer zu mindestens 90% zu SEQ ID NO: 78 identischen Aminosäuresequenz,
wobei die Nukleinsäuresequenz, welche für ein Polypeptid mit einer Δ5-Elongase-Aktivität kodiert, gegenüber der Nukleinsäuresequenz in dem Organismus, aus dem die Sequenz stammt, dadurch verändert ist, dass sie an die Kodonverwendung in einer oder mehreren Pflanzenarten angepasst ist, und wobei in den transgenen Pflanzen eine Steigerung der Ausbeute an langkettigen mehrfach ungesättigten Fettsäuren (LCPUFAs) von mindestens 50% gegenüber nicht-transgenen Pflanzen erreicht wird.

2. Verfahren nach Anspruch 1, wobei die Nukleinsäuresequenz zumindest an die Kodonverwendung in Raps, Soja und/oder Lein angepasst ist.

3. Verfahren nach einem der Ansprüche 1 oder 2, wobei die Nukleinsäuresequenz unter Berücksichtigung der natürlichen Häufigkeit einzelner Kodons angepasst ist.

4. Verfahren nach einem der Ansprüche 1 bis 3, wobei die veränderte Nukleinsäuresequenz der in SEQ ID No. 64 angegebenen Nukleinsäuresequenz entspricht.

5. Verfahren nach einem der Ansprüche 1 bis 4, wobei die Nukleinsäuresequenzen unter der Kontrolle eines samenspezifischen Promotors exprimiert werden.

6. Verfahren nach Anspruch 5, wobei es sich bei dem Promotor um den USP-, Vicilin-, Napin-, Glp-, SBP-, Peroxireduxin-, Legumin-, Fad3-, Conlinin- oder Oleosin-Promotor handelt.

7. Verfahren nach Anspruch 6, wobei der Gehalt an mehrfachungesättigten C22-Fettsäuren im Samenöl 5 Gew.-% oder mehr des Samenölgehalts beträgt.

8. Verfahren nach einem der vorangehenden Ansprüche, wobei zusätzlich eine oder mehrere Nukleinsäuresequenzen kodierend für ein Polypeptid mit der Aktivität einer ω3-Desaturase in einer Pflanze bereitgestellt wird.

9. Verfahren nach Anspruch 8, wobei der Gehalt an Docosahexaensäure im Samenöl 1 Gew.% oder mehr des Samenölgehalts beträgt.

10. Verfahren nach einem der vorangehenden Ansprüche, wobei die Eicosapentaensäure, Docosapentaensäure und/oder Docosahexaensäure in der Pflanze vorwiegend als Ester in Phospholipiden oder Triacylglyceriden gebunden vorliegt.

11. Verfahren nach einem der vorangehenden Ansprüche, wobei die Pflanze eine Öl-produzierende Pflanze ausgewählt aus der Gruppe bestehend aus Brassica napus, Brassica juncea und Glycine max ist.

12. Verfahren nach einem der vorangehenden Ansprüche, weiter umfassend das Gewinnen der Eicosapentaensäure, Docosapentaensäure und/oder Docosahexaensäure in Form ihrer Öle, Lipide oder freien Fettsäuren aus der Pflanze.

13. Rekombinantes Nukleinsäuremolekül, umfassend:
a) eine oder mehrere Kopien von mindestens einem in Pflanzenzellen aktiven Promotor,
b) mindestens eine Nukleinsäuresequenz, kodierend für ein Polypeptid mit einer Δ6-Desaturase-Aktivität, wobei das Polypeptid eine zu mindestens 90% zu SEQ ID NO. 2 identische Aminosäuresequenz aufweist,
c) mindestens eine Nukleinsäuresequenz, kodierend für ein Polypeptid mit einer Δ5-Desaturase-Aktivität, wobei das Polypeptid eine zu mindestens 90% zu SEQ ID NO. 52 identische Aminosäuresequenz aufweist,
d) mindestens eine Nukleinsäuresequenz, kodierend für ein Polypeptid mit einer Δ6-Elongase-Aktivität, wobei das Polypeptid eine zu mindestens 90% zu SEQ ID NO. 172 identische Aminosäuresequenz aufweist,
e) mindestens eine Nukleinsäuresequenz, kodierend für ein Polypeptid mit einer Δ5-Elongase-Aktivität, wobei das Polypeptid eine zu mindestens 90% zu SEQ ID NO. 65 identische Aminosäuresequenz aufweist und die Nukleinsäuresequenz gegenüber der Nukleinsäuresequenz in dem Organismus, aus dem die Sequenz stammt, dadurch verändert ist, dass sie an die Kodonverwendung in einer oder mehreren Pflanzenarten angepasst ist, und
f) optional eine Nukleinsäuresequenz, kodierend für ein Polypeptid mit einer Δ4-Desaturase-Aktivität, wobei das Polypeptid eine zu mindestens 90% zu SEQ ID NO. 78 identische Aminosäuresequenz aufweist, und
g) eine oder mehrere Kopien von mindestens einer Terminatorsequenz.

14. Rekombinantes Nukleinsäuremolekül nach Anspruch 13, wobei die veränderte Nukleinsäuresequenz der in SEQ ID No. 64 angegebenen Nukleinsäuresequenz entspricht.

15. Rekombinantes Nukleinsäuremolekül nach einem der Ansprüche 13 oder 14, zusätzlich umfassend eine oder mehrere Nukleinsäuresequenzen kodierend für ein Polypeptid mit der Aktivität
a) einer ω3-Desaturase oder
b) einer Δ4-Desaturase zwingend für die Produktion von DHA oder
c) einer ω3-Desaturase und einer Δ4-Desaturase- zwingend für die Produktion von DHA.

16. Transgene Pflanze enthaltend ein rekombinantes Nukleinsäuremolekül nach einem der Ansprüche 13 bis 15, wobei in der transgenen Pflanze eine Steigerung der Ausbeute an langkettigen mehrfach ungesättigten Fettsäuren (LCPUFAs) von mindestens 50% gegenüber nicht-transgenen Pflanzen erreicht wird.

## Claims

1. A process for producing eicosapentaenoic acid, docosapentaenoic acid and/or docosahexaenoic acid in a transgenic plant, comprising the provision, in the plant, of
• at least one nucleic acid sequence which codes for a polypeptide or a part thereof having Δ6-desaturase activity and having an amino acid identity of at least 90% to SEQ ID No: 2;
• at least one nucleic acid sequence which codes for a polypeptide or a part thereof having a Δ6-elongase activity and having an amino acid identity of at least 90% to SEQ ID No: 2;
• at least one nucleic acid sequence which codes for a polypeptide or a part thereof having a Δ5-desaturase activity and having an amino acid identity of at least 90% to SEQ ID No: 52; and
• at least one nucleic acid sequence which codes for a polypeptide or a part thereof having a Δ5-elongase activity and having an amino acid identity of at least 90% to SEQ ID No: 65; and
• insofar as the process aims at producing docosahexaenoic acid (DHA), at least one nucleic acid which codes for a polypeptide or a part thereof having a Δ4-desaturase activity and having an amino acid identity of at least 90% to SEQ ID No: 78,
where the nucleic acid sequence which codes for a polypeptide having a Δ5-elongase activity is modified by comparison with the nucleic acid sequence in the organism from which the sequence is derived in that it is adapted to the codon usage in one or more plant species, and where an increase in the yield of long-chain polyunsaturated fatty acids (LCPUFAs) of at least 50% is achieved in the transgenic plants in comparison with nontransgenic plants.

2. The process according to claim 1, where the nucleic acid sequence is adapted at least to the codon usage in oilseed rape, soybean and/or linseed.

3. The process according to either of claims 1 or 2, where the nucleic acid sequence is adapted taking account of the natural frequency of individual codons.

4. The process according to any of claims 1 to 3, where the modified nucleic acid sequence corresponds to the nucleic acid sequence indicated in SEQ ID No. 64.

5. The process according to any of claims 1 to 4, where the nucleic acid sequences are expressed under the control of a seed-specific promoter.

6. The process according to claim 5, where the promoter is the USP, vicilin, napin, Glp, SBP, peroxireduxin, legumin, Fad3, conlinin or oleosin promoter.

7. The process according to claim 6, where the content of polyunsaturated C22 fatty acids in the seed oil is 5% by weight or more of the seed oil content.

8. The process according to any of the preceding claims, where additionally one or more nucleic acid sequences coding for a polypeptide having the activity of an ω3 desaturase are provided in a plant.

9. The process according to claim 8, where the content of docosahexaenoic acid in the seed oil is 1% by weight or more of the seed oil content.

10. The process according to any of the preceding claims, where the eicosapentaenoic acid, docosapentaenoic acid and/or docosahexaenoic acid is present in the plant mainly bound as ester in phospholipids or triacylglycerides.

11. The process according to any of the preceding claims, where the plant is an oil-producing plant selected from the group consisting of Brassica napus, Brassica juncea and Glycine max.

12. The process according to any of the preceding claims, further comprising the uptake of the eicosapentaenoic acid, docosapentaenoic acid and/or docosahexaenoic acid in the form of their oils, lipids or free fatty acids from the plant.

13. A recombinant nucleic acid molecule comprising:
a) one or more copies of at least one promoter which is active in plant cells,
b) at least one nucleic acid sequence coding for a polypeptide having a Δ6-desaturase activity and having an amino acid identity of at least 90% to SEQ ID No: 2,
c) at least one nucleic acid sequence coding for a polypeptide having a Δ5-desaturase activity and having an amino acid identity of at least 90% to SEQ ID No: 52,
d) at least one nucleic acid sequence coding for a polypeptide having a Δ6-elongase activity and having an amino acid identity of at least 90% to SEQ ID No: 172,
e) at least one nucleic acid sequence coding for a polypeptide having a Δ5-elongase activity and having an amino acid identity of at least 90% to SEQ ID No: 65 and the nucleic acid sequence is modified by comparison with the nucleic acid sequence in the organism from which the sequence is derived by being adapted to the codon usage in one or more plant species, and
f) optionally a nucleic acid sequence coding for a polypeptide having a Δ4-desaturase activity and having an amino acid identity of at least 90% to SEQ ID No: 78, and
g) one or more copies of at least one terminator sequence.

14. The recombinant nucleic acid molecule according to claim 13, where the modified nucleic acid sequence corresponds to the nucleic acid sequence indicated in SEQ ID No. 64.

15. The recombinant nucleic acid molecule according to either of claims 13 or 14, additionally comprising one or more nucleic acid sequences coding for a polypeptide having the activity
a) of an ω3-desaturase or
b) of a Δ4-desaturase which is mandatory for the production of DHA or
c) of an ω3-desaturase and a Δ4-desaturase which is mandatory for the production of DHA.

16. A transgenic plant, comprising a recombinant nucleic acid molecule according to any of claims 13 to 15, wherein an increase in the yield of long-chain polyunsaturated fatty acids (LCPUFAs) of at least 50% is achieved in the transgenic plant in comparison with nontransgenic plants.

## Revendications

1. Procédé de production d'acide éicosapentaénoïque, d'acide docosapentaénoïque et/ou d'acide docosahexaénoïque dans une plante transgénique, comprenant la mise à disposition, dans la plante
- d'au moins une séquence d'acide nucléique qui code pour un polypeptide ou une partie de ce dernier ayant une activité de Δ6-désaturase, ayant une séquence d'acides aminés présentant une identité d'au moins 90 % avec SEQ ID N° 2 ;
- d'au moins une séquence d'acide nucléique qui code pour un polypeptide ou une partie de ce dernier ayant une activité de Δ6-élongase, ayant une séquence d'acides aminés présentant une identité d'au moins 90 % avec SEQ ID N° 172 ;
- d'au moins une séquence d'acide nucléique qui code pour un polypeptide ou une partie de ce dernier ayant une activité de Δ5-désaturase, ayant une séquence d'acides aminés présentant une identité d'au moins 90 % avec SEQ ID N° 52 ; et
- d'au moins une séquence d'acide nucléique qui code pour un polypeptide ou une partie de ce dernier ayant une activité de Δ5-élongase, ayant une séquence d'acides aminés présentant une identité d'au moins 90 % avec SEQ ID N° 65 ; et
- dans la mesure où le procédé vise à la production d'acide docosahexaénoïque (DHA), d'au moins un acide nucléique qui code pour un polypeptide ou une partie de ce dernier ayant une activité de Δ4-désaturase, ayant une séquence d'acides aminés présentant une identité d'au moins 90 % avec SEQ ID N° 78,
dans lequel la séquence d'acide nucléique qui code pour un polypeptide ayant une activité de Δ5-élongase est modifiée, par rapport à la séquence d'acide nucléique dans l'organisme dont provient la séquence, par le fait qu'elle est adaptée à l'utilisation des codons dans une ou plusieurs espèces végétales, et une augmentation du rendement en acides gras polyinsaturés à longue chaîne (LCPUFA) d'au moins 50 % par rapport à des plantes transgéniques est atteinte dans les plantes transgéniques.

2. Procédé selon la revendication 1, dans lequel la séquence d'acide nucléique est adaptée au moins à l'utilisation des codons dans le colza, le soja et/ou le lin.

3. Procédé selon l'une des revendications 1 ou 2, dans lequel la séquence d'acide nucléique est adaptée compte tenu de la fréquence naturelle des codons individuels.

4. Procédé selon l'une des revendications 1 à 3, dans lequel la séquence d'acide nucléique modifiée correspond à la séquence d'acide nucléique présentée dans SEQ ID N°64.

5. Procédé selon l'une des revendications 1 à 4, dans lequel les séquences d'acides nucléiques sont exprimées sous le contrôle d'un promoteur graine-spécifique.

6. Procédé selon la revendication 5, dans lequel, pour ce qui concerne le promoteur, il s'agit du promoteur USP, de la viciline, de la napine, Glp, SBP, de la peroxyréduxine, de la légumine, Fad3, de la conlinine ou de l'oléosine.

7. Procédé selon la revendication 6, dans lequel la teneur de l'huile de graine en acides gras en C22 polyinsaturés est de 5 % en poids ou plus par rapport au poids de l'huile de graine.

8. Procédé selon l'une des revendications précédentes, dans lequel on met en outre à disposition une ou plusieurs séquences d'acides nucléiques codant pour un polypeptide ayant l'activité d'une ω3-désaturase dans une plante.

9. Procédé selon la revendication 8, dans lequel la teneur de l'huile de graine en acide docosahexaénoïque est de 1 % en poids ou plus par rapport au poids de l'huile de graine.

10. Procédé selon l'une des revendications précédentes, dans lequel l'acide éicosapentaénoïque, l'acide docosapentaénoïque et/ou l'acide docosahexaénoïque sont présents dans la plante, liés essentiellement sous forme d'esters dans des phospholipides ou des triacylglycérides.

11. Procédé selon l'une des revendications précédentes, dans lequel la plante est une plante oléagineuse choisie dans le groupe consistant en Brassica napus, Brassica juncea et Glycine max.

12. Procédé selon l'une des revendications précédentes, comprenant en outre l'obtention de l'acide éicocapentaénoïque, de l'acide docosapentaénoïque et/ou de l'acide docosahexaénoïque à partir de la plante, sous forme de leurs huiles, de leurs lipides ou des acides gras libres.

13. Molécule d'acide nucléique recombinante comprenant :
a) une ou plusieurs copies d'au moins un promoteur actif dans les cellules végétales,
b) au moins une séquence d'acide nucléique codant pour un polypeptide ayant une activité de Δ6-désaturase, le polypeptide ayant une séquence d'acides aminés présentant une identité d'au moins 90 % avec SEQ ID N° 2,
c) au moins une séquence d'acide nucléique codant pour un polypeptide ayant une activité de Δ5-désaturase, le polypeptide ayant une séquence d'acides aminés présentant une identité d'au moins 90 % avec SEQ ID N° 52,
d) au moins une séquence d'acide nucléique codant pour un polypeptide ayant une activité de Δ6-élongase, le polypeptide ayant une séquence d'acides aminés présentant une identité d'au moins 90 % avec SEQ ID N° 172,
e) au moins une séquence d'acide nucléique codant pour un polypeptide ayant une activité de Δ5-élongase, le polypeptide ayant une séquence d'acides aminés présentant une identité d'au moins 90 % avec SEQ ID N° 65, et la séquence d'acide nucléique étant modifiée, par rapport à la séquence d'acide nucléique dans l'organisme dont provient la séquence, de telle sorte qu'elle soit adaptée à l'utilisation des codons dans une ou plusieurs espèces végétales, et
f) en option, une séquence d'acide nucléique codant pour un polypeptide ayant une activité de Δ4-désaturase, le polypeptide ayant une séquence d'acides aminés présentant une identité d'au moins 90 % avec SEQ ID N° 78, et
g) une ou plusieurs copies d'au moins une séquence terminatrice.

14. Molécule d'acide nucléique recombinante selon la revendication 13, dans laquelle la séquence d'acide nucléique modifiée correspond à la séquence d'acide nucléique présentée dans SEQ ID N° 64.

15. Molécule d'acide nucléique recombinante selon l'une des revendications 13 ou 14, comprenant en outre une ou plusieurs séquences d'acides nucléiques codant pour un polypeptide ayant l'activité
a) d'une ω3-désaturase, ou
b) d'une Δ4-désaturase nécessaire à la production de DHA, ou
c) d'une ω3-désaturase et d'une Δ4-désaturase, nécessaires à la production de DHA.

16. Plante transgénique contenant une molécule d'acide nucléique recombinante selon l'une des revendications 13 à 15, une augmentation, dans la plante transgénique, du rendement en acides gras polyinsaturés à longue chaîne (LCPUFA) d'au moins 50 % étant atteinte par rapport à des plantes non transgéniques.
